(19) Europäisches Patentamt
European Patent Office
Office européen des brevets

(11) **EP 4 779 300 A1**

(12) **EUROPEAN PATENT APPLICATION**
published in accordance with Art. 153(4) EPC

(43) Date of publication:
**22.07.2026 Bulletin 2026/30**

(21) Application number: **24907359.4**

(22) Date of filing: **16.12.2024**

(51) International Patent Classification (IPC):
***G01N 27/48*** *(2006.01)* ***G01N 27/26*** *(2006.01)*

(52) Cooperative Patent Classification (CPC):
**G01N 33/146; G01N 27/26; G01N 27/48**

(86) International application number:
**PCT/JP2024/044385**

(87) International publication number:
**WO 2025/134974 (26.06.2025 Gazette 2025/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **18.12.2023 JP 2023212599**

(71) Applicant: **Extend Co., Ltd.**
**Tosu-shi, Saga 841-0056 (JP)**

(72) Inventors:
- **OHMAGARI, Shinya**
  **Tosu-shi, Saga 841-0056 (JP)**
- **KITAICHI, Mitsuru**
  **Tosu-shi, Saga 841-0056 (JP)**

(74) Representative: **Appelt, Christian W.**
**Boehmert & Boehmert**
**Anwaltspartnerschaft mbB**
**Pettenkoferstrasse 22**
**80336 München (DE)**

(54) **DIAGNOSTIC DEVICE, DIAGNOSTIC SYSTEM USING SAME, AND PROGRAM TO BE EXECUTED BY COMPUTER**

(57) The diagnostic device calculates sums L(+)_sum, M(+)_sum, H(+)_sum of integral values in a positive predetermined potential section, a sum H(-)_sum of integral values in a negative predetermined potential section, and sums L(all)_sum, M(all)_sum, H(all)_sum of integral values in all predetermined potential sections based on a plurality of integral values $ITG_{1_Low}$ to $ITG_{n_Low}$, $ITG_{1_Middle}$ to $ITG_{n_Middle}$, $ITG_{1_High}$ to $ITG_{n_High}$ in a plurality of predetermined potential sections calculated using the current-potential characteristics of three cyclic voltammograms measured while changing the potential at potential scan rates $V_{r_Low}$, $V_{r_Middle}$, $V_{r_High}$ to diagnose the taste of analyte.

FIG. 12

$CAL_{uni}$

| NAME OF ANALYTE | | | | |
|---|---|---|---|---|
| TYPE OF ANALYTE | | | | |
| CLASS Cls | INTEGRAL VALUE $ITG_{_Low}$ | INTEGRAL VALUE $ITG_{_Middle}$ | INTEGRAL VALUE $ITG_{_High}$ | SUM OF INTEGRAL VALUES $ITG_{ST}$ |
| $Cls_1$ | $ITG_{1_Low}$ | $ITG_{1_Middle}$ | $ITG_{1_High}$ | $ITG_{1_Low}+ITG_{1_Middle}+ITG_{1_High}$ |
| $Cls_2$ | $ITG_{2_Low}$ | $ITG_{2_Middle}$ | $ITG_{2_High}$ | $ITG_{2_Low}+ITG_{2_Middle}+ITG_{2_High}$ |
| $Cls_3$ | $ITG_{3_Low}$ | $ITG_{3_Middle}$ | $ITG_{3_High}$ | $ITG_{3_Low}+ITG_{3_Middle}+ITG_{3_High}$ |
| $Cls_4$ | $ITG_{4_Low}$ | $ITG_{4_Middle}$ | $ITG_{4_High}$ | $ITG_{4_Low}+ITG_{4_Middle}+ITG_{4_High}$ |
| ⋮ | ⋮ | ⋮ | ⋮ | ⋮ |
| $Cls_{n-2}$ | $ITG_{n-2_Low}$ | $ITG_{n-2_Middle}$ | $ITG_{n-2_High}$ | $ITG_{n-2_Low}+ITG_{n-2_Middle}+ITG_{n-2_High}$ |
| $Cls_{n-1}$ | $ITG_{n-1_Low}$ | $ITG_{n-1_Middle}$ | $ITG_{n-1_High}$ | $ITG_{n-1_Low}+ITG_{n-1_Middle}+ITG_{n-1_High}$ |
| $Cls_n$ | $ITG_{n_Low}$ | $ITG_{n_Middle}$ | $ITG_{n_High}$ | $ITG_{n_Low}+ITG_{n_Middle}+ITG_{n_High}$ |

(a)

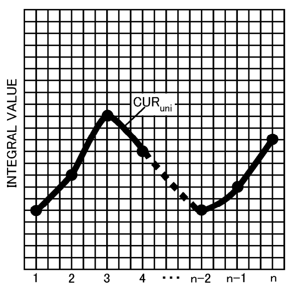


(b)



EP 4 779 300 A1

## Description

[Technical Field]

The present invention relates to a diagnostic device, a diagnostic system using the same, and a program for causing a computer to execute the program.

[Background Art]

In the technology for analyzing solutions of soft drinks, alcoholic beverages, tap water, urine, blood, and the like, analytical methods such as Fourier Transform Infrared Spectroscopy (FTIR), gas chromatography, taste sensors, and Raman spectroscopy are used (NPL 1 to NPL 3).

However, these devices are large and expensive, have problems with portability, and require specialized knowledge to handle the devices due to their complexity.

In addition, although individual sensors for measuring temperature, humidity, total acidity (total acidity of all types of acids in a solution), alcohol content, etc. are capable of on-site measurement (measurement performed on-site), many of them cannot be used alone to judge the state of a solution, and a combination of multiple sensors is required.

Electrochemical sensor can simplify measurement systems, are small, inexpensive, and highly portable, and have great potential as an in situ analytical technique for solutions in general.

[Citation List]

[Non Patent Literature]

Non Patent Literature 1: H. Yu, Y. Zhang, J. Zhao, and H. Tian, "Taste characteristics of Chinese bayberry juice characterized by sensory evaluation, chromatography analysis, and an electronic tongue," J Food Sci Technol 55(5), 1624-1631 (2018).

Non Patent Literature 2: G.F. Abreu, F.M. Borem, L.F.C. Oliveira, M.R. Almeida, and A.P.C. Alves, "Raman spectroscopy: A new strategy for monitoring the quality of green coffee beans during storage," Food Chem 287, 241-248 (2019).

Non Patent Literature 3: R. Ferrer-Gallego, J.M. Hernandez-Hierro, J.C. Rivas-Gonzalo, and M.T. Escribano-Bailon, "Evaluation of sensory parameters of grapes using near infrared spectroscopy," J Food Eng 118(3), 333-339 (2013).

[Summary of Invention]

[Technical Problem]

However, it is difficult to diagnose the taste of alcoholic beverages and the like using an electrochemical sensor system.

Therefore, according to an embodiment of the present invention, a diagnostic device capable of diagnosing the taste of alcoholic beverages and the like based on a cyclic voltammogram of the alcoholic beverages and the like is provided.

Furthermore, according to an embodiment of the present invention, a diagnostic system including a diagnostic device capable of diagnosing the taste of alcoholic beverages and the like based on a cyclic voltammogram of the alcoholic beverages and the like is provided.

Furthermore, according to an embodiment of the present invention, a program for causing a computer to execute diagnosis of the taste of alcoholic beverages or the like based on a cyclic voltammogram of the alcoholic beverages or the like is provided.

[Solution to problem]

Aspect 1

According to an embodiment of the present invention, the diagnostic device includes a first calculation unit and a taste diagnostic unit. The first calculation unit calculates a first sum (L(+)_sum) which is a sum of first integral values in a positive predetermined potential sections based on a plurality of first integral values in a plurality of predetermined potential sections calculated using a current-potential characteristic of a first cyclic voltammogram measured while changing a potential at a first potential scanning rate, calculates a second sum (M(+)_sum) which is a sum of second integral values in the positive predetermined potential sections based on a plurality of second integral values in the plurality of predetermined potential sections calculated using a current-potential characteristic of a second cyclic voltammogram measured while changing the potential at a second potential scanning rate faster than the first potential scanning rate, calculates a third sum (H(+)_sum) which is a sum of third integral values in the positive predetermined potential sections based on a plurality of third integral values in the plurality of predetermined potential sections calculated using a current-potential characteristic of a third cyclic voltammogram measured while changing the potential at a third potential scanning rate faster than the second potential scanning rate, calculates a fourth sum (H(-)_sum) which is a sum of the third integral values in a negative predetermined potential sections based on the plurality of third integral values in the plurality of predetermined potential sections, calculates a fifth sum (L(all))_sum) which is a sum of the first integral values in all of the predetermined potential sections based on the plurality of first integral values in the plurality of predetermined potential sections, calculates a sixth sum (M(all))_sum) which is a sum of the second integral values in all of the predetermined potential sections based on the plurality of second integral values in the plurality of predetermined potential sections, and calculates a seventh sum (H(all))_sum) which is a sum of the third integral values in all of the predetermined potential sections based on the plurality of third integral values in the plurality of predetermined potential sections. The taste diagnostic unit diagnoses a taste of a first analyte based on the first sum (L(+)_sum), the second sum (M(+)_sum), the third sum (H(+)_sum), the fourth sum (H(-)_sum), the fifth sum (L(all))_sum), the sixth sum (M(all))_sum and the seventh sum (H(all))_sum).

Aspect 2

In aspect 1, the first calculation unit further calculates a first factor (Body index (+)) which is a factor attributable to a diffusion coefficient of a component of the first analyte when a positive potential is applied to the first analyte based on the first sum (L(+)_sum), the second sum (M(+)_sum) and the third sum (H(+)_sum) and calculates a second factor (Body index (all)) which is a factor attributable to the diffusion coefficient of the components of the first analyte when positive and negative potentials are applied to the first analyte based on the fifth sum (L(all))_sum), the sixth sum (M(all))_sum and the seventh sum (H(all))_sum). The taste diagnostic unit diagnoses the "astringency" of the first analyte based on the first factor (Body index (+)), diagnoses the "aftertaste" of the first analyte based on the second factor (Body Index (all)), diagnoses the "sweetness" of the first analyte based on the third sum (H(+)_sum), diagnoses the "aroma" of the first analyte based on the fourth sum (H(-)_sum), and diagnoses the "bitterness" of the first analyte based on the "astringency" of the first analyte and the "sweetness" of the first analyte.

Aspect 3

In aspect 2, the taste diagnostic unit diagnoses the multiplication result obtained by multiplying a coefficient $k_1$ to the first factor (Body index (+)) as the "astringency" of the first analyte, diagnoses the multiplication result obtained by multiplying a coefficient $k_2$ to the second factor (Body index (all)) as the "aftertaste" of the first analyte, diagnoses as the "sweetness" of the first analyte the result of multiplying a coefficient $k_4$ to the result of dividing the third sum (H(+)_sum) by the coefficient $k_3$, diagnoses as the "aroma" of the first analyte the result of multiplying a coefficient $k_6$ to the result of dividing the fourth sum (H(-)_sum) by the coefficient $k_5$, and diagnoses as the "bitterness" of the first analyte the result obtained by subtracting the result of multiplying the coefficient $k_8$ to the "sweetness" of the first analyte from the result obtained by multiplying the coefficient $k_7$ to the "astringency" of the first analyte.

Aspect 4

In aspect 3, the taste diagnostic unit performs a regression analysis using the first factor (Body index (+)) as an explanatory variable and "astringency" as a response variable to obtain a regression equation, and determines a value multiplied to the first factor (Body index (+)) in the obtained regression equation as a value of coefficient $k_1$, performs a regression analysis using the second factor (Body index (all)) as an explanatory variable and the "aftertaste" as a response variable to obtain a regression equation, and determines a value multiplied to the second factor (Body index (all)) which is an explanatory variable in the obtained regression equation as the value of the coefficient $k_2$, performs a regression analysis using the third sum (H(+)_sum) as an explanatory variable and "sweetness" as a response variable to obtain a regression equation, and determines a value dividing the explanatory variable (=the third sum (H(+)_sum)) as the value of coefficient $k_3$ in the obtained regression equation, and determines a value multiplied to the explanatory variable (=the third sum (H(+)_sum)) as the value of coefficient $k_4$, performs a regression analysis using the fourth sum (H(-)_sum) as an explanatory variable and "aroma" as a response variable to obtain a regression equation, and determines a value dividing the explanatory variable (the fourth sum (H(-)_sum)) as the value of the coefficient $k_5$ and determines a value multiplied to the explanatory variable (the fourth sum (H(-)_sum)) as the value of the coefficient $k_6$ in the obtained regression equation, performs a regression analysis using the "astringency" and the "sweetness" as explanatory variables and the "bitterness" as a response variable to obtain a regression equation, and determines a value multiplied to the "astringency" as the value of the coefficient $k_7$, and determines a value multiplied to the "sweetness" as the value of the coefficient $k_8$ in the obtained regression equation.

Aspect 5

In aspect 3, when the taste diagnostic unit diagnosed the "astringency", the "aftertaste", the "sweetness", the "aroma" and the "bitterness" of v (v is an integer of 1 or more) first analytes, the taste diagnostic unit updates the values of the coefficients $k_1$ to $k_8$ and diagnoses the "astringency", the "aftertaste", the "sweetness", the "aroma" and the "bitterness" of the first analyte using the updated values of the coefficients $k_1$ to $k_8$.

Aspect 6

In aspect 1, the plurality of first integral values are any of $n^1_1$ ($n^1_1$ is the number of integral values calculated using the smallest predetermined potential section, and is an addition result obtained by adding "1" to an integer obtained by rounding down the decimal point of a division result obtained by dividing a positive potential section by the smallest predetermined potential section when the decimal point of the division result is not zero) first integral values, $n^1_2$ ($n^1_2<n^1_1$) first integral values, $n^1_3$ ($n^1_3<n^1_2$) first integral values, ..., and $n^1_b$ ($n^1_b<n^1_{b-1}$, b is an integer equal to or greater than 2) first integral values,

the plurality of second integral values are any of $n^2_1$ ($n^2_1$ is the number of integral values calculated using the smallest predetermined potential section, and is an addition result obtained by adding "1" to an integer obtained by rounding down the decimal point of a division result obtained by dividing a positive potential section by the smallest predetermined potential section when the decimal point of the division result is not zero) second integral values, $n^2_2$ ($n^2_2<n^2_1$) second integral values, $n^2_3$ ($n^2_3<n^2_2$) second integral values, ..., and $n^2_b$ ($n^2_8<n^2_{8-1}$, b is an integer equal to or greater than 2) second integral values, and
the plurality of third integral values are any of $n^3_1$ ($n^3_1$ is the number of integral values calculated using the smallest predetermined potential section, and is composed of an addition result obtained by adding "1" to an integer obtained by rounding down the decimal point of a division result obtained by dividing a positive potential section by the smallest predetermined potential section when the decimal point of the division result is not zero) third integral values, $n^3_2$ ($n^3_2<n^3_1$) third integral values, $n^3_3$ ($n^3_3<n^3_2$) third integral values, ..., and $n\ ^3_b$ ($n^3_b<n^3_{b-1}$, b is an integer equal to or greater than 2) third integral values.

Aspect 7

In aspect 1, the first calculation unit further calculates an eighth sum (L(-)_sum_th) which is a sum of the first integral values in a plurality of negative predetermined potential sections consisting of negative potentials equal to or lower than a threshold value based on the plurality of first integral values in a plurality of predetermined potential sections calculated using the current-potential characteristic of the first cyclic voltammogram, calculates a ninth sum (M(-)_sum_th) which is a sum of the second integral values in the plurality of negative predetermined potential sections consisting of negative potentials equal to or lower than the threshold value based on the plurality of second integral values in the plurality of predetermined potential sections calculated using the current-potential characteristic of the second cyclic voltammogram, calculates a tenth sum (H(-)_sum_th) which is a sum of the third integral values in the plurality of negative predetermined potential sections consisting of negative potentials equal to or lower than the threshold value based on the plurality of third integral values in the plurality of predetermined potential sections calculated using the current-potential characteristic of the third cyclic voltammogram; calculates a third factor (Body index (-)_th), which is a factor attributable to a diffusion coefficient of a component of the second analyte when a negative potential equal to or lower than the threshold value is applied to the second analyte based on the eighth sum (L(-)_sum_th), the ninth sum (M(-)_sum_th) and the tenth sum (H(-)_sum_th). The taste diagnostic unit further diagnoses the "astringency" of the second analyte based on the third factor (Body index (-)_th).

Aspect 8

In aspect 7, the taste diagnostic unit diagnoses the multiplication result obtained by multiplying a coefficient $k_9$ to the third factor (Body index (-)_th) as the "astringency" of the second analyte.

Aspect 9

In aspect 8, the taste diagnostic unit performs a regression analysis using the third factor (Body index (-)_th) as an explanatory variable and the "astringency" as a response variable to obtain a regression equation, and determines a value multiplied to the third factor (Body index (-)_th) in the obtained regression equation as the value of coefficient $k_9$.

Aspect 10

In aspect 8, when the taste diagnostic unit diagnosed the "astringency" of v (v is an integer equal to or greater than 1) of the second analytes, the taste diagnostic unit updates the value of the coefficient $k_9$ and diagnoses the "astringency" of the second analyte using the updated value of the coefficient $k_9$.

Aspect 11

In aspect 7, the sum of the first integral values in a plurality of negative predetermined potential sections consisting of negative potentials equal to or less than the threshold value is any one of a sum of $w^1_1$ ($w^1_1$ is an addition result obtained by adding "1" to an integer obtained by rounding down a decimal point of a division result obtained by dividing a negative potential section equal to or less than the threshold value by a minimum predetermined potential section when the decimal point of the division result is not zero.) first integral values, a sum of $w^1_2$ ($w^1_2$<$w^1_1$) first integral values, a sum of $w^1_3$ ($w^1_3$<$w^1_2$) first integral values, ..., and a sum of $w^1_b$ ($w^1_b$<$w^1_{b-1}$, b is an integer equal to or greater than 2) first integral values,

> the sum of the second integral values in the plurality of negative predetermined potential sections consisting of negative potentials equal to or less than the threshold value is any one of a sum of $w^2_1$ ($w^2_1$ is an addition result obtained by adding "1" to an integer obtained by rounding down a decimal point of a division result obtained by dividing a negative potential section equal to or less than a threshold value by a smallest predetermined potential section when the decimal point of the division result is not zero.) second integral values, a sum of $w^2_2$ ($w^2_2$<$w^2_1$) second integral values, a sum of $w^2_3$ ($w^2_3$<$w^2_2$) second integral values, ..., and a sum of $w^2_b$ ($w^2_b$<$w^2_{b-1}$, b is an integer equal to or greater than 2) second integral values, and
> the sum of the third integral values in a plurality of negative predetermined potential sections consisting of negative potentials equal to or less than the threshold value is any one of a sum of $w^3_1$ ($w^3_1$ is an addition result obtained by adding "1" to an integer obtained by rounding down a decimal point of a division result obtained by dividing a negative potential section equal to or less than a threshold value by a minimum predetermined potential section when the decimal point of the division result is not zero.) third integral values, a sum of $w^3_2$ ($w^3_2$<$w^3_1$) third integral values, a sum of $w^3_3$ ($w^3_3$<$w^3_2$) third integral values, ..., and a sum of w $^3_b$ ($w^3_b$<$w^3_{b-1}$, b is an integer equal to or greater than 2) third integral values.

Aspect 12

In aspect 1, the diagnostic device further includes a second calculation unit. The second calculation unit calculates a plurality of first integral values in a plurality of predetermined potential sections based on the current-potential characteristics of the first cyclic voltammogram, calculates a plurality of second integral values in a plurality of predetermined potential sections based on the current-potential characteristics of the second cyclic voltammogram, and calculates a plurality of third integral values in a plurality of predetermined potential sections based on the current-potential characteristics of the third cyclic voltammogram. The first calculation unit calculates a first sum (L(+)_sum) and a fifth sum (L(all))_sum based on the plurality of first integral values calculated by the second calculation unit, calculates a second sum (M(+)_sum) and a sixth sum (M(all))_sum based on the plurality of second integral values calculated by the second calculation unit, and calculates a third sum (H(+)_sum), a fourth sum (H(-)_sum) and a seventh sum (H(all))_sum based on the plurality of third integral values calculated by the second calculation unit.

Aspect 13

In aspect 12, the diagnostic device further comprises a creation unit. The creation unit creates, as a feature amount of the first analyte or the second analyte, a curve indicating the dependency of the plurality of sum integral values on the plurality of predetermined potential sections based on the plurality of predetermined potential sections and a plurality of sum integral values respectively corresponding to the plurality of predetermined potential sections. The second calculation unit further calculates the total integral value which is a sum of the first integral value, the second integral value, and the third integral value in one of predetermined potential section for all of the plurality of predetermined potential sections to calculate the plurality of total integral values, and outputs the plurality of predetermined potential sections and the plurality of total integral values respectively associated with the plurality of predetermined potential sections to the creation unit.

Aspect 14

In aspect 13, the calculation data includes a plurality of predetermined potential sections and a plurality of total integral values respectively associated with the plurality of predetermined potential sections. The judgment unit judges whether P (P is an integer equal to or greater than 2) pieces of [a plurality of total integral values] included in P pieces of calculation data are or not different from each other. The creation unit creates P pieces of curves when a judgment unit judges that the P pieces of [plurality of total integral values] are different from each other.

Aspect 15

According to an embodiment of the present invention, a diagnostic system includes the diagnostic device according to any one of aspects 1 to 14.

Aspect 16

Furthermore, according to an embodiment of the present invention, a program causes a computer to execute a first step in which a first calculation unit calculates a first sum (L(+)_sum) which is a sum of first integral values in a positive predetermined potential section based on a plurality of first integral values in a plurality of predetermined potential sections calculated using a current-potential characteristic of a first cyclic voltammogram measured while changing a potential at a first potential scanning rate, calculates a second sum (M(+)_sum) which is a sum of second integral values in a positive predetermined potential section based on a plurality of second integral values in a plurality of predetermined potential sections calculated using a current-potential characteristic of a second cyclic voltammogram measured while changing the potential at a second potential scanning rate faster than the first potential scanning rate, calculates a third sum (H(+) _sum) which is a sum of third integral values in a positive predetermined potential section based on a plurality of third integral values in a plurality of predetermined potential sections calculated using a current-potential characteristic of a third cyclic voltammogram measured while changing the potential at a third potential scanning rate faster than the second potential scanning rate, calculates a fourth sum (H(-)_sum) which is a sum of the third integral values in a negative predetermined potential section based on a plurality of third integral values in the plurality of predetermined potential sections, calculates a fifth sum (L(all))_sum) which is a sum of the first integral values in all of the predetermined potential sections based on the plurality of first integral values in the plurality of predetermined potential sections, calculates a sixth sum (M(all))_sum) which is a sum of the second integral values in all of the predetermined potential sections based on the plurality of second integral values in the plurality of predetermined potential sections, and calculates a seventh sum (H(all)) _sum) which is a sum of the third integral values in all of the predetermined potential sections based on the plurality of third integral values in the plurality of predetermined potential sections, and

a second step in which a taste diagnostic unit diagnoses the taste of the first analyte based on the first sum (L(+)_sum), the second sum (M(+)_sum), the third sum (H(+)_sum), the fourth sum (H(-)_sum), the fifth sum (L(all))_sum), the sixth sum (M(all))_sum and the seventh sum (H(all)_sum).

Aspect 17

In aspect 16, the first calculation unit, in the first step, further calculates a first factor (Body index (+)) which is a factor attributable to the diffusion coefficient of the components of the first analyte when a positive potential is applied to the first analyte based on the first sum (L(+)_sum), the second sum (M(+)_sum), and the third sum (H(+)_sum), and calculates a second factor (Body index (all)) which is a factor attributable to the diffusion coefficient of the components of the first analyte in when positive and negative potentials are applied to the first analyte based on the fifth sum (L(all))_sum), the sixth sum (M(all))_sum), and the seventh sum (H(all))_sum). The taste diagnostic unit, in the second step, diagnoses the "astringency" of the first analyte based on the first factor (Body index (+)), diagnoses the "aftertaste" of the first analyte based on the second factor (Body index (all)), diagnoses the "sweetness" of the first analyte based on the third sum (H(+) _sum), diagnoses the "aroma" of the first analyte based on the fourth sum (H(-)_sum), and diagnoses the "bitterness" of the first analyte based on the "astringency" of the first analyte and the "sweetness" of the first analyte.

Aspect 18

In aspect 17, in the second step, the taste diagnostic unit diagnoses the multiplication result of the first factor (Body index (+)) multiplied by the coefficient $k_1$ as the "astringency" of the first analyte, diagnoses the multiplication result of the second factor (Body index (all)) multiplied by the coefficient $k_2$ as the "aftertaste" of the first analyte, diagnoses the result of multiplying $k_4$ to the result of dividing the third sum (H(+)_sum) by the coefficient $k_3$ as the "sweetness" of the first analyte, diagnoses the result of multiplying the coefficient $k_6$ to the result of dividing the fourth sum (H(-)_sum) by the coefficient $k_5$ as the "aroma" of the first analyte, and diagnoses the result of subtracting the result of multiplying the coefficient $k_8$ to the "sweetness" of the first analyte from the result of multiplying the coefficient $k_7$ to the "astringency" of the first analyte as bitterness of the first analyte.

Aspect 19

In aspect 18, in the second step, the taste diagnostic unit performs a regression analysis using the first factor (Body index (+)) as an explanatory variable and "astringency" as a response variable to obtain a regression equation, and determines the value multiplied to the first factor (Body index (+)) in the obtained regression equation as the value of coefficient $k_1$, performs a regression analysis using the second factor (Body index (all)) as an explanatory variable and "aftertaste" as a response variable to obtain a regression equation, and determines the value multiplied to the second factor (Body index (all)) which is the explanatory variable in the obtained regression equation as the value of coefficient $k_2$, performs a regression analysis using the third sum (H(+)_sum) as an explanatory variable and "sweetness" as a response variable to obtain a regression equation, and determines the value diving the explanatory variable (=the third sum (H(+) _sum)) in the obtained regression equation as the value of the coefficient $k_3$ and determines the value multiplied to the explanatory variable (=the third sum (H(+)_sum)) as the value of the coefficient $k_4$, performs a regression analysis using the fourth sum (H(-)_sum) as an explanatory variable and "aroma" as a response variable to obtain a regression equation, and determines the value diving the explanatory variable (=the fourth sum (H(-)_sum)) in the obtained regression equation as the value of the coefficient $k_5$ and determines the value multiplied to the explanatory variable (=the fourth sum (H(-) _sum)) as the value of the coefficient $k_6$, and performs a regression analysis using "the astringency" and "the sweetness" as an explanatory variable and "bitterness" as a response variable to obtain a regression equation, and determines the value multiplied to "the astringency" in the obtained regression equation as the value of the coefficient $k_7$ and determines the value multiplied to "the sweetness" as the value of the coefficient $k_8$.

Aspect 20

In aspect 18, in the second step, when the taste diagnostic unit diagnosed the "astringency", "aftertaste", "sweetness", "aroma" and "bitterness" of v (v is an integer greater than or equal to 1) first analytes, the taste diagnostic unit updates the values of coefficients $k_1$ to $k_8$ and uses the updated values of coefficients $k_1$ to $k_8$ to diagnose the "astringency", "aftertaste", "sweetness", "aroma" and "bitterness" of the first analytes.

Aspect 21

In aspect 16, the plurality of first integral values are any of $n^1{}_1$ ($n^1{}_1$ is the number of integral values calculated using the smallest predetermined potential section, and is composed of an addition result obtained by adding "1" to an integer obtained by rounding down the decimal point of a division result obtained by dividing a positive potential section by the smallest predetermined potential section when the decimal point of the division result is not zero) first integral values, $n^1{}_2$ ($n^1{}_2 < n^1{}_1$) first integral values, $n^1{}_3$ ($n^1{}_3 < n^1{}_2$) first integral values, ..., and $n^1{}_b$ ($n^1{}_b < n^1{}_{b-1}$, b is an integer of 2 or more) first integral values,

the plurality of second integral values are any of $n^2{}_1$ ($n^2{}_1$ is the number of integral values calculated using the smallest predetermined potential section, and is composed of an addition result obtained by adding "1" to an integer obtained by rounding down the decimal point of a division result obtained by dividing a positive potential section by the smallest predetermined potential section when the decimal point of the division result is not zero) second integral values, $n^2{}_2$ ($n^2{}_2 < n^2{}_1$) second integral values, $n^2{}_3$ ($n^2{}_3 < n^2{}_2$) second integral values, ..., and $n^2{}_b$ ($n^2{}_b < n^2{}_{b-1}$, b is an integer of 2 or more) second integral values, and

the plurality of third integral values are any of $n^3{}_1$ ($n^3{}_1$ is the number of integral values calculated using the smallest predetermined potential section, and is composed of an addition result obtained by adding "1" to an integer obtained by rounding down the decimal point of a division result obtained by dividing a positive potential section by the smallest predetermined potential section when the decimal point of the division result is not zero) third integral values, $n^3{}_2$ ($n^3{}_2 < n^3{}_1$) third integral values, $n^3{}_3$ ($n^3{}_3 < n^3{}_2$) third integral values, ..., and $n^3{}_b$ ($n^3{}_b < n^3{}_{b-1}$, b is an integer of 2 or more) third integral values.

Aspect 22

In aspect 16, in the first step, the first calculation unit further calculates an eighth sum (L(-)_sum_th) which is a sum of the first integral values in a plurality of negative predetermined potential sections consisting of negative potentials equal to or less than a threshold value based on the plurality of first integral values in a plurality of predetermined potential sections calculated using the current-potential characteristic of the first cyclic voltammogram, calculates an ninth sum (M(-)_sum_th) which is a sum of the second integral values in a plurality of negative predetermined potential sections consisting of negative potentials equal to or less than the threshold value based on the plurality of second integral values in a plurality of predetermined potential sections calculated using the current-potential characteristic of the second cyclic voltammogram, calculates an tenth sum (H(-)_sum_th) which is a sum of the third integral values in a plurality of negative predetermined potential sections consisting of negative potentials equal to or less than the threshold value based on the plurality of third integral values in a plurality of predetermined potential sections calculated using the current-potential characteristic of the third cyclic voltammogram, and calculates a third factor (Body index (-)_th) which is a factor attributable to a diffusion coefficient of a component of the second analyte when a negative potential equal to or less than the threshold is applied to the second analyte based on the eighth sum (L(-)_sum_th), the ninth sum (M(-)_sum_th) and the tenth sum (H(-)_sum_th), and
in the second step, the taste diagnostic unit further diagnoses the "astringency" of the second analyte based on the third factor (Body index (-)_th).

Aspect 23

In aspect 22, in the second step, the taste diagnostic unit diagnoses the multiplication result obtained by multiplying the coefficient $k_9$ to the third factor (Body index (-)_th) as the "astringency" of the second analyte.

Aspect 24

In aspect 23, in the second step, the taste diagnostic unit performs regression analysis using the third factor (Body index (-)_th) as the explanatory variable and "astringency" as the objective variable to obtain a regression equation, and determines the value multiplied to the third factor (Body index (-)_th) in the obtained regression equation as the value of coefficient $k_9$.

Aspect 25

In aspect 23, in the second step, when the taste diagnostic unit has diagnosed the "astringency" of v (v is an integer greater than or equal to 1) second analytes, the taste diagnostic unit updates the value of coefficient $k_9$ and diagnoses the "astringency" of the second analytes using the updated value of coefficient $k_9$.

Aspect 26

In aspect 22, the sum of the first integral values in a plurality of negative predetermined potential sections consisting of negative potentials equal to or below the threshold is any one of a sum of $w^1_1$ ($w^1_1$ is the result of adding "1" to the integer obtained by truncating the number equal to or below the decimal point of the division result when number equal to or below the decimal point of the division result of dividing a negative potential section equal to or below the threshold by the minimum specified potential section is not zero.) first integral values, a sum of $w^1_2$ ($w^1_2<w^1_1$) first integral values, a sum of $w^1_3$ ($w^1_3<w^1_2$) first integral values, ..., and a sum of $w^1_b$ ($w^1_b<w^1_{b-1}$, b is an integer equal to or greater than 2) first integral values,

the sum of the second integral values in a plurality of negative predetermined potential sections consisting of negative potentials equal to or below the threshold is any one of a sum of $w^2_1$ ($w^2_1$ is the result of adding "1" to the integer obtained by truncating the number equal to or below the decimal point of the division result when number equal to or below the decimal point of the division result of dividing a negative potential section equal to or below the threshold by the minimum specified potential section is not zero.) second integral values, a sum of $w^2_2$ ($w^2_2<w^2_1$) second integral values, a sum of $w^2_3$ ($w^2_3<w^2_2$) second integral values, ..., and a sum of $w^2_b$ ($w^2_b<w^2_{b-1}$, b is an integer equal to or greater than 2) second integral values, and
the sum of the third integral values in a plurality of negative predetermined potential sections consisting of negative potentials equal to or below the threshold is any one of a sum of $w^3_1$ ($w^3_1$ is the result of adding "1" to the integer obtained by truncating the number equal to or below the decimal point of the division result when number equal to or below the decimal point of the division result of dividing a negative potential section equal to or below the threshold by the minimum specified potential section is not zero.) third integral values, a sum of $w^3_2$ ($w^3_2<w^3_1$) third integral values, a sum of $w^3_3$ ($w^3_3<w^3_2$) third integral values, ..., and a sum of $w^3_b$ ($w^3_b<w^3_{b-1}$, b is an integer equal to or greater than 2) third integral values.

Aspect 27

In aspect 16, the program causes a computer to execute further a third step in which a second calculation unit calculates a plurality of first integral values in a plurality of predetermined potential sections based on the current-potential characteristics of the first cyclic voltammogram, calculates a plurality of second integral values in a plurality of predetermined potential sections based on the current-potential characteristics of the second cyclic voltammogram, and calculates a plurality of third integral values in a plurality of predetermined potential sections based on the current-potential characteristics of the third cyclic voltammogram, and
in the first step, the first calculation unit calculates the first sum (L(+)_sum) and the fifth sum (L(all))_sum) based on the plurality of first integral values calculated by the second calculation unit, calculates the second sum (M(+)_sum) and the sixth sum (M(all))_sum) based on the plurality of second integral values calculated by the second calculation unit, and calculates the third sum (H(+)_sum), the fourth sum (H(-)_sum) and the seventh sum (H(all))_sum) based on the plurality of third integral values calculated by the second calculation unit.

Aspect 28

In aspect 27, the program causes a computer to execute further a fourth step in which the creation unit creates a curve showing the dependence of the plurality of sum integral values on the plurality of predetermined potential sections based on the plurality of predetermined potential sections and the plurality of sum integral values respectively corresponding to the plurality of predetermined potential sections, as a feature amount of the first analyte or the second analyte, and
in the third step, the second calculation unit further execute, for all of the plurality of predetermined potential sections, to calculate a total integral value which is the sum of the first integral value, the second integral value and the third integral value in one predetermined potential section to calculate a plurality of total integral values, and outputs the plurality of predetermined potential sections and the plurality of total integral values respectively corresponding to the plurality of predetermined potential sections to the creation unit.

Aspect 29

In aspect 28, calculation data includes the plurality of predetermined potential sections and the plurality of total integral values respectively corresponding to the plurality of predetermined potential sections,

the program causes the computer to execute further a fifth step in which a judgment unit judges whether P (P is an integer equal to or greater than 2) pieces of [a plurality of sum integral values] included in P pieces of calculation data are or not different from each other, and
the creation unit creates P pieces of curves in the fourth step when the judgment unit judges in the fifth step that the P pieces of [plurality of multiple sum integral values] are different from each other.

[Advantageous Effects of Invention]

According to the embodiment of the present invention, the diagnostic device can diagnose the taste of alcoholic beverages and the like based on a cyclic voltammogram of the alcoholic beverages and the like.

[Brief Description of Drawings]

[Fig. 1]
Fig. 1 is a schematic diagram of a diagnostic system according to a first embodiment of the present invention.
[Fig. 2]
Fig. 2 is a schematic diagram of the sensor device 1 shown in Fig. 1.
[Fig. 3]
Fig. 3 is a perspective view of the measuring instrument 12 shown in Fig. 2.
[Fig. 4]

Fig. 4 is a schematic diagram of the measuring instrument 12 shown in Fig. 2.
[Fig. 5]
Fig. 5 is a schematic diagram showing a timing chart of the potential supplied to the sensor 11 shown in Fig. 2.
[Fig. 6]
Fig. 6 is a schematic diagram of the measurement data MRS.
[Fig. 7]
Fig. 7 is a schematic diagram of the diagnostic device 2 shown in Fig 1.
[Fig. 8]
Fig. 8 s a schematic diagram of the analysis/diagnostic unit 21 shown in Fig. 7.
[Fig. 9]
Fig. 9 is a first conceptual diagram for explaining a method for calculating an integral value.
[Fig. 10]
Fig. 10 is a second conceptual diagram for explaining a method for calculating an integral value.
[Fig. 11]
Fig. 11 is a figure showing a part of a cyclic voltammogram.
[Fig. 12]
Fig. 12 is a diagram for explaining a method for creating a curve CUR showing the relationship between a plurality of classes Cls and a plurality of integral values ITG.
[Fig. 13]
Fig. 13 is a conceptual diagram showing the class dependency of the integral value created based on cyclic voltammograms measured while changing a potential scanning rate to the potential scanning rate $V_{r_Low}$, $V_{r_Middle}$, and $V_{r_High}$.
[Fig. 14]
Fig. 14 is a conceptual diagram of the teacher data.
[Fig. 15]
Fig. 15 is a conceptual diagram of a correspondence table showing the correspondence between coefficients and teacher data.
[Fig. 16]
Fig. 16 is a conceptual diagram of updated teacher data.
[Fig. 17]
Fig. 17 is a conceptual diagram of a correspondence table TBL1_up1 obtained by updating the correspondence table TBL1 shown in Fig. 15.
[Fig. 18]
Fig. 18 is a conceptual diagram for diagnosing the taste (astringency, aftertaste, sweetness, aroma, bitterness) of shochu.
[Fig. 19]
Fig. 19 is a diagram showing the integral value spectra for the shochu of samples No. 1 to No. 3 shown in Table 2.
[Fig. 20]
Fig. 20 is a diagram showing the integral value spectra for the shochu of samples No. 4 to No. 6 shown in Table 2.
[Fig. 21]
Fig. 21 is a diagram showing the integral value spectra for the shochu of samples No. 7 to No. 9 shown in Table 2.
[Fig. 22]
Fig. 22 is a diagram showing the integral value spectra for the shochu of samples No. 10 to No. 12 shown in Table 2.
[Fig. 23]
Fig. 23 is a diagram showing the integral value spectra for the shochu of samples No. 12 to No. 14 shown in Table 2.
[Fig. 24]
Fig. 24 is a diagram showing the integral value spectra for the shochu of samples No. 15 to No. 16 shown in Table 2.
[Fig. 25]
Fig. 25 is a diagram showing the integral value spectra for the shochu of samples No. 17 to No. 20 shown in Table 2.
[Fig. 26]
Fig. 26 is a first diagram showing judgment results on whether the curves k1 to k20 shown in Figs. 19 to 25 are or not different from one another.
[Fig. 27]
Fig. 27 is a second diagram showing judgment results on whether the curves $k_1$ to k20 shown in Figs. 19 to 25 are or not different from one another.
[Fig. 28]
Fig. 28 is a third diagram showing judgment results on whether the curves k1 to k20 shown in Figs. 19 to 25 are or not different from one another.

[Fig. 29]
Fig. 29 is a diagram showing the diagnosis results of "astringency," "aftertaste," "sweetness," "aroma," and "bitterness" for Shochu of No. 1 to No. 20 shown in Table 2.
[Fig. 30]
Fig. 30 is a diagram showing other diagnosis results of the "astringency", "aftertaste", "sweetness", "aroma" and "bitterness" for Shochu of No. 1 to No. 20 shown in Table 2.
[Fig. 31]
Fig. 31 is a diagram showing the differences between the respective values of the diagnosis results for Shochu of No. 1 to No. 20 shown in Fig. 29 and the respective values of the diagnosis results for Shochu of No. 1 to No. 20 shown in Fig. 30.
[Fig. 32]
Fig. 32 is a diagram showing integral spectra for Crimson Seedless (skin+flesh), Green Seedless (skin+flesh) and Shine Muscat (skin+flesh).
[Fig. 33]
Fig. 33 is a diagram showing integral spectra for Crimson Seedless (flesh), Green Seedless (flesh), and Shine Muscat (flesh).
[Fig. 34]
Fig. 34 is a diagram showing the result of judgment on whether the curves k21 to k26 shown in FIG. 32 and FIG. 33 are or not different from each other.
[Fig. 35]
Fig. 35 is a diagram showing the diagnosis results of "astringency" for Green Seedless (flesh only), Crimson Seedless (flesh only), Shine Muscat (flesh only), Green Seedless (flesh + skin), Crimson Seedless (flesh + skin), and Shine Muscat (flesh + skin).
[Fig. 36]
Fig. 36 is a diagram showing integral value spectra for the shochu of samples No. 1 to No. 3 shown in Table 2, in the case where the number of integral values is 138.
[Fig. 37]
Fig. 37 is a diagram showing integral value spectra for the shochu of samples No. 4 to No. 6 shown in Table 2, in the case where the number of integral values is 138.
[Fig. 38]
Fig. 38 is a diagram showing integral value spectra for the shochu of samples No. 7 to No. 9 shown in Table 2, in the case where the number of integral values is 138.
[Fig. 39]
Fig. 39 is a diagram showing integral value spectra for the shochu of samples No. 10 to No. 12 shown in Table 2, in the case where the number of integral values is 138.
[Fig. 40]
Fig. 40 is a diagram showing integral value spectra for the shochu of samples No. 12 to No. 14 shown in Table 2, in the case where the number of integral values is 138.
[Fig. 41]
Fig. 41 is a diagram showing integral value spectra for the shochu of samples No. 15 to No. 16 shown in Table 2, in the case where the number of integral values is 138.
[Fig. 42]
Fig. 42 is a diagram showing integral value spectra for the shochu of samples No. 17 to No. 20 shown in Table 2, in the case where the number of integral values is 138.
[Fig. 43]
Fig. 43 is a first diagram showing the result of judgment on whether the curves k27 to k46 shown in Figs. 36 to 42 are or not different from each other.
[Fig. 44]
Fig. 44 is a second diagram showing the result of judgment on whether the curves k27 to k46 shown in Figs. 36 to 42 are or not different from each other.
[Fig. 45]
Fig. 45 is a third diagram showing the result of judgment on whether the curves k27 to k46 shown in Figs. 36 to 42 are or not different from each other.
[Fig. 46]
Fig. 46 is a diagram showing integral value spectra for the shochu of samples No. 1 to No. 3 shown in Table 2, in the case where the number of integral values is 70.
[Fig. 47]
Fig. 47 is a diagram showing integral value spectra for the shochu of samples No. 4 to No. 6 shown in Table 2, in the case where the number of integral values is 70.

[Fig. 48]

Fig. 48 is a diagram showing integral value spectra for the shochu of samples No. 7 to No. 9 shown in Table 2, in the case where the number of integral values is 70.

[Fig. 49]

Fig. 49 is a diagram showing integral value spectra for the shochu of samples No. 10 to No. 12 shown in Table 2, in the case where the number of integral values is 70.

[Fig. 50]

Fig. 50 is a diagram showing integral value spectra for the shochu of samples No. 12 to No. 14 shown in Table 2, in the case where the number of integral values is 70.

[Fig. 51]

Fig. 51 is a diagram showing integral value spectra for the shochu of samples No. 15 to No. 16 shown in Table 2, in the case where the number of integral values is 70.

[Fig. 52]

Fig. 52 is a diagram showing integral value spectra for the shochu of samples No. 17 to No. 20 shown in Table 2, in the case where the number of integral values is 70.

[Fig. 53]

Fig. 53 is a first diagram showing the result of judgment on whether the curves k47 to k66 shown in Figs. 46 to 52 are or not different from each other.

[Fig. 54]

Fig. 54 is a second diagram showing the result of judgment on whether the curves k47 to k66 shown in Figs. 46 to 52 are or not different from each other.

[Fig. 55]

Fig. 55 is a third diagram showing the result of judgment on whether the curves k47 to k66 shown in Figs. 46 to 52 are or not different from each other.

[Fig. 56]

Fig. 56 is a diagram showing integral value spectra for the shochu of samples No. 1 to No. 3 shown in Table 2, in the case where the number of integral values is 36.

[Fig. 57]

Fig. 57 is a diagram showing integral value spectra for the shochu of samples No. 4 to No. 6 shown in Table 2, in the case where the number of integral values is 36.

[Fig. 58]

Fig. 58 is a diagram showing integral value spectra for the shochu of samples No. 7 to No. 9 shown in Table 2, in the case where the number of integral values is 36.

[Fig. 59]

Fig. 59 is a diagram showing integral value spectra for the shochu of samples No. 10 to No. 12 shown in Table 2, in the case where the number of integral values is 36.

[Fig. 60]

Fig. 60 is a diagram showing integral value spectra for the shochu of samples No. 12 to No. 14 shown in Table 2, in the case where the number of integral values is 36.

[Fig. 61]

Fig. 61 is a diagram showing integral value spectra for the shochu of samples No. 15 to No. 16 shown in Table 2, in the case where the number of integral values is 36.

[Fig. 62]

Fig. 62 is a diagram showing integral value spectra for the shochu of samples No. 17 to No. 20 shown in Table 2, in the case where the number of integral values is 36.

[Fig. 63]

Fig. 63 is a first diagram showing the result of judgment on whether the curves k67 to k86 shown in Figs. 56 to 62 are or not different from each other.

[Fig. 64]

Fig. 64 is a second diagram showing the result of judgment on whether the curves k67 to k86 shown in Figs. 56 to 62 are or not different from each other.

[Fig. 65]

Fig. 65 is a third diagram showing the result of judgment on whether the curves k67 to k86 shown in Figs. 56 to 62 are or not different from each other.

[Fig. 66]

Fig. 66 is a diagram showing integral value spectra for the shochu of samples No. 1 to No. 3 shown in Table 2, in the case where the number of integral values is 18.

[Fig. 67]
Fig. 67 is a diagram showing integral value spectra for the shochu of samples No. 4 to No. 6 shown in Table 2, in the case where the number of integral values is 18.
[Fig. 68]
Fig. 68 is a diagram showing integral value spectra for the shochu of samples No. 7 to No. 9 shown in Table 2, in the case where the number of integral values is 18.
[Fig. 69]
Fig. 69 is a diagram showing integral value spectra for the shochu of samples No. 10 to No. 12 shown in Table 2, in the case where the number of integral values is 18.
[Fig. 70]
Fig. 70 is a diagram showing integral value spectra for the shochu of samples No. 12 to No. 14 shown in Table 2, in the case where the number of integral values is 18.
[Fig. 71]
Fig. 71 is a diagram showing integral value spectra for the shochu of samples No. 15 to No. 16 shown in Table 2, in the case where the number of integral values is 18.
[Fig. 72]
Fig. 72 is a diagram showing integral value spectra for the shochu of samples No. 17 to No. 20 shown in Table 2, in the case where the number of integral values is 18.
[Fig. 73]
Fig. 73 is a first diagram showing the result of judgment on whether the curves k87 to k106 shown in Figs. 66 to 72 are or not different from each other.
[Fig. 74]
Fig. 74 is a second diagram showing the result of judgment on whether the curves k87 to k106 shown in Figs. 66 to 72 are or not different from each other.
[Fig. 75]
Fig. 75 is a third diagram showing the result of judgment on whether the curves k87 to k106 shown in Figs. 66 to 72 are or not different from each other.
[Fig. 76]
Fig. 76 is a diagram showing the correspondence between the classes and integral values when the number of integral values is 138 and the classes and integral values when the number of integral values is 276.
[Fig. 77]
Fig. 77 is a diagram showing integral value spectra of Crimson Seedless (skin+flesh), Green Seedless (skin+flesh) and Shine Muscat (skin+flesh) in when the number of integral values in a specified potential section in the range of -1362 mV to -2501 mV is 74.
[Fig. 78]
Fig. 78 is a diagram showing integral value spectra of Crimson Seedless (only flesh), Green Seedless (only flesh) and Shine Muscat (only flesh) when the number of integral values in a specified potential section in the range of -1362 mV to -2501 mV is 74.
[Fig. 79]
Fig. 79 is a diagram showing the result of judgment on whether the curves k107 to k112 shown in Fig. 77 and Fig. 78 are or not different from each other.
[Fig. 80]
Fig. 80 is a diagram showing integral value spectra of Crimson Seedless (skin+flesh), Green Seedless (skin+flesh) and Shine Muscat (skin+flesh) in when the number of integral values in a specified potential section in the range of -1362 mV to -2501 mV is 37.
[Fig. 81]
Fig. 81 is a diagram showing integral value spectra of Crimson Seedless (only flesh), Green Seedless (only flesh) and Shine Muscat (only flesh) in when the number of integral values in a specified potential section in the range of -1362 mV to -2501 mV is 37.
[Fig. 82]
Fig. 82 is a diagram showing the result of judgment on whether the curves k113 to k118 shown in Fig. 80 and Fig. 81 are or not different from each other.
[Fig. 83]
Fig. 83 is a diagram showing integral value spectra of Crimson Seedless (skin+flesh), Green Seedless (skin+flesh) and Shine Muscat (skin+flesh) in when the number of integral values in a specified potential section in the range of -1362 mV to -2501 mV is 19.
[Fig. 84]
Fig. 84 is a diagram showing integral value spectra of Crimson Seedless (only flesh), Green Seedless (only flesh) and Shine Muscat (only flesh) in when the number of integral values in a specified potential section in the range of -1362 mV to -2501 mV is 19.

[Fig. 85]

Fig. 85 is a diagram showing the result of judgment on whether the curves k119 to k124 shown in Fig. 83 and Fig. 84 are or not different from each other.

[Fig. 86]

Fig. 86 is a diagram showing integral value spectra of Crimson Seedless (skin+flesh), Green Seedless (skin+flesh) and Shine Muscat (skin+flesh) in when the number of integral values in a specified potential section in the range of -1362 mV to -2501 mV is 9.

[Fig. 87]

Fig. 87 is a diagram showing integral value spectra of Crimson Seedless (only flesh), Green Seedless (only flesh) and Shine Muscat (only flesh) in when the number of integral values in a specified potential section in the range of -1362 mV to -2501 mV is 9.

[Fig. 88]

Fig. 88 is a diagram showing the result of judgment on whether the curves k125 to k130 shown in Fig. 86 and Fig. 87 are or not different from each other.

[Fig. 89]

Fig. 89 is a diagram showing the correspondence between the classes and integral values in when the number of integral values is 37 and the classes and integral values in when the number of integral values is 74.

[Fig. 90]

Fig. 90 is a flowchart for explaining the operation of the diagnostic system 10 shown in Fig. 1.

[Fig. 91]

Fig. 91 is a flowchart for explaining the detailed operation of step S7 in Fig. 90.

[Fig. 92]

Fig. 92 is a flowchart for explaining the detailed operation of step S73 in Fig. 91.

[Fig. 93]

Fig. 93 is a flowchart for explaining the detailed operation of step S72 in Fig. 91.

[Fig. 94]

Fig. 94 is a flowchart for explaining the detailed operation of step S75 in Fig. 91.

[Fig. 95]

Fig. 95 is a flowchart for explaining the detailed operation of step S76 in Fig. 91.

[Fig. 96]

Fig. 96 is a flowchart for explaining the detailed operation of step S77 in Fig. 91.

[Fig. 97]

Fig. 97 is a flowchart for explaining the detailed operation of step S8 in Fig. 90.

[Fig. 98]

Fig. 98 is a flowchart for explaining the detailed operation of step S82 in Fig. 97.

[Fig. 99]

Fig. 99 is a flowchart for explaining the detailed operation of step S830 in Fig. 98.

[Fig. 100]

Fig. 100 is another flowchart for explaining the detailed operation of step S830 in Fig. 98.

[Fig. 101]

Fig. 101 is a flowchart for explaining the detailed operation of step S83 in Fig. 97.

[Fig. 102]

Fig. 102 is another flowchart for explaining the detailed operation of step S83 in Fig. 97.

[Fig. 103]

Fig. 103 is a diagram showing the correspondence relationship of classes in a positive predetermined potential section when the number of integral values in when the analyte is "shochu" is 18, 20, 32, 36, 56, 70, 92, 138 and 276.

[Fig. 104]

Fig. 104 is a diagram showing the correspondence relationship of classes in a negative predetermined potential section when the number of integral values in when the analyte is "shochu" is 18, 20, 32, 36, 56, 70, 92, 138 and 276.

[Fig. 105]

Fig. 105 is a diagram showing the correspondence relationship of classes when the number of integral values in when the analyte is "grapes" is 7, 9, 11, 13, 15, 19, 25, 37 and 74.

[Fig. 106]

Fig. 106 is a conceptual diagram of a judgment result indicating whether two curves $CUR_i$, $CUR_j$ of ${}_PC_2$ pairs are or not different.

[Fig. 107]

Fig. 107 is a conceptual diagram showing the update from the analytical data $ALY_D_{uni}$ to the index data $IDX_{uni}$ in step S74 of Fig. 91.
[Fig. 108]
Fig. 108 is a conceptual diagram showing the updating of P pieces of analysis data $ALY_D_1$ to $ALY_D_P$ to P pieces of index data $IDX_1$ to $IDX_P$ in step S78 of Fig. 91.
[Fig. 109]
Fig. 109 is a conceptual diagram showing the results of a taste diagnosis.
[Fig. 110]
Fig. 110 is a schematic diagram of a diagnostic system according to the second embodiment.
[Fig. 111]
Fig. 111 is a schematic diagram of the terminal device 3 shown in Fig. 110.
[Fig. 112]
Fig. 112 is a schematic diagram of the diagnostic device 2A shown in Fig. 110.
[Fig. 113]
Fig. 113 is a schematic diagram of the analysis/diagnostic unit 21A shown in Fig. 112.
[Fig. 114]
Fig. 114 is a first flowchart for explaining the operation of the diagnostic system 10A shown in Fig. 110.
[Fig. 115]
Fig. 115 is a second flowchart for explaining the operation of the diagnostic system 10A shown in Fig. 110.
[Fig. 116]
Fig. 116 is a diagram for explaining the locations of the sensor device 1, the terminal device 3, and the diagnostic device 2A.
[Fig. 117]
Fig. 117 is a schematic diagram of a diagnostic system according to the third embodiment.
[Fig. 118]
Fig. 118 is a schematic diagram of the terminal device 3A shown in Fig. 117.
[Fig. 119]
Fig. 119 is a schematic diagram of the analysis device 2C shown in Fig. 117.
[Fig. 120]
Fig. 120 is a schematic diagram of the diagnostic device 2B shown in Fig. 117.
[Fig. 121]
Fig. 121 is a first flowchart for explaining the operation of the diagnostic system 10B shown in Fig. 117.
[Fig. 122]
Fig. 122 is a second flowchart for explaining the operation of the diagnostic system 10B shown in Fig. 117.
[Fig. 123]
Fig. 123 is a diagram for explaining the locations of the sensor device 1, the terminal device 3A, the analysis device 2C, and the diagnostic device 2B.
[Fig. 124]
Fig. 124 is another schematic diagram of the measuring instrument 12 shown in Fig. 4.
[Fig. 125]
Fig. 125 is another schematic diagram of the diagnostic device 2 shown in Fig. 7.
[Fig. 126]
Fig. 126 is a schematic diagram of the analysis/diagnostic circuit 21B shown in Fig 125.
[Fig. 127]
Fig. 127 is another schematic diagram of the terminal device 3 shown in Fig 111.
[Fig. 128]
Fig. 128 is another schematic diagram of the diagnostic device 2A shown in Fig. 112.
[Fig. 129]
Fig. 129 is another schematic diagram of the analysis/diagnostic unit 21A shown in Fig 113.
[Fig. 130]
Fig. 130 is another schematic diagram of the terminal device 3A shown in Fig. 118.
[Fig. 131]
Fig. 131 is another schematic diagram of the analysis device 2C shown in Fig. 119.
[Fig. 132]
Fig. 132 is another schematic diagram of the diagnostic device 2B shown in Fig120.

[Description of Embodiments]

Embodiments of the present invention will be described in detail in conjunction with the accompanying drawings. Note that the same or corresponding portions in the drawings are denoted by the same reference numerals and their descriptions will not be repeated.

[First Embodiment]

Fig. 1 is a schematic diagram of a diagnostic system according to a first embodiment of the present invention. Referring to Fig. 1, a diagnostic system 10 according to a first embodiment of the present invention includes a sensor device 1 and a diagnostic device 2.
The diagnostic system 10 is placed, for example, in restaurants such as Japanese restaurants, Chinese restaurants, and Western restaurants, sake breweries that brew shochu, liquor stores that sell shochu, ant etc.
The sensor device 1 measures measurement data of cyclic voltammogram of an analyte consisting of, for example, shochu or grapes (grape juice) by a cyclic voltammetry method, and transmits the measurement data of cyclic voltammogram to the diagnostic device 2 by wireless communication or wired communication.
The cyclic voltammetry method (CV) is a measurement method in which electrodes are placed in a stationary solution, analyzes the current-potential curve (cyclic voltammogram CVG) obtained by analyzing the current-potential curve (cyclic voltammogram CVG) obtained by measuring the current that flows when the potential is repeatedly swept to examine redox properties, etc.
The cyclic voltammogram CVG is a current-potential curve measured by cyclic voltammetry method, and the measurement data of the cyclic voltammogram CVG includes current-potential characteristics (I-V) that have associated the current I with the potential V.
When the sensor device 1 transmits the measurement data of the cyclic voltammogram CVG to the diagnostic device 2 by wireless communication, the sensor device 1 transmits the measurement data of the cyclic voltammogram CVG to the diagnostic device 2 by wireless communication, for example, via Bluetooth (registered trademark).
Furthermore, when the sensor device 1 transmits the measurement data of the cyclic voltammogram CVG to the diagnostic device 2 by wired communication, the sensor device 1 is connected to the diagnostic device 2 by a cable, and transmits the measurement data to the diagnostic device 2 via the cable.
The diagnostic device 2 receives measurement data of the cyclic voltammogram CVG from the sensor device 1 by wireless communication or wired communication. The diagnostic device 2 then calculates, for all of the predetermined potential sections, an integral value in a predetermined potential section of the current-potential characteristic (I-V) included in the measurement data based on the measurement data of the cyclic voltammogram CVG using a method described below, thereby calculating a plurality of integral values in the plurality of predetermined potential sections, and creates a curve CUR indicating the dependency of the integral value on the predetermined potential section based on the calculated plurality of integral values in the plurality of predetermined potential sections as [an index curve which is a curve that serves as an index when identifying the analyte], and diagnoses the taste of shochu or grapes (grape juice) based on the plurality of integral values in the plurality of predetermined potential sections, and displays the diagnosis result.
Fig. 2 is a schematic diagram of the sensor device 1 shown in Fig. 1. Fig. 3 is a perspective view of the measuring instrument 12 shown in Fig. 2.
Referring to Fig. 2, the sensor device 1 includes a sensor 11 and a measuring instrument 12. The sensor 11 includes a substrate 111, a working electrode 112, a counter electrode 113, a reference electrode 114, and wires 115-117.
In Fig. 2, an x-y plane is defined. The substrate 111 has, for example, a flat plate shape and is disposed along the x-y plane.
The wirings 115 to 117 are placed on the upper surface of the substrate 111 along the x-axis direction (first direction). The wiring 116 is disposed along the x-axis direction (first direction) at a predetermined interval (for example, 2 to 3 mm) from the wiring 115 in the y-axis direction (second direction perpendicular to the first direction). Moreover, the wiring 117 is disposed along the x-axis direction (first direction) at a predetermined interval (for example, 2 to 3 mm) from the wiring 116 in the y-axis direction (second direction perpendicular to the first direction).
The working electrode 112 is disposed on one end of the wiring 115 opposite the measuring instrument 12 side of the wiring 115 and is electrically connected to the wiring 115. The counter electrode 113 is disposed on one end of the wiring 116 opposite the measuring instrument 12 side of the wiring 116 and is electrically connected to the wiring 116. The reference electrode 114 is disposed on one end of the wiring 117 opposite the measuring instrument 12 side of the wiring 117 and is electrically connected to the wiring 117.
The substrate 111 is, for example, a printed circuit board (PCB: Printed Circuit Board), a plastic plate, or a glass epoxy substrate, and has, for example, a width of 12 mm, a length of 80 mm, and a thickness of 1 mm. The working electrode 112 is made of, for example, any one of boron (B) doped diamond (BDD), a carbon electrode, glassy carbon (glassy diamond), gold (Au) and platinum (Pt). The counter electrode 113 is made of, for example, gold (Au). The reference electrode 114 is made of, for example, gold (Au) or Ag/AgCl.

When the working electrode 12 is made of diamond, the diamond may be either single crystal diamond or polycrystalline diamond, but the polycrystalline diamond is preferred. In this case, the polycrystalline diamond is further preferable that the dangling bonds on the outermost surface of the polycrystalline diamond are terminated with hydrogen.

The working electrode 112 has, for example, a rectangular planar shape with an area of $3 \times 3$ mm$^2$, the counter electrode 113 has, for example, a rectangular planar shape with an area of $3 \times 3$ mm$^2$, and the reference electrode 114 has, for example, a rectangular planar shape with an area of $1 \times 2$ mm$^2$.

When the working electrode 112 is made of diamond or gold, the working electrode 112 has a planar shape that is, for example, a circular shape with a diameter of 3.5 mm.

Furthermore, when the working electrode 112 is made of glassy carbon, the cyclic voltammogram CVG can be measured over a wide range.

The working electrode 112 is an electrode that transfers electrons to and from the analyte. The counter electrode 113 is an electrode that returns to the system a current value that is the same as the current value generated at the working electrode 112. The reference electrode 114 is an electrode that serves as a reference when determining the potential of the working electrode 112.

An analyte consisting of shochu or grapes (grape juice) is supplied to a region in which a working electrode 112, a counter electrode 113 and a reference electrode 114 are arranged.

Referring to Fig. 3, measuring instrument 12 has a recess 121A into which a portion of the other end side of sensor 11 is inserted.

When the sensor 11 is electrically connected to the measuring instrument 12, a portion of the other end side of the sensor 11 in the x-axis direction (first direction) is inserted into a recess 121 A of the measuring instrument 12. As a result, the wiring 115 to 117 of the sensor 11 is electrically connected to the measuring instrument 12. Furthermore, when the sensor 11 is not electrically connected to the measuring instrument 12, a portion of the other end side of the sensor 11 in the x-axis direction (first direction) is pulled out from the recess 121 A of the measuring instrument 12.

Therefore, by attaching and detaching a portion of the other end of the sensor 11 in the x-axis direction (first direction) to the recess 121A of the measuring instrument 12, the sensor 11 can be electrically connected to the measuring instrument 12 or electrically disconnected from the measuring instrument 12.

In the embodiment of the present invention, the sensor 11 is used to measure the cyclic voltammogram CVG of the analyte, is attachable to and detachable from a measuring instrument 12 that measures the cyclic voltammogram CVG, and is the sensor discarded each time a measurement of the cyclic voltammogram CVG is performed. In other words, the sensor 11 is a disposable sensor.

In this way, the sensor 11 is discarded each time a cyclic voltammogram measurement is performed, so there is no need to regenerate the electrodes (working electrode 112, counter electrode 113 and reference electrode 114) by physical polishing or the like, or to pretreat the sample (analyte).

Fig. 4 is a schematic diagram of the measuring instrument 12 shown in Fig. 2. Fig. 5 is a schematic diagram showing a timing chart of the potential supplied to the sensor 11 shown in Fig. 2.

Referring to Fig. 4, the measuring instrument 12 includes a supplying unit 121, a measuring unit 122, and a transmitting unit 123.

The supply unit 121 is electrically connected to the working electrode 112 by a wiring 115. The supply unit 121 accepts the potential scanning range and the potential scanning speed input by the user of the sensor device 1. The supply unit 121 supplies a potential within a potential scanning range to the working electrode 112 via the wiring 115 while changing the potential at a predetermined scanning speed.

Here, users of the sensor device 1 are, for example, staff at restaurants such as Japanese restaurants, Chinese restaurants, and Western restaurants, master brewers at sake breweries, and staff at liquor stores.

The measurement unit 122 is electrically connected to the working electrode 112, the counter electrode 113, and the reference electrode 114 by wiring 115, 116, and 117, respectively, and measures the potential V of the working electrode 112 based on the potential of the reference electrode 114 as a reference, and also measures the current value I from the counter electrode 113, and creates measurement data MRS including current-potential characteristics (I-V) that mutually correspond the measured potential V and current value I.

The potential scanning speed is, for example, 0.3 V/sec, 0.5 V/sec, or 0.6 V/sec, and the potential scanning range is, for example, -2.5 V to +2.5 V.

Referring to Fig. 5, in a period from time t1 to time t2, the supply unit 121 supplies a potential V in the range of 0 V to +2.5 V to the working electrode 112 while changing the potential V at a predetermined scanning speed.

Thereafter, during a period from time t2 to time t3, the supply unit 121 supplies a potential V in the range of +2.5 V to 0 V to the working electrode 112 while changing the potential V at a predetermined scanning speed.

Subsequently, in the period from time t3 to time t4, the supply unit 121 supplies a potential V in the range of 0 V to -2.5 V to the working electrode 112 while changing the potential V at a predetermined scanning speed.

Furthermore, in the period from time t4 to time t5, the supply unit 121 supplies a potential V in the range of -2.5 V to 0 V to the working electrode 112 while changing the potential V at a predetermined scanning speed.

In this manner, the supply unit 121 supplies the potential V with triangular wave to the working electrode 112.

In the embodiment of the present invention, the supply unit 121 supplies a potential V in a potential scanning range of -2.5 V to +2.5 V to the working electrode 112 while changing the potential V at a scan speed of 0.3 V/sec, and the measurement unit 122 measures the potential V of the working electrode 112 by using the potential of the reference electrode 114 as a criterion, measures the current value I from the counter electrode 113, and creates measurement data MRS_Low including a current-potential characteristic (I-V)_Low that the measured potential V and current value I are corresponded with each other.

In addition, the supply unit 121 supplies a potential V in a potential scanning range of -2.5 V to +2.5 V to the working electrode 112 while changing the potential V at a scan speed of 0.5 V/sec, and the measurement unit 122 measures the potential V of the working electrode 112 by using the potential of the reference electrode 114 as a criterion, measures the current value I from the counter electrode 113, and creates measurement data MRS_Middle including a current-potential characteristic (I-V)_Middle that the measured potential V and current value I are corresponded with each other.

Furthermore, the supply unit 121 supplies a potential V in a potential scanning range of -2.5 V to +2.5 V to the working electrode 112 while changing the potential V at a scan speed of 0.6 V/sec, and the measurement unit 122 measures the potential V of the working electrode 112 by using the potential of the reference electrode 114 as a criterion, measures the current value I from the counter electrode 113, and creates measurement data MRS_High including a current-potential characteristic (I-V)_High that the measured potential V and current value I are corresponded with each other.

Then, the measurement unit 122 creates measurement data MRS including the measurement data MRS _Low, the measurement data MRS_Middle, and the measurement data MRS_High, and outputs the created measurement data MRS to the transmitting unit 123.

The transmitting unit 123 receives the measurement data MRS from the measurement unit 122, and transmits the received measurement data MRS to the diagnostic device 2 by wireless communication or wired communication.

Fig. 6 is a schematic diagram of the measurement data MRS. Referring to Fig. 6, the measurement data MRS includes a name of the analyte, a type of the analyte, and the measurement data MRS _Low, MRS_Middle, and MRS_High.

The name of the analyte is composed of, for example, shochu or grapes. When the name of the analyte is shochu, the types of the analyte are composed of, for example, sweet potato shochu, barley shochu, and rice shochu etc. Furthermore, when the name of the analyte is "grape", the types of the analyte include, for example, green seedless, crimson seedless, and shine muscat etc.

The measurement data MRS_Low includes a potential scanning speed $V_{r_Low}$ and a current-potential characteristic ($I_{Low}$-$V_{Low}$). The current-potential characteristic ($I_{Low}$-$V_{Low}$) consists of a configuration in which the potential $V_{Low}$ corresponds to the current value $I_{Low}$.

The potential $V_{Low}$ consistes of $V_{1_Low}$ to $V_{d_Low}$, and the current value $I_{Low}$ consists of $I_{1_Low}$ to $I_{d_Low}$. The current values $I_{1_Low}$ to $I_{d_Low}$ are corresponded to the potentials $V_{1_Low}$ to $V_{d_Low}$, respectively.

The potentials $V_{1_Low}$ to $V_{d_Low}$ are the potentials of the working electrode 112 measured based on the potential of the reference electrode 114 when the potential scanning speed is "$V_{r_Low}$", and the current values $I_{1_Low}$ to $I_{d_Low}$ are the current values I from the counter electrode 113 in when the potential scanning speed is "$V_{r_Low}$".

The measurement data MRS_Middle includes a potential scanning speed $V_{r_Middle}$ and a current-potential characteristic ($I_{Middle}$-$V_{Middle}$). The current-potential characteristic ($I_{Middle}$-$V_{Middle}$) is configuration which a potential $V_{Middle}$ is corresponded to a current value $I_{Middle}$.

The potential $V_{Middle}$ is composed of $V_{1_Middle}$ to $V_{d_Middle}$, and the current value $I_{Middle}$ is composed of from $I_{1_Middle}$ to $I_{d_Middle}$. The current values $I_{1_Middle}$ to $I_{d_Middle}$ are corresponded to potentials $V_{1_Middle}$ to $V_{d_}$Middle, respectively.

The potentials $V_{1_Middle}$ to $V_{d_Middle}$ are the potentials of the working electrode 112 measured based on the potential of the reference electrode 114 when the potential scanning speed is "$V_{r_Middle}$", and the current values $I_{1_Middle}$ to $I_{d_Middle}$ are the current values I from the counter electrode 113 when the potential scanning speed is "$V_{r_Middle}$".

The measurement data MRS_High includes a potential scanning speed $V_{r_High}$ and a current-potential characteristic ($I_{High}$-$V_{High}$). The current-potential characteristic ($I_{High}$-$V_{High}$) consists of a correspondence between the potential $V_{High}$ and the current value $I_{High}$.

The potential $V_{High}$ ranges from $V_{1_High}$ to $V_{d_High}$, and the current value $I_{High}$ ranges from $I_{1_High}$ to Id$_{_High}$. The current values $I_{1_High}$ to $I_{d_High}$ correspond to the potentials $V_{1_High}$ to $V_{d_High}$, respectively.

The potentials $V_{1_High}$ to $V_{d_High}$ are the potentials of the working electrode 112 measured based on the potential of the reference electrode 114 when the potential scanning speed is "$V_{r_High}$", and the current values $I_{1_High}$ to $I_{d_High}$ are the current values I from the counter electrode 113 when the potential scanning speed is "$V_{r_High}$".

When the scanning range of the potential $V_{Low}$ is -2.5V to +2.5V, the potentials $V_{1_Low}$, $V_{2_Low}$, $V_{3_Low}$, $V_{4_Low}$, ...,

$V_{d-2_Low}$, $V_{d-1_Low}$, and $V_{d_Low}$ are respectively 0V, 1mV, 2mV, 3mV, ..., 2499mV, 2500mV, 2499mV, ..., 2mV, 1mV, 0mV, -1mV, -2mV, ..., -2499mV, - 2500mV, -2499mV, ..., -2mV, -1mV, and 0V. That is, the potentials $V_{1_Low}$, $V_{2_Low}$, $V_{3_Low}$, $V_{4_Low}$, ..., $V_{d-2_Low}$, $V_{d-1_Low}$, and $V_{d_Low}$ are made up of potentials that are changed by unit potential (=1 mV). As a result, d represents twice the total number of unit potentials in the scanning range of the potential V.

The same is applied to the potentials $V_{1_Middle}$ to $V_{d_Middle}$ and the potentials $V_{1_High}$ to $V_{d_High}$.

The measurement unit 122 receives the name of the analyte, the type of analyte, and the potential scanning speeds $V_{r_Low}$, $V_{r_Middle}$, and $V_{r_High}$ from the user of the sensor device 1, and measures the current-potential characteristics ($I_{Low}$-$V_{Low}$) at the potential scanning speed $V_{r_Low}$, measures the current-potential characteristics ($I_{Middle}$-$V_{Middle}$) at the potential scanning speed $V_{r_Middle}$, and measures the current-potential characteristics ($I_{High}$-$V_{High}$) at the potential scanning speed $V_{r_High}$. Then, the measurement unit 122 creates measurement data MRS including the name of the analyte, the type of analyte, the potential scanning speeds $V_{r_Low}$, $V_{r_Middle}$, $V_{r_High}$, and the current-potential characteristics ($I_{Low}$-$V_{Low}$), ($I_{Middle}$-$V_{Middle}$), and ($I_{High}$-$V_{High}$), and outputs the created measurement data MRS to the transmitting unit 123.

The transmitting unit 123 receives the measurement data MRS from the measurement unit 122, and transmits the received measurement data MRS to the diagnostic device 2 by wired communication or wireless communication.

When transmitting the measurement data MRS to the diagnostic device 2 by wireless communication, the transmitting unit 123 transmits the measurement data MRS to the diagnostic device 2 by, for example, Bluetooth (registered trademark).

Furthermore, when the transmitting unit 123 transmits the measurement data MRS to the diagnostic device 2 by wired communication, the transmitting unit 123 is connected to the diagnostic device 2 by a cable.

When the sensor device 1 measures P cyclic voltammograms of P (P is an integer greater than or equal to 2) analytes using the cyclic voltammetry method, the measurement unit 122 of the sensor device 1 creates each of the P pieces of measurement data MRS_1 to MRS_P using the method described above, and the transmitting unit 123 of the sensor device 1 transmits the P pieces of measurement data MRS_1 to MRS_P created by the measurement unit 122 to the diagnostic device 2 via wired or wireless communication. In this case, each of the P pieces of measurement data MRS_1 to MRS_P has the same configuration as the measurement data MRS shown in Fig. 6.

Fig. 7 is a schematic diagram of the diagnostic device 2 shown in Fig. 1. Referring to Fig. 7, the diagnostic device 2 includes an analysis/diagnostic unit 21 and a database 22.

The analysis/diagnostic unit 21 receives the measurement data MRS from the measuring instrument 12 (transmitting unit 123) of the sensor device 1 by wireless communication or wired communication.

Then, the analysis/diagnostic unit 21 diagnoses the taste of the analyte (shochu or grapes) based on the measurement data MRS using a method described below, stores the diagnosed taste diagnosis result in the database 22 in association with the analyte (shochu or grapes), and displays the taste diagnosis result of the analyte (shochu or grapes).

The database 22 stores the taste diagnosis results in association with the analyte (shochu or grapes).

Fig. 8 is a schematic diagram of the analysis/diagnostic unit 21 shown in Fig. 7. Referring to Fig. 8, the analysis/diagnostic unit 21 includes a receiving unit 211, a control unit 212, calculation units 213 and 216, a judgment unit 214, a creation unit 215, a taste diagnostic unit 217, a display unit 218, and a reception unit 219.

The receiving unit 211 receives the measurement data MRS from the measuring instrument 12 (transmitting unit 123) of the sensor device 1 by wireless communication or wired communication, and outputs the received measurement data MRS to the control unit 212.

Here, the measurement data MRS may be one piece of measurement data or may be a plurality of pieces of measurement data.

The control unit 212 includes a timer. When the control unit 212 receives one piece of measurement data MRS_uni from the receiving unit 211, the control unit 212 refers to the timer to detect the time $t_{uni}$ when the measurement data MRS_uni was received, and issues identification information $ID_{uni}$ for identifying the measurement data MRS_uni. The measurement data MRS_uni has the same structure as the measurement data MRS shown in Fig. 6.

Then, the control unit 212 detects the name $ALY_Na_{uni}$ of the analyte, the type $ALY_Kd_{uni}$ of the analyte, the potential scanning speed $V_{r_Low_uni}$, the current-potential characteristics $(I_{Low}\text{-}V_{Low})_{uni}$, the potential scanning speed $V_{r_Middle_uni}$, the current-potential characteristics $(I_{Middle}\text{-}V_{Middle})_{uni}$, the potential scanning speed $V_{r_High_uni}$, and the current-potential characteristics $(I_{High}\text{-}V_{High})_{uni}$ from the measurement data MRS_uni.

Thereafter, the control unit 212 generates measurement data MRS_Low_uni=$\{V_{rLow_uni}(I_{Low}\text{-}V_{Low})_{uni}\}$ in which the potential scanning speed $V_{r_Low_uni}$ and the current-potential characteristic $(I_{Low}\text{-}V_{Low})_{uni}$ are corresponded each other, measurement data MRS_Middle_uni=$\{V_{r_middle_uni}:(I_{middle}\text{-}V_{middle})_{uni}\}$ in which the potential scanning speed $V_{r_middle_uni}$ and the current-potential characteristic $(I_{middle}\text{-}V_{middle})_{uni}$ are corresponded each other, and measurement data MRS_High_uni=$\{V_{r_High_uni}(I_{High}\text{-}V_{High})_{uni}\}$ in which the potential scanning speed $V_{r_High_uni}$ and the current-potential characteristic $(I_{High}\text{-}V_{High})_{uni}$ are corresponded each other.

Then, the control unit creates analysis data $ALY_D_{uni}$= [$t_{uni}$/ $ID_{uni}$/ $ALY_Na_{uni}$/ $ALY_Kd_{uni}$/MRS_Low_uni/

MRS_Middle_uni/ MRS_High_uni] in which the time $t_{uni}$, the identification information $ID_{uni}$, the name $ALY_Na_{uni}$ of analyte, the kind $ALY_Kd_{uni}$ of the analyte, the measurement data MRS_Low_uni={$V_{r_Low_uni}$: $(I_{Low}-V_{Low})_{uni}$}, the measurement data MRS_Middle_uni={$V_{r_Middle_uni}$: $(I_{Middle}-V_{Middle})_{uni}$}, and the measurement data MRS_High_uni={$V_{r_High_uni}$: $(I_{High}-V_{High})_{uni}$} are associated with each other.

Then, the control unit 212 stores the analysis data $ALY_D_{uni}$ in the database 22 and outputs the analysis data $ALY_D_{uni}$ to the calculation unit 213.

In addition, when the control unit 212 have received P pieces of measurement data MRS_1 to MRS_P (i.e., multiple measurement data) from the receiving unit 211, the control unit 212 refers to the timer to detect the times $t_1$ to $t_P$ at when the P pieces of measurement data MRS_1 to MRS_P were received, respectively, and issues P pieces of identification information $ID_1$ to $ID_P$ for identifying the P pieces of measurement data MRS_1 to MRS_P, respectively. Here, each of the P pieces of measurement data MRS_1 to MRS_P has the same configuration as the measurement data MRS shown in Fig. 6.

Then, the control unit 212 executes to detecting the name $ALY_Na_p$ of the analyte, the kind $ALY_Kd_p$ of the analyte, the potential scanning speed $V_{r_Low_p}$, the current-potential characteristic $(I_{Low}-V_{Low})_p$, the potential scanning speed $V_{r_Middle_p}$, the current-potential characteristic $(I_{Middle}-V_{Middle})_p$, the potential scanning speed $V_{r_High_p}$, and the current-potential characteristic $(I_{High}-V_{High})_p$ from the measurement data MRS_P (p is any of 1 to P) for all of P pieces of measurement data MRS_1 to MRS_P (i.e., multiple measurement data).

Thereafter, the control unit 212 executes for all of p=1 to P generating measurement data MRS_Low_p={$V_{r_Low_p}$: $(I_{Low}-V_{Low})_p$} in which the potential scanning speed $V_{r_Low_p}$ and the current-potential characteristic $(I_{Low}-V_{Low})_p$ are corresponded to each other, measurement data MRS_Middle_p={$V_{r_Middle_p}$: $(I_{Middle}-V_{Middle})_p$} in which the potential scanning speed $V_{r_Middle_p}$ and the current-potential characteristic $(I_{Middle}-V_{Middle})_p$ are corresponded to each other, and measurement data MRS_High_p={$V_{r_High_p}$: $(I_{High} - V_{High})_p$} in which the potential scanning speed $V_{r_High_p}$ and the current-potential characteristic $(I_{High}-V_{High})_p$ are corresponded to each other, and the control unit 212 creates P pieces of measurement data MRS_Low_1={$V_{r_Low_1}$: $(I_{Low}-V_{Low})_1$} to MRS_Low_P={$V_{r_Low_P}$: $(I_{Low}-V_{Low})_P$}, P pieces of measurement data MRS_Middle_1={$V_{r_Middle_1}$: $(I_{Middle}-V_{Middle})_1$} to MRS_Middle_P={$V_{r_Middle_P}$: $(I_{Middle}-V_{Middle})_P$}, and P pieces of measurement data MRS_High_1={$V_{r_High_1}$: $(I_{High}-V_{High})_1$}-MRS_High_P={$V_{r_High_P}$: $(I_{High}-V_{High})_P$}.

Then, the control unit 212 executes for all of p=1 to P creating analysis data ALY _$D_p$= [$t_p$/$ID_p$/$ALY_Na_p$/$ALY_Kd_p$/measurement data MRS_Low_p/measurement data MRS _Middle_p/measurement data MRS_High_p] in which the time $t_p$, the identification information $ID_p$, the name $ALY_Na_p$ of the analyte, the type $ALY_Kd_p$ of the analyte, the measurement data MRS_Low_p={$V_{r_Low_p}$: $(I_{Low}-V_{Low})_p$}, the measurement data MRS_Middle_p={$V_{r_Middle_p}$: $(I_{Middle}-V_{Middle})_p$}. And the control unit 212 creates P pieces of analysis data $ALY_D_1$=[$t_1$/$ID_1$/$ALY_Na_1$/$ALY_Kd_1$/ measurement data MRS _Low_1/measurement data MRS _Middle_1/measurement data MRS_High_1] to $ALY_D_P$=[$t_P$/$ID_P$/$ALY_Na_P$/$ALY_Kd_P$/measurement data MRS _Low _P/ measurement data MRS_Middle_P/measurement data MRS_High_P].

Then, the control unit 212 stores the P pieces of analysis dataALY_$D_1$ to $ALY_D_P$ in the database 22, and outputs the P pieces of analysis data $ALY_D_1$ to $ALY_D_P$ to the calculation unit 213.

Furthermore, after outputting the analysis data $ALY_D_{uni}$ to the calculation unit 213, the control unit 212 receives from the creation unit 215 an analysis result $ALY_RLS_{uni}$=[$ID_{uni}$/$CAL_{uni}$/$CUR_{uni}$] in which the identification information $ID_{uni}$, the calculation data $CAL_{uni}$, and the curve $CUR_{uni}$ are corresponded to each other, and then detects the identification information $ID_{uni}$, the calculation data $CAL_{uni}$, and the curve $CUR_{uni}$ from the analysis result $ALY_RLS_{uni}$, and reads out from the database 22 the analysis data $ALY_D_{uni}$ having the same identification information as the detected identification information $ID_{uni}$.

Then, the control unit 212 stores the calculation data $CAL_{uni}$ and the curve $CUR_{uni}$ in the analysis data $ALY_D_{uni}$ read out from the database 22 to update the analysis data $ALY_D_{uni}$ to the index data $IDX_{uni}$, and outputs the updated index data $IDX_{uni}$ to the calculation unit 216.

Thereafter, the control unit 212 stores the index data $IDX_{uni}$ in the database 22 in place of the analysis data $ALY_D_{uni}$.

Furthermore, the control unit 212 receives from the create unit 215 the P analysis results $ALY_RLS_1$=[$ID_1$/$CAL_1$/$CUR_1$] to $ALY_RLS_P$=[$ID_P$/$CAL_P$/$CUR_P$] in which the P pieces of identification information $ID_1$ to $ID_p$, the P pieces of calculation data $CAL_1$ to $CAL_P$, and the P pieces of curves $CUR_1$ to $CUR_1$ are associated with each other and a judgment result JDGR indicating whether the P curves $CUR_1$ to $CUR_1$ are or not different from each other after the control unit 212 outputs the P pieces of analysis data $ALY_D_1$ to $ALY_D_P$ to the calculation unit 213. After that, the control unit 212 detects the identification information $ID_p$, calculation data $CAL_p$, and curve $CUR_p$ from the analysis result $ALY_RLS_p$ = [$ID_p$/$CAL_p$/$CUR_p$] (p is any of 1 to P) and executes for all of p=1 to P reading out the analysis data $ALY_D_p$ having the same identification information as the detected identification information $ID_p$ from the database 22.

Then, the control unit 212 stores the calculation data $CAL_p$ and the curve $CUR_p$ in the analysis data $ALY_D_p$ read out from the database 22 and executes for all of p=1 to P updating the analysis data $ALY_D_p$ to the index data $IDX_p$.

Thereafter, the control unit 212 outputs the P index data $IDX_1$ to $IDX_P$ to the calculation unit 216.

Then, the control unit 212 stores the P index data $IDX_1$ to $IDX_P$ in the database 22 in place of the P analysis data $ALY_D_1$ to $ALY_D_P$, respectively.

Then, the control unit 212 stores the judgment result JDGR in the database 22 in association with the P index data $IDX_1$ to $IDX_P$.

Furthermore, after outputting the index data $IDX_{uni}$ to the calculation unit 216, the control unit 212 receives a diagnosis result $JDR_{uni}$ which is the result of diagnosing the taste (astringency, aftertaste, sweetness, aroma and bitterness) of the analyte (shochu or grapes) from the taste diagnostic unit 217, and stores the received diagnosis result $JDR_{uni}$ in the database 22 in correspondence with the index data $IDX_{uni}$.

Furthermore, the control unit 212 receives from the taste diagnostic unit 217 P diagnosis results $JDR_1$ to $JDR_1$ which are the results of diagnosing the taste (astringency, aftertaste, sweetness, aroma and bitterness) of the analyte (shochu or grapes) after outputting the P pieces of index data $IDX_1$ to $IDX_P$ to the calculation unit 216. Then, the control unit 212 stores the received P diagnosis results $JDR_1$ to $JDR_1$ in the database 22 in association with P index data $IDX_1$ to $IDX_P$, respectively.

Furthermore, when the control unit 212 have received a request $RQT_{uni}$ that displays the name $ALY_Na_{uni}$ of the analyte and the diagnosis result $JDR_{uni}$ associated with the name $ALY_Na_{uni}$ from the reception unit 219, the control unit 212 detects the diagnosis result $JDR_{uni}$ associated with the name $ALY_Na_{uni}$ of the analyte from the database 22 based on the name $ALY_Na_{uni}$ of the analyte, and outputs the name $ALY_Na_{uni}$ of the analyte and the diagnosis result $JDR_{uni}$ to the display unit 218.

Furthermore, the control unit 212 receives from the reception unit 219 a request $RQT_q$ to display q (where q is an integer satisfying $1 \leqq q \leqq P$) names $ALY_Na_1$ to $ALY_Na_q$ in the P names $ALY_Na_1$ to $ALY_Na_P$ of the P analytes and q diagnosis results $JDR_1$ to $JDR_q$ associated with the q names $ALY_Na_1$ to $ALY_Na_q$, respectively. Then, the control unit 212 detects q diagnosis results $JDR_1$ to $JDR_q$ associated with q names $ALY_Na_1$ to $ALY_Na_q$ of q analytes from database22 based on the q names $ALY_Na_1$ to $ALY_Na_q$, and outputs the q names $ALY_Na_1$ to $ALY_Na_q$ of q analytes and q diagnosis results $JDR_1$ to $JDR_q$ to the display unit 218.

In this case, in the request RQTq, q types $ALY_Kd_1$ to $ALY_Kd_q$ of q analytes may be used instead of q names $ALY_Na_1$ to $ALY_Na_q$ of q analytes.

The calculation unit 213 receives the analysis data $ALY_D_{uni}$= [$t_{uni}$/$ID_{uni}$/ $ALY_Na_{uni}$/$ALY_Kd_{uni}$/MRS_Low_uni= {Low_uni:$(I_{LOW}$-$V_{Low})_{uni}$i} /MRS_Middle_uni= {Middle_ uni:$(I_{Middle}$-$V_{Middle})_{uni}$}/MRS_High_uni={High_uni:$(I_{High}$-$V_{High})_{uni}$}] from the control unit 212.

Then, the calculation unit 213 executes, for all of predetermined potential section V_ITV, calculating the integral value ITG of the cyclic voltammogram CVG for each predetermined potential section V_ITV based on the current-potential characteristics $(I_{Low}$-$V_{Low})_{uni}$, $(I_{Middle}$-$V_{Middle})_{uni}$, $(I_{High}$-$V_{High})$ uni using a method described below based on the current-potential characteristics $(I_{Low}$-$V_{Low})$, $(I_{Middle}$-$V_{Middle})$, $(I_{High}$-$V_{High})$ in the analysis data $ALY_D_{uni}$=[$t_{uni}$/$ID_{uni}$/$ALY_Na_{uni}$/$ALY_Kd_{uni}$/MRS_Low_uni= {Low_uni: $(IL_{ow}$- $V_{Low})_{uni}$}/MRS_Middle_uni={Middle_uni: $(I_{Middle}$-$V_{Middle})_{uni}$}/MRS_High_uni ={High_uni:$(I_{High}$-$V_{High})_{uni}$}].

Then, when the number of predetermined potential sections V_ITV is n, the calculation unit 213 sets the first predetermined potential section V_ITV to "class $Cls_1$", sets the second predetermined potential section V_ITV to "class $Cls_2$", ..., and similarly sets the (n-1)th predetermined potential section V_ITV to "class $Cls_{n-1}$" and sets the nth predetermined potential section V_ITV to "class $Cls_n$".

Then, based on the current-potential characteristic $(I_{Low}$-$V_{Low})_{uni}$ of the measurement data MRS_Low_uni= {Low_uni:$(I_{Low}$-$V_{Low})_{uni}$}, the calculation unit 213 sets the integral value in the first predetermined potential section V_ITV to $ITG_{1_Low}$, sets the integral value in the second predetermined potential section V_ITV to $ITG_{2_Low}$, ..., similarly sets the integral value in the (n-1)th predetermined potential section V_ITV to $ITG_{(n-1)_Low}$ and sets the integral value in the nth predetermined potential section V_ITV to $ITG_{n_Low}$.

In addition, based on the current-potential characteristic $(I_{Middle}$-$V_{Middle})_{uni}$ of the measurement data MRS_Middle_uni={Middle_uni:$(I_{Middle}$-$V_{Middle})_{uni}$}, the calculation unit 213 sets the integral value in the first predetermined potential section V_ITV to $ITG_{1_Middle}$, sets the integral value in the second predetermined potential section V_ITV to $ITG_{2_Middle}$, ..., similarly sets the integral value in the (n-1)th predetermined potential section V_ITV to $ITG_{(n-1)_Middle}$, and sets the integral value in the nth predetermined potential section V_ITV to $ITG_{n_Middle}$.

Furthermore, based on the current-potential characteristic $(I_{High}$-$V_{High})_{uni}$ of the measurement data MRS_High_uni={High_uni:$(I_{High}$-$V_{High})_{uni}$}, the calculation unit 213 sets the integral value in the first predetermined potential section V_ITV to $ITG_{1_High}$, sets the integral value in the second predetermined potential section V_ITV to $ITG_{2_High}$, ..., similarly sets the integral value in the (n-1)th predetermined potential section V_ITV to $ITG_{(n-1)_High}$ and sets the integral value in the nth predetermined potential section V_ITV to $ITG_n$_High.

Then, the calculation unit 213 adds the integral values $ITG_{1_Low}$, $ITG_{1_Middle}$, and $ITG_{1_High}$ in class $Cls_1$ to calculate the sum $ITG_{1_Low}$+$ITG_{1_Middle}$+$ITG_{1_High}$ of the integral values, adds the integral values $ITG_{2_Low}$, $ITG_{2_Middle}$, and $ITG_{2_High}$ in class $Cls_2$ to calculate the sum $ITG_{2_Low}$+$ITG_{2_Middle}$+$ITG_{2_High}$ of the integral values, similarly, adds the integral values $ITG_{n_Low}$, $ITG_{n_Middle}$, and $ITG_{n_High}$ in class $Cls_n$ to calculate the sum $ITG_{n_Low}$+$ITG_{n_Middle}$+$ITG_{n_High}$of the integral values.

Then, the calculation unit 213 creates calculation data $CAL_{uni}$ which includes n classes $Cls_1$ to $Cls_n$, n integral values $ITG_{1_Low}$ to $ITG_{n_Low}$, $ITG_{1_Middle}$ to $ITG_{n_Middle}$, and $ITG_{1_High}$ to $ITG_{n_High}$ which are respectively associated with the n classes $Cls_1$ to $Cls_n$, and the sum ($ITG_{1_Low}$+$ITG_{1_Middle}$+$ITG_{1_High}$) to ($ITG_{n_Low}$+$ITG_{n_Middle}$+$ITG_{n_High}$) of the n integral values which are respectively associated with the n classes $Cls_1$ to $Cls_n$.

Thereafter, the calculation unit 213 generates a calculation result $CAL_RLS_{uni}$=[$ID_{uni}$/$CAL_{uni}$] corresponding the calculation data $CAL_{uni}$ to the identification information $ID_{uni}$. Then, the calculation unit 213 outputs the calculation result $CAL_RLS_{uni}$=[$ID_{uni}$/$CAL_{uni}$] and a signal S_u indicating that the calculation data is one to the creation unit 215.

The calculation unit 213 also receives the P pieces of analysis data $ALY_D_1$=[$t_1$/$ID_1$/$ALY_Na_1$/$ALY_Kd_1$/MRS_Low_1={Low_1:$(I_{Low}\text{-}V_{Low})_1$}/MRS_Middle_1={Middle_1: $(I_{Middle}\text{-}V_{Middle})_1$}/MRS_High_1={High_1:$(I_{High}\text{-}V_{High})_1$}] to $ALY_D_P$=[$t_P$/$ID_P$/$ALY_Na_P$/$ALYKd_P$/MRS_Low_P={Low_P:$(I_{Low}\text{-}VL_{ow})_P$}/MRS_Middle_P={Middle_P: $(_{IMiddle}\text{-}V_{Middle})_P$}/{MRS_High_P=High_P:$(I_{High}\text{-}V_{High})_P$}] from control unit 212.

Then, the calculation unit 213 performs the following processing in the same manner as when the analysis data $ALY_D_{uni}$= [$t_{uni}$/$ID_{uni}$ /$ALY_Na_{uni}$ /$ALY_Kd_{uni}$/ MRS_Low_uni ={Low_uni: $(I_{Low}\text{-}V_{Low})_{uni}$} /MRS_Middle_uni= {Middle_uni: $(I_{Middle}\text{-}V_{Middle})_{uni}$} /MRS_High_uni={High_uni: $(I_{High}\text{-}V_{High})_{uni}$}] is received from the control unit 212. Based on the current-potential characteristics {$(I_{Low}\text{-}V_{Low})_p$, $(I_{Middle}\text{-}V_{Middle})_p$, $(I_{High}\text{-}V_{High})_p$} (p is any one of 1 to P) of the analysis data ALY $_D_p$= [$t_p$/$ID_p$/$ALY_Na_p$ /$ALY_Kd_p$/MRS_Low_p={Low_p: $(I_{Low}\text{-}V_{Low})_p$}/MRS_Middle_p= {Middle_p: $(I_{Middle}\text{-}V_{Middle})_p$}/{MRS_High_p=High_p: $(I_{High}\text{-}V_{High})_p$}], the calculation unit 213 executes for all of P pieces of analysis data $ALY_D_1$ to $ALY_D_1$ creating the calculation data $CAL_p$ that associates n classes $Cls_1$ to $Cls_n$, the n integral values $ITG_{1_Low}$ to $ITG_{n_Low}$, $ITG_{1_Middle}$ to $ITG_{n_Middle}$, and $ITG_{1_High}$ to $ITG_{n_High}$, and the sum of the n integral values ($ITG_{1_Low}$+$ITG_{1_Middle}$ +$ITG_{1_High}$) to ($ITG_{n_Low}$+$ITG_{n_Middle}$+$ITG_{n_High}$) to create P calculation data $CAL_1$ to $CAL_P$.

Then, the calculation unit 213 outputs P calculation results $CAL_RLS_1$=[$ID_1$/$CAL_1$] to $CAL_RLS_P$=[$ID_P$/$CAL_P$] in which the P pieces of identification information $ID_1$ to $ID_P$ correspond to the P pieces of calculation data $CAL_1$ to $CAL_P$, respectively, to the judgment unit 214 and the creation unit 215.

In this way, when the calculation unit 213 has created one calculation data $CAL_{uni}$, the calculation unit 213 outputs the calculation result $CAL_RLS_{uni}$ = [$ID_{uni}$/$CAL_{uni}$] to only the creation unit 215, and when the calculation unit 213 has created P calculation results $CAL_RLS_1$ = [$ID_1$/$CAL_1$] to CAL $RLS_P$ = [$ID_P$/$CAL_P$] (i.e., multiple calculation results), the calculation unit 213 outputs P calculation results $CAL_RLS_1$ to $CAL_RLS_P$ (i.e., multiple calculation results) to the judgment unit 214 and the creation unit 215.

As described above, the calculation data $CAL_{uni}$ is configured by associating n classes $Cls_1$ to $Cls_n$ with n integral values $ITG_{1_Low}$ to $ITG_{n_Low}$, $ITG_{1_Middle}$ to $ITG_{n_Middle}$, and $ITG_{1_High}$ to $ITG_{n_High}$, and the sum of the n integral values ($ITG_{1_Low}$+$ITG_{1_Middle}$+$ITG_{1_High}$) to ($ITG_{n_Low}$+$ITG_{n__Middle}$+$ITG_{nHigh}$), but since each of the n classes $Cls_1$ to $Cls_n$ is made up of a predetermined potential section V_ITV, the calculation data $CAL_{uni}$ is configured by associating n predetermined potential sections $V_ITV_1$ to $V_ITV_n$ with n integral values $ITG_{1_Low}$ to $ITG_{n_Low}$, $ITG_{1_Middle}$ to $ITG_{n_Middle}$, and $ITG_{1_High}$ to $ITG_{n_High}$, and the sum of the n integral values ($ITG_{1_Low}$+$ITG_{1_Middle}$+$ITG_{1_High}$) to ($ITG_{n_Low}$+$ITG_{n_Middle}$+$ITG_{n_High}$). The same is true for each of the P pieces of calculation data $CAL_1$ to $CAL_P$.

The judgment unit 214 receives P calculation results $CAL_RLS_1$ to $CAL_RLS_P$ (that is, a plurality of calculation results) from the calculation unit 213. Then, the judgment unit 214 detects the P pieces of calculation data $CAL_1$ to $CAL_1$ contained in the P pieces of calculation results $CAL_RLS_1$ to $CAL_RLS_P$.

Then, the judgment unit 214 detects two calculation data $CAL_i$, $CAL_j$ (i≠j) of ${}_PC_2$ sets based on the P pieces of calculation data $CAL_1$ to $CAL_P$. Here, ${}_PC_2$ is the number of combinations of two different pieces of calculation data $CAL_i$, $CAL_j$ (i≠j) when two different pieces of calculation data $CAL_i$, $CAL_j$ (i≠j) are extracted from the P pieces of calculation data $CAL_1$ to $CAL_P$.

Thereafter, the judgment unit 214 calculates differences between the multiple integral values included in the calculation data $CAL_i$ and the multiple integral values included in the calculation data $CAL_j$ for each class Cls based on two calculation data $CAL_i$ and $CAL_j$ (i≠j), and calculates the standard deviation of the calculated differences for all of the two pieces of calculation data $CAL_i$, $CAL_j$ (i≠j) of ${}_PC_2$ sets.

The judgment unit 214 calculates the differences in each class Cls between the multiple integral values included in the calculation data $CAL_i$ and the multiple integral values included in the calculation data $CAL_j$ by the following method.

The judgment unit 214 calculates a difference $DF_k$ between the integral value $ITG_{k_i}$ and the integral value $ITG_{k_j}$ in one class $Cls_k$ using the following equation, based on the multiple integral values $ITG_{1_i}$ to $ITG_{n_i}$ included in the calculation data $CAL_i$ and the multiple integral values $ITG_{1_j}$ to $ITG_{n_j}$ included in the calculation data $CAL_j$.

[Equation 1]

$$DF_k = \left[\frac{\left(|ITG_{k_i}| - |ITG_{k_j}|\right)}{\left(ITG_{k_i} + ITG_{k_j}\right)/2}\right] \times 100 \cdots (1)$$

The unit of the difference $DF_k$ calculated by the equation (1) is "%".

Then, the judgment unit 214 executes the calculation of the difference $DF_k$ of the integral value in one class $Cls_k$ using the equation (1) for all of the n classes $Cls_1$ to $Cls_n$ to calculate n differences $DF_1$ to $DF_n$.

Then, the judgment unit 214 calculates the standard deviation $\sigma_{DF}$ of the n differences $DF_1$ to $DF_n$.

Then, the judgment unit 214 judges that the two pieces of calculation data $CAL_i$ and $CAL_j$ (i≠j) are different when the standard deviation $\sigma_{DF}$ of the differences is greater than a threshold $\sigma_{th}$ (=6%), and the judgment unit 214 judges that the two pieces of calculation data $CAL_i$ and $CAL_j$ (i≠j) are not different when the standard deviation $\sigma_{DF}$ of the differences is equal or smaller than the threshold $\sigma_{th}$ (=6%). The judgment unit 214 holds the threshold value $\sigma_{th}$ (=6%) in advance.

The judgment unit 214 executes the above-mentioned method to judge whether or not the two pieces of calculation data $CAL_i$, $CAL_j$ (i≠j) differ for all of the two pieces of calculation data $CAL_i$, $CAL_j$ (i≠j) in the ${}_PC_2$ set, and judges whether or not the two pieces of calculation data $CAL_i$, $CAL_j$ (i≠j) in each set of the two pieces of calculation data $CAL_i$, $CAL_j$ (i≠j) in the ${}_PC_2$ set differ.

In addition, judging that the two pieces of calculation data $CAL_i$, $CAL_j$ (i ≠ j) are not different corresponds to judging that the two pieces of calculation data $CAL_i$, $CAL_j$ (i ≠ j) cannot be distinguished from each other, and judging that two pieces of calculation data $CAL_i$, $CAL_j$ (i≠j) are different corresponds to judging that the two pieces of calculation data $CAL_i$, $CAL_j$ (i≠j) can be distinguished from each other.

The judgment unit 214 judges whether or not the two pieces of calculation data $CAL_i$, $CAL_j$ (i≠j) of each of the two sets of ${}_PC_2$ are different using the method described above, and creates the judgment results shown in Table 1. Then, the judgment unit 214 outputs the judgment result shown in Table 1 to the creation unit 215.

[Table 1]

| | $CAL_1$ | $CAL_2$ | $CAL_3$ | ... | $CAL_P$ |
|---|---|---|---|---|---|
| $CAL_1$ | | ○ or × | ○ or × | ... | ○ or × |
| $CAL_2$ | ○ or × | | ○ or × | ... | ○ or × |
| $CAL_3$ | ○ or × | ○ or × | | ... | ○ or × |
| ⋮ | ⋮ | ⋮ | | | ○ or × |
| $CAL_P$ | ○ or × | ○ or × | ○ or × | ○ or × | |
| ○: indicates that the two calculation data $CAL_i$ and $CAL_i$ are different.<br>× : indicates that the two calculation data $CAL_i$ and $CAL_i$ are not different. | | | | | |

Judging that the P pieces of calculation data $CAL_1$ to $CAL_P$ are not different from one another corresponds to judging that the P pieces of calculation data $CAL_1$ to $CAL_P$ cannot be distinguished from one another, and judging that the P pieces of calculation data $CAL_1$ to $CAL_1$ are different from one another corresponds to judging that the P pieces of calculation data $CAL_1$ to $CAL_1$ can be distinguished from one another.

Upon receiving one calculation result $CAL_RLS_{uni}$ and a signal S_u indicating that there is one calculation data from the calculation unit 213, the creation unit 215 creates a curve $CUR_{uni}$ based on the calculation data $CAL_{uni}$ by a method described later.

In addition, when the creation unit 215 receives P calculation results $CAL_RLS_1$ to $CAL_RLS_P$ (i.e., multiple calculation results) from the calculation unit 213 and a judgment result JDGR (judgment result shown in Table 1) indicating whether the P calculation data $CAL_1$ to $CAL_1$ are different or not from the judgment unit 214, the creation unit 215 creates P curves $CUR_1$ to $CUR_1$ based on the P calculation data $CAL_1$ to $CAL_P$ using a method described below.

When the creation unit 215 created one curve $CUR_{uni}$, the creation unit 215 adds the curve $CUR_{uni}$ to the calculation result $CAL_RLS_{uni}$ to create an analysis result $ALY_RLS_{uni}$=[$ID_{uni}$/$CAL_{uni}$/$CUR_{uni}$] and outputs the created analysis result $ALY_RLS_{uni}$ =[$ID_{uni}$/$CAL_{uni}$/$CUR_{uni}$] to the control unit 212.

On the other hand, when the creation unit 215 created P curves $CUR_1$ to $CUR_P$, the creation unit 215 adds the P curves $CUR_1$ to $CUR_1$ to the P calculation results $CAL_RLS_1$ to $CAL_RLS_P$, respectively, to create P analysis results $ALY_RLS_1$ = [$ID_1$/$CAL_1$/$CUR_1$] to $ALY_RLS_P$ = [$ID_1$/$CAL_1$/$CUR_1$], and outputs the P analysis results $ALY_RLS_1$ to $ALY_RLS_P$ and the judgment result JDGR (the judgment result shown in Table 1) to the control unit 212.

The calculation unit 216 receives the index data $IDX_{uni}$ from the control unit 212. Then, the calculation unit 216 detects the calculation data $CAL_{uni}$ from the index data $IDX_{uni}$, and calculates the Body index $(+)_{uni}$, Body index $(all)_{uni}$, Body index (-)_$th_{uni}$, the sum of the integral values H(+)_$sum_{uni}$, and the sum of the integral values H(-)_$sum_{uni}$ based on the detected calculation data $CAL_{uni}$ using a method described below.

Then, the calculation unit 216 creates index data $INDX_D_{uni}$=[$ALY_Na_{uni}$/Body index $(+)_{uni}$/Body index $(all)_{uni}$/Body index (-)_$th_{uni}$/H(+)_$sum_{uni}$/H(-)_$sum_{uni}$] in which the name $ALY_Na_{uni}$ of the analyte, Body index $(+)_{uni}$, Body index

(all)$_{uni}$, Body index (-)_th$_{uni}$, the sum H(+)_sum$_{uni}$ of the integral values, and the sum H(-)_sum$_{uni}$ of the integral values are corresponded to each other, and the calculation unit 216 outputs the created index data INDX_D$_{uni}$=[ALY_Na$_{uni}$/Body index (+)$_{uni}$/Body index (all)$_{uni}$/Body index (-)_th$_{uni}$/H(+)_sum$_{uni}$/H(-)_sum$_{uni}$] to the taste diagnostic unit 217.

The calculation unit 216 also receives P index data IDX$_1$ to IDX$_P$ from the control unit 212. Then, the calculation unit 216 detects the P pieces of calculation data CAL$_1$ to CAL$_1$ from the P pieces of index data IDX$_1$ to IDX$_P$, respectively.

Then, the calculation unit 216 calculates Body index (+)$_p$ Body index (all)$_p$, Body index (-)_th$_p$, the sum H(+)_sum$_p$ of the integral values and the sum H(-)_sum$_p$ (p = 1 to P) of the integral values based on the calculation data CAL$_P$ using a method described below.

The calculation unit 216 then executes, for all of p=1 to P, creating index data INX_D$_p$=[ALY_Na$_p$/Body index (+)$_p$/Body index (all)$_p$/Body index (-)_th$_p$/H(+)_sum$_p$/H(-)_sum$_p$] in which the name ALY_Na$_p$ of the analyte, Body index (+)$_p$, Body index (all)$_p$, Body index (-)_th$_p$, the sum H(+)_sum$_p$ of the integral values, and the sum H(-)_sum$_p$ of the integral values are corresponded to each other and the calculation unit 216 creates P pieces of index data INDX_D$_1$= [ALY_Na$_1$/Body index (+)$_1$/Body index (all)$_1$/Body index (-)_thi/H(+)_sum$_1$/H(-)_sum$_1$] to INDX_D$_P$= [ALY_Na$_P$/Body index (+)$_P$/Body index (all)$_P$/Body index (-)_th$_P$/H(+)_sum$_P$/H(-)_sum$_P$].

Then, the calculation unit 216 outputs P pieces of index data INDX_D$_1$=[ALY_Na$_1$/Body index (+)$_1$/Body index (all)$_1$/Body index (-)_th$_1$/H(+)_sum$_1$/H(-)_sum$_1$] to INDX_D$_P$= [ALY_Nap/Body index (+)$_P$/ Body index (all)p/ Body index (-)_th$_P$/H(+)_sum$_P$ /H(-)_sum$_{P]}$ to the taste diagnostic unit 217.

The taste diagnostic unit 217 receives index data INDX_D$_{uni}$=[ALY_Na$_{uni}$/Body index (+)$_{uni}$/Body index (all)$_{uni}$/- Body index (-)_th$_{uni}$/H(+)sum$_{uni}$/H(-)sum$_{uni}$] from the calculation unit 216.

The taste diagnostic unit 217 detects name ALY_Na$_{uni}$ of the analyte, Body index (+)$_{uni}$, Body index (all)$_{uni}$, Body index (-)_th$_{uni}$, sum H(+)_sum$_{uni}$ of integral value, and sum H(-)_sum$_{uni}$ of integral value from the index data INDX_D$_{uni}$.

Then, the taste diagnostic unit 217 diagnoses that the "astringency" of the analyte having the name ALY_Na$_{uni}$ is the index value ASTG$_{uni}$ by a method described later based on the Body index (+)$_{uni}$.

Furthermore, the taste diagnostic unit 217 diagnoses that the "aftertaste" of the analyte having the name ALY_Na$_{uni}$ is an index value LNGS$_{uni}$ by a method described later based on the Body index (all)$_{uni}$.

Furthermore, the taste diagnostic unit 217 diagnoses that the "sweetness" of the analyte having the name ALY_Na$_{uni}$ is the index value SWT$_{uni}$ by a method described later based on the sum H(+)_sum$_{uni}$ of the integral values.

Furthermore, the taste diagnostic unit 217 diagnoses that the "aroma" of the analyte having the name ALY_N$_{auni}$ is the index value SCT$_{uni}$ by a method described later based on the sum H(-)_sum$_{uni}$ of the integral values.

Furthermore, the taste diagnostic unit 217 diagnoses that the "bitterness" of the analyte having the name ALY_Na$_{uni}$ is the index value BIT$_{uni}$ by a method described later based on the index value ASTG$_{uni}$ for "astringency" and the index value SWT$_{uni}$ for "sweetness".

Then, the taste diagnostic unit 217 creates a taste diagnosis result JDR$_{uni}$=[ALY_Na$_{uni}$/ASTG$_{uni}$/LNGS$_{uni}$/SWT$_{uni}$/SCT$_{uni}$/BIT$_{uni}$] in which the name ALY_Na$_{uni}$ of the analyte, the index value ASTG$_{uni}$ for "astringency", the index value LNGS$_{uni}$ for "aftertaste", the index value SWT$_{uni}$ for "sweetness", the index value SCT$_{uni}$ for "aroma", and the index value BIT$_{uni}$ for "bitterness" are associated with each other and outputs the created taste diagnosis result JDR$_{uni}$= [ALY_Na$_{uni}$/ ASTG$_{uni}$/ LNGS$_{uni}$/ SWT$_{uni}$/SCT$_{uni}$/BIT$_{uni}$] to the control unit 212 and the display unit 218.

In addition, when the name ALY_Na$_{uni}$ of the analyte is "grapes", the taste diagnostic unit 217 diagnoses that the "astringency" of the analyte having the name ALY_Na$_{uni}$ (=grapes) is the index value ASTG$_{th_uni}$ by a method described below based on the Body index (-)_th$_{uni}$.

Then, the taste diagnostic unit 217 creates a diagnosis result JDR$_{th_uni}$=[ALY_Na$_{uni}$ (=grapes)/ASTG$_{th_uni}$] in which the name ALY_Na$_{uni}$ (=grapes) of the analyte is associated with the index value ASTG$_{th_uni}$ of "astringency", and outputs the created diagnosis result JDR$_{th_uni}$=[ALY_Na$_{uni}$ (=grape)/ASTG$_{th_uni}$] to the control unit 212 and the display unit 218.

On the other hand, when the taste diagnostic unit 217 receives P pieces of index data INDX_D$_1$=[ALY_Na$_1$/Body index (+)$_1$/Body index (all)$_1$/Body index (-)_th$_1$/H(+)_sum$_1$/H(-)_sum$_1$] to INDX_DP=[ALY_Nap/Body index (+)p/Body index (all)P/Body index (-)_thp/H(+)_sum$_P$/H(-)_sum$_P$] from the calculation unit 216, the taste diagnostic unit 217 detects the name ALY_Na$_p$ of the analyte, Body index (+)$_p$, Body index (all)$_p$, Body index (-)_th$_p$, the sum H(+)_sum$_p$ of the integral values and the sum H(-)_sum$_p$ of the integral values from the index data INDX_D$_p$=[ALY_Na$_p$/Body index (+)$_p$/Body index (all)$_p$/Body index (-)_th$_p$/H(+)_sum$_p$/H(-)_sum$_p$].

Then, the taste diagnostic unit 217 diagnoses that the "astringency" of the analyte having the name ALY_Na$_p$ of the analyte is the index value ASTG$_p$ by a method described below based on the Body index (+)$_p$.

Furthermore, the taste diagnostic unit 217 diagnoses that the "aftertaste" of the analyte having the name ALY _Na$_p$ of the analyte is the index value LNGS$_p$ by a method described below based on the Body index (all)$_p$.

Furthermore, the taste diagnostic unit 217 diagnoses that the "sweetness" of the analyte having the name ALY _Na$_p$ of the analyte is the index value SWT$_p$ by a method described below based on the sum H(+)_sum$_p$ of the integral values.

Furthermore, the taste diagnostic unit 217 diagnoses that the "aroma" of the analyte having the name ALY _Na$_p$

of the analyte is the index value $SCT_p$ by a method described below based on the sum $H(-)_sum_p$ of the integral values.

Furthermore, the taste diagnostic unit 217 diagnoses the "bitterness" of the analyte having the name $ALY_Na_p$ of the analyte be an index value $BIT_p$ by a method described below based on the index value $ASTR_p$ of "astringency" and the index value $SWT_p$ of "sweetness".

Then, the taste diagnostic unit 217 creates a taste diagnosis result $JDR_p$=[$ALY_Na_{p/}$ $ASTG_p$/$LNGS_p$/$SWT_p$/$SCT_p$/$BIT_p$] that corresponds to the name ALY $_Na_p$ of the analyte, the index value $ASTG_p$ for "astringency", the index value $LNGS_p$ for "aftertaste", the index value $SWT_p$ for "sweetness", the index value $SCT_p$ for "aroma", and the index value $BIT_p$ for "bitterness".

The taste diagnostic unit 217 executes the above-mentioned operation for all of p=1 to P to generate P taste diagnosis results $JDR_1$=[$ALY_Na_1$/$ASTG_1$/$LNGS_1$/$SWT_1$/$SCT_1$/$BIT_1$] to $JDR_1$=[$ALY_Na_P$/$ASTG_P$/$LNGS_1$/$SWT_P$/$SCT_P$/$BIT_P$], and outputs the created P taste diagnosis results $JDR_1$=[$ALY_Na_1$ /$ASTG_1$ /$LNGS_1$/$SWT_1$/$SCT_1$/$BIT_1$] to $JDR_P$ = [$ALY_Na_P$/$ASTG_P$/$LNGS_P$/$SWT_P$/$SCT_P$/$BIT_P$] to the control unit 212 and the display unit 218.

In addition, when the name $ALY_Na_p$ of the analyte is "grapes", the taste diagnostic unit 217 diagnoses that the "astringency" of the analyte having the name ALY $_Na_p$ (=grapes) is the index value $ASTG_{th_p}$ based on the Body index (-) $_th_p$ by a method described below.

Then, the taste diagnostic unit 217 executes, for all of p=1 to P, creating a diagnosis result $JDR_{th_p}$=[$ALY_Na_p$ (=grapes)/$ASTG_{th_p}$] in which the name $ALY_Na_p$ (=grapes) of the analyte is associated with the index value $ASTG_{th_p}$ of "astringency", and creates P taste diagnosis results $JDR_{th_1}$=[$ALY_Na_1$(=grapes)/$ASTG_{th_1}$] to $JDRt_{h_P}$ = [ALY_Nap (=grapes) /$ASTG_{th_P}$], and outputs the P taste diagnosis results $JDR_{th_1}$ = [$ALY_Na_1$ (=grapes) / $ASTGt_{h_1}$] to $JDR_{th_P}$ = [$ALY_Na_P$ (=grapes)/$ASTG_{th_P}$] to the control unit 212 and display unit 218.

When the display unit 218 receives the diagnosis result $JDR_{uni}$=[$ALY_Na_{uni}$ /$ASTG_{uni}$ /$LNGS_{uni}$ /$SWT_{uni}$/$SCT_{uni}$/$BIT_{uni}$] from the taste diagnostic unit 217, the display unit 218 displays the received diagnosis result $JDR_{uni}$=[ALY $Na_{uni}$/$ASTG_{uni}$/$LNGS_{uni}$/$SWT_{uni}$ /$SCT_{uni}$/$BIT_{uni}$].

Furthermore, when the display unit 218 receives the diagnosis result $JDR_{th_uni}$=[$ALY_Na_{uni}$ (=grapes)/$ASTG_{th_uni}$] from the taste diagnostic unit 217, the display unit 218 displays the received diagnosis result $JDR_{th_uni}$=[$ALY_Na_{uni}$ (=grapes)/$ASTG_{th_uni}$].

Furthermore, when the display unit 218 receives P diagnosis results $JDR_1$=[$ALY_Na_1$ /$ASTG_1$/$LNGS_1$/$SWT_1$/$SCT_1$/$BIT_1$] to $JDR_P$=[$ALY_Na_P$/$ASTG_P$/$LNGS_P$/$SWT_P$/$SCT_P$ /$BIT_P$] from the taste diagnostic unit 217, the display unit 218 displays the received P diagnosis results $JDR_1$=[$ALY_Na_1$/$ASTG_1$/$LNGS_1$/$SWT_1$/$SCT_1$/$BIT_1$] to $JDR_P$ = [$ALY_Na_P$/$ASTG_P$P$LNGS_P$/$SWT_P$/$SCT_P$/$BIT_P$].

Furthermore, when the display unit 218 receives P taste diagnosis results $JDR_{th_1}$= [$ALY_Na_1$ (= grapes)/$ASTG_{th_1}$] to $JDR_{th_P}$=[$ALY_Na_p$ (= grapes)/$ASTG_{th_P}$] from the taste diagnostic unit 217, the display unit 218 displays the received P taste diagnosis results $JDR_{th_1}$=[$ALY_Na_1$(=grapes)/$ASTG_{th_1}$] to $JDR_{th_P}$ = [$ALY_Na_P$ (= grapes)/$ASTG_{th_P}$.

The reception unit 219 receives requests $RQT_{uni}$ or $RQT_q$ from staff at restaurants such as Japanese restaurants, Chinese restaurants, and Western restaurants, master brewers at sake breweries, and staff at liquor stores, and outputs the received requests $RQT_{uni}$ or $RQT_q$ to the control unit 212.

[Calculation of integral value]

Fig. 9 and Fig. 10 are first and second conceptual diagrams for explaining a method for calculating an integral value, respectively.

Referring to Fig. 9, the cyclic voltammogram CVG is measured, for example, by scanning the potential V from 0 V to +2500 mV at a predetermined scanning speed, then scanning the potential V from +2500 mV to 0 V at a predetermined scanning speed, further scanning the potential V from 0 V to -2500 mV at a predetermined scanning speed, and further scanning the potential V from -2500 mV to 0 V at a predetermined scanning speed.

As a result, in the cyclic voltammogram CVG, the solid line portion represents the current value I in when the potential V is scanned in the positive direction, and the dotted line portion represents the current value I in when the potential V is scanned in the negative direction.

Therefore, in the cyclic voltammogram CVG, the solid line portion represents the current value I of the oxidation wave, and the dotted line portion represents the current value I of the reduction wave.

The predetermined scanning speed is, for example, any one of 0.3 V/sec, 0.5 V/sec, and 0.6 V/sec.

When calculating the integral value ITG of the cyclic voltammogram CVG, the predetermined potential section V_ITV is, for example, [0 to 100 mV], [101 to 200 mV], [201 to 300 mV], ..., [2301 to 2400 mV], [2401 to 2500 mV], [0 to -100 mV], [-101 to - 200 mV], ..., [-2301 to -2400 mV], [-2401 to -2500 mV].

Fig. 10 shows an enlarged view of a portion at a predetermined potential section [$V_1$ to $V_2$] of the cyclic voltammogram CVG shown in Fig. 9.

Referring to Fig. 10, when the calculation unit 213 calculates the integral value ITG in a predetermined potential

section [$V_1$ to $V_2$], the calculation unit 213 detects the current value Iox_1 of the oxidation wave and the current value Ird_1 of the reduction wave at potential $V_1$, and calculates the difference (Iox_1-Ird_1) between the current value Iox_1 and the current value Ird_1 to calculate the intensity (Iox_1-Ird_1) of the cyclic voltammogram CVG at potential $V_1$.

Then, the calculation unit 213 detects current value Iox_2 of the oxidation wave and current value Ird_2 of the reduction wave at a potential $V_1$+1 obtained by adding a unit potential (=1 mV) to the potential $V_1$, and calculates the difference (=Iox_2-Ird_2) between the current value Iox_2 and the current value Ird_2 to calculate the intensity (Iox_2-Ird_2) of the cyclic voltammogram CVG at the potential $V_1$+1.

Furthermore, the calculation unit 213 detects the oxidation wave current value Iox_3 and the reduction wave current value Ird_3 at a potential $V_1$+2 obtained by adding a unit potential (=1 mV) to the potential $V_1$+1, and calculates the difference (Iox_3-Ird_3) between the current value Iox_3 and the current value Ird_3 to calculate the intensity (Iox_3-Ird_3) of the cyclic voltammogram CVG at the potential $V_1$+2.

Similarly, the calculation unit 213 detects the oxidation wave current value Iox_N and the reduction wave current value Ird_N at potential $V_2$ which is obtained by adding a unit potential (=1 mV) to potential $V_2$-1, and calculates the difference (Iox_N-Ird_N) between the current value Iox_N and the current value Ird_N to calculate the intensity (Iox_N-Ird_N) of the cyclic voltammogram CVG at potential $V_2$.

Then, the calculation unit 213 calculates the integral value ITG in the predetermined potential section [$V_1$ to $V_2$] by the following formula.

[Equation 2]

$$ITG(V_1 - V_2) = (Iox_1 - Ird_1) + (Iox_2 - Ird_2) + \cdots + (Iox_N - Ird_N) \cdots (2)$$

That is, the calculation unit 213 calculates multiple intensities ((Iox_1-Ird_1), (Iox_2-Ird_2), ..., (Iox_N-Ird_N)) of the cyclic voltammogram CVG for each unit potential (=1 mV) in the predetermined potential section [$V_1$ to $V_2$], and adds up the multiple calculated intensities ((Iox_1-Ird_1), (Iox_2-Ird_2), ..., (Iox_N-Ird_N)) to calculate an integral value ITG of the cyclic voltammogram CVG in the predetermined potential section [$V_1$ to $V_2$].

Here, in the predetermined potential section [$V_1$ to $V_2$], calculating the difference (Iox_1-Ird_1) at the potential $V_1$, calculating the difference (Iox_2-Ird_2) at the potential $V_1$+1, calculating the difference (Iox_3-Ird_3) at the potential $V_1$ +2, ..., and calculating the difference (Iox_N-Ird_N) at the potential $V_2$ corresponds to calculating a plurality of intensities in one specified potential section by executing for all of unit potential in one specified potential section calculating the intensity of the cyclic voltammogram at one unit potential by performing a subtraction process of subtracting the current value of the reduction wave from the current value of the oxidation wave of a cyclic voltammogram at one unit potential in one specified potential section for all unit potentials in one specified potential section.

Calculating the integral value ITG in a predetermined potential section [$V_1$ to $V_2$] using equation (2) is equivalent to calculating the sum of the calculated intensities as the area of a cyclic voltammogram in one predetermined potential section.

Furthermore, calculating the difference (Iox_1-Ird_1) at potential $V_1$, calculating the difference (Iox_2-Ird_2) at potential $V_1$+1, calculating the difference (Iox_3-Ird_3) at potential $V_1$+2, ..., calculating the difference (Iox_N-Ird_N) at potential $V_2$ each corresponds to calculating the intensity of a cyclic voltammogram at one unit potential by subtracting the current value of the reduction wave from the current value of the oxidation wave of a cyclic voltammogram at one unit potential in one specified potential range.

In addition, the calculation unit 213 may calculate a sum SUM_Iox of the current values Iox_1 to Iox_N of the N oxidation waves and a sum SUM_Ird of the current values Ird_1 to Ird_N of the N reduction waves in a predetermined potential section [$V_1$ to $V_2$], and subtract the sum SUM_Ird from the sum SUM_Iox to calculate an integral value ITG of the cyclic voltammogram CVG in the predetermined potential range [$V_1$ to $V_2$].

The calculation unit 213 calculates a plurality of integral values $ITG_1$, $ITG_2$, $ITG_3$, ..., $ITG_{24}$, $ITG_{25}$, $ITG_{26}$, $ITG_{27}$, ..., $ITG_{49}$, and $ITG_{50}$ in a plurality of predetermined potential sections [0 to 100 mV], [101 to 200 mV], [201 to 300 mV], ..., [2301 to 2400 mV], [2401 to 2500 mV], [0 to -100 mV], [-101 to -200 mV], ..., [-2301 to -2400 mV], and [-2401 to -2500 mV] by the method of calculating the integral value ITG of the cyclic voltammogram CVG in the predetermined potential section [$V_1$ to $V_2$] described above.

By calculating multiple integral values $ITG_1$ to $ITG_{50}$ using the methods shown in Figs. 9 and 10 described above, it is possible to correct the characteristic variations between the sensors used to measure the cyclic voltammogram CVG, and clearly show the signal intensity differences between the solutions of the analyte.

In the above-mentioned formula (2), (Iox_1-Ird_1), (Iox_2-Ird_2), ..., (Iox_N-Ird_N) each represent the current value in the oxidation reaction and reduction reaction between the electrode (working electrode 112) and the analyte at each unit potential.

Furthermore, the sum (=integral value) of the subtraction results (Iox-Ird) in one specified potential section represents the total number of electrons in the oxidation reaction and reduction reaction between the electrode (working electrode) and the analyte in one specified potential section.

Furthermore, a curve CUR showing the class dependence of multiple integral values $ITG_1$, $ITG_2$, $ITG_3$, ..., $ITG_{24}$, $ITG_{25}$, $ITG_{26}$, $ITG_{27}$, ..., $ITG_{49}$, and $ITG_{50}$ represents the dependence of the total number of electrons (=integral value) in the oxidation reaction and reduction reaction between the electrode (working electrode) and the analyte on a specified potential section.

Fig. 11 is a diagram showing a part of a cyclic voltammogram. Fig. 11 shows a cyclic voltammogram with a region REG where the oxidation wave lies below the reduction wave. Fig. 11(a) shows the case where region REG is located in the positive current region, FIG. 11(b) shows the case where, in region REG, the reduction wave is located in the positive current region and the oxidation wave is located in the negative current region, and FIG. 11(c) shows the case where region REG is located in the negative current region.

Here, the current values of the oxidation wave at the unit potential of region REG are Iox_1_REG, Iox_2_REG, ..., Iox_N'_REG (N' is the total number of unit potentials in region REG), and the current values of the reduction wave at the unit potential of region REG are Ird_1_REG, Ird_2_REG, ..., Ird_N'_REG.

Referring to (a) of Fig. 11, when calculating the integral value of a specified potential section in region REG, the oxidation wave current values $I_{ox_1_REG}$, $I_{ox_2_REG}$, ..., $I_{ox_N'_REG}$ are respectively smaller than the reduction wave current values $I_{rd_1_REG}$, $I_{rd_2_REG}$, ..., $I_{rd_N'_REG}$, and therefore the integral value ITG of the specified potential section in region REG becomes a negative value.

Referring to (b) of Fig. 11, when calculating the integral value of a specified potential section in region REG, each of the oxidation wave current values $I_{ox_1_REG}$, $I_{ox_2_REG}$, ..., $I_{ox_N'_REG}$ is a negative current value, and each of the reduction wave current values $I_{rd_1_REG}$, $I_{rd_2_REG}$, ..., $I_{rd_N'_REG}$ is a positive current value, so that the integral value ITG of the specified potential section in region REG is a negative value.

Referring to (c) of Fig. 11, when calculating the integral value of a predetermined potential section in the region REG, each of the oxidation wave current values $I_{ox_1_REG}$, $I_{ox_2_REG}$, ..., $I_{ox_N'_REG}$ is negative current values, each of the reduction wave current values $I_{rd_1_REG}$, $I_{rd_2_REG}$, ..., $I_{rd_N'_R}$ is negative current values, and since the absolute values $|I_{ox_1_REG}|$, $|I_{ox_2_REG}|$, ..., $I_{ox_N'_REG}$ of the oxidation wave current values $I_{ox_1_REG}$, $I_{ox_2_REG}$, ..., $I_{rd_N'_REG}$ are greater than the absolute values $|I_{rd_1_REG}|$, $|I_{rd_2_REG}|$, ..., $|I_{rd_N'_R}|$ of the reduction wave current values $I_{rd_1_REG}$, $I_{rd_2_REG}$, ..., $I_{rd_N'_R}$, respectively, the integral value ITG of a predetermined potential section in the region REG becomes a negative value.

Therefore, in embodiment of the present invention, the integral value in a predetermined potential section of a cyclic voltammogram having a region REG where the oxidation wave is located below the reduction wave becomes a negative value in the region REG.

The calculation unit 213 may calculate a plurality of integral values in a plurality of predetermined potential sections by a method different from the above-mentioned method.

For example, the calculation unit 213 may use the potential V as an explanatory variable and the current I as a target variable to calculate a regression curve RC1 (the curve indicated by the solid line) showing the oxidation wave of the cyclic voltammogram CVG in Fig. 9 and a regression curve RC2 (the curve indicated by the dashed line) showing the reduction wave, calculate an integral value ITG_RC1 of the regression curve RC1 and an integral value ITG_RC2 of the regression curve RC2 in a predetermined potential section [$V_1$-$V_2$], and executes for all predetermined potential sections calculating an integral value of the cyclic voltammogram CVG shown in Fig. 9 in the predetermined potential section [$V_1$-$V_{2]}$ by subtracting the integral value ITG_RC2 from the integral value ITG_RC1, thereby may calculate a plurality of integral values in a plurality of predetermined potential sections.

The calculation unit 213 may calculate the multiple integral values in the multiple predetermined potential sections by any method as long as the method can calculate the multiple integral values in the multiple predetermined potential sections.

Fig. 12 is a diagram for explaining a method for creating a curve CUR showing the relationship between a plurality of classes Cls and a plurality of integral values ITG.

Fig. 12 (a) shows one piece of calculation data $CAL_{uni}$, and Fig. 12 (b) shows a curve $CUR_{uni}$ that represents the relationship between the integral value and the class.

Referring to (a) of Fig. 12, the calculation data $CAL_{uni}$ includes a name ALY-$Na_{uni}$ of the analyte, a type ALY-$Kd_{uni}$ of the analyte, a class Cls, integral values $ITG_{_Low}$, $ITG_{_Middle}$, $ITG_{_High}$, and a sum $ITG_{ST}$ of the integral values.

The integral value $ITG_{_Low}$ is an integral value calculated by the above-mentioned method based on the cyclic voltammogram CVG measured by changing the potential V at a potential scanning rate $V_{r_Low}$ (e.g., 0.3 V/sec), the integral value $ITG_{_Middle}$ is an integral value calculated by the above-mentioned method based on the cyclic voltammogram CVG measured by changing the potential V at a potential scanning rate $V_{r_Middle}$ (e.g., 0.5 V/sec), and the integral value $ITG_{_High}$ is an integral value calculated by the above-mentioned method based on the cyclic voltammogram CVG measured by changing the potential V at a potential scanning rate $V_{r_High}$ (e.g., 0.6 V/sec).

The class Cls consists of n classes $Cls_1$ to $Cls_n$, the integral value $ITG_{_Low}$ consists of n integral values $ITG_{1_Low}$ to $ITG_{n_Low}$, the integral value $ITG_{_Middle}$ consists of n integral values $ITG_{1_Middle}$ to $ITG_{n_Middle}$, and the integral value

$ITG_{_High}$ consists of n integral values $ITG_{1_High}$ to $ITG_{n_High}$. Here, n represents the total number of predetermined potential sections, when the potential scanning range is [$-V_{S1}$ to + $V_{S2}$] and one predetermined potential section is $V_{PTS}$, n = $(|-V_{S1}|+|+V_{S2}|)/V_{PTS}$.

The n integral values $ITG_{1_Low}$ to $ITG_{n_Low}$ are integral values calculated by the above-mentioned method based on the potentials $V_{1_Low}$ to $V_{d_Low}$ and the current values $I_{1_Low}$ to $I_{d_Low}$ shown in Fig. 6, the n integral values $ITG_{1_Middle}$ to $ITG_{n_Middle}$ are integral values calculated by the above-mentioned method based on the potentials $V_{1_Middle}$ to $V_{d_Middle}$ and the current values $I_{1_Middle}$ to $I_{d_Middle}$ shown in Fig. 6, and the n integral values $ITG_{1_High}$ to $ITG_{n_ High}$ are integral values calculated by the above-mentioned method based on the potentials $V_{1_High}$ to $V_{d_High}$ and the current values $I_{1_High}$ to $I_{d_High}$ shown in Fig. 6.

The sum of the integral values $ITG_{ST}$ consists of the sum [$ITG_{1_Low}$+$ITG_{1_Middle}$+$ITG_{1_High}$] to [$ITG_{n_Low}$+$ITG_{n_Middle}$+$ITG_{n_High}$] of n integral values.

Here, the sum $IT_{G1_Low}$+$ITG_{1_Middle}$+$ITG_{1_High}$] to [$ITG_{n_Low}$+$ITG_{n_Middle}$+$ITG_{n_ High}$] of n integral values is expressed as "the sum $ITG_{_SMT1}$ to $ITG_{_SMTn}$ of n integral values".

The n integral values $ITG_{1_Low}$ to $ITG_{n_Low}$, the n integral values $ITG_{1_Middle}$ to $ITG_{n_Middle}$, the n integral values $ITG_{1_High}$ to $ITG_{n_High}$, and the sums $ITG_{_SMT1}$ to $ITG_{_SMTn}$ of the n integral values are associated with n classes $Cls_1$ to $Cls_n$, respectively.

When the creation unit 215 receives one piece of calculation data $CAL_{uni}$ and a signal S_u indicating that the calculation data is one from the calculation unit 213, the creation unit 215 judges that the calculation data CAL calculated by the calculation unit 213 is one based on the signal S_u.

Then, the creation unit 215 detects a set of (class $Cls_1$, sum $ITG_{_SMT1}$ of integral values) that corresponded to each other from the calculation data $CAL_{uni}$, then detects a set of (class $Cls_2$, sum $ITG_{_SMT2}$ of integral values) from the calculation data $CAL_{un}$i, and similarly detects a set of (class $Cls_{n-1}$, sum $ITG_{_SMTn-1}$ of integral values) from the calculation data $CAL_{uni}$, and detects a set of (class $Cls_n$, sum $ITG_{_sMTn}$ of integral values) from the calculation data $CAL_{uni}$.

Then, the creation unit 215 plots one set (class $Cl_{s1}$, sum $ITG_{_SMT1}$ of integral values), one set (class $Cls_2$, sum $ITG_{_SMT2}$ of integral values), one set (class $Cls_3$, sum $ITG_{_SMT3}$ of integral values), ..., one set ($Cls_{n-2}$, sum $ITG_{_SMTn-2}$ of integral values), one set (class $Cls_{n-1}$, sum $ITG_{_SMTn-1}$ of integral values), and one set (class $Cls_n$, sum $ITG_{_SMTn}$ of integral values) on a graph with the horizontal axis representing classes and the vertical axis representing integral values.

Thereafter, the creation unit 215 creates a curve $CUR_{uni}$ by connecting the n plotted points. In this case, the n plotted points are plotted for each class Cls, so that the creation unit 215 can create a curve $CUR_{uni}$ consisting of a smooth curve by connecting the n plotted points.

The creation unit 215 may plot n points and then create the curve $CUR_{uni}$ by determining a regression curve using the class Cls as an explanatory variable and the sum $ITG_{_SMT}$ of the integral values as a response variable.

Then, the creation unit 215 sets the curve $CUR_{uni}$ as [an index curve which is a curve that serves as an index when identifying the analyte].

In addition, the creation unit 215 receives P calculation results $CAL_RLS_1$ to $CAL_RLS_P$ (i.e., multiple calculation results) from the calculation unit 213, and also receives, from the judgment unit 214, a judgment result JDGR (judgment result shown in Table 1) indicating whether the P calculation data $CAL_1$ to $CAL_P$ are or not different.

Then, the creation unit 215 executes, for all P pieces of calculation data $CAL_1$ to $CAL_P$, creating a curve $CUR_p$ showing the class dependency of the sum $ITG_{_SMT}$ of integral values based on one calculation data (p is any of 1 to P) using the method described with reference to Fig. 12, thereby creating P curves $CUR_1$ to $CUR_P$ (i.e., multiple curves CUR).

In this case, each of the P pieces of calculation data $CAL_1$ to $CAL_P$ (that is, a plurality of calculation data) has the same configuration as the calculation data $CAL_{uni}$ shown in Fig. 12 (a).

The curve $CUR_{uni}$ represents the dependency of [the sum $ITG_{_SMT1}$ to $ITG_{_SMTn}$ of n integral values] on [n classes $Cls_1$ to $Cls_n$]. Since each of the n classes $Cls_1$ to $Cls_n$ consists of a predetermined potential section, the curve $CUR_{uni}$ is a curve that represents the dependency of the integral value on the predetermined potential section. Similarly, each of the P curves $CUR_1$ to $CUR_P$ is also a curve that indicates the dependency of the integral value on a predetermined potential section.

When the creation unit 215 have created one curve $CUR_{uni}$, the creation unit 215 adds the curve $CUR_{uni}$ to the calculation result $CAL_RLS_{uni}$ to create an analysis result $ALY_RLS_{uni}$=$ID_{uni}$/$CA_{uni}$/$CUR_{uni}$] and outputs the created analysis result $ALY_RLS_{uni}$=[$ID_{uni}$/$CAL_{uni}$/$CUR_{uni}$]to the control unit 212.

On the other hand, when the creation unit 215 have created P curves $CUR_1$ to $CUR_P$, the creation unit 215 adds the P curves $CUR_1$ to $CUR_P$ to the P calculation results $CAL_RLS_1$ to $CAL_RLS_P$, respectively, to create P analysis results $ALY_RLS_1$=[$ID_1$/$CAL_1$/$CUR_1$] to $ALY_RLS_P$=[$ID_P$/$CAL_P$/$CUR_P$], and outputs the P analysis results $ALY_RLS_1$ to $ALY_RLS_P$ and the judgment results (the judgment results shown in Table 1) to the control unit 212.

[Taste diagnosis]

(I) Taste diagnosis of Shochu

When the calculation unit 216 receives the index data $IDX_{uni}$ from the control unit 212, the calculation unit 216 detects the calculation data $CAL_{uni}$ (see Fig. 12) from the index data $IDX_{uni}$. Then, the calculation unit 216 detects the integral value $ITG_{_Low}$ ($ITG_{1_Low}$ to $ITG_{n_Low}$), the integral value $ITG_{_Middle}$, ($ITG_{1_Middle}$ to $ITG_{n_Middle}$) and the integral value $ITG_{_High}$ ($ITG_{1_High}$ to $ITG_{n_High}$) from the calculation data $CAL_{uni}$ (see Fig. 12).

Fig. 13 is a conceptual diagram showing the class dependency of the integral value created based on cyclic voltammograms measured while changing the potential scanning rate to the potential scanning $V_{r_Low}$, $V_{r_Middle}$, and $V_{r_High}$.

Fig. 13 (a) is a conceptual diagram showing the dependency of the integral value on the class created based on a cyclic voltammogram measured with the potential scanning rate set to "$V_{r_Low}$", Fig. 13 (b) is a conceptual diagram showing the dependency of the integral value on the class created based on a cyclic voltammogram measured with the potential scanning rate set to "$V_{r_Middle}$", and Fig. 13 (c) is a conceptual diagram showing the dependency of the integral value on the class created based on a cyclic voltammogram measured with the potential scanning rate set to "$V_{r_High}$".

Based on the integral value $ITG_{_Low}$ ($ITG_{1_Low}$ to $ITG_{n_Low}$), the calculation unit 216 detects x integral values $ITG_{1_Low(+)}$ to $ITG_{x_Low}$(+) in x (x is an integer satisfying x=INT(n/2)) classes (see Fig. 13(a)) corresponding to a predetermined positive potential section, and calculates a sum $SMT_{_Low}$(+) of the detected x integral values $ITG_{1_Low}$ (+) to $ITG_{x_Low(+)}$. Here, "INT(n/2)" is the result converting the result of dividing n by 2 into an integer.

In addition, the calculation unit 216 detects x integral values $ITG_{1_Middle(+)}$ to $ITG_{x_Middle(+)}$ in x classes (see (b) of Fig. 13) corresponding to a predetermined positive potential section based on the integral value $ITG_{_Middle}$ ($ITG_{1_Middle}$ to $ITG_{n_Middle}$), and calculates the sum $SMT_{_Middle(+)}$ of the detected x integral values $ITG_{1_Middle(+)}$ to $ITG_{x_Middle(+)}$.

Furthermore, the calculation unit 216 detects x integral values $ITG_{1_High(+)}$ to $ITG_{X_High(+)}$ in x classes (see (c) of Fig. 13) corresponding to a predetermined positive potential range based on the integral value $ITG_{_High}$ ($ITG_{1_High}$ to $ITG_{n_High}$), and calculates the sum $SM_{_High(+)}$ of the detected x integral values $ITG_{1_High(+)}$ to $ITG_{x_High(+)}$.

Then, the calculation unit 216 calculates the Body index (+) based on the sums $SMT_{_Low(+)}$, $SMT_{_Middle(+)}$, and $SMT_{_High(+)}$ according to the following formula:

[Equation 3]

$$Body\ index\ (+) = \frac{SMT_{_Middle(+)}}{SMT_{_High(+)}} + \frac{SMT_{_Low(+)}}{SMT_{_High}} \cdots (3)$$

The Body Index (+) is a factor based on the diffusion coefficient of the components of the analyte when a positive potential is applied to the analyte.

In addition, the calculation unit 216 detects n integral values $ITG_{1_Low(all)}$ to $ITG_{n_Low(all)}$ in n class sections (see (a) of Fig. 13) corresponding to n predetermined potential sections based on the integral value $ITG_{_Low}$ ($ITG_{1_Low}$ to $ITG_{n_Low}$), and calculates the sum $SMT_{_Low(all)}$ of the detected n integral values $ITG_{1_Low(all)}$ to $ITG_{n_Low(all)}$.

Next, the calculation unit 216 detects n integral values $ITG_{1_Middle(all)}$ to $ITG_{n_Middle(all)}$ in n class sections (see (b) of Fig. 13) corresponding to n predetermined potential sections based on the integral value $ITG_{_Middle}$ ($ITG_{1_Middle}$ to $ITG_{n_Middle}$), and calculates the sum $SMT_{_Middle(all)}$ of the detected n integral values $ITG_{1_Middle(all)}$ to $ITG_{n_Middle(all)}$.

Furthermore, the calculation unit 216 detects n integral values $ITG_{1_High(all)}$ to $ITG_{n_High(all)}$ in n class sections (see (c) of Fig. 13) corresponding to n predetermined potential sections based on the integral value $ITG_{_High}$ ($ITG_{1_High}$ to $ITG_{n_High}$), and calculates the sum $SMT_{_High(all)}$ of the detected n integral values $ITG_{1_High(all)}$ to $ITG_{n_High(all)}$.

Then, the calculation unit 216 calculates the Body index (all) based on the sums $SMT_{_Low(all)}$, $SMT_{_Middle(all)}$, and $SMT_{_High(all)}$ by the following formula.

[Equation 4]

$$Body\ index(all) = \frac{SMT_{_Middle(all)}}{SMT_{_High(all)}} + \frac{SMT_{_Low(all)}}{SMT_{_High(all)}} \cdots (4)$$

The Body Index (all) is a factor based on the diffusion coefficients of the components of the analyte when positive and negative potentials are applied to the analyte.

Furthermore, the calculation unit 216 sets the sum $SMT_{_High(+)}$ used in the calculation of the Body index(+) to H(+) _sum (=$SMT_{_High(+)}$).

Furthermore, based on the integral value $ITG_{_High}$ ($ITG_{1_High}$ to $ITG_{n_High}$), the calculation unit 216 detects x integral values $ITG_{1_High(-)}$ to $ITG_{x_High(-)}$ in x classes (see (c) of Fig. 13) corresponding to a negative predetermined potential section, and calculates the sum $SMT_{_High(-)}$ of the detected x integral values $ITG_{1_High(-)}$ to $ITG_{x_High(-)}$ as H(-)_sum.

Then, the calculation unit 216 outputs the Body index (+), the Body index (all), H(+)_sum (=$SMT_{_High(+)}$), and H(-)_sum (=$SMT_{_High(-)}$) to the taste diagnostic unit 217.

The taste diagnostic unit 217 receives the Body index (+), the Body index (all), H(+)_sum (=$SMT_{_High(+)}$), and H(-)_sum (=$SMT_{_High(-)}$) from the calculation unit 216.

(1) Diagnosis of "astringency"

When the taste diagnostic unit 217 receives the Body index (+) from the calculation unit 216, the taste diagnostic unit 217 substitutes the Body index (+) into the following formula to calculate the result (=ASTG), and diagnoses the result as the astringency ASTG of the shochu being analyte.
[Equation 5]

$$ASTG = Body\ index\ (+) \times k_1 \cdots (5)$$

In the equation (5), the coefficient $k_1$ is, for example, 1.5.

(2) Diagnosis of "Aftertaste"

When the taste diagnostic unit 217 receives the Body index (all) from the calculation unit 216, the taste diagnostic unit 217 substitutes the Body index (all) into the following formula to calculate and diagnoses the calculation result (=LNGS) as the aftertaste LNGS of the shochu being analyte.
[Equation 6]

$$LNGS = Body\ index(all) \times k_2 \cdots (6)$$

In the equation (6), the coefficient $k_2$ is, for example, 2.

(3) Diagnosis of "Sweetness"

When the taste diagnostic unit 217 receives H(+)_sum (=$SMT_{_High(+)}$) from the calculation unit 216, the taste diagnostic unit 217 diagnoses the calculation result (=SWT) calculated by substituting H(+)_sum (=$SMT_{_High(+)}$) to the following equation to diagnose the calculation result (=SWT) as the "sweetness" of the shochu, which is the analyte.
[Equation 7]

$$SWT = [H(+)_sum/k_3] \times k_4 \cdots (7)$$

In the equation (7), the coefficient $k_3$ is, for example, 3000 and the coefficient $k_4$ is, for example, 1.5.

(4) Diagnosis of "Aroma"

When the taste diagnostic unit 217 receives H(-)_sum (=$SMT_{_High(-)}$) from the calculation unit 216, the taste diagnostic unit 217 substitutes H(-)_sum (=$SMT_{_High(-)}$) into the following equation to diagnose the calculation result (=SCT) as the "aroma" of shochu, which is the analyte.
[Equation 8]

$$SCT = [H(-)_sum/k_5] \times k_6 \cdots (8)$$

In the equation (8), the coefficient $k_5$ is, for example, 2000 and the coefficient $k_6$ is, for example, 1.5.

(5) Diagnosis of "Bitterness"

The taste diagnostic unit 217 substitutes the "astringency" (=ASTG in equation (5)) and "sweetness" (=SWT in equation (7)) diagnosed by the above-mentioned method into the following formula to calculate the calculation result (=BIT), and diagnoses the calculation result (=BIT) as the "bitterness" of the shochu, which is the analyte.
[Equation 9]

$$BIT = ASTG \times k_7 - SWT \times k_8 \cdots (9)$$

In the equation (9), the coefficient $k_7$ is, for example, 1.5 and the coefficient $k_8$ is, for example, 0.4.

The coefficient $k_1$ in equation (5), the coefficient $k_2$ in equation (6), the coefficients $k_3$ and $k_4$ in equation (7), the coefficients $k_5$ and $k_6$ in equation (8), and the coefficients $k_7$ and $k_8$ in equation (9) are determined using multiple shochu as teacher data whose "astringency," "aftertaste," "sweetness," "aroma," and "bitterness" are known.

Fig. 14 is a conceptual diagram of the teacher data. Referring to Fig. 14, the teacher data consists of teacher data TH1 to TH5. The teacher data TH1 to TH5 are stored in database 22 in advance.

The teacher data TH1 is teacher data for determining the value of the coefficient $k_1$ in the formula (5), the teacher data TH2 is teacher data for determining the value of the coefficient $k_2$ in the formula (6), the teacher data TH3 is teacher data for determining the values of the coefficients $k_3$, $k_4$ in the formula (7), the teacher data TH4 is teacher data for determining the values of the coefficients $k_5$, $k_6$ in the formula (8), the teacher data TH5 is teacher data for determining the values of the coefficients $k_7$, $k_8$ in the formula (9).

When determining the values of the coefficients $k_1$ to $k_8$, the taste diagnostic unit 217 reads out the teacher data TH1 to TH5 from the database 22.

(A) Determination of coefficient $k_1$

The training data TH1 includes astringency and Body index (+). The astringency is, for example, made up of ten numbers of $a_1$ to $a_{10}$, and the Body index (+) is, for example, made up of ten numbers of $b(+)_1$ to $b(+)_{10}$. The ten of $a_1$ to $a_{10}$ are known.

The $b(+)_y$ (y=1 to 10) is obtained as follows.

(i) The sensor device 1 measures cyclic voltammograms $CVG_{_Low_y}$, $CVG_{_Middle_y}$, and $CVG_{_High_y}$ for one shochu y by changing the potential scanning rate to $V_{r_Low}$, $V_{r_Middle}$, and $V_{r_High}$, respectively, and transmits the measured cyclic voltammograms $CVG_{_Low_y}$, $CVG_{_Middle_y}$, and $CVG_{_High_y}$ to personal computer PC by wireless communication or wired communication.
(ii) The personal computer PC obtains the correspondence relationship $[(Cls_1\text{-}ITG_1)$ to $(Cls_n\text{-}ITG_n)_{_Low_y}$ between the classes Cls and the integral values ITG based on the cyclic voltammogram $CVG_{_Low_y}$ by the method described above.
(iii) The personal computer PC obtains the correspondence relationship $[(Cls_1\text{-}ITG_1)$ to $(Cls_n\text{-}ITG_n)]_{_Middle_y}$ between the class Cls and the integral value ITG based on the cyclic voltammogram $CVG_{_Middle_y}$ by the method described above.
(iv) The personal computer PC obtains the correspondence relationship $[(Cls_1\text{-}ITG_1)$ to $(Cls_n\text{-}ITG_n)]_{_High_y}$ between the class Cls and the integral value ITG based on the cyclic voltammogram $CVG_{_High_y}$ by the method described above.
(v) The personal computer PC detects (n/2) integral values associated with the class corresponding to the positive predetermined potential section based on the correspondence relationship $[(Cls_1\text{-}ITG_1)$ to $(Cls_n\text{-}ITG_n)]_{_Low_y}$, calculates the sum of the detected (n/2) integral values, and obtains $[\text{Sum of Low}(+)]_{_y}$.
(vi) The personal computer PC detects (n/2) integral values associated with the class corresponding to the positive predetermined potential section based on the correspondence relationship $[(Cls_1\text{-}ITG_1)$ to $(Cls_n\text{-}ITG_n)]_{_Middle_y}$, calculates the sum of the detected (n/2) integral values, and obtains $[\text{Sum of Middle}(+)]_{_y}$.
(vii) The personal computer PC detects (n/2) integral values associated with the class corresponding to the positive predetermined potential section based on the correspondence relationship $[(Cls_1\text{-}ITG_1)$ to $(Cls_n\text{-}ITG_n)]_{_High_y}$, calculates the sum of the detected (n/2) integral values, and obtains $[\text{Sum of High}(+)]_{_y}$.
(viii) The personal computer PC calculates $b(+)_y$ by substituting $[\text{Sum of Low}(+)]_y$, $[\text{Sum of Middle}(+)]_{_y}$, and $[\text{Sum of High}(+)]_{_y}$ into $SMT_{_Low(+)}$, $SMT_{_Middle(+)}$, and $SMT_{_High}(+)$ in equation (3), respectively.
(ix) The personal computer PC executes the above steps (i) to (viii) for all of y=1 to 10 to obtain $b(+)_1$ to $b(+)_{10}$.
(x) When the personal computer PC acquires $b(+)_1$ to $b(+)_{10}$, the personal computer PC associates the acquired $b(+)_1$ to $b(+)_{10}$ with $a_1$ to $a_{10}$, respectively, and stores them in the "Body Index (+)" column of the teacher data TH1.

Then, based on $(a_1, b(+)_1)$, $(a_2, b(+)_2)$, ..., $(a_9, b(+)_9)$, $(a_{10}, b(+)_{10})$ of the teacher data TH1, the personal computer PC executes a regression analysis with "Body index (+)" as an explanatory variable and "astringency" as a response variable to obtain a regression equation, and determines the value multiplied to the explanatory variable "Body index (+)" in the obtained regression equation as the value of coefficient $k_1$ in equation (5).

(B) Determination of coefficient $k_2$

The teacher data TH2 includes aftertaste and Body index (all). The aftertaste is made up of, for example, ten elements $c_1$ to $c_{10}$, and the Body index (all) is made up of, for example, ten elements $b(all)_1$ to $b(all)_{10}$. Ten of $c_1$ to $c_{10}$ are known.

$b(all)_y$ (y=1 to 10) is obtained as follows.

(xi) The personal computer PC calculates the sum $SUM_{all_Low_y}$ of all the integral values $ITG_1$ to $ITG_n$ based on the correspondence relationship $[(Cls_1\text{-}ITG_1)$ to $(Cls_n\text{-}ITG_n)]_{_Low_y}$ obtained in (ii) above.

(xii) The personal computer PC calculates the sum $SUM_{all_Middle_y}$ of all the integral values $ITG_1$ to $ITG_n$ based on the correspondence relationship $[(Cls_1\text{-}ITG_1)$ to $(Cls_n\text{-}ITG_n)]_{_Middle_y}$ obtained in (iii) above.

(xiii) The personal computer PC calculates the sum $SUM_{all_High_y}$ of all the integral values $ITG_1$ to $ITG_n$ based on the correspondence relationship $[(Cls_1\text{-}ITG_1)$ to $(Cls_n\text{-}ITG_n)]_{_High_y}$ obtained in (iv) above.

(xiv) The personal computer PC calculates $b(all)_y$ by substituting the sum $SUM_{all_Low_y}$, the sum $SUM_{all_Middle_y}$, and the sum $SUM_{all_High_y}$ into $SMT_{_Low(all)}$, $SMT_{_Middle(all)}$, and $SMT_{_High(all)}$ in equation (4), respectively.

(xv) The personal computer PC executes the above steps (xi) to (xiv) for all of y=1 to 10 to obtain $b(all)_1$ to $b(all)_{10}$.

(xvi) When the personal computer PC obtains $b(all)_1$ to $b(all)_{10}$, the personal computer PC associates the obtained $b(all)_1$ to $b(all)_{10}$ with $c_1$ to $c_{10}$ of the teacher data TH2, respectively, to store the obtained $b(all)_1$ to $b(all)_{10}$ in the "Body index (all)" column of the teacher data TH2.

Then, the personal computer PC executes a regression analysis using the "Body index (all)" as an explanatory variable and the "aftertaste" as a response variable based on $(c_1, b(all)_1)$, $(c_2, b(all)_2)$, ..., $(c_9, b(all)_9)$, $(c_{10}, b(all)_{10})$ of the teacher data TH2 to obtain a regression equation, and determines the value multiplied to the explanatory variable "Body index (all)" in the obtained regression equation as the value of the coefficient $k_2$ in equation (6).

(C) Determination of coefficients $k_3$, $k_4$

The teacher data TH3 includes sweetness and H(+)_sum. Sweetness is, for example, made up of ten elements $d_1$ to $d_{10}$, and H(+)_sum is, for example, made up of ten elements $h(+)_1sum$ to $h(+)_{10_sum}$. Ten values $d_1$ to $d_{10}$ are known.

$h(+)_{y_sum}$ (y=1 to 10) is obtained as follows.

(xvii) The personal computer PC executes the above (vii) for all of y=1 to 10 to obtain $h(+)_{1_}sum$ to $h(+)_{10_sum}$.

(xviii) When the personal computer PC obtains $h(+)_{1_sum}$ to $h(+)_{10_sum}$, the personal computer PC associates the obtained $h(+)_{1_sum}$ to $h(+)_{10_sum}$ with $d_1$ to $d_{10}$ of the teacher data TH3, respectively, and stores them in the "H(+)_sum" column of the teacher data TH3.

Then, based on $(d_1, h(+)_{1_sum})$, $(d_2, h(+)_{2_sum})$, ..., $(d_9, h(+)_{9_sum})$, and $(d_{10}, h(+)_{10_sum})$ of the teacher data TH3, the personal computer PC performs a regression analysis with "H(+)_sum" as the explanatory variable and "sweetness" as the target variable to obtain a regression equation, determines the value by which the explanatory variable "H(+)_sum" is divided in the obtained regression equation as the value of coefficient $k_3$ in equation (7), and determines the value multiplied to the explanatory variable "H(+)_sum" as the value of coefficient $k_4$ in equation (7).

(D) Determination of coefficients $k_5$, $k_6$

The teacher data TH4 includes an aroma and H(-)_sum. The aroma is made up of, for example, ten elements $e_1$ to $e_{10}$, and H(-)_sum is made up of, for example, ten elements $h(-)_{1_sum}$ to $h(-)_{10_sum}$. Ten of $e_1$ to $e_{10}$ are known.

h(-)y_sum (y=1 to 10) is obtained as follows.

(xix) The personal computer PC detects (n/2) integral values associated with the class corresponding to the predetermined negative potential section based on the correspondence relationship $[(Cls_1\text{-}ITG_1)$ to $(Cls_n\text{-}ITG_n)]_{_High_y}$ obtained in (iv) above, and calculates the sum of the detected (n/2) integral values to obtain $h(-)_{y_sum}$.

(xx) The personal computer PC executes (xix) for all values of y=1 to 10 to obtain $h(-)_{1_sum}$ to $h(-)_{10_sum}$.

(xxi) When the personal computer PC acquires $h(-)_{1_sum}$ to $h(-)_{10_sum}$, the personal computer PC associates the acquired $h(-)_{1_sum}$ to $h(-)_{10_sum}$ with $e_1$ to $e_{10}$ of the teacher data TH4, respectively, and stores them in "H(-)_sum" of the teacher data TH4.

Then, based on $(e_1, h(-)_{1_sum})$, $(e_2, h(-)_{2_sum})$, ..., $(e_9, h(-)_{9_sum})$, $(e_{10}, h(-)_{10_sum})$ of the teacher data TH4, the personal computer PC performs regression analysis with "H(-)_sum" as the explanatory variable and "aroma" as the target variable to obtain a regression equation, determines the value by which the explanatory variable "H(-)_sum" is divided in the obtained regression equation as the value of coefficient $k_5$ in equation (8), and determines the value multiplied to the explanatory variable "H(-)_sum" as the value of coefficient $k_6$ in equation (8).

(E) Determination of coefficients $k_7$, $k_8$

The teacher data TH5 includes "bitterness", "astringency" and "sweetness". " bitterness " is made up of, for example, ten elements $f_1$ to $f_{10}$. "Astringency" is made up of elements $a_1$ to $a_{10}$ included in the teacher data TH1. "Sweetness" is made up of elements $d_1$ to $d_{10}$ included in the teacher data TH3.

(xxii) The personal computer PC performs regression analysis with "astringency" and "sweetness" as explanatory variables and "bitterness" as the objective variable to obtain a regression equation, and determines the value multiplied to "astringency" (=ASTG) in the obtained regression equation as the value of coefficient $k_7$ in equation (9), and determines the value multiplied to "sweetness" (=SWT) as the value of coefficient $k_8$ in equation (9).

Fig. 15 is a conceptual diagram of a correspondence table showing the correspondence between coefficients and teacher data. Referring to Fig. 15, a correspondence table TBL1 includes coefficients and teacher data. The coefficients and the teacher data are associated with each other.

The coefficients are made of $k_1$ to $k_8$, and the teacher data is made of TH1 to TH5. The teacher data TH1 is associated with the coefficient $k_1$, the teacher data TH2 is associated with the coefficient $k_2$, the teacher data TH3 is associated with the coefficients $k_3$ and $k_4$, the teacher data TH4 is associated with the coefficients $k_5$ and $k_6$, and the teacher data TH5 is associated with the coefficients $k_7$ and $k_8$.

As a result, the correspondence table TBL1 indicates that coefficient $k_1$ is determined based on teacher data TH1, coefficient $k_2$ is determined based on teacher data TH2, coefficients $k_3$ and $k_4$ are determined based on teacher data TH3, coefficients $k_5$ and $k_6$ are determined based on teacher data TH4, and coefficients $k_7$ and $k_8$ are determined based on teacher data TH5.

After determining the values of the coefficients $k_1$ to $k_8$ by the above-mentioned method, the personal computer PC creates the correspondence table TBL1 and transmits the created correspondence table TBL1 to the diagnostic device 2.

The receiving unit 211 of the diagnostic device 2 receives the correspondence table TBL 1 from the personal computer PC, and outputs the received correspondence table TBL 1 to the control unit 212.

When the control unit 212 has received the correspondence table TBL 1 from the receiving unit 211, the control unit 212 stores the received correspondence table TBL 1 in the database 22.

When diagnosing "astringency", "aftertaste", "sweetness", "aroma" and "bitterness", the taste diagnostic unit 217 reads out the coefficients $k_1$ to $k_8$ of the correspondence table TBL1 stored in the database 22, and uses the read-out coefficients $k_1$ to $k_8$ to diagnose "astringency", "aftertaste", "sweetness", "aroma" and "bitterness" by the method described above.

Fig. 16 is a conceptual diagram of updated teacher data. Fig. 16 (a) shows teacher data TH1_up1 obtained by updating teacher data TH1 shown in Fig. 14 (a). Fig. 16 (b) shows teacher data TH2_up1 obtained by updating teacher data TH2 shown in Fig. 14 (b). Fig. 16 (c) shows teacher data TH3_up1 obtained by updating teacher data TH3 shown in Fig. 14 (c). Fig. 16 (d) shows teacher data TH4_up1 obtained by updating teacher data TH4 shown in Fig. 14 (d). Fig. 16 (e) shows teacher data TH5_up1 obtained by updating teacher data TH5 shown in Fig. 14 (e).

In the teacher data TH1_up1 to TH5_up1, the "1" in "up1" represents the number of updated times which the teacher data TH1 to TH5 have been updated.

With referring to Fig. 16 (a), the teacher data TH1_up1 is obtained by adding "astringency $a_{1_add}$ to $a_{v_add}$" and "Body index (+): $b(+)_{1_add}$ to $b(+)_{v_add}$" to the teacher data TH1 shown in Fig. 14(a), where v is an integer equal to or greater than 1.

With referring to Fig. 16 (b), the teacher data TH2_up1 is obtained by adding "aftertaste $c_{1_add}$ to $c_{v_add}$" and "Body index (all): $b(all)_{1_add}$ to $b(all)_{v_add}$" to the teacher data TH2 shown in Fig. 14 (b).

With referring to Fig. 16 (c), the teacher data TH3_up1 is obtained by adding "sweetness $d_{1_add}$ to $d_{v_add}$" and "H(+)_sum: $h(+)_{1_sum_add}$ to $h(+)_{v}$ sum $_{add}$" to the teacher data TH3 shown in Fig. 14 (c).

With referring to Fig. 16 (d), the teacher data TH4_up1 is obtained by adding "aroma $e_{1_add}$ to $e_{v_add}$" and "H(-)_sum: $h(-)_{1_sum_add}$ to $h(-)_{v_sum_add}$" to the teacher data TH4 shown in Fig. 14 (d).

With referring to Fig. 16 (e), the teacher data TH5_up1 is obtained by adding "bitterness $f_{1_add}$ to $f_{v_add}$", "astringency: $a_{1_add}$ to $a_{v_add}$", and "sweetness: $d_{1_add}$ to $d_{v_add}$" to the teacher data TH5 shown in Fig. 14 (e).

The taste diagnostic unit 217 uses the coefficients $k_1$ to $k_8$ to diagnose the "astringency", "aftertaste", "sweetness", "aroma" and "bitterness" of v shochus using the method described above, and then reads out teacher data TH1 to TH5 from the database 22.

Then, the taste diagnostic unit 217 adds "astringency $a_{1_add}$ to $a_{v_add}$" and "Body index (+): $b(+)_{1_add}$ to $b(+)_{v_add}$" to the teacher data TH1, and updates the teacher data TH1 to teacher data TH1_up1.

In addition, the taste diagnostic unit 217 adds "aftertaste $_{c1_add}$ to $c_{v_add}$" and "Body index (all): $b(all)_{1_add}$ to $b(all)_{v_add}$" to the teacher data TH2, and updates the teacher data TH2 to teacher data TH2_up1.

Furthermore, the taste diagnostic unit 217 adds "sweetness $d_{1_add}$ to $d_{v_add}$" and "H(+)_sum: $h(+)_{1_sum_add}$ to $h(+)_{v_sum_add}$" to the teacher data TH3, and updates the teacher data TH3 to teacher data TH3_up1.

Furthermore, the taste diagnostic unit 217 adds "aroma $e_{1_add}$ to $e_{v_add}$" and "H(-)_sum: $h(-)_{1_sum_add}$ to $h(-)_{v_sum_add}$" to the teacher data TH4, and updates the teacher data TH4 to teacher data TH4_up1.

Furthermore, the taste diagnostic unit 217 adds "bitterness $f_{1_add}$ to $f_{v_add}$", "astringency $a_{1_add}$ to $a_{v_add}$", and "sweetness: $d_{1_add}$ to $d_{v_add}$" to the teacher data TH5, and updates the teacher data TH5 to teacher data TH5_up1.

Then, the taste diagnostic unit 217 determines the value of coefficient $k_1$ based on the updated teacher data TH1_up1 using the method described above in "(A) Determination of coefficient $k_1$", and updates the value of coefficient $k_1$ to the determined value.

In addition, the taste diagnostic unit 217 determines the value of coefficient $k_2$ based on the updated teacher data TH2_up1 using the method described above in "(B) Determination of coefficient $k_2$", and updates the value of coefficient $k_2$ to the determined value.

Furthermore, the taste diagnostic unit 217 determines the values of coefficients $k_3$ and $k_4$ based on the updated teacher data TH3_up1 using the method described above in "(C) Determination of coefficients $k_3$ and $k_4$", and updates the values of coefficients $k_3$ and $k_4$ to the determined values.

Furthermore, the taste diagnostic unit 217 determines the values of coefficients $k_5$ and $k_6$ based on the updated teacher data TH4_up1 using the method described above in "(D) Determination of coefficients $k_5$ and $k_6$," and updates the values of coefficients $k_5$ and $k_6$ to the determined values.

Furthermore, the taste diagnostic unit 217 determines the values of coefficients $k_7$ and $k_8$ based on the updated teacher data TH5_up1 using the method described above in "(E) Determination of coefficients $k_7$ and $k_8$", and updates the values of coefficients $k_7$ and $k_8$ to the determined values.

Then, after updating the values of the coefficients $k_1$ to $k_8$, the taste diagnostic unit 217 stores the updated teacher data TH1_up1 to TH5_up1 in the database 22.

Fig. 17 is a conceptual diagram of a correspondence table TBL1_up1 obtained by updating the correspondence table TBL1 shown in Fig. 15.

Referring to Fig. 17, the correspondence table TBL1_up1 includes coefficients $k_{1_up1}$ to $k_{8_up1}$ and teacher data TH1_up1 to TH5_up1. The coefficients $k_{1_up1}$ to $k_{8_up1}$ are updated coefficients, and the teacher data TH1_up1 to TH5_up1 are updated teacher data.

The teacher data TH1_up1 is associated with the coefficient $k_{1_up1}$, the teacher data TH2_up1 is associated with the coefficient $k_{2_up1}$, the teacher data TH3_up1 is associated with the coefficients $k_{3_up1}$ and $k_{4_up1}$, the teacher data TH4_up1 is associated with the coefficients $k_{5_up1}$ and $k_{6_up1}$, and the teacher data TH5_up1 is associated with the coefficients $k_{7_up1}$ and $k_{8_up1.}$

When the taste diagnostic unit 217 updates the values of the coefficients $k_1$ to $k_8$ using the method described above, the taste diagnostic unit 217 updates the correspondence table TBL1 to the correspondence table TBL1_up1 based on the updated values of the coefficients $k_1$ to $k_8$, and stores the updated correspondence table TBL1_up1 in the database 22.

Then, the taste diagnostic unit 217 uses the coefficients $k_{1_up1}$ to $k_{8_up1}$ stored in the correspondence table TBL1_up1 to diagnose the "astringency," "aftertaste," "sweetness," "aroma," and "bitterness" of one or more new shochu using the method described above.

Thereafter, the taste diagnostic unit 217 repeatedly updates the teacher data TH1 to TH5, updates the values of the coefficients $k_1$ to $k_8$ using the updated teacher data TH1 to TH5, and repeatedly diagnoses the "astringency," "aftertaste," "sweetness," "aroma," and "bitterness" of one or more new shochu samples.

(II) Taste diagnosis of Grape

When the calculation unit 216 has received calculation data CAL (=calculation data having the same configuration as the calculation data $CAL_{uni}$ shown in Fig. 12) from the **control** unit 212, the calculation unit 216 detects the integral value $ITG_{_Low}$ ($ITG_{1_Low}$ to $ITG_{n_Low}$), the integral value $ITG_{_Middle}$ ($ITG_{1_Middle}$ to $ITG_{n_Middle}$), and the integral value $ITG_{_High}$ ($ITG_{1_High}$ to $ITG_{n_High}$) from the calculation data CAL.

Then, the calculation unit 216 detects a plurality of integral values $ITG_{U_Low_th}$ to $ITG_{n_Low_th}$ corresponding to a predetermined potential section (=class $Cls_{th}$) including a potential V equal to or lower than the threshold value $V_{th}$, among the predetermined negative potential sections, based on the classes $C1s_1$ to $C1s_n$ and the integral value $ITG_{_Low}$ ($ITG_{1_Low}$ to $ITG_{n_Low}$). U indicates the class $Cls_{th}$ corresponding to the predetermined potential section including the potential V corresponding to the threshold value $V_{th}$.

Furthermore, the calculation unit 216 detects a plurality of integral values $ITG_{U_Middle\ _th}$ to $ITG_{n_Middle}$ corresponding to a predetermined potential section (=class $Cls_{th}$) equal to or lower than the threshold value $V_{th}$, among the predetermined negative potential sections, based on the classes $C1s_1$ to $C1s_n$ and the integral value $ITG_{\ Middle}$ ($ITG_{1_Middle}$ to $ITG_{n_Middle}$).

Furthermore, the calculation unit 216 detects a plurality of integral values $ITG_{U_High_th}$ to $ITG_{n_High_th}$ corresponding to a predetermined potential section (=class Cls) equal to or lower than the threshold value $V_{th}$, out of a

predetermined negative potential section, based on the classes $C1s_1$ to $C1s_n$ and the integral value $ITG_{_High}$ ($ITG_{1_High}$ to $ITG_{n_High}$).

The subscript "U" in the multiple integral values $ITG_{U_Low_th}$ to $ITG_{n_Low_th}$, $ITG_{U_Middle_th}$ to $ITG_{n_Middle\ th}$, and $ITG_{U_High_th}$ to $ITG_{n_High_th}$ represents a predetermined potential section including the potential V corresponding to the threshold value $V_{th}$, so each of the integral values $ITG_{U_Low_th}$, $ITG_{U_Middle_th}$, and $ITG_{U_High_th}$ is an integral value in a predetermined potential section including the potential V corresponding to the threshold value $V_{th}$.

When the calculation unit 216 has detected multiple integrated values $ITG_{U_Low_th}$ to $ITG_{n_Low_th}$, $ITG_{U_Middle_th}$ to $ITG_{n_Middle_th}$, and $ITG_{U_High_th}$ to $ITG_{n_High_th}$, the calculation unit 216 calculates a sum L(-)_sum_th of the multiple integrated values $ITG_{U_Low_th}$ to $ITG_{n_Low_th}$, calculates a sum M(-)_sum_th of the multiple integrated values $ITG_{U_Middle_th}$ to $ITG_{n_Middle_}$th, and calculates a sum H(-)_sum_th of the multiple integrated values $ITG_{U_High_th}$ to $ITG_{n_High_th}$.

Then, the calculation unit 216 substitutes the sums L(-)_sum_th, M(-)_sum_th, and H(-)_sum_th of the integral values into the following equation to calculate the Body index (-)_th.

[Equation 10]

$$Body\ index(-)_th = \frac{M(-)_sum_th}{H(-)_sum_th} + \frac{L(-)_sum_th}{H(-)_sum_th} \cdots (10)$$

After calculating Body index (-)_th, the calculation unit 216 outputs the calculated Body index (-)_th to the taste diagnostic unit 217.

When the taste diagnostic unit 217 has received Body index (-)_th from calculation unit 216, the taste diagnostic unit 217 substitutes Body index (-)_th and coefficient $k_9$ into the following equation to calculate value ASTG_GRP of "astringency" of the grape, and diagnoses the calculated value ASTG_GRP as the "astringency" of the grape.

[Equation 11]

$$ASTG_GRP = Body\ index(-)_th \times k_9 \cdots (11)$$

In equation 11, the coefficient $k_9$, for example, is 1.5.

Then, the taste diagnostic unit 217 determines the value of the coefficient $k_9$ by the same manner as the above-mentioned method for determining the value of the coefficient $k_1$.

Fig. 18 is a conceptual diagram for diagnosing the taste (astringency, aftertaste, sweetness, aroma, bitterness) of shochu. Referring to Fig. 18, the taste diagnostic unit 217 uses multiple shochu with known "astringency," "aftertaste," "sweetness," "aroma," and "bitterness" as teacher data TH1 to TH5 to determine the values $vle_{1_Q}$ to $vle_{8_Q}$ of the coefficients $k_1$ to $k_8$ by the above-mentioned method (block BLK1).

Then, the taste diagnostic unit 217 uses the values $vle_{1_Q}$ to $vle_{8_Q}$ (Q = 0, 1, 2, ...) of the coefficients $k_1$ to $k_8$ to diagnose the v "astringency," v "aftertaste," v "sweetness," v "aroma," and v "bitterness" of the v shochu to be diagnosed by the method described above (block BLK2).

Thereafter, the taste diagnostic unit 217 sets Q=Q+1 (block BLK3), and updates the teacher data TH1 -TH5 to teacher data TH1_up_Q to TH5_up_Q, respectively, based on the diagnosis results of the v Shochus (block BLK4).

Next, the taste diagnostic unit 217 uses the teacher data TH1_up_Q to TH5_up_Q to determine the values $vle_{1_Q}$ to $vle_{8_Q}$ of the coefficients $k_1$ to $k_8$ by the method described above, and updates the values $vle_{1_Q-1}$ to $vle_{8_Q-1}$ of the coefficients $k_1$ to $k_8$ to the values $vle_{1_Q}$ to $vle_{8_Q}$, respectively (block BLK5).

After block BLK5, the taste diagnostic unit 217 repeatedly executes blocks BLK2 to BLK5.

When moving from block BLK1 to block BLK2, since the values $vle_{1_Q}$ to $vle_{8_Q}$ of coefficients $k_1$ to $k_8$ are set to values $vle_{1\text{-}0}$ to $vle_{8_0}$, respectively, the taste diagnostic unit 217 uses, in block BLK2, the values $vle_{1_0}$ to $vle_{8_0}$ of coefficients $k_1$ to $k_8$ to diagnose the v "astringency," v "aftertaste," v "sweetness," v "aroma," and v "bitterness" of the v shochu to be diagnosed. In other words, the taste diagnostic unit 217 diagnoses v "astringency," v "aftertaste," v "sweetness," v "aroma," and v "bitterness" of the v shochu to be diagnosed using the values $vle_{1_0}$ to $vle_{8_0}$ of the coefficients $k_1$ to $k_8$ determined using multiple shochu whose "astringency," "aftertaste," "sweetness," "aroma," and "bitterness" are known as teacher data TH1 to TH5.

After block BLK2, the taste diagnostic unit 217 sets Q=Q+1 (block BLK3) and then executes block BLK4.

In this case, since Q=Q+1=0+1=1 is set in block BLK3, the taste diagnostic unit 217 updates the teacher data TH1 to TH5 to teacher data TH1_up_1 to TH5_up_1, respectively, in block BLK4.

Then, in block BLK5, the taste diagnostic unit 217 determines the values $vle_{1_1}$ to $vle_{8_1}$ of the coefficients $k_1$ to $k_8$ using the updated teacher data TH1_up_1 to TH5_up_1 by the method described above, and updates the values $vle_{1_0}$ to $vle_{8_0}$ of the coefficients $k_1$ to $k_8$ to the values $vle_{1_1}$ to $vle_{8_1}$, respectively.

After block BLK5, the taste diagnostic unit 217 uses the values $vle_{1_1}$ to $vle_{8_1}$ of the coefficients $k_1$ to $k_8$ to

diagnose the v "astringency," v "aftertaste," v "sweetness," v "aroma," and v "bitterness" of the v shochu to be diagnosed by the method described above (block BLK2). In other words, in block BLK2, the taste diagnostic unit 217 uses the values $vle_{1_1}$ to $vle_{8_1}$ of the coefficients $k_1$ to $k_8$ determined using the updated teacher data TH1_up_1 to TH5_up_1 to diagnose the v "astringency," v "aftertaste," v "sweetness," v "aroma," and v "bitterness" of the v shochu to be diagnosed.

After block BLK2, the taste diagnostic unit 217 sets Q=Q+1 (block BLK3) and then executes block BLK4.

In this case, since Q=Q+1=1+1=2 is set in block BLK3, the taste diagnostic unit 217 updates the teacher data TH1_up_1 to TH5_up_1 to teacher data TH1_up_2 to TH5_up_2, respectively, in block BLK4.

Then, in block BLK5, the taste diagnostic unit 217 determines the values $vle_{1_2}$ to $vle_{8_2}$ of the coefficients $k_1$ to $k_8$ using the updated teacher data TH1_up_2 to TH5_up_2 by the method described above, and updates the values $vle_{1_1}$ to $vle_{8_1}$ of the coefficients $k_1$ to $k_8$ to the values $vle_{1_2}$ to $vle_{8_2}$, respectively.

After block BLK5, the taste diagnostic unit 217 uses the values $vle_{1_2}$ to $vle_{8_2}$ of the coefficients $k_1$ to $k_8$ to diagnose the v "astringency," v "aftertaste," v "sweetness," v "aroma," and v "bitterness" of the v shochu to be diagnosed by the method described above (block BLK2). In other words, in block BLK2, the taste diagnostic unit 217 uses the values $vle_{1_2}$ to $vle_{8_2}$ of the coefficients $k_1$ to $k_8$ determined using the updated teacher data TH1_up_2 to TH5_up_2 to diagnose the v "astringency," v "aftertaste," v "sweetness," v "aroma," and v "bitterness" of the v shochu to be diagnosed.

After block BLK2, the taste diagnostic unit 217 sets Q=Q+1 (block BLK3) and then executes block BLK4.

In this case, since Q=Q+1=2+1=3 is set in block BLK3, the taste diagnostic unit 217 updates the teacher data TH1_up - 2 to TH5_up_2 to teacher data TH1_up_3 to TH5_up_3, respectively, in block BLK4.

Thereafter, the taste diagnostic unit 217 repeatedly executes blocks BLK2 to BLK5.

Each time blocks BLK2 to BLK5 are one time executed, v pieces of "astringency" and v pieces of "Body index (+)" are added to teacher data TH1, v pieces of "aftertaste" and v pieces of "Body index (all)" are added to teacher data TH2, v pieces of "sweetness" and v pieces of "H(+)_sum" are added to teacher data TH3, v pieces of "aroma" and v pieces of "H(-)_sum" are added to teacher data TH4, and v pieces of "bitterness", v pieces of "astringency", and v pieces of "sweetness" are added to teacher data TH5.

Therefore, as the number of times blocks BLK2 to BLK5 are repeatedly executed increases, it is possible to set to higher value the accuracy of the values $vle_1$ to $vle_8$ of the coefficients $k_1$ to $k_8$ determined by regression analysis in block BLK5.

In addition, in Fig. 18, when blocks BLK2 to BLK5 are executed for the gth time (g is an integer equal to or greater than 1), v may be set to $v_Q$ ($v_Q$ is an integer equal to or greater than 1) to sequentially execute blocks BLK2 to BLK5, and when blocks BLK2 to BLK5 are executed the (g+1)th time, v may be set to $v_R$ ($v_R$ is an integer equal to or greater than 1 and different from $v_Q$) to sequentially execute blocks BLK2 to BLK5. In other words, the number ($v=v_R$) of "shochu" diagnosed in the (g+1)th time may be different from the number of "shochu" diagnosed in the gth time ($v=v_Q$).

Also, when the taste diagnostic unit 217 diagnoses the "astringency" of grapes, the taste diagnostic unit 217 executes blocks BLK1 to BLK5 shown in Fig. 18 to diagnose the "astringency" of grapes while updating the teacher data and the value of the coefficient $k_9$.

In this case, the number ($v=v_R$) of "grapes" diagnosed in the (g+1)th time may be different from the number ($v=v_Q$) of "grapes" diagnosed in the gth time.

(Example)

The curve CUR generated by the diagnostic device 2 will be described for the case where the analyte is shochu and grapes.

(1) Shochu

The shochu used to create the curve CUR are shown in Table 2.

[Table 2]

| Name | Alcohol content(%) | Alcoholic beverages |
|---|---|---|
| Sample No.1 | 25% | Wheat |
| Sample No.2 | 25% | Wheat |
| Sample No.3 | 25% | Wheat |
| Sample No.4 | 25% | Wheat |
| Sample No.5 | 25% | Wheat |
| Sample No.6 | 25% | Wheat |

(continued)

| Name | Alcohol content(%) | Alcoholic beverages |
|---|---|---|
| Sample No.7 | 25% | Potato |
| Sample No.8 | 25% | Potato |
| Sample No.9 | 25% | Potato |
| Sample No.10 | 25% | Potato |
| Sample No.11 | 25% | Potato |
| Sample No.12 | 25% | Potato |
| Sample No.13 | 25% | Potato |
| Sample No.14 | 25% | Potato |
| Sample No.15 | 25% | Rice |
| Sample No.16 | 25% | Rice |
| Sample No.17 | 25% | Sake lees |
| Sample No.18 | 40% | Barrel-stored wheat |
| Sample No.19 | 36% | Barrel-stored wheat |
| Sample No.20 | 40% | Barrel-stored wheat |

The measurement conditions for the cyclic voltammograms CVG for the shochu of samples No. 1 to No. 20 shown in Table 2 are shown in Table 3.

[Table 3]

| | |
|---|---|
| Working electrode | Diamond electrode (diameter: 3mm) |
| Counter electrode | Gold(rod-shaped electrode) |
| Reference electrode | Gold(rod-shaped electrode) |
| Potential scanning range | ±2.5V |
| Integral value extraction potential | 18.1mV |
| Potential scanning speeds | 300mV/s, 500mV/s, 600mV/s |

As shown in Table 3, the working electrode was made of diamond having a circular planar shape, and the counter electrode and reference electrode were made of rod-shaped gold. The potential scanning range in when measuring the cyclic voltammogram is -2.5 V to +2.5 V, the predetermined potential section (integral value extraction potential) in when calculating the integral value is 18.1 mV, and the potential scanning speeds are 300 mV/s, 500 mV/s, and 600 mV/s.
In terms of the potential scanning speed, 300 mV/s corresponds to a potential scanning speed $V_{r_Low}$, 500 mV/s corresponds to a potential scanning speed $V_{r_Middle}$, and 600 mV/s corresponds to a potential scanning speed $V_{r_High}$.
Fig. 19 is a diagram showing the integral value spectra for the shochu of samples No. 1 to No. 3 shown in Table 2.
Referring to Fig. 19, each of the curves k1 to k3 is a curve CUR (index curve) generated by the creation unit 215 of the diagnostic device 2 using the method described above. Curve k1 shows the integral value spectrum of the barley shochu of sample No. 1, curve k2 shows the integral value spectrum of the barley shochu of sample No. 2, and curve k3 shows the integral value spectrum of the barley shochu of sample No. 3.
Fig. 20 is a diagram showing the integral value spectra for the shochu of samples No. 4 to No. 6 shown in Table 2.
Referring to Fig. 20, each of the curves k4 to k6 is a curve CUR (index curve) created by the creation unit 215 of the diagnostic device 2 using the method described above. Curve k4 shows the integral value spectrum of the barley shochu of sample No. 4, curve k5 shows the integral value spectrum of the barley shochu of sample No. 5, and curve k6 shows the integral value spectrum of the barley shochu of sample No. 6.
Fig. 21 is a diagram showing the integral value spectra for the shochu samples No. 7 to No. 9 shown in Table 2.
Referring to Fig. 21, each of the curves k7 to k9 is a curve CUR (index curve) created by the creation unit 215 of the diagnostic device 2 using the method described above. Curve k7 shows the integral value spectrum of the sweet potato shochu of sample No. 7, curve k8 shows the integral value spectrum of the sweet potato shochu of sample No. 8, and curve k9 shows the integral value spectrum of the sweet potato shochu of sample No. 9.

Fig. 22 is a diagram showing integral value spectra for the shochu of samples No. 10 to No. 12 shown in Table 2.

Referring to Fig. 22, each of the curves k10 to k12 is a curve CUR (index curve) created by the creation unit 215 of the diagnostic device 2 using the method described above. A curve k10 indicates the integral value spectrum of the sweet potato shochu of sample No. 10, a curve k11 indicates the integral value spectrum of the sweet potato shochu of sample No. 11, and a curve k12 indicates the integral value spectrum of the sweet potato shochu of sample No. 12.

Fig. 23 is a diagram showing integral value spectra for the shochu of samples No. 12 to No. 14 shown in Table 2.

Referring to Fig. 23, each of the curves k12 to k14 is a curve CUR (index curve) created by the creation unit 215 of the diagnostic device 2 using the method described above. Curve k12 shows the integral value spectrum of the sweet potato shochu of sample No. 12, curve k13 shows the integral value spectrum of the sweet potato shochu of sample No. 13, and curve k14 shows the integral value spectrum of the sweet potato shochu of sample No. 14.

Fig. 24 is a diagram showing integral value spectra for the shochu of samples No. 15 to No. 16 shown in Table 2.

Referring to Fig. 24, each of the curves k15 and k16 is a curve CUR (index curve) created by the creation unit 215 of the diagnostic device 2 using the above-mentioned method. The curve k15 shows the integral value spectrum of the rice shochu of sample No. 15, and the curve k16 shows the integral value spectrum of the rice shochu sample No. 16.

Fig. 25 is a diagram showing integral value spectra for the shochu of samples No. 17 to No. 20 shown in Table 2.

Referring to Fig. 25, each of the curves k17 to k20 is a curve CUR (index curve) created by the creation unit 215 of the diagnostic device 2 using the method described above. Curve k17 shows the integral value spectrum of shochu made with sake lees of sample No. 17. Curve k18 shows the integral value spectrum of barley shochu of sample No. 18, all of which was stored for 3 years, all of which was stored in barrels, and some of which was stored in cherry wood barrels. Curve k19 shows the integral value spectrum of sample No. 19, all of which was stored for 15 years, all of which was made from koji barley, all of which was stored in barrels, and all of which was heated. Curve k20 shows rice shochu of sample No. 20, all of which was aged for three years and all of which was stored in barrels.

Figs. 26 to 28 are first to third diagrams respectively showing the results of judging whether or not the curves k1 to k20 shown in Figs. 19 to 25 are different from one another.

Fig. 26 shows the results of judging whether or not ten curves k1 to k10 are different from one another. Fig. 27 shows the results of judging whether or not ten curves k1 to k10 and ten curves k11 to k20 are different from each other. Fig. 28 shows the results of judging whether or not ten curves k11 to k20 are different from one another.

The judging of whether the 20 curves $k_1$ to k20 are or not different from each other is performed by judging whether the two curves are or not different for all combinations (${}_{20}C_2$ = 190) in when selecting two different curves from the 20 curves $k_1$ to k20.

Then, it is executed by the following method to judge whether two different curves $CUR_i$, $CUR_j$ ($i \neq j$) are or not different. A difference $DF_k$ between an integral value $ITG_{k_i}$ and an integral value $ITG_{k_j}$ in one class $Cls_k$ is calculated using equation (1) based on a plurality of integral values $ITG_{1_i}$ to $ITG_{n_i}$ in the curve $CUR_i$ and a plurality of integral values $ITG_{1_j}$ to $ITG_{n_j}$ in the curve $CUR_j$, and this is performed for all classes $C1s_1$ to $C1s_n$ to calculate n differences $DF_1$ to $DF_n$, and a judging whether two different curves $CUR_i$, $CUR_j$ ($i \neq j$) are different is performed by judging is made as to whether the standard deviation $\sigma_{DFk}$ of the calculated n differences $DF_1$ to $DF_n$ is or not greater than a threshold value $\sigma_{th}$ (=6%).

Referring to Fig. 26, when two different curves are selected from 10 curves k1 to k10, all of the 45 standard deviations $\sigma_{DFk1,\ k2}$ to $\sigma_{DFk9,k10}$ of the differences for all 45 combinations of two different curves are greater than the threshold value $\sigma_{th}$ (= 6%).

Referring to Fig. 27, when two different curves are selected from 10 curves $k_1$ to k10 and 10 curves k11 to k20, all of the 100 standard deviations $\sigma_{DFk1,\ k11}$ to $\sigma_{DFk10,\ k20}$ of the differences for all 100 combinations of two different curves are greater than the threshold value σth (= 6%).

Referring to Fig. 28, when two different curves are selected from 10 curves k11 to k20, all of the 45 standard deviations $\sigma_{DFk11,\ k12}$ to $\sigma_{DFk19,\ k20}$ of the differences for all 45 combinations of two different curves are greater than the threshold value $\sigma_{th}$ (= 6%).

Therefore, when two different curves are selected from the 20 curves k1 to k20, all of the 190 standard deviations $\sigma_{DFk1,\ k2}$ to $\sigma_{DFk19,\ k20}$of the differences for all of 190 combinations of two different curves are greater than the threshold value $\sigma_{th}$ (=6%).

Therefore, the 20 curves k1 to k20 are mutually different curves. When it is judged that the 20 curves k1 to k20 are mutually different, the curves k1 to k20 are curves for uniquely identifying Shochu No. 1 to Shochu No. 20, respectively. The curves k1 to k20 are also fingerprints that represent feature amounts based on integral values for Shochu No. 1 to Shochu No. 20, respectively.

Fig. 29 is a diagram showing the results of diagnosis of "astringency," "aftertaste," "sweetness," "aroma," and "bitterness" for Shochu No. 1 to No. 20 shown in Table 2.

The values of "astringency", "aftertaste", "sweetness", "aroma" and "bitterness" for Shochu No. 1 to No. 20 shown in Fig. 29 are the diagnosis results obtained by the taste diagnostic unit 217 using coefficients $k_1$=1.5, $k_2$=2, $k_3$=3000, $k_4$=1.5, $k_5$=2000, $k_6$=1.5, $k_7$=1.5 and $k_8$=0.4.

Fig. 30 is a diagram showing other diagnosis results of the "astringency", "aftertaste", "sweetness", "aroma" and "bitterness" of Shochu No. 1 to No. 20 shown in Table 2.

The values of "astringency", "aftertaste", "sweetness", "aroma" and "bitterness" for Shochu No. 1 to No. 20 shown in Fig. 30 are the average values of "astringency", "aftertaste", "sweetness", "aroma" and "bitterness" judged by seven people.

Fig. 31 is a diagram showing the differences between the respective values of the diagnosis results for Shochu No. 1 to No. 20 shown in Fig. 29 and the respective values of the diagnosis results for Shochu No. 1 to No. 20 shown in Fig. 30.

The differences between "astringency", "aftertaste", "sweetness", "aroma" and "bitterness" shown in Fig. 31 consist of the subtraction results obtained by subtracting each of values of "astringency", "aftertaste", "sweetness", "aroma" and "bitterness" shown in Fig. 30 from each of values of "astringency", "aftertaste", "sweetness", "aroma" and "bitterness" shown in Fig. 29.

Therefore, in Fig. 31, when the difference is a positive value, it indicates that each value of "astringency", "aftertaste", "sweetness", "aroma" and "bitterness" diagnosed by the taste diagnostic unit 217 is greater than each value of "astringency", "aftertaste", "sweetness", "aroma" and "bitterness" diagnosed by a human and when the difference is a negative value, it indicates that the values of "astringency", "aftertaste", "sweetness", "aroma" and "bitterness" diagnosed by the taste diagnostic unit 217 are smaller than the values of "astringency", "aftertaste", "sweetness", "aroma" and "bitterness" diagnosed by a human..

Referring to Fig. 31, for Shochu of No. 1 to No. 20, the average value of the difference in "astringency" is 0.183, the average value of the difference in "aftertaste" is 0.192, the average value of the difference in "sweetness" is 0.134, the average value of the difference in "aroma" is 0.047, and the average value of the difference in "bitterness" is -0.165.

In addition, for Shochu of No. 1 to No. 20, the standard deviation of the difference in "astringency" is 0.271, the standard deviation of the difference in "aftertaste" is 0.408, the standard deviation of the difference in "sweetness" is 0.646, the standard deviation of the difference in "aroma" is 0.393, and the standard deviation of the difference in "bitterness" is 0.419.

As a result, the average deviation of the "astringency", "aftertaste", "sweetness", "aroma" and "bitterness" diagnosed by the taste diagnostic unit 217 from the "astringency", "aftertaste", "sweetness", "aroma" and "bitterness" diagnosed by humans was 19.2% or less, and the variation from the average deviation was 0.646 or less.

Therefore, it was found that the taste diagnostic unit 217 can diagnose the "astringency", "aftertaste", "sweetness", "aroma" and "bitterness" of shochu in the same manner as a human being, by using the above-mentioned method.

(2) Grape

The grapes used to create the curve CUR were Crimson Seedless (skin + body), Green Seedless (skin + body), Shine Muscat (skin + body), Crimson Seedless (body), Green Seedless (body) and Shine Muscat (body).

Each of Crimson Seedless (skin + flesh), Green Seedless (skin + flesh) and Shine Muscat (skin + flesh) are the juice of grapes that have been pressed together with the skin and flesh, while each of Crimson Seedless (flesh), Green Seedless (flesh) and Shine Muscat (flesh) are the juice of grapes that have been pressed only with the flesh.

In addition, the measurement conditions of the cyclic voltammograms CVG for Crimson Seedless (skin + flesh), Green Seedless (skin + flesh), Shine Muscat (skin + flesh), Crimson Seedless (flesh), Green Seedless (flesh) and Shine Muscat (flesh) are the same as the measurement conditions shown in Table 3.

Fig. 32 is a diagram showing integral value spectra about Crimson Seedless (skin + flesh), Green Seedless (skin + flesh) and Shine Muscat (skin + flesh).

Referring to Fig. 32, each of the curves k21 to k23 is a curve CUR (index curve) which the diagnostic device 2 created by the above-mentioned method. The curve k21 shows the integral value spectrum of Crimson Seedless (skin + flesh), the curve k22 shows the integral value spectrum of Green Seedless (skin + flesh), and the curve k23 shows the integral value spectrum of Shine Muscat (skin + flesh).

Fig. 33 is a diagram showing integral value spectra for Crimson Seedless (flesh only), Green Seedless (flesh only), and Shine Muscat (flesh only).

Referring to Fig. 33, each of the curves k24 to k26 is a curve CUR (index curve) which the diagnostic device 2 created by the above-mentioned method. The curve k24 shows the integral value spectrum of Crimson Seedless (flesh only), the curve k25 shows the integral value spectrum of Green Seedless (flesh only), and the curve k26 shows the integral value spectrum of Shine Muscat (flesh only).

Fig. 34 is a diagram showing the result of judgment as to whether or not the curves k21 to k26 shown in Fig. 32 and Fig. 33 are different from each other.

Whether or not the curves k21 to k26 are different from one another is judged by executing a judgment as to whether or not any two different curves among the curves k21 to k26 are different for all combinations of two different curves among the curves k21 to k26.

There are 15 combinations of two different curves among the six curves k21 to k26: (k21, k22), (k21, k23), (k21, k24), (k21, k25), (k21, k26), (k22, k23), (k22, k24), (k22, k25), (k22, k26), (k23, k24), (k23, k25), (k23, k26), (k24, k25), (k24, k26), and (k25, k26).

Referring to Fig. 34, the standard deviation $\sigma_{DF_k21, k22}$ of the difference between the multiple integral values on the curve k21 and the multiple integral values on the curve k22 is $\sigma_{DF_k21, k22}$=58.9%, the standard deviation $\sigma_{DF_k21,k23}$ of the difference between the multiple integral values on the curve k21 and the multiple integral values on the curve k23 is $\sigma_{DF-k21, k23}$=31.2%, the standard deviation $\sigma_{DF_k21, k24}$ of the difference between the multiple integral values on the curve k21 and the multiple integral values on the curve k24 is $\sigma_{DF_k21, k24}$=62.0%, the standard deviation $\sigma_{DFk_21,k25}$ of the difference between the multiple integral values on the curve k21 and the multiple integral values on the curve k25 is $\sigma_{DF_k21, k25}$=24.1 %, and the standard deviation $\sigma_{DF_k21, k26}$ of the difference between the multiple integral values on the curve k21 and the multiple integral values on the curve k26 is $\sigma_{DF_k21, k25}$=62.5%.

Furthermore, the standard deviation $\sigma_{DF_k22, k23}$ of the difference between the multiple integral values on the curve k22 and the multiple integral values on the curve k23 is $\sigma_{DF_k22, k23}$=65.3%, the standard deviation $\sigma_{DF_k22, k24}$ of the difference between the multiple integral values on the curve k22 and the multiple integral values on the curve k24 is $\sigma_{DF_k22, k24}$=16.0%, the standard deviation $\sigma_{DF_k22, k25}$ of the difference between the multiple integral values on the curve k22 and the multiple integral values on the curve k25 is $\sigma_{DF_k22, k25}$=16.0%, and the standard deviation $\sigma_{DF_k22, k26}$ of the difference between the multiple integral values on the curve k22 and the multiple integral values on the curve k26 is $\sigma_{DF_k22, k26}$=23.0%.

Furthermore, the standard deviation $\sigma_{DF_k23, k24}$ of the difference between the multiple integral values on the curve k23 and the multiple integral values on the curve k24 is $\sigma_{DF_k23, k24}$=30.1%, the standard deviation $\sigma_{DF_k23, k25}$ of the difference between the multiple integral values on the curve k23 and the multiple integral values on the curve k25 is $\sigma_{DF_k23, k25}$=31.4%, and the standard deviation $\sigma_{DF_k23, k26}$ of the difference between the multiple integral values on the curve k23 and the multiple integral values on the curve k26 is $\sigma_{DF_k23, k26}$=24.9%.

Furthermore, the standard deviation $\sigma_{DF_k24, k25}$ of the difference between the multiple integral values on the curve k24 and the multiple integral values on the curve k25 is $\sigma_{DF_k24, k25}$=30.3%, and the standard deviation $\sigma_{DF_k24, k26}$ of the difference between the multiple integral values on the curve k24 and the multiple integral values on the curve k26 is $\sigma_{DF_k24, k26}$=22.9%.

Furthermore, the standard deviation $\sigma_{DF_k25, k26}$ of the difference between the multiple integral values on the curve k25 and the multiple integral values on the curve k26 is $\sigma_{DF_k25, k26}$=24.3%.

As a result, the standard deviation σDF_k21, k22 (= 58.9%) of the differences, the standard deviation σDF_k21, k23 (= 31.2%) of the differences, the standard deviation σDF_k21, k24 (= 62.0%) of the differences, the standard deviation σDF_k21, k25 (= 24.1%) of the differences, the standard deviation σDF_k21, k26 (= 62.5%) of the differences, the standard deviation σDF_k22, k23 (= 65.3%) of the differences, the standard deviation σDF_k22, k24 (= 16.0%) of the differences, the standard deviation σDF_k22, k25 (= 16.0%) of the differences, the standard deviation σDF_k22,k26 (=23.0%) of differences, the standard deviation σDF_k23,k24 (=30.1%) of differences, the standard deviation σDF_k23,k25 (=31.4%) of differences, the standard deviation σDF_k23,k26 (=24.9%) of differences, the standard deviation σDF_k24,k25 (=30.3%) of differences, the standard deviation σDF_k24,k26 (=22.9%) of differences, and the standard deviation σDF_k25,k26 (=24.3%) of differences are all greater than the threshold value σth (=15%).

Therefore, the two curves k21, k22 are different, the two curves k21, k23 are different, the two curves k21, k24 are different, the two curves k21, k25 are different, the two curves k21, k26 are different, the two curves k22, k23 are different, the two curves k22, k24 are different, the two curves k22, k25 are different, the two curves k22, k26 are different, the two curves k23, k24 are different, the two curves k23, k25 are different, the two curves k23, k26 are different, the two curves k24, k25 are different, the two curves k24, k26 are different, and the two curves k25, k26 are different. Therefore, the curves k21 to k26 are mutually different curves.

When it is judged that the six curves k21 to k26 are different from each other, the curves k21 to k26 are curves for uniquely identifying Crimson Seedless (flesh + skin), Green Seedless (flesh + skin), Shine Muscat (flesh + skin), Crimson Seedless (flesh only), Green Seedless (flesh only) and Shine Muscat (flesh only), respectively. And, the curves k21 to k26 are fingerprints representing the feature amounts by integral values for Crimson Seedless (flesh + skin), Green Seedless (flesh + skin), Shine Muscat (flesh + skin), Crimson Seedless (flesh only), Green Seedless (flesh only) and Shine Muscat (flesh only), respectively.

Fig. 35 is a diagram showing the diagnosis results of "astringency" for Green Seedless (flesh only), Crimson Seedless (flesh only), Shine Muscat (flesh only), Green Seedless (flesh + skin), Crimson Seedless (flesh + skin), and Shine Muscat (flesh + skin).

Note that the "Astringency" shown in Fig. 35 is calculated using L(-)_sum_th, M(-)_sum_th, and H(-)_sum_th calculated based on the integral value in a specified potential section (=class) in the range from -1362 mV to -2501 mV, with the threshold value $V_{th}$ of the above-mentioned potential V being set to "-1362 mV."

Referring to Fig. 35, the astringency of green seedless (flesh only) is 2.27, and the astringency of green seedless (flesh + skin) is 2.58.

Additionally, the astringency of Crimson Seedless (flesh only) is 2.96, ang the astringency of Crimson Seedless (flesh + skin) is 4.65.

Furthermore, the astringency of Shine Muscat (flesh only) is 2.06, and the astringency of Shine Muscat (flesh + skin) is 2.47.

As a result, the "astringency" value increases in the order of Shine Muscat (flesh only), Green Seedless (flesh only), Shine Muscat (flesh + skin), Green Seedless (flesh + skin), Crimson Seedless (flesh only), and Crimson Seedless (flesh + skin).

In addition, the astringency of Green Seedless (flesh only) is less than that of Green Seedless (flesh + skin), the astringency of Crimson Seedless (flesh only) is less than that of Crimson Seedless (flesh + skin), and the astringency of Shine Muscat (flesh only) is less than that of Shine Muscat (flesh + skin).

Therefore, "the astringency" of Green Seedless (flesh only), Crimson Seedless (flesh only) and Shine Muscat (flesh only), which are juices made by squeezing grape flesh, is less than the astringency of Green Seedless (flesh + skin), Crimson Seedless (flesh + skin) and Shine Muscat (flesh + skin), which are juices made by squeezing grape flesh and skin.

Therefore, by diagnosing the astringency of grapes using the taste diagnostic unit 217, it was found that the grape skin is a factor that increases "the astringency".

[Relationship between taste diagnosis and number of integral values for shochu]

(I) Number of integral values: 138

Fig. 36 is a diagram showing integral value spectra for the shochu with samples No. 1 to No. 3 shown in Table 2, in the case where the number of integral values is 138.

Referring to Fig. 36, each of the curves k27 to k29 is a curve CUR (index curve) created by the creation unit 215 of the diagnostic device 2 using the method described above. Curve k27 shows the integral value spectrum of the barley shochu of sample No. 1, curve k28 shows the integral value spectrum of the barley shochu of sample No. 2, and curve k29 shows the integral value spectrum of the barley shochu of sample No. 3.

Fig. 37 is a diagram showing integral value spectra for the shochu of samples No. 4 to No. 6 shown in Table 2, in the case where the number of integral values is 138.

Referring to Fig. 37, each of the curves k30 to k32 is a curve CUR (index curve) created by the creation unit 215 of the diagnostic device 2 using the method described above. Curve k30 shows the integral value spectrum of the barley shochu of sample No. 4, curve k31 shows the integral value spectrum of the barley shochu of sample No. 5, and curve k32 shows the integral value spectrum of the barley shochu of sample No. 6.

Fig. 38 is a diagram showing integral value spectra for the shochu of samples No. 7 to No. 9 shown in Table 2 when the number of integral values is 138.

Referring to Fig. 38, each of the curves k33 to k35 is a curve CUR (index curve) created by the creation unit 215 of the diagnostic device 2 using the method described above. A curve k33 indicates the integral value spectrum of the sweet potato shochu of sample No. 7, a curve k34 indicates the integral value spectrum of the sweet potato shochu of sample No. 8, and a curve k35 indicates the integral value spectrum of the sweet potato shochu of sample No. 9.

Fig. 39 is a diagram showing integral value spectra for the shochu of samples No. 10 to No. 12 shown in Table 2, in the case where the number of integral values is 138.

Referring to Fig. 39, each of the curves k36 to k38 is a curve CUR (index curve) created by the creation unit 215 of the diagnostic device 2 using the above-mentioned method. The curve k36 shows the integral value spectrum of the sweet potato shochu of sample No. 10, the curve k37 shows the integral value spectrum of the sweet potato shochu of sample No. 11, and the curve k38 shows the integral value spectrum of the sweet potato shochu of sample No. 12.

Fig. 40 is a diagram showing integral value spectra for the shochu of samples No. 12 to No. 14 shown in Table 2 when the number of integrals is 138.

Referring to Fig. 40, each of the curves k38 to k40 is a curve CUR (index curve) created by the creation unit 215 of the diagnostic device 2 using the above-mentioned method. The curve k38 shows the integral value spectrum of the sweet potato shochu of sample No. 12, the curve k39 shows the integral value spectrum of the sweet potato shochu of sample No. 13, and the curve k40 shows the integral value spectrum of the sweet potato shochu of sample No. 14.

Fig. 41 is a diagram showing integral value spectra for the shochu of samples No. 15 and No. 16 shown in Table 2, in the case where the number of integral values is 138.

Referring to Fig. 41, each of the curves k41 and k42 is a curve CUR (index curve) created by the creation unit 215 of the diagnostic device 2 using the above-mentioned method. The curve k41 shows the integral value spectrum of the rice shochu of sample No. 15, and the curve k42 shows the integral value spectrum of the rice shochu of sample No. 16.

Fig. 42 is a diagram showing integral value spectra for the shochu of samples No. 17 to No. 20 shown in Table 2, in the case where the number of integral values is 138.

Referring to Fig. 42, each of the curves k43 to k46 is a curve CUR (index curve) created by the creation unit 215 of the diagnostic device 2 by the above-mentioned method. The curve k43 shows the integral value spectrum of the shochu made with sake lees of sample No. 17, the curve k44 shows the integral value spectrum of the barley shochu stored for three years in its entirety, stored in barrels, and partially stored in cherry wood barrels of sample No. 18, the curve k45 shows the integral value spectrum of the barley shochu stored for 15 years in its entirety, all koji barley, all stored in barrels, and heated, of sample No. 19, and the curve k46 shows the integral value spectrum of the rice shochu stored for three years in its entirety, and stored in barrels of sample No. 20.

In Fig. 36 to Fig. 42, each of the classes 1 to 138 is composed of a predetermined potential section (integral value extraction potential) of 36.2 mV. Classes 1 to 69 are classes in the positive predetermined potential section, and classes 70 to 138 are classes in the negative predetermined potential section.

As a result, it is possible to caculate the sums $SMT_{_Low(+)}$, $SMT_{_Middle(+)}$, and $SMT_{_High(+)}$ of the integral values in the positive predetermined potential sections can be calculated based on the integral value spectra (curves $CUR_{_Low_k27}$ to $CUR_{_Low_k46}$, $CUR_{_Middle_k27}$ to $CUR_{_Middle_k46}$, and $CUR_{_High_k27}$ to $CUR_{_High_k46}$ similar to curves k27 to k46) in which all classes are composed of a predetermined potential section (integral value extraction potential) of 36.2 mV. Therefore, it is possible to calculate Body Index (+) based on the integral value spectra (curves $CUR_{_Low_k27}$ to $CUR_{_Low_k46}$, $CUR_{_Middle_k27}$ to $CUR_{_Middle\ k46}$, and $CUR_{_High_k27}$ to $CUR_{_High_k46}$ similar to curves k27 to k46) in which all classes are composed of a predetermined potential section (integral value extraction potential) of 36.2 mV to diagnose the astringency ASTG of the shochu using equation (5). In addition, it is possible to calculate H(+)_sum (=SMT_High(+)) based on the integral value spectra (curves $CUR_{_Low_k27}$ to $CUR_{_Low_k46}$, $CUR_{_Middle_k27}$ to $CUR_{_Middle_k46}$, and $CUR_{_High_k27}$ to $CUR_{_High_k46}$ similar to curves k27 to k46) in which all classes are composed of a predetermined potential section (integral value extraction potential) of 36.2 mV to diagnose the sweetness SWT of the shochu using equation (7). Furthermore, it is possible to calculate H(-)_sum (=SMT_High(+))based on the integral value spectra (curves $CUR_{_Low_k27}$ to $CUR_{_Low_k46}$, $CUR_{_Middle_k27}$ to $CUR_{_Middle_k46}$, and $CUR_{_High_k27}$ to $CUR_{_High_k46}$ similar to curves k27 to k46) in which all classes are composed of a predetermined potential section (integral value extraction potential) of 36.2 mV to diagnose the aroma SCT of the shochu using equation (8). As a result, the bitterness BIT of shochu can be diagnosed by equation (9).

Figs. 43 to 45 are first to third diagrams respectively showing the results of judging whether or not the curves k27 to k46 shown in Figs. 36 to 42 are different from one another.

Fig. 43 shows the results of the judging as to whether or not ten curves k27 to k36 are different from each other, Fig. 44 shows the results of the judging as to whether or not ten curves k27 to k36 and ten curves k37 to k46 are different from each other, and Fig. 45 shows the results of the judging as to whether or not ten curves k37 to k46 are different from each other.

The judgment of whether the 20 curves k27 to k46 are or not different from each other is performed by judging whether the two curves are or not different for all combinations (${}_{20}C_2$ = 190) in when selecting two different curves from the 20 curves k27 to k46.

Referring to Fig. 43, when two different curves are selected from the 10 curves k27 to k36, all of the 45 standard deviations of the differences $\sigma_{DF_k27,k28}$ to $\sigma_{DFk35,k36}$ for all of 45 combinations of two different curves are greater than the threshold value $\sigma_{th}$ (=5%).

Referring to Fig. 44, when two different curves are selected from 10 curves k27 to k36 and 10 curves k37 to k46, all of the 100 standard deviations of the differences $\sigma_{DF_k27},_{k37}$ to $\sigma DF_{k36,\ k46}$ for all of 100 combinations of two different curves are greater than the threshold value $\sigma_{th}$ (=5%).

Referring to Fig. 45, when two different curves are selected from 10 curves k37 to k46, all of the 45 standard deviations of the differences $\sigma_{DF_k37,k38}$ to $\sigma_{DF_k45,k46}$ for all of 45 combinations of two different curves are greater than the threshold value $\sigma_{th}$ (=5%).

Therefore, when two different curves are selected from the 20 curves k27 to k46, all of the 190 standard deviations of the differences $\sigma_{DF_k27,k28}$ to $\sigma_{DF_k45,k46}$ for all of 190 combinations of two different curves are greater than the threshold value $\sigma_{th}$ (=5%).

Therefore, the 20 curves k27 to k46 are mutually different curves. When it is judged that the 20 curves k27 to k46 are mutually different, the curves k27 to k46 are curves for uniquely identifying the shochu No. 1 to No. 20, respectively. When the number of integral values is 138, the curves k27 to k46 are fingerprints representing feature amounts by integral values for the shochu No. 1 to No. 20, respectively.

(II) Number of integral values: 70

Fig. 46 is a diagram showing integral value spectra for the shochu of samples No. 1 to No. 3 shown in Table 2, in the case where the number of integral values is 70.

Referring to Fig. 46, each of the curves k47 to k49 is a curve CUR (index curve) created by the creation unit 215 of the diagnostic device 2 using the above-mentioned method. The curve k47 shows the integral value spectrum of the barley shochu of sample No. 1, the curve k48 shows the integral value spectrum of the barley shochu of sample No. 2, and the curve k49 shows the integral value spectrum of the barley shochu of sample No. 3.

Fig. 47 is a diagram showing integral value spectra for the shochu of samples No. 4 to No. 6 shown in Table 2, in the case where the number of integral values is 70.

Referring to Fig. 47, each of the curves k50 to k52 is a curve CUR (index curve) created by the creation unit 215 of the diagnostic device 2 using the above-mentioned method. The curve k50 shows the integral value spectrum of the barley shochu of sample No. 4, the curve k51 shows the integral value spectrum of the barley shochu of sample No. 5, and the curve k52 shows the integral value spectrum of the barley shochu of sample No. 6.

Fig. 48 is a diagram showing integral value spectra for the shochu of samples No. 7 to No. 9 shown in Table 2, in the case where the number of integral values is 70.

Referring to Fig. 48, each of the curves k53 to k55 is a curve CUR (index curve) created by the creation unit 215 of the diagnostic device 2 using the above-mentioned method. The curve k53 shows the integral value spectrum of the sweet potato shochu of sample No. 7, the curve k54 shows the integral value spectrum of the sweet potato shochu of sample No. 8, and the curve k55 shows the integral value spectrum of the sweet potato shochu of sample No. 9.

Fig. 49 is a diagram showing integral value spectra for the shochu of samples No. 10 to No. 12 shown in Table 2, in the case where the number of integral values is 70.

Referring to Fig. 49, each of the curves k56 to k58 is a curve CUR (index curve) created by the creation unit 215 of the diagnostic device 2 using the above-mentioned method. A curve k56 indicates the integral value spectrum of the sweet potato shochu of sample No. 10, a curve k57 indicates the integral value spectrum of the sweet potato shochu of sample No. 11, and a curve k58 indicates the integral value spectrum of the sweet potato shochu of sample No. 12.

Fig. 50 is a diagram showing integral value spectra for the shochu of samples No. 12 to No. 14 shown in Table 2, in the case where the number of integral values is 70.

Referring to Fig. 50, each of the curves k58 to k60 is a curve CUR (index curve) created by the creation unit 215 of the diagnostic device 2 using the above-mentioned method. The curve k58 shows the integral value spectrum of the sweet potato shochu of sample No. 12, the curve k59 shows the integral value spectrum of the sweet potato shochu of sample No. 13, and the curve k60 shows the integral value spectrum of the sweet potato shochu of sample No. 14.

Fig. 51 is a diagram showing integral value spectra for the shochu of samples No. 15 and No. 16 shown in Table 2, in the case where the number of integral values is 70.

Referring to Fig. 51, each of the curves k61, k62 is a curve CUR (index curve) created by the creation unit 215 of the diagnostic device 2 using the above-mentioned method. The curve k61 shows the integral value spectrum of the rice shochu of sample No. 15, and the curve k62 shows the integral value spectrum of the rice shochu of sample No. 16.

Fig. 52 is a diagram showing integral value spectra for the shochu of samples No. 17 to No. 20 shown in Table 2, in the case where the number of integral values is 70.

Referring to Fig. 52, each of the curves k63 to k66 is a curve CUR (index curve) created by the creation unit 215 of the diagnostic device 2 using the above-mentioned method. Curve k63 shows the integral value spectrum of shochu made from sake lees with sample No. 17, curve k64 shows the integral value spectrum of barley shochu for all shochu aged for 3 years, all stored in barrels, some in cherry wood barrels with sample No. 18, curve k65 shows the integral value spectrum of barley shochu stored for 15 years, all koji barley, stored in barrels, and heated with sample No. 19, and curve k66 shows rice shochu stored for 3 years and stored in barrels with sample No. 20.

In from Fig. 46 to Fig. 52, each of classes 1 to 34 has a predetermined potential section (integral value extraction potential) of 72.4 mV, class 35 has a predetermined potential section (integral value extraction potential) of 36.2 mV, each of classes 36 to 69 has a predetermined potential section (integral value extraction potential) of 72.4 mV, and class 70 has a predetermined potential section (integral value extraction potential) of 36.2 mV. Classes 1 to 35 are classes in the positive potential section, and classes 36 to 70 are classes in the negative potential section.

The reason why class 35 has a predetermined potential section (integral value extraction potential) of 36.2 mV, which is smaller than the predetermined potential section (integral value extraction potential) of 72.4 mV in each of classes 1 to 34, is to make the class 35 the "last class in the positive predetermined potential section."

In other words, when class 35 is composed of the same predetermined potential section (integral value extraction potential) as the predetermined potential section (integral value extraction potential) of 72.4 mV in each of classes 1 to 34, the integral value in class 35 is the sum of the integral value in the positive predetermined potential section and the integral value in the negative predetermined potential section, and it is not possible to calculate the sums $SMT_{_Low(+)}$, $SMT_{_Middle(+)}$, and $SMT_{_High(+)}$ of the integral values in the positive predetermined potential section based on an integral value spectrum in which all classes are composed of the predetermined potential section (integral value extraction potential) of 72.4 mV. As a result, it is not possible to diagnose the astringency ASTG of shochu using formula (5) by calculating the Body Index (+) based on the integral value spectrum consisting of a predetermined potential section (integral value extraction potential) of 72.4 mV for all classes, it is not possible to diagnose the sweetness SWT of shochu using formula (7) by calculating H(+)_sum (=SMT_High(+)) based on the integral value spectrum consisting of a predetermined potential section (integral value extraction potential) of 72.4 mV for all classes, it is not possible to diagnose the aroma SCT of shochu using formula (8) by calculating H(-)_sum (=$SMT_{_High(-)}$) based on the integral value spectrum consisting of a predetermined potential section (integral value extraction potential) of 72.4 mV for all classes, and as a result, it is not possible to diagnose the bitterness BIT of shochu using formula (9). Therefore, the class 35 has a predetermined potential section (integral value extraction potential) of 36.2 mV.

Figs. 53 to 55 are first to third diagrams respectively showing the results of judging whether or not the curves k47 to k66 shown in Figs. 46 to 52 are different from one another, respectively.

Fig. 53 shows the results of a judging as to whether or not ten curves k47 to k56 are different from each other, Fig. 54 shows the results of a judging as to whether or not ten curves k47 to k56 and ten curves k57 to k66 are different from each other, and Fig. 55 shows the results of a judging as to whether or not ten curves k57 to k66 are different from each other.

The judgment of whether the 20 curves k47 to k66 are or not different from each other is performed by judging whether the two different curves are or not different for all combinations ($_{20}C_2$ = 190) on when selecting two different curves from the 20 curves k47 to k66.

Referring to Fig. 53, when two different curves are selected from the ten curves k47 to k56, all of the 45 standard deviations $\sigma_{DF_k47, k48}$ to $\sigma_{DF_k55, k56}$ of the differences for all 45 combinations of two different curves are greater than the threshold value $\sigma_{th}$ (=5%).

Referring to Fig. 54, when two different curves are selected from 10 curves k47 to k56 and 10 curves k57 to k66, all of the 100 standard deviations $\sigma_{DF_k47, k57}$ to $\sigma_{DF_k56, k66}$ of the differences for all of 100 combinations of two different curves are greater than the threshold value $\sigma_{th}$ (=5%).

Referring to Fig. 55, when two different curves are selected from the ten curves k57 to k66, all of the 45 standard deviations of the differences $\sigma DF_{k57, k58}$ to $\sigma_{DF_k65, k66}$ for all of 45 combinations of two different curves are greater than the threshold value $\sigma_{th}$ (=5%).

Therefore, when two different curves are selected from the 20 curves k47 to k66, all of the 190 standard deviations of the differences $\sigma_{DF_k47, k48}$ to $\sigma_{DF_k65, k66}$ for all 190 combinations of two different curves are greater than the threshold value $\sigma_{th}$ (=5%).

Therefore, the 20 curves k47 to k66 are mutually different curves. When it is judged that the 20 curves k47 to k66 are mutually different, the curves k47 to k66 are curves for uniquely identifying the shochu No. 1 to No. 20, respectively. When the number of integral values is 70, the curves k47 to k66 are fingerprints representing feature amounts based on integral values for the shochu No. 1 to No. 20, respectively.

Other explanations for the case where the number of integral values is 70 are the same as those for the case where the number of integral values is 138.

(III) Number of integral values: 36

Fig. 56 is a diagram showing integral value spectra for the shochu of samples No. 1 to No. 3 shown in Table 2, in the case where the number of integral values is 36.

Referring to Fig. 56, each of the curves k67 to k69 is a curve CUR (index curve) created by the creation unit 215 of the diagnostic device 2 using the above-mentioned method. The curve k67 shows the integral value spectrum of the barley shochu of sample No. 1, the curve k68 shows the integral value spectrum of the barley shochu of sample No. 2, and the curve k69 shows the integral value spectrum of the barley shochu of sample No. 3.

Fig. 57 is a diagram showing integral value spectra for the shochu of samples No. 4 to No. 6 shown in Table 2, in the case where the number of integral values is 36.

Referring to Fig. 57, each of the curves k70 to k72 is a curve CUR (index curve) created by the creation unit 215 of the diagnostic device 2 using the above-mentioned method. The curve k70 shows the integral value spectrum of the barley shochu of sample No. 4, the curve k71 shows the integral value spectrum of the barley shochu of sample No. 5, and the curve k72 shows the integral value spectrum of the barley shochu of sample No. 6.

Fig. 58 is a diagram showing integral value spectra for the shochu of samples No. 7 to No. 9 shown in Table 2, in the case where the number of integral values is 36.

Referring to Fig. 58, each of the curves k73 to k75 is a curve CUR (index curve) created by the creation unit 215 of the diagnostic device 2 using the above-mentioned method. The curve k73 shows the integral value spectrum of the sweet potato shochu of sample No. 7, the curve k74 shows the integral value spectrum of the sweet potato shochu of sample No. 8, and the curve k75 shows the integral value spectrum of the sweet potato shochu of sample No. 9.

Fig. 59 is a diagram showing integral value spectra for the shochu of samples No. 10 to No. 12 shown in Table 2, in the case where the number of integral values is 36.

Referring to Fig. 59, each of the curves k76 to k78 is a curve CUR (index curve) created by the creation unit 215 of the diagnostic device 2 using the above-mentioned method. The curve k76 shows the integral value spectrum of the sweet potato shochu of sample No. 10, the curve k77 shows the integral value spectrum of the sweet potato shochu of sample No. 11, and the curve k78 shows the integral value spectrum of the sweet potato shochu of sample No. 12.

Fig. 60 is a diagram showing integral value spectra for the shochu of samples No. 12 to No. 14 shown in Table 2, in the case where the number of integral values is 36.

Referring to Fig. 60, each of the curves k78 to k80 is a curve CUR (index curve) created by the creation unit 215 of the diagnostic device 2 using the above-mentioned method. The curve k78 shows the integral value spectrum of the sweet potato shochu of sample No. 12, the curve k79 shows the integral value spectrum of the sweet potato shochu of sample No. 13, and the curve k80 shows the integral value spectrum of the sweet potato shochu of sample No. 14.

Fig. 61 is a diagram showing integral value spectra for the shochu of samples No. 15 to No. 16 shown in Table 2, in the case where the number of integral values is 36.

Referring to Fig. 61, each of the curves k81 to k82 is a curve CUR (index curve) created by the creation unit 215 of the diagnostic device 2 using the above-mentioned method. The curve k81 shows the integral value spectrum of the rice shochu of sample No. 15, and the curve k82 shows the integral value spectrum of the rice shochu of sample No. 16.

Fig. 62 is a diagram showing integral value spectra for the shochu of samples No. 17 to No. 20 shown in Table 2, in the case where the number of integral values is 36.

Referring to Fig. 62, each of the curves k83 to k86 is a curve CUR (index curve) created by the creation unit 215 of the diagnostic device 2 using the above-mentioned method. The curve k83 shows the integral value spectrum of shochu made with sake lees in sample No. 17, the curve k84 shows the integral value spectrum of sample No. 18, barley shochu stored for 3 years, stored in barrels, and partially stored in cherry wood barrels, the curve k85 shows the integral value spectrum of sample No. 19, barley shochu stored for 15 years, all koji barley, stored in barrels, and heated, and the curve k86 shows the integral value spectrum of sample No. 20, rice shochu stored for 3 years and stored in barrels.

In Fig. 56 to Fig. 62, each of classes 1 to 17 has a predetermined potential section (integral value extraction potential) of 144.8 mV, class 18 has a predetermined potential section (integral value extraction potential) of 36.2 mV, each of classes 19 to 35 has a predetermined potential section (integral value extraction potential) of 144.8 mV, and class 36 has a predetermined potential section (integral value extraction potential) of 36.2 mV. Classes 1 to 18 are classes in the positive potential section, and classes 19 to 36 are classes in the negative potential section.

The reason why class 18 has a predetermined potential section (integral value extraction potential) of 36.2 mV, which is smaller than the predetermined potential section (integral value extraction potential) of 144.8 mV in each of classes 1 to 17, is to make class 18 the "last class in the positive predetermined potential section."

In other words, when class 18 is composed of the same predetermined potential section (integral value extraction potential) as the predetermined potential section (integral value extraction potential) of 144.8 mV in each of classes 1 to 17, the integral value in class 18 is the sum of the integral value in the positive predetermined potential section and the integral value in the negative predetermined potential section, and it is not possible to calculate the sums $SMT_{_Low(+)}$, $SMT_{_Middle(+)}$, and $SMT_{_High(+)}$ of the integral values in the positive predetermined potential section based on an integral value spectrum in which all classes are composed of the predetermined potential section (integral value extraction potential) of 144.8 mV. As a result, it is not possible to diagnose the astringency ASTG of shochu using formula (5) by calculating the Body index(+) based on the integral value spectrum consisting of a predetermined potential section (integral value extraction potential) of 144.8 mV for all classes, it is not possible to diagnose the sweetness SWT of shochu using formula (7) by calculating H(+)_sum ($=SMT_{_High(+)}$) based on the integral value spectrum consisting of a predetermined potential section (integral value extraction potential) of 144.8 mV for all classes, it is not possible to diagnose the aroma SCT of shochu using formula (8) by calculating H(-)_sum ($=SMT_{_High(-)}$) based on the integral value spectrum consisting of a predetermined potential section (integral value extraction potential) of 144.8 mV for all classes, and as a result, it is not possible to diagnose the bitterness BIT of shochu using formula (9). Therefore, the class 35 has a predetermined potential section (integral value extraction potential) of 36.2 mV.

Figs. 63 to 65 are first to third diagrams respectively showing the results of judging whether the curves k67 to k86 shown in Figs. 56 to 62 are or not different from one another.

Fig. 63 shows the results of the judgment as to whether or not ten curves k67 to k76 are different from each other, Fig. 64 shows the results of the judgment as to whether or not ten curves k67 to k76 and ten curves k77 to k86 are different from each other, and Fig. 65 shows the results of the judgment as to whether or not ten curves k77 to k86 are different from each other.

The judgment of whether the 20 curves k67 to k86 are or not different from each other is performed by judging whether the two different curves are or not different for all combinations (${}_{20}C_2$ = 190) when selecting two different curves from the 20 curves k67 to k86.

Referring to Fig. 63, when two different curves are selected from the ten curves k67 to k76, all of the 45 standard deviations $\sigma DF_{k67,\,k68}$ to $\sigma_{DF_k75,\,k76}$ of the differences for all 45 combinations of two different curves are greater than the threshold value $\sigma_{th}$ (=5%).

Referring to Fig. 64, when two different curves are selected from 10 curves k67 to k76 and 10 curves k77 to k86, all of the 100 standard deviations $\sigma_{DF_k67,\,k77}$ to $\sigma_{DF_k76,\,k86}$ of the differences for all of 100 combinations of two different curves are greater than the threshold value $\sigma_{th}$ (=5%).

Referring to Fig. 65, when two different curves are selected from the ten curves k77 to k86, all of the 45 standard deviations $\sigma_{DF\text{--}k77,\,k78}$ to $\sigma_{DF_k85,\,k86}$ of the differences for all of 45 combinations of two different curves are greater than the threshold value $\sigma_{th}$ (= 5%).

Therefore, when two different curves are selected from the 20 curves k67 to k86, all of the 190 standard deviations $\sigma_{DF_k67, k68}$ to $\sigma_{DF_k85, k86}$ of the differences for all of 190 combinations of two different curves are greater than the threshold value $\sigma_{th}$ (=5%).

Therefore, the 20 curves k67 to k86 are mutually different curves. When it is judged that the 20 curves k67 to k86 are mutually different, the curves k67 to k86 are curves for uniquely identifying the shochu No. 1 to No. 20, respectively. When the number of integral values is 36, the curves k67 to k86 are fingerprints representing feature amounts based on integral values for the shochu of No. 1 to No. 20, respectively.

Other explanations for the case where the number of integral values is 36 are the same as explanations for the case where the number of integral values is 138.

(IV) Number of integral values: 18

Fig. 66 is a diagram showing integral value spectra for the shochu of samples No. 1 to No. 3 shown in Table 2, in the case where the number of integral values is 18.

Referring to Fig. 66, each of the curves k87 to k89 is a curve CUR (index curve) created by the creation unit 215 of the diagnostic device 2 using the above-mentioned method. The curve k87 shows the integral value spectrum of the barley shochu of sample No. 1, the curve k88 shows the integral value spectrum of the barley shochu of sample No. 2, and the curve k89 shows the integral value spectrum of the barley shochu of sample No. 3.

Fig. 67 is a diagram showing integral value spectra for the shochu of samples No. 4 to No. 6 shown in Table 2, in the case where the number of integral values is 18.

Referring to Fig. 67, each of the curves k90 to k92 is a curve CUR (index curve) created by the creation unit 215 of the diagnostic device 2 using the above-mentioned method. The curve k90 shows the integral value spectrum of the barley shochu of sample No. 4, the curve k91 shows the integral value spectrum of the barley shochu of sample No. 5, and the curve k92 shows the integral value spectrum of the barley shochu of sample No. 6.

Fig. 68 is a diagram showing integral value spectra for the Shochu of samples No. 7 to No. 9 shown in Table 2 when the number of integral values is 18.

Referring to Fig. 68, each of the curves k93 to k95 is a curve CUR (index curve) created by the creation unit 215 of the diagnostic device 2 using the above-mentioned method. The curve k93 shows the integral value spectrum of the sweet potato shochu of sample No. 7, the curve k94 shows the integral value spectrum of the sweet potato shochu of sample No. 8, and the curve k95 shows the integral value spectrum of the sweet potato shochu of sample No. 9.

Fig. 69 is a diagram showing integral value spectra for the shochu of samples No. 10 to No. 12 shown in Table 2 when the number of integral values is 18.

Referring to Fig. 69, each of the curves k96 to k98 is a curve CUR (index curve) created by the creation unit 215 of the diagnostic device 2 using the above-mentioned method. The curve k96 shows the integral value spectrum of the sweet potato shochu of sample No. 10, the curve k97 shows the integral value spectrum of the sweet potato shochu of sample No. 11, and the curve k98 shows the integral value spectrum of the sweet potato shochu of sample No. 12.

Fig. 70 is a diagram showing integral value spectra for the shochu of samples No. 12 to No. 14 shown in Table 2 when the number of integral values is 18.

Referring to Fig. 70, each of the curves k98 to k100 is a curve CUR (index curve) created by the creation unit 215 of the diagnostic device 2 using the above-mentioned method. The curve k98 shows the integral value spectrum of the sweet potato shochu of sample No. 12, the curve k99 shows the integral value spectrum of the sweet potato shochu of sample No. 13, and the curve k100 shows the integral value spectrum of the sweet potato shochu of sample No. 14.

Fig. 71 is a diagram showing integral value spectra for the shochu of samples No. 15 and No. 16 shown in Table 2 when the number of integral values is 18.

Referring to Fig. 71, each of curves k101 and k102 is a curve CUR (index curve) created by the creation unit 215 of the diagnostic device 2 using the above-mentioned method. The curve k101 shows the integral value spectrum of the rice shochu of sample No. 15, and the curve k102 shows the integral value spectrum of the rice shochu of sample No. 16.

Fig. 72 is a diagram showing integral value spectra for the shochu of samples No. 17 to No. 20 shown in Table 2 when the number of integral values is 18.

Referring to Fig. 72, each of the curves k103 to k106 is a curve CUR (index curve) created by the creation unit 215 of the diagnostic device 2 by the above-mentioned method. The curve k103 shows the integral value spectrum of the shochu made with sake lees of sample No. 17, the curve k104 shows the integral value spectrum of the barley shochu stored for three years in its entirety, stored in a barrel, and partially stored in a cherry wood barrel of sample No. 18, the curve k105 shows the integral value spectrum of the barley shochu stored for 15 years in its entirety, all koji barley, stored in a barrel, and heated, of sample No. 19, and the curve k106 shows the integral value spectrum of the rice shochu stored for three years in its entirety, and stored in a barrel of sample No. 20.

In Figs. 66 to 72, each of classes 1 to 8 has a predetermined potential section (integral value extraction potential) of 307.7 mV, class 9 has a predetermined potential section (integral value extraction potential) of 36.2 mV, each of classes 10 to 17 has a predetermined potential section (integral value extraction potential) of 307.7 mV, and class 18 has a predetermined potential section (integral value extraction potential) of 36.2 mV. Classes 1 to 9 are classes in the positive potential section, and classes 10 to 18 are classes in the negative potential section.

The reason why class 9 has a predetermined potential section (integral value extraction potential) of 36.2 mV, which is smaller than the predetermined potential section (integral value extraction potential) of 307.7 mV in each of classes 1 to 8, is to make class 9 the "last class in the positive predetermined potential section."

In other words, when class 9 is composed of the same predetermined potential section (integral value extraction potential) as the predetermined potential section (integral value extraction potential) of 307.7 mV in each of classes 1 to 8, the integral value in class 9 is the sum of the integral value in the positive predetermined potential section and the integral value in the negative predetermined potential section, and it is not possible to calculate the sums $SMT_{_Low(+)}$, $SMT_{_Middle(+)}$, and $SMT_{_High(+)}$ of the integral values in the positive predetermined potential section based on an integral value spectrum in which all classes are composed of the predetermined potential section (integral value extraction potential) of 307.7 mV. As a result, it is not possible to diagnose the astringency ASTG of shochu using formula (5) by calculating the Body index(+) based on the integral value spectrum consisting of a predetermined potential section (integral value extraction potential) of 307.7 mV for all classes, it is not possible to diagnose the sweetness SWT of shochu using formula (7) by calculating H(+)_sum (=$SMT_{_High(+)}$) based on the integral value spectrum consisting of a predetermined potential section (integral value extraction potential) of 307.7 mV for all classes, it is not possible to diagnose the aroma SCT of shochu using formula (8) by calculating H(-)_sum (=$SMT_{_High(-)}$) based on the integral value spectrum consisting of a predetermined potential section (integral value extraction potential) of 307.7 mV for all classes, and as a result, it is not possible to diagnose the bitterness BIT of shochu using formula (9). Therefore, the class 9 has a predetermined potential section (integral value extraction potential) of 36.2 mV.

Figs. 73 to 75 are first to third diagrams respectively showing the results of judging whether the curves k87 to k106 shown in Figs. 66 to 72 are or not different from one another.

Fig. 73 shows the results of judging whether ten curves k87 to k96 are or not different from each other, Fig. 74 shows the results of judging whether ten curves k87 to k96 and ten curves k97 to k106 are or not different from each other, and Fig. 75 shows the results of judging whether ten curves k97 to k106 are or not different from each other.

The judgment of whether the 20 curves k87 to k106 are or not different from each other is executed by judging whether the two different curves are or not different for all combinations (${}_{20}C_2$ = 190) in when selecting two different curves from the 20 curves k87 to k106.

Referring to Fig. 73, when two different curves are selected from the ten curves k87 to k96, all of the 45 standard deviations $\sigma_{DF_k87,k88}$ to $\sigma_{DF_k95,k96}$ of the differences for all of 45 combinations of two different curves are greater than the threshold value $\sigma_{th}$ (=5%).

Referring to Fig. 74, when two different curves are selected from ten curves k87 to k96 and ten curves k97 to k106, all of the 100 standard deviations $\sigma_{DF_k87,k97}$ to $\sigma DF_{_k96,k106}$ of the differences for all of 100 combinations of two different curves are greater than the threshold value $\sigma_{th}$ (=5%).

Referring to Fig. 75, when two different curves are selected from the ten curves k97 to k106, all of the 45 standard deviations $\sigma_{DF_k97,k98}$ to $\sigma_{DF_k105,k106}$ of the differences for all 45 combinations of two different curves are greater than the threshold value $\sigma_{th}$ (=5%).

Therefore, when two different curves are selected from the 20 curves k87 to k106, all of the 190 standard deviations $\sigma_{DF_k87,k88}$ to $\sigma_{DF_k105,k106}$ of the differences for all 190 combinations of two different curves are greater than the threshold value $\sigma_{th}$ (=5%).

Therefore, the 20 curves k87 to k106 are mutually different curves. When it is judged that the 20 curves k87 to k106 are mutually different, the curves k87 to k106 are curves for uniquely identifying shochu No. 1 to No. 20, respectively. When the number of integral values is 18, the curves k87 to k106 are fingerprints representing feature amounts based on integral values for shochu No. 1 to No. 20, respectively.

Although not shown in the figure, it was confirmed that when the number of integral values was 92, 56, 32, and 20, the standard deviation of the differences was greater than the threshold value $\sigma_{th}$ (5%) for all combinations (${}_{20}C_2$ = 190) when selecting two different curves from 20 curves.

Other explanations regarding the case where the number of integral values is 18 are the same as those regarding the case where the number of integral values is 138.

As described above, the index curve for uniquely identifying Shochu No. 1 is made up of a plurality of curves k1, k27, k47, k67, and k87 each having a different number of integral values, the index curve for uniquely identifying Shochu No. 2 is made up of a plurality of curves k2, k28, k48, k68, and k88 each having a different number of integral values, the index curve for uniquely identifying Shochu No. 3 is made up of a plurality of curves k3, k29, k49, k69, and k89 each having a different number of integral values, the index curve for uniquely identifying Shochu No. 4 is made up of a plurality of curves k4, k30, k50, k70, and k90 each having a different number of integral values, and the index curve for uniquely identifying Shochu No. 5 is made up of a plurality of curves k5, k31, k51, k71, and k91 each having a different number of integral values.

In addition, the index curve for uniquely identifying Shochu No. 6 is made up of a plurality of curves k6, k32, k52, k72, and k92 each having a different number of integral values, the index curve for uniquely identifying Shochu No. 7 is made up of a plurality of curves k7, k33, k53, k73, and k93 each having a different number of integral values, the index curve for uniquely identifying Shochu No. 8 is made up of a plurality of curves k8, k34, k54, k74, and k94 each having a different number of integral values, the index curve for uniquely identifying Shochu No. 9 is made up of a plurality of curves k9, k35, k55, k75, and k95 each having a different number of integral values, and the index curve for uniquely identifying Shochu No. 1 0 is made up of a plurality of curves k10, k36, k56, k76, and k96 each having a different number of integral values.

Furthermore, the index curve for uniquely identifying Shochu No. 11 is made up of a plurality of curves k11, k37, k57, k77, and k97 having different numbers of integral values, the index curve for uniquely identifying Shochu No. 12 is made up of a plurality of curves k12, k38, k58, k78, and k98 having different numbers of integral values, the index curve for uniquely identifying Shochu No. 13 is made up of a plurality of curves k13, k39, k59, k79, and k99 having different numbers of integral values, the index curve for uniquely identifying Shochu No. 14 is made up of a plurality of curves k14, k40, k60, k80, and k100 having different numbers of integral values, and the index curve for uniquely identifying Shochu No. 15 is made up of a plurality of curves k15, k41, k61, k81, and k101 each having a different number of integral values.

Furthermore, the index curve for uniquely identifying Shochu No. 16 is made up of a plurality of curves k16, k42, k62, k82, and k102 having different numbers of integral values, the index curve for uniquely identifying Shochu No. 17 is made up of a plurality of curves k17, k43, k63, k83, and k103 having different numbers of integral values, the index curve for uniquely identifying Shochu No. 18 is made up of a plurality of curves k18, k44, k64, k84, and k104 having different numbers of integral values, the index curve for uniquely identifying Shochu No. 19 is made up of a plurality of curves k19, k45, k65, k85, and k105 having different numbers of integral values, and the index curve for uniquely identifying Shochu No. 20 is made up of a number of curves k20, k46, k66, k86, and k106 each having a different number of integral values.

In taste diagnosis of shochu, the taste diagnostic unit 217 diagnoses the astringency ASTG of the shochu using formula (5), diagnoses the aftertaste LNGS of the shochu using formula (6), diagnoses the sweetness SWT of the shochu using formula (7), diagnoses the aroma SCT of the shochu using formula (8), and diagnoses the bitterness BIT of the shochu using formula (9).

The taste diagnostic unit 217 uses the Body index (+) shown in equation (3) when diagnosing the astringency ASTG of shochu using equation (5), uses the Body index (all) when diagnosing the aftertaste LNGS of shochu using equation (6), uses $H(+)_{sum}$ when diagnosing the sweetness SWT of shochu using equation (7), uses H(-)_sum when diagnosing the aroma SCT of shochu using equation (8), and uses the bitterness ASTG of shochu and the sweetness SWT of shochu when diagnosing the bitterness BIT of shochu using equation (9).

The taste diagnostic unit 217 calculates, in equation (3), the Body Index (+) using the sum $SMT_{_Low(+)}$ of integral values in the positive potential section of the integral value spectrum created based on the cyclic voltammogram measured using the potential scanning speed $V_{r_Low}$, the sum $SMT_{_Middle(+)}$ of integral value in the positive potential section of the integral value spectrum created based on the cyclic voltammogram measured using the potential scanning speed $V_{r_Middle}$, and the sum $SMT_{_High(+)}$ of integral value in the positive potential section of the integral value spectrum created based on the cyclic voltammogram measured using the potential scanning speed $V_{r_High}$.

In addition, the taste diagnostic unit 217 calculates, in equation (4), the Body Index (all) using the sum $SMT_{_Low(all)}$ of integral values in all potential sections of the integral value spectrum created based on the cyclic voltammogram measured using the potential scanning speed $V_{r_Low}$, the sum $SMT_{_Middle(all)}$ of integral values in all potential sections of the integral value spectrum created based on the cyclic voltammogram measured using the potential scanning speed $V_{r_Middle}$, and the sum $SMT_{_High(all)}$ of integral values in all potential sections of the integral value spectrum created based on the cyclic voltammogram measured using the potential scanning speed $V_{r_High}$.

Fig. 76 is a diagram showing the correspondence between the classes and integral values when the number of integral values is 138 and the classes and integral values when the number of integral values is 276.

Referring to Fig. 76 (a) and (b), when the number of integral value (=the number of classes) is 138, the integral value $ITG_{1_138}$ in class 1 is the sum of the integral values $ITG_{1_276}$ and $ITG_{2_276}$ in classes 1 and 2 when the number of integral value (=the number of classes) is 276, the integral value $ITG_{2_138}$ in class 2 is the sum of the integral values $ITG_{3_276}$ and $ITG_{4_276}$ in classes 3 and 4 when the number of integral value (=the number of classes) is 276,..., the integral value $ITG_{68_138}$ in class 68 is the sum of the integral values $ITG_{137_276}$ and $ITG_{138_276}$ in classes 137 and 138 when the number of integral values (=the number of classes) is 276, the integral value $ITG_{69_138}$ in class 69 is the sum of the integral values $ITG_{139_276}$ and $ITG_{140_276}$ in classes 139 and 140 when the number of integral values (=the number of classes) is 276,..., the integral value $ITG_{138_138}$ in class 138 is the sum of the integral values $ITG_{275_276}$ and $ITG_{276_276}$ in classes 275 and 276 when the number of integral values (=the number of classes) is 276.

In Fig. 76 (a), classes 1 to 138 are classes in the positive potential section, integral values $ITG_{1_276}$ to $ITG_{138_276}$ are integral values in the positive potential section, classes 139 to 276 are classes in the negative potential section, and integral values $ITG_{139_276}$ to $ITG_{276_276}$ are integral values in the negative potential section.

As a result, in (b) of Fig. 76, classes 1 to 68 are classes in the positive potential section, integral values $ITG_{1_138}$ to $ITG_{68_138}$ are integral values in the positive potential section, classes 69 to 138 are classes in the negative potential section, and integral values $ITG_{69_138}$ to $ITG_{138_138}$ are integral values in the negative potential section.

In this case, the sum of the integral values $ITG_{1_138}$ to $ITG_{68_138}$ in classes 1 to 68 in Fig. 76 (b) is equal to the sum of the integral values $ITG_{1_276}$ to $ITG_{138_276}$ in classes 1 to 138 in Fig. 76 (a).

In addition, the sum of the integral values $ITG_{69_138}$ to $ITG_{138_138}$ in classes 69 to 138 in Fig. 76 (b) is equal to the sum of the integral values $ITG_{139_276}$ to $ITG_{276_276}$ in classes 139 to 276 in Fig. 76 (a).

Furthermore, the sum of the integral values $ITG_{1_138}$ to $ITG_{138_138}$ in classes 1 to 138 in Fig. 76 (b) is equal to the sum of the integral values $ITG_{1_276}$ to $ITG_{276_276}$ in classes 1 to 276 in Fig. 76 (a).

Therefore, the sum $SMT_{_Low(+)_138}$ of integral values in the positive potential section of the integral value spectrum consisting of 138 integral values created based on the cyclic voltammogram measured using the potential scanning speed $V_{r_Low}$ is equal to the sum $SMT_{_Low(+)276}$ of integral values in the positive potential section of the integral value spectrum consisting of 276 integral values created based on the cyclic voltammogram measured using the potential scanning speed $V_{r_Low}$.

The same is true for the sum $SMT_{_Middle(+)_138}$ of integral values in the positive potential section of an integral value spectrum consisting of 138 integral values created based on a cyclic voltammogram measured using a potential scanning speed $V_{r_Middle}$, and the sum $SMT_{_High(+)_138}$ of integral values in the positive potential section of an integral value spectrum consisting of 138 integral values created based on a cyclic voltammogram measured using a potential scanning speed $V_{r_High}$.

As a result, in equation (3), Body Index $(+)_{_138}$ calculated based on the sums of the integral values $SMT_{_Low(+)_138}$, $SMT_{_Middle(+)_138}$, and $SMT_{_High(+)_138}$ is equal to Body Index $(+)_{_276}$ calculated based on the sums $SMT_{_Low(+)_276}$, $SMT_{_Middle(+)_276}$, and $SMT_{_High(+)_276}$ of the integral values .

In addition, the sum $SMT_{_Low(all)_138}$ of the integral values in all potential sections of the integral value spectrum consisting of 138 integral values created based on the cyclic voltammogram measured using the potential scanning speed $V_{r_Low}$ is equal to the sum $SMT_{_Low(all)_276}$ of the integral values in all potential sections of the integral value spectrum consisting of 276 integral values created based on the cyclic voltammogram measured using the potential scanning speed $V_{r_Low}$.

The same is true for the sum $SMT_{_Middle(all)_138}$ of the integral values over all potential sections of an integral value spectrum consisting of 138 integral values created based on a cyclic voltammogram measured using a potential scanning speed $V_{r_Middle}$, and the sum $SMT_{_High(all)_138}$ of the integral values over all potential sections of an integral value spectrum consisting of 138 integral values created based on a cyclic voltammogram measured using a potential scanning speed $V_{r_High}$.

As a result, in equation (4), Body index $(all)_{_138}$ calculated using the sums $SMT_{_Low(all)_138}$, $SMT_{_Middle(all)_138}$, and $SMT_{_High(all)138}$ of the integral values is equal to Body index $(all)_{_276}$ calculated using the sums $SMT_{_Low(all)_276}$, $SMT_{_Middle(all)_276}$, and $SMT_{_High(all)_276}$ of the integral values.

Furthermore, $H(+)_sum_{_138}$ which is the sum $SMT_{_High(+)}$ of the integral values in the positive potential section of an integral value spectrum consisting of 138 integral values created based on a cyclic voltammogram measured using a potential scanning speed $V_{r_High}$, is equal to $H(+)_sum_{276}$ which is the sum $SMT_{_High(+)}$ of the integral values in the positive potential section of an integral value spectrum consisting of 276 integral values created based on a cyclic voltammogram measured using a potential scanning speed $V_{r_High}$.

Furthermore, $H(-)_sum_{_138}$ which is the sum $SMT_{_High(-)}$ of the integral values in the negative potential section of an integral value spectrum consisting of 138 integral values created based on a cyclic voltammogram measured using a potential scanning speed $V_{r_High}$, is equal to $H(-)_sum_{_276}$ which is the sum $SMT_{_High(-)}$ of the integral values in the negative potential section of an integral value spectrum consisting of 276 integral values created based on a cyclic voltammogram measured using a potential scanning speed $V_{r_High}$.

Even if the number of integral values changes, since the coefficients $k_1$ to $k_8$ in formulas (5) to (9) do not change, in equation (5), the astringency $ASTG_{_138}$ of shochu calculated using the Body index $(+)_{_138}$ is equal to the astringency $ASTG_{_276}$ of shochu calculated using the Body index $(+)_{_276}$, in formula (6), the aftertaste $LNGS_{_138}$ of the shochu calculated using the Body index $(all)_{_138}$ is equal to the aftertaste $LNGS_{_276}$ of the shochu calculated using the Body index $(all)_{_276}$, in formula (7), the sweetness $SWT_{_138}$ of shochu calculated using $H(+)_sum_{_138}$ (=$SMT_{_High(+)_138}$) is equal to the sweetness $SWT_{_276}$ of shochu calculated using $H(+)_{_sum_276}$ (=$SMT_{_High(+)276}$), in formula (8), the aroma $SCT_{_138}$ of shochu calculated using $H(-)_sum_{_138}$ (=$SMT_{_High(-)_138}$) is consistent with the aroma $SCT_{_276}$ of shochu calculated using $H(-)_sum_{_276}$ (=$SMT_{_High(-)_276}$), and in formula (9), the bitterness $BIT_{_138}$ of shochu calculated using the astringency $ASTG_{_138}$ and sweetness $SWT_{_138}$ of shochu is equal to the bitterness $BIT_{_276}$ of shochu calculated using the astringency $ASTG_{_276}$ and sweetness $SWT_{_276}$ of shochu.

The same is applied to the cases where the number of integral values is any of 92, 70, 56, 36, 32, 20, and 18.

Therefore, even if the number of integral values in the integral value spectrum which is the fingerprint for Shochu No. 1 to Shochu No. 20 changes, the results of the taste diagnosis of Shochu No. 1 to Shochu No. 20 will match the "astringency", "aftertaste", "sweetness", "aroma" and "bitterness" shown in Fig. 29.

As a result, the diagnostic device 2 may perform the taste diagnosis for Shochu No. 1 to Shochu No. 20 by the above-mentioned method based on the integral value spectrum $ITG_{_SPC_n}$ in which the number of integral values is any one of 18, 20, 32, 36, 56, 70, 92, 138, and 276.

In this case, the integral value spectrum $ITG_{_SPC_n}$ is composed of an integral value spectrum consisting of integral values $ITG_{1_Low}$ to $ITG_{n_Low}$, an integral value spectrum consisting of integral values $ITG_{1_Middle}$ to $ITG_{n_Middle}$, and an integral value spectrum consisting of integral values $ITG_{1_High}$ to $ITG_{n_High}$, where n is any of 18, 20, 32, 36, 56, 70, 92, 138, and 276.

[Relationship between taste diagnosis and number of integral values in grapes]

As described above, in the taste diagnosis of Crimson Seed (skin + flesh), Green Seedless (skin + flesh), Shine Muscat (skin + flesh), Crimson Seedless (flesh only), Green Seedless (flesh only) and Shine Muscat (flesh only), the threshold value Vth of the potential V is set to -1362 mV, and L(-)_sum_th, M(-)_sum_th and H(-)_sum_th calculated based on the integral value in a specified potential section (=class) in the range of -1362 mV to - 2501 mV are used.

Therefore, an integral value spectrum in which the number of integral values in a predetermined potential section (=class) in the range of -1362 mV to -2501 mV used for taste diagnosis is changed will be explained.

(I) Number of integral values: 74

Fig. 77 is a diagram showing integral value spectra of Crimson Seedless (skin+flesh), Green Seedless (skin+flesh) and Shine Muscat (skin+flesh) when the number of integral values in a predetermined potential section in the range of -1362 mV to -2501 mV is 74.

Referring to Fig. 77, each of the curves k107 to k109 is a curve CUR created by the diagnostic device 2 using the above-mentioned method in a predetermined potential section ranging from -1362 mV to -2501 mV. The curve k107 shows the integral value spectrum of Crimson Seedless (skin + flesh), the curve k108 shows the integral value spectrum of Green Seedless (skin + flesh), and the curve k109 shows the integral value spectrum of Shine Muscat (skin + flesh).

Fig. 78 is a diagram showing integral value spectra of Crimson Seedless (flesh only), Green Seedless (flesh only), and Shine Muscat (flesh only) when the number of integral values in a predetermined potential section in the range of -1362 mV to -2501 mV is 74.

Referring to Fig. 78, each of the curves k110 to k112 is a curve CUR generated by the diagnostic device 2 using the above-mentioned method in a predetermined potential section ranging from -1362 mV to -2501 mV. The curve k110 shows the integral value spectrum of Crimson Seedless (flesh only), the curve k111 shows the integral value spectrum of Green Seedless (flesh only), and the curve k112 shows the integral value spectrum of Shine Muscat (flesh only).

In Figs. 77 and 78, each of classes 1 to 74 consists of a predetermined potential section of 18.1 mV.

Fig. 79 is a diagram showing the result of judgment as to whether the curves k107 to k112 shown in Fig. 77 and Fig. 78 are or not different from each other.

Whether the curves k107 to k112 are or not different from one another is judged by executing a judgment as to whether any two different curves among the curves k107 to k112 are or not different for all combinations of two different curves among the curves k107 to k112.

There are 15 combinations of two different curves among the six curves k107 to k112: (k107, k108), (k107, k109), (k107, k110), (k107, k111), (k107, k112), (k108, k109), (k108, k110), (k108, k111), (k108, k112), (k109, k110), (k109, k111), (k109, k112), (k110, k111), (k110, k112), and (k111, k112).

Referring to Fig. 79, in the 15 standard deviations $\sigma_{DF_k107,\,k108}$, $\sigma_{DF_k107,\,k109}$, · · · , $\sigma_{DF_k111,\,k112}$ of differences $\sigma_{DF_k107,\,k108}$, $\sigma_{DF_k107,\,k109}$, ··· , $\sigma_{DF_k111,\,k112}$, the minimum value is 8.69% and the maximum value is 89.09%.

Therefore, all of the 15 standard deviations $\sigma_{DF_k107,\,k108}$, $\sigma_{DF_k107,\,k109}$, · · · , $\sigma_{DF_k111,\,k112}$ of the difference are greater than the threshold value $\sigma_{th}$ (=8%).

Therefore, curves k107 to k112 are mutually different curves, and are fingerprints that represent the features based on integral values for Crimson Seedless (skin + flesh), Green Seedless (skin + flesh), Shine Muscat (skin + flesh), Crimson Seedless (flesh only), Green Seedless (flesh only), and Shine Muscat (flesh only), respectively.

(II) Number of integral values: 37

Fig. 80 is a diagram showing integral value spectra of Crimson Seedless (skin+flesh), Green Seedless (skin+flesh) and Shine Muscat (skin+flesh) when the number of integral values in a predetermined potential section in the range of -1362 mV to -2501 mV is 37.

Referring to Fig. 80, each of the curves k113 to k115 is a curve CUR which the diagnostic device 2 created by the method mentioned above in a predetermined potential section in the range of -1362 mV to -2501 mV. The curve k113 shows the integral value spectrum of Crimson Seedless (skin + flesh), the curve k114 shows the integral value spectrum of Green Seedless (skin + flesh), and the curve k115 shows the integral value spectrum of Shine Muscat (skin + flesh).

Fig. 81 is a diagram showing integral value spectra of Crimson Seedless (flesh only), Green Seedless (flesh only), and Shine Muscat (flesh only) when the number of integral values in a s predetermined potential section in the range of -1362 mV to -2501 mV is 37.

Referring to Fig. 81, each of the curves k116 to k118 is a curve CUR which the diagnostic device 2 created by the method mentioned above in a predetermined potential section in the range of -1362 mV to -2501 mV. The curve k116 shows the integral value spectrum of Crimson Seedless (flesh only), the curve k117 shows the integral value spectrum of Green Seedless (flesh only), and the curve k118 shows the integral value spectrum of Shine Muscat (flesh only).

In Figs. 80 and 81, each of classes 1 to 37 consists of a predetermined potential section of 36.2 mV.

Fig. 82 is a diagram showing the result of judgment as to whether or not the curves k113 to k118 shown in Figs. 80 and 81 are different from each other.

Whether or not the curves k113 to k118 are different from one another is judged by executing a judgment as to whether or not any two different curves among the curves k113 to k118 are different for all combinations of two different curves among the curves k113 to k118.

There are 15 possible combinations of two different curves among the six curves k113 to k118: (k113, k114), (k113, k115), (k113, k116), (k113, k117), (k113, k118), (k114, k115), (k114, k116), (k114, k117), (k114, k118), (k115, k116), (k115, k117), (k115, k118), (k116, k117), (k116, k118), and (k117, k118).

Referring to Fig. 82, in the 15 standard deviations $\sigma_{DF_k113, k114}$, $\sigma_{DF_k113, k115}$, · · · , $\sigma_{DF_k117, k118}$ of differences, the minimum value is 8.64% and the maximum value is 115.64%.

Therefore, all of the 15 standard deviations $\sigma_{DF_k113, k114}$, $\sigma_{DF_k113, k115}$, · · · , $\sigma_{DF_k117, k118}$ of differences are greater than the threshold value $\sigma t_h$ (=8%).

Therefore, curves k113 to k118 are mutually different curves, and are fingerprints that represent features based on integral values for Crimson Seedless (skin + flesh), Green Seedless (skin + flesh), Shine Muscat (skin + flesh), Crimson Seedless (flesh only), Green Seedless (flesh only), and Shine Muscat (flesh only), respectively.

(III) Number of integral values: 19

Fig. 83 is a diagram showing integral value spectra of Crimson Seedless (skin+flesh), Green Seedless (skin+flesh) and Shine Muscat (skin+flesh) when the number of integral values in a predetermined potential section in the range of -1362 mV to -2501 mV is 19.

Referring to Fig. 83, each of the curves k119 to k121 is a curve CUR which the diagnostic device 2 created by the method mentioned above in a predetermined potential section in the range of -1362 mV to -2501 mV. Curve k119 shows the integral value spectrum of Crimson Seedless (skin + flesh), curve k120 shows the integral value spectrum of Green Seedless (skin + flesh), and curve k121 shows the integral value spectrum of Shine Muscat (skin + flesh).

Fig. 84 is a diagram showing integral value spectra of Crimson Seedless (flesh only), Green Seedless (flesh only), and Shine Muscat (flesh only) in when the number of integral values in a predetermined potential section in the range of -1362 mV to -2501 mV is 19.

Referring to Fig. 84, each of the curves k122 to k124 is a curve CUR which the diagnostic device 2 created by the method mentioned above in a predetermined potential section in the range of -1362 mV to -2501 mV. Curve k122 shows the integral value spectrum of Crimson Seedless (flesh only), curve k123 shows the integral value spectrum of Green Seedless (flesh only), and curve k124 shows the integral value spectrum of Shine Muscat (flesh only).

In Figs. 83 and 84, each of classes 1 to 18 is made up of a predetermined potential section of 72.4 mV, and class 19 is made up of a predetermined potential section of 36.2 mV.

Fig. 85 is a diagram showing the result of judgment as to whether or not the curves k119 to k124 shown in Fig. 83 and Fig. 84 are different from each other.

Whether or not the curves k119 to k124 are different from one another is judged by executing a judgment as to whether or not any two different curves among the curves k119 to k124 are different for all combinations of two different curves among the curves k119 to k124.

There are 15 combinations of two different curves among the six curves k119 to k124: (k119, k120), (k119, k121), (k119, k122), (k119, k123), (k119, k124), (k120, k121), (k120, k122), (k120, k123), (k120, k124), (k121, k122), (k121, k123), (k121, k124), (k122, k123), (k122, k124), and (k123, k124).

Referring to Fig. 85, in the 15 standard deviations $\sigma_{DF_k119, k120}$, $\sigma_{DF_k119, k121}$, · · · , $\sigma_{DF_k123, k124}$ of differences, the minimum value is 8.54% and the maximum value is 120.43%.

Therefore, all of the standard deviations $\sigma_{DF_k119,k120}$, $\sigma_{DF_k119,k121}$, ··· , $\sigma_{DFk123, k124}$ of the 15 differences are greater than the threshold value $\sigma_{th}$ (=8%).

Therefore, curves k119 to k124 are mutually different curves and are fingerprints representing features based on integral values for Crimson Seedless (skin + flesh), Green Seedless (skin + flesh), Shine Muscat (skin + flesh), Crimson Seedless (flesh only), Green Seedless (flesh only), and Shine Muscat (flesh only).

(IV) Number of integral values: 9

Fig. 86 is a diagram showing integral value spectra of Crimson Seedless (skin+flesh), Green Seedless (skin+flesh) and Shine Muscat (skin+flesh) in when the number of integral values in a predetermined potential section in the range of -1362 mV to -2501 mV is 9.

Referring to Fig. 86, each of the curves k125 to k127 is a curve CUR which the diagnostic device 2 created by the method mentioned above in a predetermined potential section in the range of -1362 mV to -2501 mV. Curve k125 shows the integral value spectrum of Crimson Seedless (skin + flesh), curve k126 shows the integral value spectrum of Green Seedless (skin + flesh), and curve k127 shows the integral value spectrum of Shine Muscat (skin + flesh).

Fig. 87 is a diagram showing integral value spectra of Crimson Seedless (flesh only), Green Seedless (flesh only), and Shine Muscat (flesh only) when the number of integral values in a predetermined potential section in the range of -1362 mV to -2501 mV is 9.

Referring to Fig. 87, each of the curves k128 to k130 is a curve CUR which the diagnostic device 2 created by the method mentioned above in a predetermined potential section in the range of -1362 mV to -2501 mV. Curve k128 shows the integral value spectrum of Crimson Seedless (flesh only), curve k129 shows the integral value spectrum of Green Seedless (flesh only), and curve k130 shows the integral value spectrum of Shine Muscat (flesh only).

In Figs. 86 and 87, each of classes 1 to 8 is made up of a predetermined potential section of 162.9 mV, and class 9 is made up of a predetermined potential section of 36.2 mV.

Fig. 88 is a diagram showing the result of judgment as to whether the curves k125 to k130 shown in Fig. 86 and Fig. 87 are or not different from each other.

Whether or not the curves k125 to k130 are different from one another is judged by executing a judgment as to whether or not any two different curves among the curves k125 to k130 are different for all combinations of two different curves among the curves k125 to k130.

There are 15 combinations of two different curves among the six curves k125 to k130: (k125, k126), (k125, k127), (k125, k128), (k125, k129), (k125, k130), (k126, k127), (k126, k128), (k126, k129), (k126, k130), (k127, k128), (k127, k129), (k127, k130), (k128, k129), (k128, k130), and (k129, k130).

Referring to Fig. 88, in the 15 standard deviations $\sigma_{DF_k125, k126}$, $\sigma_{DF_k125, k127}$, ···, $\sigma_{DF_k129, k130}$ of differences, the minimum value is 8.18% and the maximum value is 129.56%.

Therefore, all of the 15 standard deviations $\sigma_{DF_k125, k126}$, $\sigma_{DF_k125, k127}$, · · · , $\sigma_{DF_k129, k130}$ of the differences are greater than the threshold value $\sigma_{th}$ (=8%).

Therefore, curves k125 to k130 are mutually different curves and are fingerprints that represent features based on integral values for Crimson Seedless (skin + flesh), Green Seedless (skin + flesh), Shine Muscat (skin + flesh), Crimson Seedless (flesh only), Green Seedless (flesh only), and Shine Muscat (flesh only), respectively.

Although not shown in the figures, it was also confirmed that when the number of integral values in a predetermined potential section in the range of -1362 mV to -2501 mV was 25, 15, 13, 11, and 7, the standard deviation of the differences was greater than the threshold value (8%) for all combinations (${}_6C_2$ = 15) in when selecting two different curves from six curves.

As described above, the index curve for uniquely identifying Crimson Seedless (skin + flesh) consists of a plurality of curves k107, k113, k119, and k125 having different numbers of integral values, the index curve for uniquely identifying Green Seedless (skin + flesh) consists of a plurality of curves k108, k114, k120, and k126 having different numbers of integral values, the index curve for uniquely identifying Shine Muscat (skin + flesh) consists of a plurality of curves k109, k115, k121, and k127 having different numbers of integral values, the index curve for uniquely identifying Crimson Seedless (flesh only) consists of a plurality of curves k110, k116, k122, and k128 having different numbers of integral values, the index curve for uniquely identifying Green Seedless (flesh only) consists of a plurality of curves k111, k117, k123, and k129 having different numbers of integral values, and the index curve for uniquely identifying Shine Muscat (flesh only) consists of a plurality of curves k112, k118, k124, and k130 having different numbers of integral values.

As described above, the "astringency" of grapes is calculated by calculating the Body index $(-)_{th}$ shown in formula (10) and substituting the calculated Body index $(-)_{th}$ into equation (11).

The Body index $(-)_{th}$ is calculated based on the sums of the integral values L(-)_sum_th, M(-)_sum_th, and H(-)_sum_th, as shown in equation (10).

The sum L(-)_sum_th of integral values is the sum of integral values in a predetermined potential section in range from -1362 mV to -2501 mV among a plurality of integral values calculated based on a cyclic voltammogram measured by setting the potential scanning rate to the potential scanning rate $V_{r_Low}$, the um M(-)_sum_th of integral values is the sum of integral values in a predetermined potential section ranging in range from - 1362 mV to -2501 mV among a plurality of integral values calculated based on a cyclic voltammogram measured by setting the potential scanning rate to the potential scanning rate $V_{r_Middle}$, and the sum H(-)_sum_th of integral values is the sum of integral values in a predetermined potential section in range from -1362 mV to -2501 mV among a plurality of integral values calculated based on a cyclic voltammogram measured by setting the potential scanning rate to the potential scanning rate $V_{r_High}$.

Fig. 89 is a diagram showing the correspondence relationship between the classes and integral values when the number of integral values is 37 and the correspondence relationship between the classes and integral values when the number of integral values is 74.

Referring to Fig. 89, when the number of integral values in a predetermined potential section in the range of -1362 mV to -2501 mV is 74, the integral values in classes 1 to 74 are $ITG_{1_74}$ to $ITG_{74_74}$, respectively.

When the number of integral values in a predetermined potential section in the range of -1362 mV to -2501 mV is 37, the integral value $ITG_{1_37}$ in class 1 consists of the sum of the integral values $ITG_{1_74}$ and $ITG_{2_74}$ when the number of integral values is 74, the integral value $ITG_{2_37}$ in class 2 consists of the sum of the integral values $ITG_{3_74}$ and $ITG_{4_74}$ when the number of integral values is 74,..., the integral value $ITG_{36_37}$ in class 36 consists of the sum of the integral values $ITG_{71_74}$ and $ITG_{72_74}$ when the number of integral values is 74, and the integral value $ITG_{37_37}$ in class 37 consists of the sum of the integral values $ITG_{73_74}$ and $ITG_{74_74}$ when the number of integral values is 74.

As a result, when the number of integral values is 37, the sum of the 37 integral values is equal to the sum of the 74 integral values when the number of integral values is 74.

In this case, although not shown in the figure, when the number of integral values in a predetermined potential section in the range of -1362 mV to -2501 mV is 19, the sum of the 19 integral values is equal to the sum of the 74 integral values when the number of integral values is 74, and the sum of the 9 integral values when the number of integral values in a predetermined potential section in the range of -1362 mV to -2501 mV is 9 is equal to the sum of the 74 integral values when the number of integral values is 74.

Therefore, the sum $L(-)_sum_th_{74}$ of integral values that is the sum L(-)_sum_th of integral values in when the number of integral values in a predetermined potential section in the range of -1362 mV to -2501 mV is 74, the sum $L(-)_sum_th_{37}$ of integral values that is the sum L(-)_sum_th of integral values in when the number of integral values in a predetermined potential section in the range of -1362 mV to -2501 mV is 37, the sum $L(-)_sum_th_{19}$ of integral values that is the sum L(-)_sum_th of integral values in when the number of integral values in a predetermined potential section in the range of -1362 mV to -2501 mV is 19, and the sum $L(-)_sum_th_{9}$ of integral values that is the sum L(-)_sum_th of integral values in when the number of integral values in a predetermined potential section in the range of -1362 mV to -2501 mV is 9, are equal to each other.

Similarly, the sum $M(-)_sum_th_{74}$ of integral values that is the sum M(-)_sum_th of integral values in when the number of integral values in a predetermined potential section in the range of -1362 mV to -2501 mV is 74, the sum $M(-)_sum_th_{37}$ of integral values that is the sum M(-)_sum_th of integral values in when the number of integral values in a predetermined potential section in the range of -1362 mV to -2501 mV is 37, the sum $M(-)_sum_th_{19}$ of integral values that is the sum M(-)_sum_th of integral values in when the number of integral values in a predetermined potential section in the range of -1362 mV to -2501 mV is 19, and the sum $M(-)_sum_th_{9}$ of integral values that is the sum M(-)_sum_th of integral values in when the number of integral values in a predetermined potential section in the range of -1362 mV to -2501 mV is 9, are equal to each other, and the sum $H(-)_sum_th_{74}$ of integral values that is the sum H(-)_sum_th of integral values in when the number of integral values in a predetermined potential section in the range of -1362 mV to -2501 mV is 74, the sum $H(-)_sum_th_{37}$ of integral values that is the sum H(-)_sum_th of integral values in when the number of integral values in a predetermined potential section in the range of -1362 mV to -2501 mV is 37, the sum $H(-)_sum_th_{19}$ of integral values that is the sum H(-)_sum_th of integral values in when the number of integral values in a predetermined potential section in the range of -1362 mV to -2501 mV is 19, and the sum $H(-)_sum_th_{9}$ of integral values that is the sum H(-)_sum_th of integral values in when the number of integral values in a predetermined potential section in the range of -1362 mV to -2501 mV is 9, are equal to each other.

As a result, in a predetermined potential section in the range of -1362 mV to -2501 mV, Body index $(-)_{th_74}$ in when the number of integral values is 74, Body index $(-)_{th_37}$ in when the number of integral values is 37, Body index $(-)_{th_19}$ in when the number of integral values is 19, and Body index $(-)_{th_9}$ in when the number of integral values is 9 are equal to each other.

In this case, even if the number of integral values changes, the coefficient $k_9$ shown in equation (11) does not change, so the "astringency" of grapes in when the number of integral values in a predetermined potential section in the range of -1362 mV to -2501 mV is 74, the "astringency" of grapes in when the number of integral values in a predetermined potential section in the range of -1362 mV to -2501 mV is 37, the "astringency" of grapes in when the number of integral values in a predetermined potential section in the range of -1362 mV to -2501 mV is 19, and the "astringency" of grapes in when the number of integral values in a predetermined potential section in the range of -1362 mV to -2501 mV is 9 are equal to each other.

For the same reason, when the number of integral values in a predetermined potential section in the range of -1362 mV to -2501 mV is 25, 15, 13, 11, and 7, also "the plurality of astringency" of the grapes are equal to each other.

Therefore, the diagnostic device 2 may perform a taste diagnosis of "grapes" by the above-mentioned method based on an integral value spectrum in which the number of integral values in a predetermined potential section in the range of -1362 mV to -2501 mV is any one of 7, 9, 11, 13, 15, 19, 25, 37, and 74.

Fig. 90 is a flowchart for explaining the operation of the diagnostic system 10 shown in Fig. 1.

Referring to Fig. 90, when the operation of the diagnostic system 10 is started, the supply unit 121 of the sensor device 1 receives the potential scanning range V_s and the potential scan rates $V_{r_Low}$, $V_{r_Middle}$, and $V_{r_High}$ input to the measuring instrument 12 by the user of the sensor device 1 (step S1).

In addition, the supply unit 121 of the sensor device 1 accepts a start signal input to the measuring instrument 12 by the user of the sensor device 1, and the measuring unit 122 of the sensor device 1 accepts an end signal input to the measuring instrument 12 by the user of the sensor device 1.

Then, when the supply unit 121 of the sensor device 1 received the start signal, the supply unit 121 applies a potential V of the potential scanning range V_s to the solution while changing the potential applied to the solution (analyte) at scan speeds of $V_{r_Low}$, $V_{r_Middle}$, and $V_{r_High}$, and the measuring unit 122 measures the potential V of the working electrode 112 based on the potential of the reference electrode 114 and measures the current value I from the counter electrode 113 to measure the current-potential characteristics [I-V]_Low, [I-V]_Middle, and [I-V]_High of the cyclic voltammogram (step S2).

In this case, the supply unit 121 applies a potential V of the potential scanning range V_s to the solution while changing the potential V at the scan speed $V_{r_Low}$, and the measurement unit 122 measures the potential V of the working electrode 112 based on the potential of the reference electrode 114, and measures the current value I from the counter electrode 113 to measure the current-potential characteristic [I-V]_Low of the cyclic voltammogram.

In addition, the supply unit 121 applies a potential V in a potential scanning range V_s to the solution while changing the potential V at the scan speed $V_{r_Middle}$, and the measurement unit 122 measures the potential V of the working electrode 112 based on the potential of the reference electrode 114, and measures the current value I from the counter electrode 113 to measure the current-potential characteristic [I-V]_Middle of the cyclic voltammogram.

Furthermore, the supply unit 121 applies a potential V of a potential scanning range V_s to the solution while changing the potential V at the scan speed $V_{r_High}$, and the measurement unit 122 measures the potential V of the working electrode 112 based on the potential of the reference electrode 114, and measures the current value I from the counter electrode 113 to measure the current-potential characteristic [I-V]_High of the cyclic voltammogram.

After the step S2, the measurement unit 122 creates measurement data MRS including the correspondence between the current values $I_{I_Low}$ to $I_{d_Low}$ and the potentials $V_{I_Low}$ to $V_{d_Low}$ in the current-potential characteristics [I-V] _Low, the correspondence between the current values $I_{I_Middle}$ to $I_{d_Middle}$ and the potentials $V_{I_Middle}$ to $V_{d_Middle}$ in the current-potential characteristics [I-V]_Middle, and the correspondence between the current values $I_{I_High}$ to $I_{d_High}$ and the potentials $V_{I_High}$ to $V_{d_High}$ in the current-potential characteristics [I-V]_High (step S3).

Then, the measuring unit 122 judges whether or not to end the measurement of the cyclic voltammogram of the solution (step S4).

In this case, when the measurement unit 122 receives an end signal input to the measuring instrument 12 by the user of the sensor device 1, the measurement unit 122 judges to end the measurement, and when the measurement unit 122 does not receive the end signal, the measurement unit 122 judges not to end the measurement.

When it is judged in step S4 that the measurement of the cyclic voltammogram of the solution is not to be terminated, the operation of the sensor device 1 transitions to the step S1, and steps S1 to S4 are repeatedly executed until it is judged in the step S4 that the measurement of the cyclic voltammogram of the solution is to be terminated.

In this case, each time it is judged in step S4 that the measurement should not be terminated, the sensor 11 used to measure the cyclic voltammogram is discarded, and a sensor 11 not used to measure the cyclic voltammogram is attached to the measuring instrument 12, and the above-mentioned steps S1 to S4 are executed sequentially.

Then, when it is judged in step S4 that the measurement of the cyclic voltammogram of the solution is to be terminated, the measuring unit 122 of the sensor device 1 outputs m (m is an integer equal to or greater than 1) pieces of measurement data MRS_1 to MRS_m that have been created in when it is judged that the measurement of the cyclic voltammogram of the solution is to be terminated to the transmitting unit 123. The transmitting unit 123 receives the m pieces of measurement data MRS_1 to MRS_m from the measuring unit 122, and transmits the received m pieces of measurement data MRS_1 to MRS_m to the diagnostic device 2 by wired communication or wireless communication (step S5).

The receiving unit 211 of the diagnostic device 2 receives the m pieces of measurement data MRS_1 to MRS_m from the transmitting unit 123 of the sensor device 1 by wired communication or wireless communication (step S6), and outputs the received m pieces of measurement data MRS_1 to MRS_m to the control unit 212.

The control unit 212 of the diagnostic device 2 receives the m pieces of measurement data MRS_1 to MRS_m from the receiving unit 211. Then, the control unit 212 creates m pieces of analysis data $ALY_D_1$ to $ALY_D_m$ based on the m pieces of measurement data MRS_1 to MRS_m, and updates the m pieces of analysis data $ALY_D_1$ to $ALY_D_m$ to m pieces of index data $IDX_1$ to $IDX_m$ (step S7).

Then, the control unit 212 outputs the m pieces of index data $IDX_1$ to $IDX_m$ to a diagnostic unit (comprised of a calculation unit 216 and a taste diagnostic unit 217).

The diagnostic unit (comprised of the calculation unit 216 and the taste diagnostic unit 217) receives the m pieces of index data $IDX_1$ to $IDX_m$ from the control unit 212. The diagnostic unit (comprised of the calculation unit 216 and the taste diagnostic unit 217) then detects m pieces of calculation data $CAL_1$ to $CAL_m$ from the m pieces of index data $IDX_1$ to $IDX_m$, respectively, and diagnoses the tastes of the m analyte based on the detected m pieces of calculation data $CAL_1$ to $CAL_m$ (step S8). This completes the operation of the diagnostic system 10.

Fig. 91 is a flowchart for explaining the detailed operation of step S7 in Fig. 90.

Referring to Fig. 91, after the step S6 in Fig. 90, the control unit 212 judges whether a plurality of measurement data have been or not received from the receiving unit 211 (step S71).

In this case, when the control unit 212 has not received the plurality of measurement data from the receiving unit 211, the control unit 212 judges that the control unit 212 has received measurement data MRS_uni from the receiving unit 211, and when the control unit 212 have received the plurality of measurement data from the receiving unit 211, the control unit 212 judges that the control unit 212 has received P pieces of measurement data MRS_1 to MRS_P (multiple measurement data MRS) from the receiving unit 211.

When it is judged in step S71 that the plurality of measurement data have not been received, the control unit 212 creates one piece of analysis data $ALY_D_{uni}$ based on one piece of measurement data MRS_uni using the method described above (step S72), stores the created analysis data $ALY_D_{uni}$ in the database 22, and outputs the analysis data $ALY_D_{uni}$ to the calculation unit 213.

The calculation unit 213 receives the analysis data $ALY_D_{uni}$ from the control unit 212. Then, the calculation unit 213 and the creation unit 215 create a curve $CUR_{uni}$ indicating the class dependency of the integral value as an index curve based on the analysis data $ALY_D_{uni}$ (step S73).

Then, the creation unit 215 creates an analysis result $ALY_RLS_{uni}$= [$ID_{uni}$/ $CAL_{uni}$/$CUR_{uni}$] that associates the identification information $ID_{uni}$, the calculation data $CAL_{uni}$, and the curve $CUR_{uni}$, and outputs the created analysis result $ALY_RLS_{uni}$= [$ID_{uni}$/ $CAL_{uni}$/ $CUR_{uni}$] to the control unit 212.

The control unit 212 receives the analysis result $ALY_RLS_{uni}$ from the creation unit 215, updates the analysis data $ALY_D_{uni}$ to index data $IDX_{uni}$ based on the received analysis result $ALY_RLS_{uni}$ (step S74), and stores the updated index data $IDX_{uni}$ in the database 22 in place of the analysis data $ALY_D_{uni}$.

On the other hand, when it is judged in step S71 that a plurality of measurement data have been received, the control unit 212 creates P pieces of analysis data $ALY_D_1$ to $ALY_D_P$ based on the P pieces of measurement data MRS_1 to MRS_P by the method described above (step S75), stores the created P pieces of analysis data $ALY_D_1$ to $ALY_D_P$ in the database 22, and outputs the P pieces of analysis data $ALY_D_1$ to $ALY_D_P$ to the calculation unit 213.

The calculation unit 213 receives the P pieces of analysis data $ALY_D_1$ to $ALY_D_P$ from the control unit 212. Then, the calculation unit 213 and the creation unit 215 create P curves $CUR_1$ to $CUR_P$ indicating the class dependency of the integral value based on the P pieces of analysis data $ALY_D_1$ to $ALY_D_P$ as P index curves of the P analytes (step S76).

Then, the judgment unit 214 creates a judgment result (judgment result shown in Table 1) indicating whether the P curves $CUR_1$ to $CUR_P$ are different or not (step S77), and outputs the created judgment result (judgment result shown in Table 1) to the creation unit 215.

When the creation unit 215 receives the judgment results (the judgment results shown in Table 1) from the judgment unit 214, the creation unit 215 adds P curves $CUR_1$ to $CUR_P$ to the P calculation results $CAL_RLS_1$=[$ID_1$/$CAL_1$] to CAL_RLSP=[$ID_P$/$CAL_P$], respectively, to create P analysis results $ALY_RLS_1$ = [$ID_1$/$CAL_1$/$CUR_1$] to $ALY_RLS_P$ = [$ID_P$/$CAL_P$/$CUR_P$]. Then, the creation unit 215 outputs the P analysis results $ALY_RLS_1$ to $ALY_RLS_P$ and the judgment results (the judgment results shown in Table 1) to the control unit 212.

Then, when the control unit 212 receives the P analysis results $ALY_RLS_1$ to $ALY_RLS_P$ and the judgment results (the judgment results shown in Table 1) from the creation unit 215, the control unit 212 updates the P analysis data $ALY_D_1$ to $ALY_D_P$ to P index data $IDX_1$ to $IDX_P$, respectively, based on the P analysis results $ALY_RLS_1$ to $ALY_RLS_P$ (step S78), and stores the updated P index data $IDX_1$ to $IDX_P$ in the database 22 in place of the P analysis data $ALY_D_1$ to $ALY_D_P$, respectively.

In this case, the control unit 212 detects the identification information $ID_p$, the calculation data $CAL_p$, and the curve $CUR_p$ from the analysis result $ALY_RLS_p$ (p is any of 1 to P), reads out the analysis data $ALY_D_p$ having the same identification information as the detected identification information $ID_p$ from the database 22, and updates the analysis data $ALY_D_p$ to index data $IDX_p$ by adding the calculation data $CAL_p$ and the curve $CUR_p$ to the read analysis data $ALY_D_p$, performing this process for all of P analysis data $ALY_D_1$ to $ALY_D_P$.

As a result, the P pieces of analysis data $ALY_D_1$ to $ALY_D_P$ are updated to the P pieces of index data $IDX_1$ to $IDX_P$, respectively.

Then, the control unit 212 stores the P index data $IDX_1$ to $IDX_P$ in the database 22 in place of the P analysis data $ALY_D_1$ to $ALY_D_P$, respectively, and stores the judgment result (the judgment result shown in Table 1) in the database 22 in association with the P index data $IDX_1$ to $IDX_P$.

After step S74 or step S78, the operation of the diagnostic device 2 proceeds to step S8 in Fig. 90.

According to the flowchart shown in Fig. 91, when the diagnostic device 2 judges in step S71 that multiple measurement data have not been received, the diagnostic device 2 creates one curve $CUR_{uni}$ (see step S73), and when the diagnostic device 2 judges in step S71 that multiple measurement data have been received, the diagnostic device 2

creates P curves $CUR_1$ to $CUR_P$ and the judgment result (the judgment result shown in Table 1) indicating whether the P curves $CUR_1$ to $CUR_P$ are or not different (see steps S76 and S77).

Therefore, the diagnostic device 2 can create the curve $CUR_{uni}$ (or P curves $CUR_1$ to $CUR_P$) as an index curve that serves as an index when identifying the analyte.

Fig. 92 is a flowchart for explaining the detailed operation of step S73 in Fig. 91

In Fig. 92, the potential scanning speed $V_r$ is represented by "S." Furthermore, "S=1" represents the potential scanning speed $V_{r_Low}$, "S=2" represents the potential scanning speed $V_{r_Middle}$, and "S=3" represents the potential scanning speed $V_{r_High}$.

Referring to Fig. 92, after step S72 in Fig. 91, the calculation unit 213 receives one piece of analysis data ALY_$D_{uni}$ from the control unit 212 (step S731).

Then, the calculation unit 213 sets S=1 (step S732) and sets k=1 (step S733). Here, k is an argument representing the predetermined potential section.

After step S733, the calculation unit 213 detects N combinations (Iox_1_k_S, Ird_1_k_S) to (Iox_N_k_S, Ird_N_k_S) of N current values {Iox_1_k S to Iox_N_k_S} of the oxidation wave and N current values {Ird_1_k_S to Ird_N_k_S} of the reduction wave in the predetermined potential range $V_k$ from the current-potential characteristic $(I_S\text{-}V_S)_{uni}$ of the analysis data ALY_$D_{uni}$ (step S734).

Here, N represents the total number of unit potentials (for example, 1 mV) in one predetermined potential section $V_k$.

Also, for example, when one predetermined potential section $V_k$ is [0 to 100 mV], since the current-potential characteristic $(I_S\text{-}V_S)_{uni}$ includes a current value $I_{0\rightarrow 100}$ in when the potential V was scanned from 0 mV to 100 mV and a current value $I_{100\rightarrow 0}$ in when the potential V was scanned from 100 mV to 0 mV, the calculation unit 213 detects the current value $I_{0\rightarrow 100}$ in when the potential V was scanned from 0 mV to 100 mV as [N current values {Iox_1_k_S to Iox_N_k_S} of oxidation wave], and detects the current value $I_{100\rightarrow 0}$ in when the potential V is scanned from 100 mV to 0 mV as [N current values {Ird_1_k_S to Ird_N_k_S} of reduction wave], and N combinations (Iox_1_k_S, Ird_1_k_S) to (Iox_N_k_S, Ird_N_k_S). The same is applied to the case where one predetermined potential section $V_k$ is other than [0 to 100 mV].

After step S734, the calculation unit 23 sets $n_{uip}$=1 (step S735). Here, $n_{uip}$ is an argument representing each of the N unit potentials in one predetermined potential section $V_k$.

After step S735, the calculation unit 213 subtracts the current value (Ird_$n_{uip}$_k_S) of the reduction wave from the current value (Iox_$n_{uip}$_k_S) of the oxidation wave to calculate the subtraction result (R_sbt_$n_{uip}$_k_S) (step S736).

Then, the calculation unit 213 judges whether or not $n_{uip}$=N (step S737).

When it is judged in step S737 that $n_{uip}$=N is not satisfied, the calculation unit 213 sets $n_{uip}$=$n_{uip}$+1 (step S738). Thereafter, the operation of the diagnostic device 2 proceeds to step S736, and steps S736 to S738 are executed repeatedly until it is judged in step S737 that $n_{uip}$=N is satisfied.

If it is judged in step S737 that $n_{uip}$=N, the calculation unit 213 adds up the N subtraction results (R_sbt_1_k_S to R_sbt_N_k_S) to calculate the integral value $ITG_{k_S}$ in the predetermined potential section $V_k$ (step S739).

Thereafter, the calculation unit 213 sets the predetermined potential section $V_k$ as the class Cls_k_S, and creates a combination (Cls_k_S, $ITG_{k_S}$) of the class Cls_k_S and the integral value $ITG_{k_S}$ (step S740).

Then, the calculation unit 213 judges whether or not k=n holds (step S741). Here, n is the total number of the predetermined potential sections $V_k$.

When it is judged in step S741 that k=n is not true, the calculation unit 213 sets k=k+1 (step S742). After that, the operation of the diagnostic device 2 proceeds to step S734, and steps S734 to S742 are repeatedly executed until it is judged in step S741 that k=n is true.

Then, when it is judged in step S741 that k=n is true, the calculation unit 213 generates n combinations (Cls_1_S, $ITG_{1_S}$) to (Cls_n_S, $ITG_{m_S}$) (step S743).

Then, the calculation unit 213 judges whether S=3 is or not true (step S744).

When it is judged in step S744 that S is not 3, the calculation unit 213 sets S=S+1 (step S745). After that, the operation of the diagnostic device 2 proceeds to step S733. Then, steps S733 to S745 are repeatedly executed until it is judged in step S744 that S=3 is true.

Then, if it is judged in step S744 that S=3 is true, the calculation unit 213 adds up the three integral values $ITG_{1_1}$, $ITG_{1_2}$, and $ITG_{1_3}$ in class Cls_1 to calculate the sum $ITG_{1_1}+ITG_{1_2}+ITG_{1_3}$ of the integral values , adds up the three integral values $ITG_{2_1}$, $ITG_{2_2}$, and $ITG_{2_3}$ in class Cls_2 to calculate the sum $ITG_{2_1}+ITG_{2_2}+ITG_{2_3}$ of the integral values, and similarly adds up the three integral values $ITG_{n_1}$, $ITG_{n_2}$, and $ITG_{n_3}$ in class Cls_n to calculate the sum $ITG_{n_1}+ITG_{n_2}+ITG_{n_3}$ of the integral values.

Then, the calculation unit 213 generates n combinations (Cls_1, $ITG_{1_1}+ITG_{1_2}+ITG_{1_3}$), (Cls_2, $ITG_{2_1}+ITG_{2_2}+ITG_{2_3}$), . . . , (Cls_n, $ITG_{n_1}+ITG_{n_2}+ITG_{n_3}$) (step S746).

Then, the calculation unit 213 creates the calculation data CAL consisting of n combinations (Cls_1, $ITG_{1_1}+ITG_{1_2}+ITG_{1_3}$), (Cls_2, $ITG_{2_1}+ITG_{2_2}+ITG_{2_3}$), ..., (Cls_n, $ITG_{n_1}+ITG_{n_2}+ITG_{n_3}$) and outputs a calculation result CAL_RLS = [identification information ID/calculation data CAL] in which the calculation data CAL is corresponded to the identification information ID of the analyte to the creation unit 215.

The creation unit 215 receives the calculation result CAL_RLS=[identification information ID/calculated data CAL] from the calculation unit 213. Then, the creation unit 215 detects n combinations (Cls_1, $ITG_{1_1}+ITG_{1_2}+ITG_{1_3}$), (Cls_2, $ITG_{2_1}+ITG_{2_2}+ITG_{2_3}$), ..., (Cls_n, $ITG_{n_1}+ITG_{n2}+ITG_{n_3}$) from the calculation data CAL of the calculation result CAL_RLS, plots the detected n combinations (Cls_1, $ITG_{1_1}+ITG_{1_2}+ITG_{1_3}$), (Cls_2, $ITG_{2_1}+ITG_{2_2}+ITG_{2_3}$),..., (Cls_n, $ITG_{n_1}+ITG_{n2}+ITG_{n_3}$) to create a curve CUR indicating the dependency of the integral value on the class (step S747).

After step S747, the operation of the diagnostic device 2 proceeds to step S74 in Fig. 91.

In the flowchart shown in Fig. 92, the calculation unit 213 calculates n integral values $ITG_{1_1}$ to $ITG_{n_1}$ in n classes Cls_1_1 to Cls_n_1 based on the cyclic voltammogram measured at the potential scanning speed $V_{r_Low}$ to create n combinations (Cls_1_1, $ITG_{1_1}$) to (Cls_n_1, $ITG_{n_1}$) of classes and integral values, the calculation unit 213 calculates n integral values $ITG_{2_1}$ to $ITG_{n_2}$ in n classes Cls_1_2 to Cls_n_2 based on the cyclic voltammogram measured at the potential scanning speed $V_{r_Middle}$ to create n combinations (Cls_1_2, $ITG_{1_2}$) to (Cls_n_2, $ITG_{n_2}$) of classes and integral values, and the calculation unit 213 calculates n integral values $ITG_{1_3}$ to $ITG_{n_3}$ in n classes Cls_1_3 to Cls_n_3 based on the cyclic voltammogram measured at the potential scanning speed $V_{r_High}$ to create n combinations (Cls_1_3, $ITG_{1_3}$) to (Cls_n_3, $ITG_{n_3}$) of classes and integral values.

Then, the calculation unit 213 executes for all of n of classes to calculate the sum ($ITG_{k_1}+ITG_{k_2}+ITG_{k_3}$) of the three integral values $ITG_{k_1}$, $ITG_{k_2}$, $ITG_{k_3}$ in one class k (=predetermined potential section $V_k$) based on the n combinations (Cls_1_1, $ITG_{1_1}$) to (Cls_n_1, $ITG_{n_1}$), n combinations (Cls_1_2, $ITG_{1_2}$) to (Cls_n_2, $ITG_{n_2}$), and n combinations (Cls_1_3, $ITG_{1_3}$) to (Cls_n_3, $ITG_{n_3}$) to calculate the n sums ($ITG_{1_1}+ITG_{1_2}+ITG_{1_3}$) to ($ITG_{n_1}+ITG_{n_2}+ITG_{n_3}$).

Then, the calculation unit 213 generates n combinations (Cls_1, $ITG_{1_1}+ITG_{1_2}+ITG_{1_3}$), (Cls_2, $ITG_{2_1}+ITG_{2_2}+ITG_{2_3}$), ..., (Cls_n, $ITG_{n_1}+ITG_{n_2}+ITG_{n_3}$) based on n classes Cls_1 to Cls_n and n sums ($ITG_{1_1}+ITG_{1_2}+ITG_{1_3}$) to ($ITG_{n_1}+ITG_{n2}+ITG_{n_3}$) (see step S746), and the calculation unit 213 outputs the generated n combinations (Cls_1, $ITG_{1_1}+ITG_{1_2}+ITG_{1_3}$), (Cls_2, $ITG_{2_1}+ITG_{2_2}+ITG_{2_3}$), ..., (Cls_n, $ITG_{n_1}+ITG_{n_2}+ITG_{n_3}$) to the creation unit 215.

The creation unit 215 receives n combinations (Cls_1, $ITG_{1_1}+ITG_{1_2}+ITG_{1_3}$), (Cls_2, $ITG_{2_1}+ITG_{2_2}+ITG_{2_3}$), ..., (Cls_n, $ITG_{n_1}+ITG_{n2}+ITG_{n_3}$) from the calculation unit 213, and plots the received n combinations (Cls_1, $ITG_{1_1}+ITG_{1_2}+ITG_{1_3}$), (Cls_2, $ITG_{2_1}+ITG_{2_2}+ITG_{2_3}$), ..., (Cls_n, $ITG_{n_1}+ITG_{n_2}+ITG_{n_3}$) to create a curve CUR indicating the class dependency of the integral value (see step S747).

Fig. 93 is a flowchart for explaining the detailed operation of step S72 in Fig. 91.

Referring to Fig. 93, when it is judged in step S71 of Fig. 91 that a plurality of measurement data have not been received, the control unit 212 of the diagnostic device 2 receives the measurement data MRS_uni from the receiving unit 211. Then, the control unit 212 refers to a timer to detect the time $t_{uni}$ at which the measurement data MRS_uni was received (step S721), and issues identification information $ID_{uni}$ for identifying the measurement data MRS_uni (step S722).

Then, the control unit 212 detects the name $ALY_Na_{uni}$ of the analyte, the type $ALY_Kd_{uni}$ of the analyte, the potential scanning speeds $V_{r_Low_uni}$, $V_{r_Middle_uni}$, $V_{r_High_uni}$, and the current-potential characteristics $(I_{Low}-V_{Low})_{uni}$, $(I_{Middle}-V_{Middle})_{uni}$, and $(I_{High}-V_{High})_{uni}$ from the measurement data MRS_uni (step S723).

Then, the control unit 212 creates measurement data MRS_Low_uni in which the potential scanning speed $V_{r_Low_uni}$ and the current-potential characteristic $(I_{Low}-V_{Low})_{uni}$ are corresponded to each other (step S724).

Furthermore, the control unit 212 creates measurement data MRS_Middle_uni in which the potential scanning speed $V_{r_Middle_uni}$ and the current-potential characteristic $(I_{Middle}-V_{Middie})_{uni}$ are associated with each other (step S725).

Furthermore, the control unit 212 creates measurement data MRS_High_uni in which the potential scanning speed $V_{r_High-uni}$ and the current-potential characteristic $(I_{High}-V_{High})_{uni}$ are associated with each other (step S726).

Then, the control unit 212 creates analysis data $ALY_D_{uni}$ = [$t_{uni}$/$ID_{uni}$/$ALY_Na_{uni}$/ $ALY_Kd_{uni}$/MRS_Low_uni/MRS_Middle_uni/MRS_High_uni] that corresponds the time $t_{uni}$, identification information $ID_{uni}$, name of the analyte $ALY_Na_{uni}$, type of the analyte $ALY_Kd_{uni}$, and measurement data MRS_Low_uni, MRS_Middle_uni, and MRS_High_uni to each other (step S727).

After step S727, the operation of the diagnostic device 2 proceeds to step S73 in Fig. 91.

Fig. 94 is a flowchart for explaining the detailed operation of step S75 in Fig. 1.

Referring to Fig. 94, when it is judged in step S71 of Fig. 91 that a plurality of measurement data have been received, the control unit 212 of the diagnostic device 2 receives P pieces of measurement data MRS_1 to MRS_P from the receiving unit 211. Then, the control unit 212 sets p=1 (step S751). Here, p is an argument representing each of the P pieces of measurement data MRS_1 to MRS_P, and p=1 to P.

After step S751, the control unit 212 refers to the timer to detect the time $t_p$ at when the measurement data MRS_p was received (step S752), and issues identification information $ID_p$ for identifying the measurement data MRS_p (step S753).

Then, the control unit 212 detects the name $ALY_Na_p$ of the analyte, the type $ALY_Kd_p$ of the analyte, the potential scanning speeds $V_{r_Low_p}$, $V_{r_Middle_p}$, $V_{r_High_p}$, and the current-potential characteristics $(I_{Low}-V_{Low})_p$, $(I_{Mi-}$

$_{ddle}$-$V_{Middle})_p$, $(I_{High}$-$V_{High})_p$ from the measurement data MRS_p (step S754).

Then, the control unit 212 creates measurement data MRS_Low_p in which the potential scanning speed $V_{r_Low_p}$ and the current-potential characteristic $(I_{Low}$-$V_{Low})_p$ are associated with each other (step S755).

Furthermore, the control unit 212 creates measurement data MRS_Middle_p in which the potential scanning speed $V_{r_Middle_p}$ and the current-potential characteristic $(I_{Middle}$ -$V_{Middle})_p$ are associated with each other (step S756).

Furthermore, the control unit 212 creates measurement data MRS_High_p in which the potential scanning speed $V_{r_High_p}$ and the current-potential characteristic $(I_{High}$ -$V_{High})_p$ are associated with each other (step S757).

The control unit 212 then creates analysis data ALY_$D_p$ = [$t_p$/ $ID_p$/ ALY_$Na_p$/ ALY_$Kd_p$/ MRS_Low_p/MRS_Middle_p/MRS_High_p] that the time $t_p$, the identification information $ID_p$, the name ALY_$Na_p$ of the analyte, the type ALY_$Kd_p$ of analyte, and the measurement data MRS_Low_p, MRS_Middle_p, and MRS_High_p are associated with each other (step S758).

Then, the control unit 212 judges whether p=P or not (step S759).

When it is judged in step S759 that p=P is not satisfied, control unit 212 sets p=p+1 (step S760). Thereafter, the operation of diagnostic device 2 proceeds to step S752, and steps S752 to S760 are repeatedly executed until it is judged in step S759 that p=P is satisfied.

Then, if it is judged in step S759 that p=P, the operation of the diagnostic device 2 proceeds to step S76 in Fig. 91.

In the flowchart shown in Fig. 94, when it was judged in step S759 that p=P, P pieces of analysis data ALY_$D_1$ to ALY_$D_P$ have been created.

Fig. 95 is a flowchart for explaining the detailed operation of step S76 in Fig. 91.

Referring to Fig. 95, after step S75 in Fig. 91, the calculation unit 213 receives P pieces of analysis data ALY_$D_1$ to ALY_$D_P$ from the control unit 212 (step S761).

Then, the calculation unit 213 sets p=1 (step S762). Here, p is an argument representing each of the P pieces of analysis data ALY_$D_1$ to ALY_$D_P$.

After step S762, the calculation unit 213 and the creation unit 215 sequentially execute steps S731 to S747 in Fig. 92 based on the analysis data ALY_$D_p$ to create the curve $CUR_p$ as the p-th index curve (step S763).

In this case, in step S731 of Fig. 92, "analysis data ALY_$D_{uni}$" is replaced with "analysis data ALY_$D_p$", and in step S734 of Fig. 92, "analysis data ALY_$D_{uni}$" is replaced with "analysis data ALY_$D_p$", and "current-potential characteristic $(I-V)_{uni}$" is replaced with "current-potential characteristic $(I-V)_p$".

After the step 763, the calculation unit 213 judges whether p=P or not (step S764).

When it is judged in step S764 that p=P is not satisfied, the calculation unit 213 sets p=p+1 (step S765). Thereafter, the operation of the diagnostic device 2 proceeds to step S763, and steps S763 to S765 are executed repeatedly until it is judged in step S764 that p=P is satisfied.

Then, if it is judged in step S764 that p=P, the operation of the diagnostic device 2 proceeds to step S77 in Fig. 91.

According to the flowchart shown in Fig. 95, when it is judged in step S764 that p=P, P curves $CUR_1$ to $CUR_P$ have been created as P index curves for P analytes, respectively.

Fig. 96 is a flowchart for explaining the detailed operation of step S77 in Fig. 91.

Referring to Fig. 96, after step S76 in FIG. 91, the judgment unit 214 receives P pieces of calculation data $CAL_1$ to $CAL_P$ for creating P pieces of curves $CUR_1$ to $CUR_P$ from the calculation unit 213 (step S771).

Then, the judgment unit 214 selects Z ($Z={}_PC_2$) sets of two pieces of calculation data {$CAL_i$, $CAL_j$ (i≠j)}_1 to {$CAL_i$, $CAL_j$ (i≠j)}_Z from the P pieces of calculation data $CAL_1$ to $CAL_P$ (step S772). Here, the two pieces of calculation data $CAL_i$, $CAL_j$ are different calculation data.

After step S772, the judgment unit 214 sets z=1 (step S773). Here, z is an argument representing each of Z sets of two calculation data {$CAL_i$, $CAL_j$ (i≠j)}_1 to {$CAL_i$, $CAL_j$ (i≠j)}_Z, respectively.

After step S773, the judgment unit 214 judges whether the two pieces of calculation data {$CAL_i$, $CAL_j$ (i≠j)}_z are or not different based on the two pieces of calculation data {$CAL_i$, $CAL_j$ (i≠j)}_z, and creates a judgment result $JDGR_z$ (step S774).

In this case, the judgment unit 214 judges whether the two pieces of calculation data {$CAL_i$, $CAL_j$ (i≠j)}_z are or not different by the following method.

Each of the two pieces of calculation data {$CAL_i$, $CAL_j$ (i≠j)}_z has the same configuration as the calculation data $CAL_{uni}$ shown in Fig. 12.

As a result, the calculation data $CAL_i$_z includes the sums $\{ITG_{1_Low}+ ITG_{1_Middle}+ITG_{1_High}\}_i$_z to $\{ITG_{n_Low}+ ITG_{n_Middle}+ ITG_{n_Highl}\}_i$_z of n integral values corresponding to the n classes $Cls_1$ to $Cls_n$, respectively. Also, the calculation data CAL _z includes the sums $\{ITG_{1_Low}+ITG1_{_Middle}+ITG_{1_High}\}_j$_z to $\{ITG_{n_Low}+ITG_{n_Middle}+ ITG_{n_High}\}_j$_z of n integral values corresponding to the n classes $Cls_1$ to $Cls_n$, respectively.

The judgment unit 214 executes, for all of g=1 to n, calculating the difference $DF_g$ between the sum $\{ITG_{g_Low} + ITG_{g_Middle} + ITG_{g_High}\}$i_z of the integral values and the sum $\{ITG_{g_Low} + ITG_{g_Middle} + ITG_{g_High}\}$j_z of the integral values in one class $Cls_g$ (g =1 to n) to calculate n differences $DF_1$ to $DF_n$.

Then, the judgment unit 214 calculates the standard deviation $\sigma_{i,j}$ of the n differences $DF_1$ to $DF_n$.

Then, when the standard deviation $\sigma_{i,j}$ is greater than the threshold value $\sigma_{th}$, the judgment unit 214 judges that the two calculated data {$CAL_i$, $CAL_j$(i≠j)}_z are different, and when the standard deviation $\sigma_{i,j}$ is equal to or less than the threshold value $\sigma_{th}$, the judgment unit 214 judges that the two calculated data {$CAL_i$, $CAL_j$(i≠j)}_z are not different.

Therefore, the judgment result $JDGR_z$ includes a judgment result (○) that the two of calculation data {$CAL_i$, $CAL_j$ (i≠j)}_z are different or a judgment result (×) that the two calculation data {$CAL_i$, $CAL_j$(i≠j)}_z are not different about the two of calculation data {$CAL_i$, $CAL_j$(i≠j)}_z.

After the step S774, the judgment unit 214 judges whether z=Z or not (step S775). Here, Z represents the total number of Z sets of two pieces of calculation data {$CAL_i$, $CAL_j$ (i≠j)}_1 to {$CAL_i$, $CAL_j$ (i≠j)}_Z.

When it is judged in step S775 that z=Z is not satisfied, the judgment unit 214 sets z=z+1 (step S776). After that, the operation of the judgment unit 214 proceeds to step S774, and steps S774 to S776 are executed repeatedly until it is judged in step S775 that z=Z is satisfied.

Then, when it is judged in step S775 that z = Z is true, the judgment unit 214 creates a judgment result indicating whether the two calculation data {$CAL_i$, $CAL_j$ (i ≠ j)}_z in each of the Z sets of two calculation data {CALi, CALj (i ≠ j)}_1 to {CALi, CALj (i ≠ j)}_Z are or not different based on the Z judgment results $JDGR_1$ to $JDGR_Z$ (step S777).

After the step S777, the operation of the diagnostic device 2 proceeds to the step S78 in Fig. 91.

Fig. 97 is a flowchart for explaining the detailed operation of the step S8 in Fig. 90.

Referring to Fig. 97, after the step S7 in Fig. 90, the calculation unit 216 receives m pieces of index data $IDX_1$ to $IDX_m$ from the control unit 212.

Then, the calculation unit 216 judges whether a plurality of index data have been or not received from the control unit 212 (step S81).

In this case, when the calculation unit 216 has not been received multiple index data from the control unit 212, the calculation unit 216 judges that the calculation unit 216 has received index data $IDX_{uni}$ from the control unit 212, and when the calculation unit 216 has received multiple index data from the control unit 212, the calculation unit 216 judges that the calculation unit 216 has received P pieces of index data $IDX_1$ to $IDX_P$ (multiple index data IDX) from the control unit 212.

When it is judged in the step S81 that a plurality of index data have not been received from the control unit 212, the calculation unit 216 and the taste diagnostic unit 217 diagnose the taste of the analyte based on the index data $IDX_{uni}$ (step S82).

On the other hand, when it is judged in the step S81 that multiple index data have been received from the control unit 212, the calculation unit 216 and the taste diagnostic unit 217 diagnose the taste of the P analytes based on the P index data $IDX_1$ to $IDX_P$ (multiple index data IDX) (step S83).

After the step S82 or the step S83, the operation of the diagnostic device 2 proceeds to "End" in Fig. 90.

Fig. 98 is a flowchart for explaining the detailed operation of the step S82 in Fig. 97.

Referring to Fig. 98, when it is judged in the step S81 of Fig. 97 that multiple index data have not been received, the calculation unit 216 detects calculation data $CAL_{uni}$ from the index data $IDX_{uni}$, and calculates a sum L(+)_sum of the integral values in a positive predetermined potential section based on the n integral values $ITG_{1_Low}$ to $ITG_{n_Low}$ of the detected calculation data $CAL_{uni}$ (=n integral values $ITG_{1_Low}$ to $ITG_{n_Low}$ in n predetermined potential sections calculated from the current-potential characteristics of a cyclic voltammogram measured by scanning the potential V at a potential scanning speed $V_{r_Low}$) (step S821).

In addition, the calculation unit 216 calculates the sum M(+)_sum of the integral values in a positive predetermined potential section based on the n integral values $ITG_{1_Middle}$ to $ITG_{n_Middle}$ (=n integral values $ITG_{1_Middle}$ to $ITG_{n_Middle}$ in n predetermined potential sections calculated from the current-potential characteristics of a cyclic voltammogram measured by scanning the potential V at a potential scanning speed $V_{r_Middle}$) of the calculation data $CAL_{uni}$ (step S822).

Furthermore, the calculation unit 216 calculates the sum H(+)_sum of the integral values in a positive predetermined potential section based on the n integral values $ITG_{1_High}$ to $ITG_{n_High}$ (=n integral values $ITG_{1_High}$ to $ITG_{n_High}$ in n predetermined potential sections calculated from the current-potential characteristics of a cyclic voltammogram measured by scanning the potential V at a potential scanning speed $V_{r_High}$) of the calculation data $CAL_{uni}$ (step S823).

Furthermore, the calculation unit 216 calculates the sum L(all)_sum of the integral values in all predetermined potential sections based on the n integral values $ITG_{1_Low}$ to $ITG_{n_Low}$, calculates the sum M(all)_sum of the integral values in all predetermined potential sections based on the n integral values $ITG_{1_Middle}$ to $ITG_{n_Middle}$, and calculates the sum H(all)_sum of the integral values in all predetermined potential sections based on the n integral values $ITG_{1_High}$ to $ITG_{n_High}$ (step S824).

Furthermore, the calculation unit 216 calculates the sum H(-)_sum of the integral values in a negative predetermined potential section based on the n integral values $ITG_{1_High}$ to $ITG_{n_High}$ (step S825).

Furthermore, the calculation unit 216 calculates the sum H(-)_sum_th of the integral values in a predetermined negative potential section below or equal to a threshold value $V_{th}$ based on the n integral values $ITG_{1_High}$ to $ITG_{n_High}$, calculates the sum M(-)_sum_th of the integral values in a predetermined negative potential section below or equal to a

threshold value $V_{th}$ based on the n integral values $ITG_{1_Middle}$ to $ITG_{n_Middle}$, and calculates the sum L(-)_sum_th of the integral values in a predetermined negative potential section below or equal to a threshold value $V_{th}$ based on the n integral values $ITG_{1_Low}$ to $ITG_{n_Low}$ (step S826).

Furthermore, the calculation unit 216 calculates the Body index (+) by dividing the adding result adding the sum M(+)_sum of the integral values to the sum L(+)_sum of the integral values by the sum H(+)_sum of the integral values (step S827).

Furthermore, the calculation unit 216 calculates the Body index (all) by dividing the adding result adding the sum M(all)_sum of the integral values to the sum L(all)_sum of the integral values by the sum H(all)_sum of the integral values (step S828).

Furthermore, the calculation unit 216 calculates the Body index (-)_th by dividing the adding result adding the sum M(-)_sum_th of the integral values to the sum L(-)_sum_th of the integral values by the sum H(-)_sum_th of the integral values (step S829).

Then, the calculation unit 216 outputs the Body index (+), the Body index (all), the sum H(+)_sum of the integral values, the sum H(-)_sum of the integral values and the Body index (-)_th to the taste diagnostic unit 217.

The taste diagnostic unit 217 receives the Body index (+), the Body index (all), the sum H(+)_sum of the integral values, the sum H(-)_sum of the integral values, and the Body index (-)_th from the calculation unit 216.

Then, the taste diagnostic unit 217 diagnoses the "astringency", "aftertaste", "sweetness", "aroma" and "bitterness" of the analyte based on Body index (+), Body index (all), the sum H(+)_sum of the integral values, the sum H(-)_sum of the integral values and Body index (-)_th (step S830).

In this case, the taste diagnostic unit 217 diagnoses the "astringency" of the analyte (shochu) using the Body index (+) and diagnoses the "astringency" of the analyte (grapes) using the Body index (-)_th.

Then, after step S830, the taste diagnostic unit 217 creates a taste diagnosis result JDR of the analyte, and outputs the created taste diagnosis result JDR to the control unit 212 and the display unit 218.

Upon receiving by the control unit 212 the taste diagnosis result JDR from the taste diagnostic unit 217, the control unit 212 stores the received taste diagnosis result JDR in the database 22.

Furthermore, upon receiving the taste diagnosis result JDR from the taste diagnostic unit 217, the display unit 218 displays the received taste diagnosis result JDR.

After the step S830, the operation of the diagnostic device 2 proceeds to "End" in Fig. 90.

Fig. 99 is a flowchart for explaining the detailed operation of the step S830 in Fig. 98.

The flowchart shown in Fig. 99 is a flowchart for explaining the detailed operation of step S830 in Fig. 98 in when the analyte is "shochu".

Referring to Fig. 99, after the step S829 in Fig. 98, the taste diagnostic unit 217 receives the Body index (+), the Body index (all), the sum H(+)_sum of the integral values and the sum H(-)_sum of the integral values from the calculation unit 216.

Then, the taste diagnostic unit 217 substitutes the Body index (+) and coefficient $k_1$ into equation (5) to calculate the astringency ASTG value of the analyte (shochu), and diagnoses the calculated value as the astringency ASTG of the analyte (shochu) (step S8301).

After the step S8301, the taste diagnostic unit 217 substitutes the Body index (all) and coefficient $k_2$ into equation (6) to calculate the value of the aftertaste LNGS of the analyte (shochu), and diagnoses the calculated value as the aftertaste LNGS of the analyte (shochu) (step S8302).

After the step S8302, the taste diagnostic unit 217 substitutes the sum H(+)_sum of the integral values and the coefficients $k_3$ and $k_4$ into equation (7) to calculate the value of the sweetness SWT, and diagnoses the calculated value as the sweetness SWT of the analyte (shochu) (step S8303).

After the step S8303, the taste diagnostic unit 217 substitutes the sum H(-)_sum of the integral values and the coefficients $k_5$ and $k_6$ into equation (8) to calculate the value of the aroma SCT, and diagnoses the calculated value as the aroma SCT of the analyte (shochu) (step S8304).

After the step S8304, the taste diagnostic unit 217 substitutes the astringency ASTG, sweetness SWT and coefficients $k_7$ and $k_8$ into equation (9) to calculate the value of bitterness BIT, and diagnoses the calculated value as the bitterness BIT of the analyte (shochu) (step S8305).

Then, after the step S8305, the operation of the diagnostic device 2 proceeds to "End" in Fig. 90.

Fig. 100 is another flowchart for explaining the detailed operation of the step S830 in Fig. 98.

Referring to Fig. 100, after the step S829 in Fig. 98, the taste diagnostic unit 217 receives the Body index (-)_th from the calculation unit 216.

Then, the taste diagnostic unit 217 substitutes the Body index (-)_th and the coefficient $k_9$ into the formula (11) to calculate the value of the astringency ASTG, and diagnoses the calculated value as the astringency ASTG of the analyte (grapes) (step S8301A).

Thereafter, the operation of the diagnostic device 2 proceeds to "End" in Fig. 90.

Fig. 101 is a flowchart for explaining the detailed operation of the step S83 in Fig. 97.

Referring to Fig. 101, when it has been judged in the step S81 of Fig. 97 that a plurality of index data have been received, the calculation unit 216 already has received P pieces of index data $IDX_1$ to $IDX_P$ from the control unit 212.

Then, the calculation unit 216 sets p=1 (step S831), and detects the calculation data $CAL_p$ from the index data $IDX_p$ (step S832).

Thereafter, the calculation unit 216 sequentially executes the steps S821 to S829 in Fig. 98 based on the calculation data $CAL_p$ of the index data $IDX_p$ (step S833).

Then, the taste diagnostic unit 217 sequentially executes the steps S8301 to S8305 in Fig. 99 based on the Body index (+), the Body index (all), the sum H(+)_sum of the integral values and the sum H(-)_sum of the integral values (step S834).

Then, the calculation unit 216 judges whether or not p=P (step S835).

When it is judged in step S835 that p is not equal to P, the calculation unit 216 sets p = p+1 (step S836).

Thereafter, the operation of the diagnostic device 2 proceeds to step S832, and steps S832 to S836 are executed repeatedly until it is judged in the step S835 that p is equal to P.

Then, in the step S835, when it was judged that p is equal to P, the taste diagnostic unit 217 creates a diagnosis result $JDR_P$ of P tastes for the P analytes, and outputs the created diagnosis result $JDR_P$ of P tastes to the control unit 212 and the display unit 218.

When the control unit 212 receives the P taste diagnosis results $JDR_P$ from the taste diagnostic unit 217, the control unit 212 stores the received P taste diagnosis results $JDR_P$ in the database 22.

Furthermore, upon receiving the P taste diagnosis results $JDR_P$ from the taste diagnostic unit 217, the display unit 218 displays the received P taste diagnosis results $JDR_P$.

Then, the operation of the diagnostic device 2 proceeds to "End" in Fig. 90.

Fig. 102 is another flowchart for explaining the detailed operation of the step S83 in Fig. 97.

The flowchart shown in Fig. 102 is the same as the flowchart shown in Fig. 101, except that the step S834 in the flowchart shown in Fig. 101 is replaced with step S834A.

Referring to Fig. 102, when it was judged in the step S81 of Fig. 97 that a plurality of index data were received, the calculation unit 216 has received P pieces of index data $IDX_1$ to $IDX_P$ from the control unit 212.

Then, the above-mentioned steps S831 to S833 are executed sequentially. After the step S833, the taste diagnostic unit 217 executes step S8301A in Fig. 100 based on the Body index (-)_th (step S834A).

Then, the above-mentioned the step S835 is executed, and when it is judged in step S835 that p=P is not satisfied, the above-mentioned the step S836 is executed, and then the operation of the diagnostic device 2 proceeds to the step S832.

Then, the steps S832, S833, S834A, S835, and S836 are repeatedly executed until it is judged in the step S835 that p=P.

Then, in the step S835, when it was judged that p is equal to P, the taste diagnostic unit 217 creates a diagnosis result $JDR_P$ of P tastes for the P analytes, and outputs the created diagnosis result $JDR_P$ of P tastes to the control unit 212 and the display unit 218.

Upon receiving the P pieces of taste diagnosis results $JDR_P$ from the taste diagnostic unit 217, the control unit 212 stores the received P pieces of taste diagnosis results $JDR_P$ in the database 22.

Upon receiving the P pieces of taste diagnosis results $JDR_P$ from the taste diagnostic unit 217, the display unit 218 displays the received P pieces of taste diagnosis results $JDR_P$.

Then, the operation of the diagnostic device 2 proceeds to "End" in Fig. 90.

Fig. 103 is a diagram showing the correspondence relationship of classes in a positive predetermined potential section when the number of integral values in when the analyte is "shochu" is 18, 20, 32, 36, 56, 70, 92, 138 and 276.

Referring to Fig. 103, when the analyte is "shochu", class $1_{_138}$ in when the number of integral values is 138 is associated with classes $1_{_276}$ and $2_{_276}$ in when the number of integral values is 276, class $2_{_138}$ in when the number of integral values is 138 is associated with classes $3_{_276}$ and $4_{_276}$ in when the number of integral values is 276, class $3_{_138}$ in when the number of integral values is 138 is associated with classes $5_{_276}$ and $6_{_276}$ in when the number of integral values is 276, similarly, class $67_{_138}$ in when the number of integral values is 138 is associated with classes $133_{_276}$ and $134_{_276}$ in when the number of integral values is 276, class $68_{_138}$ in when the number of integral values is 138 is associated with classes $135_{_276}$ and $136_{_276}$ in when the number of integral values is 276, class $69_{_138}$ in when the number of integral values is 138 is associated with classes $137_{_276}$ and $138_{_276}$ in when the number of integral values is 276 (See (b) of Fig. 103).

As a result, the integral value $ITG_{1_Low_138}$ in class $1_{_138}$ in when the number of integral values is 138 consists of the sum (=$ITG_{1_Low_276}$+$ITG_{2_Low_276}$) of the integral value $ITG_{1_Low_276}$ in class $1_{_276}$ and the integral value $ITG_{2_Low_276}$ in class $2_{_276}$ in when the number of integral values is 276, the integral value $ITG_{1_MIdddle_138}$ in class $1_{_138}$ in when the number of integral values is 138 consists of the sum (=$ITG_{1_ Middle _276}$+$ITG_{2_ Middle _276}$) of the integral value $ITG_{1_Middle_276}$ in class $1_{_276}$ and the integral value $ITG_{2_ Middle _276}$ in class $2_{_276}$ in when the number of integral values is 276, and the integral value $ITG_{1_High_138}$ in class $1_{_138}$ in when the number of integral values is 138 consists of the sum

(=$ITG_{1_High_276}$+$ITG_{2_High_276}$) of the integral value $ITG_{1_High_276}$ in class $1_{_276}$ and the integral value $ITG_{2_High_276}$ in class $2_{_276}$ in when the number of integral values is 276.

Furthermore, an integral value $ITG_{2_Low_138}$ in class $2_{_138}$ in when the number of integral values is 138 is consists of the sum (=$ITG_{3_Low_276}$+$ITG_{4_Low_276}$) of an integral value $ITG_{3_Low_276}$ in class $3_{_276}$ and an integral value $ITG_{4_Low_276}$ in class $4_{_276}$ in when the number of integral values is 276, an integral value $ITG_{2_Middle_138}$ in class $2_{_138}$ in when the number of integral values is 138 is consists of the sum (=$ITG_{3_Middle_276}$+$ITG_{4_Middle_276}$) of an integral value $ITG_{3_Middle_276}$ in class $3_{_276}$ and an integral value $ITG_{4_Middle_276}$ in class $4_{_276}$ in when the number of integral values is 276, and an integral value $ITG_{2_High_138}$ in class $2_{_138}$ in when the number of integral values is 138 is consists of the sum (=$ITG_{3_High_276}$+$ITG_{4_High_276}$) of an integral value $ITG_{3_High_276}$ in class $3_{_276}$ and an integral value $ITG_{4_High_276}$ in class $4_{_276}$ in when the number of integral values is 276.

Similarly, an integral value $ITG_{69_Low_138}$ in class $69_{_138}$ in when the number of integral values is 138 is consists of the sum (=$ITG_{137_Low_276}$+$ITG_{138_Low_276}$) of an integral value $ITG_{137_Low_276}$ in class $137_{_276}$ and an integral value $ITG_{138_Low_276}$ in class $138_{_276}$ in when the number of integral values is 276, an integral value $ITG_{69_Middle_138}$ in class $69_{_138}$ in when the number of integral values is 138 is consists of the sum (=$ITG_{137_Middle_276}$+$ITG_{138_Middle_276}$) of an integral value $ITG_{137_Middle_276}$ in class $3_{_276}$ and an integral value $ITG_{138_Middle_276}$ in class $138_{_276}$ in when the number of integral values is 276, and an integral value $ITG_{69_High_138}$ in class $69_{_138}$ in when the number of integral values is 138 is consists of the sum (=$ITG_{137_High_276}$+$ITG_{138_High_276}$) of an integral value $ITG_{137_High_276}$ in class $137_{_276}$ and an integral value $ITG_{138_High_276}$ in class $138_{_276}$ in when the number of integral values is 276.

In addition, when the analyte is "shochu", class $1_{_92}$ in when the number of integral values is 92 is corresponded with classes $1_{_276}$ to $3_{_276}$ in when the number of integral values is 276, class $2_{_92}$ in when the number of integral values is 92 is corresponded with classes $4_{_276}$ to $6_{_276}$ in when the number of integral values is 276, class $3_{_92}$ in when the number of integral values is 92 is corresponded with classes $7_{_276}$ to $9_{_276}$ in when the number of integral values is 276, similarly, class $45_{_92}$ in when the number of integral values is 92 is corresponded with classes $133_{_276}$ to $135_{_276}$ in when the number of integral values is 276, and class $46_{_92}$ in when the number of integral values is 92 is corresponded with classes $136_{_276}$ to $138_{_276}$ in when the number of integral values is 276 (See (c) of Fig. 103).

As a result, an integral value $ITG_{1_Low_92}$ in class $1_{_92}$ in when the number of integral values is 92 is composed of the sum (=$ITG_{1_Low_276}$+$ITG_{2_Low_276}$+$ITG_{3_Low_276}$) of an integral value $ITG_{1_Low_276}$ in class $1_{_276}$, an integral value $ITG_{2_Low_276}$ in class $2_{_276}$, and an integral value $ITG_{3_Low_276}$ in class $3_{_276}$ in when the number of integral values is 276, an integral value $ITG_{1_Middle_92}$ in class $1_{_92}$ in when the number of integral values is 92 is composed of the sum (=$ITG_{1_Middle_276}$+$ITG_{2_Middle_276}$+$ITG_{3_Middle_276}$) of an integral value $ITG_{1_Middle_276}$ in class $1_{_276}$, an integral value $ITG_{2_Middle_276}$ in class $2_{_276}$, and an integral value $ITG_{3_Middle_276}$ in class $3_{_276}$ in when the number of integral values is 276, and an integral value $ITG_{1_High_92}$ in class $1_{_92}$ in when the number of integral values is 92 is composed of the sum (=$ITG_{1_High_276}$+$ITG_{2_High_276}$+$ITG_{3_High_276}$) of an integral value $ITG_{1_High_276}$ in class $1_{_276}$, an integral value $ITG_{2_High_276}$ in class $2_{_276}$, and an integral value $ITG_{3_High_276}$ in class $3_{_276}$ in when the number of integral values is 276.

In addition, an integral value $ITG_{2_Low_92}$ in class $2_{_92}$ in when the number of integral values is 92 is composed of the sum (=$ITG_{4_Low_276}$+$ITG_{5_Low_276}$+$ITG_{6_Low_276}$) of an integral value $ITG_{4_Low_276}$ in class $4_{_276}$, an integral value $ITG_{5_Low_276}$ in class $5_{_276}$, and an integral value $ITG_{6_Low_276}$ in class $6_{_276}$ in when the number of integral values is 276, an integral value $ITG_{2_Middle_92}$ in class $2_{_92}$ in when the number of integral values is 92 is composed of the sum (=$ITG_{4_Middle_276}$+$ITG_{5_Middle_276}$+$1TG_{6_Middle_276}$) of an integral value $ITG_{4_Middle_276}$ in class $4_{_276}$, an integral value $ITG_{5_Middle_276}$ in class $5_{_276}$, and an integral value $ITG_{6_Middle_276}$ in class $6_{_276}$ in when the number of integral values is 276, and an integral value $ITG_{2_High_92}$ in class $2_{_92}$ in when the number of integral values is 92 is composed of the sum (=$ITG_{4_High_276}$+$ITG_{5_High_276}$+ $ITG_{6_High_276}$) of an integral value $ITG_{4_High_276}$ in class $4_{_276}$, an integral value $ITG_{5_High_276}$ in class $5_{_276}$, and an integral value $ITG_{6_High_276}$ in class $6_{_276}$ in when the number of integral values is 276.

Similarly, an integral value $ITG_{46_Low_92}$ in class $46_{_92}$ in when the number of integral values is 92 is composed of the sum (=$ITG_{136_Low_276}$+$ITG_{137_Low_276}$+$ITG_{138_Low_276}$) of an integral value $ITG_{136_Low_276}$ in class $136_{_276}$, an integral value $ITG_{137_Low_276}$ in class $137_{_276}$, and an integral value $ITG_{138_Low_276}$ in class $138_{_276}$ in when the number of integral values is 276, an integral value $ITG_{46_Middle_92}$ in class $46_{_92}$ in when the number of integral values is 92 is composed of the sum (=$ITG_{136_Middle_276}$+$ITG_{137_Middle_276}$+$ITG_{138_Middle_276}$) of an integral value $ITG_{136_Middle_276}$ in class $136_{_276}$, an integral value $ITG_{137_Middle_276}$ in class $137_{_276}$, and an integral value $ITG_{138_Middle_276}$ in class $138_{_276}$ in when the number of integral values is 276, and an integral value $ITG_{46_High_92}$ in class $46_{_92}$ in when the number of integral values is 92 is composed of the sum (= $ITG_{136_High_276}$+ $ITG_{137_High_276}$+ $ITG_{138_High_276}$) of an integral value $ITG_{136_High_276}$ in class $136_{_276}$, an integral value $ITG_{137_High_276}$ in class $137_{_276}$, and an integral value $ITG_{138_High_276}$ in class $138_{_276}$ in when the number of integral values is 276.

Furthermore, when the analyte is "shochu", class $1_{_70}$ in when the number of integral values is 70 is corresponded with classes $1_{_276}$ to$4_{_276}$ in when the number of integral values is 276, class $2_{_70}$ in when the number of integral values is70 is corresponded with classes $5_{_276}$ to $8_{_276}$ in when the number of integral values is 276, class $3_{_70}$ in when the number of integral values is 70 is corresponded with classes $9_{_276}$ to $12_{_276}$ in when the number of integral values is 276, similarly, class $33_{_70}$ in when the number of integral values is 70 is corresponded with classes $129_{_276}$ to $132_{_276}$ in when the number of integral values is 276, and class $34_{_70}$ in when the number of integral values is 70 is corresponded with classes $133_{_276}$ to $136_{_276}$ in when the number of integral values is 276, and class $35_{_70}$ in when the number of integral values is 70 is corresponded with classes $137_{_276}$, $138_{_276}$ in when the number of integral values is 276, (See (d) of Fig. 103).

Here, the reason why class $35_{_70}$ in when the number of integral values is 70 is corresponded with classes $137_{_276}$ and $138_{_276}$ in when the number of integral values is 276, because it is necessary to set class $35_{_70}$ in when the number of integral values is 70 to the last class in the positive predetermined potential section.

As a result, the integral value $ITG_{1_Low_70}$ in class $1_{_70}$ in when the number of integral values is 70 consists of the sum (=$ITG_{1_Low_276}$+$ITG_{2_Low_276}$+$ITG_{3_Low_276}$+$ITG_{4_Low_276}$) of the integral value $ITG_{1_Low_276}$ in class $1_{_276}$, the integral value $ITG_{2_Low_276}$ in class $2_{_276}$, the integral value $ITG_{3_Low_276}$ in class $3_{_276}$, the integral value $ITG_{4_Low_276}$ in class $4_{_276}$ in when the number of integral values is 276, the integral value $ITG_{1_Middle_70}$ in class $1_{_70}$ in when the number of integral values is 70 consists of the sum (=$ITG_{1_ Middle_276}$+$ITG_{2_ Middle_276}$+$ITG_{3_ Middle_276}$+$ITG_{4_ Middle_276}$) of the integral value $ITG_{1_Middle_276}$ in class $1_{_276}$, the integral value $ITG_{2_ Middle_276}$ in class $2_{_276}$, the integral value $ITG_{3_ Middle_276}$ in class $3_{_276}$, and the integral value $ITG_{4_ Middle_276}$ in class $4_{_276}$ in when the number of integral values is 276, and the integral value $ITG_{1_High_70}$ in class $1_{_70}$ in when the number of integral values is 70 consists of the sum (=$ITG_{1_High_276}$+ $ITG_{2_High_276}$+$ITG_{3_High_276}$+$ITG_{4_High_276}$) of the integral value $ITG_{1_High_276}$ in class $1_{_276}$, the integral value $ITG_{2_High_276}$ in class $2_{_276}$, the integral value $ITG_{3_High_276}$ in class $3_{_276}$, and the integral value $ITG_{4_High_276}$ in class $4_{_276}$ in when the number of integral values is 276.

Also, the integral value $ITG_{2_Low_70}$ in class $2_{_70}$ in when the number of integral values is 70 consists of the sum (=$ITG_{5_Low_276}$+$ITG_{6_Low_276}$+$ITG_{7_Low_276}$+$ITG_{8_Low_276}$) of the integral value $ITG_{5_Low_276}$ in class $5_{_276}$, the integral value $ITG_{6_Low_276}$ in class $6_{_276}$, the integral value $ITG_{7_Low_276}$ in class $7_{_276}$, and the integral value $ITG_{8_Low_276}$ in class $8_{_276}$ in when the number of integral values is 276, the integral value $ITG_{2_Middle_70}$ in class $2_{_70}$ in when the number of integral values is 70 consists of the sum (=$ITG_{5_Low_276}$+ $ITG_{6_Low_276}$+ $ITG_{7_Low_276}$ +$ITG_{8_Low_276}$) of the integral value $ITG_{5_Middle_276}$ in class $5_{_276}$, the integral value $ITG_{6_Middle_276}$ in class $6_{_276}$, the integral value $ITG_{7_Middle_276}$ in class $7_{_276}$, and the integral value $ITG_{8_Middle_276}$ in class $8_{_276}$ in when the number of integral values is 276, and the integral value $ITG_{2_High_70}$ in class $2_{_70}$ in when the number of integral values is 70 consists of the sum (=$ITG_{5_High_276}$+ $ITG_{6_High_276}$+ $ITG_{7_High_276}$+ $ITG_{8_High_276}$) of the integral value $ITG_{5_ High_276}$ in class $5_{_276}$, the integral value $ITG_{6_ High_276}$ in class $6_{_276}$, the integral value $ITG_{7_High_276}$ in class $7_{_276}$, and the integral value $ITG_{8_ High_276}$ in class $8_{_276}$ in when the number of integral values is 276.

Similarly, the integral value $ITG_{34_Low_70}$ in class $34_{_70}$ in when the number of integral values is 70 consists of the sum (=$ITG_{133_Low_276}$+ $ITG_{134_Low_276}$+ $ITG_{135_Low_276}$+ $ITG_{136_Low_276}$) of the integral value $ITG_{133_Low_276}$ in class $133_{_276}$, the integral value $ITG_{134_Low_276}$ in class $134_{_276}$, the integral value $ITG_{135_Low_276}$ in class $135_{_276}$, and the integral value $ITG_{136_Low_276}$ in class $136_{_276}$ in when the number of integral values is 276, the integral value $ITG_{34_Middle_70}$ in class $34_{_70}$ in when the number of integral values is 70 consists of the sum (=$ITG_{5_Low_276}$+ $ITG_{6_Low_276}$+ $ITG_{7_Low_276}$+ $ITG_{8_Low_276}$) of the integral value $ITG_{133_Middle_276}$ in class $133_{_276}$, the integral value $ITG_{134_Middle_276}$ in class $134_{_276}$, the integral value $ITG_{135_Middle_276}$ in class $135_{_276}$, and the integral value $ITG_{136_Middle_276}$ in class $136_{_276}$ in when the number of integral values is 276, and the integral value $ITG_{34_High_70}$ in class $34_{_70}$ in when the number of integral values is 70 consists of the sum (= $ITG_{133_High_276}$+ $ITG_{134_High_276}$+ $ITG_{135_High_276}$ +$ITG_{136_High_276}$) of the integral value $ITG_{133_High_276}$ in class $133_{_276}$, the integral value $ITG_{134_High_276}$ in class $134_{_276}$, the integral value $ITG_{135_ High_276}$ in class $135_{_276}$, and the integral value $ITG_{136_ High_276}$ in class $136_{_276}$ in when the number of integral values is 276.

Then, the integral value $ITG_{35_Low_70}$ in class $35_{_70}$ in when the number of integral values is 70 is made up of the sum (=$ITG_{137_Low_276}$+$ITG_{138_Low_276}$) of the integral value $ITG_{137_Low_276}$ in class $137_{_276}$ and the integral value $ITG_{138_Low_276}$ in class $138_{_276}$ in when the number of integral values is 276, the integral value $ITG_{35_Middle_70}$ in class $35_{_70}$ in when the number of integral values is 70 is made up of the sum (=$ITG_{137_Middle_276}$+$ITG_{138_Middle_276}$) of an integral value $ITG_{137_Middle_276}$ in class $137_{_276}$ and an integral value $ITG_{138_Middle_276}$ in class $138_{_276}$ in when the number of integral values is 276, and the integral value $ITG_{35_High_70}$ in class $35_{_70}$ in when the number of integral values is 70 is made up of the sum (=$ITG_{137_High_276}$+ $ITG_{138_High_276}$) of an integral value $ITG_{137_High_276}$ in class $137_{_276}$ and an integral value $ITG_{138_High_276}$ in class $138_{_276}$ in when the number of integral values is 276.

Furthermore, when the analyte is "shochu", class $1_{_56}$ in when the number of integral values is 56 is associated with classes $1_{_276}$ to $5_{_276}$ in when the number of integral values is 276, class $2_{_56}$ in when the number of integral values is 56 is associated with classes $6_{_276}$ to $10_{_276}$ in when the number of integral values is 276, similarly, class $26_{_56}$ in when the number of integral values is 56 is associated with classes $126_{_276}$ to $130_{_276}$ in when the number of integral values is 276, class $27_{_56}$ in when the number of integral values is 56 is associated with classes $131_{_276}$ to $135_{_276}$ in when the number of integral values is 276, and class $28_{_56}$ in when the number of integral values is 56 is associated with classes $136_{_276}$ to $138_{_276}$ in when the number of integral values is 276 (See (e) of Fig. 103).

Here, the reason why class $28_{_56}$ in when the number of integral values is 56 is associated with classes $136_{_276}$ to $138_{_276}$ in when the number of integral values is 276, in order to make class $28_{_56}$ in when the number of integral values is 56 to the last class in the positive predetermined potential section.

As a result, the integral value $ITG_{1_Low_56}$ in class $1_{_56}$ in when the number of integral values is 56 consists of the sum(=$ITG_{1_Low_276}$+ $ITG_{2_Low_276}$+ $ITG_{3_Low_276}$+ $ITG_{4_Low_276}$+ $ITG_{5_Low_276}$) from integral value $ITG_{1_Low_276}$ in class $1_{_276}$ to $ITG_{5_Low_276}$ in class $5_{_276}$ in when the number of integral values is 276, the integral value $ITG_{1_Middle_56}$ in class $1_{_56}$ in when the number of integral values is 56 consists of the sum(=$ITG_{1_Middle_276}$+$ITG_{2_Middle_276}$+$ITG_{3_Middle_276}$ +$ITG_{4_Middle_276}$+$ITG_{5_Middle_276}$) from integral value $ITG_{1_Middle_276}$ in class $1_{_276}$ to $ITG_{5_Middle_276}$ in class $5_{_276}$ in when the number of integral values is 276, and the integral value $ITG_{1_High_56}$ in class $1_{_56}$ in when the number of integral values is 56 consists of the sum (= $ITG_{1_High_276}$+ $ITG_{2_High_276}$+ $ITG_{3_High_276}$+ $ITG_{4_High_276}$+ $ITG_{5_High_276}$ ) from integral value $ITG_{1_High_276}$ in class $1_{_276}$ to $ITG_{5_High_276}$ in class $5_{_276}$ in when the number of integral values is 276.

In addition, the integral value $ITG_{2_Low_56}$ in class $2_{_56}$ in when the number of integral values is 56 consists of the sum(=$ITG_{6_Low_276}$+ $ITG_{7_Low_276}$+ $ITG_{8_Low_276}$+ $ITG_{9_Low_276}$+ $ITG_{10_Low_276}$) from integral values $ITG_{6_Low_276}$ in class $6_{_276}$ to $ITG_{10_Low_276}$ in class $10_{_276}$ in when the number of integral values is 276, the integral value $ITG_{2_Middle_56}$ in class $2_{_56}$ in when the number of integral values is 56 consists of the sum(=$ITG_{6_Middle_276}$+$ITG_{7_Middle_276}$+$ITG_{8_Middle_276}$+$ITG_{9_Middle_276}$+$ITG_{10_Middle_276}$) from integral value $ITG_{6_Middle_276}$ in class $6_{_276}$ to $ITG_{10_Middle_276}$ in class $10_{_276}$ in when the number of integral values is 276, and the integral value $ITG_{2_High_56}$ in class $2_{_56}$ in when the number of integral values is 56 consists of the sum(=$ITG_{6_High_276}$+ $ITG_{7_High_276}$+ $ITG_{8_High_276}$+$ITG_{9_High_276}$+$ITG_{10_High_276}$) from integral value $ITG_{6_High_276}$ in class $6_{_276}$ to $ITG_{10_High_276}$ in class $10_{_276}$ in when the number of integral values is 276.

Similarly, the integral value $ITG_{27_Low_56}$ in class $27_{_56}$ in when the number of integral values is 56 consists of the sum(=$ITG_{131_Low_276}$+$ITG_{132_Low_276}$+$ITG_{133_Low_276}$+ $ITG_{134_Low_276}$+ $ITG_{135_Low_276}$) from integral values $ITG_{131_Low_276}$ in class $131_{_276}$ to $ITG_{135_Low_276}$ in class $135_{_276}$ in when the number of integral values is 276, the integral value $ITG_{27_Middle_56}$ in class $27_{_56}$ in when the number of integral values is 56 consists of the sum (=$ITG_{131_Middle_276}$+$ITG_{132_Middle_276}$+$ITG_{133_Middle_276}$+$ITG_{134_Middle_276}$+ $ITG_{135_Middle_276}$) from integral value $ITG_{131_Middle_276}$ in class $131_{_276}$ to $ITG_{135_Middle_276}$ in class $135_{_276}$ in when the number of integral values is 276, and the integral value $ITG_{27_High_56}$ in class $27_{_56}$ in when the number of integral values is 56 consists of the sum (= $ITG_{131_High_276}$ +$ITG_{132_High_276}$+$ITG_{133_High_276}$+$ITG_{134_High_276}$+$ITG_{135_High_276}$) from integral values $ITG_{131_High_276}$ in class $131_{_276}$ to $ITG_{135_High_276}$ in class $135_{_276}$ in when the number of integral values is 276.

Then, the integral value $ITG_{28_Low_56}$ in class $28_{_56}$ in when the number of integral values is 56 consists of the sum(=$ITG_{136_Low_276}$+$ITG_{137_Low_276}$+$ITG_{138_Low_276}$) integral value $ITG_{136_Low_276}$ in class $136_{_276}$, integral value $ITG_{137_Low_276}$ in class $137_{_276}$ and integral value $ITG_{138_Low_276}$ in class $138_{_276}$ in when the number of integral values is 276, the integral value $ITG_{28_Middle_56}$ in class $28_{_56}$ in when the number of integral values is 56 consists of the sum(=$ITG_{136_Middle_276}$+$ITG_{137_Middle_276}$+$ITG_{138_Middle_276}$) of integral value $ITG_{136_Middle_276}$ in class $136_{_276}$, integral value $ITG_{137_Middle_276}$ in class $137_{_276}$ and integral value $ITG_{138_Middle_276}$ in class $138_{_276}$ in when the number of integral values is 276, and the integral value $ITG_{28_High_56}$ in class $28_{_56}$ in when the number of integral values is 56 consists of the sum(=$ITG_{136_High_276}$+$ITG_{137_High_276}$+$ITG_{138_High_276}$) of integral value $ITG_{136_High_276}$ in class $136_{_276}$, integral value $ITG_{137_High_276}$ in class $137_{_276}$ and integral value $ITG_{138_High_276}$ in class $138_{_276}$ in when the number of integral values is 276.

Furthermore, when the analyte is "shochu", class $1_{_36}$ in when the number of integral values is 36 is associated with classes $1_{_276}$ to $8_{_276}$ in when the number of integral values is 276, class $2_{_36}$ in when the number of integral values is 36 is associated with classes $9_{_276}$ to $16_{_276}$ in when the number of integral values is 276, similarly, class $16_{_36}$ in when the number of integral values is 36 is associated with classes $121_{_276}$ to $128_{_276}$ in when the number of integral values is 276, class $17_{_36}$ in when the number of integral values is 36 is associated with classes $129_{_276}$ to $136_{_276}$ in when the number of integral values is 276, and class $18_{_36}$ in when the number of integral values is 36 is associated with classes $137_{_276}$ and $138_{_276}$ in when the number of integral values is 276 (See (f) of Fig. 103).

Here, the reason why class $18_{_36}$ in when the number of integral values is 36 is associated with classes $137_{_276}$ and $138_{_276}$ in when the number of integral values is 276 is to make class $18_{_36}$ in when the number of integral values is 36 the last class in the positive predetermined potential section.

As a results, the integral value $ITG_{1_Low_36}$ in class $1_{_36}$ in when the number of integral values is 36 consists of the sum(=$ITG_{1_Low_276}$+ $ITG_{2_Low_276}$+ $ITG_{3_Low_276}$+ $ITG_{4_Low_276}$+ $ITG_{5_Low_276}$+$ITG_{6_Low_276}$+$ITG_{7_Low_276}$+$ITG_{8_Low_276}$) from integral value $ITG_{1_Low_276}$ in class $1_{_276}$ to integral value $ITG_{8_Low_276}$ in class $8_{_276}$ in when the number of integral values is 276, the integral value $ITG_{1_Middle_36}$ in class $1_{_36}$ in when the number of integral values is 36 consists of the sum(=$ITG_{1_Middle_276}$+$ITG_{2_Middle_276}$+ $ITG_{3_Middle_276}$+$ITG_{4_Middle_276}$+$ITG_{5_Middle_276}$+$ITG_{6_Middle_276}$ +$ITG_{7_Middle_276}$+$ITG_{8_Middle_276}$) from integral value $ITG_{1_Middle_276}$ in class $1_{_276}$ to integral value $ITG_{8_Middle_276}$ in class $8_{_276}$ in when the number of integral values is 276, and the integral value $ITG_{1_High_36}$ in class $1_{_36}$ in when the number of integral values is 36 consists of the sum(=$ITG_{1_High_276}$+$ITG_{2_High_276}$+$ITG_{3_High_276}$+$ITG_{4_High_276}$+$ITG_{5_High_276}$+$ITG_{6_High_276}$ +$ITG_{7_High_276}$+$ITG_{8_High_276}$) from integral value $ITG_{1_High_276}$ in class $1_{_276}$ to integral value

$ITG_{8_High_276}$ in class $8_{_276}$ in when the number of integral values is 276.

Similarly, the integral value $ITG_{17_Low_36}$ in class $17_{_36}$ in when the number of integral values is 36 consists of the sum(=$ITG_{129_Low_276}$+$ITG_{130_Low_276}$+$ITG_{131_Low_276}$+ $ITG_{132_Low_276}$+$ITG_{133_Low_276}$+$ITG_{134_Low_276}$+$ITG_{135_Low_276}$+$ITG_{136_Low_276}$) from integral value $ITG_{129_Low_276}$ in class $129_{_276}$ to integral value $ITG_{136_Low_276}$ in class $136_{_276}$ in when the number of integral values is 276, the integral value $ITG_{17_Middle_36}$ in class $17_{_36}$ in when the number of integral values is 36 consists of the sum(=$ITG_{129_Middle_276}$+ $ITG_{130_Middle_276}$+ $ITG_{131_Middle_276}$+ $ITG_{132_Middle_276}$+ $ITG_{133_Middle_276}$+$ITG_{134_Middle_276}$+$ITG_{135_Middle_276}$+ $ITG_{136_Low_276}$) from integral value $ITG_{129_Middle_276}$ in class $129_{_276}$ to integral value $ITG_{136_Middle_276}$ in class $136_{_276}$ in when the number of integral values is 276, and the integral value $ITG_{17_High_36}$ in class $17_{_36}$ in when the number of integral values is 36 consists of the sum (=$ITG_{129_High_276}$ + $ITG_{130_High_276}$+ $ITG_{131_High_276}$+ $ITG_{132_High_276}$+ $ITG_{133_High_276}$+ $ITG_{134_High_276}$+ $ITG_{135_High_276}$+ $ITG_{136_High_276}$) from integral value $ITG_{129_High_276}$ in class $129_{_276}$ to integral value $ITG_{136_High_276}$ in class $136_{_276}$ in when the number of integral values is 276.

In addition, the integral value $ITG_{18_Low_36}$ in class $18_{_36}$ in when the number of integral values is 36 consists of the sum(=$ITG_{137_Low_276}$+$ITG_{138_Low_276}$) of integral value $ITG_{137_Low_276}$ in class $137_{_276}$ and integral value $ITG_{138_Low_276}$ in class $138_{_276}$ in when the number of integral values is 276, the integral value $ITG_{18_Middle_36}$ in class $18_{_36}$ in when the number of integral values is 36 consists of the sum(=$ITG_{137_Middle_276}$+$ITG_{138_Middle_276}$) of integral value $ITG_{137_Middle_276}$ in class $137_{_276}$ and integral value $ITG_{138_Middle_276}$ in class $138_{_276}$ in when the number of integral values is 276, and the integral value $ITG_{18_High_36}$ in class $18_{_36}$ in when the number of integral values is 36 consists of the sum(=$ITG_{137_High_276}$+$ITG_{138_High_276}$) of integral value $ITG_{137_High_276}$ in class $137_{_276}$ and integral value $ITG_{138_High__276}$ in class $138_{_276}$ in when the number of integral values is 276.

Furthermore, when the analyte is "shochu", class $1_{_32}$ in when the number of integral values is 32 is associated with classes $1_{_276}$ to $9_{_276}$ in when the number of integral values is 276, similarly, class $15_{_32}$ in when the number of integral values is 32 is associated with classes $127_{_276}$ to $135_{_276}$ in when the number of integral values is 276, and class $16_{_32}$ in when the number of integral values is 32 is associated with classes $136_{_276}$ to $138_{_276}$ in when the number of integral values is 276 (See (g) of Fig. 103).

Here, the reason why class $16_{_32}$ in when the number of integral values is 32 is associated with classes $136_{_276}$ to $138_{_276}$ in when the number of integral values is 276 is to make class $16_{_36}$ in when the number of integral values is 32 to the last class in the positive predetermined potential section.

As a result, the integral value $ITG_{1_Low_32}$ in class $1_{_32}$ in when the number of integral values is 32 consists of the sum(=$ITG_{1_Low_276}$+ $ITG_{2_Low_276}$+ $ITG_{3_Low_276}$+ $ITG_{4_Low_276}$+ $ITG_{5_Low_276}$+$ITG_{6_Low_276}$+$ITG_{7_Low_276}$+$ITG_{8_Low_276}$+$ITG_{9_Low_276}$) from integral value $ITG_{1_Low_276}$ in class $1_{_276}$ to integral value $ITG_{9_Low_276}$ in class $9_{_276}$ in when the number of integral values is 276, the integral value $ITG_{1_Middle_32}$ in class $1_{_32}$ in when the number of integral values is 32 consists of the sum(=$ITG_{1_Middle_276}$+ $ITG_{2_Middle_276}$+$ITG_{3_Middle_276}$+$ITG_{4_Middle_276}$+$ITG_{5_Middle_276}$ +$ITG_{6_Middle_276}$+$ITG_{7_Middle_ 276}$+$ITG_{8_Middle276}$ +$ITG_{9_Middle_276}$) from integral value $ITG_{1_Middle_276}$ in class $1_{_276}$ to integral value $ITG_{9_Middle_276}$ in class $9_{_276}$ in when the number of integral values is 276, and the integral value $ITG_{1_High_32}$ in class $1_{_32}$ in when the number of integral values is 32 consists of the sum(=$ITG_{1_High_276}$+ $ITG_{2_High_276}$+ $ITG_{3_High_276}$+ $ITG_{4_High_276}$+ $ITG_{5_High_276}$+$ITG_{6_High_276}$+$ITG_{7_High_276}$+$ITG_{8_High_276}$ +$ITG_{9_High_276}$) from integral value $ITG_{1_High_276}$ in class $1_{_276}$ to integral value $ITG_{9_High_276}$ in class $9_{_276}$ in when the number of integral values is 276.

Similarly, the integral value $ITG_{15_Low_32}$ in class $15_{_32}$ in when the number of integral values is 32 consists of the sum(=$ITG_{127_Low_276}$+ $ITG_{128_Low_276}$+ $ITG_{129_Low_276}$+ $ITG_{130_Low_276}$+$ITG_{131_Low_276}$+$ITG_{132_Low_276}$+$ITG_{133_Low_276}$+$ITG_{134_Low276}$+$ITG_{135_Low_ 276}$) from integral value $ITG_{127_Low_276}$ in class $127_{_276}$ to integral value $ITG_{135_Low_276}$ in class $135_{_276}$ in when the number of integral values is 276, the integral value $ITG_{15_Middle_32}$ in class $15_{_32}$ in when the number of integral values is 32 consists of the sum (=$ITG_{127_Middle_276}$+ $ITG_{128_Middle_276}$+ $ITG_{129_Middle_276}$+ $ITG_{130_Middle_276}$+ $ITG_{131_Middle_276}$+ $ITG_{132_Middle_276}$+ $ITG_{133_Middle_276}$+ $ITG_{134_Middle_276}$+ $ITG_{135_Middle_276}$) from integral value $ITG_{127_Middle_276}$ in class $127_{_276}$ to integral value $ITG_{135_Middle_276}$ in class $135_{_276}$ in when the number of integral values is 276, and the integral value $ITG_{15_High_32}$ in class $15_{_32}$ in when the number of integral values is 32 consists of the sum(=$ITG_{127_High_276}$+ $ITG_{128_High_276}$+ $ITG_{129_High_276}$+ $ITG_{130_High_276}$+ $ITG_{131_High_276}$+ $ITG_{132_High_276}$+ $ITG_{133_High_276}$+ $ITG_{134_High_276}$+$ITG_{135_High_276}$) from integral value $ITG_{127_High_276}$ in class $127_{_276}$ to integral value $ITG_{135_High_276}$ in class $135_{_276}$ in when the number of integral values is 276.

Then, the integral value $ITG_{16_Low_32}$ in class $16_{_32}$ in when the number of integral values is 32 consists of the sum(=$ITG_{136_Low_276}$+$ITG_{137_Low_276}$+$ITG_{138_Low_276}$) of integral value $ITG_{136_Low_276}$ in class $136_{_276}$, integral value $ITG_{137_Low_276}$ in class $137_{_276}$, and integral value $ITG_{138_Low_276}$ in class $138_{_276}$ in when the number of integral values is 276, the integral value $ITG_{16_Middle_32}$ in class $16_{_32}$ in when the number of integral values is 32 consists of the sum(=$ITG_{136_Middle_276}$+$ITG_{137_Middle_276}$+$ITG_{138_Middle_276}$) of integral value $ITG_{136_Middle_276}$ in class $136_{_276}$, integral value $ITG_{137__Middle_276}$ in class $137_{_276}$, and integral value $ITG_{138_Middle_276}$ in class $138_{_276}$ in when the number of integral values is 276, and the integral value $ITG_{16_High_32}$ in class $16_{_32}$ in when the number of integral values is 32 consists of the sum(=$ITG_{136_High_276}$+$ITG_{137_High_276}$+$ITG_{138_High_276}$) of integral value $ITG_{136_High_276}$ in class $136_{_276}$,

integral value $ITG_{137_High_276}$ in class $137_{_276}$, and integral value $ITG_{138_High_276}$ in class $138_{_276}$ in when the number of integral values is 276.

Furthermore, when the analyte is "shochu", class $1_{_20}$ in when the number of integral values is 20 is associated with classes $1_{_276}$ to $15_{_276}$ in when the number of integral values is 276, similarly, class $9_{_20}$ in when the number of integral values is 20 is associated with classes $121_{_276}$ to $135_{_276}$ in when the number of integral values is 276, and class $10_{_20}$ in when the number of integral values is 20 is associated with classes $136_{_276}$ to $138_{_276}$ in when the number of integral values is 276 (See (h) of Fig. 103),

Here, the reason why class $10_{_20}$ in when the number of integral values is 20 is associated with classes $136_{_276}$ to $138_{_276}$ in when the number of integral values is 276 is to make class $10_{_20}$ in when the number of integral values is 20 to the last class in the positive predetermined potential section.

As a results, the integral value $ITG_{1_Low_20}$ in class $1_{_20}$ in when the number of integral values is 20 consists of the sum(=$ITG_{1_Low_276}$+ $ITG_{2_Low_276}$+ $ITG_{3_Low_276}$+ $ITG_{4_Low_276}$+ $ITG_{5_Low_276}$+$ITG_{6_Low_276}$+$ITG_{7_Low_276}$+$ITG_{8_Low_276}$+$ITG_{9_Low_276}$+,..., + $ITG_{15_Low_276}$) from integral value $ITG_{1_Low_276}$ in class $1_{_276}$ to integral value $ITG_{15_Low_276}$ in class $15_{_276}$ in when the number of integral values is 276, the integral value $ITG_{1_Middle_20}$ in class $1_{_20}$ in when the number of integral values is 20 consists of the sum(=$ITG_{1_Middle_276}$+$ITG_{2_Middle_276}$+ $ITG_{3_Middle_276}$+ $ITG_{4_Middle_276}$+ $ITG_{5_Middle_276}$+ $ITG_{6_Middle_276}$+$ITG_{7_Middle_276}$+$ITG_{8_Middle_276}$+$ITG_{9_Middle_276}$+, ...,+ $ITG_{15_Middle_276}$) from integral value $ITG_{1_Middle_276}$ in class $1_{_276}$ to integral value $ITG_{15_Middle_276}$ in class $15_{_276}$ in when the number of integral values is 276, and the integral value $ITG_{1_High_20}$ in class $1_{_20}$ in when the number of integral values is 20 consists of the sum(=$ITG_{1_High_276}$+ $ITG_{2_High_276}$+ $ITG_{3_High_276}$+ $ITG_{4_High_276}$+$ITG_{5_High_276}$+$ITG_{6_High_276}$+$ITG_{7_High_276}$+$ITG_{8_High_276}$+$ITG_{9_High_276}$+,..., + $[TG_{15_High_276}$) from integral value $ITG_{1_High_276}$ in class $1_{_276}$ to integral value $ITG_{15_High_276}$ in class $15_{_276}$ in when the number of integral values is 276.

Similarly, the integral value $ITG_{9_Low_20}$ in class $9_{_20}$ in when the number of integral values is 20 consists of the sum(=$ITG_{121_Low_276}$+$ITG_{122_Low_276}$+$ITG_{123_Low_276}$+$ITG_{124_Low_276}$+ $ITG_{125_Low_276}$+$ITG_{126_Low_276}$+$ITG_{127_Low_276}$+$ITG_{128_Low_276}$+$ITG_{128_Low_276}$+,...,+$ITG_{135_Low_276}$) from integral value $ITG_{121_Low_276}$ in class $121_{_276}$ to integral value $ITG_{135_Low_276}$ in class $135_{_276}$ in when the number of integral values is 276, the integral value $ITG_{9_Middle_20}$ in class $9_{_20}$ in when the number of integral values is 20 consists of the sum(=$ITG_{121_Middle_276}$+$ITG_{122_Middle_276}$+$ITG_{123_Middle_276}$+$ITG_{124_Middle_276}$+$ITG_{125_Middle_276}$+$ITG_{126_Middle_276}$+$ITG_{127_Middle_276}$+$ITG_{128_Middle_276}$ +$ITG_{129_Middle_276}$+,...,$ITG_{135_Middle_276}$) from integral value $ITG_{121_Middle_276}$ in class $121_{_276}$ to integral value $ITG_{135_Middle_276}$ in class $135_{_276}$ in when the number of integral values is 276, and the integral value $ITG_{9_High_20}$ in class $9_{_20}$ in when the number of integral values is 20 consists of the sum(=$ITG_{121_High_276}$+$ITG_{122_High_276}$+$ITG_{123_High_276}$+$ITG_{124_High_276}$+$ITG_{125_High_276}$+$ITG_{126_High_276}$+ $ITG_{127_High_276}$+$ITG_{128_High_276}$+ $ITG_{129_High_276}$ +,...,+ $ITG_{135_High_276}$) from integral value $ITG_{121_High_276}$ in class $121_{_276}$ to integral value $ITG_{135_High_276}$ in class $135_{_276}$ in when the number of integral values is 276.

Then, the integral value $ITG_{10_Low_20}$ in class $10_{_20}$ in when the number of integral values is 20 consists of the sum(=$ITG_{136_Low_276}$+$ITG_{137_Low_276}$+$ITG_{138_Low_276}$) from integral value $ITG_{136_Low_276}$ in class $136_{_276}$ to integral value $ITG_{138_Low_276}$ in class $138_{_276}$ in when the number of integral values is 276, the integral value $ITG_{10_Middle_20}$ in class $10_{_20}$ in when the number of integral values is 20 consists of the sum(=$ITG_{136_Middle_276}$+ $ITG_{137_Middle_276}$+ $ITG_{138_Middle_276}$) from integral value $ITG_{136_Middle_276}$ in class $136_{_276}$ to integral value $ITG_{138_Middle_276}$ in class $138_{_276}$ in when the number of integral values is 276, and the integral value $ITG_{10_High_20}$ in class $10_{_20}$ in when the number of integral values is 20 consists of the sum(=$ITG_{136_High_276}$+$ITG_{137_High_276}$+$ITG_{138_High_276}$) from integral value $ITG_{136_High_276}$ in class $136_{_276}$ to integral value $ITG_{138_High_276}$ in class $138_{_276}$ in when the number of integral values is 276.

Furthermore, when the analyte is "shochu", class $1_{_18}$ in when the number of integral values is 18 is associated with classes $1_{_276}$ to $17_{_276}$ in when the number of integral values is 276, similarly, when the analyte is "shochu", class $8_{_18}$ in when the number of integral values is 18 is associated with classes $120_{_276}$ to $136_{_276}$ in when the number of integral values is 276, and when the analyte is "shochu", class $9_{_18}$ in when the number of integral values is 18 is associated with classes $137_{_276}$ and $138_{_276}$ in when the number of integral values is 276 (See (i) of Fig. 103).

Here, the reason why class $9_{_18}$ in when the number of integral values is 18 is associated with classes $137_{_276}$ and $138_{_276}$ in when the number of integral values is 276 is to make class $9_{_18}$ in when the number of integral values is 18 to the last class in the positive predetermined potential section.

As a result, the integral value $ITG_{1_Low_18}$ in class $1_{_18}$ in when the number of integral values is 18 consists of the sum(=$ITG_{1_Low_276}$+$ITG_{2_Low_276}$+$ITG_{3_Low_276}$+$ITG_{4_Low_276}$+ $ITG_{5_Low_276}$+$ITG_{6_Low_276}$+$ITG_{7_Low_276}$+$ITG_{8_Low_276}$+$ITG_{9_Low_276}$+,..., + $ITG_{17_Low_276}$) from integral value $ITG_{1_Low_276}$ in class $1_{_276}$ to integral value $ITG_{17_Low_276}$ in class $17_{_276}$ in when the number of integral values is 276, the integral value $ITG_{1_Middle_18}$ in class $1_{_18}$ in when the number of integral values is 18 consists of the sum(=$ITG_{1_Middle_276}$+ $ITG_{2_Middle_276}$+$ITG_{3_Middle_276}$+$ITG_{4_Middle_276}$+$ITG_{5_Middle_276}$+$ITG_{6_Middle_276}$+$ITG_{7_Middle_276}$+$ITG_{8_Middle_276}$+$ITG_{9_Middle_276}$+,...,+$ITG_{17_Middle_276}$) from integral value $ITG_{1_Middle_276}$ in class $1_{_276}$ to integral value $ITG_{17_Middle_276}$ in class $17_{_276}$ in when the number of integral values is 276, and the integral value $ITG_{1_High_18}$ in class $1_{_18}$ in when the number of integral values is 18 consists of the sum(=$ITG_{1_High_276}$+$ITG_{2_High_276}$+ $ITG_{3_High_276}$+$ITG_{4_High_276}$+$ITG_{5_High_276}$+$ITG_{6_High_276}$+$ITG_{7_High_276}$+$ITG_{8_High_276}$+$ITG_{9\ _High_276}$+,..., + $ITG_{17_High_276}$) from integral value $ITC_{1_High_276}$ in class $1_{_276}$ to integral value $ITG_{17_High_276}$ in class $17_{_276}$ in when the number of integral values is 276.

Similarly, the integral value $ITG_{8_Low_18}$ in class $8_{_18}$ in when the number of integral values is 18 consists of the sum(=$ITG_{120_Low_276}$+$ITG_{121_Low_276}$+$ITG_{122_Low_276}$+$ITG_{123_Low_276}$+ $ITG_{124_Low_276}$+ $ITG_{125_Low_276}$+ $ITG_{126_Low_276}$+ $ITG_{127_Low_276}$+$ITG_{128_Low_}$276+, ..., + $ITG_{136_Low_276}$) from integral value $ITG_{120_Low_276}$ in class $120_{_276}$ to integral value $ITG_{136_Low_276}$ in class $136_{_276}$ in when the number of integral values is 276, the integral value $ITG_{8_Middle_18}$ in class $8_{_18}$ in when the number of integral values is 18 consists of the sum (=$ITG_{120_Middle_276}$+ $ITG_{121_Middle_276}$+ $ITG_{122_Middle_276}$+ $ITG_{123_Middle_276}$+ $ITG_{124_Middle_276}$+$ITG_{125_Middle_276}$+$TG_{126_Middle_276}$ +$ITG_{127_Middle_276}$+$ITG_{128_Middle_276}$+..., +$ITG_{136_Middle_276}$) from integral value $ITG_{120_Middle_276}$ in class $120_{_276}$ to integral value $ITG_{136_Middle_276}$ in class $136_{_276}$ in when the number of integral values is 276, and the integral value $ITG_{8_High_18}$ in class $8_{_18}$ in when the number of integral values is 18 consists of the sum(=$ITG_{120_High_276}$+ $ITG_{121_High_276}$+ $ITG_{122_High_276}$+ $ITG_{123_High_276}$+ $ITG_{124_High_276}$+ $ITG_{125_High_276}$+ $ITG_{126_High_276}$+ $ITG_{127_High_276}$+ $ITG_{128_High_276}$+,...,+ $ITG_{136_High_276}$) from integral value $ITG_{120_High_276}$ in class $120_{_276}$ to integral value $ITG_{136_High_276}$ in class $136_{_276}$ in when the number of integral values is 276.

Then, the integral value $ITG_{9_Low_18}$ in class $9_{_18}$ in when the number of integral values is 18 consists of the sum(=$ITG_{137_Low_276}$+$ITG_{138_Low_276}$) of integral value $ITG_{137_Low_276}$ in class $137_{_276}$ and integral value $ITG_{138_Low_276}$ in class $138_{_276}$ in when the number of integral values is 276, the integral value $ITG_{9_Middle_18}$ in class $9_{_18}$ in when the number of integral values is 18 consists of the sum(=$ITG_{137_Middle_276}$+$ITG_{138_Middle_276}$) of integral value $ITG_{137_Middle_276}$ in class $137_{_276}$ and integral value $ITG_{138_Middle_276}$ in class $138_{_276}$ in when the number of integral values is 276, and the integral value $ITG_{9_High_18}$ in class $9_{_18}$ in when the number of integral values is 18 consists of the sum(=$ITG_{137_High_276}$+ $ITG_{138_High_276}$) of integral value $ITG_{137_High_276}$ in class $137_{_276}$ and integral value $ITG_{138_High_276}$ in class $138_{_276}$ in when the number of integral values is 276.

As described above, the integral values $ITG_{1_Low_138}$ to $ITG_{69_Low_138}$ in classes $1_{_138}$ to $69_{_138}$ in when the number of integral values is 138 are calculated using the integral values $ITG_{1_Low_276}$ to $ITG_{138_Low_276}$ in classes $1_{_276}$ to $138_{_276}$ in when the number of integral values is 276, the integral values $ITG_{1_Middle_138}$ to $ITG_{69_Low_138}$ in classes $1_{_138}$ to $69_{_138}$ in when the number of integral values is 138 are calculated using the integral values $ITG_{1_Middle_276}$ to $ITG_{138_Middle_276}$ in classes $1_{_276}$ to $138_{_276}$ in when the number of integral values is 276, and the integral values $ITG_{1_High_138}$ to $ITG_{69_Low_138}$ in classes $1_{_138}$ to $69_{_138}$ in when the number of integral values is 138 are calculated using the integral values $ITG_{1_High_276}$ to $ITG_{138_High_276}$ in classes $1_{_276}$ to $138_{_276}$ in when the number of integral values is 276.

Also, the integral values $ITG_{1_Low_92}$ to $ITG_{46_Low_92}$ in classes $1_{_92}$ to $46_{_92}$ in when the number of integral values is 92, the integral values $ITG_{1_Low_70}$ to $ITG_{35_Low_70}$ in classes $1_{_70}$ to $35_{_70}$ in when the number of integral values is 70, the integral values $ITG_{1_Low_56}$ to $ITG_{28_Low_56}$ in classes $1_{_56}$ to $28_{_56}$ in when the number of integral values is 56, the integral values $ITG_{1_Low_36}$ to $ITG_{18_Low_36}$ in classes $1_{_36}$ to $18_{_36}$ in when the number of integral values is 36, the integral values $ITG_{1_Low_32}$ to $ITG_{16_Low_32}$ in classes $1_{_32}$ to $16_{_32}$ in when the number of integral values is 32, the integral values $ITG_{1_Low_20}$ to $ITG_{10_Low_20}$ in classes $1_{_20}$ to $10_{_20}$ in when the number of integral values is 20, and the integral values $ITG_{1_Low_18}$ to $ITG_{9_Low_18}$ in classes $1_{_18}$ to $9_{_18}$ in when the number of integral values is 18, similarly, are calculated using the integral values $ITG_{1_High_276}$ to $ITG_{138_High_276}$ in class $1_{_276}$ to class $138_{_276}$ in when the number of integral values is 276.

Therefore, classes $1_{_138}$ to $69_{_138}$ in when the number of integral values is 138, classes $1_{_92}$ to $46_{_92}$ in when the number of integral values is 92, classes $1_{_70}$ to $35_{_70}$ in when the number of integral values is 70, classes $1_{_56}$ to $28_{_56}$ in when the number of integral values is 56, classes $1_{_36}$ to $18_{_36}$ in when the number of integral values is 36, classes $1_{_32}$ to $16_{_32}$ in when the number of integral values is 32, classes $1_{_20}$ to $10_{_20}$ in when the number of integral values is 20, and classes $1_{_18}$ to $9_{_18}$ in when the number of integral values is 18 are associated with classes $1_{_276}$ to $138_{_276}$ in when the number of integral values is 276, as a results, regardless of whether the number of integral values is 138, 92, 70, 56, 36, 32, 20, or 18, the sum of the integral values in the positive predetermined potential section can be made to match the sum of the integral values in classes $1_{_276}$ to $138_{_276}$ (=positive predetermined potential section) in when the number of integral values is 276.

Fig. 104 is a diagram showing the correspondence relationship of classes in a negative predetermined potential range about when the number of integral values in when the analyte is "shochu" is 18, 20, 32, 36, 56, 70, 92, 138 and 276.

Referring to Fig. 104, when the analyte is "shochu", the class $70_{_138}$ in when the number of integral values is 138 is corresponded to classes $139_{_276}$ and $140_{_276}$ in when the number of integral values is 276, the class $71_{_138}$ in when the number of integral values is 138 is corresponded to classes $141_{_276}$ and $142_{_276}$ in when the number of integral values is 276, the class $72_{_138}$ in when the number of integral values is 138 is corresponded to classes $143_{_276}$ and $144_{_276}$ in when the number of integral values is 276, similarly, the class $136_{_138}$ in when the number of integral values is 138 is corresponded to classes $271_{_276}$ and $272_{_276}$ in when the number of integral values is 276, the class $137_{_138}$ in when the number of integral values is 138 is corresponded to classes $273_{_276}$ and $274_{_276}$ in when the number of integral values is 276, and the class 138 $_{138}$ in when the number of integral values is 138 is corresponded to classes 275 $_{276}$ and 276 $_{276}$ in when the number of integral values is 276 (See (b) of Fig. 104).

As a result, the integral value $ITG_{70_Low_138}$ in class 70 $_{138}$ in when the number of integral values is 138 consists of the sum(=$ITG_{139_Low_276}$+$ITG_{140_Low_276}$) of integral value $ITG_{139_Low_276}$ in class 139 $_{276}$ and integral value $ITG_{140_Low_276}$ in class 140 $_{276}$ in when the number of integral values is 276, the integral value $ITG_{70_Middle_138}$ in class 70 $_{138}$ in when the number of integral values is 138 consists of the sum(=$ITG_{139_Middle_276}$ + $ITG_{140_Middle_276}$) of integral value $ITG_{139_Middle_276}$ in class 139 $_{276}$ and integral value $ITG_{140_Middle_276}$ in class 140 $_{276}$ in when the number of integral values is 276, and he integral value $ITG_{70_High_138}$ in class 70 $_{138}$ in when the number of integral values is 138 consists of the sum(=$ITG_{139_High_276}$+$ITG_{140_High_276}$) of integral value $ITG_{139_High_276}$ in class 139 $_{276}$ and integral value $ITG_{140_High_276}$ in class 140 $_{276}$ in when the number of integral values is 276.

Also, the integral value $ITG_{71_Low_138}$ in class 71 $_{138}$ in when the number of integral values is 138 consists of the sum(=$ITG_{141_Low_276}$+$ITG_{142_Low_276}$) of integral value $ITG_{141_Low_276}$ in class 141 $_{276}$ and integral value $ITG_{142_Low_276}$ in class 142 $_{276}$ in when the number of integral values is 276, the integral value $ITG_{71_Middle_138}$ in class 71 $_{138}$ in when the number of integral values is 138 consists of the sum(=$ITG_{141_Middle_276}$+$ITG_{142_Middle_276}$) of integral value $ITG_{141_Middle_276}$ in class 141 $_{276}$ and integral value $ITG_{142_Middle_276}$ in class 142 $_{276}$ in when the number of integral values is 276, and the integral value $ITG_{71_High_138}$ in class 71 $_{138}$ in when the number of integral values is 138 consists of the sum(=$ITG_{141_High_276}$+$ITG_{142_High_276}$) of integral value $ITG_{141_High_276}$ in class 141 $_{276}$ and integral value $ITG_{142_High_276}$ in class 142 $_{276}$ in when the number of integral values is 276.

Similarly, the integral value $ITG_{138_Low_138}$ in class 138 $_{138}$ in when the number of integral values is 138 consists of the sum(=$ITG_{275_Low_276}$+$ITG_{276_Low_276}$) of integral value $ITG_{275_Low_276}$ in class 275 $_{276}$ and integral value $ITG_{276_Low_276}$ in class 276 $_{276}$ in when the number of integral values is 276, the integral value $ITG_{138_Middle_138}$ in class 138 $_{138}$ in when the number of integral values is 138 consists of the sum(=$ITG_{275_Middle_276}$+ $ITG_{276_Middle_276}$) of integral value $ITG_{275_Middle_276}$ in class 275 $_{276}$ and integral value $ITG_{276_Middle_276}$ in class 276 $_{276}$ in when the number of integral values is 276, and the integral value $ITG_{138_High_138}$ in class 138 $_{138}$ in when the number of integral values is 138 consists of the sum(=$ITG_{275_High_276}$+$ITG_{276_High_276}$) of integral value $ITG_{275_High_276}$ in class 275 $_{276}$ and integral value $ITG_{276_High_276}$ in class 276 $_{276}$ in when the number of integral values is 276.

Also, when the analyte is "shochu", the class 47 $_{92}$ in when the number of integral values is 92 is corresponded to classes 139 $_{276}$ to 141 $_{276}$ in when the number of integral values is 276, the class 48 $_{92}$ in when the number of integral values is 92 is corresponded to classes 142 $_{276}$ to 144 $_{276}$ in when the number of integral values is 276, the class 49 $_{92}$ in when the number of integral values is 92 is corresponded to classes 145 $_{276}$ to 147 $_{276}$ in when the number of integral values is 276, similarly, the class 91 $_{92}$ in when the number of integral values is 92 is corresponded to classes 271 $_{276}$ to 273 $_{276}$ in when the number of integral values is 276, and the class 92 $_{92}$ in when the number of integral values is 92 is corresponded to classes 274 $_{276}$ to 276 $_{276}$ in when the number of integral values is 276 (See (c) of Fig. 104).

As a result, the integral value $ITG_{47_Low_92}$ in class 47 $_{92}$ in when the number of integral values is 92 consists of the sum(=$ITG_{139_Low_276}$+$ITG_{140_Low_276}$+$ITG_{141_Low_276}$) of integral value $ITG_{139Low_276}$ in class139 $_{276}$, integral value $ITG_{140_Low_276}$ in class 140 $_{276}$, and integral value $ITG_{141_Low_276}$ in class 141 $_{276}$ in when the number of integral values is 276, the integral value $ITG_{47_Middle_92}$ in class 47 $_{92}$ in when the number of integral values is 92 consists of the sum(=$ITG_{139_Middle_276}$+$ITG_{140_Middle_276}$+$ITG_{141_Middle_276}$) of integral value $ITG_{139_Middle_276}$ in class139 $_{276}$, integral value $ITG_{140_Middle_276}$ in class 140 $_{276}$ , and integral value $ITG_{141_Middle_276}$ in class 141 $_{276}$ in when the number of integral values is 276, and the integral value $ITG_{47_High_92}$ in class 47 $_{92}$ in when the number of integral values is 92 consists of the sum(=$ITG_{139_High_276}$+$ITG_{140_High_276}$+$ITG_{141_High_276}$) of integral value $ITG_{139_High_276}$ in class139 $_{276}$, integral value $ITG_{140_High_276}$ in class 140 $_{276}$, and integral value $ITG_{141_High_276}$ in class 141 $_{276}$ in when the number of integral values is 276.

Also, the integral value $ITG_{48_Low_92}$ in class 48 $_{92}$ in when the number of integral values is 92 consists of the sum(=$ITG_{142_Low_276}$+$ITG_{143_Low_276}$+$ITG_{144_Low_276}$) of integral value $ITG_{142_Low_276}$ in class142 $_{276}$, integral value $ITG_{143_Low_276}$ in class 143 $_{276}$, and integral value $ITG_{144_Low_276}$ in class 144 $_{276}$ in when the number of integral values is 276, the integral value $ITG_{48_Middle_92}$ in class 48 $_{92}$ in when the number of integral values is 92 consists of the sum(=$ITG_{142_Middle_276}$+$ITG_{143_Middle_276}$+$ITG_{144_Middle_276}$) of integral value $ITG_{142_Middle_276}$ in class142 $_{276}$, integral value $ITG_{143_Middle_276}$ in class 143 $_{276}$, and integral value $ITG_{144_Middle_276}$ in class 144 $_{276}$ in when the number of integral values is 276, and the integral value $ITG_{48_High_92}$ in class 48 $_{92}$ in when the number of integral values is 92 consists of the sum(=$ITG_{142_High_276}$+$ITG_{143_High_276}$+$ITG_{144_High_276}$) of integral value $ITG_{142_High_276}$ in class142 $_{276}$, integral value $ITG_{143_High_276}$ in class 143 $_{276}$, and integral value $ITG_{144_High_276}$ in class 144 $_{276}$ in when the number of integral values is 276.

Similarly, the integral value $ITG_{92_Low_92}$ in class 92 $_{92}$ in when the number of integral values is 92 consists of the sum(=$ITG_{274_Low_276}$+$ITG_{275_Low_276}$+$ITG_{276_Low_276}$) of integral value $ITG_{274_Low_276}$ in class274 $_{276}$, integral value $ITG_{275_Low_276}$ in class 275 $_{276}$, and integral value $ITG_{276_Low_276}$ in class 276 $_{276}$ in when the number of integral values is 276, the integral value $ITG_{92_Middle_92}$ in class 92 $_{92}$ in when the number of integral values is 92 consists of the sum(=$ITG_{274_Middle_276}$+$ITG_{275_Middle_276}$+$ITG_{276_Middle_276}$) of integral value $ITG_{274_Middle_276}$ in class274 $_{276}$, integral value $ITG_{275_Middle_276}$ in class 275 $_{_276}$, and integral value $ITG_{276_Middle_276}$ in class 276 $_{_276}$ in when the number of integral values is 276, and the integral value $ITG_{92_High_92}$ in class 92 $_{_92}$ in when the number of integral values is 92 consists of the sum(=$ITG_{274_High_276}$+$ITG_{275_High_276}$+$ITG_{276_High_276}$) of integral value $ITG_{274_High_276}$ in class274 $_{_276}$, integral value $ITG_{275_High_276}$ in class 275 $_{_276}$, and integral value $ITG_{276_High_276}$ in class 276 $_{_276}$ in when the number of integral values is 276.

Furthermore, when the analyte is "shochu", class 36 $_{_70}$ in when the number of integral values is 70 is associated with classes 139 $_{_276}$ to 142 $_{_276}$ in when the number of integral values is 276, class 37 $_{_70}$ in when the number of integral values is 70 is associated with classes 143 $_{_276}$ to 146 $_{_276}$ in when the number of integral values is 276, class 38 $_{_70}$ in when the number of integral values is 70 is associated with classes 147 $_{_276}$ to 150 $_{_276}$ in when the number of integral values is 276, similarly, class 69 $_{_70}$ in when the number of integral values is 70 is associated with classes 271 $_{_276}$ to 274 $_{_276}$ in when the number of integral values is 276, and class 70 $_{_70}$ in when the number of integral values is 70 is associated with classes 275 $_{_276}$ and 276 $_{_276}$ in when the number of integral values is 276 (See (d) of Fig. 104).

As a result, the integral value $ITG_{36_Low_70}$ in class 36 $_{_70}$ in when the number of integral values is 70 consists of the sum(=$ITG_{139_Low_276}$+ $ITG_{140Low_276}$+ $ITG_{141_Low_276}$+ $ITG_{142_Low_276}$) of integral value $ITG_{139_Low_276}$ in class139 $_{_276}$, integral value $ITG_{140_Low_276}$ in class140 $_{276}$, integral value $ITG_{141_Low_276}$ in class 141 $_{_276}$, and integral value $ITG_{142_Low_276}$ in class 142 $_{_276}$ in when the number of integral values is 276, the integral value $ITG_{36_Middle_70}$ in class 36 $_{_70}$ in when the number of integral values is 70 consists of the sum(=$ITG_{139_Middle_276}$+$ITG_{140_Middle_276}$ +$ITG_{141_Middle_276}$+$ITG_{142_Middle_276}$) of integral value $ITG_{139_Middle_276}$ in class139 $_{_276}$, integral value $ITG_{140_Middle_276}$ in class $_{276}$, integral value $ITG_{141_Middle_276}$ in class 141 $_{_276}$, and integral value $ITG_{142_middle_276}$ in class 142 $_{_276}$ in when the number of integral values is 276, and the integral value $ITG_{36_High_70}$ in class 36 $_{_70}$ in when the number of integral values is 70 consists of the sum(=$ITG_{139_High_276}$+ $ITG_{140_High_276}$+$IT_{G141_High_276}$+$ITG_{142_High_276}$) of integral value $ITG_{139_High_276}$ in class139 $_{_276}$, integral value $ITG_{140High_276}$ in class $_{276}$, integral value $ITG_{141_High_276}$ in class 141 $_{_276}$, and integral value $ITG_{142_High_276}$ in class 142 $_{_276}$ in when the number of integral values is 276.

Also, the integral value $ITG_{37_Low_70}$ in class 37 $_{_70}$ in when the number of integral values is 70 consists of the sum(=$ITG_{143_Low_276}$+ $ITG_{144_Low_276}$+ $ITG_{145_Low_276}$+ $ITG_{146_Low_276}$) of integral value $ITG_{143_Low_276}$ in class143 $_{_276}$, integral value $ITG_{144_Low_276}$ in class $_{276}$, integral value $ITG_{145_Low_276}$ in class 145 $_{_276}$, and integral value $ITG_{146_Low_276}$ in class 146 $_{_276}$ in when the number of integral values is 276, the integral value $ITG_{37_Middle_70}$ in class 37 $_{_70}$ in when the number of integral values is 70 consists of the sum(=$ITG_{143_Middle_276}$+ $ITG_{144_Middle_276}$+ $ITG_{145_Middle_276}$+ $ITG_{146_Middle_276}$) of integral value $ITG_{143_Middle_276}$ in class143 $_{_276}$, integral value $ITG_{144_Middle_276}$ in class $_{276}$, integral value $ITG_{145_Middle_276}$ in class 145 $_{_276}$, and integral value $ITG_{146_Middle_276}$ in class 146 $_{_276}$ in when the number of integral values is 276, the integral value $ITG_{37_High_70}$ in class 37 $_{_70}$ in when the number of integral values is 70 consists of the sum(=$ITG_{143_High_276}$+ $ITG_{144_High_276}$+ $ITG_{145_High_276}$+ $ITG_{146_High_276}$) of integral value $ITG_{143_High_276}$ in class143 $_{_276}$, integral value $ITG_{144High_276}$ in class $_{276}$, integral value $ITG_{145_High_276}$ in class 145 $_{_276}$, and integral value $ITG_{146_High_276}$ in class 146 $_{_276}$ in when the number of integral values is 276.

Similarly, the integral value $ITG_{69_Low_70}$ in class 69 $_{_70}$ in when the number of integral values is 70 consists of the sum(=$ITG_{271_Low_276}$+ $ITG_{272_Low_276}$+ $ITG_{273_Low_276}$+ $ITG_{274_Low_276}$) of integral value $ITG_{271_Low_276}$ in class 271 $_{_276}$, integral value $ITG_{272_Low_276}$ in class 272 $_{_276}$, integral value $ITG_{273_Low_276}$ in class 273 $_{_276}$, and integral value $ITG_{274_Low_276}$ in class 274 $_{_276}$ in when the number of integral values is 276, the integral value $ITG_{69_Middle_70}$ in class 69 $_{_70}$ in when the number of integral values is 70 consists of the sum(=$ITG_{271_Middle_276}$+ $ITG_{272_Middle_276}$+ $ITG_{273_Middle_276}$+ $ITG_{274_Middle_276}$) of integral value $ITG_{271_Middle_276}$ in class 271 $_{_276}$, integral value $ITG_{272_Middle_276}$ in class 272 $_{_276}$, integral value $ITG_{273_Middle_276}$ in class 273 $_{_276}$, and integral value $ITG_{274_Middle_276}$ in class 274 $_{_276}$ in when the number of integral values is 276, and the integral value $ITG_{69_High_70}$ in class 69 $_{_70}$ in when the number of integral values is 70 consists of the sum(=$ITG_{271_High_276}$+ $ITG_{272_High_276}$+ $ITG_{273_High_276}$+ $ITG_{274_High_276}$) of integral value $ITG_{271_High_276}$ in class 271 $_{_276}$, integral value $ITG_{272_High_276}$ in class 272 $_{_276}$, integral value $ITG_{273_High_276}$ in class 273 $_{_276}$, and integral value $ITG_{274_High_276}$ in class 274 $_{_276}$ in when the number of integral values is 276.

Then, the integral value $ITG_{70_Low_70}$ in class 70 $_{_70}$ in when the number of integral values is 70 consists of the sum(=$ITG_{275_Low_276}$+ $ITG_{276Low_276}$) of integral value $ITG_{275_Low_276}$ in class 275 $_{_276}$ and integral value $ITG_{276_Low_276}$ in class 276 $_{_276}$ in when the number of integral values is 276, the integral value $ITG_{70_Middle_70}$ in class 70 $_{_70}$ in when the number of integral values is 70 consists of the sum(=$ITG_{275_Middle_276}$+ $ITG_{276_Middle_276}$) of integral value $ITG_{275_Middle_276}$ in class 275 $_{_276}$ and integral value $ITG_{276_Middle_276}$ in class 276 $_{_276}$ in when the number of integral values is 276, and the integral value $ITG_{70_High_70}$ in class 70 $_{_70}$ in when the number of integral values is 70 consists of the sum(=$ITG_{275_High_276}$+ $ITG_{276_High_276}$) of integral value $ITG_{275_High_276}$ in class 275 $_{_276}$ and integral value $ITG_{276_High_276}$ in class 276 $_{_276}$ in when the number of integral values is 276.

Furthermore, when the analyte is "shochu", class 29 $_{_56}$ in when the number of integral values is 56 is associated with classes 139 $_{_276}$ to 143 $_{_276}$ in when the number of integral values is 276, class 30 $_{_56}$ in when the number of integral values is 56 is associated with classes 144 $_{_276}$ to 148$_{_276}$ in when the number of integral values is 276, similarly, class 55 $_{_56}$ in when the number of integral values is 56 is associated with classes 269 $_{_276}$ to 273 $_{_276}$ in when the number of integral values is 276, class 56 $_{_56}$ in when the number of integral values is 56 is associated with classes 274 $_{_276}$ to 276 $_{_276}$

in when the number of integral values is 276 (See (e) of Fig. 104).

As a result, the integral value $ITG_{29_Low_56}$ in the class 29 $_{_56}$ in when the number of integral values is 56 consists of the sum(=$ITG_{139_Low_276}$+ $ITG_{140_Low_276}$+ $ITG_{141_Low_276}$+ $ITG_{142_Low_276}$+$ITG_{143_Low_276}$) of integral value $ITG_{139_Low_276}$ in class 139$_{_276}$, integral value $ITG_{140_Low_276}$ in class 140$_{_276}$, integral value $ITG_{141_Low_276}$ in class 141$_{_276}$, integral value $ITG_{142_Low_276}$ in class 142$_{_276}$, and integral value $ITG_{143_Low_276}$ in class 143$_{_276}$ in when the number of integral values is 276, the integral value $ITG_{29_Middle_56}$ in the class 29 $_{_56}$ in when the number of integral values is 56 consists of the sum (=$ITG_{139_Middle_276}$+$ITG_{140_Middle_276}$+$ITG_{141_Middle_276}$+$ITG_{142_Middle_276}$+$ITG_{143_Middle_276}$) of integral value $ITG_{139_Middle_276}$ in class 139$_{_276}$, integral value $ITG_{140_Middle_276}$ in class 140$_{_276}$, integral value $ITG_{141_Middle_276}$ in class 141$_{_276}$, integral value $ITG_{142_Middle_276}$ in class 142$_{_276}$, and integral value $ITG_{143_Middle_276}$ in class 143$_{_276}$ in when the number of integral values is 276, and the integral value $ITG_{29_High_56}$ in the class 29 $_{_56}$ in when the number of integral values is 56 consists of the sum (=$ITG_{139_High_276}$+$ITG_{140_High_276}$+ $ITG_{141_High_276}$+$ITG_{142_High_276}$+$ITG_{143_High_276}$) of integral value $ITG_{139_High_276}$ in class 139$_{_276}$, integral value $ITG_{140_High_276}$ in class 140$_{_276}$, integral value $ITG_{141_High_276}$ in class 141$_{_276}$, integral value $ITG_{142_High_276}$ in class 142$_{_276}$, and integral value $ITG_{143_High_276}$ in class 143$_{_276}$ in when the number of integral values is 276.

Also, the integral value $ITG_{30_Low_56}$ in the class30 $_{_56}$ in when the number of integral values is 56 consists of the sum(=$ITG_{144_Low_276}$+ $ITG_{145_Low_276}$+ $ITG_{146_Low_276}$+ $ITG_{147_Low_276}$+$ITG_{148_Low_276}$) of integral value $ITG_{144_Low_276}$ in class 144$_{_276}$, integral value $ITG_{145_Low_276}$ in class 145$_{_276}$, integral value $ITG_{146_Low_276}$ in class 146$_{_276}$, integral value $ITG_{147_Low_276}$ in class 147$_{_276}$, and integral value $ITG_{148_Low_276}$ in class 148$_{_276}$ in when the number of integral values is 276, the integral value $ITG_{30_Middle_56}$ in the class30 $_{_56}$ in when the number of integral values is 56 consists of the sum(=$ITG_{144_Middle_276}$+ $ITG_{145_Middle_276}$+ $ITG_{146_Middle_276}$+ $ITG_{147_Middle_276}$+ $ITG_{148_Middle_276}$) of integral value $ITG_{144_Middle_276}$ in class 144$_{_276}$, integral value $ITG_{145_Middle_276}$ in class 145$_{_276}$, integral value $ITG_{146_Middle_276}$ in class 146$_{_276}$, integral value $ITG_{147_Middle_276}$ in class 147$_{_276}$, and integral value $ITG_{148_Middle_276}$ in class 148$_{_276}$ in when the number of integral values is 276, and the integral value $ITG_{30_High_56}$ in the class30 $_{_56}$ in when the number of integral values is 56 consists of the sum(=$ITG_{144_High_276}$+ $ITG_{145_High_276}$+ $ITG_{146_High_276}$+ $ITG_{147_High_276}$+$ITG_{148_High_276}$) of integral value $ITG_{144_High_276}$ in class 144$_{_276}$, integral value $ITG_{145_High_276}$ in class 145$_{_276}$, integral value $ITG_{146_High_276}$ in class 146$_{_276}$, integral value $ITG_{147_High_276}$ in class 147$_{_276}$, and integral value $ITG_{148_High_276}$ in class 148$_{_276}$ in when the number of integral values is 276.

Similarly, the integral value $ITG_{55_Low_56}$ in the class55 $_{_56}$ in when the number of integral values is 56 consists of the sum(=$ITG_{269_Low_276}$+ $ITG_{270_Low_276}$+ $ITG_{271_Low_276}$+ $ITG_{272_Low_276}$+$ITG_{273_Low_276}$) of integral value $ITG_{269_Low_276}$ in class 269$_{_276}$, integral value $ITG_{270_Low_276}$ in class 270$_{_276}$, integral value $ITG_{271_Low_276}$ in class 271$_{_276}$, integral value $ITG_{272_Low_276}$ in class 272$_{_276}$, and integral value $ITG_{273_Low_276}$ in class 273$_{_276}$ in when the number of integral values is 276, the integral value $ITG_{55_Middle_56}$ in the class55 $_{_56}$ in when the number of integral values is 56 consists of the sum(=$ITG_{269_Middle_276}$+ ITG270$_{_Middle_276}$+ $ITG_{271_Middle_276}$+ ITG272$_{_Middle_276}$+ $ITG_{273_Middle_276}$) of integral value $ITG_{269_Middle_276}$ in class 269$_{_276}$, integral value $ITG_{270_Middle_276}$ in class 270$_{_276}$, integral value $ITG_{271_Middle_276}$ in class 271$_{_276}$, integral value $ITG_{272_Middle_276}$ in class 272$_{_276}$, and integral value $ITG_{273_Middle_276}$ in class 273$_{_276}$ in when the number of integral values is 276, and the integral value $ITG_{55_High_56}$ in the class55 $_{_56}$ in when the number of integral values is 56 consists of the sum(=$ITG_{269_High_276}$+ $ITG_{270_High_276}$+ $ITG_{271_High_276}$+ $ITG_{272_High_276}$+$ITG_{273_High_276}$) of integral value $ITG_{269_High_276}$ in class 269$_{_276}$, integral value $ITG_{270_High_276}$ in class 270$_{_276}$, integral value $ITG_{271_High_276}$ in class 271$_{_276}$, integral value $ITG_{272_High_276}$ in class 272$_{_276}$, and integral value $ITG_{273_High_276}$ in class 273$_{_276}$ in when the number of integral values is 276.

Then, the integral value $ITG_{56_Low_56}$ in the class56 $_{_56}$ in when the number of integral values is 56 consists of the sum(=$ITG_{274_Low_276}$+ $ITG_{275_Low_276}$+ $ITG_{276_Low_276}$) of integral value $ITG_{274_Low_276}$ in class 274$_{_276}$, integral value $ITG_{275_Low_276}$ in class 275$_{_276}$, and integral value $ITG_{276_Low_276}$ in class 276$_{_276}$ in when the number of integral values is 276, the integral value $ITG_{56_Middle_56}$ in the class56 $_{_56}$ in when the number of integral values is 56 consists of the sum(=$ITG_{274_Middle_276}$+ $ITG_{275_Middle_276}$+ $ITG_{276_Middle_276}$) of integral value $ITG_{274_Middle_276}$ in class 274$_{_276}$, integral value $ITG_{275_Middle_276}$ in class 275$_{_276}$, and integral value $ITG_{276_Middle_276}$ in class 276$_{_276}$ in when the number of integral values is 276, and the integral value $ITG_{56_High_56}$ in the class56 $_{_56}$ in when the number of integral values is 56 consists of the sum(=$ITG_{274_High_276}$+ $ITG_{275_High_276}$+ $ITG_{276_High_276}$) of integral value $ITG_{274_High_276}$ in class 274$_{_276}$, integral value $ITG_{275_High_276}$ in class 275$_{_276}$, and integral value $ITG_{276_High_276}$ in class 276$_{_276}$, in when the number of integral values is 276.

Furthermore, when the analyte is "shochu", the class19 $_{_36}$ in when the number of integral values is 36 is associated with classes 139 $_{_276}$ to 146 $_{_276}$ in when the number of integral values is 276, the class20 $_{_36}$ in when the number of integral values is 36 is associated with classes 147 $_{_276}$ to 154 $_{_276}$ in when the number of integral values is 276, similarly, the class34 $_{_36}$ in when the number of integral values is 36 is associated with classes 259 $_{_276}$ to 266 $_{_276}$ in when the number of integral values is 276, the class35 $_{_36}$ in when the number of integral values is 36 is associated with classes 267 $_{_276}$ to 274 $_{_276}$ in when the number of integral values is 276, and the class36 $_{_36}$ in when the number of integral values is 36 is associated with classes 275 $_{_276}$ and 276$_{_276}$ in when the number of integral values is 276 (See (f) of Fig. 104).

As a result, the integral value $ITG_{19_Low_36}$ in the class 19 $_{_36}$ in when the number of integral values is 36 consists of the sum(=$ITG_{139_Low_276}$+$ITG_{140_Low_276}$+,...,+$ITG_{146_Low_276}$) from integral value $ITG_{139_Low_276}$ in class $139_{_276}$ to integral value $ITG_{146_Low_276}$ in class $146_{_276}$ in when the number of integral values is 276, the integral value $ITG_{19_Middle_36}$ in the class $19_{_36}$ in when the number of integral values is 36 consists of the sum(=$ITG_{139_Middle_276}$ +$ITG_{140_Middle_276}$+,...,+$ITG_{146_Middle_276}$) from integral value $ITG_{139_Middle_276}$ in class $139_{_276}$ to integral value $ITG_{146_Middle_276}$ in class $146_{_276}$ in when the number of integral values is 276, and the integral value $ITG_{19_High_36}$ in the class $19_{_36}$ in when the number of integral values is 36 consists of the sum(=$ITG_{139_High_276}$+ $ITG_{140_High_276}$+,..., +$ITG_{146_High_276}$) from integral value $ITG_{139_High_276}$ in class $139_{_276}$ to integral value $ITG_{146_High_276}$ in class $146_{_276}$ in when the number of integral values is 276.

Also, the integral value $ITG_{20_Low_36}$ in the class $20_{_36}$ in when the number of integral values is 36 consists of the sum(=$ITG_{147_Low_276}$+$ITG_{148_Low_276}$+,...,+$ITG_{154_Low_276}$) from integral value $ITG_{147_Low_276}$ in class $147_{_276}$ to integral value $ITG_{154_Low_276}$ in class $154_{_276}$ in when the number of integral values is 276, the integral value $ITG_{20_Middle_36}$ in the class $20_{_36}$ in when the number of integral values is 36 consists of the sum (=$ITG_{147_Middle_276}$+ $ITG_{148Middle_276}$+ ,...,+ $ITG_{154_Middle_276}$) from integral value $ITG_{147_Middle_276}$ in class $147_{_276}$ to integral value $ITG_{154_Middle_276}$ in class $154_{_276}$ in when the number of integral values is 276, and the integral value $ITG_{20_High_36}$ in the class $20_{_36}$ in when the number of integral values is 36 consists of the sum (=$ITG_{147_High_276}$+ $ITG_{148_High_276}$+ ,...,+ $ITG_{154_High_276}$) from integral value $ITG_{147_High_276}$ in class $147_{_276}$ to integral value $ITG_{154_High_276}$ in class $154_{_276}$ in when the number of integral values is 276.

Similarly, the integral value $ITG_{35_Low_36}$ in the class $35_{_36}$ in when the number of integral values is 36 consists of the sum(=$ITG_{267_Low_276}$+$ITG_{268_Low_276}$+,...,+$ITG_{274_Low_276}$) from integral value $ITG_{267_Low_276}$ in class $267_{_276}$ to integral value $ITG_{274_Low_276}$ in class $274_{_276}$ in when the number of integral values is 276, the integral value $ITG_{35_Middle_36}$ in the class $35_{_36}$ in when the number of integral values is 36 consists of the sum(=$ITG_{267_Middle_276}$ +$ITG_{268_Middle_276}$+,...,+$ITG_{274_Middle_276}$) from integral value $ITG_{267_Middle_276}$ in class $267_{_276}$ to integral value $ITG_{274_Middle_276}$ in class $274_{_276}$ in when the number of integral values is 276, and the integral value $ITG_{35_High_36}$ in the class $35_{_36}$ in when the number of integral values is 36 consists of the sum(=$ITG_{267_High_276}$+ $ITG_{268_High_276}$ +,...,+$ITG_{274_High_276}$) from integral value $ITG_{267_High_276}$ in class $267_{_276}$ to integral value $ITG_{274_High_276}$ in class $274_{_276}$ in when the number of integral values is 276.

Then, the integral value $ITG_{36_Low_36}$ in the class $36_{_36}$ in when the number of integral values is 36 consists of the sum(=$ITG_{275_Low_276}$+$ITG_{276_Low_276}$) of integral value $ITG_{275_Low_276}$ in class $275_{_276}$ and integral value $ITG_{276_Low_276}$ in class $276_{_276}$ in when the number of integral values is 276, the integral value $ITG_{36_Middle_36}$ in the class $36_{_36}$ in when the number of integral values is 36 consists of the sum(=$ITG_{275_Middle_276}$+ $ITG_{276_Middle_276}$) of integral value $ITG_{275_Middle276}$ in class $275_{_276}$ and integral value $ITG_{276_Middle_276}$ in class $276_{_276}$ in when the number of integral values is 276, and the integral value $ITG_{36_High_36}$ in the class $36_{_36}$ in when the number of integral values is 36 consists of the sum(=$ITG_{275_High_276}$+ $ITG_{276_High_276}$) of integral value $ITG_{275_High_276}$ in class $275_{_276}$ and integral value $ITG_{276_High_276}$ in class $276_{_276}$ in when the number of integral values is 276.

Furthermore, when the analyte is "shochu", the class$17_{_32}$ in when the number of integral values is 32 is associated with classes $139_{_276}$ to $147_{_276}$ in when the number of integral values is 276, similarly, the class$31_{_32}$ in when the number of integral values is 32 is associated with classes $265_{_276}$ to $273_{_276}$ in when the number of integral values is 276, the class$32_{_32}$ in when the number of integral values is 32 is associated with classes $274_{_276}$ to $276_{_276}$ in when the number of integral values is 276 (See (g) if Fig. 104).

As a result, the integral value $ITG_{17_Low_32}$ in the class $17_{_32}$ in when the number of integral values is 32 consists of the sum(=$ITG_{139_Low_276}$+$ITG_{140_Low_276}$+,...,+$ITG_{147_Low_276}$) from integral value $ITG_{139_Low_276}$ in class $139_{_276}$ to integral value $ITG_{147_Low_276}$ in class $147_{_276}$ in when the number of integral values is 276, the integral value $ITG_{17_Middle_32}$ in the class $17_{_32}$ in when the number of integral values is 32 consists of the sum(=$ITG_{139_Middle_276}$ +$ITG_{140_Middle_276}$+,...,+$ITG_{147_Middle_276}$) from integral value $ITG_{139_Middke_276}$ in class $139_{_276}$ to integral value $ITG_{147_Middle_276}$ in class $147_{_276}$ in when the number of integral values is 276, and the integral value $ITG_{17_High_32}$ in the class $17_{_32}$ in when the number of integral values is 32 consists of the sum(=$ITG_{139_High_276}$+ $ITG_{140_High_276}$ +,...,+$ITG_{147_High_276}$) from integral value $ITG_{139_High_276}$ in class $139_{_276}$ to integral value $ITG_{147_High_276}$ in class $147_{_276}$ in when the number of integral values is 276.

Similarly, the integral value $ITG_{31_Low_32}$ in the class $31_{_32}$ in when the number of integral values is 32 consists of the sum(=$ITG_{265_Low_276}$+$ITG_{266_Low_276}$+,...,+$ITG_{273_Low_276}$ ) from integral value $ITG_{265_Low_276}$ in class $265_{_276}$ to integral value $ITG_{273_Low_276}$ in class $273_{_276}$ in when the number of integral values is 276, the integral value $ITG_{31_Middle_32}$ in the class $31_{_32}$ in when the number of integral values is 32 consists of the sum (=$ITG_{265_Middle_276}$ +$ITG_{266_Middle_276}$+,...,+$ITG_{273_Middle_276}$) from integral value $ITG_{265_Middle_276}$ in class $265_{_276}$ to integral value $ITG_{273_Middle_276}$ in class $273_{_276}$ in when the number of integral values is 276, and the integral value $ITG_{31_High_32}$ in the class $31_{_32}$ in when the number of integral values is 32 consists of the sum (=$ITG_{265_High_276}$+ $ITG_{266_High_276}$ +,...,+$ITG_{273_High_276}$ ) from integral value $ITG_{265_High_276}$ in class $265_{_276}$ to integral value $ITG_{273_High_276}$ in class $273_{_276}$ in when the number of integral values is 276.

Then, the integral value $ITG_{32_Low_32}$ in the class $32_{_32}$ in when the number of integral values is 32 consists of the sum(=$ITG_{274_Low_276}$+$ITG_{275_Low_276}$+$ITG_{276_Low_276}$) from integral value $ITG_{274_Low_276}$ in class 274$_{_276}$ to integral value $ITG_{276_Low_276}$ in class 276$_{_276}$ in when the number of integral values is 276, the integral value $ITG_{32_Middle_32}$ in the class 32 $_{_32}$ in when the number of integral values is 32 consists of the sum (=$ITG_{274_Middle_276}$+$ITG_{275_Middle_276}$ +$ITG_{276_Middle_276}$) from integral value $ITG_{274_Middle_276}$ in class 274$_{_276}$ to integral value $ITG_{276_Middle_276}$ in class 276$_{_276}$ in when the number of integral values is 276, and the integral value $ITG_{32_High_32}$ in the class 32 $_{_32}$ in when the number of integral values is 32 consists of the sum(=$ITG_{274_High_276}$+ $ITG_{275_High_276}$+$ITG_{276_High_276}$ ) from integral value $ITG_{274_High_276}$ in class 274$_{_276}$ to integral value $ITG_{276_High_276}$ in class 276$_{_276}$ in when the number of integral values is 276.

Furthermore, when the analyte is "shochu", the class11 $_{_20}$ in when the number of integral values is 20 is associated with classes 139 $_{_276}$ to 153 $_{_276}$ in when the number of integral values is 276, similarly, the class19 $_{_20}$ in when the number of integral values is 20 is associated with classes 259 $_{_276}$ to 273$_{_276}$ in when the number of integral values is 276, and the class 20 $_{_20}$ in when the number of integral values is 20 is associated with classes 274 $_{_276}$ to 276$_{_276}$ in when the number of integral values is 276 (See (h) of Fig. 104).

As a result, the integral value $ITG_{11_Low_20}$ in the class 11 $_{_20}$ in when the number of integral values is 20 consists of the sum(=$ITG_{139_Low_276}$+$ITG_{140_Low_276}$+,..., $ITG_{153_Low_276}$) from integral value $ITG_{139_Low__276}$ in class 139$_{_276}$ to integral value $ITG_{153_Low_276}$ in class 153 $_{_276}$ in when the number of integral values is 276, the integral value $ITG_{11_Middle_20}$ in the class 11 $_{_20}$ in when the number of integral values is 20 consists of the sum(=$ITG_{139_Middle_276}$ +$ITG_{140_Middle_276}$+,...,$ITG_{153_Middle_276}$) from integral value $ITG_{139_Middle_276}$ in class 139$_{_276}$ to integral value $ITG_{153_Middle_276}$ in class 153 $_{_276}$ in when the number of integral values is 276, and the integral value $ITG_{11_High_20}$ in the class 11 $_{_20}$ in when the number of integral values is 20 consists of the sum(=$ITG_{139_High_276}$+ $ITG_{140_High_276}$ +,...,$ITG_{153_High_276}$) from integral value $ITG_{139_High_276}$ in class 139$_{_276}$ to integral value $ITG_{153_High_276}$ in class 153 $_{_276}$ in when the number of integral values is 276.

Similarly, the integral value $ITG_{19_Low_20}$ in the class 19 $_{_20}$ in when the number of integral values is 20 consists of the sum(=$ITG_{259_Low_276}$+$ITG_{260_Low_276}$+,...,+$ITG_{273_Low_276}$) from integral value $ITG_{259_Low_276}$ in class 259$_{_276}$ to integral value $ITG_{273_Low_276}$ in class 273$_{_276}$ in when the number of integral values is 276, the integral value $ITG_{19_Middle_20}$ in the class 19 $_{_20}$ in when the number of integral values is 20 consists of the sum(=$ITG_{259_Middle_276}$ +$ITG_{260_Middle_276}$+,...,+$ITG_{273_Middle_276}$) from integral value $ITG_{259_Middle_276}$ in class 259$_{_276}$ to integral value $ITG_{273_Middle_276}$ in class 273$_{_276}$ in when the number of integral values is 276, and the integral value $ITG_{19_High_20}$ in the class 19 $_{_20}$ in when the number of integral values is 20 consists of the sum(=$ITG_{259_High_276}$+ $ITG_{260_High_276}$+,..., +$ITG_{273_High_276}$) from integral value $ITG_{259_High_276}$ in class 259$_{_276}$ to integral value $ITG_{273_High_276}$ in class 273$_{_276}$ in when the number of integral values is 276.

Then, the integral value $ITG_{20_Low_20}$ in the class 20 $_{_20}$ in when the number of integral values is 20 consists of the sum(=$ITG_{274_Low_276}$+$ITG_{275_Low_276}$+$ITG_{276_Low_276}$) from integral value $ITG_{274_Low_276}$ in class 274$_{_276}$ to integral value $ITG_{276_Low_276}$ in class 276$_{_276}$ in when the number of integral values is 276, the integral value $ITG_{20_Middle_20}$ in the class 20 $_{_20}$ in when the number of integral values is 20 consists of the sum(=$ITG_{274_Middle_276}$+$ITG_{275_Middle_276}$ +$ITG_{276_Middle_276}$) from integral value $ITG_{274_Middle_276}$ in class 274$_{_276}$ to integral value $ITG_{276_Middle_276}$ in class 276$_{_276}$ in when the number of integral values is 276, and the integral value $ITG_{20_High_20}$ in the class 20 $_{_20}$ in when the number of integral values is 20 consists of the sum(=$ITG_{274_High_276}$+ $ITG_{275_High_276}$+$ITG_{276_High_276}$) from integral value $ITG_{274_High_276}$ in class 274$_{_276}$ to integral value $ITG_{276_High_276}$ in class 276$_{_276}$ in when the number of integral values is 276.

Furthermore, when the analyte is "shochu", the class10 $_{_18}$ in when the number of integral values is 18 is associated with classes 139 $_{_276}$ to 155 $_{_276}$ in when the number of integral values is 276, similarly, the class17 $_{_18}$ in when the number of integral values is 18 is associated with classes 258 $_{_276}$ to 274$_{_276}$ in when the number of integral values is 276, and the class18 $_{_18}$ in when the number of integral values is 18 is associated with classes 275$_{_276}$ and 276$_{_276}$ in when the number of integral values is 276 (See (i) of Fig. 104).

As a result, the integral value $ITG_{10_Low_18}$ in the class 10 $_{_18}$ in when the number of integral values is 18 consists of the sum(=$ITG_{139_Low_276}$+$ITG_{140_Low_276}$+,...,$ITG_{155_Low_276}$) from integral value $ITG_{139_Low_276}$ in class 139$_{_276}$ to integral value $ITG_{155_Low_276}$ in class 155 $_{_276}$ in when the number of integral values is 276, the integral value $ITG_{10_Middle_18}$ in the class 10 $_{_18}$ in when the number of integral values is 18 consists of the sum(=$ITG_{139_Middle_276}$ +$ITG_{140_Middle_276}$+,...,$ITG_{155_Middle_276}$) from integral value $ITG_{139_Middle_276}$ in class 139$_{_276}$ to integral value $ITG_{155_Middle_276}$ in class 155 $_{_276}$ in when the number of integral values is 276, and the integral value $ITG_{10_High_18}$ in the class 10 $_{_18}$ in when the number of integral values is 18 consists of the sum(=$ITG_{139_High_276}$+ $ITG_{140_High_276}$ +,...,$ITG_{155_High_276}$) from integral value $ITG_{139_High_276}$ in class 139 $_{_276}$ to integral value $ITG_{155_High_276}$ in class 155 $_{_276}$ in when the number of integral values is 276.

Similarly, the integral value $ITG_{17_Low_18}$ in the class 17 $_{_18}$ in when the number of integral values is 18 consists of the sum(=$ITG_{258_Low_276}$+$ITG_{259_Low_276}$+,...,$ITG_{274_Low_276}$) from integral value $ITG_{258_Low_276}$ in class 258$_{_276}$ to integral value $ITG_{274_Low_276}$ in class 274$_{_276}$ in when the number of integral values is 276, the integral value $ITG_{17_Middle_18}$ in the class 17 $_{_18}$ in when the number of integral values is 18 consists of the sum(=$ITG_{258_Middle_276}$

$+ITG_{259_Middle_276}+,...,ITG_{274_Middle_276}$) from integral value $ITG_{258_Middle_276}$ in class $258_{_276}$ to integral value $ITG_{274_Middle_276}$ in class $274_{_276}$ in when the number of integral values is 276, and the integral value $ITG_{17_High_18}$ in the class $17_{_18}$ in when the number of integral values is 18 consists of the sum(=$ITG_{258_High_276}+ITG_{259_High_276}+,...,ITG_{274_High_276}$) from integral value $ITG_{258_High_276}$ in class $258_{_276}$ to integral value $ITG_{274_High_276}$ in class $274_{_276}$ in when the number of integral values is 276.

Then, the integral value $ITG_{18_Low_18}$ in the class $18_{_18}$ in when the number of integral values is 18 consists of the sum(=$ITG_{275_Low_276}+ITG_{276_Low_276}$) of integral value $ITG_{275_Low_276}$ in class $275_{_276}$ and integral value $ITG_{276_Low_276}$ in class $276_{_276}$ in when the number of integral values is 276, the integral value $ITG_{18_Middle_18}$ in the class $18_{_18}$ in when the number of integral values is 18 consists of the sum(=$ITG_{275_Middle_276}+ITG_{276_Middle_276}$) of integral value $ITG_{275_Middle_276}$ in class $275_{_276}$ and integral value $ITG_{276_Middle_276}$ in class $276_{_276}$ in when the number of integral values is 276, and the integral value $ITG_{18_High_18}$ in the class $18_{_18}$ in when the number of integral values is 18 consists of the sum(=$ITG_{275_High_276}+ITG_{276_High_276}$) of integral value $ITG_{275_High_276}$ in class $275_{_276}$ and integral value $ITG_{276_High_276}$ in class $276_{_276}$ in when the number of integral values is 276.

According to the correspondence of the classes shown in Fig. 103, by associating "classes $1_{_138}$ to $69_{_138}$ in when the number of integral values is 138, classes $1_{_92}$ to $46_{_92}$ in when the number of integral values is 92, classes $1_{_70}$ to $35_{_70}$ in when the number of integral values is 70, classes $1_{_56}$ to $28_{_56}$ in when the number of integral values is 56, classes $1_{_36}$ to $18_{_36}$ in when the number of integral values is 36, classes $1_{_32}$ to $16_{_32}$ in when the number of integral values is 32, and classes $1_{_20}$ to $10_{_20}$ in when the number of integral values is 20 and classes $1_{_18}$ to $9_{_18}$ in when the number of integral values is 18" with "classes $1_{_276}$ to $138_{_276}$ in when the number of integral values is 276", it is possible to match "the sum of the integral values in the positive predetermined potential section" to "the sum of the integral values in classes $1_{_276}$ to $138_{_276}$ (=the positive predetermined potential section) in when the number of integral values is 276" even if the number of integral values is any one of 138, 92, 70, 56, 36, 32, 20, and 18.

Also, according to the correspondence of the classes shown in Fig. 104, by associating "classes $70_{_138}$ to $138_{_138}$ in when the number of integral values is 138, classes $47_{_92}$ to $92_{_92}$ in when the number of integral values is 92, classes $36_{_70}$ to $70_{_70}$ in when the number of integral values is 70, classes $29_{_56}$ to $56_{_56}$ in when the number of integral values is 56, classes $19_{_36}$ to $36_{_36}$ in when the number of integral values is 36, classes $17_{_32}$ to $32_{_32}$ in when the number of integral values is 32, and classes $11_{_20}$ to $20_{_20}$ in when the number of integral values is 20 and classes $10_{_18}$ to $18_{_18}$ in when the number of integral values is 18" with "classes $139_{_276}$ to $276_{_276}$ in when the number of integral values is 276", it is possible to match "the sum of the integral values in the negative predetermined potential section" to "the sum of the integral values in classes $139_{_276}$ to $276_{_276}$ (= the negative predetermined potential section) in when the number of integral values is 276" even if the number of integral values is any one of 138, 92, 70, 56, 36, 32, 20, and 18.

As a result, by associating "classes $1_{_138}$ to $138_{_138}$ in when the number of integral values is 138, classes $1_{_92}$ to $92_{_92}$ in when the number of integral values is 92, classes $1_{_70}$ to $70_{_70}$ in when the number of integral values is 70, classes $1_{_56}$ to $56_{_56}$ in when the number of integral values is 56, classes $1_{_36}$ to $36_{_36}$ in when the number of integral values is 36, classes $1_{_32}$ to $32_{_32}$ in when the number of integral values is 32, classes $1_{_20}$ to $20_{_20}$ when in the number of integral values is 20 and classes $1_{_18}$ to $18_{_18}$ in when the number of integral values is 18" with "classes $1_{_276}$ to $276_{_276}$ in when the number of integral values is 276", it is possible to match "the sum of the integral values in the all predetermined potential section (=the positive predetermined potential section + the negative predetermined potential section)" to "the sum of the integral values in classes $1_{_276}$ to $276_{_276}$ in when the number of integral values is 276" even if the number of integral values is any one of 138, 92, 70, 56, 36, 32, 20, and 18.

Therefore, the taste diagnosis results for shochu in when the number of integral values is any of 18, 20, 32, 36, 56, 70, 92, and 138 coincide with the taste diagnosis result (=the taste diagnosis result shown in Fig. 29) for shochu in when the number of integral values is 276.

Therefore, the diagnostic device 2 may perform a taste diagnosis of "shochu" using the above-mentioned method based on the correspondence (see Fig. 12) between the classes and the integral values in when the number of integral values is any of 18, 20, 32, 36, 56, 70, 92, 138 and 276.

Fig. 105 is a diagram showing the correspondence relationship of classes in the case that the number of integral values in when the analyte is "grapes" is 7, 9, 11, 13, 15, 19, 25, 37 and 74.

Referring to Fig. 105, when the analyte is "grapes", class $1_{_37}$ in when the number of integral values is 37 is corresponded to classes $1_{_74}$ and $2_{_74}$ in when the number of integral values is 74, class $2_{_37}$ in when the number of integral values is 37 is corresponded to classes $3_{_74}$ and $4_{_74}$ in when the number of integral values is 74, class $3_{_37}$ in when the number of integral values is 37 is corresponded to classes $5_{_74}$ and $6_{_74}$ in when the number of integral values is 74, similarly, class $36_{_37}$ in when the number of integral values is 37 is corresponded to classes $71_{_74}$ and $72_{_74}$ in when the number of integral values is 74, and class $37_{_37}$ in when the number of integral values is 37 is corresponded to classes $73_{_74}$ and $74_{_74}$ in when the number of integral values is 74 (See (b) of Fig. 105).

As a result, an integral value $ITG_{1_Low_37}$ in class $1_{_37}$ in when the number of integral values is 37 consists of the sum (=$ITG_{1_Low_74}+ITG_{2_Low_74}$) of an integral value $ITG_{1_Low_74}$ in class $1_{_74}$ and an integral value $ITG_{2_Low_74}$ in class $2_{_74}$ in when the number of integral values is 74, an integral value $ITG_{1_Middle_37}$ in class $1_{_37}$ in when the number of integral values is 37 consists of the sum (=$ITG_{1_Middle_74}$+$ITG_{2_Middle_74}$) of an integral value $ITG_{1_Middle_74}$ in class 1$_{_74}$ and an integral value $ITG_{2_Middle_74}$ in class 2$_{_74}$ in when the number of integral values is 74, and an integral value $ITG_{1_High_37}$ in class 1$_{_37}$ in when the number of integral values is 37 consists of the sum (=$ITG_{1_High_74}$+$ITG_{2_High_74}$) of an integral value $ITG_{1_High_74}$ in class 1$_{_74}$ and an integral value $ITG_{2_High_74}$ in class 2$_{_74}$ in when the number of integral values is 74.

Also, an integral value $ITG_{2_Low_37}$ in class 2$_{_37}$ in when the number of integral values is 37 consists of the sum (=$ITG_{3_Low_74}$+$ITG_{4_Low_74}$) of an integral value $ITG_{3_Low_74}$ in class 3$_{_74}$ and an integral value $ITG_{4_Low_74}$ in class 4$_{_74}$ in when the number of integral values is 74, an integral value $ITG_{2_Middle_37}$ in class 2$_{_37}$ in when the number of integral values is 37 consists of the sum (=$ITG_{3_Middle_74}$+$ITG_{4_Middle_74}$) of an integral value $ITG_{3_Middle_74}$ in class 3$_{_74}$ and an integral value $ITG_{4_Middle_74}$ in class 4$_{_74}$ in when the number of integral values is 74, and an integral value $ITG_{2_High_37}$ in class 2$_{_37}$ in when the number of integral values is 37 consists of the sum (=$ITG_{3_High_74}$+$ITG_{4_High_74}$) of an integral value $ITG_{3_High_74}$ in class 3$_{_74}$ and an integral value $ITG_{4_High_74}$ in class 4$_{_74}$ in when the number of integral values is 74.

Similarly, an integral value $ITG_{37_Low_37}$ in class 37$_{_37}$ in when the number of integral values is 37 consists of the sum (=$ITG_{73_Low_74}$+$ITG_{74_Low_74}$) of an integral value $ITG_{73_Low_74}$ in class 73$_{_74}$ and an integral value $ITG_{74_Low_74}$ in class 74$_{_74}$ in when the number of integral values is 74, an integral value $ITG_{3_Middle_37}$ in class 3$_{_37}$ in when the number of integral values is 37 consists of the sum (=$ITG_{73_Middle_74}$+$ITG_{74_Middle_74}$) of an integral value $ITG_{73_Middle_74}$ in class 73$_{_74}$ and an integral value $ITG_{74_Middle_74}$ in class 74$_{_74}$ in when the number of integral values is 74, and an integral value $ITG_{3_High_37}$ in class 3$_{_37}$ in when the number of integral values is 37 consists of the sum (=$ITG_{73_High_74}$+$ITG_{74_High_74}$) of an integral value $ITG_{73_High_74}$ in class 73$_{_74}$ and an integral value $ITG_{74_High_74}$ in class 74$_{_74}$ in when the number of integral values is 74.

Also, when the analyte is "grapes", class 1$_{_25}$ in when the number of integral values is 25 corresponds to classes 1$_{_74}$ to 3$_{_74}$ in when the number of integral values is 74, class 2$_{_25}$ in when the number of integral values is 25 corresponds to classes 4$_{_74}$ to 6$_{_74}$ in when the number of integral values is 74, class 3$_{_25}$ in when the number of integral values is 25 corresponds to classes 7$_{_74}$ to 9$_{_74}$ in when the number of integral values is 74, similarly, class 24$_{_25}$ in when the number of integral values is 25 corresponds to classes 70$_{_74}$ to 72$_{_74}$ in when the number of integral values is 74, and class 25$_{_25}$ in when the number of integral values is 25 corresponds to classes 73$_{_74}$ and 74$_{_74}$ in when the number of integral values is 74 (See (c) of Fig. 105).

As a result, an integral value $ITG_{1_Low_25}$ in class 1$_{_25}$ in when the number of integral values is 25 consists of the sum (=$ITG_{1_Low_74}$+$ITG_{2_Low_74}$+$ITG_{3_Low74}$) of an integral value $ITG_{1_Low_74}$ in class 1$_{_74}$, an integral value $ITG_{2_Low_74}$ in class 2$_{_74}$, and an integral value $ITG_{3_Low_74}$ in class 3$_{_74}$ in when the number of integral values is 74, an integral value $ITG_{1_Middle_25}$ in class 1$_{_25}$ in when the number of integral values is 25 consists of the sum (=$ITG_{1Middle_74}$+$ITG_{2_Middle_74}$+$ITG_{3_Middle_74}$) of an integral value $ITG_{1_Middle_74}$ in class 1$_{_74}$, an integral value $ITG_{2_Middle_74}$ in class 2$_{_74}$, and an integral value $ITG_{3_Middle_74}$ in class 3$_{_74}$ in when the number of integral values is 74, and an integral value $ITG_{1_High_25}$ in class 1$_{_25}$ in when the number of integral values is 25 consists of the sum (=$ITG_{1_High_74}$+$ITG_{2_High_74}$+$ITG_{3_Low74}$) of an integral value $ITG_{1_High_74}$ in class 1$_{_74}$, an integral value $ITG_{2_High_74}$ in class 2$_{_74}$, and an integral value $ITG_{3_High_74}$ in class 3$_{_74}$ in when the number of integral values is 74.

Also, an integral value $ITG_{2_Low_25}$ in class 2$_{_25}$ in when the number of integral values is 25 consists of the sum (=$ITG_{4_Low_74}$+$ITG_{5_Low_74}$+$ITG_{6_Low_74}$) of an integral value $ITG_{4_Low_74}$ in class 4$_{_74}$, an integral value $ITG_{5_Low_74}$ in class 5$_{_74}$, and an integral value $ITG_{6_Low_74}$ in class 6$_{_74}$ in when the number of integral values is 74, an integral value $ITG_{2_Middle_25}$ in class 2$_{_25}$ in when the number of integral values is 25 consists of the sum (=$ITG_{4Middle_74}$+$ITG_{5_Middle_74}$+$ITG_{6_Middle_74}$) of an integral value $ITG_{4_Middle_74}$ in class 4$_{_74}$, an integral value $ITG_{5_Middle_74}$ in class 5$_{_74}$, and an integral value $ITG_{6_Middle_74}$ in class 6$_{_74}$ in when the number of integral values is 74, and an integral value $ITG_{2_High_25}$ in class 2$_{_25}$ in when the number of integral values is 25 consists of the sum (=$ITG_{4_High_74}$+ $ITG_{5_High_74}$+$ITG_{6_High_74}$) of an integral value $ITG_{4_High_74}$ in class 4$_{_74}$, an integral value $ITG_{5_High_74}$ in class 5$_{_74}$, and an integral value $ITG_{6_High_74}$ in class 6$_{_74}$ in when the number of integral values is 74.

Similarly, an integral value $ITG_{24_Low_25}$ in class 24$_{_25}$ in when the number of integral values is 25 consists of the sum (=$ITG_{70_Low_74}$+$ITG_{71_Low_74}$+$ITG_{72_Low_74}$) of an integral value $ITG_{70_Low_74}$ in class 70$_{_74}$, an integral value $ITG_{71_Low_74}$ in class 71$_{_74}$, and an integral value $ITG_{72_Low_74}$ in class 72$_{_74}$ in when the number of integral values is 74, an integral value $ITG_{24_Middle_25}$ in class 24$_{_25}$ in when the number of integral values is 25 consists of the sum (=$ITG_{70_Middle_74}$+$ITG_{71_Middle_74}$+$ITG_{72_Middle_74}$) of an integral value $ITG_{70_Middle_74}$ in class 70$_{_74}$, an integral value $ITG_{71_Middle_74}$ in class 71$_{_74}$, and an integral value $ITG_{72_Middle_74}$ in class 72$_{_74}$ in when the number of integral values is 74, and an integral value $ITG_{24_High_25}$ in class 24$_{_25}$ in when the number of integral values is 25 consists of the sum (=$ITG_{70_High_74}$+$ITG_{71_High_74}$+$ITG_{72_High_74}$) of an integral value $ITG_{70_High_74}$ in class 70$_{_74}$, an integral value $ITG_{71_High_74}$ in class 71$_{_74}$, and an integral value $ITG_{72_High_74}$ in class 72$_{_74}$ in when the number of integral values is 74.

Also, an integral value $ITG_{25_Low_25}$ in class 25$_{_25}$ in when the number of integral values is 25 consists of the sum (=$ITG_{73_Low_74}$+$ITG_{74_Low_74}$) of an integral value $ITG_{73_Low_74}$ in class 73$_{_74}$, and an integral value $ITG_{74_Low_74}$ in class 74$_{_74}$ in when the number of integral values is 74, an integral value $ITG_{25_Middle_25}$ in class 25$_{_25}$ in when the number of integral values is 25 consists of the sum (=$ITG_{73_Middle_74}$+$ITG_{74_Middle_74}$) of an integral value $ITG_{73_Middle_74}$ in class 73$_{_74}$ and an integral value $ITG_{74_Middle_74}$ in class 74$_{_74}$ in when the number of integral values is 74, and an integral value

$ITG_{25_High_25}$ in class 25 $_{_25}$ in when the number of integral values is 25 consists of the sum (=$ITG_{73_High_74}$+$ITG_{74_High_74}$) of an integral value $ITG_{73_High_74}$ in class 73 $_{_74}$, and an integral value $ITG_{74_High_74}$ in class 74 $_{_74}$ in when the number of integral values is 74.

Furthermore, when the analyte is "grapes", class 1 $_{_19}$ in when the number of integral values is 19 corresponds to classes 1 $_{_74}$ to 4 $_{_74}$ in when the number of integral values is 74, class 2 $_{_19}$ in when the number of integral values is 19 corresponds to classes 5 $_{_74}$ to 8 $_{_74}$ in when the number of integral values is 74, class 3 $_{_19}$ in when the number of integral values is 19 corresponds to classes 9 $_{_74}$ to 12 $_{_74}$ in when the number of integral values is 74, similarly, class 18 $_{_19}$ in when the number of integral values is 19 corresponds to classes 69 $_{_74}$ to 72 $_{_74}$ in when the number of integral values is 74, and class 19 $_{_19}$ in when the number of integral values is 19 corresponds to classes 73 $_{_74}$ and 74 $_{_74}$ in when the number of integral values is 74 (See (d) of Fig. 105).

As a result, an integral value $ITG_{1_Low_19}$ in class 1 $_{_19}$ in when the number of integral values is 19 consists of the sum (=$ITG_{1_Low_74}$+$ITG_{2_Low_74}$+$ITG_{3_Low_74}$+$ITG_{4_Low_74}$) from an integral value $ITG_{1_Low_74}$ in class 1 $_{_74}$ to an integral value $ITG_{4_Low_74}$ in class 4 $_{_74}$ in when the number of integral values is 74, an integral value $ITG_{1_Middle_19}$ in class 1 $_{_19}$ in when the number of integral values is 19 consists of the sum (=$ITG_{1_Middle_74}$+$ITG_{2_Middle_74}$+$ITG_{3_Middle_74}$+$ITG_{4_Middle_74}$) from an integral value $ITG_{1_Middle_74}$ in class 1 $_{_74}$ to an integral value $ITG_{4_Middle_74}$ in class 4 $_{_74}$ in when the number of integral values is 74, and an integral value $ITG_{1_High_19}$ in class 1 $_{_19}$ in when the number of integral values is 19 consists of the sum (=$ITG_{1_High_74}$+ $ITG_{2_High_74}$+ $ITG_{3_High_74}$+ $ITG_{4_High_74}$) from an integral value $ITG_{1_High_74}$ in class 1 $_{_74}$ to an integral value $ITG_{4_High_74}$ in class 4 $_{_74}$ in when the number of integral values is 74.

Also, an integral value $ITG_{2_Low_19}$ in class 2 $_{_19}$ in when the number of integral values is 19 consists of the sum (=$ITG_{5_Low_74}$+$ITG_{6_Low_74}$+$ITG_{7_Low_74}$+$ITG_{8_Low_74}$) from an integral value $ITG_{5_Low_74}$ in class 5 $_{_74}$ to an integral value $ITG_{8_Low_74}$ in class 8 $_{_74}$ in when the number of integral values is 74, an integral value $ITG_{2_Middle_19}$ in class 2 $_{_19}$ in when the number of integral values is 19 consists of the sum (=$ITG_{5_Middle_74}$+ $ITG_{6_Middle_74}$+$ITG_{7_Middle_74}$+$ITG_{8_Middle_74}$) from an integral value $ITG_{5_Middle_74}$ in class 5 $_{_74}$ to an integral value $ITG_{8_Middle_74}$ in class 8 $_{_74}$ in when the number of integral values is 74, and an integral value $ITG_{2_High_19}$ in class 2 $_{_19}$ in when the number of integral values is 19 consists of the sum (=$ITG_{5_High_74}$+$ITG_{6_High_74}$+$ITG_{7_High_74}$+$ITG_{8_High_74}$) from an integral value $ITG_{5_High_74}$ in class 5 $_{_74}$ to an integral value $ITG_{8_High_74}$ in class 8 $_{_74}$ in when the number of integral values is 74.

Similarly, an integral value $ITG_{18_Low_19}$ in class 18 $_{_19}$ in when the number of integral values is 19 consists of the sum (=$ITG_{69_Low_74}$+$ITG_{70_Low_74}$+$ITG_{71_Low_74}$+$ITG_{72_Low_74}$) from an integral value $ITG_{69_Low_74}$ in class 69 $_{_74}$ to an integral value $ITG_{72_Low_74}$ in class 72 $_{_74}$ in when the number of integral values is 74, an integral value $ITG_{18_Middle_19}$ in class 18 $_{_19}$ in when the number of integral values is 19 consists of the sum (=$ITG_{69_Middle_74}$+ $ITG_{70_Middle_74}$+ $ITG_{71_Middle_74}$+ $ITG_{72_Middle_74}$) from an integral value $ITG_{69_Middle_74}$ in class 69 $_{_74}$ to an integral value $ITG_{72_Middle_74}$ in class 72 $_{_74}$ in when the number of integral values is 74, and an integral value $ITG_{18_High_19}$ in class 18 $_{_19}$ in when the number of integral values is 19 consists of the sum (=$ITG_{69_High_74}$+ $ITG_{70_High_74}$+ $ITG_{71_High_74}$+$ITG_{72_High_74}$) from an integral value $ITG_{69_High_74}$ in class 69 $_{_74}$ to an integral value $ITG_{72_High_74}$ in class 72 $_{_74}$ in when the number of integral values is 74.

And, an integral value $ITG_{19_Low_19}$ in class 19 $_{_19}$ in when the number of integral values is 19 consists of the sum (=$ITG_{73_Low_74}$+$ITG_{74_Low_74}$) of an integral value $ITG_{73_Low_74}$ in class 73 $_{_74}$ and an integral value $ITG_{74_Low_74}$ in class 74 $_{_74}$ in when the number of integral values is 74, an integral value $ITG_{19_Middle_19}$ in class 19 $_{_19}$ in when the number of integral values is 19 consists of the sum (=$ITG_{73_Middle_74}$+$ITG_{74_Middle_74}$) of an integral value $ITG_{73_Middle_74}$ in class 73 $_{_74}$ and an integral value $ITG_{74_Middle_74}$ in class 74 $_{_74}$ in when the number of integral values is 74, and an integral value $ITG_{19_High_19}$ in class 19 $_{_19}$ in when the number of integral values is 19 consists of the sum (=$ITG_{73_High_74}$+$ITG_{74_High_74}$) of an integral value $ITG_{73_High_74}$ in class 73 $_{_74}$ and an integral value $ITG_{74_High_74}$ in class 74 $_{_74}$ in when the number of integral values is 74.

Furthermore, when the analyte is "grapes", class 1 $_{_15}$ in when the number of integral values is 15 corresponds to classes 1 $_{_74}$ to 5 $_{_74}$ in when the number of integral values is 74, class 2 $_{_15}$ in when the number of integral values is 15 corresponds to classes 6 $_{_74}$ to 10 $_{_74}$ in when the number of integral values is 74, class 3 $_{_15}$ in when the number of integral values is 15 corresponds to classes 11 $_{_74}$ to 15 $_{_74}$ in when the number of integral values is 74, similarly, class 14 $_{_15}$ in when the number of integral values is 15 corresponds to classes 66 $_{_74}$ to 70 $_{_74}$ in when the number of integral values is 74, and class 15 $_{_15}$ in when the number of integral values is 15 corresponds to classes 71 $_{_74}$ to 74 $_{_74}$ in when the number of integral values is 74 (See of (e) in Fig. 105).

As a result, an integral value $ITG_{1_Low_15}$ in class 1 $_{_15}$ in when the number of integral values is 15 consists of the sum (=$ITG_{1_Low_74}$+ $ITG_{2_Low_74}$+ $ITG_{3_Low_74}$+ $ITG_{4_Low_74}$+ $ITG_{5_Low_74}$) from an integral value $ITG_{1_Low_74}$ in class 1 $_{_74}$ to an integral value $ITG_{5_Low_74}$ in class 5 $_{_74}$ in when the number of integral values is 74, an integral value $ITG_{1_Middle_15}$ in class 1 $_{_15}$ in when the number of integral values is 15 consists of the sum (=$ITG_{1_Middle_74}$+ $ITG_{2_Middle_74}$+ $ITG_{3_Middle_74}$ $ITG_{4_Middle_74}$+ $ITG_{5_Middle_74}$) from an integral value $ITG_{1_Middle_74}$ in class 1 $_{_74}$ to an integral value $ITG_{5_Middle_74}$ in class 5 $_{_74}$ in when the number of integral values is 74, and an integral value $ITG_{1_High_15}$ in class 1 $_{_15}$ in when the number of integral values is 15 consists of the sum (=$ITG_{1_High_74}$+ $ITG_{2_High_74}$+ $ITG_{3_High_74}$+ $ITG_{4_High_74}$+

$ITG_{5_High_74}$) from an integral value $ITG_{1_High_74}$ in class $1_{_74}$ to an integral value $ITG_{5_High_74}$ in class $5_{_74}$ in when the number of integral values is 74.

Also, an integral value $ITG_{2_Low_15}$ in class $2_{_15}$ in when the number of integral values is 15 consists of the sum (=$ITG_{6_Low_74}$+$ITG_{7_Low74}$+$ITG_{8_Low_74}$+$ITG_{9_Low_74}$+$ITG_{10_Low_74}$) from an integral value $ITG_{6_Low_74}$ in class $6_{_74}$ to an integral value $ITG_{10_Low_74}$ in class $10_{_74}$ in when the number of integral values is 74, an integral value $ITG_{2_Middle_15}$ in class $2_{_15}$ in when the number of integral values is 15 consists of the sum (=$ITG_{6_Middle_74}$+ $ITG_{7_Middle_74}$+$ITG_{8_Middle_74}$ +$ITG_{9_Middle_74}$+$ITG_{10_Middle_74}$) from an integral value $ITG_{6_Middle_74}$ in class $6_{_74}$ to an integral value $ITG_{10_Middle_74}$ in class $10_{_74}$ in when the number of integral values is 74, and an integral value $ITG_{2_High_15}$ in class $2_{_15}$ in when the number of integral values is 15 consists of the sum (=$ITG_{6_High_74}$+ $ITG_{7_High_74}$+ $ITG_{8_High_74}$+$ITG_{9_High_74}$+$ITG_{10_High_74}$) from an integral value $ITG_{6_High_74}$ in class $6_{_74}$ to an integral value $ITG_{10_High_74}$ in class $10_{_74}$ in when the number of integral values is 74.

Similarly, an integral value $ITG_{14_Low_15}$ in class $14_{_15}$ in when the number of integral values is 15 consists of the sum (=$ITG_{66_Low_74}$+ $ITG_{67_Low_74}$+ $ITG_{68_Low_74}$+ $ITG_{69_Low_74}$+ $ITG_{70_Low_74}$) from an integral value $ITG_{66_Low_74}$ in class $66_{_74}$ to an integral value $ITG_{70_Low_74}$ in class $70_{_74}$ in when the number of integral values is 74, an integral value $ITG_{14_Middle_15}$ in class $14_{_15}$ in when the number of integral values is 15 consists of the sum (=$ITG_{66_Middle_74}$ +$ITG_{67_Middle_74}$+ $ITG_{68_Middle_74}$+ $ITG_{69_Middle_74}$+ $ITG_{70_Middle_74}$) from an integral value $ITG_{66_Middle_74}$ in class $66_{_74}$ to an integral value $ITG_{70_Middle_74}$ in class $70_{_74}$ in when the number of integral values is 74, and an integral $ITG_{14_High_15}$ in class $14_{_15}$ in when the number of integral values is 15 consists of the sum (=$ITG_{66_High_74}$+ ITG67_High_74 +$ITG_{68_High_74}$+$ITG_{69_High_74}$+$ITG_{70_High74}$) from an integral value $ITG_{66_High_74}$ in class $66_{_74}$ to an integral value $ITG_{70_High_74}$ in class $70_{_74}$ in when the number of integral values is 74.

And, an integral value $ITG_{15_Low_15}$ in class $15_{_15}$ in when the number of integral values is 15 consists of the sum (=$ITG_{71_Low_74}$+ $ITG_{72_Low_74}$+ $ITG_{73_Low_74}$+ $ITG_{74_Low_74}$) from an integral value $ITG_{71_Low_74}$ in class $71_{_74}$ to an integral value $ITG_{74_Low_74}$ in class $74_{_74}$ in when the number of integral values is 74, an integral value $ITG_{15_Middle_15}$ in class $15_{_15}$ in when the number of integral values is 15 consists of the sum (=$ITG_{71_Middle_74}$+ $ITG_{72_Middle_74}$+ $ITG_{73_Middle_74}$+ $ITG_{74_Middle_74}$) from an integral value $ITG_{71_Middle_74}$ in class $71_{_74}$ to an integral value $ITG_{74_Middle_74}$ in class $74_{_74}$ in when the number of integral values is 74, and an integral value $ITG_{15_High_15}$ in class $15_{_15}$ in when the number of integral values is 15 consists of the sum (=$ITG_{71_High_74}$+ $ITG_{72_High_74}$+ $ITG_{73_High_74}$+ $ITG_{74_High_74}$) from an integral value $ITG_{71_High_74}$ in class $71_{_74}$ to an integral value $ITG_{74_High_74}$ in class $74_{_74}$ in when the number of integral values is 74.

Furthermore, when the analyte is "grapes", class $1_{_13}$ in when the number of integral values is 13 corresponds to classes $1_{_74}$ to $6_{_74}$ in when the number of integral values is 74, class $2_{_13}$ in when the number of integral values is 13 corresponds to classes $7_{_74}$ to $12_{_74}$ in when the number of integral values is 74, similarly, class $12_{_13}$ in when the number of integral values is 13 corresponds to classes $67_{_74}$ to $72_{_74}$ in when the number of integral values is 74, and class $13_{_13}$ in when the number of integral values is 13 corresponds to classes $73_{_74}$ and $74_{_74}$ in when the number of integral values is 74 (See (f) of Fig. 105).

As a result, an integral value $ITG_{1_Low_13}$ in class $1_{_13}$ in when the number of integral values is 13 consists of the sum (=$ITG_{1_Low_74}$+ $ITG_{2_Low_74}$+ $ITG_{3_Low_74}$ + ,...,+ $ITG_{6_Low_74}$) from an integral value $ITG_{1_Low_74}$ in class $1_{_74}$ to an integral value $ITG_{6_Low_74}$ in class $6_{_74}$ in when the number of integral values is 74, an integral value $ITG_{1_Middle_13}$ in class $1_{_13}$ in when the number of integral values is 13 consists of the sum (=$ITG_{1_Middle_74}$+$ITG_{2_Middle_74}$+$ITG_{3_Middle_74}$ +, ...,+$ITG_{6_Middle_74}$) from an integral value $ITG_{1_Middle_74}$ in class $1_{_74}$ to an integral value $ITG_{6_Middle_74}$ in class $6_{_74}$ in when the number of integral values is 74, and an integral value $ITG_{1_High_13}$ in class $1_{_13}$ in when the number of integral values is 13 consists of the sum (=$ITG_{1_High_74}$+$ITG_{2_High_74}$+ $ITG_{3_High_74}$+,...,+ $ITG_{6_High_74}$) from an integral value $ITG_{1_High_74}$ in class $1_{_74}$ to an integral value $ITG_{6_High_74}$ in class $6_{_74}$ in when the number of integral values is 74.

Also, an integral value $ITG_{2_Low_13}$ in class $2_{_13}$ in when the number of integral values is 13 consists of the sum (=$ITG_{7_Low_74}$+$ITG_{8_Low_74}$+$ITG_{9_Low_74}$+,...,+$ITG_{12_Low_74}$) from an integral value $ITG_{7_Low_74}$ in class $7_{_74}$ to an integral value $ITG_{12_Low_74}$ in class $12_{_74}$ in when the number of integral values is 74, an integral value $ITG_{2_Middle_13}$ in class $2_{_13}$ in when the number of integral values is 13 consists of the sum (=$ITG_{7_Middle_74}$+$ITG_{8_Middle_74}$+$ITG_{9_Middle_74}$+,...,+$ITG_{12_Middle_74}$) from an integral value $ITG_{7_Middle_74}$ in class $7_{_74}$ to an integral value $ITG_{12_Middle_74}$ in class $12_{_74}$ in when the number of integral values is 74, and an integral value $ITG_{2_High_13}$ in class $2_{_13}$ in when the number of integral values is 13 consists of the sum (=$ITG_{7_High_74}$+ $ITG_{8_High_74}$+ $ITG_{9_High_74}$+,...,+ $ITG_{12_High_74}$) from an integral value $ITG_{7_High_74}$ in class $7_{_74}$ to an integral value $ITG_{12_High_74}$ in class $12_{_74}$ in when the number of integral values is 74.

Similarly, an integral value $ITG_{12_Low_13}$ in class $12_{_13}$ in when the number of integral values is 13 consists of the sum (=$ITG_{67_Low_74}$+ $ITG_{68_Low_74}$+ $ITG_{69_Low_74}$+,...,+ $ITG_{72_Low_74}$) from an integral value $ITG_{67_Low_74}$ in class $67_{_74}$ to an integral value $ITG_{72_Low_74}$ in class $72_{_74}$ in when the number of integral values is 74, an integral value $ITG_{12_Middle_13}$ in class $12_{_13}$ in when the number of integral values is 13 consists of the sum (=$ITG_{67_Middle_74}$+ $ITG_{68_Middle_74}$+ $ITG_{69_Middle_74}$+,...,+ $ITG_{72_Middle_74}$) from an integral value $ITG_{67_Middle_74}$ in class $67_{_74}$ to an integral value $ITG_{72_Middle_74}$ in class $72_{_74}$ in when the number of integral values is 74, and an integral value $ITG_{12_High_13}$ in class $12_{_13}$ in when the number of integral values is 13 consists of the sum (=$ITG_{67_High_74}$+ $ITG_{68_High_74}$+$ITG_{69_High_74}$

+,...,+$ITG_{72_High_74}$) from an integral value $ITG_{67_High_74}$ in class 67 $_{_74}$ to an integral value $ITG_{72_High_74}$ in class 72 $_{_74}$ in when the number of integral values is 74.

And, an integral value $ITG_{13_Low_13}$ in class 13 $_{_13}$ in when the number of integral values is 13 consists of the sum (=$ITG_{73_Low_74}$+$ITG_{74_Low_74}$) of an integral value $ITG_{73_Low_74}$ in class 73 $_{_74}$ and an integral value $ITG_{74Low_74}$ in class 74 $_{_74}$ in when the number of integral values is 74, an integral value $ITG_{13Middle_13}$ in class 13 $_{_13}$ in when the number of integral values is 13 consists of the sum (=$ITG_{73_Middle_74}$+$ITG_{74_Middle_74}$) of an integral value $ITG_{73_Middle_74}$ in class 73 $_{_74}$ and an integral value $ITG_{74_Middle_74}$ in class 74 $_{_74}$ in when the number of integral values is 74, and an integral value $ITG_{13_High_13}$ in class 13 $_{_13}$ in when the number of integral values is 13 consists of the sum (=$ITG_{73_High_74}$+$ITG_{74_High_74}$) of an integral value $ITG_{73_High_74}$ in class 73 $_{_74}$ and an integral value $ITG_{74_High_74}$ in class 74 $_{_74}$ in when the number of integral values is 74.

Furthermore, when the analyte is "grapes", class 1 $_{_11}$ in when the number of integral values is 11 corresponds to classes 1 $_{_74}$ to 7 $_{_74}$ in when the number of integral values is 74, class 2 $_{_11}$ in when the number of integral values is 11 corresponds to classes 8 $_{_74}$ to 14 $_{74}$ in when the number of integral values is 74, similarly, class 10 $_{_11}$ in when the number of integral values is 11 corresponds to classes 64 $_{_74}$ to 70 $_{_74}$ in when the number of integral values is 74, and class 11 $_{_11}$ in when the number of integral values is 11 corresponds to classes 71 $_{_74}$ to 74 $_{_74}$ in when the number of integral values is 74 (See (g) of Fig. 105).

As a result, an integral value $ITG_{1_Low_11}$ in class 1 $_{_11}$ in when the number of integral values is 11 consists of the sum (=$ITG_{1_Low_74}$+ $ITG_{2_Low_74}$+ $ITG_{3_Low_74}$+,...,+ $ITG_{7_Low_74}$) from an integral value $ITG_{1_Low_74}$ in class 1 $_{_74}$ to an integral value $ITG_{7_Low_74}$ in class 7 $_{_74}$ in when the number of integral values is 74, an integral value $ITG_{1_Middle_11}$ in class 1 $_{_11}$ in when the number of integral values is 11 consists of the sum (=$ITG_{1_Middle_74}$+$ITG_{2_Middle_74}$+$ITG_{3Middle_74}$ +,...,+$ITG_{7_Middle_74}$) from an integral value $ITG_{1_Middle_74}$ in class 1 $_{_74}$ to an integral value $ITG_{7_Middle_74}$ in class 7 $_{_74}$ in when the number of integral values is 74, and an integral value $ITG_{1_High_11}$ in class 1 $_{_11}$ in when the number of integral values is 11 consists of the sum (=$ITG_{1_High_74}$+ $ITG_{2_High_74}$+ $ITG_{3_High_74}$+,...,+ $ITG_{7_High_74}$) from an integral value $ITG_{1_High_74}$ in class 1 $_{_74}$ to an integral value $ITG_{7_High_74}$ in class 7 $_{_74}$ in when the number of integral values is 74.

Also, an integral value $ITG_{2_Low_11}$ in class 2 $_{_11}$ in when the number of integral values is 11 consists of the sum (=$ITG_{8_Low_74}$+$ITG_{9_Low_74}$+$ITG_{10_Low_74}$+,...,+$ITG_{14_Low_74}$) from an integral value $ITG_{8_Low_74}$ in class 8 $_{_74}$ to an integral value $ITG_{14_Low_74}$ in class 14 $_{_74}$ in when the number of integral values is 74, an integral value $ITG_{2_Middle_11}$ in class 2 $_{_11}$ when the number of integral values is 11 consists of the sum (=$ITG_{8_Middle_74}$+ $ITG_{9_Middle_74}$+$ITG_{10_Middle_74}$ +,...,+$ITG_{14_Middle_74}$) from an integral value $ITG_{8_Middle_74}$ in class 8 $_{_74}$ to an integral value $ITG_{14_Middle_74}$ in class 14 $_{_74}$ in when the number of integral values is 74, and an integral value $ITG_{2_High_11}$ in class 2 $_{_11}$ in when the number of integral values is 11 consists of the sum (=$ITG_{8_High_74}$+ $ITG_{9_High_74}$+ $ITG_{10_High_74}$+,...,+ $ITG_{14_High_74}$) from an integral value $ITG_{8_High_74}$ in class 8 $_{_74}$ to an integral value $ITG_{14_High_74}$ in class 14 $_{_74}$ in when the number of integral values is 74.

Similarly, an integral value $ITG_{10_Low_11}$ in class 10 $_{_11}$ in when the number of integral values is 11 consists of the sum (=$ITG_{64_Low_74}$+ $ITG_{65_Low_74}$+ $ITG_{66_Low_74}$+,...,+ $ITG_{70_Low_74}$) from an integral value $ITG_{64_Low_74}$ in class 64 $_{_74}$ to an integral value $ITG_{70_Low_74}$ in class 70 $_{_74}$ in when the number of integral values is 74, an integral value $ITG_{10_Middle_11}$ in class 10 $_{_11}$ in when the number of integral values is 11 consists of the sum (=$ITG_{64_Middle_74}$+ $ITG_{65_Middle_74}$+ $ITG_{66_Middle_74}$+,...,+ $ITG_{70_Middle_74}$) from an integral value $ITG_{64_Middle_74}$ in class 64 $_{_74}$ to an integral value $ITG_{70_Middle_74}$ in class 70 $_{_74}$ in when the number of integral values is 74, an integral value $ITG_{10_High_11}$ in class 10 $_{_11}$ in when the number of integral values is 11 consists of the sum (=$ITG_{64_High_74}$+ $ITG_{65_High_74}$+ $ITG_{66_High_74}$+,...,+$ITG_{70_High_74}$) from an integral value $ITG_{64_High_74}$ in class 64 $_{_74}$ to an integral value $ITG_{70_High_74}$ in class 70 $_{_74}$ in when the number of integral values is 74.

And, an integral value $ITG_{11_Low_11}$ in class 11 $_{_11}$ in when the number of integral values is 11 consists of the sum (=$ITG_{71_Low_74}$+$ITG_{72_Low_74}$+$ITG_{73_Low_74}$+$ITG_{74_Low_74}$) from an integral value $ITG_{71_Low_74}$ in class 71 $_{_74}$ to an integral value $ITG_{74_Low_74}$ in class 74 $_{_74}$ in when the number of integral values is 74, an integral value $ITG_{11_Middle_11}$ in class 11 $_{_11}$ in when the number of integral values is 11 consists of the sum (=$ITG_{71_Middle_74}$+ $ITG_{72_Middle_74}$ +$ITG_{73_Middle_74}$+$ITG_{74_Middle_74}$) from an integral value $ITG_{71_Middle_74}$ in class 71 $_{_74}$ to an integral value $ITG_{74_Middle_74}$ in class 74 $_{_74}$ in when the number of integral values is 74, and an integral value $ITG_{11_High_11}$ in class 11 $_{_11}$ in when the number of integral values is 11 consists of the sum (=$ITG_{71_High_74}$+ $ITG_{72_High_74}$+ $ITG_{73_High_74}$+ $ITG_{74_High_74}$) from an integral value $ITG_{71_High_74}$ in class 71 $_{_74}$ to an integral value $ITG_{74_High_74}$ in class 74 $_{_74}$ in when the number of integral values is 74.

Furthermore, when the analyte is "grapes", class 1 $_{_9}$ in when the number of integral values is 9 corresponds to classes 1 $_{_74}$ to 9 $_{_74}$ in when the number of integral values is 74, similarly, class 8 $_{_9}$ in when the number of integral values is 9 corresponds to classes 64 $_{_74}$ to 72 $_{_74}$ in when the number of integral values is 74, and class 9 $_{_9}$ in when the number of integral values is 9 corresponds to classes 73 $_{_74}$ and 74 $_{_74}$ in when the number of integral values is 74 (See (h) of Fig. 105).

As a result, an integral value $ITG_{1_Low_9}$ in class 1 $_{_9}$ in when the number of integral values is 9 consists of the sum (=$ITG_{1_Low_74}$+$ITG_{2_Low_74}$+$ITG_{3_Low_74}$+,...,+ $ITG_{9_Low_74}$) from an integral value $ITG_{1_Low_74}$ in class 1 $_{_74}$ to an integral value $ITG_{9_Low_74}$ in class 9 $_{_74}$ in when the number of integral values is 74, an integral value $ITG_{1_Middle_9}$ in class 1 $_{_9}$ in when the number of integral values is 9 consists of the sum (=$ITG_{1_Middle_74}$+ $ITG_{2_Middle_74}$+$ITG_{3_Middle_74}$+,...,+

$ITG_{9_Middle_74}$) from an integral value $ITG_{1_Middle_74}$ in class $1_{_74}$ to an integral value $ITG_{9_Middle_74}$ in class $9_{_74}$ in when the number of integral values is 74, and an integral value $ITG_{1_High_9}$ in class $1_{_9}$ in when the number of integral values is 9 consists of the sum (=$ITG_{1_High_74}$+ $ITG_{2_High_74}$+ $ITG_{3_High_74}$+ ,...,+ $ITG_{9_High_74}$) from an integral value $ITG_{1_High_74}$ in class $1_{_74}$ to an integral value $ITG_{9_High_74}$ in class $9_{_74}$ in when the number of integral values is 74.

Similarly, an integral value $ITG_{8_Low_9}$ in class $8_{_9}$ in when the number of integral values is 9 consists of the sum (=$ITG_{64_Low_74}$+$ITG_{65_Low_74}$+$ITG_{66_Low_74}$+,...,+$ITG_{72_Low_74}$) from an integral value $ITG_{64_Low_74}$ in class $64_{_74}$ to an integral value $ITG_{72_Low_74}$ in class $72_{_74}$ when the number of integral values is 74, an integral value $ITG_{8_Middle_9}$ in class $8_{_9}$ in when the number of integral values is 9 consists of the sum (=$ITG_{64_Middle_74}$+ $ITG_{65_Middle_74}$+ $ITG_{66_Middle_74}$ +,...,+$ITG_{72_Middle_74}$) from an integral value $ITG_{64_Middle_74}$ in class $64_{_74}$ to an integral value $ITG_{72_Middle_74}$ in class $72_{_74}$ in when the number of integral values is 74, and an integral value $ITG_{8_High_9}$ in class $8_{_9}$ in when the number of integral values is 9 consists of the sum (=$ITG_{64_High_74}$+ $ITG_{65_High_74}$+ $ITG_{66_High_74}$+,...,+ $ITG_{72_High_74}$) from an integral value $ITG_{64_High_74}$ in class $64_{_74}$ to an integral value $ITG_{72_High_74}$ in class $72_{_74}$ in when the number of integral values is 74.

And, an integral value $ITG_{9_Low_9}$ in class $9_{_9}$ in when the number of integral values is 9 consists of the sum (=$ITG_{73_Low_74}$+$ITG_{74_Low_74}$) of an integral value $ITG_{73_Low_74}$ in class $73_{_74}$ and an integral value $ITG_{74_Low_74}$ in class $74_{_74}$ in when the number of integral values is 74, an integral value $ITG_{9_Middle_9}$ in class $9_{_9}$ in when the number of integral values is 9 consists of the sum (=$ITG_{73_Middle_74}$+$ITG_{74_Middle_74}$) of an integral value $ITG_{73_Middle_74}$ in class $73_{_74}$ and an integral value $ITG_{74_Middle_74}$ in class $74_{_74}$ in when the number of integral values is 74, and an integral value $ITG_{9_High_9}$ in class $9_{_9}$ in when the number of integral values is 9 consists of the sum (=$ITG_{73_High_74}$+$ITG_{74_High_74}$) of an integral value $ITG_{73_High_74}$ in class $73_{_74}$ and an integral value $ITG_{74High_74}$ in class $74_{_74}$ in when the number of integral values is 74.

Furthermore, when the analyte is "grapes", class $1_{_7}$ in when the number of integral values is 7 corresponds to classes $1_{_74}$ to $11_{_74}$ in when the number of integral values is 74, similarly, class $6_{_7}$ in when the number of integral values is 7 corresponds to classes $56_{_74}$ to $66_{_74}$ in when the number of integral values is 74, and class $7_{_7}$ in when the number of integral values is 7 corresponds to classes $67_{_74}$ to $74_{_74}$ in when the number of integral values is 74 (See (i) of Fig. 105).

As a result, an integral value $ITG_{1_Low_7}$ in class $1_{_7}$ in when the number of integral values is 7 consists of the sum (=$ITG_{1_Low_74}$+$ITG_{2_Low_74}$+$ITG_{3_Low_74}$+,..., + $ITG_{11_Low_74}$) from an integral value $ITG_{1_Low_74}$ in class $1_{_74}$ to an integral value $ITG_{11_Low_74}$ in class $11_{_74}$ in when the number of integral values is 74, an integral value $ITG_{1_Middle_7}$ in class $1_{_7}$ in when the number of integral values is 7 consists of the sum (=$ITG_{1_Middle_74}$+ $ITG_{2_Middle_74}$+$ITG_{3_Middle_74}$ +,...,+$ITG_{11_Middle_74}$) from an integral value $ITG_{1_Middle_74}$ in class $1_{_74}$ to an integral value $ITG_{11_Middle_74}$ in class $11_{_74}$ in when the number of integral values is 74, and an integral value $ITG_{1_High_7}$ in class $1_{_7}$ in when the number of integral values is 7 consists of the sum (=$ITG_{1_High_74}$+ $ITG_{2_High_74}$+ $ITG_{3_High_74}$ +,...,+ $ITG_{11_High74}$) from an integral value $ITG_{1_High_74}$ in class $1_{_74}$ to an integral value $ITG_{11_High_74}$ in class $11_{_74}$ in when the number of integral values is 74.

Similarly, an integral value $ITG_{6_Low_7}$ in class $6_{_7}$ in when the number of integral values is 7 consists of the sum (=$ITG_{56_Low_74}$+$ITG_{57_Low_74}$+$ITG_{58_Low_74}$+,...,+ $ITG_{66_Low_74}$) from an integral value $ITG_{56_Low_74}$ in class $56_{_74}$ to an integral value $ITG_{66_Low_74}$ in class $66_{_74}$ in when the number of integral values is 74, an integral value $ITG_{6_Middle_7}$ in class $6_{_7}$ in when the number of integral values is 7 consists of the sum (=$ITG_{56_Middle_74}$+ $ITG_{57_Middle_74}$+$ITG_{58_Middle_74}$ +,..., + $ITG_{66_Middle_74}$) from an integral value $ITG_{56_Middle_74}$ in class $56_{_74}$ to an integral value $ITG_{66_Middle_74}$ in class $66_{_74}$ in when the number of integral values is 74, and an integral value $ITG_{6_High_7}$ in class $6_{_7}$ in when the number of integral values is 7 consists of the sum (=$ITG_{56_High_74}$+ $ITG_{57_High_74}$+ $ITG_{58_High_74}$+,...,+ $ITG_{66_High_74}$) from an integral value $ITG_{56_High_74}$ in class $56_{_74}$ to an integral value $ITG_{66_High_74}$ in class $66_{_74}$ in when the number of integral values is 74.

And, an integral value $ITG_{7_Low_7}$ in class $7_{_7}$ in when the number of integral values is 7 consists of the sum (=$ITG_{67_Low_74}$+$ITG_{68_Low_74}$+$ITG_{69_Low_74}$+,..., + $ITG_{74_Low_74}$) from an integral value $ITG_{67_Low_74}$ in class $67_{_74}$ to an integral value $ITG_{74_Low_74}$ in class $74_{_74}$ in when the number of integral values is 74, an integral value $ITG_{7_Middle_7}$ in class $7_{_7}$ in when the number of integral values is 7 consists of the sum (=$ITG_{67_Middle_74}$+$ITG_{68_Middle_74}$+$ITG_{69_Middle_74}$ +,..., + $ITG_{74_Middle_74}$) from an integral value $ITG_{67_Middle_74}$ in class $67_{_74}$ to an integral value $ITG_{74_Middle_74}$ in class $74_{_74}$ in when the number of integral values is 74, and an integral value $ITG_{7_High_7}$ in class $7_{_7}$ in when the number of integral values is 7 consists of the sum (=$ITG_{67_High_74}$+ $ITG_{68_High_74}$+ $ITG_{69_High_74}$+,..., + $ITG_{74_High_74}$) from an integral value $ITG_{67_High_74}$ in class $67_{_74}$ to an integral value $ITG_{74_High_74}$ in class $74_{_74}$ in when the number of integral values is 74.

As a result, by associating each of "classes $1_{_37}$ to $37_{_37}$ in when the number of integral values is 37, classes $1_{_25}$ to $25_{_25}$ in when the number of integral values is 25, classes $1_{_19}$ to $19_{_19}$ in when the number of integral values is 19, classes $1_{_15}$ to $15_{_15}$ in when the number of integral values is 15, classes $1_{_13}$ to $13_{_13}$ in when the number of integral values is 13, classes $1_{_11}$ to $11_{_11}$ in when the number of integral values is 11, classes $1_{_9}$ to $9_{_9}$ in when the number of integral values is 9, and classes $1_{_7}$ to $7_{_7}$ in when the number of integral values is 7," with "classes $1_{_74}$ to $74_{_74}$ in when the number of integral values is 74", it is possible to match "the sum of integral values in a predetermined potential range including a potential equal to or lower than the threshold value $V_{th}$" to "the sum of the integral values in a predetermined potential section including a potential equal to or lower than the threshold value $V_{th}$ in when the number of integral values is 74" even if the number of integral values is any one of 37, 25, 19, 15, 13, 11, 9, and 7.

Therefore, the taste diagnosis result of grape in when the number of integral values is any one of 7, 9, 11, 13, 15, 19, 25, and 37 coincides with the taste diagnosis result of grape (=the taste diagnosis result shown in Fig. 35) in when the number of integral values is 74.

Therefore, the diagnostic device 2 may perform a taste diagnosis of "grapes" using the above-mentioned method based on the correspondence (see Fig. 12) between the classes and the integral values in when the number of integral values is any of 7, 9, 11, 13, 15, 19, 25, 37, and 74.

[Taste diagnosis of "Shochu" in when the number of integral values is multiple]

When the diagnostic device 2 diagnoses the taste of shochu, the control unit 212 stores the calculation data $CAL_{uni}$ and the curve $CUR_{uni}$ in the analysis data $ALY_D_{uni}$ read out from the database 22 to update the analysis data $ALY_D_{uni}$ to index data $IDX_{uni}$, and outputs the updated index data $IDX_{uni}$ to the calculation unit 216. In addition, the control unit 212 determines the number $n_{TGC1}$ of integral values used for the taste diagnosis of shochu to be any one of 18, 20, 32, 36, 56, 70, 92, and 138, and outputs the determined number $n_{TGC1}$ of integral values to the calculation unit 216.

Here, the index data $IDX_{uni}$ has a configuration of $IDX_{uni}$=[time $t_{uni}$/identification information $ID_{uni}$/name $ALY_Na_{uni}$ of analyte/type $ALY_Kd_{uni}$ of analyte/current-potential characteristic $(I\text{-}V)_{uni}$/calculation data $CAL_{uni}$/curve $CUR_{uni}$].

The calculation unit 216 receives the number $n_{TGC1}$ of integral values used in the taste diagnosis of Shochu and the index data $IDX_{uni}$ from the control unit 212, and detects the calculation data $CAL_{uni}$ from the received index data $IDX_{uni}$.

Here, it is assumed that n in the calculation data $CAL_{uni}$ received from the control unit 212 is set to n=276. It is also assumed that the calculation unit 216 holds in advance the "diagram showing the correspondence relationship of classes" shown in Figs. 103 and 104.

The calculation unit 216 refers to the "diagram showing the correspondence relationship of classes" and converts the 276 integral values $ITG_{1_Low}$ to $ITG_{276_Low}$, $ITG_{1_Middle}$ to $ITG_{276_Middle}$, and $ITG_{1_High}$ to $ITG_{276_High}$ into $n_{TGC1}$ of integral values $ITG_{1_Low}$ to $ITG_{nTGC1_Low}$, $ITG_{1_Middle}$ to $ITG_{nTGC1_Middle}$, and $ITG_{1_High}$ to $ITG_{nTGC1_High}$, respectively.

Then, the calculation unit 216 associates the classes $Cls_1$ to $Cls_{nTGC1}$ with the $n_{TGCl}$ integral values $ITG_{1_Low}$ to $ITG_{nTGC1_Low}$, $ITG_{1_Middle}$ to $ITG_{nTGC1_Middle}$, and $ITG_{1_High}$ to $ITG_{nTGC1_High}$ to output them to the taste diagnostic unit 217.

The taste diagnostic unit 217 receives the correspondence between the classes $Cls_1$ to $Cls_{nTGC1}$ and the $n_{TGC1}$ integral values $ITG_{1_Low}$ to $ITG_{nTGC1_Low}$, $ITG_{1_Middle}$ to $ITG_{nTGC1_Middle}$, and $ITG_{1_High}$ to $ITG_{nTGC1_High}$ from the calculation unit 216, and the $n_{TGC1}$ of $ITG_{1_High}$ to $ITG_{nTGC1_High}$, executes sequentially the steps S821 to S825, S827, S828, and S830 shown in Fig. 98 (including the flowchart shown in Fig. 99) to diagnose the taste of shochu based on the correspondence between the classes $Cls_1$ to $Cls_{nTGC1}$ and the $n_{TGC1}$ of integral values $ITG_{1_Low}$ to $ITG_{nTGC1_Low}$, the $n_{TGC1}$ of $ITG_{1_Middle}$ to $ITG_{nTGC1_Middle}$, and the $n_{TGC1}$ of $ITG_{1_High}$ to $ITG_{nTGC1_High}$.

In this case, "n" in steps S821 to S825 in Fig. 98 is set to "$n_{TGC1}$."

In Figs. 103 and 104, when the number of integral values is 276, the 276 classes are all composed of the same predetermined potential section, when the number of integral values is 138, the 138 classes are all composed of the same predetermined potential section, and when the number of integral values is 92, the 92 classes are all composed of the same predetermined potential section.

On the other hand, when the number of integral values is 70, classes 1 to 34 and 36 to 69 are composed of the same predetermined potential section, and classes 35 and 70 are composed of predetermined potential sections smaller than the predetermined potential sections in each of classes 1 to 34 and 36 to 69. The same is also true for the cases where the number of integral values is 56, 36, 32, 20, and 18.

Therefore, when the analyte is "shochu", the $n_{TGC1}$ pieces of integral values used for taste diagnosis consists of multiple integral values in the same predetermined potential section (=See 276 integral values, 138 integral values, and 92 integral values), or consists of multiple integral values including integral values in a first predetermined potential section and integral values in a second predetermined potential section smaller than the first predetermined potential section (=See 70 integral values, 56 integral values, 36 integral values, 32 integral values, 20 integral values, and 18 integral values).

[Taste diagnosis of "grapes" in when the number of integral values is multiple]

When the diagnostic device 2 diagnoses the taste of "grapes", the calculation unit 216 receives the index data $IDX_{uni}$ from the control unit 212 as described above. Here, it is assumed that n in the calculation data $CAL_{uni}$ contained in the index data $IDX_{uni}$ is set to n=74 and that the calculation unit 216 holds in advance the "diagram showing the correspondence relationship of classes" shown in Fig. 105.

The calculation unit 216 also receives from the control unit 212 the number $n_{TGC2}$ of integral values composed of any one of 7, 9, 11, 13, 15, 19, 25, and 37.

The calculation unit 216 refers to the "diagram showing the correspondence relationship of classes" to convert the 74 integral values $ITG_{1_Low}$ to $ITG_{74_Low}$, $ITG_{1_Middle}$ to $ITG_{74_Middle}$, and $ITG_{1_High}$ to $ITG_{74_High}$ into $n_{TGC2}$ integral values $ITG_{1_Low}$ to $ITG_{nTGC2_Low}$, $ITG_{1_Middle}$ to $ITG_{nTGC2_Middle}$, and $ITG_{1_High}$ to $ITG_{nTGC2_High}$, respectively.

Then, the calculation unit 216 associates the classes $Cls_1$ to $Cls_{nTGC2}$ with the $n_{TGC2}$ integral values $ITG_{1_Low}$ to $ITG_{nTGC2_Low}$, $ITG_{1_Middle}$ to $ITG_{nTGC2_Middle}$, and $ITG_{1_High}$ to $ITG_{nTGC2_High}$ to output them to the taste diagnostic unit 217.

The taste diagnostic unit 217 receives the correspondence relationship between the classes $Cls_1$ to $Cls_{nTGC2}$ and the $n_{TGC2}$ integral values $ITG_{1_Low}$ to $ITG_{nTGC2_Low}$, $ITG_{1_Middle}$ to $ITG_{nTGC2_Middle}$, and $ITG_{1_High}$ to $ITG_{nTGC2_High}$ from the calculation unit 216, and executes sequentially steps S826 and S830 (including the flowchart shown in Fig. 100) shown in Fig. 98 in sequence to diagnose the taste of "grapes" based on the correspondence relationship between the classes $Cls_1$ to $Cls_{nTGC2}$ and the $n_{TGC2}$ integral values $ITG_{1_Low}$ to $ITG_{nTGC2_Low}$, $ITG_{1_Middle}$ to $ITG_{nTGC2_Middle}$, and $ITG_{1_High}$ to $ITG_{nTGC2_High}$.

In this case, "n" in step S826 of Fig. 98 is set to "$n_{TGC2}$".

In Fig. 105, when the number of integral values is 74, the 74 classes consist of mutually the same predetermined potential section, and when the number of integral values is 37, the 37 classes consist of mutually the same predetermined potential section.

On the other hand, when the number of integral values is 25, classes 1 to 24 are composed of the same predetermined potential section, and class 25 is composed of a predetermined potential section smaller than the predetermined potential sections in each of classes 1 to 24. The same is true for the cases where the number of integral values is 19, 15, 13, 11, 9, and 7.

Therefore, when the analyte is "grapes," the two $n_{TGC2}$ integral values used for taste diagnosis consist of a plurality of integral values in the same predetermined potential section (see = 74 integral values and 37 integral values), or consist of a plurality of integral values including integral values in a first predetermined potential section and a plurality of integral values in a second predetermined potential section smaller than the first predetermined potential section (see = 25 integral values, 19 integral values, 15 integral values, 13 integral values, 11 integral values, 9 integral values, and 7 integral values).

Fig. 106 is a conceptual diagram of a judgment result indicating whether ${}_PC_2$ pairs of two curves $CUR_i$, $CUR_j$ are or not different.

Referring to Fig. 106, in ${}_PC_2$ pairs of the two curves CURi, CURj (i≠j), the threshold value $\sigma_{th(CURi, CURj)}$ for judging whether the two curves $CUR_i$, $CUR_j$ (i≠j; i=1 to P, j=1 to P) are or not different is, for example, 6% or 15%. Here, ${}_PC_2$ is the number of combinations of two different curves $CUR_i$, $CUR_j$ (i≠j) in when extracting the two different curves $CUR_i$, $CUR_j$ (i≠j) from the P curves $CUR_1$ to $CUR_P$.

When a judgment result is the judgment result (○) that all of the ${}_PC_2$ pairs of two curves $CUR_i$, $CUR_j$ are different, the P curves $CUR_1$ to $CUR_P$ are judged to be different from each other. In this case, the P curves $CUR_1$ to $CUR_1$ each represent a feature amount by an integral value (= the sum $ITG_{ST}$ of the integral values shown in Fig. 12) for the P analytes.

On the other hand, when at least one pair of two curves $CUR_i$, $CUR_j$ in "the ${}_PC_2$ pairs of two curves $CUR_i$, $CUR_j$" do not differ, the P curves $CUR_1$ to $CUR_1$ are not different from one another.

And, when at least one pair of two curves CURi, CURj among the ${}_pC_2$ pairs of two curves $CUR_i$, $CUR_j$ are not different, by referring to the judgment result shown in Fig. 106, it is possible to easily understand the two curves $CUR_i$, $CUR_j$ that differ and the two curves $CUR_{i'}$, $CUR_{j'}$ that do not differ.

Furthermore, when all of ${}_PC_2$ pairs of two curves $CUR_i$, $CUR_j$ are not different (x), the P curves $CUR_1$ to $CUR_1$ are judged to be not different from one another.

The judgment unit 214 creates, in step S77 of Fig. 91, the judgment result shown in Fig. 106. Then, the judgment unit 214 outputs the judgment result shown in Fig. 106 to the creation unit 215.

The creation unit 215 receives P calculation results $CAL_RLS_1$=[$ID_1$/$CAL_1$] to $CAL_RLS_P$=[$ID_P$/$CAL_P$] from the calculation unit 213 and the judgment result shown in Fig. 106 from the judgment unit 214, and then adds curves $CUR_1$ to $CUR_1$ to the P calculation results $CAL_RLS_1$=[$ID_1$/$CAL_1$] to $CAL_RLS_1$=[$ID_1$/$CAL_1$], respectively, to create P analysis results $ALY_RLS_1$=[$ID_1$/$CAL_1$/$CUR_1$] to $ALY_RLS_P$= [$ID_P$/ $CAL_P$/ $CUR_P$].

Then, the creation unit 215 outputs the P analysis results $ALY_RLS_1$=[$ID_1$/$CAL_1$/$CUR_1$] to $ALY_RLS_P$=[$ID_P$/ $CAL_P$/$CUR_P$] and the judgment results shown in Fig. 106 to the control unit 212.

In Fig. 106, a threshold value $\sigma_{th(CURi, CURj)}$ of 5% is used when it is judged whether the 20 curves k27 to k46 for Shochu of No. 1 to No. 20 are or not different from one another. The threshold value $\sigma_{th(CURi, CURj)}$ of 5% is used similary in the case where it is judged whether the 20 curves k47 to k66 are or not different from one another for Shochu of No. 1 to No. 20, in the case where it is judged whether the 20 curves k67 to k86 are or not different from one another for Shochu of No. 1 to No. 20, and in the case where when it is judged whether the 20 curves k87 to k106 are or not different from one another for Shochu of No. 1 to No. 20.

Also, in Fig. 106, a threshold value $\sigma_{th(CURi, CURj)}$ of 8% is used when it is judged whether the six curves k107 to k112 are or not different from one another, when it is judged whether the six curves k113 to k118 are or not different from one another, when it is judged whether the six curves k119 to k124 are or not different from one another, and when it is judged whether the six curves k125 to k130 are or not different from one another, for grapes.

Fig. 107 is a conceptual diagram showing the update from the analysis data $ALY_D_{uni}$ to the index data $IDX_{uni}$ in step S74 of Fig. 91.

Referring to Fig. 107, in step S72 of Fig. 91, the control unit 212 of the diagnostic device 2 creates analysis data $ALY_D_{uni}$ in which includes the time $t_{uni}$, identification information $ID_{uni}$, name $ALY_N_{auni}$ of the analyte, type $ALY_Kd_{uni}$ of analyte and current-potential characteristic $(I\text{-}V)_{uni}$ based on the measurement data MRS_uni (see (a) of Fig. 107).

Then, the control unit 212 receives the analysis result $ALY_RLS_{uni}$= [$ID_{uni}$/ $CAL_{uni}$/ $CUR_{uni}$] from the creation unit 215 and reads out the analysis data $ALY_D_{uni}$ having the same identification information as the identification information $ID_{uni}$ in the analysis result $ALY_RLS_{uni}$ from the database 22 after outputting the analysis data $ALY_D_{uni}$ to the calculation unit 213.

Then, the control unit 212 detects the calculation data $CAL_{uni}$ and the curve $CUR_{uni}$ from the analysis result $ALY_RLS_{uni}$=[$ID_{uni}$/$CAL_{uni}$/$CUR_{uni}$], adds the detected calculation data $CAL_{uni}$ and the curve $CUR_{uni}$ to the analysis data $ALY_D_{uni}$ to create the index data $IDX_{uni}$, and updates the analysis data $ALY_D_{uni}$ to the index data $IDX_{uni}$ (see Fig. 107(b)). In this case, the calculation data $CAL_{uni}$ has the structure shown in Fig. 12.

Then, the control unit 212 stores the index data $IDX_{uni}$ in the database 22 in place of the analysis data $ALY_D_{uni}$.

Fig. 108 is a conceptual diagram showing the updating of from P pieces of analysis data $ALY_D_1$ to $ALY_D_P$ to P pieces of index data $IDX_1$ to $IDX_P$ in step S78 of Fig. 91.

Referring to Fig. 108, in step S75 of Fig. 91, the control unit 212 of the diagnostic device 2 creates analysis data $ALY_D_1$ including time $t_1$, identification information $ID_1$, name $ALY_Na_1$ of the analyte, kind $ALY_Kd_1$ of the analyte, and current-potential characteristic $(I\text{-}V)_1$, analysis data $ALY_D_2$ including time $t_2$, identification information $ID_2$, name $ALY_Na_2$ of the analyte, kind $ALY_Kd_2$ of the analyte, and current-potential characteristic $(I\text{-}V)_2$, ..., analysis data $ALY_D_1$ including time $t_P$, identification information $ID_P$, name $ALY_Na_P$ of the analyte, kind $ALY_Kd_P$ of the analyte, and current-potential characteristic $(I\text{-}V)_P$, based on P pieces of measurement data MRS_1 to MRS_P (see (a) of Fig. 108). That is, the control unit 212 creates P pieces of analysis data $ALY_D_1$ to $ALY_D_1$ based on the P pieces of measurement data MRS_1 to MRS_P.

Then, the control unit 212 reads out, from the database 22, P pieces of analysis data $ALY_D_1$ to $ALY_D_P$ which have the same identification information as the P identification information $ID_1$ to $ID_P$, respectively, in the P analysis results $ALY_RLS_1$ to $ALY_RLS_P$ when receiving P analysis results $ALY_RLS_1$= [$ID_1$/ $CAL_1$/ $CUR_1$] to $ALY_RLS_P$=[$ID_P$/$CAL_P$/ $CUR_P$] from the creation unit 215 after the control unit 212 outputs P pieces of analysis data $ALY_D_1$ to $ALY_D_P$ to the calculation unit 213.

Then, the control unit 212 detects the calculation data $CAL_p$ and the curve $CUR_p$ from the analysis result $ALY_RLS_p$=[$ID_p$/$CAL_p$/$CUR_p$] (p is any of 1 to P), adds the detected calculation data $CAL_p$ and curve $CUR_p$ to the analysis data $ALY_D_p$ to create index data $IDX_p$, and performing, for all P pieces of analysis data $ALY_D_1$ to $ALY_D_P$, updating the analysis data $ALY_D_p$ to the index data $IDX_p$, thereby updating the P pieces of analysis data $ALY_D_1$ to $ALY_D_P$ to P pieces of index data $IDX_1$ to $IDX_P$, respectively (see (b) of Fig. 108).

Then, the control unit 212 associates the judgment result (the judgment result shown in Fig. 106) with the P index data $IDX_1$ to $IDX_P$ (see (b) of Fig. 108).

In response, the control unit 22 stores the P index data $IDX_1$ to $IDX_P$ in the database 22 instead of the P analysis data $ALY_D_1$ to $ALY_D_P$, and stores the judgment result (the judgment result shown in Fig. 106) in the database 22 in association with the P index data $IDX_1$ to $IDX_P$.

Fig. 109 is a conceptual diagram showing the results of a taste diagnosis. Fig. 109 (a) shows the results of a taste diagnosis for one analyte, and Fig. 109 (b) shows the results of a taste diagnosis for P analytes.

Referring to (a) of Fig. 109, the taste diagnosis result $JDR_{uni}$ is configured to correspond to the identification information $ID_{uni}$ of the analyte, the name $ALY_Na_{uni}$ of the analyte, the type $ALY_Kd_{uni}$ of the analyte, and the diagnosis result shown in Fig. 29 or the diagnosis result shown in Fig. 35.

Referring to (b) of Fig. 109, the taste diagnosis result $JDR_P$ includes [the identification information $ID_1$/ the name $ALY_Na_1$/ the type $ALY_Kd_1$/ the diagnosis result shown in Fig. 29 or the diagnosis result shown in Fig. 35] corresponding to the identification information $ID_1$ of the analyte, the name $ALY_Na_1$ of the analyte, the type $ALY_Kd_1$ of the analyte, and the diagnosis result shown in Fig. 29 or the diagnosis result shown in Fig. 35., [the identification information $ID_2$/ the name $ALY_Na_2$/ the type $ALY_Kd_2$/ the diagnosis result shown in Fig. 29 or the diagnosis result shown in Fig. 35] corresponding to the identification information $ID_2$ of the analyte, the name $ALY_Na_2$ of the analyte, the type $ALY_Kd_2$ of the analyte, and the diagnosis result shown in Fig. 29 or the diagnosis result shown in Fig. 35.,..., [the identification information $ID_P$/ the name $ALY_Na_P$/ the type $ALY_Kd_P$/ the diagnosis result shown in Fig. 29 or the diagnosis result shown in Fig. 35] corresponding to the identification information $ID_1$ of the analyte, the name $ALY_Na_P$ of the analyte, the type $ALY_Kd_P$ of the analyte, and the diagnosis result shown in Fig. 29 or the diagnosis result shown in Fig. 35.

The taste diagnostic unit 217 creates a taste diagnosis result $JDR_{uni}$ and a taste diagnosis result $JDR_P$, stores the created taste diagnosis result $JDR_{uni}$ and taste diagnosis result $JDR_P$ in the database 22 and outputs them to the display unit 218.

When the display unit 218 receives the taste diagnosis result $JDR_{uni}$ from the taste diagnostic unit 217, the display unit 218 displays the taste diagnosis result $JDR_{uni}$, and when the display unit 218 receives the taste diagnosis result $JDR_P$ from the taste diagnostic unit 217, the display unit 218 displays the taste diagnosis result $JDR_P$.

In an embodiment of the present invention, the operation of the diagnostic device 2 may be performed by software. In this case, the diagnostic device 2 includes a CPU (Central Processing Unit), a ROM (Read Only Memory), and a RAM (Random Access Memory).

The ROM stores a program Prog_A consisting of steps S6 to S8 (including the flowcharts shown in Figs. 91 to 99, and Fig. 101) shown in Fig. 90, or a program Prog_B consisting of steps S6 to S8 (including the flowcharts shown in Figs. 91 to 98, Fig. 100, and Fig. 102) shown in Fig. 90.

The CPU reads out the program Prog_A or the program Prog_B from the ROM, and executes the program Prog_A which is read out or the program Prog_B which is read out to diagnose the taste of the analyte.

In this case, the RAM temporarily stores the standard deviations $\sigma_{DF_CURi,\ CURj}$ ($i \neq j$, i=1 to P, j=1 to P) of differences, the standard deviations $\sigma_{DF_DFFi,\ DFFj}$ ($i \neq j$, i=1 to P, j=1 to P) of differences, the threshold value $\sigma_{th}$, the sums L(+)_sum, M(+)_sum, H(+)_sum, L(all)_sum, M(all)_sum, H(all)_sum, H(-)_sum, L(-)_sum_th, M(-)_sum_th, H(-)_sum_th of integral values, Body index (+), Body index (all), Body index (-)_th, and etc.

Therefore, the program Prog_A or the program Prog_B is a program for causing a computer (CPU) to execute a diagnosis of the taste of the analyte.

[Second Embodiment]

Fig. 110 is a schematic diagram of a diagnostic system according to embodiment 2. Referring to Fig. 110, a diagnostic system 10A according to embodiment 2 includes a sensor device 1, a diagnostic device 2A, and a terminal device 3.

In the embodiment 2, the sensor device 1 and the terminal device 3 are arranged in, for example, restaurants such as Japanese restaurants, Chinese restaurants, and Western restaurants, sake breweries that brew shochu, and liquor stores that sell shochu, etc. Furthermore, the diagnostic device 2A is arranged in the server 30.

The terminal device 3 receives measurement data MRS_uni (consisting of the measurement data MRS shown in Fig. 6) or P pieces of measurement data MRS_1 to MRS_P (each of the measurement data MRS_1 to MRS_P is composed of the measurement data MRS shown in Fig. 6) from the sensor device 1 via wireless communication or wired communication.

Then, the terminal device 3 transmits the measurement data MRS_uni (consisting of the measurement data MRS shown in Fig. 6) to the diagnostic device 2A via the network 20.

In addition, the terminal device 3 receives the diagnosis result of the taste of the analyte (diagnosis result $JDR_{uni}$ or diagnosis result $JDR_P$ shown in Fig. 109) from the diagnostic device 2A via the network 20, and displays the received diagnosis result of the taste of the analyte (diagnosis result $JDR_{uni}$ or diagnosis result $JDR_P$ shown in Fig. 109).

The diagnostic device 2A receives the measurement data MRS_uni (comprised of the measurement data MRS shown in Fig. 6) from the terminal device 3 via the network 20.

Then, the diagnostic device 2A creates the above-mentioned analysis data $ALY_D_{uni}$ based on the measurement data MRS_uni, creates calculation data $CAL_{uni}$ based on the analysis data $ALY_D_{uni}$, and creates a curve $CUR_{uni}$ based on the calculation data $CAL_{uni}$.

Then, the diagnostic device 2A updates the analysis data $ALY_D_{uni}$ to the index data $IDX_{uni}$, diagnoses the taste of the analyte based on the updated index data $IDX_{uni}$ to create a diagnosis result $JDR_{uni}$ by the method described in the first embodiment. Then, the diagnostic device 2A transmits the diagnosis result $JDR_{uni}$ to the terminal device 3 via the network 20.

Moreover, the diagnostic device 2A receives P pieces of measurement data MRS_1 to MRS_P (each of the measurement data MRS_1 to MRS_P is made up of the measurement data MRS shown in Fig. 6) from the terminal device 3 via the network 20.

Then, the diagnostic device 2A creates the above-mentioned P pieces of analysis data $ALY_D_1$ to $ALY_D_P$ based on the P pieces of measurement data MRS_1 to MRS_P, creates P pieces of calculation data $CAL_1$ to $CAL_1$ based on the P pieces of analysis data $ALY_D_1$ to $ALY_D_p$, respectively, and creates P pieces of curves $CUR_1$ to $CUR_1$ based on the P pieces of calculation data $CAL_1$ to $CAL_P$, respectively. Also, the diagnostic device 2A creates a judgment result (the judgment result shown in Fig. 106) indicating whether the P curves $CUR_1$ to $CUR_P$ are different or not. , respectively,

Then, the diagnostic device 2A updates the P pieces of analysis data $ALY_D_1$ to $ALY_D_P$ to P pieces of index data $IDX_1$ to $IDX_P$, respectively, using the method described in embodiment 1, and diagnoses the taste of the P analytes based on the updated P pieces of index data $IDX_1$ to $IDX_P$ to create a diagnosis result $JDR_P$.

Then, diagnostic device 2A transmits the diagnosis result $JDR_P$ to the terminal device 3 via network 20.

Fig. 111 is a schematic diagram of the terminal device 3 shown in Fig. 110. Referring to Fig. 111, the terminal device 3 includes an antenna 31, a receiving unit 32, a control unit 33, a transmitting unit 34, a display unit 35, a reception unit 36, and a database 37.

The receiving unit 32 receives the measurement data MRS_uni or the P pieces of measurement data MRS_1 to MRS_P from the sensor device 1 via the antenna 31 by wireless communication, or receives the measurement data MRS_uni or the P pieces of measurement data MRS_1 to MRS_P from the sensor device 1 by wired communication. The receiving unit 32 is connected to the sensor device 1 through a wired cable in the case of receiving the measurement data MRS_uni or the P pieces of measurement data MRS_1 to MRS_P from the sensor device 1 through wired communication.

Then, the receiving unit 32 outputs the measurement data MRS_uni or the P pieces of measurement data MRS_1 to MRS_P to the control unit 33.

In addition, the receiving unit 32 receives the taste diagnosis result $JDR_{uni}$ (or the taste diagnosis result $JDR_P$) from the diagnostic device 2A via the network 20 and the antenna 31, and outputs the received taste diagnosis result $JDR_{uni}$ (or the taste diagnosis result $JDR_P$) to the control unit 33.

The control unit 33 has a built-in timer. Then, when the control unit 33 receives the measurement data MRS_uni from the receiving unit 32, the control unit 33 refers to the timer and detects the time $t_{uni}$ in when the measurement data MRS_uni was received from the receiving unit 32. Then, the control unit 33 store the measurement data MRS_uni in the database 37 by associating the measurement data MRS_uni to the time $t_{uni}$.

Then, the control unit 33 outputs the measurement data MRS_uni to the transmitting unit 34.

On the other hand, the control unit 33 refers to the timer to detect the times $t_1$ to $t_1$ at which the P pieces of measurement data MRS_1 to MRS_P were received, respectively, when the control unit 33 has received the P pieces of measurement data MRS_1 to MRS_P from the receiving unit 32. Then, the control unit 33 associates the P pieces of measurement data MRS_1 to MRS_P with the times $t_1$ to $t_1$, respectively, to store the P pieces of measurement data MRS_1 to MRS_P in the database 37.

Then, the control unit 33 outputs the P pieces of measurement data MRS_1 to MRS_P to the transmitting unit 34.

Furthermore, when the control unit 33 received the taste diagnosis result $JDR_{uni}$ from the receiving unit 32, the control unit 33 stores the received taste diagnosis result $JDR_{uni}$ in association with [time $t_{uni}$/measurement data MRS_uni] in the database 37.

On the other hand, when the control unit 33 received the taste diagnosis result $JDR_P$ from the receiving unit 32, the control unit 33 stores the received taste diagnosis result $JDR_P$ in association with [time $t_1$/measurement data MRS_1] to [time $t_P$/measurement data MRS_P] in the database 37.

Furthermore, the control unit 33 detects the diagnosis result $JDR_{uni}$ associated with the name $ALY_Na_{uni}$ of the analyte from the database 37 based on the name $ALY_Na_{uni}$ of the analyte, and outputs the name $ALY_Na_{uni}$ of the analyte and the diagnosis result $JDR_{uni}$ to the display unit 35 when the control unit 33 received a request $RQT_{uni}$ from the reception unit 36 to display the name $ALY_Na_{uni}$ of the analyte and the diagnosis result $JDR_{uni}$ associated with the name $ALY_Na_{uni}$.

Furthermore, the control unit 33 detects the q (q is an integer satisfying $1 \leqq q \leqq P$) names $ALY_Na_1$ to $ALY_Na_q$ of the q analytes and the q diagnosis results $JDR_1$ to $JDR_q$ associated with the q names $ALY_Na_1$ to $ALY_Na_q$ of the q analytes, respectively, from the database 37 based on the q names $ALY_Na_1$ to $ALY_Na_q$ and outputs the q names $ALY_Na_1$ to $ALY_Na_q$ of the q analytes and the q diagnosis results $JDR_1$ to $JDR_q$ to the display unit 35 when the control unit 33 received, from the reception unit 36, a request $RQT_q$ to display q names $ALY_Na_1$ to $ALY_Na_q$ out of the P names $ALY_Na_1$ to $ALY_Na_P$ of the P analytes and the q diagnosis results $JDR_1$ to $JDR_q$ associated with the q names $ALY_Na_1$ to $ALY_Na_q$, respectively.

In this case, in the request $RQT_q$, q types $ALY_Kd_1$ to $ALY_Kd_q$ of the q analytes may be used instead of the q names $ALY_Na_1$ to $ALY_Na_q$ of the q analytes.

When the transmitting unit 34 receives the measurement data MRS_uni from the control unit 33, the transmitting unit 34 transmits the measurement data MRS_uni to the diagnostic device 2A via the antenna 31 and the network 20.

On the other hand, when the transmitting unit 34 received the P pieces of measurement data MRS_1 to MRS_P from the control unit 33, the transmitting unit 34 transmits the P pieces of measurement data MRS_1 to MRS_P via the antenna 31 and the network 20 to the diagnostic device 2A.

When the display unit 35 received the taste diagnosis result $JDR_{uni}$ from the control unit 33, the display unit 35 displays the received taste diagnosis result $JDR_{uni}$.

Furthermore, when the display unit 35 received taste diagnosis results (=q diagnosis results $JDR_1$ to $JDR_q$) from the control unit 33, the display unit 35 displays the received taste diagnosis results (=q diagnosis results $JDR_1$ to $JDR_q$).

The reception unit 36 receives a request $RQT_{uni}$ to display the name $ALY_Na_{uni}$ of the analyte and the diagnosis result $JDR_{uni}$ associated with the name $ALY_Na_{uni}$, and outputs the received request $RQT_{uni}$ to the control unit 33.

In addition, the reception unit 36 receives a request $RQT_q$ to display q (q is an integer satisfying $1 \leqq q \leqq P$) names $ALY_Na_1$ to $ALY_Na_q$ out of the P names $ALY_Na_1$ to $ALY_Na_P$ of the P analytes, and q diagnosis results $JDR_1$ to $JDR_q$ respectively associated with the q names $ALY_Na_1$ to $ALY_Na_q$, and outputs the received request $RQT_q$ to the control unit 33.

Fig. 112 is a schematic diagram of the diagnostic device 2A shown in Fig. 110. Referring to Fig. 112, the diagnostic device 2A is the same as the diagnostic device 2 shown in Fig. 7 except that the analysis/diagnostic unit 21 of the diagnostic device 2 shown in FIG. 7 is replaced with an analysis/diagnostic unit 21A.

The analysis/ diagnostic unit 21 A receives the measurement data MRS from the terminal device 3 via the network 20.

Then, the analysis/diagnostic unit 21A diagnoses the taste of the analyte (shochu or grapes) using the method described above based on the measurement data MRS, stores the diagnosed taste diagnosis result JDR in the database 22 in association with the analyte (shochu or grapes), and transmits the taste diagnosis result JDR of the analyte (shochu or grapes) to the terminal device 3 via the network 20.

Fig. 113 is a schematic diagram of the analysis/diagnostic unit 21A shown in Fig. 112. Referring to Fig. 113, the analysis/diagnostic unit 21A changes the receiving unit 211 of the analysis/diagnostic unit 21 shown in Fig. 8 to a receiving unit 211A, changes the display unit 218 and the reception unit 219 of the analysis/diagnostic unit 21 shown in Fig. 8 to a transmitting unit 220, and changes the control unit 212 of the analysis/diagnostic unit 21 shown in Fig. 8 to a control unit 212A, otherwise the analysis/diagnostic unit 21A is the same as the analysis/diagnostic unit 21 shown in Fig. 8.

The receiving unit 211A receives the measurement data MRS_uni or P pieces of measurement data MRS_1 to MRS_P from the terminal device 3 via the network 20, and outputs the received measurement data MRS_uni or P pieces of measurement data MRS_1 to MRS_P to the control unit 212A.

The control unit 212A receives the measurement data MRS_uni or P pieces of measurement data MRS_1 to MRS_P from the receiving unit 211A. Then, the control unit 212A stores the measurement data MRS _uni or the P pieces of measurement data MRS_1 to MRS_P in the database 22.

In addition, when the control unit 212A received the taste diagnosis result $JDR_{uni}$ of the analyte from the taste diagnostic unit 217, the control unit 212A stores the received taste diagnosis result $JDR_{uni}$ of the analyte in the database 22 and outputs the taste diagnosis result $JDR_{uni}$ of the analyte to the transmitting unit 220.

Furthermore, when the control unit 212A received a diagnosis result $JDR_P$ of the taste of the analyte from the taste diagnostic unit 217, the control unit 212A stores the received diagnosis result $JDR_P$ of the taste of the analyte in the database 22 and outputs the diagnosis result $JDR_P$ of the taste of the analyte to the transmitting unit 220.

Otherwise, the control unit 212A performs the same functions as the control unit 212 described above.

When the transmitting unit 220 received the diagnosis result $JDR_{uni}$ of the taste of the analyte from the control unit 212A, the transmitting unit 220 transmits the diagnosis result $JDR_{uni}$ of the taste of the analyte to the terminal device 3 via the network 20.

Furthermore, when the transmitting unit 220 receives the diagnosis result $JDR_P$ of the taste of the analyte from the control unit 212A, the transmitting unit 220 transmits the diagnosis result $JDR_P$ of the taste of the analyte to the terminal device 3 via the network 20.

Figs. 114 and 115 are first and second flowcharts, respectively, for explaining the operation of the diagnostic system 10A shown in Fig. 110.

The flowcharts shown in Figs. 114 and 115 are the same as the flowchart shown in Fig. 90, except that step S5 of the flowchart shown in Fig. 90 is replaced with step S5A, step S6 of the flowchart shown in Fig. 90 is replaced with step S6A, and steps S9 to S14 are added.

Referring to Fig. 114, when the operation of diagnostic system 10A is started, steps S1 to S4 described above are sequentially executed.

Then, when it is judged in step S4 that the measurement has ended, the sensor device 1 transmits the m pieces of measurement data MRS_1 to MRS_m to the terminal device 3 by wireless communication or wired communication (step S5A).

Referring to Fig. 115, after the step S5A, the terminal device 3 receives m pieces of measurement data MRS_1 to MRS_m from the sensor device 1 by wireless communication or wired communication (step S9).

Then, the terminal device 3 stores the m pieces of measurement data MRS_1 to MRS_m in the database 37 (step S10), and transmits the m pieces of measurement data MRS_1 to MRS_m to the diagnostic device 2A via the network 20 (step S11).

The diagnostic device 2A receives the m pieces of measurement data MRS_1 to MRS_m from the terminal device 3 via the network 20 (step S6A).

After the step S6A, the above-mentioned steps S7 and S8 are sequentially executed. Then, after the step S8, the diagnostic device 2A transmits the taste diagnosis results JDR of the m analytes to the terminal device 3 via the network 20 (step S12).

The terminal device 3 receives the taste diagnosis results JDR of the m analytes from the diagnostic device 2A via the network 20 (step S13).

Then, the terminal device 3 stores the taste diagnosis results JDR of the m analytes in the database 37 and displays the taste diagnosis results JDR (step S14). This completes the operation of the diagnostic system 10A.

In the embodiment of the present invention, the operation of the diagnostic device 2A may be executed by software. In this case, the diagnostic device 2A includes a CPU, a ROM and a RAM.

The ROM stores a program Prog_C consisting of steps S6A, S7, S8, and S12 (including the flowcharts shown in Fig. 91 to Fig. 99, and Fig. 101) shown in Fig. 115, or a program Prog_D consisting of steps S6A, S7, S8, and S12 (including the flowcharts shown in Fig. 91 to Fig. 98, Fig. 100, and Fig. 102) shown in Fig. 115.

The CPU reads out the program Prog_C or the program Prog_D from the ROM, and executes the read out program Prog_C or the read out program Prog_D to diagnose the taste of the analyte.

In this case, the RAM temporarily stores the standard deviations $\sigma_{DF_CURi,\ CURj}$ (i≠j, i=1 to P, j=1 to P) of differences, the standard deviations $\sigma_{DF_DFFi,\ DFFj}$ (i≠j, i=1 to P, j=1 to P) of differences, the threshold value $\sigma_{th}$, the sums of integral values L(+)_sum, M(+)_sum, H(+)_sum, L(all)_sum, M(all)_sum, H(all)_sum, H(-)_sum, L(-)_sum_th, M(-)_sum_th, H(-)_sum_th, Body index (+), Body index (all), and Body index (-)_th, etc.

Therefore, the program Prog_C or the program Prog_D is a program for causing a computer (CPU) to execute a diagnosis of the taste of analyte.

In the embodiment of the present invention, the operation of the terminal device 3 may be executed by software. In this case, the terminal device 3 includes a CPU, a ROM, and a RAM.

The ROM stores a program Prog_E consisting of steps S9 to S11, S13, and S14 shown in Fig. 115.

The CPU reads out the program Prog_E from the ROM, executes the read out program Prog_E to receive m pieces of measurement data MRS_1 to MRS_m from the sensor device 1, transmits the received m pieces of measurement data MRS_1 to MRS_m to the diagnostic device 2A, and also receives a taste diagnosis result JDR of the analyte from the diagnostic device 2A to store the taste diagnosis result JDR in the database 37, and displays the taste diagnosis result JDR.

In this case, the RAM temporarily stores the m pieces of measurement data MRS_1 to MRS_m and the taste diagnosis result JDR.

Fig. 116 is a diagram for explaining the locations of the sensor device 1, the terminal device 3, and the diagnostic device 2A.

Referring to Fig. 116, the sensor device 1, the terminal device 3 and the diagnostic device 2A are placed in country A.

In addition, the sensor device 1 and the terminal device 3 are placed in country A, and the diagnostic device 2A is placed in country B which is different from country A.

When the sensor device 1, terminal device 3 and diagnostic device 2A are located in country A, the diagnostic device 2A may receive measurement data MRS_uni or P pieces of measurement data MRS_1 to MRS_P from a terminal device 5 (terminal device having the same configuration as the terminal device 3) located in country C, which is different from country A, via the network 20, diagnose the taste of the analyte based on the received measurement data MRS_uni or P pieces of measurement data MRS_1 to MRS_P, and may transmit a diagnosis result JDR of the taste of the analyte to the terminal device 5 (terminal device having the same configuration as the terminal device 3) via the network 20.

In addition, when the sensor device 1 and the terminal device 3 are located in country A, and the diagnostic device 2A is located in country B which is different from country A, the diagnostic device 2A may receive measurement data MRS_uni or P pieces of measurement data MRS_1 to MRS_P from a terminal device 6 (a terminal device having the same configuration as the terminal device 3) located in country B, diagnose the taste of the analyte based on the received measurement data MRS_uni or P pieces of measurement data MRS_1 to MRS_P, and transmit a taste diagnosis result JDR of the analyte to the terminal device 6 (a terminal device having the same configuration as the terminal device 3) via the network 20.

Furthermore, when the sensor device 1 and the terminal device 3 are located in country A, and the diagnostic device 2A is located in country B which is different from country A, the diagnostic device 2A may receive measurement data MRS_uni or P pieces of measurement data MRS_1 to MRS_P from a terminal device 7 (a terminal device having the same configuration as the terminal device 3) located in country D which is different from countries A and B, diagnose the taste of the analyte based on the received measurement data MRS_uni or P pieces of measurement data MRS_1 to MRS_P, and transmit a diagnosis result JDR of the taste of the analyte to the terminal device 7 (a terminal device having the same configuration as the terminal device 3) via the network 20.

The rest of the description of the second embodiment is the same as that of the first embodiment.

[Embodiment 3]

Fig. 117 is a schematic diagram of a diagnostic system according to embodiment 3. Referring to Fig. 117, a diagnostic system 10B according to embodiment 3 includes a sensor device 1, a diagnostic device 2B, an analysis device 2C, and a terminal device 3A.

In the third embodiment, the sensor device 1 and the terminal device 3A are arranged in, for example, restaurants such as Japanese restaurants, Chinese restaurants, and Western restaurants, sake breweries that brew shochu, and liquor stores that sell shochu, etc. Furthermore, the diagnostic device 2B is arranged in a server 30, and the analysis device 2C is arranged in a server 40.

The terminal device 3A receives measurement data MRS_uni (consisting of the measurement data MRS shown in Fig. 6) or P pieces of measurement data MRS_1 to MRS_P (each of the measurement data MRS_1 to MRS_P is composed of the measurement data MRS shown in Fig. 6) from the sensor device 1 via wireless communication or wired communication.

Then, the terminal device 3A transmits the measurement data MRS_uni (consisting of the measurement data MRS shown in Fig. 6) or P pieces of measurement data MRS_1 to MRS_P (each of the measurement data MRS_1 to MRS_P is composed of the measurement data MRS shown in Fig. 6) to the analysis device 2C via the network 20.

Thereafter, the terminal device 3A receives the index data $IDX_{uni}$ or the P pieces of index data $IDX_1$ to $IDX_P$ from the analysis device 2C via the network 20.

Then, the terminal device 3A stores the index data $IDX_{uni}$ or the P pieces of index data $IDX_1$ to $IDX_P$ in the database 37, and transmits the index data $IDX_{uni}$ or the P pieces of index data $IDX_1$ to $IDX_P$ to the diagnostic device 2B via the network 20.

Then, the terminal device 3A receives the taste diagnosis result (diagnosis result $JDR_{uni}$ or diagnosis result $JDR_P$ shown in Fig. 109) of the analyte from the diagnostic device 2B via the network 20, stores the received taste diagnosis result (diagnosis result $JDR_{uni}$ or diagnosis result $JDR_P$ shown in Fig. 109) of the analyte in the database 37, and displays the taste diagnosis result (diagnosis result $JDR_{uni}$ or diagnosis result $JDR_P$ shown in Fig. 109) of the analyte.

The analysis device 2C receives measurement data MRS_uni (consisting of the measurement data MRS shown in Fig. 6) or P pieces of measurement data MRS_1 to MRS_P (each of the measurement data MRS_1 to MRS_P is composed of the measurement data MRS shown in Fig. 6) from the terminal device 3A via the network 20.

Then, the analysis device 2C creates analysis data $ALY_D_{uni}$ based on the measurement data MRS_uni (consisting of the measurement data MRS shown in Fig. 6) using the same method as the diagnostic device 2 in embodiment 1, and updates the created analysis data $ALY_D_{uni}$ to index data $IDX_{uni}$.

Then, the analysis device 2C transmits the index data $IDX_{uni}$ to the terminal device 3A via the network 20.

In addition, the analysis device 2C creates P pieces of analysis data $ALY_D_1$ to $ALY_D_P$, respectively, based on P pieces of measurement data MRS_1 to MRS_P (each of the measurement data MRS_1 to MRS_P is composed of the measurement data MRS shown in Fig. 6) using the same method as the diagnostic device 2 in embodiment 1, and updates the created P pieces of analysis data $ALY_D_1$ to $ALY_D_P$ to P pieces of index data $IDX_1$ to $IDX_P$, respectively.

Then, the analysis device 2C transmits the P pieces of index data $IDX_1$ to $IDX_P$ to the terminal device 3A via the network 20.

The diagnostic device 2B receives the index data $IDX_{uni}$ or the P pieces of index data $IDX_1$ to $IDX_P$ from the terminal device 3A via the network 20.

Then, the diagnostic device 2B diagnoses the taste of the analyte by the method explained in the first embodiment based on the index data $IDX_{uni}$, and creates a diagnosis result $JDR_{uni}$ of the taste of the analyte.

Furthermore, the diagnostic device 2B diagnoses the taste of the P analytes by the method explained in the first embodiment based on the P index data $IDX_1$ to $IDX_P$, and creates a diagnosis result $JDR_P$ of the taste of the P analytes.

Then, the diagnostic device 2B transmits the diagnosis result $JDR_{uni}$ of the taste of the analyte or the diagnosis result $JDR_P$ of the taste of the P analytes to the terminal device 3A via the network 20.

Fig. 118 is a schematic diagram of the terminal device 3A shown in Fig. 117. Referring to Fig. 118, the terminal device 3A is the same as the terminal device 3, except that the receiving unit 32, the control unit 33, and the transmitting unit 34 of the terminal device 3 shown in Fig. 111 are replaced with a receiving unit 32A, a control unit 33A, and a transmitting unit 34A, respectively.

The receiving unit 32A receives the index data $IDX_{uni}$ or the P pieces of index data $IDX_1$ to $IDX_P$ from the analysis device 2C via the network 20 and the antenna 31, and outputs the received index data $IDX_{uni}$ or the P pieces of index data $IDX_1$ to $IDX_P$ to the control unit 33A.

Otherwise, the receiving unit 32A performs the same functions as the receiving unit 32 described above.

When the control unit 33A receives the index data $IDX_{uni}$ or the P pieces of index data $IDX_1$ to $IDX_P$ from the receiving unit 32A, the control unit 33A stores the received index data $IDX_{uni}$ or the P pieces of index data $IDX_1$ to $IDX_P$ in the database 37 and outputs the index data $IDX_{uni}$ or the P pieces of index data $IDX_1$ to $IDX_P$ to the transmitting unit 34A.

Otherwise, the control unit 33A performs the same functions as the control unit 33 described above.

When the transmitting unit 34A receives the index data $IDX_{uni}$ or the P pieces of index data $IDX_1$ to $IDX_P$ from the control unit 33A, the transmitting unit 34A transmits the index data $IDX_{uni}$ or the P pieces of index data $IDX_1$ to $IDX_P$ to the diagnostic device 2B via the antenna 31 and the network 20.

Otherwise, the transmitting unit 34A performs the same functions as the transmitting unit 34 described above.

Fig. 119 is a schematic diagram of the analysis device 2C shown in Fig. 117. Referring to Fig. 119, in the analysis device 2C, the calculation unit 216 and the taste diagnostic unit 217 of the analysis/diagnostic unit 21A shown in Fig. 113 are deleted, the control unit 212A is changed to a control unit 212C, the transmitting unit 220 is changed to a transmitting unit 221, and a database 222 is added.

Of the operations of the control unit 212 described above, the control unit 212C executes the following: creation of analysis data $ALY_D_{uni}$, storage of the analysis data $ALY_D_{uni}$ in database 222, creation of P pieces of analysis data $ALY_D_1$ to $ALY_D_P$, storage of the P pieces of analysis data $ALY_D_1$ to $ALY_D_P$ in database 222, output of the analysis data $ALY_D_{uni}$ to the calculation unit 213, output of the P pieces of analysis data $ALY_D_1$ to $ALY_D_P$ to the calculation unit 213, updating of the analysis data $ALY_D_{uni}$ to index data $IDX_{uni}$, updating of the P pieces of analysis data $ALY_D_1$ to $ALY_D_P$ to P pieces of index data $IDX_1$ to $IDX_P$, respectively, storage of index data $IDX_{uni}$ in database 222, and storage of the P pieces of index data $IDX_1$ to $IDX_P$ in database 222.

In addition, the control unit 212C outputs the index data $IDX_{uni}$ or the P index data $IDX_1$ to $IDX_P$ to the transmitting unit 221.

When the transmitting unit 221 receives the index data $IDX_{uni}$ from the control unit 212C, the transmitting unit 221 transmits the index data $IDX_{uni}$ via the network 20 to the terminal device 3A.

Furthermore, when the transmitting unit 221 received the P pieces of index data $IDX_1$ to $IDX_P$ from the control unit 212C, the transmitting unit 221 transmits the P pieces of index data $IDX_1$ to $IDX_P$ via the network 20 to the terminal device 3A.

Fig. 120 is a schematic diagram of diagnostic device 2B shown in Fig. 117. Referring to Fig. 120, diagnostic device 2B is obtained by deleting calculation unit 213, judgment unit 214, and creation unit 215 of analysis/diagnostic unit 21A shown in Fig. 113, changing receiving unit 211A to receiving unit 211B, changing control unit 212A to control unit 212B, changing transmitting unit 220 to transmitting unit 220A, and adding database 223.

The receiving unit 211B receives the index data $IXD_{uni}$ from the terminal device 3A via the network 20, and outputs the received index data $IXD_{uni}$ to the control unit 212B.

Furthermore, the receiving unit 211B receives P pieces of index data $IXD_1$ to $IXD_P$ from the terminal device 3A via the network 20, and outputs the received P pieces of index data $IXD_1$ to $IXD_1$ to the control unit 212B.

When the control unit 212B received the index data $IXD_{uni}$ from the receiving unit 211B, the control unit 212B stores the index data $IXD_{uni}$ in the database 223 and outputs the index data $IXD_{uni}$ to the calculation unit 216.

Furthermore, when the control unit 212B received the P pieces of index data $IXD_1$ to $IXD_P$ from the receiving unit 211B, the control unit 212B stores the P pieces of index data $IXD_1$ to $IXD_P$ in the database 223 and outputs the P pieces of index data $IXD_1$ to $IXD_P$ to the calculation unit 216.

Furthermore, when the control unit 212B received the taste diagnosis result $JDR_{uni}$ of the analyte from the taste diagnostic unit 217, the control unit 212B stores the received taste diagnosis result $JDR_{uni}$ in the database 223 and outputs the taste diagnosis result $JDR_{uni}$ to the transmitting unit 220A.

Furthermore, when the control unit 212B received the taste diagnosis result $JDR_P$ of the analyte from the taste diagnostic unit 217, the control unit 212B stores the received taste diagnosis result $JDR_P$ in the database 223 and outputs the taste diagnosis result $JDR_P$ to the transmitting unit 220A.

When the transmitting unit 220A received the taste diagnosis result $JDR_{uni}$ from the control unit 212B, the transmitting unit 220A transmits the taste diagnosis result $JDR_{uni}$ to the terminal device 3A via the network 20.

Furthermore, when the transmitting unit 220A received the taste diagnosis result $JDR_P$ from the control unit 212B, the transmitting unit 220A transmits the taste diagnosis result $JDR_P$ to the terminal device 3A via the network 20.

Figs. 121 and 122 are first and second flowcharts, respectively, for explaining the operation of the diagnostic system 10B shown in Fig. 117.

Referring to Fig. 121, when the operation of diagnostic system 10B is started, the sensor device 1 sequentially executes steps S1 to S4 and S5A described above.

After the step S5A, the terminal device 3A sequentially executes the steps S9 and S10 described above.

After the step S10, the terminal device 3A transmits m pieces of measurement data MRS_1 to MRS_m to analysis device 2C via network 20 (step S11A).

Referring to Fig. 122, after step S11A in Fig. 121, analysis device 2C sequentially executes steps S6A and S7 described above.

After the step S7, the analysis device 2C transmits the m pieces of index data $IDX_1$ to $IDX_m$ to the terminal device 3A via the network 20 (step S15).

Referring to Fig. 121, after the step S15 in Fig. 122, the terminal device 3A receives m pieces of index data $IDX_1$ to $IDX_m$ from analysis device 2C via network 20 (step S16).

Then, the terminal device 3A stores the m pieces of index data $IDX_1$ to $IDX_m$ in the database 37 (step S17).

Thereafter, the terminal device 3A transmits the m pieces of index data $IDX_1$ to $IDX_m$ to the diagnostic device 2B via the network 20 (step S18).

Referring to Fig. 122, after step S18 in Fig. 121, the diagnostic device 2B receives m pieces of index data $IDX_1$ to $IDX_m$ from terminal device 3A via network 20 (step S19).

Thereafter, the diagnostic device 2B sequentially executes the above-mentioned steps S8 and S12.

Referring to Fig. 121, after the step S12 of Fig. 122, the terminal device 3A receives the taste diagnosis results JDR of m analytes via the network 20, stores the taste diagnosis results JDR in the database 37, and displays the taste diagnosis results JDR (step S23).

This completes the operation of the diagnostic system 10B.

In the embodiment of the present invention, the operation of the diagnostic device 2B may be executed by software. In this case, the diagnostic device 2B includes a CPU, a ROM and a RAM.

The ROM stores a program Prog_F consisting of steps S19, S8, and S12 (including the flowcharts shown in Figs. 97 to 99 and 101) shown in Fig. 122, or a program Prog_G consisting of steps S19, S8, and S12 (including the flowcharts shown in Figs. 97, 98, 100, and 102) shown in Fig. 119.

The CPU reads out the program Prog_F or the program Prog_G from the ROM, and executes the read out program Prog_F or the program Prog_G to diagnose the taste of the analyte.

In this case, the RAM temporarily stores the sums L(+)_sum, M(+)_sum, H(+)_sum, L(all)_sum, M(all)_sum, H(all)_sum, H(-)_sum, L(-)_sum_th, M(-)_sum_th, H(-)_sum_th of integrated values, Body index (+), Body index (all), Body index (-)_th, and etc.

Therefore, the program Prog_F or the program Prog_G is a program for causing a computer (CPU) to execute a diagnosis of the taste of analyte.

In the embodiment of the present invention, the operation of the analysis device 2C may be executed by software. In this case, the analysis device 2C includes a CPU, a ROM, and a RAM.

The ROM stores a program Prog_H consisting of steps S6A, S7, and S15 (including the flowcharts shown in Figs. 91 to 96) shown in Fig. 122.

The CPU reads out the program Prog_H from the ROM, and executes the read out program Prog_H to create m pieces of analysis data $ALY_D_1$ to $ALY_D_m$ based on m pieces of measurement data MRS_1 to MRS_m, and update the m pieces of analysis data $ALY_D_1$ to $ALY_D_m$ to m pieces of index data $IDX_1$ to $IDX_m$.

In this case, the RAM temporarily stores m pieces of measurement data MRS_1 to MRS_m and m pieces of analysis data $ALY_D_1$ to $ALY_D_m$.

Furthermore, in the embodiment of the present invention, the operation of the terminal device 3A may be executed by software. In this case, the terminal device 3A includes a CPU, a ROM and a RAM.

The ROM stores a program Prog_I consisting of steps S9, S10, S11A, S16 to S18, and S23 shown in Fig. 121.

The CPU reads out the program Prog_I from the ROM and executes the read out program Prog_I to receive m pieces of measurement data MRS_1 to MRS_m from the sensor device 1, and transmits the received m pieces of measurement data MRS_1 to MRS_m to the analysis device 2C, receives m pieces of index data $IDX_1$ to $IDX_m$ from the analysis device 2C to store them in the database 37, and transmits the m pieces of index data $IDX_1$ to $IDX_m$ to the diagnostic device 2B, receives taste diagnosis results JDR of the m analytes from the diagnostic device 2B to store them in the database 37 and displays the taste diagnosis results JDR.

In this case, the RAM temporarily stores m pieces of measurement data MRS_1 to MRS_m, m pieces of index data $IDX_1$ to $IDX_m$, and a taste diagnosis result JDR.

Fig. 123 is a diagram for explaining the locations of the sensor device 1, the terminal device 3A, the analysis device 2C, and the diagnostic device 2B. In Fig. 123, country A, country B, and country C are different from each other.

Referring to Fig. 123, the sensor device 1, the terminal device 3A, the analysis device 2C and the diagnostic device 2B are placed in country A.

In addition, the sensor device 1, the terminal device 3A and the analysis device 2C are located in country A, and the diagnostic device 2B is located in country B.

Furthermore, the sensor device 1 and the terminal device 3A are located in country B, and the analysis device 2C and the diagnostic device 2B are located in country A.

Furthermore, the sensor device 1 and the terminal device 3A are located in country A, the analysis device 2C is located in country B, and the diagnostic device 2B is located in country A.

Furthermore, the sensor device 1 and the terminal device 3A are located in country A, the analysis device 2C is located in country B, and the diagnostic device 2B is located in country C.

The "1" of deployment country is a pattern in which the sensor device 1, the terminal device 3A, the analysis device 2C, and the diagnostic device 2B are all deployed in the same country.

In this case, the diagnostic device 2B may receive index data $IDX_{uni}$ or P pieces of index data $IDX_1$ to $IDX_P$ from a terminal device 5 (a terminal device having the same configuration as the terminal device 3) located in a country different from country A via the network 20, diagnose the taste of the analyte based on the received index data $IDX_{uni}$ or P pieces of index data $IDX_1$ to $IDX_P$, and may transmit a diagnosis result JDR of the taste of the analyte to the terminal device 5 (a terminal device having the same configuration as the terminal device 3) via the network 20.

The "2" of deployment country is a pattern in which the sensor device 1, the terminal device 3A and the analysis device 2C are deployed in the same country, and the diagnostic device 2B is deployed in a country different from that of the sensor device 1, the terminal device 3A and the analysis device 2C.

In this case, the diagnostic device 2B may diagnose the taste of the analyte based on the index data IDX received from an analysis device (an analysis device different from the analysis device 2C) located in country B, or may diagnose the taste of the analyte based on the index data IDX received from an analysis device (an analysis device different from analysis device 2C) located in country D which is different from countries A and B.

The "3" of deployment country is a pattern in which the analysis device 2C and the diagnostic device 2B are deployed in the same country, and the sensor device 1 and the terminal device 3A are deployed in a country different from that of the analysis device 2C and the diagnostic device 2B.

In this case, the diagnostic device 2B may diagnose the taste of the analyte based on the index data IDX received from an analysis device (an analysis device different from analysis device 2C) located in country A, or may diagnose the taste of the analyte based on the index data IDX received from an analysis device (an analysis device different from analysis device 2C) located in a country other than country A.

The "4" of deployment country is a pattern in which the sensor device 1, the terminal device 3A and the diagnostic device 2B are deployed in the same country, and the analysis device 2C is deployed in a country different from that of the sensor device 1, the terminal device 3A and the diagnostic device 2B.

In this case, the diagnostic device 2B may diagnose the taste of the analyte based on the index data IDX received from an analysis device (an analysis device different from analysis device 2C) located in country A, or may diagnose the taste of the analyte based on the index data IDX received from an analysis device (an analysis device different from analysis device 2C) located in a country other than country A and country B.

The "5" of deployment is a pattern in which the sensor device 1, the terminal device 3A, the analysis device 2C, and the diagnostic device 2B are deployed in different countries each other.

In this case, the diagnostic device 2B may diagnose the taste of the analyte based on the index data IDX received from an analysis device (an analysis device different from analysis device 2C) located in country C, or may diagnose the taste of the analyte based on the index data IDX received from an analysis device (an analysis device different from analysis device 2C) located in a country other than country C.

The rest of the description of the third embodiment is the same as that in the first embodiment.

Fig. 124 is another schematic diagram of the measuring instrument 12 shown in Fig. 4. The measuring instrument 12 shown in Fig. 4 may be a measuring instrument 12A shown in Fig. 124.

Referring to Fig. 124, a measuring instrument 12A has the supply unit 121, the measuring unit 122 and the transmitting unit 123 of the measuring instrument 12 shown in Fig. 4 replaced with a supply circuit 121B, a measuring circuit 122A and a transmitting circuit 123A, respectively.

A supply circuit 121B, a measurement circuit 122A, and a transmitting circuit 123A execute the same operations as the supply unit 121, the measurement unit 122, and the transmitting unit 123 described above, respectively.

Fig. 125 is another schematic diagram of the diagnostic device 2 shown in Fig. 7. The diagnostic device 2 shown in Fig. 7 may be a diagnostic device 2D shown in Fig. 125.

Referring to Fig. 125, in a diagnostic device 2D, the analysis/diagnostic unit 21 of the diagnostic device 2 shown in Fig. 7 is replaced with an analysis/diagnostic circuit 21B.

Analysis/diagnostic circuitry 21B then performs the same operations as analysis/diagnostic unit 21 described above.

Fig. 126 is a schematic diagram of the analysis/diagnostic circuit 21B shown in Fig. 125. Referring to Fig. 126, the analysis/diagnostic circuit 21B is obtained by replacing the receiving unit 211, the control unit 212, the calculation unit 213, the judgment unit 214, the creation unit 215, the calculation unit 216, the taste diagnostic unit 217, the display unit 218 and the reception unit 219 of the analysis/diagnostic unit 21 shown in Fig. 8 with a receiving circuit 211A, a control circuit 212A, an calculation circuit 213A, a judgment circuit 214A, a creation circuit 215A, an calculation circuit 216A, a taste diagnostic circuit 217A, a display circuit 218A and a reception circuit 219A, respectively.

The receiving circuit 211A, the control circuit 212A, the calculation circuit 213A, the judgment circuit 214A, the creation circuit 215A, the calculation circuit 216A, the taste diagnostic circuit 217A, the display circuit 218A and the reception circuit 219A perform the same operations as the receiving unit 211, the control unit 212, the calculation unit 213, the judgment unit 214, the creation unit 215, the calculation unit 216, the taste diagnostic unit 217, the display unit 218 and the receiving unit 219 described above, respectively.

Fig. 127 is another schematic diagram of the terminal device 3 shown in Fig. 111. The terminal device 3 shown in Fig. 111 may be a terminal device 3B shown in Fig. 127.

Referring to Fig. 127, the terminal device 3B has the receiving unit 32, the control unit 33, the transmitting unit 34, the display unit 35 and the receiving unit 36 of terminal device 3 shown in Fig. 111 replaced with a receiving circuit 32A, a control circuit 33A, a transmitting circuit 34A, a display circuit 35A and a reception circuit 36A, respectively.

The receiving circuit 32A, the control circuit 33A, the transmitting circuit 34A, the display circuit 35A and the reception circuit 36A perform the same operations as the receiving unit 32, the control unit 33, the transmitting unit 34, the display unit 35 and the reception unit 36 described above, respectively.

Fig. 128 is another schematic diagram of the diagnostic device 2A shown in Fig. 112. The diagnostic device 2A shown in Fig. 112 may be constituted by a diagnostic device 2E shown in Fig. 128.

Referring to Fig. 128, a diagnostic device 2E has an analysis/diagnostic circuit 21C replacing the analysis/-

diagnostic unit 21A shown in Fig. 112.

The analysis/diagnostic circuitry 21C then performs the same operations as analysis/diagnostic unit 21A described above.

Fig. 129 is another schematic diagram of the analysis/diagnostic unit 21A shown in Fig. 113. The analysis/-diagnostic unit 21A shown in Fig. 113 may be composed of an analysis/diagnostic circuit 21D shown in Fig. 129.

Referring to Fig. 129, the analysis/diagnostic circuit 21D has the receiving unit 211A, control unit 212A, the calculation unit 213, the judgment unit 214, the creation unit 215, the calculation unit 216, the taste diagnostic unit 217 and the transmitting unit 220 of the analysis/diagnostic unit 21A shown in Fig. 113 replaced with a receiving circuit 211B, a control circuit 212B, an calculation circuit 213A, a judgment circuit 214A, a creation circuit 215A, an calculation circuit 216A, a taste diagnostic circuit 217A and a transmitting circuit 220A, respectively.

The receiving circuit 211B, the control circuit 212B, the calculation circuit 213A, the judgment circuit 214A, the creation circuit 215A, the calculation circuit 216A, the taste diagnostic circuit 217A and the transmitting circuit 220A respectively perform the same operations as the receiving unit 211A, the control unit 212A, the calculation unit 213, the judgment unit 214, the creation unit 215, the calculation unit 216, the taste diagnostic unit 217 and the transmitting unit 220 described above.

Fig. 130 is another schematic diagram of the terminal device 3A shown in Fig. 118. The terminal device 3A shown in Fig. 118 may be constituted by the terminal device 3C shown in Fig. 130.

Referring to Fig. 130, terminal device 3C is obtained by replacing the receiving unit 32A, the control unit 33A, the transmitting unit 34A, the display unit 35 and the reception unit 36 of the terminal device 3A shown in Fig. 118 with a receiving circuit 32B, a control circuit 33B, a transmitting circuit 34B, a display circuit 35A and a reception circuit 36A, respectively.

The receiving circuit 32B, the control circuit 33B, the transmitting circuit 34B, the display circuit 35A and the reception circuit 36A respectively perform the same operations as the receiving unit 32A, the control unit 33A, the transmitting unit 34A, the display unit 35 and the reception unit 36 described above.

Fig. 131 is another schematic diagram of the analysis device 2C shown in Fig. 119. The analysis device 2C shown in Fig. 119 may be constituted by the analysis device 2F shown in Fig. 131.

Referring to Fig. 131, the analysis device 2F has the receiving unit 211A, the control unit 212C, the calculation unit 213, the judgment unit 214, the creation unit 215 and the transmitting unit 221 of the analysis device 2C shown in Fig. 119 replaced with a receiving circuit 211B, a control circuit 212D, a calculation circuit 213A, a judgment circuit 214A, a creation circuit 215A and a transmitting circuit 221A, respectively.

The receiving circuit 211B, the control circuit 212D, the calculation circuit 213A, the judgment circuit 214A, the creation circuit 215A and the transmitting circuit 221A execute the same operations as the receiving unit 211A, the control unit 212C, the calculation unit 213, the judgment unit 214, the creation unit 215 and the transmitting unit 221 described above, respectively.

Fig. 132 is another schematic diagram of the diagnostic device 2B shown in Fig. 120. The diagnostic device 2B shown in Fig. 120 may be constituted by a diagnostic device 2G shown in Fig. 132.

Referring to Fig. 132, in a diagnostic device 2G, the receiving unit 211B, the control unit 212B, the calculation unit 216, the taste diagnostic unit 217 and the transmitting unit 220A of the diagnostic device 2B shown in Fig. 120 are replaced with a receiving circuit 211C, a control circuit 212C, a calculation circuit 216A, a taste diagnostic circuit 217A and a transmitting circuit 220B, respectively.

The receiving circuit 211C, the control circuit 212C, the calculation circuit 216A, the taste diagnostic circuit 217A and the transmitting circuit 220B respectively execute the same operations as the receiving unit 211B, the control unit 212B, the calculation unit **216, the** taste diagnostic unit 217 and the transmitting unit 220A described above.

The diagnostic device 2 in the first embodiment, the diagnostic device 2A in the second embodiment, and the diagnostic device 2B in the third embodiment described above are common each other in comprising of the calculation unit 216 calculating the sum $SMT_{_Low}(+)$ of the integral values $ITG_{1_Low}(+)$ to $ITG_{x_Low(+)}$, the sum $SMT_{_Middle(+)}$ of the integral values $ITG_{1_Middle(+)}$ to $ITG_{x_Middle(+)}$, the sum $SMT_{_High(+)}$ of x integral values $ITG_{1_High(+)}$ to $ITG_{x_High(+)}$, the sum $SMT_{_Low(all)}$ of the integral values $ITG1_{_Low(all)}$ to $ITG_{n_Low(all)}$, the sum $SMT_{_Middle(all)}$ of the integral values $ITG_{1_Middle(all)}$ to $ITG_{n_Middle}$(all), the sum $SMT_{_High(all)}$ of the integral values $ITG_{1_ High(all)}$ to $ITG_{n_ High(all)}$, and the sum H(-)_sum of integral values, and
the taste diagnostic unit 217 diagnosing the taste ("astringency", "aftertaste", "sweetness", "aroma" and "bitterness") of the analyte based on the sums $SMT_{_Low(+)}$, $SMT_{_Middle(+)}$, $SMT_{_High(+)}$, $SMT_{_Low(all)}$, $SMT_{_Middle(all)}$, $SMT_{_High(all)}$ and H(-)_sum.

Therefore, according to an embodiment of the present invention,

the diagnostic device may comprises of a first calculation unit calculating a first sum (L(+)_sum) which is a sum of first integral values in a positive predetermined potential section based on a plurality of first integral values in a plurality of predetermined potential sections calculated using the current-potential characteristic of a first cyclic voltammogram measured while changing the potential at a first potential scanning rate, calculating a second sum (M(+)_sum) which is a sum of second integral values in a positive predetermined potential section based on a plurality of second integral values in a plurality of predetermined potential sections calculated using the current-potential characteristic of a second cyclic voltammogram measured while changing the potential at a second potential scanning rate faster than the first potential scanning rate, calculating a third sum (H(+)_sum) which is the sum of the third integral values in a positive predetermined potential section is calculated based on the plurality of third integral values in the plurality of predetermined potential sections calculated using the current-potential characteristics of a third cyclic voltammogram measured while changing the potential at a third potential scanning speed faster than the second potential scanning speed, calculating a fourth sum (H(-)_sum) which is the sum of the third integral values in the negative predetermined potential section based on the plurality of third integral values in the plurality of predetermined potential sections, calculating a fifth sum (L(all))_sum) which is the sum of the first integral values in all of the predetermined potential sections based on the plurality of first integral values in the plurality of predetermined potential sections, calculating a sixth sum (M(all))_sum) which is the sum of the second integral values in all of the predetermined potential sections based on the plurality of second integral values in the plurality of predetermined potential sections, and calculating a seventh sum (H(all))_sum which is the sum of the third integral values in all of the predetermined potential sections based on the plurality of third integral values in the plurality of predetermined potential sections and,

a taste diagnostic unit diagnosing the taste of the analyte based on the first sum (L(+)_sum), the second sum (M(+) _sum), the third sum (H(+)_sum), the fourth sum (H(-)_sum), the fifth sum (L(all))_sum), the sixth sum (M(all))_sum and the seventh sum (H(all))_sum).

Also, according to an embodiment of the present invention,
the program causes execute to computer a first step in which a first calculation unit calculates a first sum (L(+)_sum) which is a sum of first integral values in a positive predetermined potential section based on a plurality of first integral values in a plurality of predetermined potential sections calculated using a current-potential characteristic of a first cyclic voltammogram measured while changing the potential at a first potential scanning rate, calculates a second sum (M(+)_sum) which is a sum of second integral values in a positive predetermined potential section based on a plurality of second integral values in a plurality of predetermined potential sections calculated using a current-potential characteristic of a second cyclic voltammogram measured while changing the potential at a second potential scanning rate which is faster than the first potential scanning rate, calculates a third sum (H(+)_sum) which is the sum of the third integral values in a positive predetermined potential section based on the plurality of third integral values in the plurality of predetermined potential sections calculated using the current-potential characteristics of a third cyclic voltammogram measured while changing the potential at a third potential scanning speed faster than the second potential scanning speed, calculates a fourth sum (H(-)_sum) which is the sum of the third integral values in a negative predetermined potential section based on a plurality of third integral values in the plurality of predetermined potential sections, calculates a fifth sum (L(all))_sum) which is the sum of the first integral values in all predetermined potential sections based on a plurality of first integral values in the plurality of predetermined potential sections, calculates a sixth sum (M(all)_sum) which is sum of the second integral values in all predetermined potential sections based on a plurality of second integral values in the plurality of predetermined potential sections, and calculates a seventh sum (H(all)_sum) which is sum of the third integral values in all predetermined potential sections based on a plurality of third integral values in the plurality of predetermined potential sections and a second step in which the taste diagnostic unit diagnostics the taste of analyte based on the first sum (L(+) _sum), the second sum (M(+)_sum), the third sum (H(+)_sum), the fourth sum (H(-)_sum), the fifth sum (L(all))_sum), the sixth sum (M(all))_sum) and the seventh sum (H(all))_sum).

In an embodiment of the present invention, the scanning speed $V_{r_Low}$ of the potential V constitutes a "first potential scanning speed", the scanning speed $V_{r_Middle}$ of the potential V constitutes a "second potential scanning speed" that is faster than the first potential scanning speed, and the scanning speed $V_{r_High}$ of the potential V constitutes a "third potential scanning speed" that is faster than the second potential scanning speed.

Furthermore, in an embodiment of the present invention, the sum L(+)_sum of the integral values constitutes a "first sum" that is the sum of the first integral values in a positive predetermined potential section based on a plurality of first integral values in a plurality of predetermined potential sections calculated using the current-potential characteristics of a first cyclic voltammogram measured while changing the potential at a potential scan speed $V_{r_Low}$.

Here, the plurality of first integral values may be any of $n^1{}_1$ ($n^1{}_1$ is the number of integral values when the integral value is calculated using the smallest specified potential section, and is composed of an addition result obtained by adding "1" to the integer obtained by rounding down the decimal point of the division result obtained by dividing a positive potential section by the smallest specified potential section when the decimal point of the division result is not zero) first integral values, $n^1{}_2$ ($n^1{}_2<n^1{}_1$) first integral values, $n^1{}_3$ ($n^1{}_3<n^1{}_2$) first integral values, ..., and $n^1{}_b$ ($n^1{}_b<n^1{}_{b-1}$, b is an integer of 2 or more) first integral values.

Furthermore, in an embodiment of the present invention, the sum M(+)_sum of the integral values constitutes a "second sum" that is the sum of the second integral values in a positive predetermined potential section based on a plurality of second integral values in a plurality of predetermined potential sections calculated using the current-potential characteristics of a second cyclic voltammogram measured while changing the potential at the potential scan speed $V_{r_Middle}$.

Here, the plurality of second integral values may be any of $n^2{}_1$ ($n^2{}_1$ is the number of integral values when the integral value is calculated using the smallest specified potential section, and is composed of an addition result obtained by adding "1" to the integer obtained by rounding down the decimal point of the division result obtained by dividing a positive potential section by the smallest specified potential section when the decimal point of the division result is not zero) second integral values, $n^2{}_2$ ($n^2{}_2$<$n^2{}_1$) second integral values, $n^2{}_3$ ($n^2{}_3$<$n^2{}_2$) second integral values, ..., and $n^2{}_b$ ($n^2{}_b$<$n^2{}_{b-1}$, b is an integer of 2 or more) second integral values.

Furthermore, in an embodiment of the present invention, the sum H(+)_sum of the integral values constitutes a "third sum" that is the sum of the third integral values in a positive predetermined potential section based on a plurality of third integral values in a plurality of predetermined potential sections calculated using the current-potential characteristics of a third cyclic voltammogram measured while changing the potential at the potential scan speed $V_{r_High}$.

Here, the plurality of third integral values may be any of $n^3{}_1$ ($n^3{}_1$ is the number of integral values when the integral value is calculated using the smallest specified potential section, and is composed of an addition result obtained by adding "1" to the integer obtained by rounding down the decimal point of the division result obtained by dividing a positive potential section by the smallest specified potential section when the decimal point of the division result is not zero) third integral values, $n^3{}_2$ ($n^3{}_2$<$n^3{}_1$) third integral values, $n^3{}_3$ ($n^3{}_3$<$n^3{}_2$) third integral values, ..., and $n^3{}_b$ ($n^3{}_b$<$n^3{}_{b-1}$, b is an integer of 2 or more) third integral values.

Furthermore, in an embodiment of the present invention, the sum H(-)_sum of the integral values constitutes a "fourth sum" which is the sum of the third integral values in a negative predetermined potential section based on a plurality of third integral values in a plurality of predetermined potential sections calculated using the current-potential characteristics of a third cyclic voltammogram measured while changing the potential at the potential scan speed $V_{r_High}$.

Here, the plurality of third integral values may be any of $n^3{}_1$ ($n^3{}_1$ is the number of integral values when the integral value is calculated using the smallest specified potential section, and is composed of an addition result obtained by adding "1" to the integer obtained by rounding down the decimal point of the division result obtained by dividing a positive potential section by the smallest specified potential section when the decimal point of the division result is not zero) third integral values, $n^3{}_2$ ($n^3{}_2$<$n^3{}_1$) third integral values, $n^3{}_3$ ($n^3{}_3$<$n^3{}_2$) third integral values, ..., and $n^3{}_b$ ($n^3{}_b$<$n^3{}_{b-1}$, b is an integer of 2 or more) third integral values.

Furthermore, in an embodiment of the present invention, the sum L(all)_sum of the integral values constitutes a "fifth sum" which is the sum of the first integral values in all predetermined potential sections based on a plurality of first integral values in a plurality of predetermined potential sections calculated using the current-potential characteristics of the first cyclic voltammogram measured while changing the potential at the potential scan speed $V_{r_Low}$.

Here, the plurality of third integral values may be any of $n^1{}_1$ ($n^1{}_1$ is the number of integral values when the integral value is calculated using the smallest specified potential section, and is composed of an addition result obtained by adding "1" to the integer obtained by rounding down the decimal point of the division result obtained by dividing a positive potential section by the smallest specified potential section when the decimal point of the division result is not zero) first integral values, $n^1{}_2$ ($n^1{}_2$<$n^1{}_1$) first integral values, $n^1{}_3$ ($n^1{}_3$<$n^1{}_2$) first integral values, ..., and $n^1{}_b$ ($n^1{}_b$<$n^1{}_{b-1}$, b is an integer of 2 or more) first integral values.

Furthermore, in an embodiment of the present invention, the sum M(all)_sum of the integral values constitutes a "sixth sum" which is the sum of the second integral values in all of the predetermined potential sections based on a plurality of second integral values in a plurality of predetermined potential sections calculated using the current-potential characteristics of the second cyclic voltammogram measured while changing the potential at the potential scan rate $V_{r_Middle}$.

Here, the plurality of third integral values may be any of $n^2{}_1$ ($n^2{}_1$ is the number of integral values when the integral value is calculated using the smallest specified potential section, and is composed of an addition result obtained by adding "1" to the integer obtained by rounding down the decimal point of the division result obtained by dividing a positive potential section by the smallest specified potential section when the decimal point of the division result is not zero) second integral values, $n^2{}_2$ ($n^2{}_2$<$n^2{}_1$) second integral values, $n^2{}_3$ ($n^2{}_3$<$n^2{}_2$) second integral values, ..., and $n^2{}_b$ ($n^2{}_b$<$n^2{}_{b-1}$, b is an integer of 2 or more) second integral values.

Furthermore, in an embodiment of the present invention, the sum H(all)_sum of the integral values constitutes a "seventh sum" which is the sum of the third integral values in all of the predetermined potential sections based on a plurality of third integral values in a plurality of predetermined potential sections calculated using the current-potential characteristics of the third cyclic voltammogram measured while changing the potential at the potential scan speed $V_{r_High}$.

Here, the plurality of third integral values may be any of $n^3{}_1$ ($n^3{}_1$ is the number of integral values when the integral value is calculated using the smallest specified potential section, and is composed of an addition result obtained by adding "1" to the integer obtained by rounding down the decimal point of the division result obtained by dividing a positive potential section by the smallest specified potential section when the decimal point of the division result is not zero) third integral

values, $n^3_2$ ($n^3_2<n^3_1$) third integral values, $n^3_3$ ($n^3_3<n^3_2$) third integral values, ..., and $n^3_b$ ($n^3_b<n^3_{b-1}$, b is an integer of 2 or more) third integral values.

Furthermore, in an embodiment of the present invention, the sum L(-)_sum_th of the integral values constitutes an "eighth sum" which is the sum of the first integral values in a plurality of negative predetermined potential sections consisting of negative potentials below or equal to a threshold value based on the first cyclic voltammogram measured while changing the potential at the potential scanning speed $V_{r_Low}$.

Here, the "sum of first integral values in a plurality of negative predetermined potential sections consisting of negative potentials below or equal to a threshold value" may be any one of the sum of $w^1_1$ ($w^1_1$ is the sum of an integer obtained by rounding down the decimal point of a division result obtained by dividing a negative potential section below a threshold value by the smallest predetermined potential section when the decimal point of the division result is not zero, and then adding "1" to the integer) first integral values, the sum of $w^1_2$ ($w^1_2<w^1_1$) first integral values, the sum of $w^1_3$ ($w^1_3<w^1_2$) first integral values, ..., and the sum of $w^1_b$ ($w^1_b<w^1_{b-1}$, b is an integer equal to or greater than 2) first integral values.

Furthermore, in an embodiment of the present invention, the sum M(-)_sum_th of the integral values constitutes a "ninth sum" which is the sum of the second integral values in a plurality of negative predetermined potential sections consisting of negative potentials below or equal to a threshold value based on a second cyclic voltammogram measured while changing the potential at a potential scanning speed $V_{r_Middle}$.

Here, the "sum of the second integral values in a plurality of negative predetermined potential sections consisting of negative potentials below or equal to a threshold value" may be any one of the sum of $w^2_1$ ($w^2_1$ is an addition result obtained by adding "1" to an integer obtained by rounding down the decimal point of a division result obtained by dividing a negative potential section below or equal to a threshold value by the smallest predetermined potential section when the decimal point of the division result is not zero) second integral values, the sum of $w^2_2$ ($w^2_2<w^2_1$) second integral values, the sum of $w^2_3$ ($w^2_3<w^2_2$) second integral values, ..., and the sum of $w^2_b$ ($w^2_b<w^2_{b-1}$, b is an integer equal to or greater than 2) second integral values.

Furthermore, in this embodiment of the present invention, the sum H(-)_sum_th of the integral values constitutes a "tenth sum" which is the sum of the third integral values in a plurality of negative predetermined potential sections consisting of negative potentials below or equal to a threshold value based on a third cyclic voltammogram measured while changing the potential at the potential scanning speed $V_{r_High}$.

Here, the "sum of the third integral values in a plurality of negative predetermined potential sections consisting of negative potentials below or equal to the threshold value" may be any one of a sum of $w^3_1$ ($w^3_1$ is an addition result obtained by adding "1" to an integer obtained by rounding down the decimal point of a division result obtained by dividing a negative potential section below or equal to the threshold value by the smallest predetermined potential section when the decimal point of the division result is not zero) third integral values, a sum of $w^3_2$ ($w^3_2<w^3_1$) third integral values, a sum of $w^3_3$ ($w^3_3<w^3_2$) third integral values, ..., and a sum of $w^3_b$ ($w^3_b<w^3_{b-1}$, b is an integer equal to or greater than 2) third integral values.

Furthermore, in an embodiment of the present invention, the Body Index (+) constitutes a "first factor" which is a factor resulting from the diffusion coefficient of a component of the analyte when a positive potential is applied to the analyte.

Furthermore, in an embodiment of the present invention, the Body Index (all) constitutes a "second factor" which is a factor resulting from the diffusion coefficient of the components of the analyte when positive and negative potentials are applied to the analyte.

Furthermore, in an embodiment of the present invention, the Body index (-)_th constitutes a "third factor" which is a factor resulting from the diffusion coefficient of the components of the analyte when a negative potential below or equal to a threshold value is applied to the analyte.

Furthermore, in this embodiment of the present invention, each of the sums $\{ITG_{1_Low}+ ITG_{1_Middle}+ ITG_{1_High}\}$, $\{ITG_{2_Low}+ ITG_{2_Middle}+ITG_{2_High}\}$, ..., $\{ITG_{n-1_Low}+ ITG_{n-1_Middle}+ ITG_{n-1_High}\}$, $\{ITG_{n_Low}+ ITG_{n_Middie}+ ITG_{n_High}\}$ of the integral values shown in Fig. 12 constitutes a "total integral value."

The embodiments disclosed herein are to be considered illustrative in all respects and not restrictive. The scope of the invention is defined by the appended claims, not by the foregoing description of the embodiments, and it is intended that all modifications fall within the scope of claims and their equivalents.

[Industrial Applicability]

The present invention is applicable to a diagnostic device, a diagnostic system using the same, and a program for causing a computer to execute.

## Claims

1. A diagnostic device comprising:

   a first calculation unit configured to calculate a first sum (L(+)_sum) which is a sum of first integral values in a positive predetermined potential section based on a plurality of first integral values in a plurality of predetermined potential sections calculated using a current-potential characteristics of a first cyclic voltammogram measured while changing a potential at a first potential scanning rate, calculate a second sum (M(+)_sum) which is a sum of second integral values in the positive predetermined potential section based on a plurality of second integral values in the plurality of predetermined potential sections calculated using a current-potential characteristic of a second cyclic voltammogram measured while changing the potential at a second potential scanning rate faster than the first potential scanning rate, calculate a third sum (H(+)_sum) which is a sum of third integral values in the positive predetermined potential section based on a plurality of third integral values in the plurality of predetermined potential sections calculated using a current-potential characteristic of a third cyclic voltammogram measured while changing the potential at a third potential scanning rate faster than the second potential scanning rate, calculate a fourth sum (H(-)_sum) which is a sum of the third integral values in a negative predetermined potential sections based on the plurality of third integral values in the plurality of predetermined potential sections, calculate a fifth sum (L(all)_sum) which is a sum of the first integral values in all of the predetermined potential sections based on the plurality of first integral values in the plurality of predetermined potential sections, calculate a sixth sum (M(all)_sum) which is a sum of the second integral values in all of the predetermined potential sections based on the plurality of second integral values in the plurality of predetermined potential sections, and calculate a seventh sum (H(all)_sum) which is a sum of the third integral values in all of the predetermined potential sections based on the plurality of third integral values in the plurality of predetermined potential sections, and
   a taste diagnostic unit configured to diagnose a taste of a first analyte based on the first sum (L(+)_sum), the second sum (M(+)_sum), the third sum (H(+)_sum), the fourth sum (H(-)_sum), the fifth sum (L(all))_sum), the sixth sum (M(all))_sum and the seventh sum (H(all))_sum).

2. The diagnostic device according to claim 1, wherein the first calculation unit further calculates a first factor (Body index (+)) which is a factor attributable to a diffusion coefficient of a component of the first analyte when a positive potential is applied to the first analyte based on the first sum (L(+)_sum), the second sum (M(+)_sum) and the third sum (H(+) _sum) and calculates a second factor (Body index (all)) which is a factor attributable to the diffusion coefficient of the component of the first analyte when positive and negative potentials are applied to the first analyte based on the fifth sum (L(all)_sum), the sixth sum (M(all))_sum, and the seventh sum (H(all)_sum), and
   the taste diagnostic unit diagnoses "astringency" of the first analyte based on the first factor (Body index (+)), diagnoses "aftertaste" of the first analyte based on the second factor (Body Index (all)), diagnoses "sweetness" of the first analyte based on the third sum (H(+)_sum), diagnoses "aroma" of the first analyte based on the fourth sum (H(-) _sum), and diagnoses "bitterness" of the first analyte based on the "astringency" of the first analyte and the "sweetness" of the first analyte.

3. The diagnostic device according to claim 2, wherein the taste diagnostic unit diagnoses the multiplication result obtained by multiplying a coefficient $k_1$ to the first factor (Body index (+)) as the "astringency" of the first analyte, diagnoses the multiplication result obtained by multiplying a coefficient $k_2$ to the second factor (Body Index (all)) as the "aftertaste" of the first analyte, diagnoses as the "sweetness" of the first analyte the result of multiplying a coefficient $k_4$ to the result of dividing the third sum (H(+)_sum) by a coefficient $k_3$, diagnoses as the "aroma" of the first analyte the result of multiplying a coefficient $k_6$ to the result of dividing the fourth sum (H(-)_sum) by a coefficient $k_5$, and diagnoses as the "bitterness" of the first analyte the result obtained by subtracting the result of multiplying a coefficient $k_8$ to the "sweetness" of the first analyte from the result obtained by multiplying a coefficient $k_7$ to the "astringency" of the first analyte.

4. The diagnostic device according to claim 3, wherein the taste diagnostic unit performs a regression analysis using the first factor (Body index (+)) as an explanatory variable and the "astringency" as a response variable to obtain a regression equation, and determines a value multiplied to the first factor (Body index (+)) in the obtained regression equation as the value of the coefficient $k_1$, performs a regression analysis using the second factor (Body index (all)) as an explanatory variable and the "aftertaste" as a response variable to obtain a regression equation, and determines a value multiplied to the second factor (Body index (all)) which is an explanatory variable in the obtained regression equation as a value of the coefficient $k_2$, performs a regression analysis using the third sum (H(+)_sum) as an explanatory variable and the "sweetness" as a response variable to obtain a regression equation, and determines a value dividing the explanatory variable (=the third sum (H(+)_sum)) as a value of the coefficient $k_3$ in the obtained

regression equation, and determines a value multiplied to the explanatory variable (=the third sum (H(+)_sum)) as a value of the coefficient $k_4$, performs a regression analysis using the fourth sum (H(-)_sum) as an explanatory variable and the "aroma" as a response variable to obtain a regression equation, and determines that a value dividing the explanatory variable (the fourth sum (H(-)_sum)) as a value of the coefficient $k_5$ and determines a value multiplied to the explanatory variable (the fourth sum (H(-)_sum)) as a value of the coefficient $k_6$ in the obtained regression equation, and performs a regression analysis using the "astringency" and the "sweetness" as explanatory variables and the "bitterness" as a response variable to obtain a regression equation, and determines a value multiplied to the "astringency" as a value of the coefficient $k_7$ and determines a value multiplied to the "sweetness" as a value of the coefficient $k_8$ in the obtained regression equation.

**5.** The diagnostic device according to claim 3, wherein the taste diagnostic unit updates the values of the coefficients $k_1$ to $k_8$ when the taste diagnostic unit diagnosed the "astringency", the "aftertaste", the "sweetness", the "aroma" and the "bitterness" of v (v is an integer of 1 or more) of the first analytes, and diagnoses the "astringency", the "aftertaste", the "sweetness", the "aroma" and the "bitterness" of the first analyte using the updated values of the coefficients $k_1$ to $k_8$.

**6.** The diagnostic device according to claim 1, wherein the plurality of first integral values are any of $n^1{}_1$ ($n^1{}_1$ is the number of integral values calculated using the smallest predetermined potential section, and is an addition result obtained by adding "1" to an integer obtained by rounding down the decimal point of a division result obtained by dividing a positive potential section by the smallest predetermined potential section when the decimal point of the division result is not zero) first integral values, $n^1{}_2$ ($n^1{}_2<n^1{}_1$) first integral values, $n^1{}_3$ ($n^1{}_3<n^1{}_2$) first integral values, ..., and $n^1{}_b$ ($n^1{}_b<n^1{}_{b-1}$, b is an integer equal to or greater than 2) first integral values,

the plurality of second integral values are any of $n^2{}_1$ ($n^2{}_1$ is the number of integral values calculated using the smallest predetermined potential section, and is an addition result obtained by adding "1" to an integer obtained by rounding down the decimal point of a division result obtained by dividing a positive potential section by the smallest predetermined potential section when the decimal point of the division result is not zero) second integral values, $n^2{}_2$ ($n^2{}_2<n^2{}_1$) second integral values, $n^2{}_3$ ($n^2{}_3<n^2{}_2$) second integral values, ..., and $n^2{}_b$ ($n^2{}_b<n^2{}_{b-1}$, b is an integer equal to or greater than 2) second integral values, and
the plurality of third integral values are any of $n^3{}_1$ ($n^3{}_1$ is the number of integral values calculated using the smallest predetermined potential section, and is composed of an addition result obtained by adding "1" to an integer obtained by rounding down the decimal point of a division result obtained by dividing a positive potential section by the smallest predetermined potential section when the decimal point of the division result is not zero) third integral values, $n^3{}_2$ ($n^3{}_2<n^3{}_1$) third integral values, $n^3{}_3$ ($n^3{}_3<n^3{}_2$) third integral values, ..., and $n^3{}_b$ ($n^3{}_b<n^3{}_{b-1}$, b is an integer equal to or greater than 2) third integral values.

**7.** The diagnostic device according to claim 1, wherein the first calculation unit further calculates an eighth sum (L(-)_sum_th) which is a sum of the first integral values in a plurality of negative predetermined potential sections consisting of negative potentials equal to or lower than a threshold value based on a plurality of first integral values in a plurality of predetermined potential sections calculated using the current-potential characteristic of the first cyclic voltammogram, calculates a ninth sum (M(-)_sum_th) which is a sum of the second integral values in the plurality of negative predetermined potential sections consisting of negative potentials equal to or lower than the threshold value based on a plurality of second integral values in the plurality of predetermined potential sections calculated using the current-potential characteristic of the second cyclic voltammogram, calculates a tenth sum (H(-)_sum_th) which is a sum of the third integral values in the plurality of negative predetermined potential sections consisting of negative potentials equal to or lower than the threshold value based on the plurality of third integral values in the plurality of predetermined potential sections calculated using the current-potential characteristic of the third cyclic voltammogram, and calculates a third factor (Body index (-)_th) which is a factor attributable to a diffusion coefficient of a component of a second analyte when a negative potential equal to or less than the threshold value is applied to the second analyte based on the eighth sum (L(-)_sum_th), the ninth sum (M(-)_sum_th) and the tenth sum (H(-)_sum_th), and
the taste diagnostic unit further diagnoses the "astringency" of the second analyte based on the third factor (Body index (-)_th).

**8.** The diagnostic device according to claim 7, wherein the taste diagnostic unit diagnoses the multiplication result obtained by multiplying a coefficient $k_9$ to the third factor (Body index (-)_th) as the "astringency" of the second analyte.

**9.** The diagnostic device according to claim 8, wherein the taste diagnostic unit performs a regression analysis using the third factor (Body index (-)_th) as an explanatory variable and the "astringency" as a response variable to obtain a regression equation, and determines a value multiplied to the third factor (Body index (-)_th) in the obtained regression equation as a value of the coefficient $k_9$.

**10.** The diagnostic device according to claim 8, wherein the taste diagnostic unit updates the value of the coefficient $k_9$ and diagnoses the "astringency" of the second analyte using the updated value of the coefficient $k_9$ when the taste diagnostic unit diagnosed the "astringency" of v (v is an integer equal to or greater than 1) of the second analyte(s).

**11.** The diagnostic device according to claim 7, wherein the sum of the first integral values in a plurality of negative predetermined potential sections consisting of negative potentials equal to or less than the threshold value is any one of a sum of $w^1_1$ ($w^1_1$ is an addition result obtained by adding "1" to an integer obtained by rounding down a decimal point of a division result obtained by dividing a negative potential section equal to or less than the threshold value by a minimum predetermined potential section when the decimal point of the division result is not zero) first integral values, a sum of $w^1_2$ ($w^1_2<w^1_1$) first integral values, a sum of $w^1_3$ ($w^1_3<w^1_2$) first integral values, ..., and a sum of $w^1_b$ ($w^1_b<w^1_{b-1}$, b is an integer equal to or greater than 2) first integral values,

the sum of the second integral values in the plurality of negative predetermined potential sections consisting of negative potentials equal to or less than the threshold value is any one of a sum of $w^2_1$ ($w^2_1$ is an addition result obtained by adding "1" to an integer obtained by rounding down a decimal point of a division result obtained by dividing a negative potential section equal to or less than a threshold value by a smallest predetermined potential section when the decimal point of the division result is not zero.) second integral values, a sum of $w^2_2$ ($w^2_2<w^2_1$) second integral values, a sum of $w^2_3$ ($w^2_3<w^2_2$) second integral values, ..., and a sum of $w^2_b$ ($w^2_b<_w{}^2_{b-1}$, b is an integer equal to or greater than 2) second integral values, and

the sum of the third integral values in the plurality of negative predetermined potential sections consisting of negative potentials equal to or less than the threshold value is any one of a sum of $w^3_1$ ($w^3_1$ is an addition result obtained by adding "1" to an integer obtained by rounding down a decimal point of a division result obtained by dividing a negative potential section equal to or less than a threshold value by a minimum predetermined potential section when the decimal point of the division result is not zero.) third integral values, a sum of $w^3_2$ ($w^3_2<w^3_1$) third integral values, a sum of $w^3_3$ ($w^3_3<w^3_2$) third integral values, ..., and a sum of $w^3_b$ ($w^3_b<_w{}^3_{b-1}$, b is an integer equal to or greater than 2) third integral values.

**12.** The diagnostic device according to claim 1, wherein further comprising a second calculation unit calculates the plurality of first integral values in the plurality of predetermined potential sections based on the current-potential characteristics of the first cyclic voltammogram, calculates the plurality of second integral values in the plurality of predetermined potential sections based on the current-potential characteristics of the second cyclic voltammogram, and calculates the plurality of third integral values in the plurality of predetermined potential sections based on the current-potential characteristics of the third cyclic voltammogram, and
the first calculation unit calculates the first sum (L(+)_sum) and the fifth sum (L(all)_sum) based on the plurality of first integral values calculated by the second calculation unit, calculates the second sum (M(+)_sum) and the sixth sum (M(all)_sum) based on the plurality of second integral values calculated by the second calculation unit, and calculates the third sum (H(+)_sum), the fourth sum (H(-)_sum), and the seventh sum (H(all)_sum) based on the plurality of third integral values calculated by the second calculation unit.

**13.** The diagnostic device according to claim 12, wherein further comprising a creation unit configured to create, as a feature amount of the first analyte or the second analyte, a curve indicating the dependency of the plurality of sum integral values on the plurality of predetermined potential sections based on the plurality of predetermined potential sections and a plurality of sum integral values respectively corresponding to the plurality of predetermined potential sections, and
the second calculation unit further calculates the total integral value which is a sum of the first integral value, the second integral value, and the third integral value in one of the predetermined potential sections for all of the plurality of predetermined potential sections to calculate the plurality of total integral values, and outputs the plurality of predetermined potential sections and the plurality of total integral values respectively associated with the plurality of predetermined potential sections to the creation unit.

**14.** The diagnostic device according to claim 13, wherein calculation data includes the plurality of predetermined potential sections and the plurality of total integral values respectively associated with the plurality of predetermined potential sections,

further comprising a judgment unit judging whether P (P is an integer equal to or greater than 2) pieces of "the plurality of total integral values" included in P pieces of the calculation data are or not different from each other, and the creation unit creates P pieces of the curves when the judgment unit judges that the P pieces of "the plurality of total integral values" are different from each other.

**15.** A diagnostic system comprising the diagnostic device according to any one of claims 1 to 14.

**16.** A program causing a computer to execute:

a first step in which a first calculation unit calculates a first sum (L(+)_sum) which is a sum of first integral values in a positive predetermined potential section based on a plurality of first integral values in a plurality of predetermined potential sections calculated using a current-potential characteristics of a first cyclic voltammogram measured while changing a potential at a first potential scanning rate, calculates a second sum (M(+)_sum) which is a sum of second integral values in the positive predetermined potential section based on a plurality of second integral values in the plurality of predetermined potential sections calculated using a current-potential characteristic of a second cyclic voltammogram measured while changing the potential at a second potential scanning rate faster than the first potential scanning rate, calculates a third sum (H(+)_sum) which is a sum of third integral values in the positive predetermined potential section based on a plurality of third integral values in the plurality of predetermined potential sections calculated using a current-potential characteristic of a third cyclic voltammogram measured while changing the potential at a third potential scanning rate faster than the second potential scanning rate, calculates a fourth sum (H(-)_sum) which is a sum of the third integral values in a negative predetermined potential sections based on the plurality of third integral values in the plurality of predetermined potential sections, calculates a fifth sum (L(all)_sum) which is a sum of the first integral values in all of the predetermined potential sections based on the plurality of first integral values in the plurality of predetermined potential sections, calculates a sixth sum (M(all)_sum) which is sum of the second integral values in all of the predetermined potential sections based on the plurality of second integral values in the plurality of predetermined potential sections, and calculates a seventh sum (H(all)_sum) which is sum of the third integral values in all of the predetermined potential sections based on the plurality of third integral values in the plurality of predetermined potential sections and,
a second step in which a taste diagnostic unit diagnoses a taste of a first analyte based on the first sum (L(+) _sum), the second sum (M(+)_sum), the third sum (H(+)_sum), the fourth sum (H(-)_sum), the fifth sum (L(all)) _sum), the sixth sum (M(all))_sum and the seventh sum (H(all))_sum).

**17.** The program according to claim 16, wherein the first calculation unit, in the first step, further calculates a first factor (Body index (+)), which is a factor attributable to a diffusion coefficient of a component of the first analyte when a positive potential is applied to the first analyte based on the first sum (L(+)_sum), the second sum (M(+)_sum) and the third sum (H(+)_sum), and calculates a second factor (Body index (all)) which is a factor attributable to the diffusion coefficient of the component of the first analyte when positive and negative potentials are applied to the first analyte based on the fifth sum (L(all))_sum, the sixth sum (M(all))_sum, and the seventh sum (H(all))_sum, and
the taste diagnostic unit, in the second step, diagnoses "astringency" of the first analyte based on the first factor (Body index (+)), diagnoses "aftertaste" of the first analyte based on the second factor (Body index (all)), diagnoses "sweetness" of the first analyte based on the third sum (H(+)_sum), diagnoses "aroma" of the first analyte based on the fourth sum (H(-)_sum), and diagnoses "bitterness" of the first analyte based on the "astringency" of the first analyte and the "sweetness" of the first analyte.

**18.** The program according to claim 17, wherein the taste diagnostic unit, in the second step, diagnoses the multiplication result obtained by multiplying a coefficient $k_1$ to the first factor (Body index (+)) as the "astringency" of the first analyte, diagnoses the multiplication result obtained by multiplying a coefficient $k_2$ to the second factor (Body index (all)) as the "aftertaste" of the first analyte, diagnoses the result of multiplying a coefficient $k_4$ to the result of dividing the third sum (H(+)_sum) by a coefficient $k_3$ as the "sweetness" of the first analyte, diagnoses the result of multiplying a coefficient $k_6$ to the result of dividing the fourth sum (H(-)_sum) by a coefficient $k_5$ as the "aroma" of the first analyte, and diagnoses as the "bitterness" of the first analyte a subtraction result subtracting the multiplication result obtained by multiplying a coefficient $k_8$ to the "sweetness" of the first analyte from the multiplication result obtained by multiplying a coefficient $k_7$ to the "astringency" of the first analyte.

**19.** The program according to claim 18, wherein the taste diagnostic unit, in the second step, performs a regression analysis using the first factor (Body index (+)) as an explanatory variable and the "astringency" as a response variable to obtain a regression equation, and determines as s value of the coefficient $k_1$ a value multiplied to the first factor (Body index (+)) in the obtained regression equation, performs a regression analysis using the second factor (Body index (all)) as an explanatory variable and the "aftertaste" as a response variable to obtain a regression equation, and determines as a value of the coefficient $k_2$ a value multiplied to the second factor (Body index (all)) which is the explanatory variable in the obtained regression equation,

performs a regression analysis using the third sum (H(+)_sum) as an explanatory variable and the "sweetness" as a response variable to obtain a regression equation, and determines, as a value of the coefficient $k_3$, a value dividing the explanatory variable (=the third sum (H(+)_sum)) in the obtained regression equation, and determines a value multiplied to the explanatory variable (=the third sum (H(+)_sum)) as a value of the coefficient $k_4$,
performs a regression analysis using the fourth sum (H(-)_sum) as an explanatory variable and the "aroma" as a response variable to obtain a regression equation, and determines a value dividing the explanatory variable (the fourth sum (H(-)_sum)) in the obtained regression equation as a value of the coefficient $k_5$, and determines a value multiplied to the explanatory variable (the fourth sum (H(-)_sum)) as a value of the coefficient $k_6$,
and performs a regression analysis using the "astringency" and the "sweetness" as explanatory variables and the "bitterness" as a response variable to obtain a regression equation, determines a value multiplied to the "astringency" in the obtained regression equation as a value of the coefficient $k_7$, and determines a value multiplied to the "sweetness" as a value of the coefficient $k_8$.

**20.** The program according to claim 18, wherein the taste diagnostic unit updates values of the coefficients $k_1$ to $k_8$, and diagnoses the "astringency", the "aftertaste", the "sweetness", the "aroma" and the "bitterness" of the first analyte using the updated values of the coefficients $k_1$ to $k_8$ when the taste diagnostic unit, in the second step, diagnosed the "astringency", the "aftertaste", the "sweetness", the "aroma" and the "bitterness" of v (v is an integer of 1 or more) first analytes.

**21.** The program according to claim 16, wherein the plurality of first integral values are any one of $n^1_1$ ($n^1_1$ is the number of integral values calculated using the smallest predetermined potential section, and is an addition result obtained by adding "1" to an integer obtained by rounding down the decimal point of a division result obtained by dividing a positive potential section by the smallest predetermined potential section when the decimal point of the division result is not zero) first integral values, $n^1_2$ ($n^1_2<n^1_1$) first integral values, $n^1_3$ ($n^1_3<n^1_2$) first integral values, ..., and $n^1_b$ ($n^1_b<n^1_{b-1}$, b is an integer equal to or greater than 2) first integral values,

the plurality of second integral values are any of $n^2_1$ ($n^2_1$ is the number of integral values calculated using the smallest predetermined potential section, and is an addition result obtained by adding "1" to an integer obtained by rounding down the decimal point of a division result obtained by dividing a positive potential section by the smallest predetermined potential section when the decimal point of the division result is not zero) second integral values, $n^2_2$ ($n^2_2<n^2_1$) second integral values, $n^2_3$ ($n^2_3<n^2_2$) second integral values, ..., and $n^2_b$ ($n^2_b<n^2_{b-1}$, b is an integer equal to or greater than 2) second integral values, and
the plurality of third integral values are any of $n^3_1$ ($n^3_1$ is the number of integral values calculated using the smallest predetermined potential section, and is composed of an addition result obtained by adding "1" to an integer obtained by rounding down the decimal point of a division result obtained by dividing a positive potential section by the smallest predetermined potential section when the decimal point of the division result is not zero) third integral values, $n^3_2$ ($n^3_2<n^3_1$) third integral values, $n^3_3$ ($n^3_3<n^3_2$) third integral values, ..., and $n^3_b$ ($n^3_b<n^3_{b-1}$, b is an integer equal to or greater than 2) third integral values.

**22.** The program according to claim 16, wherein the first calculation unit, in the first step, further calculates an eighth sum (L(-)_sum_th) which is a sum of the first integral values in a plurality of negative predetermined potential sections consisting of negative potentials equal to or less than a threshold value based on a plurality of first integral values in a plurality of predetermined potential sections calculated using the current-potential characteristic of the first cyclic voltammogram, calculates a ninth sum (M(-)_sum_th) which is a sum of the second integral values in a plurality of negative predetermined potential sections consisting of negative potentials equal to or lower than the threshold value based on a plurality of second integral values in the plurality of predetermined potential sections calculated using the current-potential characteristic of the second cyclic voltammogram, calculates a tenth sum (H(-)_sum_th) which is a sum of the third integral values in the plurality of negative predetermined potential sections consisting of negative potentials equal to or lower than the threshold value based on a plurality of third integral values in the plurality of predetermined potential sections calculated using the current-potential characteristic of the third cyclic voltammogram, and calculates a third factor (Body index (-)_th) which is a factor attributable to a diffusion coefficient of a component of a second analyte when a negative potential equal to or less than the threshold value is applied to the second analyte based on the eighth sum (L(-)_sum_th), the ninth sum (M(-)_sum_th) and the tenth sum (H(-)_sum_th), and

the taste diagnostic unit, in the second step, further diagnoses the "astringency" of the second analyte based on the third factor (Body index (-)_th).

**23.** The program according to claim 22, wherein the taste diagnostic unit, in the second step, diagnoses, as the "astringency" of the second analyte, the multiplication result obtained by multiplying the coefficient $k_9$ to the third factor (Body index (-)_th).

**24.** The program according to claim 23, wherein the taste diagnostic unit, in the second step, performs a regression analysis using the third factor (Body index (-)_th) as an explanatory variable and the "astringency" as a response variable to obtain a regression equation, and determines a value multiplied to the third factor (Body index (-)_th) in the obtained regression equation as a value of the coefficient $k_9$.

**25.** The program according to claim 23, wherein the taste diagnostic unit updates the value of the coefficient $k_9$ and diagnoses the "astringency" of the second analyte using the updated value of the coefficient $k_9$ when the taste diagnostic unit, in the second step, diagnosed the "astringency" of v (v is an integer equal to or greater than 1) second analytes.

**26.** The program according to claim 22, wherein the sum of the first integral values in a plurality of negative predetermined potential sections consisting of negative potentials equal to or less than the threshold value is any one of a sum of $w^1_1$ ($w^1_1$ is an addition result obtained by adding "1" to an integer obtained by rounding down a decimal point of a division result obtained by dividing a negative potential section equal to or less than the threshold value by a minimum predetermined potential section when the decimal point of the division result is not zero) first integral values, a sum of $w^1_2$ ($w^1_2$<$w^1_1$) first integral values, a sum of $w^1_3$ ($w^1_3$<$w^1_2$) first integral values, ..., and a sum of $w^1_b$ ($w^1_b$<$w^1_{b-1}$, b is an integer equal to or greater than 2) first integral values,

the sum of the second integral values in the plurality of negative predetermined potential sections consisting of negative potentials equal to or less than the threshold value is any one of a sum of $w^2_1$ ($w^2_1$ is an addition result obtained by adding "1" to an integer obtained by rounding down a decimal point of a division result obtained by dividing a negative potential section equal to or less than a threshold value by a smallest predetermined potential section when the decimal point of the division result is not zero) second integral values, a sum of $w^2_2$ ($w^2_2$<$w^2_1$) second integral values, a sum of $w^2_3$ ($w^2_3$<$w^2_2$) second integral values, ..., and a sum of $w^2_b$ ($w^2_b$<$W^2_{b-1}$, b is an integer equal to or greater than 2) second integral values, and

the sum of the third integral values in the plurality of negative predetermined potential sections consisting of negative potentials equal to or less than the threshold value is any one of a sum of $w^3_1$ ($w^3_1$ is an addition result obtained by adding "1" to an integer obtained by rounding down a decimal point of a division result obtained by dividing a negative potential section equal to or less than a threshold value by a minimum predetermined potential section when the decimal point of the division result is not zero) third integral values, a sum of $w^3_2$ ($w^3_2$<$w^3_1$) third integral values, a sum of $w^3_3$ ($w^3_3$<$w^3_2$) third integral values, ..., and a sum of $w^3_b$ ($w^3_b$<$w^3_{b-1}$, b is an integer equal to or greater than 2) third integral values.

**27.** The program according to claim 16, wherein further causing a computer to execute a third step in which a second calculation unit calculates the plurality of first integral values in the plurality of predetermined potential sections based on the current-potential characteristic of the first cyclic voltammogram, calculates the plurality of second integral values in the plurality of predetermined potential sections based on the current-potential characteristic of the second cyclic voltammogram, and calculates the plurality of third integral values in the plurality of predetermined potential sections based on the current-potential characteristic of the third cyclic voltammogram, and

the first calculation unit, in the first step, calculates the first sum (L(+)_sum) and the fifth sum (L(all)_sum) based on the plurality of first integral values calculated by the second calculation unit, calculates the second sum (M(+)_sum) and the sixth sum (M(all)_sum) based on the plurality of second integral values calculated by the second calculation unit, and calculates the third sum (H(+)_sum), the fourth sum (H(-)_sum), and the seventh sum (H(all)_sum) based on the plurality of third integral values calculated by the second calculation unit.

**28.** The program according to claim 27, wherein further causing a computer to execute a fourth step in which a creation unit creates, as a feature amount of the first analyte or the second analyte, a curve showing the dependency of the plurality of total integral values on the plurality of predetermined potential sections based on the plurality of predetermined potential sections and a plurality of total integral values respectively corresponding to the plurality of predetermined potential sections, and

the second calculation unit further, in the third step, executes to calculate the total integral value that is a sum of the first integral value, the second integral value, and the third integral value in one of the predetermined potential sections for all of the plurality of predetermined potential sections to calculate the plurality of total integral values, and outputs the plurality of predetermined potential sections and the plurality of total integral values respectively associated with the plurality of predetermined potential sections to the creation unit.

**29.** The program according to claim 28, wherein calculation data includes the plurality of predetermined potential sections and the plurality of total integral values respectively corresponding to the plurality of predetermined potential sections,

the program causes the computer to execute further a fifth step in which a judgment unit judges whether P (P is an integer equal to or greater than 2) pieces of [the plurality of sum integral values] included in P pieces of the calculation data are or not different from each other, and
the creation unit creates P pieces of the curves in the fourth step when the judgment unit judged in the fifth step that the P pieces of [the plurality of total integral values] are different from each other.

FIG. 1

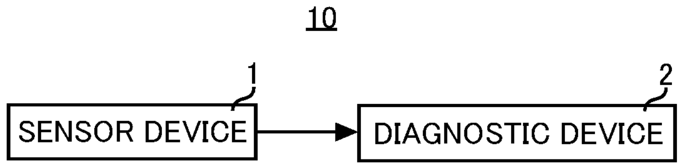


FIG. 2

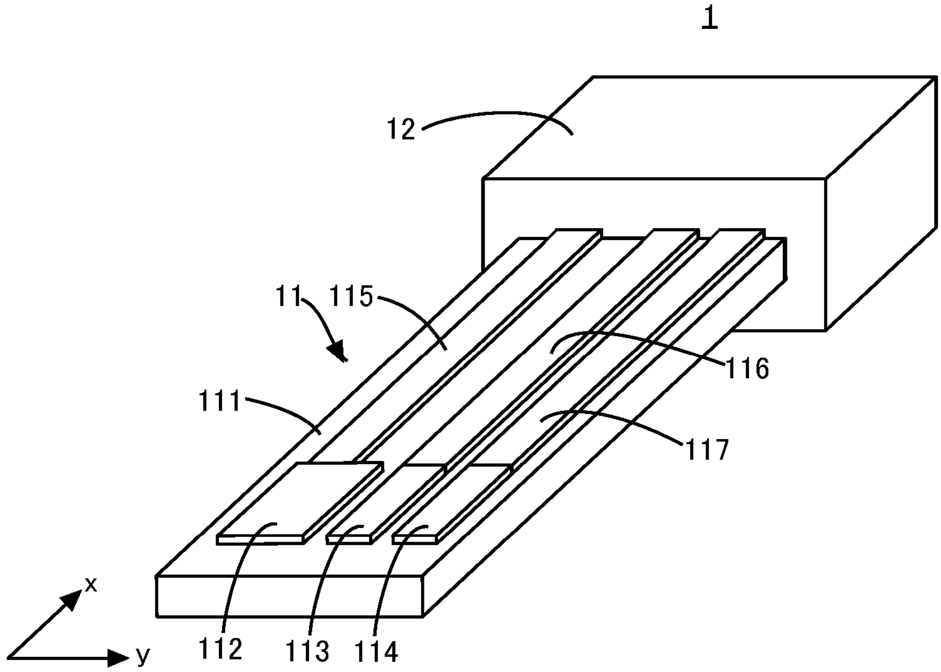


FIG. 3

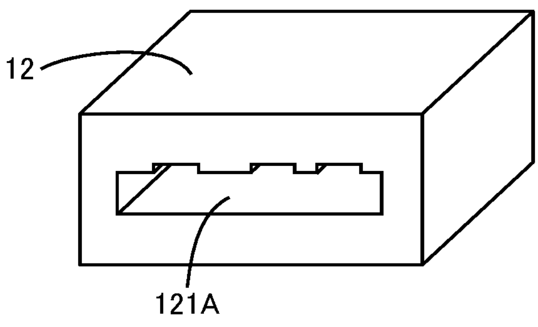

FIG. 4

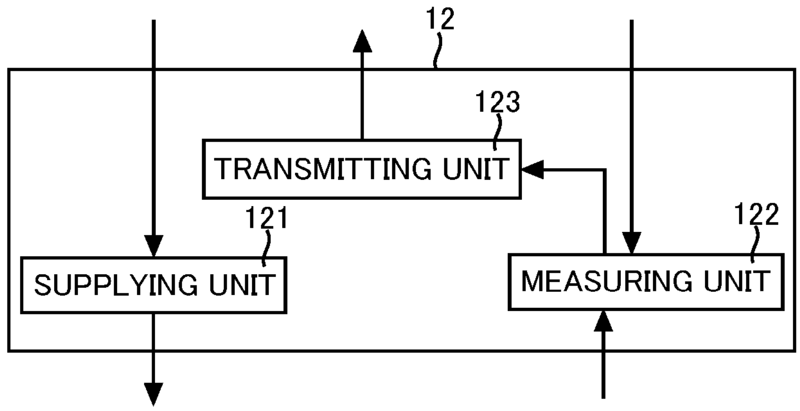
12
123
TRANSMITTING UNIT
121
122
SUPPLYING UNIT
MEASURING UNIT

FIG. 5

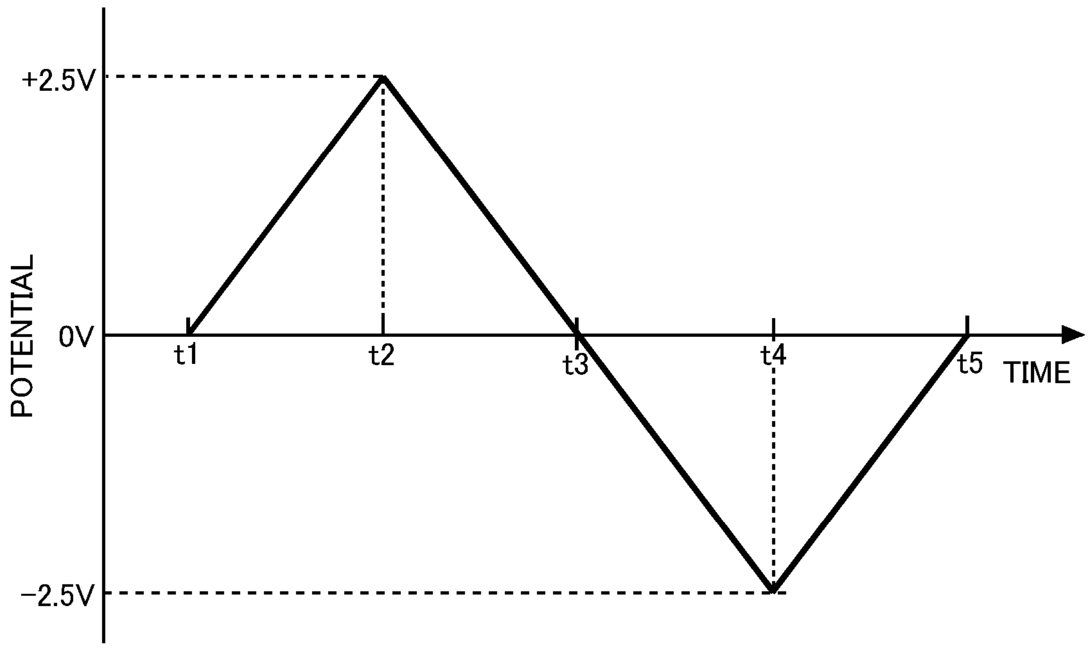
+2.5V
POTENTIAL
0V
−2.5V
t1
t2
t3
t4
t5
TIME

FIG. 6

MRS

| NAME OF ANALYTE | | | | | |
|---|---|---|---|---|---|
| TYPE OF ANALYTE | | | | | |
| MEASUREMENT DATA MRS_Low | | MEASUREMENT DATA MRS_Middle | | MEASUREMENT DATA MRS_High | |
| POTENTIAL SCANNING SPEED : $V_{r_Low}$ | | POTENTIAL SCANNING SPEED : $V_{r_Middle}$ | | POTENTIAL SCANNING SPEED : $V_{r_High}$ | |
| POTENTIAL $V_{Low}$ | CURRENT $I_{Low}$ | POTENTIAL $V_{Middle}$ | CURRENT $I_{Middle}$ | POTENTIAL $V_{High}$ | CURRENT $I_{High}$ |
| $V_{1_Low}$ | $I_{1_Low}$ | $V_{1_Middle}$ | $I_{1_Middle}$ | $V_{1_High}$ | $I_{1_High}$ |
| $V_{2_Low}$ | $I_{2_Low}$ | $V_{2_Middle}$ | $I_{2_Middle}$ | $V_{2_High}$ | $I_{2_High}$ |
| $V_{3_Low}$ | $I_{3_Low}$ | $V_{3_Middle}$ | $I_{3_Middle}$ | $V_{3_High}$ | $I_{3_High}$ |
| $V_{4_Low}$ | $I_{4_Low}$ | $V_{4_Middle}$ | $I_{4_Middle}$ | $V_{4_High}$ | $I_{4_High}$ |
| ⋮ | ⋮ | ⋮ | ⋮ | ⋮ | ⋮ |
| $V_{d-2_Low}$ | $I_{d-2_Low}$ | $V_{d-2_Middle}$ | $I_{d-2_Middle}$ | $V_{d-2_High}$ | $I_{d-2_High}$ |
| $V_{d-1_Low}$ | $I_{d-1_Low}$ | $V_{d-1_Middle}$ | $I_{d-1_Middle}$ | $V_{d-1_High}$ | $I_{d-1_High}$ |
| $V_{d_Low}$ | $I_{d_Low}$ | $V_{d_Middle}$ | $I_{d_Middle}$ | $V_{d_High}$ | $I_{d_High}$ |

FIG. 7

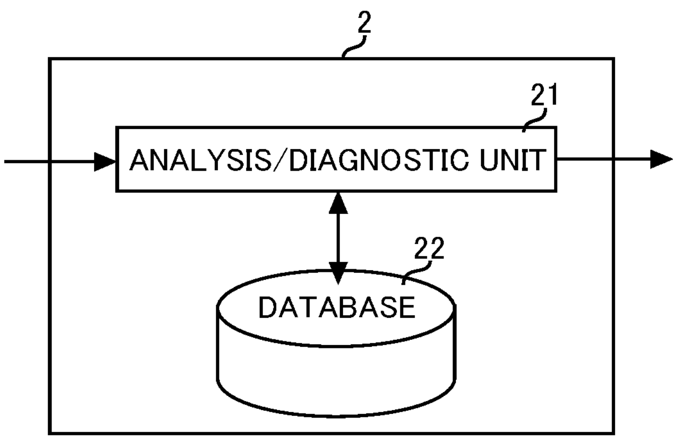

FIG. 8

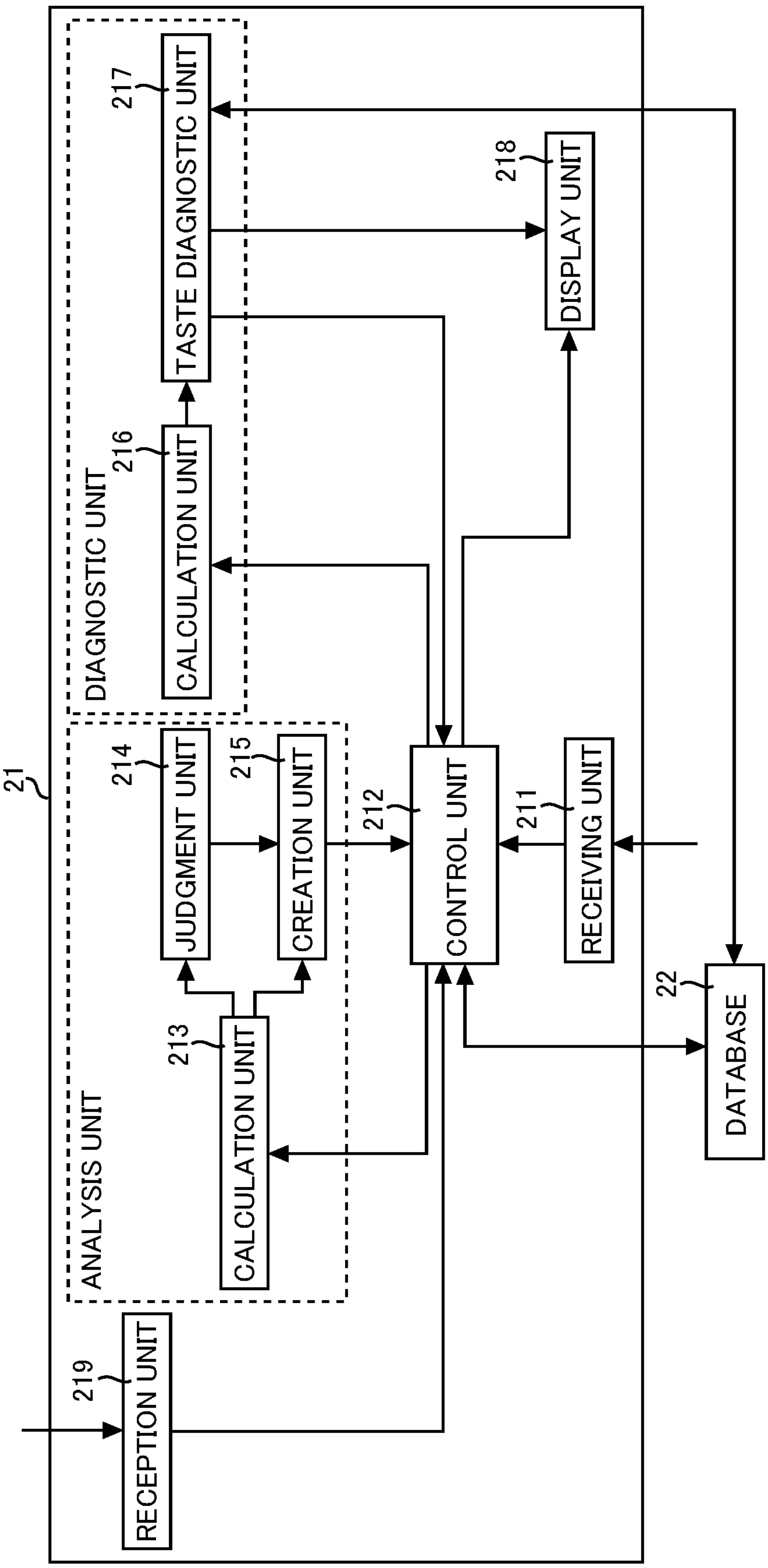
21
RECEPTION UNIT
219
ANALYSIS UNIT
CALCULATION UNIT
213
JUDGMENT UNIT
214
CREATION UNIT
215
DIAGNOSTIC UNIT
CALCULATION UNIT
216
TASTE DIAGNOSTIC UNIT
217
CONTROL UNIT
212
RECEIVING UNIT
211
DISPLAY UNIT
218
DATABASE
22

FIG. 9

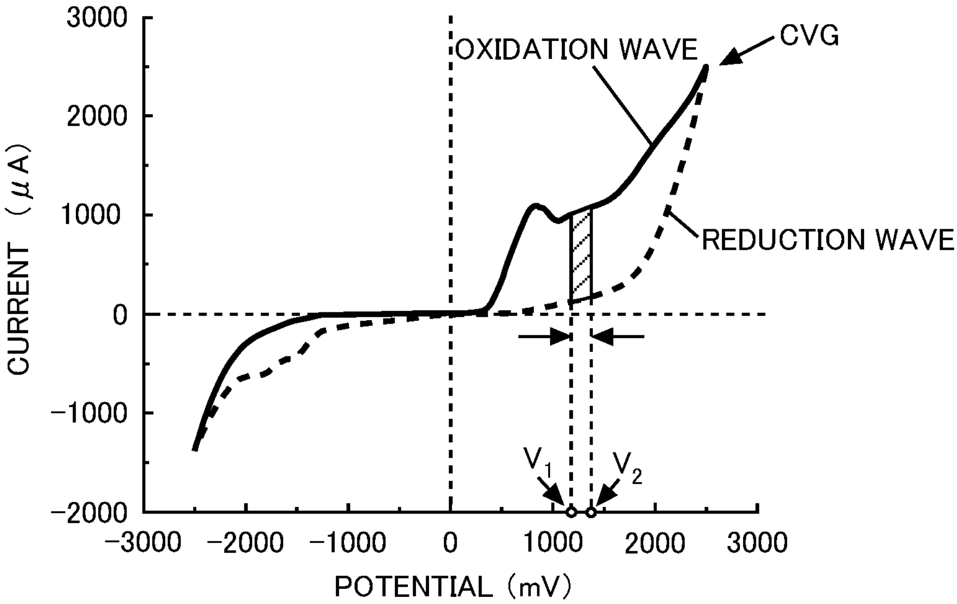
3000
2000
1000
0
−1000
−2000
CURRENT (μA)
−3000
−2000
−1000
0
1000
2000
3000
POTENTIAL (mV)
OXIDATION WAVE
CVG
REDUCTION WAVE
V1
V2

FIG. 10

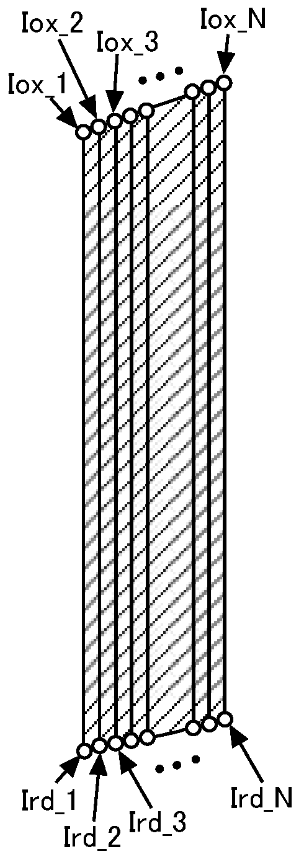
Iox_1
Iox_2
Iox_3
Iox_N
Ird_1
Ird_2
Ird_3
Ird_N

FIG. 11

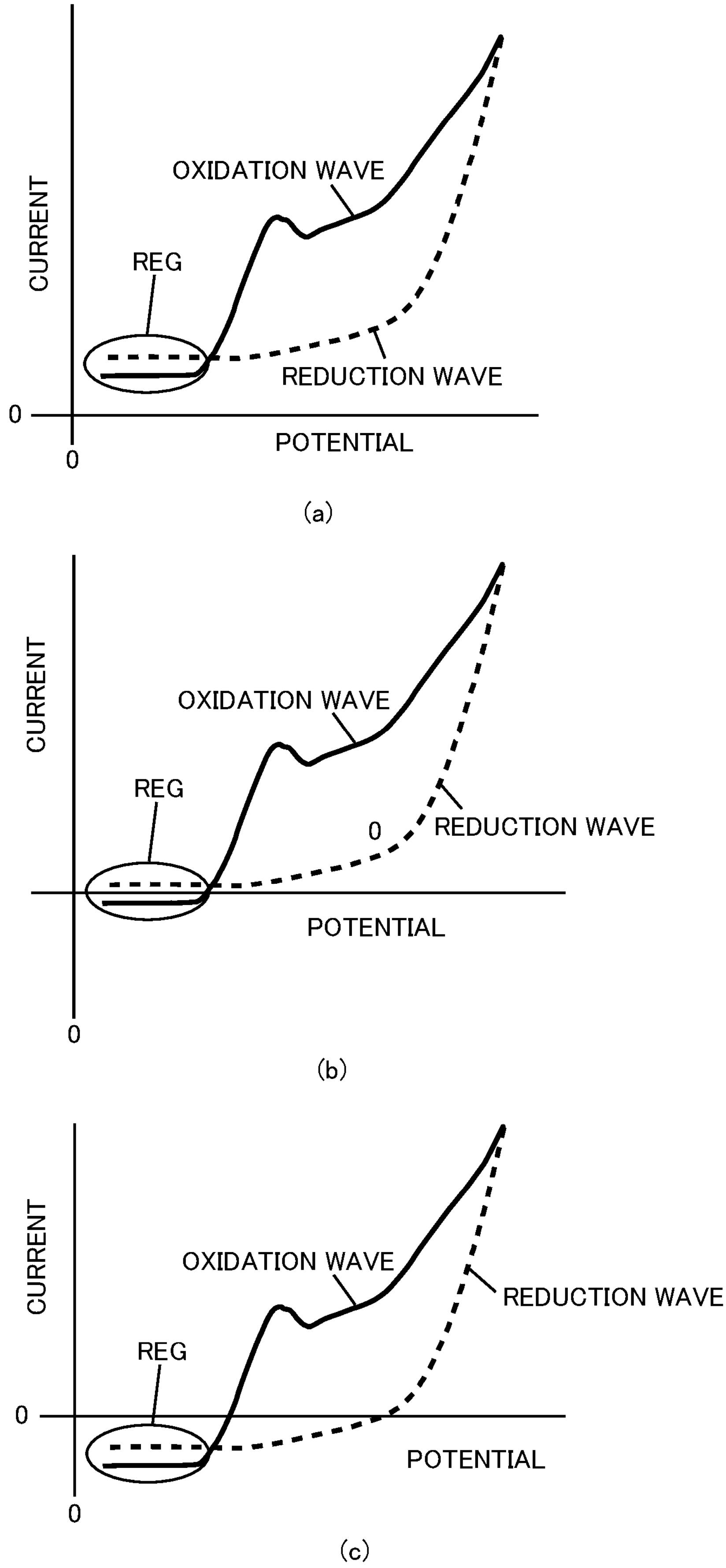
CURRENT
OXIDATION WAVE
REG
REDUCTION WAVE
0
POTENTIAL
0
(a)
CURRENT
OXIDATION WAVE
REG
0
REDUCTION WAVE
POTENTIAL
0
(b)
CURRENT
OXIDATION WAVE
REDUCTION WAVE
REG
0
POTENTIAL
0
(c)

FIG. 12

$CAL_{uni}$

| NAME OF ANALYTE | | | | |
|---|---|---|---|---|
| TYPE OF ANALYTE | | | | |
| CLASS Cls | INTEGRAL VALUE $ITG_{_Low}$ | INTEGRAL VALUE $ITG_{_Middle}$ | INTEGRAL VALUE $ITG_{_High}$ | SUM OF INTEGRAL VALUES $ITG_{ST}$ |
| $Cls_1$ | $ITG_{1_Low}$ | $ITG_{1_Middle}$ | $ITG_{1_High}$ | $ITG_{1_Low}+ITG_{1_Middle}+ITG_{1_High}$ |
| $Cls_2$ | $ITG_{2_Low}$ | $ITG_{2_Middle}$ | $ITG_{2_High}$ | $ITG_{2_Low}+ITG_{2_Middle}+ITG_{2_High}$ |
| $Cls_3$ | $ITG_{3_Low}$ | $ITG_{3_Middle}$ | $ITG_{3_High}$ | $ITG_{3_Low}+ITG_{3_Middle}+ITG_{3_High}$ |
| $Cls_4$ | $ITG_{4_Low}$ | $ITG_{4_Middle}$ | $ITG_{4_High}$ | $ITG_{4_Low}+ITG_{4_Middle}+ITG_{4_High}$ |
| ⋮ | ⋮ | ⋮ | ⋮ | ⋮ |
| $Cls_{n-2}$ | $ITG_{n-2_Low}$ | $ITG_{n-2_Middle}$ | $ITG_{n-2_High}$ | $ITG_{n-2_Low}+ITG_{n-2_Middle}+ITG_{n-2_High}$ |
| $Cls_{n-1}$ | $ITG_{n-1_Low}$ | $ITG_{n-1_Middle}$ | $ITG_{n-1_High}$ | $ITG_{n-1_Low}+ITG_{n-1_Middle}+ITG_{n-1_High}$ |
| $Cls_n$ | $ITG_{n_Low}$ | $ITG_{n_Middle}$ | $ITG_{n_High}$ | $ITG_{n_Low}+ITG_{n_Middle}+ITG_{n_High}$ |

(a)

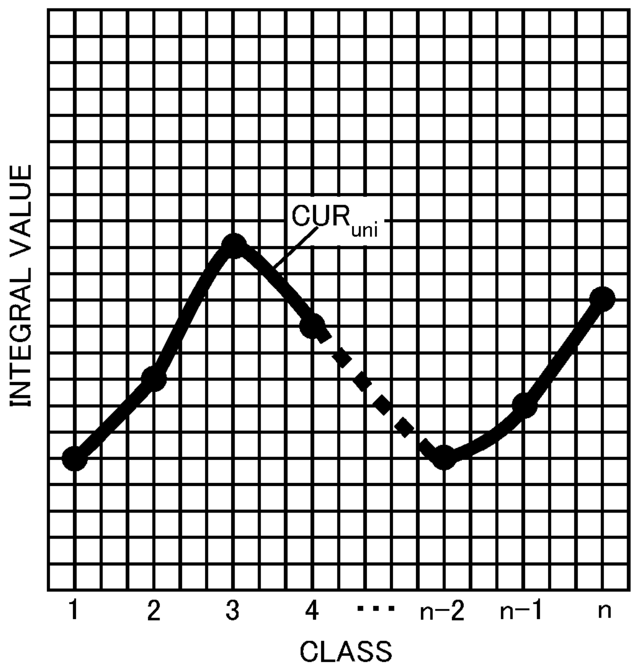


(b)

FIG. 13

(a)

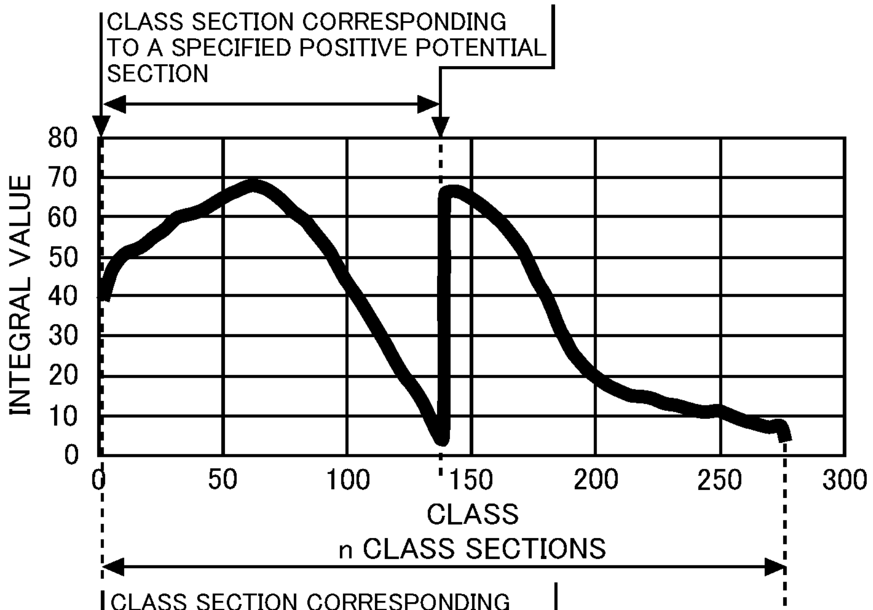
CLASS SECTION CORRESPONDING TO A SPECIFIED POSITIVE POTENTIAL SECTION
INTEGRAL VALUE
0
10
20
30
40
50
60
70
80
0
50
100
150
200
250
300
CLASS
n CLASS SECTIONS

(b)

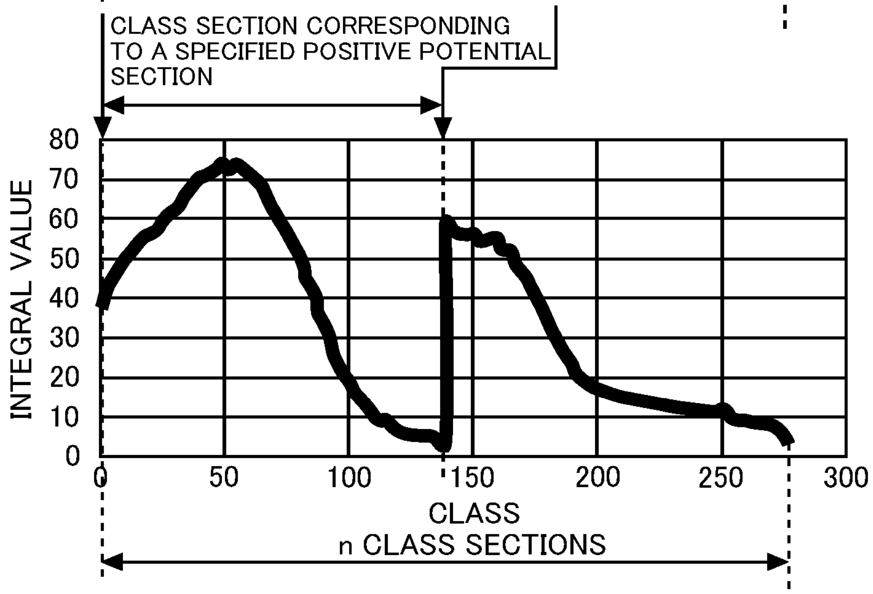
CLASS SECTION CORRESPONDING TO A SPECIFIED POSITIVE POTENTIAL SECTION
INTEGRAL VALUE
0
10
20
30
40
50
60
70
80
0
50
100
150
200
250
300
CLASS
n CLASS SECTIONS

(c)

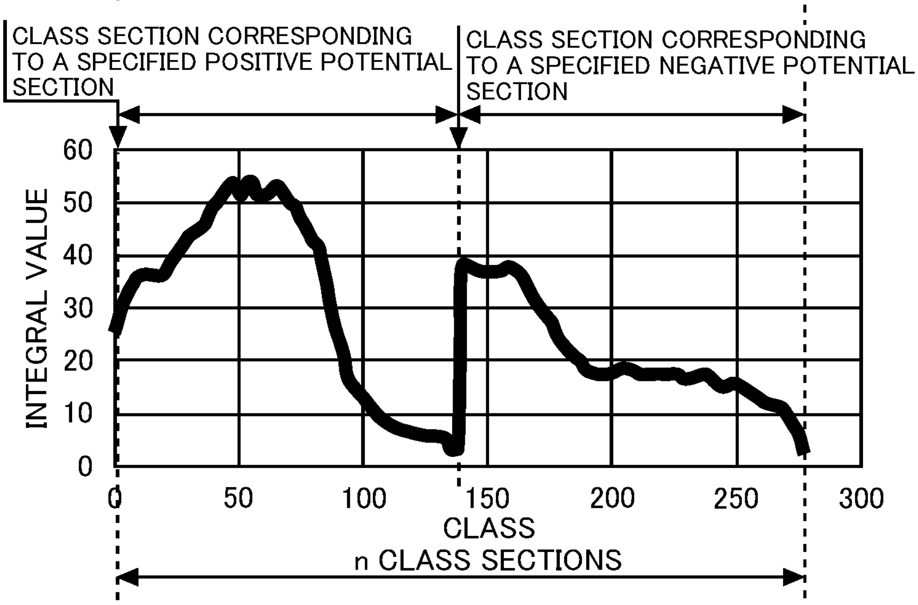
CLASS SECTION CORRESPONDING TO A SPECIFIED POSITIVE POTENTIAL SECTION
CLASS SECTION CORRESPONDING TO A SPECIFIED NEGATIVE POTENTIAL SECTION
INTEGRAL VALUE
0
10
20
30
40
50
60
0
50
100
150
200
250
300
CLASS
n CLASS SECTIONS

FIG. 14

TH1

| | ASTRINGENCY | Body INDEX(+) |
|---|---|---|
| 1 | $a_1$ | $b(+)_1$ |
| 2 | $a_2$ | $b(+)_2$ |
| 3 | $a_3$ | $b(+)_3$ |
| 4 | $a_4$ | $b(+)_4$ |
| 5 | $a_5$ | $b(+)_5$ |
| 6 | $a_6$ | $b(+)_6$ |
| 7 | $a_7$ | $b(+)_7$ |
| 8 | $a_8$ | $b(+)_8$ |
| 9 | $a_9$ | $b(+)_9$ |
| 10 | $a_{10}$ | $b(+)_{10}$ |

(a)

TH2

| | AFTERTASTE | Body INDEX(all) |
|---|---|---|
| 1 | $c_1$ | $b(all)_1$ |
| 2 | $c_2$ | $b(all)_2$ |
| 3 | $c_3$ | $b(all)_3$ |
| 4 | $c_4$ | $b(all)_4$ |
| 5 | $c_5$ | $b(all)_5$ |
| 6 | $c_6$ | $b(all)_6$ |
| 7 | $c_7$ | $b(all)_7$ |
| 8 | $c_8$ | $b(all)_8$ |
| 9 | $c_9$ | $b(all)_9$ |
| 10 | $c_{10}$ | $b(all)_{10}$ |

(b)

TH3

| | SWEETNESS | H(+)_sum |
|---|---|---|
| 1 | $d_1$ | $h(+)_{1_sum}$ |
| 2 | $d_2$ | $h(+)_{2_sum}$ |
| 3 | $d_3$ | $h(+)_{3_sum}$ |
| 4 | $d_4$ | $h(+)_{4_sum}$ |
| 5 | $d_5$ | $h(+)_{5_sum}$ |
| 6 | $d_6$ | $h(+)_{6_sum}$ |
| 7 | $d_7$ | $h(+)_{7_sum}$ |
| 8 | $d_8$ | $h(+)_{8_sum}$ |
| 9 | $d_9$ | $h(+)_{9_sum}$ |
| 10 | $d_{10}$ | $h(+)_{10_sum}$ |

(c)

TH4

| | AROMA | H(−)_sum |
|---|---|---|
| 1 | $e_1$ | $h(-)_{1_sum}$ |
| 2 | $e_2$ | $h(-)_{2_sum}$ |
| 3 | $e_3$ | $h(-)_{3_sum}$ |
| 4 | $e_4$ | $h(-)_{4_sum}$ |
| 5 | $e_5$ | $h(-)_{5_sum}$ |
| 6 | $e_6$ | $h(-)_{6_sum}$ |
| 7 | $e_7$ | $h(-)_{7_sum}$ |
| 8 | $e_8$ | $h(-)_{8_sum}$ |
| 9 | $e_9$ | $h(-)_{9_sum}$ |
| 10 | $e_{10}$ | $h(-)_{10_sum}$ |

(d)

TH5

| | BITTERNESS | ASTRINGENCY | SWEETNESS |
|---|---|---|---|
| 1 | $f_1$ | $a_1$ | $d_1$ |
| 2 | $f_2$ | $a_2$ | $d_2$ |
| 3 | $f_3$ | $a_3$ | $d_3$ |
| 4 | $f_4$ | $a_4$ | $d_4$ |
| 5 | $f_5$ | $a_5$ | $d_5$ |
| 6 | $f_6$ | $a_6$ | $d_6$ |
| 7 | $f_7$ | $a_7$ | $d_7$ |
| 8 | $f_8$ | $a_8$ | $d_8$ |
| 9 | $f_9$ | $a_9$ | $d_9$ |
| 10 | $f_{10}$ | $a_{10}$ | $d_{10}$ |

(e)

FIG. 15

TBL1

| COEFFICIENT | TEACHER DATA |
|---|---|
| $k_1$ | TH1 |
| $k_2$ | TH2 |
| $k_3,k_4$ | TH3 |
| $k_5,k_6$ | TH4 |
| $k_7,k_8$ | TH5 |

# FIG. 16

TH1_up1

| | ATG | Body INDEX(+) |
|---|---|---|
| 1 | $a_1$ | $b(+)_1$ |
| 2 | $a_2$ | $b(+)_2$ |
| 3 | $a_3$ | $b(+)_3$ |
| 4 | $a_4$ | $b(+)_4$ |
| 5 | $a_5$ | $b(+)_5$ |
| 6 | $a_6$ | $b(+)_6$ |
| 7 | $a_7$ | $b(+)_7$ |
| 8 | $a_8$ | $b(+)_8$ |
| 9 | $a_9$ | $b(+)_9$ |
| 10 | $a_{10}$ | $b(+)_{10}$ |
| 11 | $a_{1_add}$ | $b(+)_{1_add}$ |
| 12 | $a_{2_add}$ | $b(+)_{2_add}$ |
| 13 | $a_{3_add}$ | $b(+)_{3_add}$ |
| ⋮ | ⋮ | ⋮ |
| 1z | $a_{v_add}$ | $b(+)_{v_add}$ |

ATG=ASTRINGENCY

(a)

TH2_up1

| | AFT | Body INDEX(all) |
|---|---|---|
| 1 | $c_1$ | $b(all)_1$ |
| 2 | $c_2$ | $b(all)_2$ |
| 3 | $c_3$ | $b(all)_3$ |
| 4 | $c_4$ | $b(all)_4$ |
| 5 | $c_5$ | $b(all)_5$ |
| 6 | $c_6$ | $b(all)_6$ |
| 7 | $c_7$ | $b(all)_7$ |
| 8 | $c_8$ | $b(all)_8$ |
| 9 | $c_9$ | $b(all)_9$ |
| 10 | $c_{10}$ | $b(all)_{10}$ |
| 11 | $c_{1_add}$ | $b(all)_{1_add}$ |
| 12 | $c_{2_add}$ | $b(all)_{2_add}$ |
| 13 | $c_{3_add}$ | $b(all)_{3_add}$ |
| ⋮ | ⋮ | ⋮ |
| 1z | $c_{v_add}$ | $b(all)_{v_add}$ |

AFT=AFTERTASTE

(b)

TH3_up1

| | SWEETNESS | H(+)_sum |
|---|---|---|
| 1 | $d_1$ | $h(+)_{1_sum}$ |
| 2 | $d_2$ | $h(+)_{2_sum}$ |
| 3 | $d_3$ | $h(+)_{3_sum}$ |
| 4 | $d_4$ | $h(+)_{4_sum}$ |
| 5 | $d_5$ | $h(+)_{5_sum}$ |
| 6 | $d_6$ | $h(+)_{6_sum}$ |
| 7 | $d_7$ | $h(+)_{7_sum}$ |
| 8 | $d_8$ | $h(+)_{8_sum}$ |
| 9 | $d_9$ | $h(+)_{9_sum}$ |
| 10 | $d_{10}$ | $h(+)_{10_sum}$ |
| 11 | $d_{1_add}$ | $h(+)_{1_sum_add}$ |
| 12 | $d_{2_add}$ | $h(+)_{2_sum_add}$ |
| 13 | $d_{3_add}$ | $h(+)_{3_sum_add}$ |
| ⋮ | ⋮ | ⋮ |
| 1z | $d_{v_add}$ | $h(+)_{v_sum_add}$ |

(c)

TH4_up1

| | AROMA | H(−)_sum |
|---|---|---|
| 1 | $e_1$ | $h(-)_{1_sum}$ |
| 2 | $e_2$ | $h(-)_{2_sum}$ |
| 3 | $e_3$ | $h(-)_{3_sum}$ |
| 4 | $e_4$ | $h(-)_{4_sum}$ |
| 5 | $e_5$ | $h(-)_{5_sum}$ |
| 6 | $e_6$ | $h(-)_{6_sum}$ |
| 7 | $e_7$ | $h(-)_{7_sum}$ |
| 8 | $e_8$ | $h(-)_{8_sum}$ |
| 9 | $e_9$ | $h(-)_{9_sum}$ |
| 10 | $e_{10}$ | $h(-)_{10_sum}$ |
| 11 | $e_{1_add}$ | $h(-)_{1_sum_add}$ |
| 12 | $e_{2_add}$ | $h(-)_{2_sum_add}$ |
| 13 | $e_{3_add}$ | $h(-)_{3_sum_add}$ |
| ⋮ | ⋮ | ⋮ |
| 1z | $e_{v_add}$ | $h(-)_{v_sum_add}$ |

(d)

TH5_up1

| | BITTERNESS | ASTRINGENCY | SWEETNESS |
|---|---|---|---|
| 1 | $f_1$ | $a_1$ | $d_1$ |
| 2 | $f_2$ | $a_2$ | $d_2$ |
| 3 | $f_3$ | $a_3$ | $d_3$ |
| 4 | $f_4$ | $a_4$ | $d_4$ |
| 5 | $f_5$ | $a_5$ | $d_5$ |
| 6 | $f_6$ | $a_6$ | $d_6$ |
| 7 | $f_7$ | $a_7$ | $d_7$ |
| 8 | $f_8$ | $a_8$ | $d_8$ |
| 9 | $f_9$ | $a_9$ | $d_9$ |
| 10 | $f_{10}$ | $a_{10}$ | $d_{10}$ |
| 11 | $f_{1_add}$ | $a_{1_add}$ | $d_{1_add}$ |
| 12 | $f_{2_add}$ | $a_{2_add}$ | $d_{2_add}$ |
| 13 | $f_{3_add}$ | $a_{3_add}$ | $d_{3_add}$ |
| ⋮ | ⋮ | ⋮ | ⋮ |
| 1z | $f_{v_add}$ | $a_{v_add}$ | $d_{v_add}$ |

(e)

FIG. 17

TBL1_up1

| COEFFICIENT | TEACHER DATA |
| --- | --- |
| $k_{1_up1}$ | TH1_up1 |
| $k_{2_up1}$ | TH2_up1 |
| $k_{3_up1}$,$k_{4_up1}$ | TH3_up1 |
| $k_{5_up1}$,$k_{6_up1}$ | TH4_up1 |
| $k_{7_up1}$,$k_{8_up1}$ | TH5_up1 |

FIG. 18

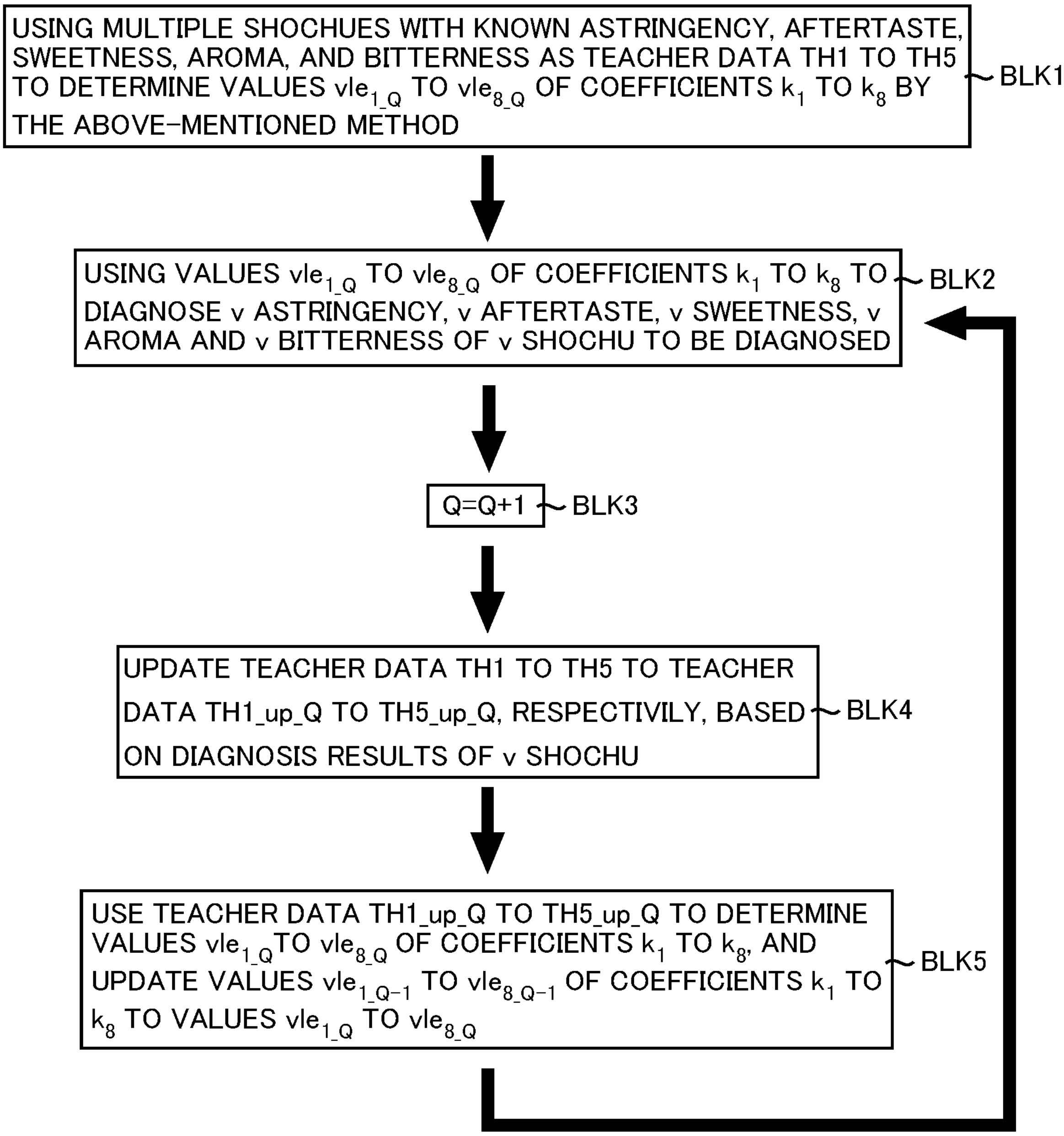
USING MULTIPLE SHOCHUES WITH KNOWN ASTRINGENCY, AFTERTASTE, SWEETNESS, AROMA, AND BITTERNESS AS TEACHER DATA TH1 TO TH5 TO DETERMINE VALUES $vle_{1_Q}$ TO $vle_{8_Q}$ OF COEFFICIENTS $k_1$ TO $k_8$ BY THE ABOVE-MENTIONED METHOD
BLK1
USING VALUES $vle_{1_Q}$ TO $vle_{8_Q}$ OF COEFFICIENTS $k_1$ TO $k_8$ TO DIAGNOSE v ASTRINGENCY, v AFTERTASTE, v SWEETNESS, v AROMA AND v BITTERNESS OF v SHOCHU TO BE DIAGNOSED
BLK2
Q=Q+1
BLK3
UPDATE TEACHER DATA TH1 TO TH5 TO TEACHER DATA TH1_up_Q TO TH5_up_Q, RESPECTIVILY, BASED ON DIAGNOSIS RESULTS OF v SHOCHU
BLK4
USE TEACHER DATA TH1_up_Q TO TH5_up_Q TO DETERMINE VALUES $vle_{1_Q}$TO $vle_{8_Q}$ OF COEFFICIENTS $k_1$ TO $k_8$, AND UPDATE VALUES $vle_{1_Q-1}$ TO $vle_{8_Q-1}$ OF COEFFICIENTS $k_1$ TO $k_8$ TO VALUES $vle_{1_Q}$ TO $vle_{8_Q}$
BLK5

FIG. 19

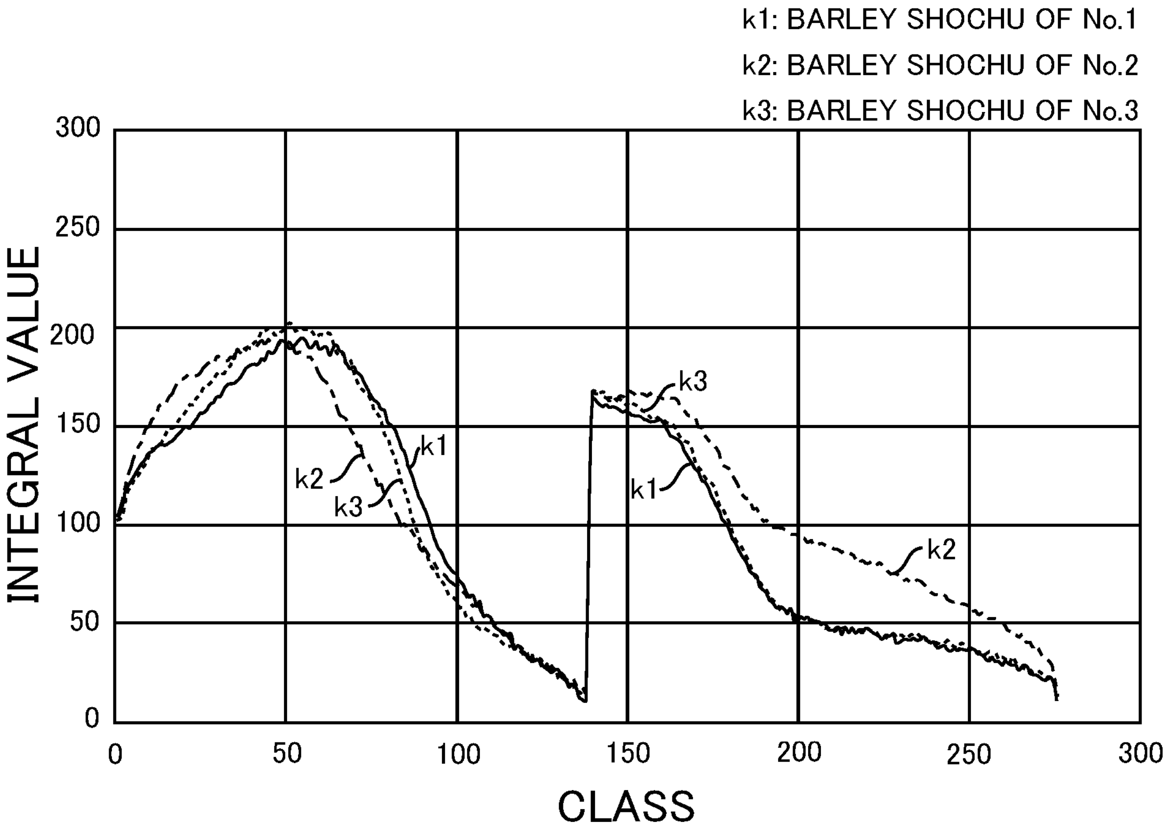
k1: BARLEY SHOCHU OF No.1
k2: BARLEY SHOCHU OF No.2
k3: BARLEY SHOCHU OF No.3
INTEGRAL VALUE
CLASS
0
50
100
150
200
250
300
k1
k2
k3

FIG. 20

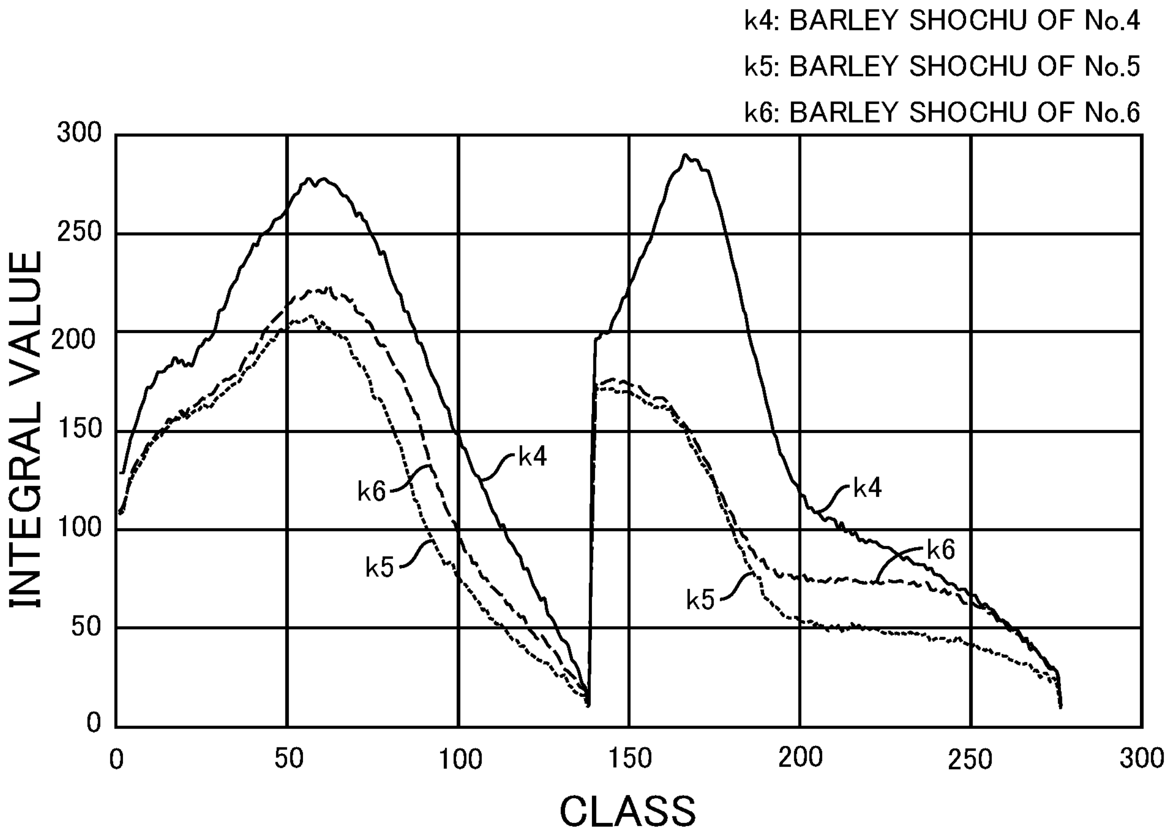
k4: BARLEY SHOCHU OF No.4
k5: BARLEY SHOCHU OF No.5
k6: BARLEY SHOCHU OF No.6
INTEGRAL VALUE
CLASS
0
50
100
150
200
250
300
k4
k5
k6

FIG. 21

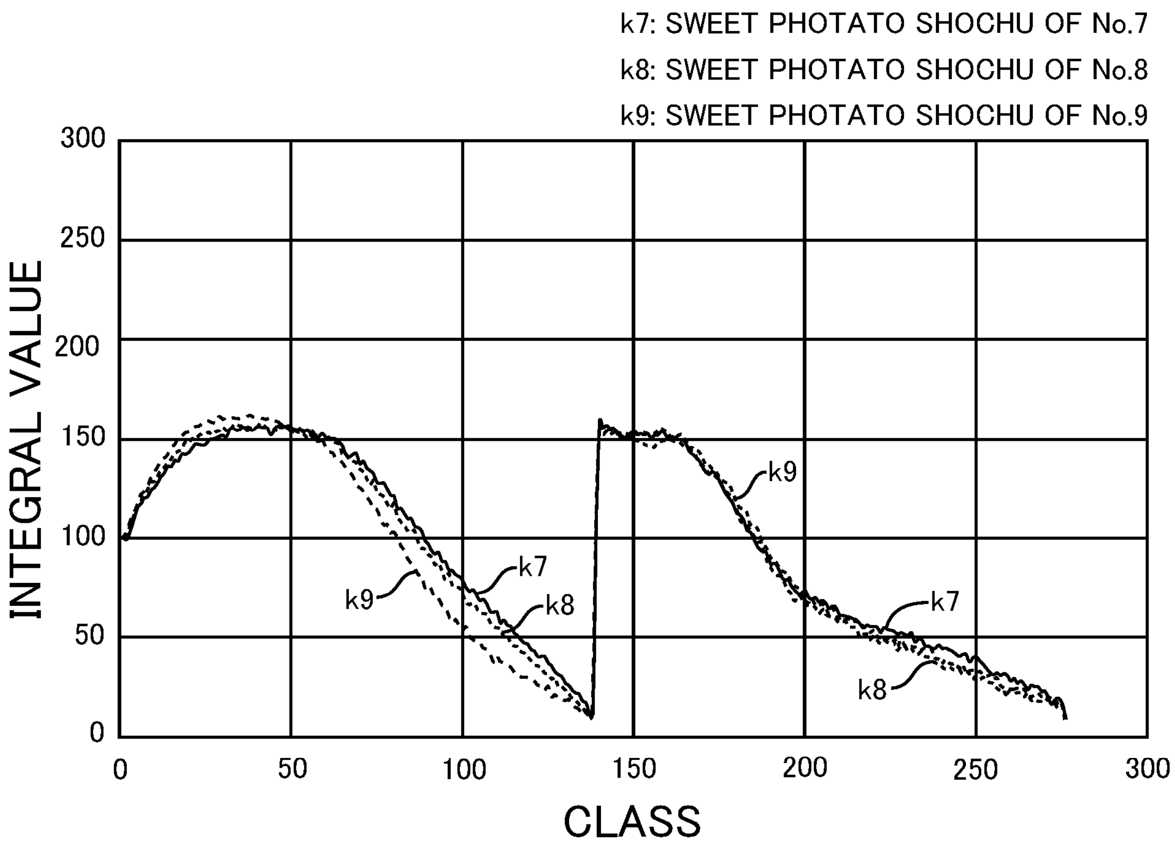
k7: SWEET PHOTATO SHOCHU OF No.7
k8: SWEET PHOTATO SHOCHU OF No.8
k9: SWEET PHOTATO SHOCHU OF No.9
INTEGRAL VALUE
300
250
200
150
100
50
0
CLASS
0
50
100
150
200
250
300
k7
k8
k9

FIG. 22

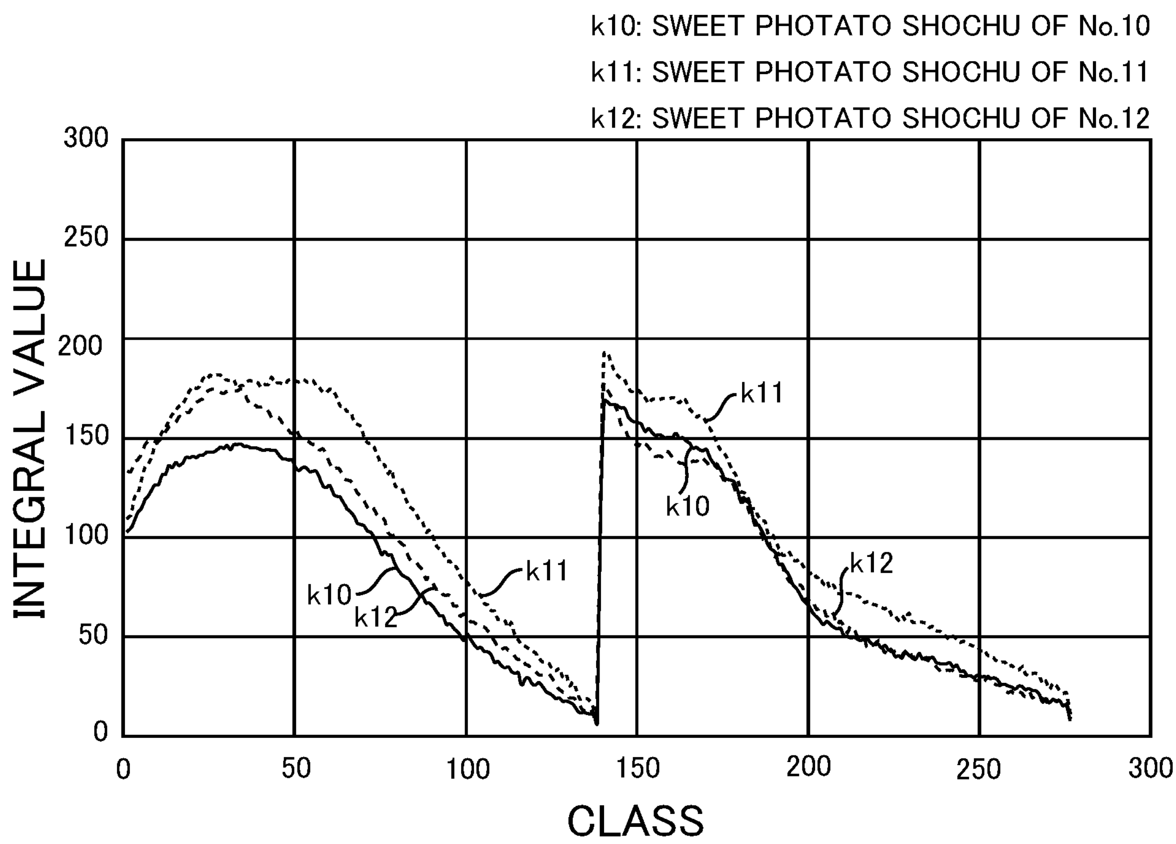
k10: SWEET PHOTATO SHOCHU OF No.10
k11: SWEET PHOTATO SHOCHU OF No.11
k12: SWEET PHOTATO SHOCHU OF No.12
INTEGRAL VALUE
300
250
200
150
100
50
0
CLASS
0
50
100
150
200
250
300
k10
k11
k12

FIG. 23

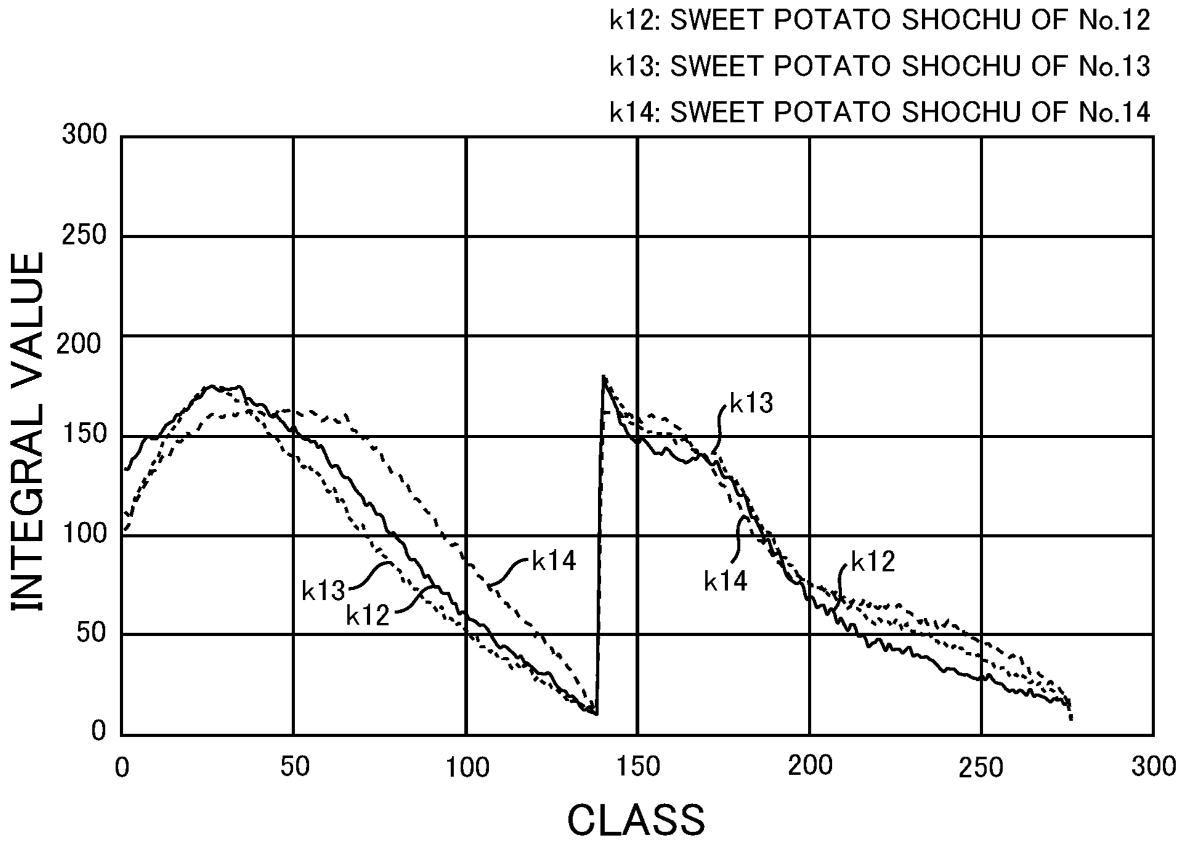
k12: SWEET POTATO SHOCHU OF No.12
k13: SWEET POTATO SHOCHU OF No.13
k14: SWEET POTATO SHOCHU OF No.14
300
250
200
150
100
50
0
INTEGRAL VALUE
0
50
100
150
200
250
300
CLASS
k13
k14
k12
k13
k12
k14

FIG. 24

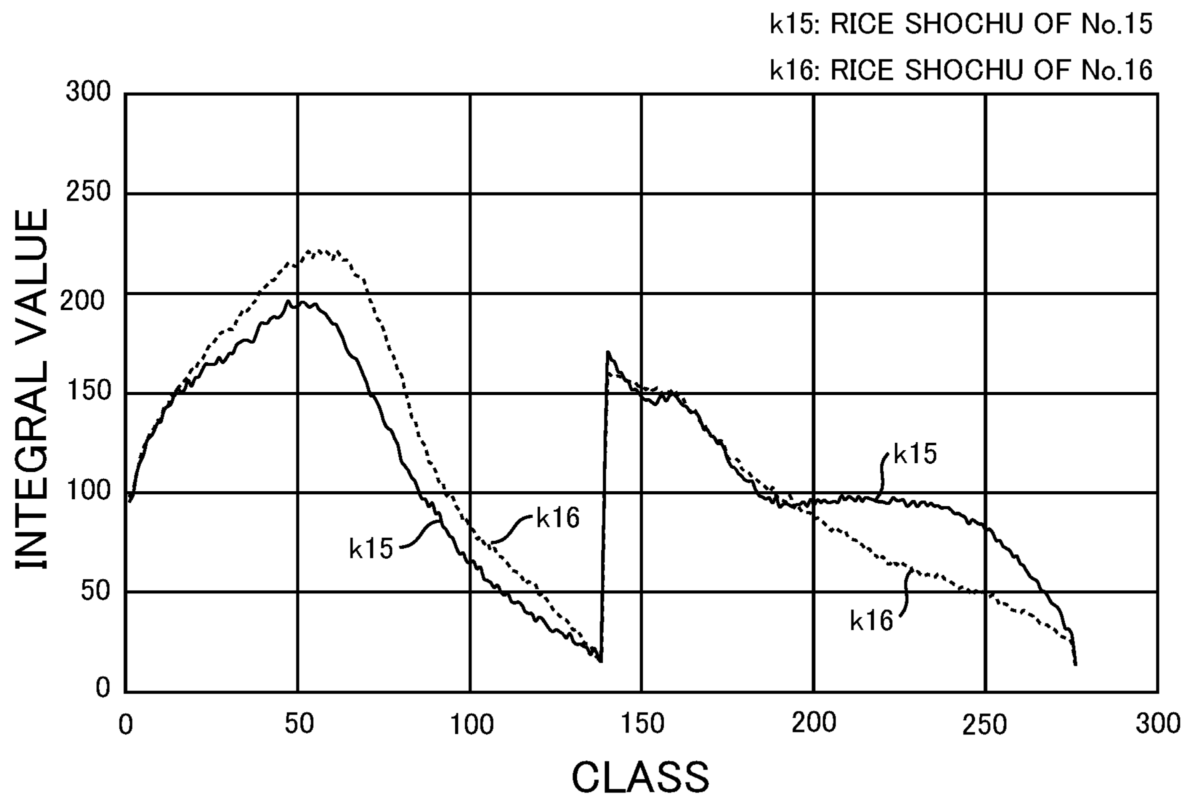
k15: RICE SHOCHU OF No.15
k16: RICE SHOCHU OF No.16
300
250
200
150
100
50
0
INTEGRAL VALUE
0
50
100
150
200
250
300
CLASS
k15
k16
k15
k16

FIG. 25

k17: SHOCHU MADE WITH SAKE LEES OF No.17
k18: BARLEY SHOCHU OF No.18, ALL OF WHICH WAS STORED FOR 3 YEARS, ALL OF WHICH WAS STORED IN BARRELS, AND SOME OF WHICH WAS STORED IN CHERRY WOOD BARRELS
k19: SAMPLE NO. 19, ALL OF WHICH WAS STORED FOR 15 YEARS, ALL OF WHICH WAS MADE FROM KOJI BARLEY, ALL OF WHICH WAS STORED IN BARRELS, AND ALL OF WHICH WAS HEATED
k20: RICE SHOCHU OF SAMPLE No. 20, ALL OF WHICH WAS AGED FOR THREE YEARS AND ALL OF WHICH WAS STORED IN BARRELS

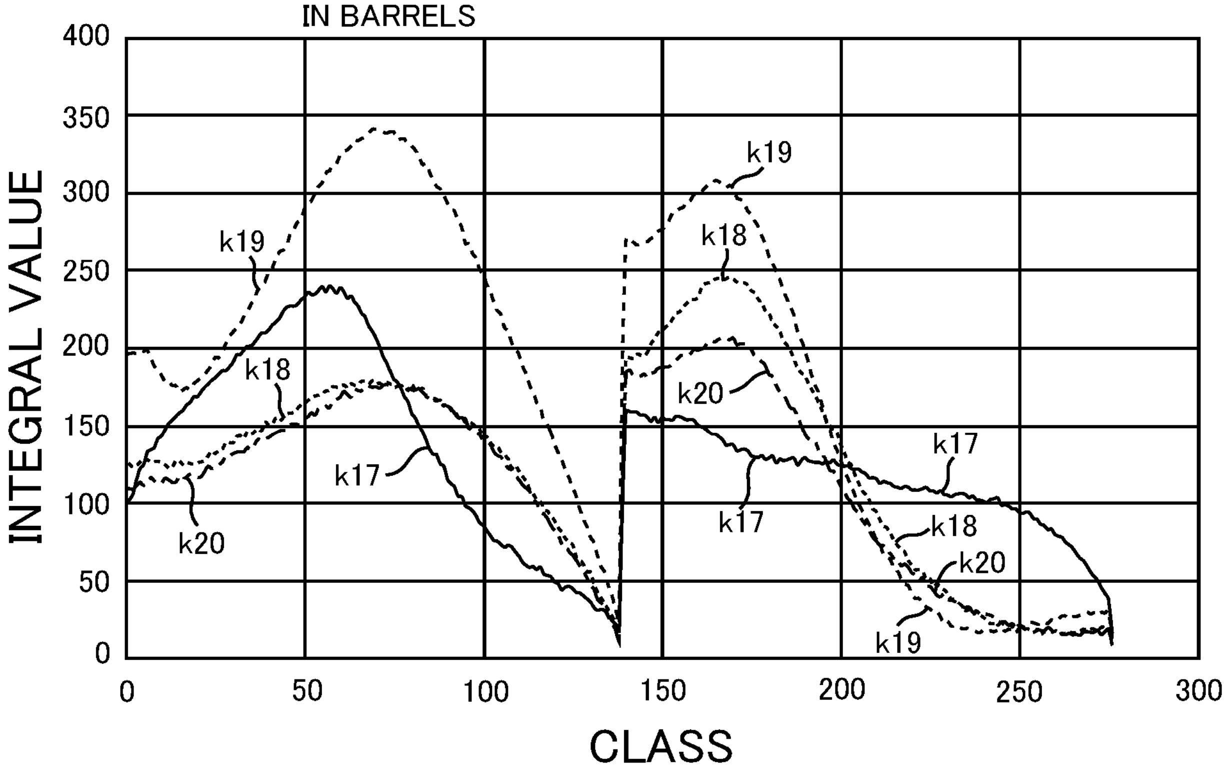

FIG. 26

| | k1 | k2 | k3 | k4 | k5 | k6 | k7 | k8 | k9 | k10 |
|---|---|---|---|---|---|---|---|---|---|---|
| k1 | | $\sigma_{DF_k1,k2}$ =26.20% | $\sigma_{DF_k1,k3}$ =8.18% | $\sigma_{DF_k1,k4}$ =20.50% | $\sigma_{DF_k1,k5}$ =6.12% | $\sigma_{DF_k1,k6}$ =17.36% | $\sigma_{DF_k1,k7}$ =16.52% | $\sigma_{DF_k1,k8}$ =16.30% | $\sigma_{DF_k1,k9}$ =20.66% | $\sigma_{DF_k1,k10}$ =23.71% |
| k2 | $\sigma_{DF_k2,k1}$ =26.20% | | $\sigma_{DF_k2,k3}$ =23.99% | $\sigma_{DF_k2,k4}$ =24.69% | $\sigma_{DF_k2,k5}$ =24.20% | $\sigma_{DF_k2,k6}$ =20.78% | $\sigma_{DF_k2,k7}$ =20.46% | $\sigma_{DF_k2,k8}$ =23.71% | $\sigma_{DF_k2,k9}$ =17.78% | $\sigma_{DF_k2,k10}$ =20.14% |
| k3 | $\sigma_{DF_k3,k1}$ =8.18% | $\sigma_{DF_k3,k2}$ =23.99% | | $\sigma_{DF_k3,k4}$ =103.04% | $\sigma_{DF_k3,k5}$ =7.15% | $\sigma_{DF_k3,k6}$ =19.03% | $\sigma_{DF_k3,k7}$ =18.09% | $\sigma_{DF_k3,k8}$ =17.70% | $\sigma_{DF_k3,k9}$ =18.72% | $\sigma_{DF_k3,k10}$ =21.89% |
| k4 | $\sigma_{DF_k4,k1}$ =20.50% | $\sigma_{DF_k4,k2}$ =24.69% | $\sigma_{DF_k4,k3}$ =103.04% | | $\sigma_{DF_k4,k5}$ =20.85% | $\sigma_{DF_k4,k6}$ =19.17% | $\sigma_{DF_k4,k7}$ =12.58% | $\sigma_{DF_k4,k8}$ =16.20% | $\sigma_{DF_k4,k9}$ =21.21% | $\sigma_{DF_k4,k10}$ =23.40% |
| k5 | $\sigma_{DF_k5,k1}$ =6.12% | $\sigma_{DF_k5,k2}$ =24.20% | $\sigma_{DF_k5,k3}$ =7.15% | $\sigma_{DF_k5,k4}$ =20.85% | | $\sigma_{DF_k5,k6}$ =15.11% | $\sigma_{DF_k5,k7}$ =16.66% | $\sigma_{DF_k5,k8}$ =17.66% | $\sigma_{DF_k5,k9}$ =20.77% | $\sigma_{DF_k5,k10}$ =23.69% |
| k6 | $\sigma_{DF_k6,k1}$ =17.36% | $\sigma_{DF_k6,k2}$ =20.78% | $\sigma_{DF_k6,k3}$ =19.03% | $\sigma_{DF_k6,k4}$ =19.17% | $\sigma_{DF_k6,k5}$ =15.11% | | $\sigma_{DF_k6,k7}$ =17.53% | $\sigma_{DF_k6,k8}$ =22.89% | $\sigma_{DF_k6,k9}$ =25.72% | $\sigma_{DF_k6,k10}$ =29.23% |
| k7 | $\sigma_{DF_k7,k1}$ =16.52% | $\sigma_{DF_k7,k2}$ =20.46% | $\sigma_{DF_k7,k3}$ =18.09% | $\sigma_{DF_k7,k4}$ =12.58% | $\sigma_{DF_k7,k5}$ =16.66% | $\sigma_{DF_k7,k6}$ =17.53% | | $\sigma_{DF_k7,k8}$ =8.94% | $\sigma_{DF_k7,k9}$ =15.89% | $\sigma_{DF_k7,k10}$ =18.88% |
| k8 | $\sigma_{DF_k8,k1}$ =16.30% | $\sigma_{DF_k8,k2}$ =23.71% | $\sigma_{DF_k8,k3}$ =17.70% | $\sigma_{DF_k8,k4}$ =16.20% | $\sigma_{DF_k8,k5}$ =17.66% | $\sigma_{DF_k8,k6}$ =22.89% | $\sigma_{DF_k8,k7}$ =8.94% | | $\sigma_{DF_k8,k9}$ =12.82% | $\sigma_{DF_k8,k10}$ =15.93% |
| k9 | $\sigma_{DF_k9,k1}$ =20.66% | $\sigma_{DF_k9,k2}$ =17.78% | $\sigma_{DF_k9,k3}$ =18.72% | $\sigma_{DF_k9,k4}$ =21.21% | $\sigma_{DF_k9,k5}$ =20.77% | $\sigma_{DF_k9,k6}$ =25.72% | $\sigma_{DF_k9,k7}$ =15.89% | $\sigma_{DF_k9,k8}$ =12.82% | | $\sigma_{DF_k9,k10}$ =8.28% |
| k10 | $\sigma_{DF_k10,k1}$ =23.71% | $\sigma_{DF_k10,k2}$ =20.14% | $\sigma_{DF_k10,k3}$ =21.89% | $\sigma_{DF_k10,k4}$ =23.40% | $\sigma_{DF_k10,k5}$ =23.69% | $\sigma_{DF_k10,k6}$ =29.23% | $\sigma_{DF_k10,k7}$ =18.88% | $\sigma_{DF_k10,k8}$ =15.93% | $\sigma_{DF_k10,k9}$ =8.28% | |

FIG. 27

| | k11 | k12 | k13 | k14 | k15 | k16 | k17 | k18 | k19 | k20 |
|---|---|---|---|---|---|---|---|---|---|---|
| k1 | $\sigma_{DF_k1,k11}$ =16.66% | $\sigma_{DF_k1,k12}$ =20.65% | $\sigma_{DF_k1,k13}$ =27.26% | $\sigma_{DF_k1,k14}$ =18.74% | $\sigma_{DF_k1,k15}$ =33.97% | $\sigma_{DF_k1,k16}$ =15.73% | $\sigma_{DF_k1,k17}$ =33.45% | $\sigma_{DF_k1,k18}$ =46.19% | $\sigma_{DF_k1,k19}$ =52.98% | $\sigma_{DF_k1,k20}$ =42.51% |
| k2 | $\sigma_{DF_k2,k11}$ =15.31% | $\sigma_{DF_k2,k12}$ =24.36% | $\sigma_{DF_k2,k13}$ =15.42% | $\sigma_{DF_k2,k14}$ =19.14% | $\sigma_{DF_k2,k15}$ =13.91% | $\sigma_{DF_k2,k16}$ =18.26% | $\sigma_{DF_k2,k17}$ =18.03% | $\sigma_{DF_k2,k18}$ =54.10% | $\sigma_{DF_k2,k19}$ =68.46% | $\sigma_{DF_k2,k20}$ =51.89% |
| k3 | $\sigma_{DF_k3,k11}$ =16.40% | $\sigma_{DF_k3,k12}$ =19.90% | $\sigma_{DF_k2,k13}$ =24.77% | $\sigma_{DF_k3,k14}$ =20.13% | $\sigma_{DF_k3,k15}$ =31.93% | $\sigma_{DF_k3,k16}$ =16.08% | $\sigma_{DF_k3,k17}$ =32.39% | $\sigma_{DF_k3,k18}$ =48.95% | $\sigma_{DF_k3,k19}$ =55.67% | $\sigma_{DF_k3,k20}$ =45.76% |
| k4 | $\sigma_{DF_k4,k11}$ =16.78% | $\sigma_{DF_k4,k12}$ =24.03% | $\sigma_{DF_k4,k13}$ =25.32% | $\sigma_{DF_k4,k14}$ =13.61% | $\sigma_{DF_k4,k15}$ =32.44% | $\sigma_{DF_k4,k16}$ =16.89% | $\sigma_{DF_k4,k17}$ =31.42% | $\sigma_{DF_k4,k18}$ =35.53% | $\sigma_{DF_k4,k19}$ =51.56% | $\sigma_{DF_k4,k20}$ =31.64% |
| k5 | $\sigma_{DF_k5,k11}$ =16.07% | $\sigma_{DF_k5,k12}$ =21.84% | $\sigma_{DF_k5,k13}$ =26.33% | $\sigma_{DF_k5,k14}$ =17.50% | $\sigma_{DF_k4,k15}$ =31.51% | $\sigma_{DF_k5,k16}$ =14.87% | $\sigma_{DF_k5,k17}$ =31.67% | $\sigma_{DF_k5,k18}$ =48.60% | $\sigma_{DF_k5,k19}$ =56.11% | $\sigma_{DF_k5,k20}$ =44.96% |
| k6 | $\sigma_{DF_k6,k11}$ =18.41% | $\sigma_{DF_k6,k12}$ =28.94% | $\sigma_{DF_k6,k13}$ =28.54% | $\sigma_{DF_k6,k14}$ =13.28% | $\sigma_{DF_k6,k15}$ =23.36% | $\sigma_{DF_k6,k16}$ =11.95% | $\sigma_{DF_k6,k17}$ =22.04% | $\sigma_{DF_k6,k18}$ =50.68% | $\sigma_{DF_k6,k19}$ =62.01% | $\sigma_{DF_k6,k20}$ =46.45% |
| k7 | $\sigma_{DF_k7,k11}$ =8.02% | $\sigma_{DF_k7,k12}$ =16.31% | $\sigma_{DF_k7,k13}$ =19.76% | $\sigma_{DF_k7,k14}$ =7.75% | $\sigma_{DF_k7,k15}$ =29.58% | $\sigma_{DF_k7,k16}$ =12.72% | $\sigma_{DF_k6,k17}$ =29.01% | $\sigma_{DF_k7,k18}$ =39.38% | $\sigma_{DF_k7,k19}$ =53.91% | $\sigma_{DF_k7,k20}$ =36.01% |
| k8 | $\sigma_{DF_k8,k11}$ =10.07% | $\sigma_{DF_k8,k12}$ =10.90% | $\sigma_{DF_k8,k13}$ =19.51% | $\sigma_{DF_k8,k14}$ =15.03% | $\sigma_{DF_k8,k15}$ =33.85% | $\sigma_{DF_k8,k16}$ =17.13% | $\sigma_{DF_k8,k17}$ =33.63% | $\sigma_{DF_k7,k18}$ =36.78% | $\sigma_{DF_k8,k19}$ =50.39% | $\sigma_{DF_k8,k20}$ =33.93% |
| k9 | $\sigma_{DF_k9,k11}$ =10.59% | $\sigma_{DF_k9,k12}$ =10.56% | $\sigma_{DF_k9,k13}$ =10.95% | $\sigma_{DF_k9,k14}$ =20.02% | $\sigma_{DF_k9,k15}$ =29.27% | $\sigma_{DF_k9,k16}$ =19.71% | $\sigma_{DF_k9,k17}$ =31.35% | $\sigma_{DF_k9,k18}$ =44.24% | $\sigma_{DF_k9,k19}$ =56.91% | $\sigma_{DF_k9,k20}$ =42.50% |
| k10 | $\sigma_{DF_k10,k11}$ =13.75% | $\sigma_{DF_k10,k12}$ =11.84% | $\sigma_{DF_k10,k13}$ =10.36% | $\sigma_{DF_k10,k14}$ =23.06% | $\sigma_{DF_k10,k15}$ =31.65% | $\sigma_{DF_k10,k16}$ =24.36% | $\sigma_{DF_k10,k17}$ =34.39% | $\sigma_{DF_k10,k18}$ =44.56% | $\sigma_{DF_k10,k19}$ =56.36% | $\sigma_{DF_k10,k20}$ =43.18% |

FIG. 28

| | k11 | k12 | k13 | k14 | k15 | k16 | k17 | k18 | k19 | k20 |
|---|---|---|---|---|---|---|---|---|---|---|
| k11 | | $\sigma_{DF_k11,k12}$ =13.80% | $\sigma_{DF_k11,k13}$ =14.03% | $\sigma_{DF_k11,k14}$ =11.06% | $\sigma_{DF_k11,k15}$ =26.26% | $\sigma_{DF_k11,k16}$ =13.04% | $\sigma_{DF_k11,k17}$ =27.61% | $\sigma_{DF_k11,k18}$ =43.88% | $\sigma_{DF_k11,k19}$ =58.04% | $\sigma_{DF_k11,k20}$ =41.02% |
| k12 | $\sigma_{DF_k12,k11}$ =13.80% | | $\sigma_{DF_k12,k13}$ =15.93% | $\sigma_{DF_k12,k14}$ =21.72% | $\sigma_{DF_k12,k15}$ =35.35% | $\sigma_{DF_k12,k16}$ =22.58% | $\sigma_{DF_k12,k17}$ =36.56% | $\sigma_{DF_k12,k18}$ =41.01% | $\sigma_{DF_k12,k19}$ =52.32% | $\sigma_{DF_k12,k20}$ =39.17% |
| k13 | $\sigma_{DF_k13,k11}$ =14.03% | $\sigma_{DF_k13,k12}$ =15.93% | | $\sigma_{DF_k13,k14}$ =21.98% | $\sigma_{DF_k13,k15}$ =26.87% | $\sigma_{DF_k13,k16}$ =23.85% | $\sigma_{DF_k13,k17}$ =30.74% | $\sigma_{DF_k13,k18}$ =49.35% | $\sigma_{DF_k13,k19}$ =62.97% | $\sigma_{DF_k13,k20}$ =47.95% |
| k14 | $\sigma_{DF_k14,k11}$ =11.06% | $\sigma_{DF_k14,k12}$ =21.72% | $\sigma_{DF_k14,k13}$ =21.98% | | $\sigma_{DF_k14,k15}$ =26.21% | $\sigma_{DF_k14,k16}$ =11.51% | $\sigma_{DF_k14,k17}$ =25.52% | $\sigma_{DF_k14,k18}$ =43.20% | $\sigma_{DF_k14,k19}$ =57.86% | $\sigma_{DF_k14,k20}$ =39.52% |
| k15 | $\sigma_{DF_k15,k11}$ =26.26% | $\sigma_{DF_k15,k12}$ =35.35% | $\sigma_{DF_k15,k13}$ =26.87% | $\sigma_{DF_k15,k14}$ =26.21% | | $\sigma_{DF_k15,k16}$ =24.82% | $\sigma_{DF_k15,k17}$ =9.85% | $\sigma_{DF_k15,k18}$ =61.78% | $\sigma_{DF_k15,k19}$ =75.95% | $\sigma_{DF_k15,k20}$ =59.25% |
| k16 | $\sigma_{DF_k16,k11}$ =13.04% | $\sigma_{DF_k16,k12}$ =22.58% | $\sigma_{DF_k16,k13}$ =23.85% | $\sigma_{DF_k16,k14}$ =11.51% | $\sigma_{DF_k16,k15}$ =24.82% | | $\sigma_{DF_k16,k17}$ =22.75% | $\sigma_{DF_k16,k18}$ =46.27% | $\sigma_{DF_k16,k19}$ =58.31% | $\sigma_{DF_k16,k20}$ =42.65% |
| k17 | $\sigma_{DF_k17,k11}$ =27.61% | $\sigma_{DF_k17,k12}$ =36.56% | $\sigma_{DF_k17,k13}$ =30.74% | $\sigma_{DF_k17,k14}$ =25.52% | $\sigma_{DF_k17,k15}$ =9.85% | $\sigma_{DF_k17,k16}$ =22.75% | | $\sigma_{DF_k17,k18}$ =58.70% | $\sigma_{DF_k17,k19}$ =73.47% | $\sigma_{DF_k17,k20}$ =55.75% |
| k18 | $\sigma_{DF_k18,k11}$ =43.88% | $\sigma_{DF_k18,k12}$ =41.01% | $\sigma_{DF_k18,k13}$ =49.35% | $\sigma_{DF_k18,k14}$ =43.20% | $\sigma_{DF_k18,k15}$ =61.78% | $\sigma_{DF_k18,k16}$ =46.27% | $\sigma_{DF_k18,k17}$ =58.70% | | $\sigma_{DF_k18,k19}$ =32.05% | $\sigma_{DF_k18,k20}$ =8.33% |
| k19 | $\sigma_{DF_k19,k11}$ =58.04% | $\sigma_{DF_k19,k12}$ =52.32% | $\sigma_{DF_k19,k13}$ =62.97% | $\sigma_{DF_k19,k14}$ =57.86% | $\sigma_{DF_k19,k15}$ =75.95% | $\sigma_{DF_k19,k16}$ =58.31% | $\sigma_{DF_k19,k17}$ =73.47% | $\sigma_{DF_k19,k18}$ =32.05% | | $\sigma_{DF_k19,k20}$ =31.40% |
| k20 | $\sigma_{DF_k20,k11}$ =41.02% | $\sigma_{DF_k20,k12}$ =39.17% | $\sigma_{DF_k20,k13}$ =47.95% | $\sigma_{DF_k20,k14}$ =39.52% | $\sigma_{DF_k20,k15}$ =59.25% | $\sigma_{DF_k20,k16}$ =42.65% | $\sigma_{DF_k20,k17}$ =55.75% | $\sigma_{DF_k20,k18}$ =8.33% | $\sigma_{DF_k20,k19}$ =31.40% | |

FIG. 29

| No. | ASTRINGENCY | AFTERTASTE | SWEETNESS | AROMA | BITTERNESS |
|---|---|---|---|---|---|
| 1 | 2.30 | 3.13 | 3.37 | 2.61 | 2.10 |
| 2 | 2.59 | 3.37 | 2.69 | 3.83 | 2.81 |
| 3 | 2.31 | 3.17 | 3.30 | 2.89 | 2.15 |
| 4 | 2.70 | 3.75 | 4.40 | 4.80 | 2.30 |
| 5 | 2.22 | 3.14 | 3.51 | 2.95 | 1.93 |
| 6 | 2.23 | 3.18 | 3.91 | 3.44 | 1.77 |
| 7 | 2.42 | 3.08 | 2.85 | 3.35 | 2.49 |
| 8 | 2.37 | 3.15 | 2.83 | 3.07 | 2.43 |
| 9 | 2.32 | 3.07 | 2.36 | 3.04 | 2.54 |
| 10 | 2.24 | 3.00 | 2.59 | 3.23 | 2.32 |
| 11 | 2.41 | 3.04 | 3.18 | 3.90 | 2.35 |
| 12 | 2.86 | 3.39 | 2.51 | 3.18 | 3.29 |
| 13 | 2.64 | 3.30 | 2.41 | 3.38 | 3.00 |
| 14 | 2.46 | 3.25 | 3.03 | 3.36 | 2.48 |
| 15 | 2.07 | 3.05 | 3.39 | 3.75 | 1.74 |
| 16 | 2.51 | 3.41 | 3.48 | 3.23 | 2.37 |
| 17 | 2.34 | 3.48 | 3.81 | 3.87 | 1.99 |
| 18 | 3.18 | 3.71 | 2.97 | 4.63 | 3.58 |
| 19 | 3.24 | 4.03 | 4.63 | 4.75 | 3.01 |
| 20 | 3.09 | 3.77 | 2.90 | 3.84 | 3.47 |

FIG. 30

| No. | ASTRINGENCY | AFTERTASTE | SWEETNESS | AROMA | BITTERNESS |
|---|---|---|---|---|---|
| 1 | 1.64 | 2.86 | 3.29 | 3.07 | 2.29 |
| 2 | 2.14 | 3.07 | 2.64 | 4.07 | 2.86 |
| 3 | 1.93 | 2.21 | 2.71 | 2.50 | 2.21 |
| 4 | 2.21 | 3.21 | 3.64 | 3.93 | 2.36 |
| 5 | 1.93 | 2.36 | 2.93 | 2.79 | 2.29 |
| 6 | 2.00 | 3.00 | 2.57 | 3.36 | 2.29 |
| 7 | 2.50 | 3.50 | 3.36 | 3.86 | 2.64 |
| 8 | 2.43 | 3.14 | 3.21 | 3.14 | 2.71 |
| 9 | 2.07 | 3.07 | 3.57 | 3.07 | 2.86 |
| 10 | 2.64 | 2.86 | 3.43 | 3.93 | 3.36 |
| 11 | 2.36 | 3.71 | 2.64 | 3.79 | 2.50 |
| 12 | 2.86 | 3.21 | 2.71 | 3.50 | 2.79 |
| 13 | 2.36 | 3.21 | 2.86 | 3.64 | 2.64 |
| 14 | 2.29 | 3.29 | 2.86 | 3.14 | 2.50 |
| 15 | 2.36 | 3.36 | 3.43 | 3.79 | 2.64 |
| 16 | 2.43 | 2.50 | 2.93 | 2.71 | 2.79 |
| 17 | 2.00 | 2.86 | 3.43 | 3.86 | 2.71 |
| 18 | 3.21 | 3.50 | 2.86 | 4.21 | 3.29 |
| 19 | 2.64 | 3.93 | 3.21 | 4.07 | 2.79 |
| 20 | 2.86 | 3.79 | 3.14 | 3.71 | 2.93 |

FIG. 31

| No. | DIFFERENCE IN ASTRINGENCY | DIFFERENCE IN AFTERTASTE | DIFFERENCE IN SWEETNESS | DIFFERENCE IN AROMA | DIFFERENCE IN BITTERNESS |
|---|---|---|---|---|---|
| 1 | 0.658 | 0.271 | 0.081 | −0.466 | −0.181 |
| 2 | 0.446 | 0.301 | 0.050 | −0.243 | −0.051 |
| 3 | 0.382 | 0.955 | 0.581 | 0.395 | −0.067 |
| 4 | 0.489 | 0.539 | 0.753 | 0.875 | −0.061 |
| 5 | 0.296 | 0.783 | 0.581 | 0.160 | −0.353 |
| 6 | 0.226 | 0.183 | 1.343 | 0.078 | −0.513 |
| 7 | −0.080 | −0.418 | −0.509 | −0.512 | −0.152 |
| 8 | −0.056 | 0.008 | −0.384 | −0.074 | −0.287 |
| 9 | 0.251 | −0.005 | −1.209 | −0.029 | −0.319 |
| 10 | −0.407 | 0.142 | −0.839 | −0.701 | −1.039 |
| 11 | 0.058 | −0.677 | 0.537 | 0.113 | −0.150 |
| 12 | 0.004 | 0.178 | −0.205 | −0.316 | 0.502 |
| 13 | 0.286 | 0.090 | −0.443 | −0.267 | 0.356 |
| 14 | 0.177 | −0.031 | 0.177 | 0.215 | −0.020 |
| 15 | −0.292 | −0.306 | −0.043 | −0.031 | −0.899 |
| 16 | 0.084 | 0.905 | 0.556 | 0.519 | −0.412 |
| 17 | 0.341 | 0.626 | 0.379 | 0.014 | −0.726 |
| 18 | −0.035 | 0.206 | 0.110 | 0.415 | 0.296 |
| 19 | 0.601 | 0.102 | 1.414 | 0.681 | 0.229 |
| 20 | 0.231 | −0.012 | −0.248 | 0.126 | 0.545 |
| AVERAGE OF DIFFERENCES | 0.183 | 0.192 | 0.134 | 0.047 | −0.165 |
| STANDARD DEVIATION OF DIFFERENCES | 0.271 | 0.408 | 0.646 | 0.393 | 0.419 |

FIG. 32

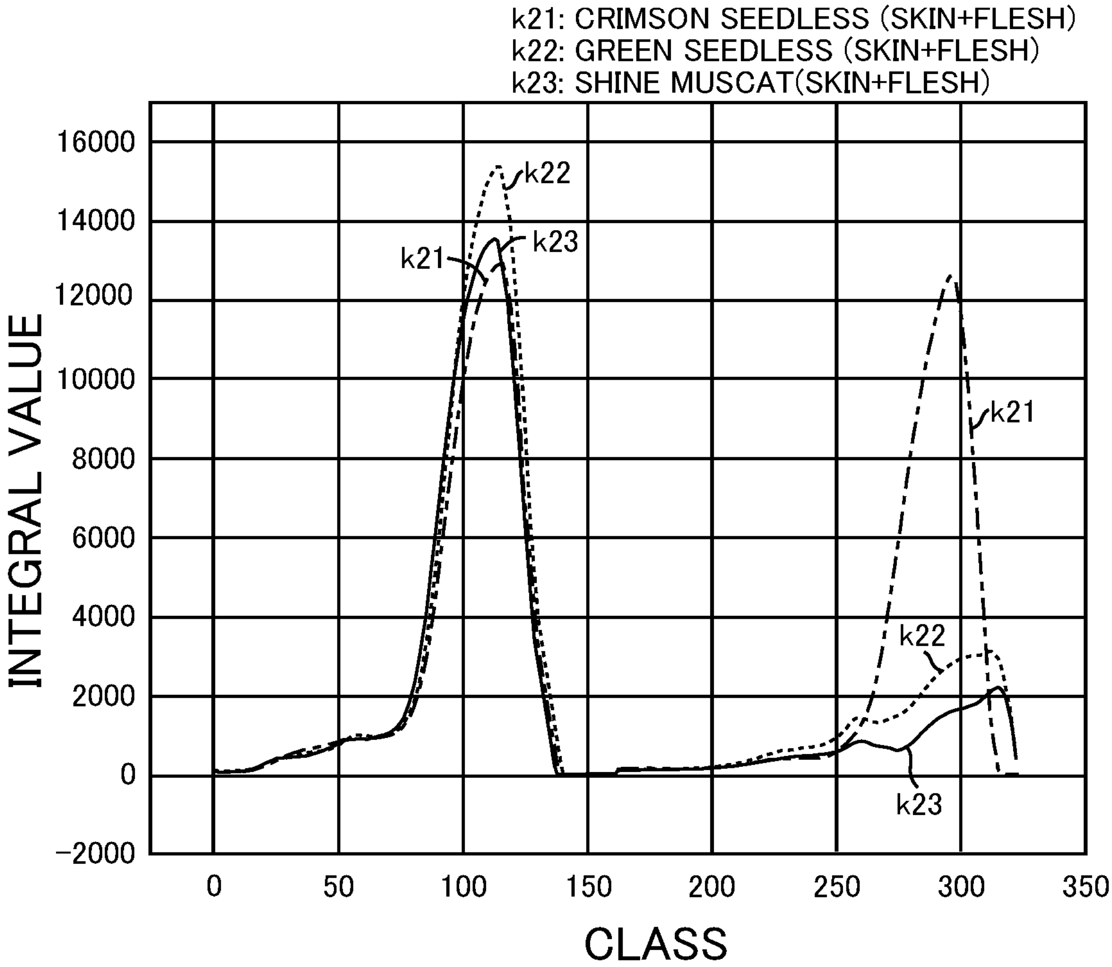
k21: CRIMSON SEEDLESS (SKIN+FLESH)
k22: GREEN SEEDLESS (SKIN+FLESH)
k23: SHINE MUSCAT(SKIN+FLESH)
INTEGRAL VALUE
16000
14000
12000
10000
8000
6000
4000
2000
0
−2000
0
50
100
150
200
250
300
350
CLASS
k21
k22
k23

FIG. 33

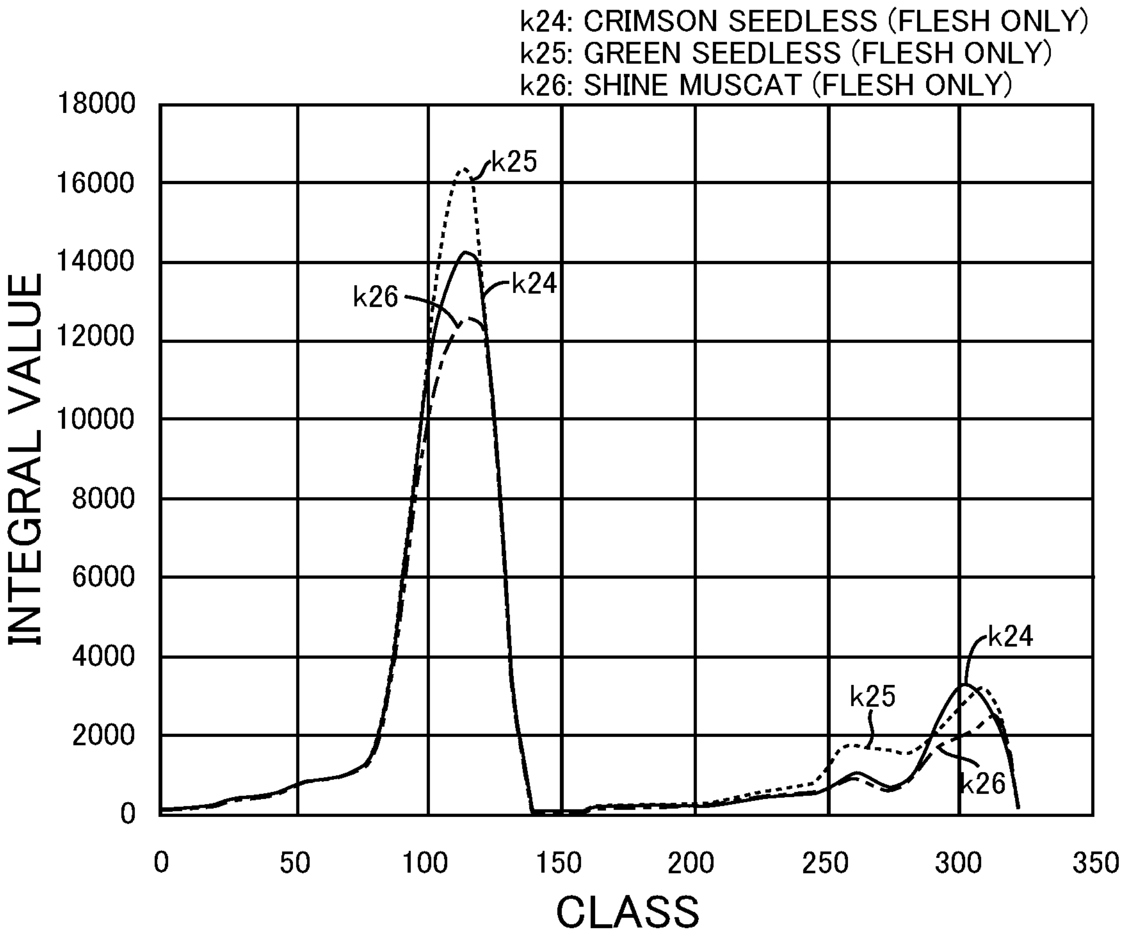
k24: CRIMSON SEEDLESS (FLESH ONLY)
k25: GREEN SEEDLESS (FLESH ONLY)
k26: SHINE MUSCAT (FLESH ONLY)
INTEGRAL VALUE
18000
16000
14000
12000
10000
8000
6000
4000
2000
0
0
50
100
150
200
250
300
350
CLASS
k25
k24
k26
k24
k25
k26

FIG. 34

| | k21 | k22 | k23 | k24 | k25 | k26 |
|---|---|---|---|---|---|---|
| k21 | | $\sigma_{DF_k21,k22}$ =58.9% | $\sigma_{DF_k21,k23}$ =31.2% | $\sigma_{DF_k21,k24}$ =62.0% | $\sigma_{DF_k21,k25}$ =24.1% | $\sigma_{DF_k21,k26}$ =62.5% |
| k22 | $\sigma_{DF_k22,k21}$ =58.9% | | $\sigma_{DF_k22,k23}$ =65.3% | $\sigma_{DF_k22,k24}$ =16.0% | $\sigma_{DF_k22,k25}$ =16.0% | $\sigma_{DF_k22,k26}$ =23.0% |
| k23 | $\sigma_{DF_k23,k21}$ =31.2% | $\sigma_{DF_k23,k22}$ =65.3% | | $\sigma_{DF_k23,k24}$ =30.1% | $\sigma_{DF_k23,k25}$ =31.4% | $\sigma_{DF_k23,k26}$ =24.9% |
| k24 | $\sigma_{DF_k24,k21}$ =62.0% | $\sigma_{DF_k24,k22}$ =16.0% | $\sigma_{DF_k24,k23}$ =30.1% | | $\sigma_{DF_k24,k25}$ =30.3% | $\sigma_{DF_k24,k26}$ =22.9% |
| k25 | $\sigma_{DF_k25,k21}$ =24.1% | $\sigma_{DF_k25,k22}$ =16.0% | $\sigma_{DF_k25,k23}$ =31.4% | $\sigma_{DF_k25,k24}$ =30.3% | | $\sigma_{DF_k25,k26}$ =24.3% |
| k26 | $\sigma_{DF_k26,k21}$ =62.5% | $\sigma_{DF_k26,k22}$ =23.0% | $\sigma_{DF_k26,k23}$ =24.9% | $\sigma_{DF_k26,k24}$ =22.9% | $\sigma_{DF_k26,k25}$ =24.3% | |

FIG. 35

| | ASTRINGENCY |
|---|---|
| GREEN SEEDLESS (FLESH ONLY) | 2.27 |
| GREEN SEEDLESS (FLESH+SKIN) | 2.58 |
| CRIMSON SEEDLESS (FLESH ONLY) | 2.96 |
| CRIMSON SEEDLESS (FLESH+SKIN) | 4.65 |
| SHINE MUSCAT (FLESH ONLY) | 2.06 |
| SHINE MUSCAT (FLESH+SKIN) | 2.47 |

FIG. 36

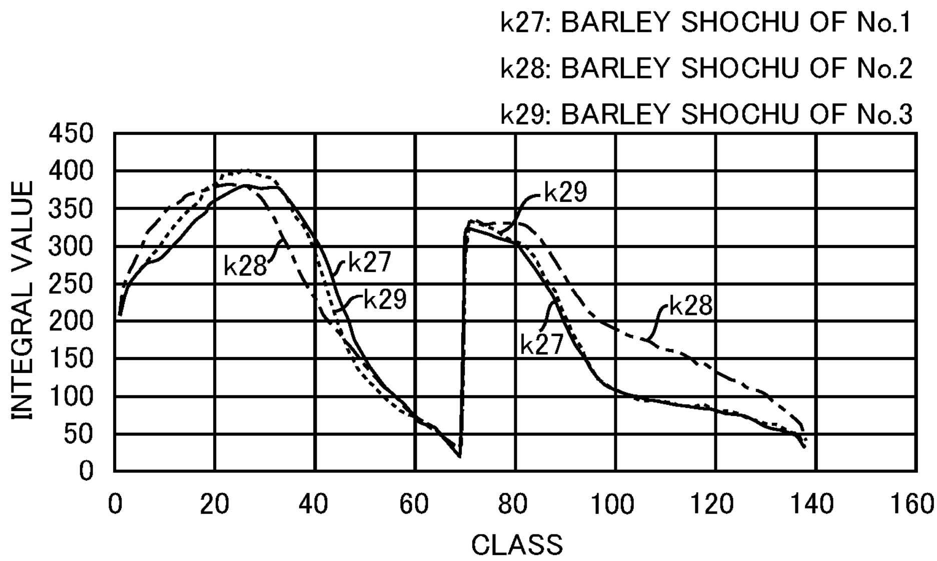


FIG. 37

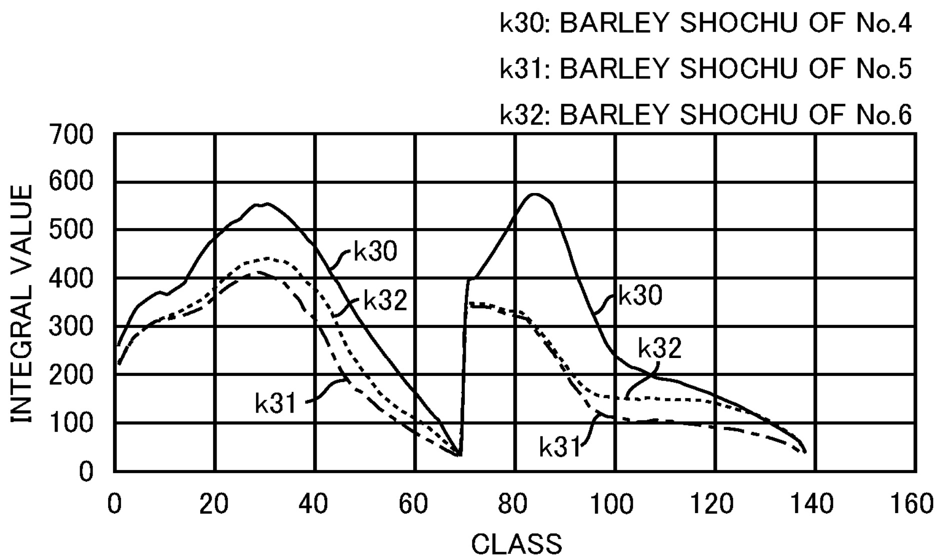


FIG. 38

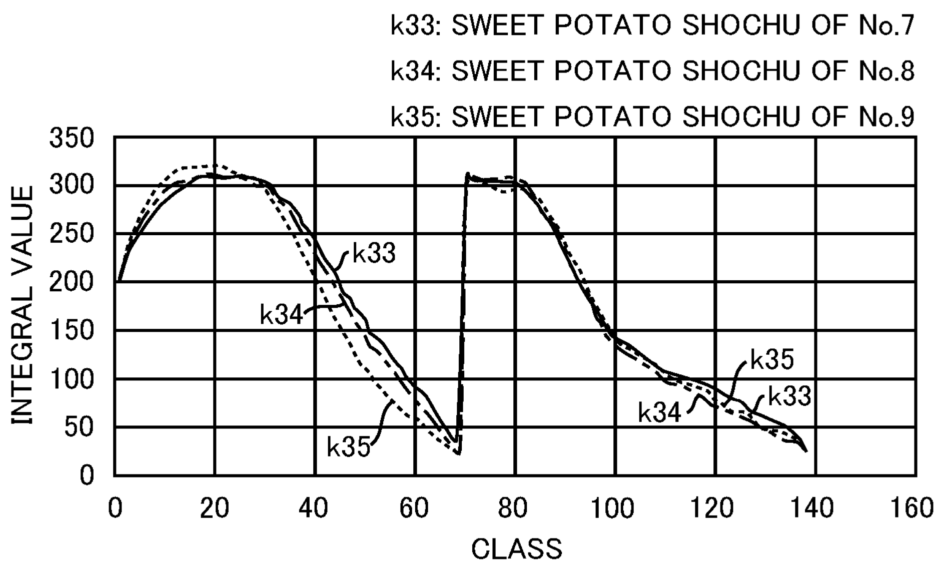

FIG. 39

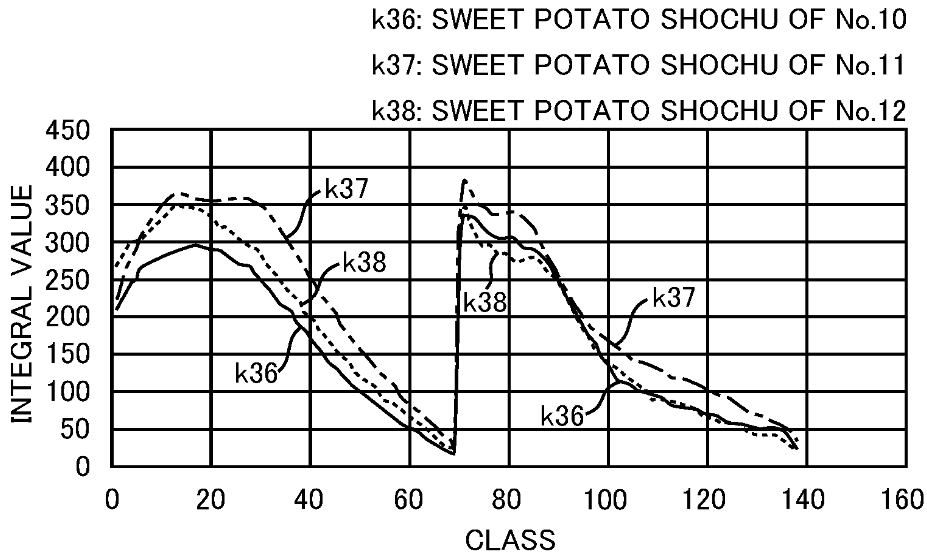


FIG. 40

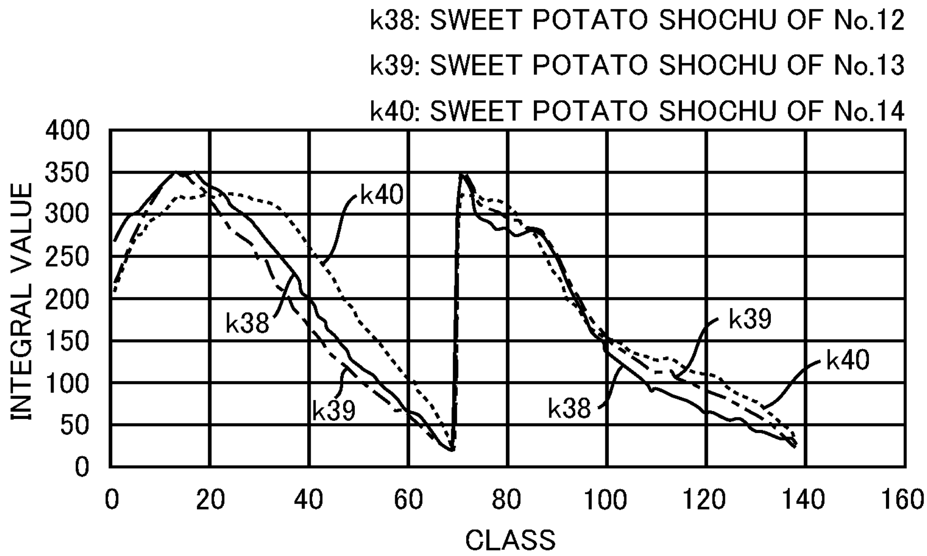


FIG. 41

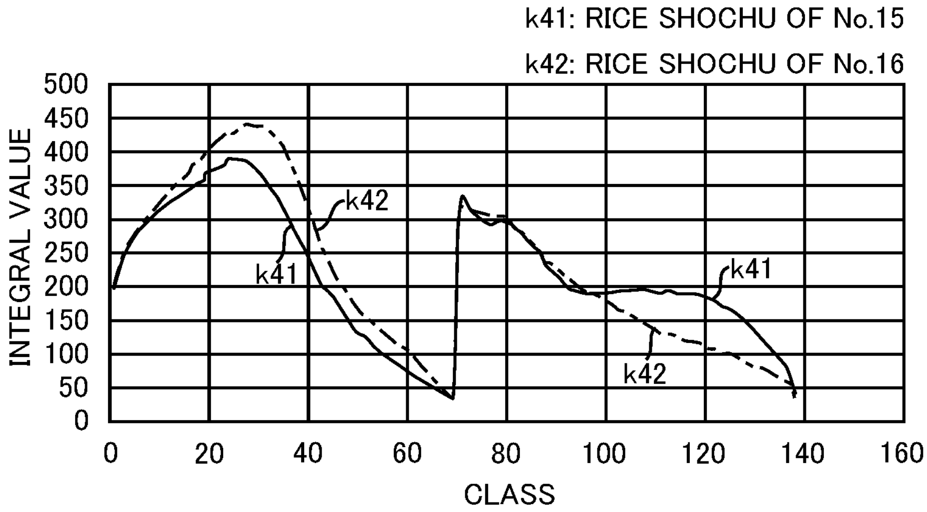

FIG. 42

k43: SHOCHU MADE WITH SAKE LEES OF No.17
k44: BARLEY SHOCHU STORED FOR THREE YEARS IN ITS ENTIRETY, STORED IN BARRELS, AND PARTIALLY STORED IN CHERRY WOOD BARRELS OF No.18
k45: BARLEY SHOCHU STORED FOR 15 YEARS IN ITS ENTIRELY, ALL KOJI BARLEY, ALL STORED IN BARRELS, AND HEATED OF No.19
k46: RICE SHOCHU STORED FOR THREE YEARS IN ITS ENTIRELY, AND STORED IN BARRELS OF No.20

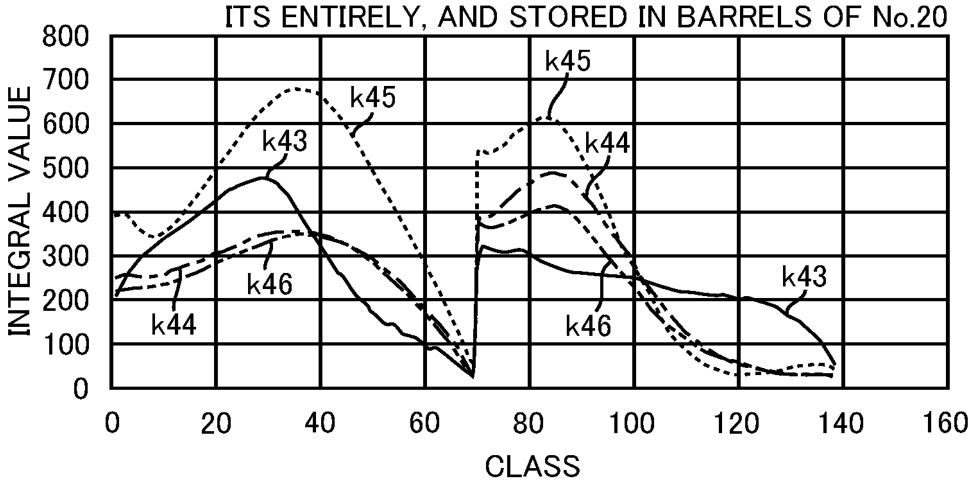

FIG. 43

| | k27 | k28 | k29 | k30 | k31 | k32 | k33 | k34 | k35 | k36 |
|---|---|---|---|---|---|---|---|---|---|---|
| k27 | | $\sigma_{DF_k27,k28}$ =26.12% | $\sigma_{DF_k27,k29}$ =7.75% | $\sigma_{DF_k27,k30}$ =20.36% | $\sigma_{DF_k27,k31}$ =5.47% | $\sigma_{DF_k27,k32}$ =17.14% | $\sigma_{DF_k27,k33}$ =16.33% | $\sigma_{DF_k27,k34}$ =16.06% | $\sigma_{DF_k27,k35}$ =20.31% | $\sigma_{DF_k27,k36}$ =23.47% |
| k28 | $\sigma_{DF_k28,k27}$ =26.12% | | $\sigma_{DF_k28,k29}$ =23.87% | $\sigma_{DF_k28,k30}$ =24.60% | $\sigma_{DF_k28,k31}$ =24.08% | $\sigma_{DF_k28,k32}$ =20.65% | $\sigma_{DF_k28,k33}$ =20.37% | $\sigma_{DF_k28,k34}$ =23.58% | $\sigma_{DF_k28,k35}$ =17.58% | $\sigma_{DF_k28,k36}$ =19.96% |
| k29 | $\sigma_{DF_k29,k27}$ =7.75% | $\sigma_{DF_k29,k28}$ =23.87% | | $\sigma_{DF_k29,k30}$ =22.66% | $\sigma_{DF_k29,k31}$ =6.55% | $\sigma_{DF_k29,k32}$ =18.79% | $\sigma_{DF_k29,k33}$ =17.95% | $\sigma_{DF_k29,k34}$ =17.47% | $\sigma_{DF_k29,k35}$ =18.45% | $\sigma_{DF_k29,k36}$ =21.53% |
| k30 | $\sigma_{DF_k30,k27}$ =20.36% | $\sigma_{DF_k30,k28}$ =24.60% | $\sigma_{DF_k30,k29}$ =22.66% | | $\sigma_{DF_k30,k31}$ =20.76% | $\sigma_{DF_k30,k32}$ =19.13% | $\sigma_{DF_k30,k33}$ =12.38% | $\sigma_{DF_k30,k34}$ =16.04% | $\sigma_{DF_k30,k35}$ =21.07% | $\sigma_{DF_k30,k36}$ =23.26% |
| k31 | $\sigma_{DF_k31,k27}$ =5.47% | $\sigma_{DF_k31,k28}$ =24.08% | $\sigma_{DF_k31,k29}$ =6.55% | $\sigma_{DF_k31,k30}$ =20.76% | | $\sigma_{DF_k31,k32}$ =15.01% | $\sigma_{DF_k31,k33}$ =16.53% | $\sigma_{DF_k31,k34}$ =17.50% | $\sigma_{DF_k31,k35}$ =20.59% | $\sigma_{DF_k31,k36}$ =23.56% |
| k32 | $\sigma_{DF_k32,k27}$ =17.14% | $\sigma_{DF_k32,k28}$ =20.65% | $\sigma_{DF_k32,k29}$ =18.79% | $\sigma_{DF_k32,k30}$ =19.13% | $\sigma_{DF_k32,k31}$ =15.01% | | $\sigma_{DF_k32,k33}$ =17.42% | $\sigma_{DF_k32,k34}$ =22.77% | $\sigma_{DF_k32,k35}$ =25.60% | $\sigma_{DF_k32,k36}$ =29.14% |
| k33 | $\sigma_{DF_k33,k27}$ =16.33% | $\sigma_{DF_k33,k28}$ =20.37% | $\sigma_{DF_k33,k29}$ =17.95% | $\sigma_{DF_k33,k30}$ =12.38% | $\sigma_{DF_k33,k31}$ =16.53% | $\sigma_{DF_k33,k32}$ =17.42% | | $\sigma_{DF_k33,k34}$ =8.54% | $\sigma_{DF_k33,k35}$ =15.61% | $\sigma_{DF_k33,k36}$ =18.65% |
| k34 | $\sigma_{DF_k34,k27}$ =16.06% | $\sigma_{DF_k34,k28}$ =23.58% | $\sigma_{DF_k34,k29}$ =17.47% | $\sigma_{DF_k34,k30}$ =16.04% | $\sigma_{DF_k34,k31}$ =17.50% | $\sigma_{DF_k34,k32}$ =22.77% | $\sigma_{DF_k34,k33}$ =8.54% | | $\sigma_{DF_k34,k35}$ =12.51% | $\sigma_{DF_k34,k36}$ =15.64% |
| k35 | $\sigma_{DF_k35,k27}$ =20.31% | $\sigma_{DF_k35,k28}$ =17.58% | $\sigma_{DF_k35,k29}$ =18.45% | $\sigma_{DF_k35,k30}$ =21.07% | $\sigma_{DF_k35,k31}$ =20.59% | $\sigma_{DF_k35,k32}$ =25.60% | $\sigma_{DF_k35,k33}$ =15.61% | $\sigma_{DF_k35,k34}$ =12.51% | | $\sigma_{DF_k35,k36}$ =7.53% |
| k36 | $\sigma_{DF_k36,k27}$ =23.47% | $\sigma_{DF_k36,k28}$ =19.96% | $\sigma_{DF_k36,k29}$ =21.53% | $\sigma_{DF_k36,k30}$ =23.26% | $\sigma_{DF_k36,k31}$ =23.56% | $\sigma_{DF_k36,k32}$ =29.14% | $\sigma_{DF_k36,k33}$ =18.65% | $\sigma_{DF_k36,k34}$ =15.64% | $\sigma_{DF_k36,k35}$ =7.53% | |

FIG. 44

| | k37 | k38 | k39 | k40 | k41 | k42 | k43 | k44 | k45 | k46 |
|---|---|---|---|---|---|---|---|---|---|---|
| k27 | $\sigma_{DF_k27,k37}$ =23.98% | $\sigma_{DF_k27,k38}$ =20.41% | $\sigma_{DF_k27,k39}$ =27.11% | $\sigma_{DF_k27,k40}$ =18.52% | $\sigma_{DF_k27,k41}$ =33.82% | $\sigma_{DF_k27,k42}$ =15.60% | $\sigma_{DF_k27,k43}$ =33.39% | $\sigma_{DF_k27,k44}$ =46.05% | $\sigma_{DF_k27,k45}$ =52.89% | $\sigma_{DF_k27,k46}$ =42.39% |
| k28 | $\sigma_{DF_k28,k37}$ =22.11% | $\sigma_{DF_k28,k38}$ =24.20% | $\sigma_{DF_k28,k39}$ =15.22% | $\sigma_{DF_k28,k40}$ =19.01% | $\sigma_{DF_k28,k41}$ =13.73% | $\sigma_{DF_k28,k42}$ =18.18% | $\sigma_{DF_k28,k43}$ =17.96% | $\sigma_{DF_k28,k44}$ =54.02% | $\sigma_{DF_k28,k45}$ =68.41% | $\sigma_{DF_k28,k46}$ =51.82% |
| k29 | $\sigma_{DF_k29,k37}$ =23.83% | $\sigma_{DF_k29,k38}$ =19.75% | $\sigma_{DF_k29,k39}$ =24.63% | $\sigma_{DF_k29,k40}$ =19.98% | $\sigma_{DF_k29,k41}$ =31.84% | $\sigma_{DF_k29,k42}$ =15.93% | $\sigma_{DF_k29,k43}$ =32.33% | $\sigma_{DF_k29,k44}$ =48.80% | $\sigma_{DF_k29,k45}$ =55.60% | $\sigma_{DF_k29,k46}$ =45.63% |
| k30 | $\sigma_{DF_k30,k37}$ =21.24% | $\sigma_{DF_k30,k38}$ =23.82% | $\sigma_{DF_k30,k39}$ =25.25% | $\sigma_{DF_k30,k39}$ =13.47% | $\sigma_{DF_k30,k41}$ =32.44% | $\sigma_{DF_k30,k42}$ =16.80% | $\sigma_{DF_k30,k43}$ =31.38% | $\sigma_{DF_k30,k44}$ =35.48% | $\sigma_{DF_k30,k45}$ =51.52% | $\sigma_{DF_k30,k46}$ =31.57% |
| k31 | $\sigma_{DF_k31,k37}$ =23.35% | $\sigma_{DF_k31,k38}$ =21.63% | $\sigma_{DF_k31,k39}$ =26.22% | $\sigma_{DF_k31,k40}$ =17.38% | $\sigma_{DF_k31,k41}$ =31.43% | $\sigma_{DF_k31,k42}$ =14.73% | $\sigma_{DF_k31,k43}$ =31.63% | $\sigma_{DF_k31,k44}$ =48.53% | $\sigma_{DF_k31,k45}$ =56.06% | $\sigma_{DF_k31,k46}$ =44.90% |
| k32 | $\sigma_{DF_k32,k37}$ =23.96% | $\sigma_{DF_k32,k38}$ =28.78% | $\sigma_{DF_k32,k39}$ =28.48% | $\sigma_{DF_k32,k40}$ =13.16% | $\sigma_{DF_k32,k41}$ =23.26% | $\sigma_{DF_k32,k42}$ =11.80% | $\sigma_{DF_k32,k43}$ =21.97% | $\sigma_{DF_k32,k44}$ =50.64% | $\sigma_{DF_k32,k45}$ =61.98% | $\sigma_{DF_k32,k46}$ =46.41% |
| k33 | $\sigma_{DF_k33,k37}$ =19.04% | $\sigma_{DF_k33,k38}$ =16.09% | $\sigma_{DF_k33,k39}$ =19.60% | $\sigma_{DF_k33,k40}$ =7.45% | $\sigma_{DF_k33,k41}$ =29.52% | $\sigma_{DF_k33,k42}$ =12.60% | $\sigma_{DF_k33,k43}$ =28.98% | $\sigma_{DF_k33,k44}$ =39.28% | $\sigma_{DF_k33,k45}$ =53.86% | $\sigma_{DF_k33,k46}$ =35.92% |
| k34 | $\sigma_{DF_k34,k37}$ =20.28% | $\sigma_{DF_k34,k38}$ =10.43% | $\sigma_{DF_k34,k39}$ =19.32% | $\sigma_{DF_k34,k40}$ =14.84% | $\sigma_{DF_k34,k41}$ =33.75% | $\sigma_{DF_k34,k42}$ =17.00% | $\sigma_{DF_k34,k43}$ =33.58% | $\sigma_{DF_k34,k44}$ =36.68% | $\sigma_{DF_k34,k45}$ =50.32% | $\sigma_{DF_k34,k46}$ =33.78% |
| k35 | $\sigma_{DF_k35,k37}$ =20.72% | $\sigma_{DF_k35,k38}$ =10.08% | $\sigma_{DF_k35,k39}$ =10.60% | $\sigma_{DF_k35,k40}$ =19.90% | $\sigma_{DF_k35,k41}$ =29.17% | $\sigma_{DF_k35,k42}$ =19.52% | $\sigma_{DF_k35,k43}$ =31.27% | $\sigma_{DF_k35,k44}$ =44.15% | $\sigma_{DF_k35,k45}$ =56.86% | $\sigma_{DF_k35,k46}$ =42.36% |
| k36 | $\sigma_{DF_k36,k37}$ =23.05% | $\sigma_{DF_k36,k38}$ =11.19% | $\sigma_{DF_k36,k39}$ =9.72% | $\sigma_{DF_k36,k40}$ =22.85% | $\sigma_{DF_k36,k41}$ =31.53% | $\sigma_{DF_k36,k42}$ =24.20% | $\sigma_{DF_k36,k43}$ =34.31% | $\sigma_{DF_k36,k44}$ =44.44% | $\sigma_{DF_k36,k45}$ =56.28% | $\sigma_{DF_k36,k46}$ =43.06% |

FIG. 45

| | k37 | k38 | k39 | k40 | k41 | k42 | k43 | k44 | k45 | k46 |
|---|---|---|---|---|---|---|---|---|---|---|
| k37 | | $\sigma_{DF_k37,k38}$ =22.42% | $\sigma_{DF_k37,k39}$ =22.73% | $\sigma_{DF_k37,k40}$ =19.85% | $\sigma_{DF_k37,k41}$ =30.39% | $\sigma_{DF_k37,k42}$ =20.56% | $\sigma_{DF_k37,k43}$ =30.94% | $\sigma_{DF_k37,k44}$ =45.74% | $\sigma_{DF_k37,k45}$ =58.48% | $\sigma_{DF_k37,k46}$ =43.42% |
| k38 | $\sigma_{DF_k38,k37}$ =22.42% | | $\sigma_{DF_k38,k39}$ =15.63% | $\sigma_{DF_k38,k40}$ =21.55% | $\sigma_{DF_k38,k41}$ =35.25% | $\sigma_{DF_k38,k42}$ =22.43% | $\sigma_{DF_k38,k43}$ =36.50% | $\sigma_{DF_k38,k44}$ =40.85% | $\sigma_{DF_k38,k45}$ =52.24% | $\sigma_{DF_k38,k46}$ =39.01% |
| k39 | $\sigma_{DF_k39,k37}$ =22.73% | $\sigma_{DF_k39,k38}$ =15.63% | | $\sigma_{DF_k39,k40}$ =21.87% | $\sigma_{DF_k39,k41}$ =26.78% | $\sigma_{DF_k39,k42}$ =23.76% | $\sigma_{DF_k39,k43}$ =30.69% | $\sigma_{DF_k39,k44}$ =49.27% | $\sigma_{DF_k39,k45}$ =62.93% | $\sigma_{DF_k39,k46}$ =47.85% |
| k40 | $\sigma_{DF_k40,k37}$ =19.85% | $\sigma_{DF_k40,k38}$ =21.55% | $\sigma_{DF_k40,k39}$ =21.87% | | $\sigma_{DF_k40,k41}$ =26.13% | $\sigma_{DF_k40,k42}$ =11.34% | $\sigma_{DF_k40,k43}$ =25.48% | $\sigma_{DF_k40,k44}$ =43.14% | $\sigma_{DF_k40,k45}$ =57.83% | $\sigma_{DF_k40,k46}$ =39.44% |
| k41 | $\sigma_{DF_k41,k37}$ =30.39% | $\sigma_{DF_k41,k38}$ =35.25% | $\sigma_{DF_k41,k39}$ =26.78% | $\sigma_{DF_k41,k40}$ =26.13% | | $\sigma_{DF_k41,k42}$ =24.72% | $\sigma_{DF_k41,k43}$ =9.70% | $\sigma_{DF_k41,k44}$ =61.71% | $\sigma_{DF_k41,k45}$ =75.91% | $\sigma_{DF_k41,k46}$ =59.18% |
| k42 | $\sigma_{DF_k42,k37}$ =20.56% | $\sigma_{DF_k42,k38}$ =22.43% | $\sigma_{DF_k42,k39}$ =23.76% | $\sigma_{DF_k42,k40}$ =11.34% | $\sigma_{DF_k42,k41}$ =24.72% | | $\sigma_{DF_k42,k43}$ =22.72% | $\sigma_{DF_k42,k44}$ =46.19% | $\sigma_{DF_k42,k45}$ =58.27% | $\sigma_{DF_k42,k46}$ =42.58% |
| k43 | $\sigma_{DF_k43,k37}$ =30.94% | $\sigma_{DF_k43,k38}$ =36.50% | $\sigma_{DF_k43,k39}$ =30.69% | $\sigma_{DF_k43,k40}$ =25.48% | $\sigma_{DF_k43,k41}$ =9.70% | $\sigma_{DF_k43,k42}$ =22.72% | | $\sigma_{DF_k43,k44}$ =58.66% | $\sigma_{DF_k43,k45}$ =73.45% | $\sigma_{DF_k43,k46}$ =55.72% |
| k44 | $\sigma_{DF_k44,k37}$ =45.74% | $\sigma_{DF_k44,k38}$ =40.85% | $\sigma_{DF_k44,k39}$ =49.27% | $\sigma_{DF_k44,k40}$ =43.14% | $\sigma_{DF_k44,k41}$ =61.71% | $\sigma_{DF_k44,k42}$ =46.19% | $\sigma_{DF_k44,k43}$ =58.66% | | $\sigma_{DF_k44,k45}$ =31.96% | $\sigma_{DF_k44,k46}$ =7.89% |
| k45 | $\sigma_{DF_k45,k37}$ =58.48% | $\sigma_{DF_k45,k38}$ =52.24% | $\sigma_{DF_k45,k39}$ =62.93% | $\sigma_{DF_k45,k40}$ =57.83% | $\sigma_{DF_k45,k41}$ =75.91% | $\sigma_{DF_k45,k42}$ =58.27% | $\sigma_{DF_k45,k43}$ =73.45% | $\sigma_{DF_k45,k44}$ =31.96% | | $\sigma_{DF_k45,k46}$ =31.30% |
| k46 | $\sigma_{DF_k46,k37}$ =43.42% | $\sigma_{DF_k46,k38}$ =39.01% | $\sigma_{DF_k46,k39}$ =47.85% | $\sigma_{DF_k46,k40}$ =39.44% | $\sigma_{DF_k46,k41}$ =59.18% | $\sigma_{DF_k46,k42}$ =42.58% | $\sigma_{DF_k46,k43}$ =55.72% | $\sigma_{DF_k46,k44}$ =7.89% | $\sigma_{DF_k46,k45}$ =31.30% | |

FIG. 46

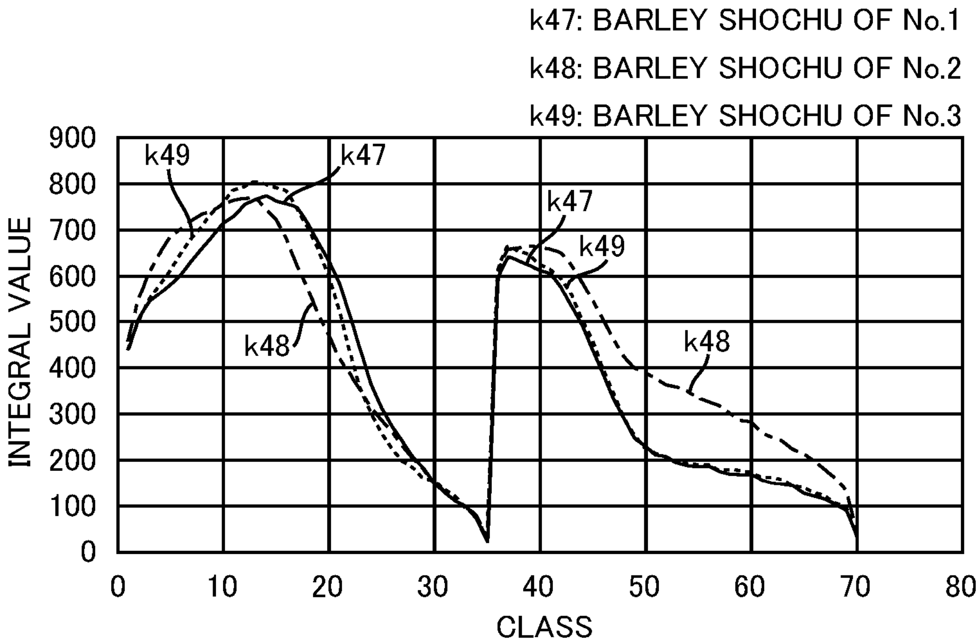
k47: BARLEY SHOCHU OF No.1
k48: BARLEY SHOCHU OF No.2
k49: BARLEY SHOCHU OF No.3
INTEGRAL VALUE
900
800
700
600
500
400
300
200
100
0
0
10
20
30
40
50
60
70
80
CLASS
k49
k47
k48
k47
k49
k48

FIG. 47

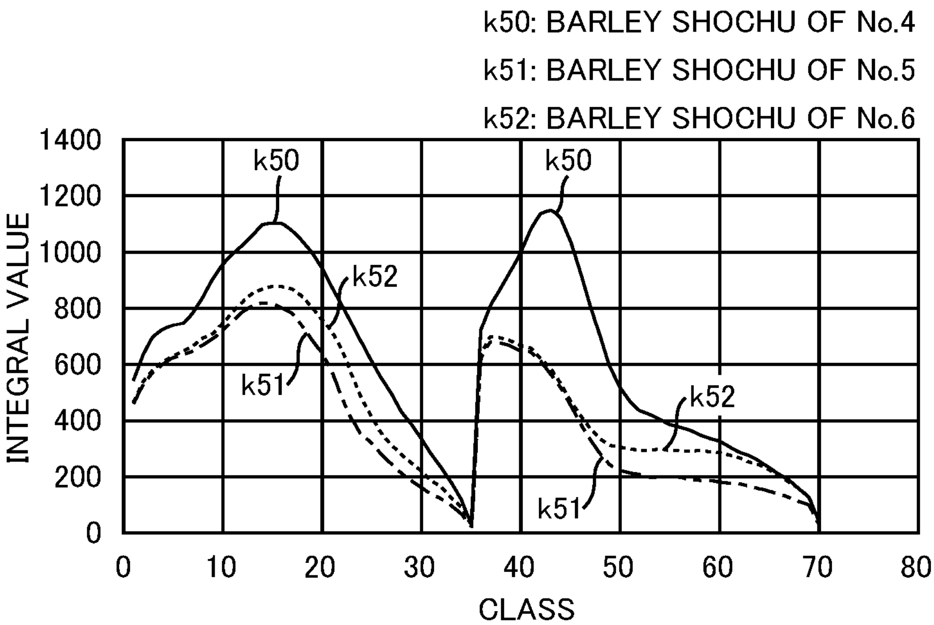
k50: BARLEY SHOCHU OF No.4
k51: BARLEY SHOCHU OF No.5
k52: BARLEY SHOCHU OF No.6
INTEGRAL VALUE
1400
1200
1000
800
600
400
200
0
0
10
20
30
40
50
60
70
80
CLASS
k50
k52
k51
k50
k52
k51

FIG. 48

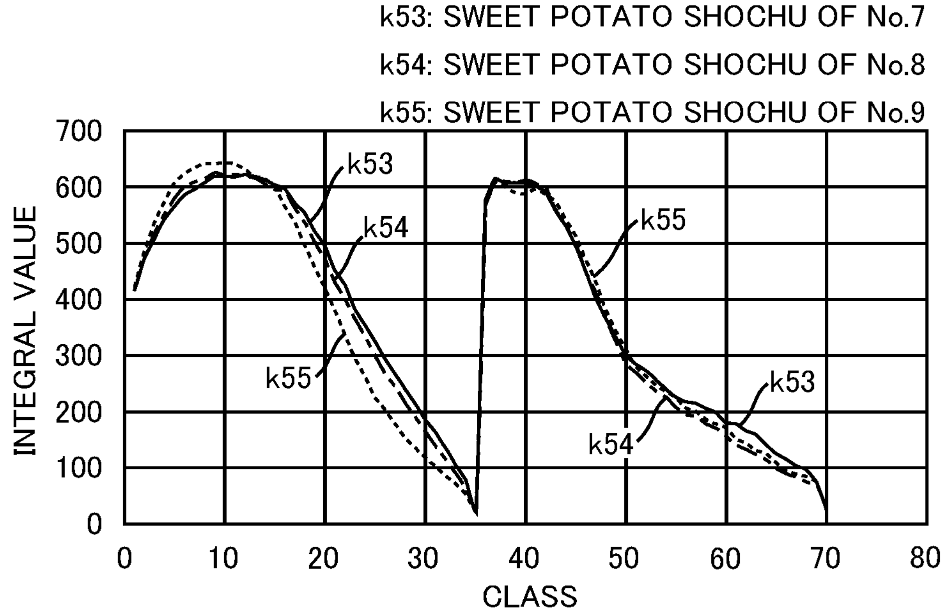
k53: SWEET POTATO SHOCHU OF No.7
k54: SWEET POTATO SHOCHU OF No.8
k55: SWEET POTATO SHOCHU OF No.9
INTEGRAL VALUE
CLASS
0
100
200
300
400
500
600
700
0
10
20
30
40
50
60
70
80
k53
k54
k55
k55
k53
k54

FIG. 49

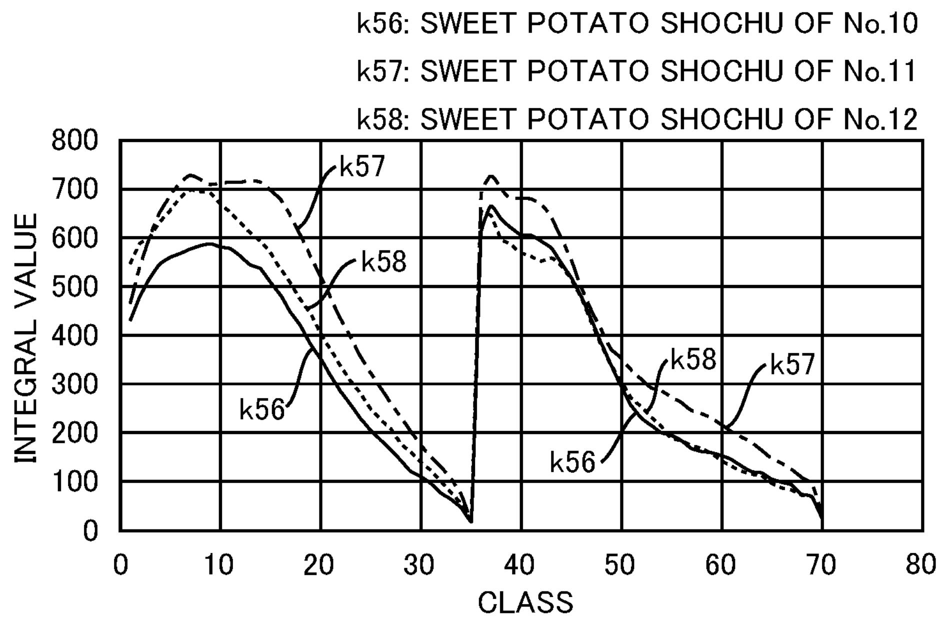
k56: SWEET POTATO SHOCHU OF No.10
k57: SWEET POTATO SHOCHU OF No.11
k58: SWEET POTATO SHOCHU OF No.12
INTEGRAL VALUE
CLASS
0
100
200
300
400
500
600
700
800
0
10
20
30
40
50
60
70
80
k57
k58
k56
k58
k57
k56

FIG. 50

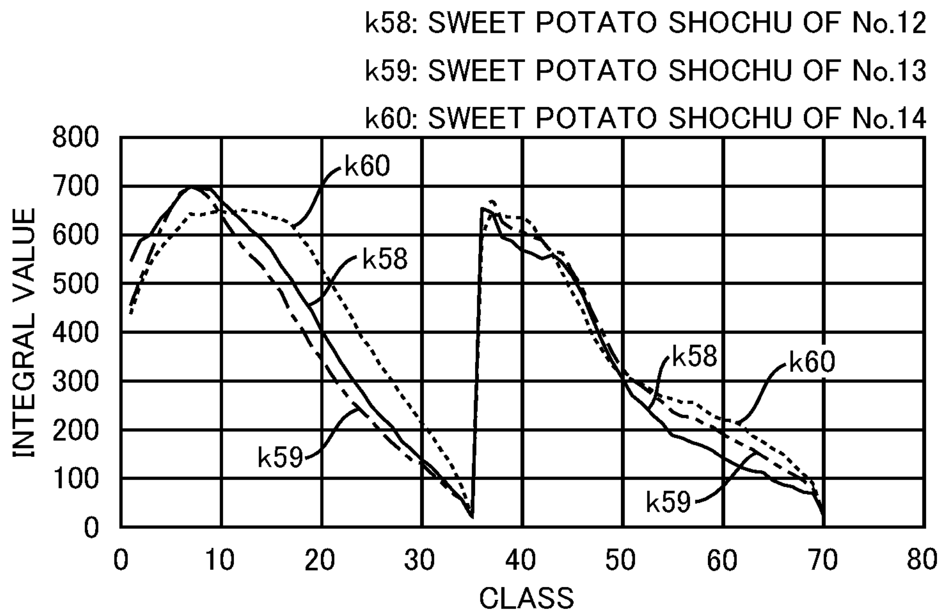
k58: SWEET POTATO SHOCHU OF No.12
k59: SWEET POTATO SHOCHU OF No.13
k60: SWEET POTATO SHOCHU OF No.14
INTEGRAL VALUE
CLASS
0
100
200
300
400
500
600
700
800
10
20
30
40
50
60
70
80
k58
k59
k60

FIG. 51

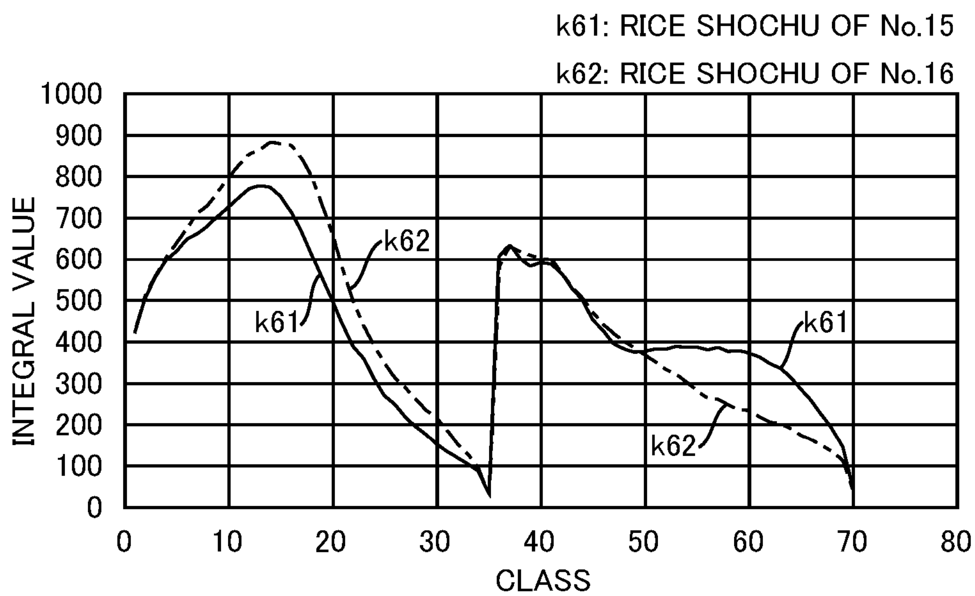
k61: RICE SHOCHU OF No.15
k62: RICE SHOCHU OF No.16
INTEGRAL VALUE
CLASS
0
100
200
300
400
500
600
700
800
900
1000
10
20
30
40
50
60
70
80
k61
k62

FIG. 52

k63: SHOCHU MADE FROM SAKE LEES OF No.17

k64: ALL SHOCHU AGED FOR 3 YEARS, ALL STORED IN BARRELS, SOME IN CHERRY WOOD BARRELS OF No.18

k65: BARLEY SHOCHU STORED FOR 15 YEARS, ALL KOJI BARLEY, STORED IN BARRELS, AND HEATED OF No.19

k66: RICE SHOCHU STORED FOR 3 YEARS AND STORED IN BARRELS OF No.20

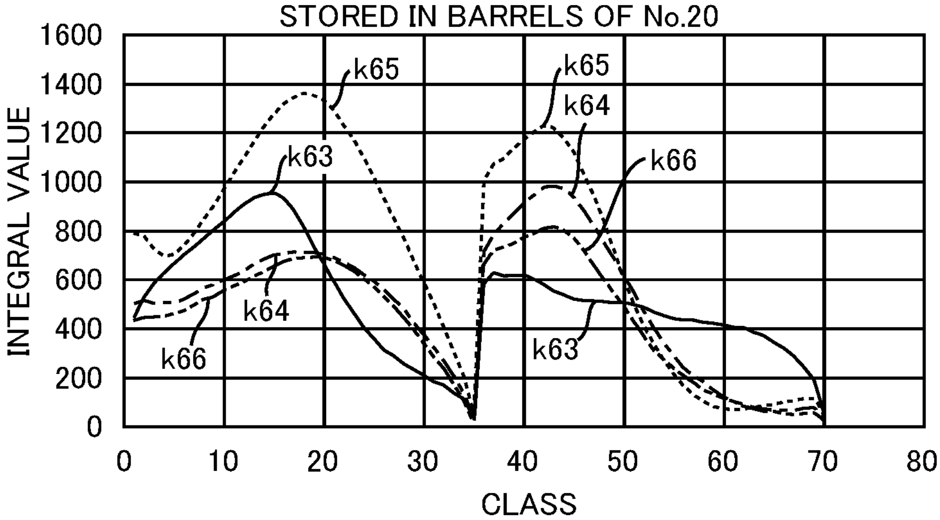

FIG. 53

| | k47 | k48 | k49 | k50 | k51 | k52 | k53 | k54 | k55 | k56 |
|---|---|---|---|---|---|---|---|---|---|---|
| k47 | | $\sigma_{DF_k47,k48}$ =25.93% | $\sigma_{DF_k47,k49}$ =7.90% | $\sigma_{DF_k47,k50}$ =20.32% | $\sigma_{DF_k47,k51}$ =5.33% | $\sigma_{DF_k47,k52}$ =16.98% | $\sigma_{DF_k47,k53}$ =16.24% | $\sigma_{DF_k47,k54}$ =15.87% | $\sigma_{DF_k47,k55}$ =20.17% | $\sigma_{DF_k47,k56}$ =23.25% |
| k48 | $\sigma_{DF_k48,k47}$ =25.93% | | $\sigma_{DF_k48,k49}$ =23.66% | $\sigma_{DF_k48,k50}$ =24.66% | $\sigma_{DF_k48,k51}$ =23.89% | $\sigma_{DF_k48,k52}$ =20.50% | $\sigma_{DF_k48,k53}$ =20.35% | $\sigma_{DF_k48,k54}$ =23.45% | $\sigma_{DF_k48,k55}$ =17.50% | $\sigma_{DF_k48,k56}$ =19.91% |
| k49 | $\sigma_{DF_k49,k47}$ =7.90% | $\sigma_{DF_k49,k48}$ =23.66% | | $\sigma_{DF_k49,k50}$ =22.80% | $\sigma_{DF_k49,k51}$ =6.45% | $\sigma_{DF_k49,k52}$ =18.69% | $\sigma_{DF_k49,k53}$ =18.03% | $\sigma_{DF_k49,k54}$ =17.43% | $\sigma_{DF_k49,k55}$ =18.39% | $\sigma_{DF_k49,k56}$ =21.52% |
| k50 | $\sigma_{DF_k50,k47}$ =20.32% | $\sigma_{DF_k50,k48}$ =24.66% | $\sigma_{DF_k50,k49}$ =22.80% | | $\sigma_{DF_k50,k51}$ =20.74% | $\sigma_{DF_k50,k52}$ =19.13% | $\sigma_{DF_k50,k53}$ =12.24% | $\sigma_{DF_k50,k54}$ =15.96% | $\sigma_{DF_k50,k55}$ =20.93% | $\sigma_{DF_k50,k56}$ =23.06% |
| k51 | $\sigma_{DF_k51,k47}$ =5.33% | $\sigma_{DF_k51,k48}$ =23.89% | $\sigma_{DF_k51,k49}$ =6.45% | $\sigma_{DF_k51,k50}$ =20.74% | | $\sigma_{DF_k51,k52}$ =14.85% | $\sigma_{DF_k51,k53}$ =16.42% | $\sigma_{DF_k51,k54}$ =17.33% | $\sigma_{DF_k51,k55}$ =20.40% | $\sigma_{DF_k51,k56}$ =23.38% |
| k52 | $\sigma_{DF_k52,k47}$ =16.98% | $\sigma_{DF_k52,k48}$ =20.50% | $\sigma_{DF_k52,k49}$ =18.69% | $\sigma_{DF_k52,k50}$ =19.13% | $\sigma_{DF_k52,k51}$ =14.85% | | $\sigma_{DF_k52,k53}$ =17.30% | $\sigma_{DF_k52,k54}$ =22.58% | $\sigma_{DF_k52,k55}$ =25.40% | $\sigma_{DF_k52,k56}$ =28.91% |
| k53 | $\sigma_{DF_k53,k47}$ =16.24% | $\sigma_{DF_k53,k48}$ =20.35% | $\sigma_{DF_k53,k49}$ =18.03% | $\sigma_{DF_k53,k50}$ =12.24% | $\sigma_{DF_k53,k51}$ =16.42% | $\sigma_{DF_k53,k52}$ =17.30% | | $\sigma_{DF_k53,k54}$ =8.32% | $\sigma_{DF_k53,k55}$ =15.43% | $\sigma_{DF_k53,k56}$ =18.42% |
| k54 | $\sigma_{DF_k54,k47}$ =15.87% | $\sigma_{DF_k54,k48}$ =23.45% | $\sigma_{DF_k54,k49}$ =17.43% | $\sigma_{DF_k54,k50}$ =15.96% | $\sigma_{DF_k54,k51}$ =17.33% | $\sigma_{DF_k54,k52}$ =22.58% | $\sigma_{DF_k54,k53}$ =8.32% | | $\sigma_{DF_k54,k55}$ =12.35% | $\sigma_{DF_k54,k56}$ =15.42% |
| k55 | $\sigma_{DF_k55,k47}$ =20.17% | $\sigma_{DF_k55,k48}$ =17.50% | $\sigma_{DF_k55,k49}$ =18.39% | $\sigma_{DF_k55,k50}$ =20.93% | $\sigma_{DF_k55,k51}$ =20.40% | $\sigma_{DF_k55,k52}$ =25.40% | $\sigma_{DF_k55,k53}$ =15.43% | $\sigma_{DF_k55,k54}$ =12.35% | | $\sigma_{DF_k55,k56}$ =7.43% |
| k56 | $\sigma_{DF_k56,k47}$ =23.25% | $\sigma_{DF_k56,k48}$ =19.91% | $\sigma_{DF_k56,k49}$ =21.52% | $\sigma_{DF_k56,k50}$ =23.06% | $\sigma_{DF_k56,k51}$ =23.38% | $\sigma_{DF_k56,k52}$ =28.91% | $\sigma_{DF_k56,k53}$ =18.42% | $\sigma_{DF_k56,k54}$ =15.42% | $\sigma_{DF_k56,k55}$ =7.43% | |

FIG. 54

| | k57 | k58 | k59 | k60 | k61 | k62 | k63 | k64 | k65 | k66 |
|---|---|---|---|---|---|---|---|---|---|---|
| k47 | $\sigma_{DF_k47,k57}$ =16.34% | $\sigma_{DF_k47,k58}$ =20.23% | $\sigma_{DF_k47,k59}$ =27.20% | $\sigma_{DF_k47,k60}$ =18.71% | $\sigma_{DF_k47,k61}$ =33.55% | $\sigma_{DF_k47,k62}$ =15.49% | $\sigma_{DF_k47,k63}$ =33.08% | $\sigma_{DF_k47,k64}$ =46.07% | $\sigma_{DF_k47,k65}$ =52.68% | $\sigma_{DF_k47,k66}$ =42.26% |
| k48 | $\sigma_{DF_k48,k57}$ =15.15% | $\sigma_{DF_k48,k58}$ =23.96% | $\sigma_{DF_k48,k59}$ =15.59% | $\sigma_{DF_k48,k60}$ =19.22% | $\sigma_{DF_k48,k61}$ =13.53% | $\sigma_{DF_k48,k62}$ =18.02% | $\sigma_{DF_k48,k63}$ =17.71% | $\sigma_{DF_k48,k64}$ =53.84% | $\sigma_{DF_k48,k65}$ =67.96% | $\sigma_{DF_k48,k66}$ =51.54% |
| k49 | $\sigma_{DF_k49,k57}$ =16.23% | $\sigma_{DF_k49,k58}$ =19.60% | $\sigma_{DF_k49,k59}$ =24.88% | $\sigma_{DF_k49,k60}$ =20.28% | $\sigma_{DF_k49,k61}$ =31.53% | $\sigma_{DF_k49,k62}$ =15.76% | $\sigma_{DF_k49,k63}$ =31.99% | $\sigma_{DF_k49,k64}$ =48.70% | $\sigma_{DF_k49,k65}$ =55.24% | $\sigma_{DF_k49,k66}$ =45.45% |
| k50 | $\sigma_{DF_k50,k57}$ =16.88% | $\sigma_{DF_k50,k58}$ =23.83% | $\sigma_{DF_k50,k59}$ =25.02% | $\sigma_{DF_k50,k60}$ =13.39% | $\sigma_{DF_k50,k61}$ =32.49% | $\sigma_{DF_k50,k62}$ =17.16% | $\sigma_{DF_k50,k63}$ =31.50% | $\sigma_{DF_k50,k64}$ =35.25% | $\sigma_{DF_k50,k65}$ =51.22% | $\sigma_{DF_k50,k66}$ =31.36% |
| k51 | $\sigma_{DF_k51,k57}$ =15.90% | $\sigma_{DF_k51,k58}$ =21.44% | $\sigma_{DF_k51,k59}$ =26.20% | $\sigma_{DF_k51,k60}$ =17.41% | $\sigma_{DF_k51,k61}$ =31.19% | $\sigma_{DF_k51,k62}$ =14.53% | $\sigma_{DF_k51,k63}$ =31.33% | $\sigma_{DF_k51,k64}$ =48.33% | $\sigma_{DF_k51,k65}$ =55.67% | $\sigma_{DF_k51,k66}$ =44.64% |
| k52 | $\sigma_{DF_k52,k57}$ =18.23% | $\sigma_{DF_k52,k58}$ =28.53% | $\sigma_{DF_k52,k59}$ =28.25% | $\sigma_{DF_k52,k60}$ =13.06% | $\sigma_{DF_k52,k61}$ =23.23% | $\sigma_{DF_k52,k62}$ =11.81% | $\sigma_{DF_k52,k63}$ =21.93% | $\sigma_{DF_k52,k64}$ =50.30% | $\sigma_{DF_k52,k65}$ =61.51% | $\sigma_{DF_k52,k66}$ =46.05% |
| k53 | $\sigma_{DF_k53,k57}$ =7.40% | $\sigma_{DF_k53,k58}$ =15.88% | $\sigma_{DF_k53,k59}$ =19.59% | $\sigma_{DF_k53,k60}$ =7.21% | $\sigma_{DF_k53,k61}$ =29.31% | $\sigma_{DF_k53,k62}$ =12.52% | $\sigma_{DF_k53,k63}$ =28.75% | $\sigma_{DF_k53,k64}$ =39.24% | $\sigma_{DF_k53,k65}$ =53.55% | $\sigma_{DF_k53,k66}$ =35.73% |
| k54 | $\sigma_{DF_k54,k57}$ =9.60% | $\sigma_{DF_k54,k58}$ =10.17% | $\sigma_{DF_k54,k59}$ =19.27% | $\sigma_{DF_k54,k60}$ =14.78% | $\sigma_{DF_k54,k61}$ =33.49% | $\sigma_{DF_k54,k62}$ =16.79% | $\sigma_{DF_k54,k63}$ =33.27% | $\sigma_{DF_k54,k64}$ =36.59% | $\sigma_{DF_k54,k65}$ =49.99% | $\sigma_{DF_k54,k66}$ =33.64% |
| k55 | $\sigma_{DF_k55,k57}$ =10.43% | $\sigma_{DF_k55,k58}$ =10.26% | $\sigma_{DF_k55,k59}$ =10.40% | $\sigma_{DF_k55,k60}$ =19.68% | $\sigma_{DF_k55,k61}$ =29.11% | $\sigma_{DF_k55,k62}$ =19.48% | $\sigma_{DF_k55,k63}$ =31.11% | $\sigma_{DF_k55,k64}$ =43.80% | $\sigma_{DF_k55,k65}$ =56.44% | $\sigma_{DF_k55,k66}$ =42.05% |
| k56 | $\sigma_{DF_k56,k57}$ =13.32% | $\sigma_{DF_k56,k58}$ =10.98% | $\sigma_{DF_k56,k59}$ =9.92% | $\sigma_{DF_k56,k60}$ =22.74% | $\sigma_{DF_k56,k61}$ =31.28% | $\sigma_{DF_k56,k62}$ =23.97% | $\sigma_{DF_k56,k63}$ =33.97% | $\sigma_{DF_k56,k64}$ =44.35% | $\sigma_{DF_k56,k65}$ =55.98% | $\sigma_{DF_k56,k66}$ =42.85% |

FIG. 55

| | k57 | k58 | k59 | k60 | k61 | k62 | k63 | k64 | k65 | k66 |
|---|---|---|---|---|---|---|---|---|---|---|
| k57 | | $\sigma_{DF_k57,k58}$ =13.32% | $\sigma_{DF_k57,k59}$ =13.96% | $\sigma_{DF_k57,k60}$ =11.01% | $\sigma_{DF_k57,k61}$ =26.01% | $\sigma_{DF_k57,k62}$ =12.79% | $\sigma_{DF_k57,k63}$ =27.33% | $\sigma_{DF_k57,k64}$ =43.71% | $\sigma_{DF_k57,k65}$ =57.66% | $\sigma_{DF_k57,k66}$ =40.74% |
| k58 | $\sigma_{DF_k58,k57}$ =13.32% | | $\sigma_{DF_k58,k59}$ =15.67% | $\sigma_{DF_k58,k60}$ =21.43% | $\sigma_{DF_k58,k61}$ =34.93% | $\sigma_{DF_k58,k62}$ =22.17% | $\sigma_{DF_k58,k63}$ =36.13% | $\sigma_{DF_k58,k64}$ =40.73% | $\sigma_{DF_k58,k65}$ =51.90% | $\sigma_{DF_k58,k66}$ =38.78% |
| k59 | $\sigma_{DF_k59,k57}$ =13.96% | $\sigma_{DF_k59,k58}$ =15.67% | | $\sigma_{DF_k59,k60}$ =21.66% | $\sigma_{DF_k59,k61}$ =26.82% | $\sigma_{DF_k59,k62}$ =23.75% | $\sigma_{DF_k59,k63}$ =30.60% | $\sigma_{DF_k59,k64}$ =48.90% | $\sigma_{DF_k59,k65}$ =62.50% | $\sigma_{DF_k59,k66}$ =47.50% |
| k60 | $\sigma_{DF_k60,k57}$ =11.01% | $\sigma_{DF_k60,k58}$ =21.43% | $\sigma_{DF_k60,k59}$ =21.66% | | $\sigma_{DF_k60,k61}$ =26.19% | $\sigma_{DF_k60,k62}$ =11.56% | $\sigma_{DF_k60,k63}$ =25.49% | $\sigma_{DF_k60,k64}$ =42.81% | $\sigma_{DF_k60,k65}$ =57.40% | $\sigma_{DF_k60,k66}$ =39.12% |
| k61 | $\sigma_{DF_k61,k57}$ =26.01% | $\sigma_{DF_k61,k58}$ =34.93% | $\sigma_{DF_k61,k59}$ =26.82% | $\sigma_{DF_k61,k60}$ =26.19% | | $\sigma_{DF_k61,k62}$ =24.52% | $\sigma_{DF_k61,k63}$ =9.51% | $\sigma_{DF_k61,k64}$ =61.42% | $\sigma_{DF_k61,k65}$ =75.38% | $\sigma_{DF_k61,k66}$ =58.82% |
| k62 | $\sigma_{DF_k62,k57}$ =12.79% | $\sigma_{DF_k62,k58}$ =22.17% | $\sigma_{DF_k62,k59}$ =23.75% | $\sigma_{DF_k62,k60}$ =11.56% | $\sigma_{DF_k62,k61}$ =24.52% | | $\sigma_{DF_k62,k63}$ =22.51% | $\sigma_{DF_k62,k64}$ =45.99% | $\sigma_{DF_k62,k65}$ =57.84% | $\sigma_{DF_k62,k66}$ =42.31% |
| k63 | $\sigma_{DF_k63,k57}$ =27.33% | $\sigma_{DF_k63,k58}$ =36.13% | $\sigma_{DF_k63,k59}$ =30.60% | $\sigma_{DF_k63,k60}$ =25.49% | $\sigma_{DF_k63,k61}$ =9.51% | $\sigma_{DF_k63,k62}$ =22.51% | | $\sigma_{DF_k63,k64}$ =58.38% | $\sigma_{DF_k63,k65}$ =72.94% | $\sigma_{DF_k63,k66}$ =55.37% |
| k64 | $\sigma_{DF_k64,k57}$ =43.71% | $\sigma_{DF_k64,k58}$ =40.73% | $\sigma_{DF_k64,k59}$ =48.90% | $\sigma_{DF_k64,k60}$ =42.81% | $\sigma_{DF_k64,k61}$ =61.42% | $\sigma_{DF_k64,k62}$ =45.99% | $\sigma_{DF_k64,k63}$ =58.38% | | $\sigma_{DF_k64,k65}$ =31.75% | $\sigma_{DF_k64,k66}$ =8.00% |
| k65 | $\sigma_{DF_k65,k57}$ =57.66% | $\sigma_{DF_k65,k58}$ =51.90% | $\sigma_{DF_k65,k59}$ =62.50% | $\sigma_{DF_k65,k60}$ =57.40% | $\sigma_{DF_k65,k61}$ =75.38% | $\sigma_{DF_k65,k62}$ =57.84% | $\sigma_{DF_k65,k63}$ =72.94% | $\sigma_{DF_k65,k64}$ =31.75% | | $\sigma_{DF_k65,k66}$ =31.10% |
| k66 | $\sigma_{DF_k66,k57}$ =40.74% | $\sigma_{DF_k66,k58}$ =38.78% | $\sigma_{DF_k66,k59}$ =47.50% | $\sigma_{DF_k66,k60}$ =39.12% | $\sigma_{DF_k66,k61}$ =58.82% | $\sigma_{DF_k66,k62}$ =42.31% | $\sigma_{DF_k66,k63}$ =55.37% | $\sigma_{DF_k66,k64}$ =8.00% | $\sigma_{DF_k66,k65}$ =31.10% | |

FIG. 56

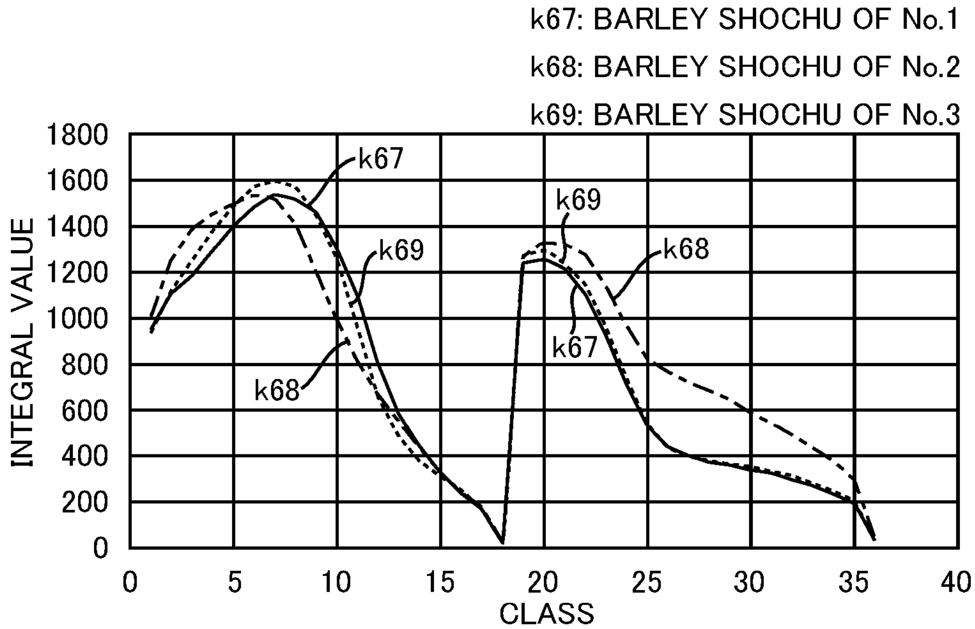
k67: BARLEY SHOCHU OF No.1
k68: BARLEY SHOCHU OF No.2
k69: BARLEY SHOCHU OF No.3
INTEGRAL VALUE
CLASS
k67
k68
k69
0
200
400
600
800
1000
1200
1400
1600
1800
0
5
10
15
20
25
30
35
40

FIG. 57

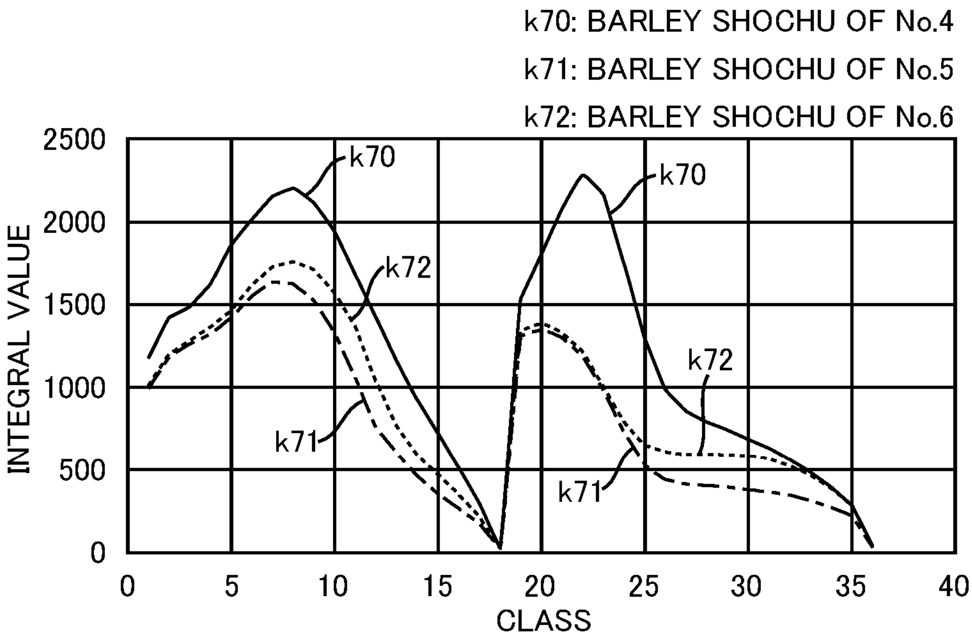
k70: BARLEY SHOCHU OF No.4
k71: BARLEY SHOCHU OF No.5
k72: BARLEY SHOCHU OF No.6
INTEGRAL VALUE
CLASS
k70
k71
k72
0
500
1000
1500
2000
2500
0
5
10
15
20
25
30
35
40

FIG. 58

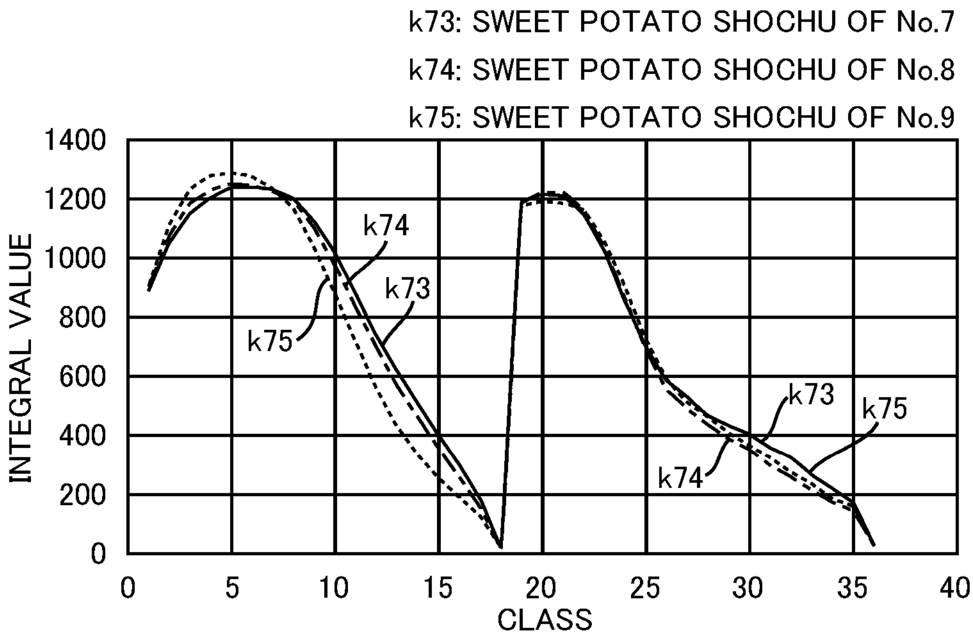
k73: SWEET POTATO SHOCHU OF No.7
k74: SWEET POTATO SHOCHU OF No.8
k75: SWEET POTATO SHOCHU OF No.9
INTEGRAL VALUE
CLASS
k73
k74
k75
0
200
400
600
800
1000
1200
1400
0
5
10
15
20
25
30
35
40

FIG. 59

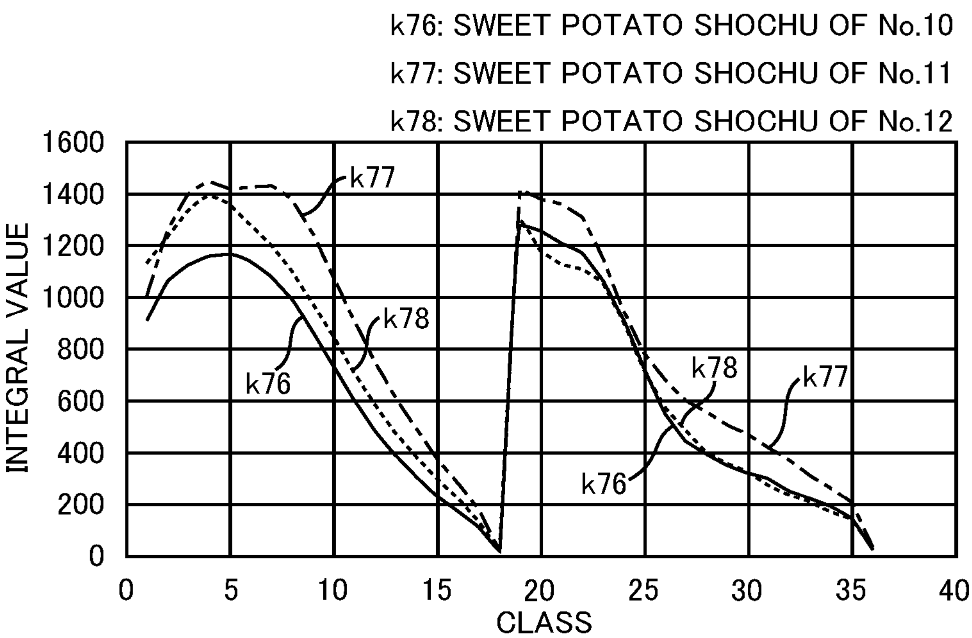
k76: SWEET POTATO SHOCHU OF No.10
k77: SWEET POTATO SHOCHU OF No.11
k78: SWEET POTATO SHOCHU OF No.12
INTEGRAL VALUE
CLASS
k76
k77
k78
0
200
400
600
800
1000
1200
1400
1600
0
5
10
15
20
25
30
35
40

FIG. 60

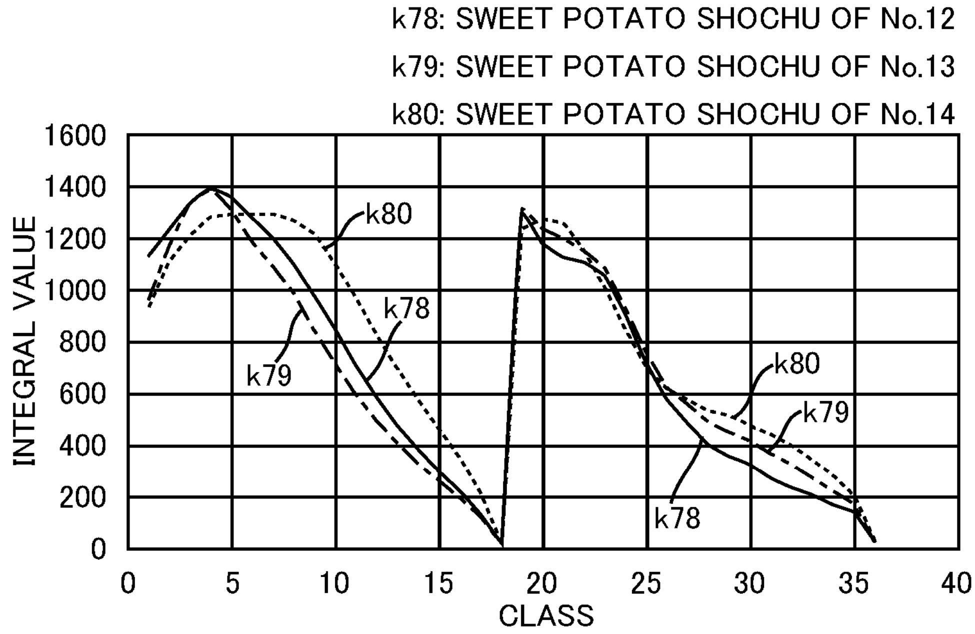
k78: SWEET POTATO SHOCHU OF No.12
k79: SWEET POTATO SHOCHU OF No.13
k80: SWEET POTATO SHOCHU OF No.14
INTEGRAL VALUE
CLASS
k78
k79
k80
0
200
400
600
800
1000
1200
1400
1600
0
5
10
15
20
25
30
35
40

FIG. 61

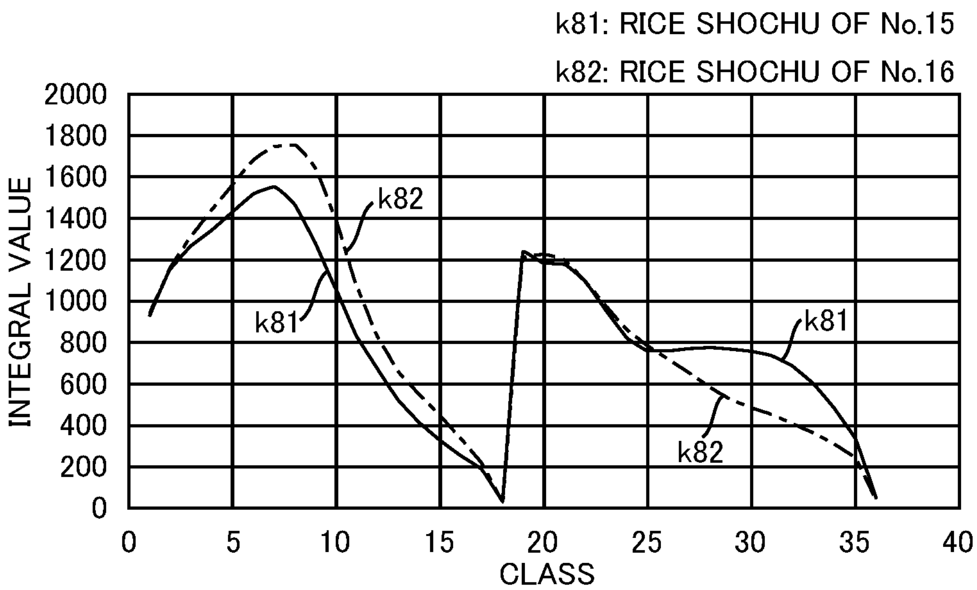
k81: RICE SHOCHU OF No.15
k82: RICE SHOCHU OF No.16
INTEGRAL VALUE
CLASS
k81
k82
0
200
400
600
800
1000
1200
1400
1600
1800
2000
0
5
10
15
20
25
30
35
40

FIG. 62

k83: SHOCHU MADE WITH SAKE LEES OF No.17
k84: BARLEY SHOCHU STORED FOR 3 YEARS, STORED IN BARRELS, AND PARTIALLY STOREDIN CHERRY BARRELS OF No.18
k85: BARLEY SHOCHU STORED FOR 15 YEARS, ALL KOJI BARLEY, STORED IN BARRELS, AND HEATED OF No.19
k86: RICE SHOCHU STORED FOR 3 YEARS AND STORED IN BARRELS OF No.20

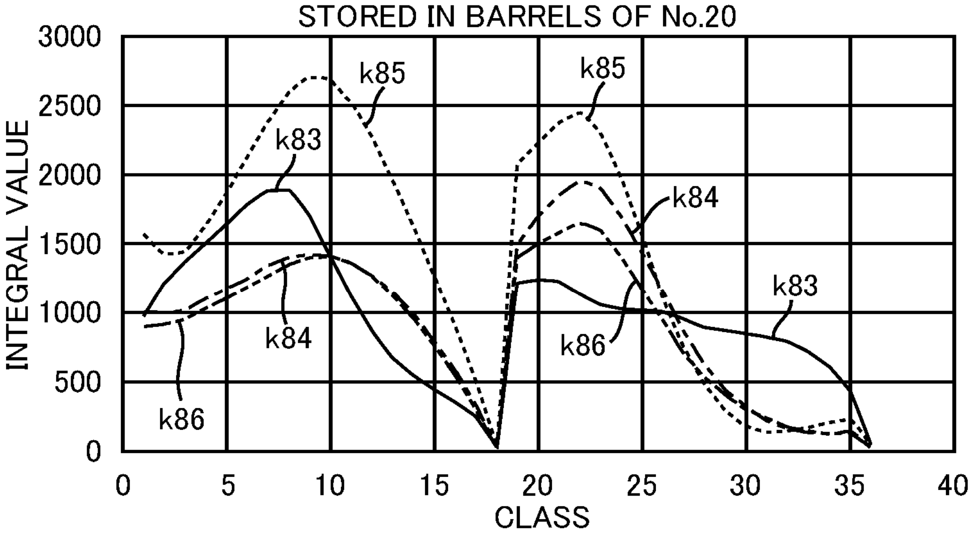

FIG. 63

| | k67 | k68 | k69 | k70 | k71 | k72 | k73 | k74 | k75 | k76 |
|---|---|---|---|---|---|---|---|---|---|---|
| k67 | | $\sigma_{DF_k67,k68}$ =25.59% | $\sigma_{DF_k67,k69}$ =8.25% | $\sigma_{DF_k67,k70}$ =20.27% | $\sigma_{DF_k67,k71}$ =5.45% | $\sigma_{DF_k67,k72}$ =16.72% | $\sigma_{DF_k67,k73}$ =16.01% | $\sigma_{DF_k67,k74}$ =15.64% | $\sigma_{DF_k67,k75}$ =20.01% | $\sigma_{DF_k67,k76}$ =22.93% |
| k68 | $\sigma_{DF_k68,k67}$ =25.59% | | $\sigma_{DF_k68,k69}$ =23.26% | $\sigma_{DF_k68,k70}$ =24.75% | $\sigma_{DF_k68,k71}$ =23.51% | $\sigma_{DF_k68,k72}$ =20.21% | $\sigma_{DF_k68,k73}$ =20.32% | $\sigma_{DF_k68,k74}$ =23.20% | $\sigma_{DF_k68,k75}$ =17.43% | $\sigma_{DF_k68,k76}$ =19.79% |
| k69 | $\sigma_{DF_k69,k67}$ =8.25% | $\sigma_{DF_k69,k68}$ =23.26% | | $\sigma_{DF_k69,k70}$ =22.97% | $\sigma_{DF_k69,k71}$ =6.44% | $\sigma_{DF_k69,k72}$ =18.48% | $\sigma_{DF_k69,k73}$ =18.13% | $\sigma_{DF_k69,k74}$ =17.33% | $\sigma_{DF_k69,k75}$ =18.26% | $\sigma_{DF_k69,k76}$ =21.52% |
| k70 | $\sigma_{DF_k70,k67}$ =20.27% | $\sigma_{DF_k70,k68}$ =24.75% | $\sigma_{DF_k70,k69}$ =22.97% | | $\sigma_{DF_k70,k71}$ =20.67% | $\sigma_{DF_k70,k72}$ =19.08% | $\sigma_{DF_k70,k73}$ =12.01% | $\sigma_{DF_k70,k74}$ =15.76% | $\sigma_{DF_k70,k75}$ =20.67% | $\sigma_{DF_k70,k76}$ =22.70% |
| k71 | $\sigma_{DF_k71,k67}$ =5.45% | $\sigma_{DF_k71,k68}$ =23.51% | $\sigma_{DF_k71,k69}$ =6.44% | $\sigma_{DF_k71,k70}$ =20.67% | | $\sigma_{DF_k71,k72}$ =14.58% | $\sigma_{DF_k71,k73}$ =16.22% | $\sigma_{DF_k71,k74}$ =17.01% | $\sigma_{DF_k71,k75}$ =20.08% | $\sigma_{DF_k71,k76}$ =23.10% |
| k72 | $\sigma_{DF_k72,k67}$ =16.72% | $\sigma_{DF_k72,k68}$ =20.21% | $\sigma_{DF_k72,k69}$ =18.48% | $\sigma_{DF_k72,k70}$ =19.08% | $\sigma_{DF_k72,k71}$ =14.58% | | $\sigma_{DF_k72,k73}$ =17.11% | $\sigma_{DF_k72,k74}$ =22.20% | $\sigma_{DF_k72,k75}$ =25.00% | $\sigma_{DF_k72,k76}$ =28.47% |
| k73 | $\sigma_{DF_k73,k67}$ =16.01% | $\sigma_{DF_k73,k68}$ =20.32% | $\sigma_{DF_k73,k69}$ =18.13% | $\sigma_{DF_k73,k70}$ =12.01% | $\sigma_{DF_k73,k71}$ =16.22% | $\sigma_{DF_k73,k72}$ =17.11% | | $\sigma_{DF_k73,k74}$ =8.09% | $\sigma_{DF_k73,k75}$ =15.19% | $\sigma_{DF_k73,k76}$ =18.00% |
| k74 | $\sigma_{DF_k74,k67}$ =15.64% | $\sigma_{DF_k74,k68}$ =23.20% | $\sigma_{DF_k74,k69}$ =17.33% | $\sigma_{DF_k74,k70}$ =15.76% | $\sigma_{DF_k74,k71}$ =17.01% | $\sigma_{DF_k74,k72}$ =22.20% | $\sigma_{DF_k74,k73}$ =8.09% | | $\sigma_{DF_k74,k75}$ =12.17% | $\sigma_{DF_k74,k76}$ =15.16% |
| k75 | $\sigma_{DF_k75,k67}$ =20.01% | $\sigma_{DF_k75,k68}$ =17.43% | $\sigma_{DF_k75,k69}$ =18.26% | $\sigma_{DF_k75,k70}$ =20.67% | $\sigma_{DF_k75,k71}$ =20.08% | $\sigma_{DF_k75,k72}$ =25.00% | $\sigma_{DF_k75,k73}$ =15.19% | $\sigma_{DF_k75,k74}$ =12.17% | | $\sigma_{DF_k75,k76}$ =7.50% |
| k76 | $\sigma_{DF_k76,k67}$ =22.93% | $\sigma_{DF_k76,k68}$ =19.79% | $\sigma_{DF_k76,k69}$ =21.52% | $\sigma_{DF_k76,k70}$ =22.70% | $\sigma_{DF_k76,k71}$ =23.10% | $\sigma_{DF_k76,k72}$ =28.47% | $\sigma_{DF_k76,k73}$ =18.00% | $\sigma_{DF_k76,k74}$ =15.16% | $\sigma_{DF_k76,k75}$ =7.50% | |

FIG. 64

| | k77 | k78 | k79 | k80 | k81 | k82 | k83 | k84 | k85 | k86 |
|---|---|---|---|---|---|---|---|---|---|---|
| k67 | $\sigma_{DF_k67,k77}$ =15.97% | $\sigma_{DF_k67,k78}$ =19.90% | $\sigma_{DF_k67,k79}$ =26.70% | $\sigma_{DF_k67,k80}$ =18.31% | $\sigma_{DF_k67,k81}$ =31.15% | $\sigma_{DF_k67,k82}$ =15.27% | $\sigma_{DF_k67,k83}$ =32.74% | $\sigma_{DF_k67,k84}$ =45.30% | $\sigma_{DF_k67,k85}$ =51.86% | $\sigma_{DF_k67,k86}$ =41.62% |
| k68 | $\sigma_{DF_k68,k77}$ =15.14% | $\sigma_{DF_k68,k78}$ =23.67% | $\sigma_{DF_k68,k79}$ =15.53% | $\sigma_{DF_k68,k80}$ =19.08% | $\sigma_{DF_k68,k81}$ =13.31% | $\sigma_{DF_k68,k82}$ =17.73% | $\sigma_{DF_k68,k83}$ =17.51% | $\sigma_{DF_k68,k84}$ =53.02% | $\sigma_{DF_k68,k85}$ =66.91% | $\sigma_{DF_k68,k86}$ =50.87% |
| k69 | $\sigma_{DF_k69,k77}$ =16.29% | $\sigma_{DF_k69,k78}$ =19.31% | $\sigma_{DF_k69,k79}$ =24.59% | $\sigma_{DF_k69,k80}$ =20.08% | $\sigma_{DF_k69,k81}$ =31.09% | $\sigma_{DF_k69,k82}$ =15.48% | $\sigma_{DF_k69,k83}$ =31.63% | $\sigma_{DF_k69,k84}$ =47.82% | $\sigma_{DF_k69,k85}$ =54.25% | $\sigma_{DF_k69,k86}$ =44.80% |
| k70 | $\sigma_{DF_k70,k77}$ =16.40% | $\sigma_{DF_k70,k78}$ =23.24% | $\sigma_{DF_k70,k79}$ =24.59% | $\sigma_{DF_k70,k80}$ =13.11% | $\sigma_{DF_k70,k81}$ =32.29% | $\sigma_{DF_k70,k82}$ =17.16% | $\sigma_{DF_k70,k83}$ =31.49% | $\sigma_{DF_k70,k84}$ =34.80% | $\sigma_{DF_k70,k85}$ =50.65% | $\sigma_{DF_k70,k86}$ =30.87% |
| k71 | $\sigma_{DF_k71,k77}$ =15.65% | $\sigma_{DF_k71,k78}$ =20.95% | $\sigma_{DF_k71,k79}$ =25.75% | $\sigma_{DF_k71,k80}$ =17.16% | $\sigma_{DF_k71,k81}$ =30.76% | $\sigma_{DF_k71,k82}$ =14.20% | $\sigma_{DF_k71,k83}$ =30.97% | $\sigma_{DF_k71,k84}$ =47.48% | $\sigma_{DF_k71,k85}$ =54.74% | $\sigma_{DF_k71,k86}$ =43.93% |
| k72 | $\sigma_{DF_k72,k77}$ =17.84% | $\sigma_{DF_k72,k78}$ =27.96% | $\sigma_{DF_k72,k79}$ =27.94% | $\sigma_{DF_k72,k80}$ =13.04% | $\sigma_{DF_k72,k81}$ =22.85% | $\sigma_{DF_k72,k82}$ =11.43% | $\sigma_{DF_k72,k83}$ =21.66% | $\sigma_{DF_k72,k84}$ =49.60% | $\sigma_{DF_k72,k85}$ =60.61% | $\sigma_{DF_k72,k86}$ =45.44% |
| k73 | $\sigma_{DF_k73,k77}$ =7.23% | $\sigma_{DF_k73,k78}$ =15.52% | $\sigma_{DF_k73,k79}$ =19.14% | $\sigma_{DF_k73,k80}$ =7.07% | $\sigma_{DF_k73,k81}$ =29.25% | $\sigma_{DF_k73,k82}$ =12.71% | $\sigma_{DF_k73,k83}$ =28.78% | $\sigma_{DF_k73,k84}$ =38.53% | $\sigma_{DF_k73,k85}$ =52.77% | $\sigma_{DF_k73,k86}$ =35.13% |
| k74 | $\sigma_{DF_k74,k77}$ =9.41% | $\sigma_{DF_k74,k78}$ =9.87% | $\sigma_{DF_k74,k79}$ =18.88% | $\sigma_{DF_k74,k80}$ =14.48% | $\sigma_{DF_k74,k81}$ =33.17% | $\sigma_{DF_k74,k82}$ =16.68% | $\sigma_{DF_k74,k83}$ =33.06% | $\sigma_{DF_k74,k84}$ =35.95% | $\sigma_{DF_k74,k85}$ =49.20% | $\sigma_{DF_k74,k86}$ =33.07% |
| k75 | $\sigma_{DF_k75,k77}$ =10.03% | $\sigma_{DF_k75,k78}$ =9.61% | $\sigma_{DF_k75,k79}$ =10.22% | $\sigma_{DF_k75,k80}$ =19.38% | $\sigma_{DF_k75,k81}$ =28.76% | $\sigma_{DF_k75,k82}$ =19.18% | $\sigma_{DF_k75,k83}$ =30.87% | $\sigma_{DF_k75,k84}$ =43.13% | $\sigma_{DF_k75,k85}$ =55.56% | $\sigma_{DF_k75,k86}$ =41.38% |
| k76 | $\sigma_{DF_k76,k77}$ =13.10% | $\sigma_{DF_k76,k78}$ =10.87% | $\sigma_{DF_k76,k79}$ =9.37% | $\sigma_{DF_k76,k80}$ =22.31% | $\sigma_{DF_k76,k81}$ =31.09% | $\sigma_{DF_k76,k82}$ =23.82% | $\sigma_{DF_k76,k83}$ =33.81% | $\sigma_{DF_k76,k84}$ =43.64% | $\sigma_{DF_k76,k85}$ =55.20% | $\sigma_{DF_k76,k86}$ =42.22% |

FIG. 65

| | k77 | k78 | k79 | k80 | k81 | k82 | k83 | k84 | k85 | k86 |
|---|---|---|---|---|---|---|---|---|---|---|
| k77 | | $\sigma_{DF_k77,k78}$ =13.10% | $\sigma_{DF_k77,k79}$ =13.57% | $\sigma_{DF_k77,k80}$ =10.67% | $\sigma_{DF_k77,k81}$ =25.84% | $\sigma_{DF_k77,k82}$ =12.81% | $\sigma_{DF_k77,k83}$ =27.21% | $\sigma_{DF_k77,k84}$ =43.02% | $\sigma_{DF_k77,k85}$ =56.84% | $\sigma_{DF_k77,k86}$ =40.15% |
| k78 | $\sigma_{DF_k78,k77}$ =13.10% | | $\sigma_{DF_k78,k79}$ =15.15% | $\sigma_{DF_k78,k80}$ =20.90% | $\sigma_{DF_k78,k81}$ =34.53% | $\sigma_{DF_k78,k82}$ =21.82% | $\sigma_{DF_k78,k83}$ =35.80% | $\sigma_{DF_k78,k84}$ =39.95% | $\sigma_{DF_k78,k85}$ =51.06% | $\sigma_{DF_k78,k86}$ =38.11% |
| k79 | $\sigma_{DF_k79,k77}$ =13.57% | $\sigma_{DF_k79,k78}$ =15.15% | | $\sigma_{DF_k79,k80}$ =21.33% | $\sigma_{DF_k79,k81}$ =26.69% | $\sigma_{DF_k79,k82}$ =23.54% | $\sigma_{DF_k79,k83}$ =30.52% | $\sigma_{DF_k79,k84}$ =48.18% | $\sigma_{DF_k79,k85}$ =61.62% | $\sigma_{DF_k79,k86}$ =46.77% |
| k80 | $\sigma_{DF_k80,k77}$ =10.67% | $\sigma_{DF_k80,k78}$ =20.90% | $\sigma_{DF_k80,k79}$ =21.33% | | $\sigma_{DF_k80,k81}$ =26.04% | $\sigma_{DF_k80,k82}$ =11.54% | $\sigma_{DF_k80,k83}$ =25.48% | $\sigma_{DF_k80,k84}$ =42.15% | $\sigma_{DF_k80,k85}$ =56.56% | $\sigma_{DF_k80,k86}$ =38.50% |
| k81 | $\sigma_{DF_k81,k77}$ =25.84% | $\sigma_{DF_k81,k78}$ =34.53% | $\sigma_{DF_k81,k79}$ =26.69% | $\sigma_{DF_k81,k80}$ =26.04% | | $\sigma_{DF_k81,k82}$ =24.17% | $\sigma_{DF_k81,k83}$ =9.32% | $\sigma_{DF_k81,k84}$ =60.57% | $\sigma_{DF_k81,k85}$ =74.26% | $\sigma_{DF_k81,k86}$ =58.11% |
| k82 | $\sigma_{DF_k82,k77}$ =12.81% | $\sigma_{DF_k82,k78}$ =21.82% | $\sigma_{DF_k82,k79}$ =23.54% | $\sigma_{DF_k82,k80}$ =11.54% | $\sigma_{DF_k82,k81}$ =24.17% | | $\sigma_{DF_k82,k83}$ =22.28% | $\sigma_{DF_k82,k84}$ =45.26% | $\sigma_{DF_k82,k85}$ =56.91% | $\sigma_{DF_k82,k86}$ =41.73% |
| k83 | $\sigma_{DF_k83,k77}$ =27.21% | $\sigma_{DF_k83,k78}$ =35.80% | $\sigma_{DF_k83,k79}$ =30.52% | $\sigma_{DF_k83,k80}$ =25.48% | $\sigma_{DF_k83,k81}$ =9.32% | $\sigma_{DF_k83,k82}$ =22.28% | | $\sigma_{DF_k83,k84}$ =57.72% | $\sigma_{DF_k83,k85}$ =71.96% | $\sigma_{DF_k83,k86}$ =54.83% |
| k84 | $\sigma_{DF_k84,k77}$ =43.02% | $\sigma_{DF_k84,k78}$ =39.95% | $\sigma_{DF_k84,k79}$ =48.18% | $\sigma_{DF_k84,k80}$ =42.15% | $\sigma_{DF_k84,k81}$ =60.57% | $\sigma_{DF_k84,k82}$ =45.26% | $\sigma_{DF_k84,k83}$ =57.72% | | $\sigma_{DF_k84,k85}$ =31.18% | $\sigma_{DF_k84,k86}$ =7.62% |
| k85 | $\sigma_{DF_k85,k77}$ =56.84% | $\sigma_{DF_k85,k78}$ =51.06% | $\sigma_{DF_k85,k79}$ =61.62% | $\sigma_{DF_k85,k80}$ =56.56% | $\sigma_{DF_k85,k81}$ =74.26% | $\sigma_{DF_k85,k82}$ =56.91% | $\sigma_{DF_k85,k83}$ =71.96% | $\sigma_{DF_k85,k84}$ =31.18% | | $\sigma_{DF_k85,k86}$ =30.64% |
| k86 | $\sigma_{DF_k86,k77}$ =40.15% | $\sigma_{DF_k86,k78}$ =38.11% | $\sigma_{DF_k86,k79}$ =46.77% | $\sigma_{DF_k86,k80}$ =38.50% | $\sigma_{DF_k86,k81}$ =58.11% | $\sigma_{DF_k86,k82}$ =41.73% | $\sigma_{DF_k86,k83}$ =54.83% | $\sigma_{DF_k86,k84}$ =7.62% | $\sigma_{DF_k86,k85}$ =30.64% | |

FIG. 66

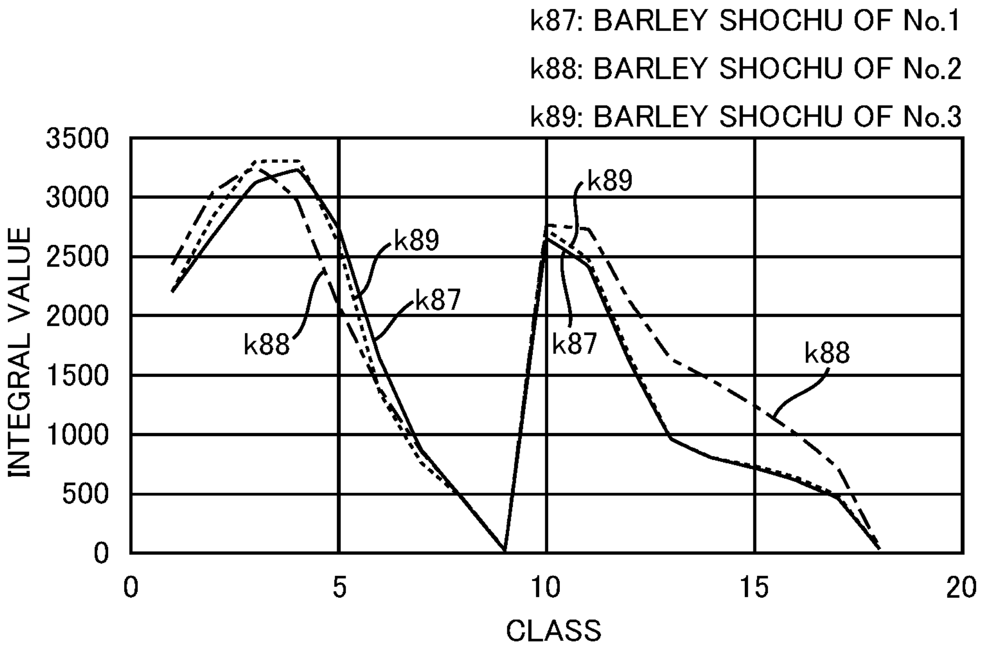
k87: BARLEY SHOCHU OF No.1
k88: BARLEY SHOCHU OF No.2
k89: BARLEY SHOCHU OF No.3
INTEGRAL VALUE
3500
3000
2500
2000
1500
1000
500
0
0
5
10
15
20
CLASS
k89
k87
k88
k89
k87
k88

FIG. 67

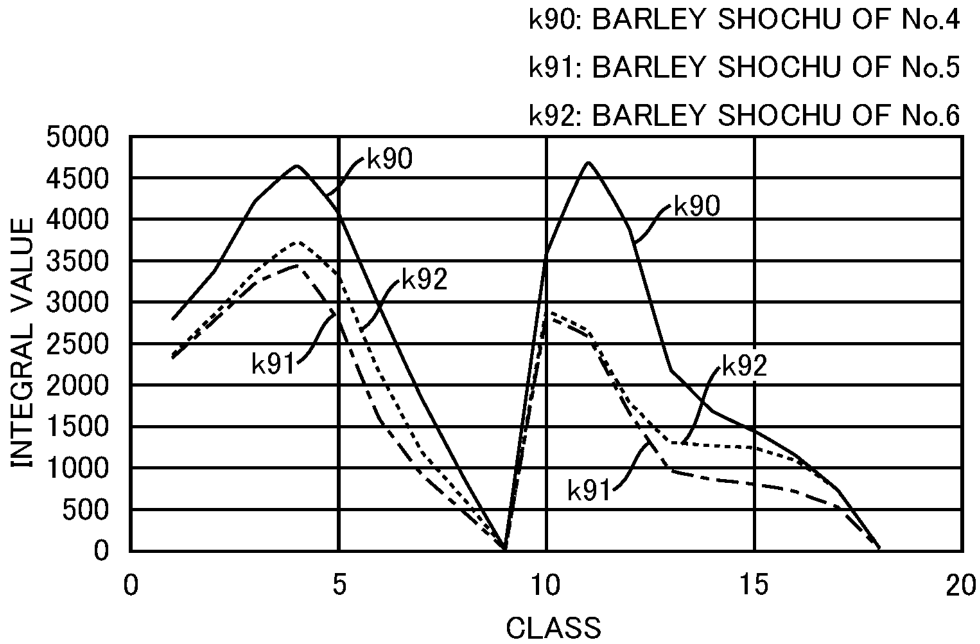
k90: BARLEY SHOCHU OF No.4
k91: BARLEY SHOCHU OF No.5
k92: BARLEY SHOCHU OF No.6
INTEGRAL VALUE
5000
4500
4000
3500
3000
2500
2000
1500
1000
500
0
0
5
10
15
20
CLASS
k90
k92
k91
k90
k92
k91

FIG. 68

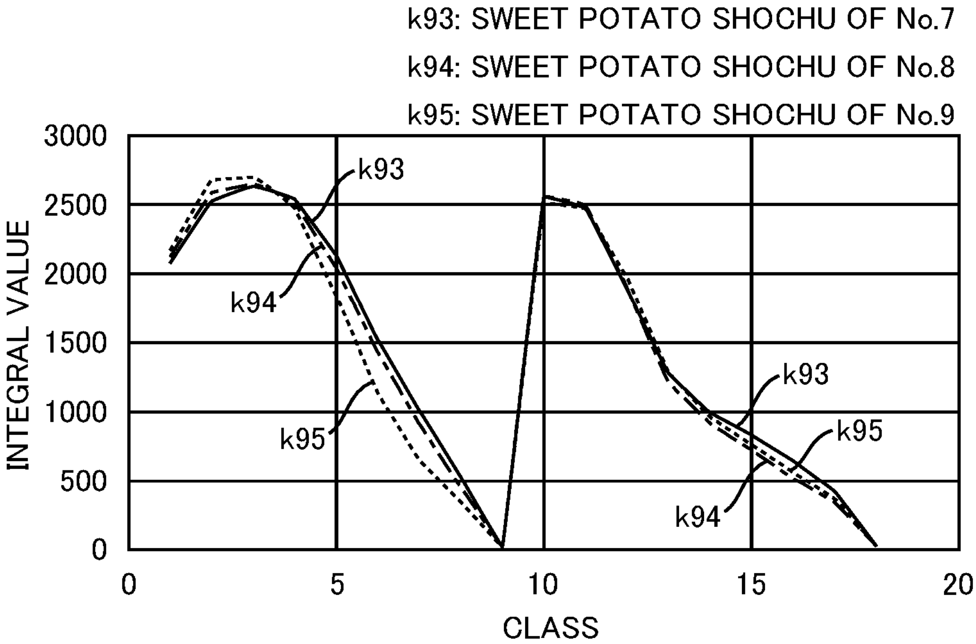
k93: SWEET POTATO SHOCHU OF No.7
k94: SWEET POTATO SHOCHU OF No.8
k95: SWEET POTATO SHOCHU OF No.9
INTEGRAL VALUE
3000
2500
2000
1500
1000
500
0
0
5
10
15
20
CLASS
k93
k94
k95
k93
k95
k94

FIG. 69

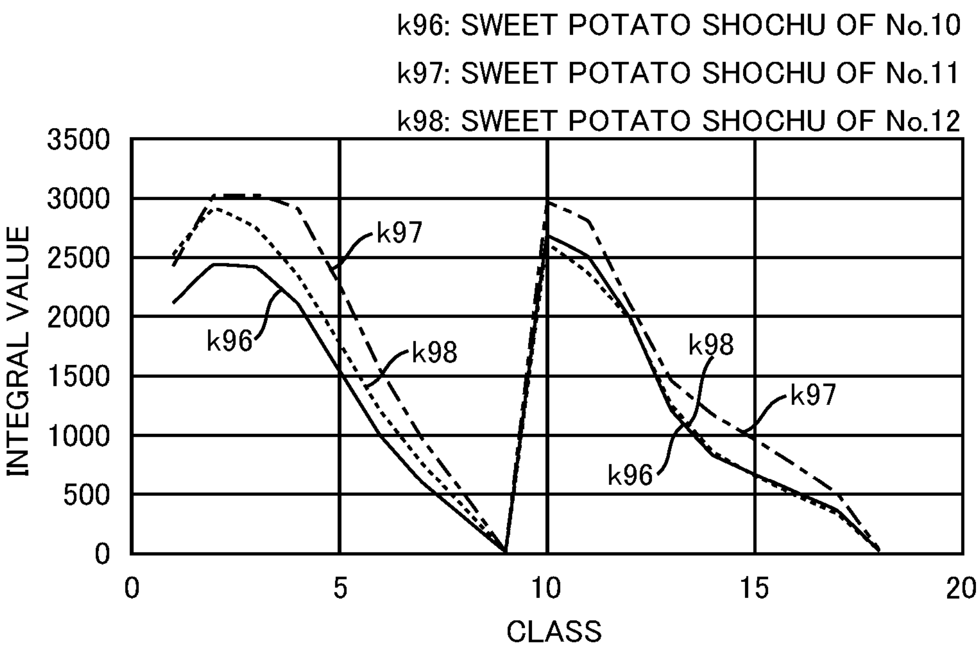
k96: SWEET POTATO SHOCHU OF No.10
k97: SWEET POTATO SHOCHU OF No.11
k98: SWEET POTATO SHOCHU OF No.12
INTEGRAL VALUE
3500
3000
2500
2000
1500
1000
500
0
0
5
10
15
20
CLASS
k97
k96
k98
k98
k97
k96

FIG. 70

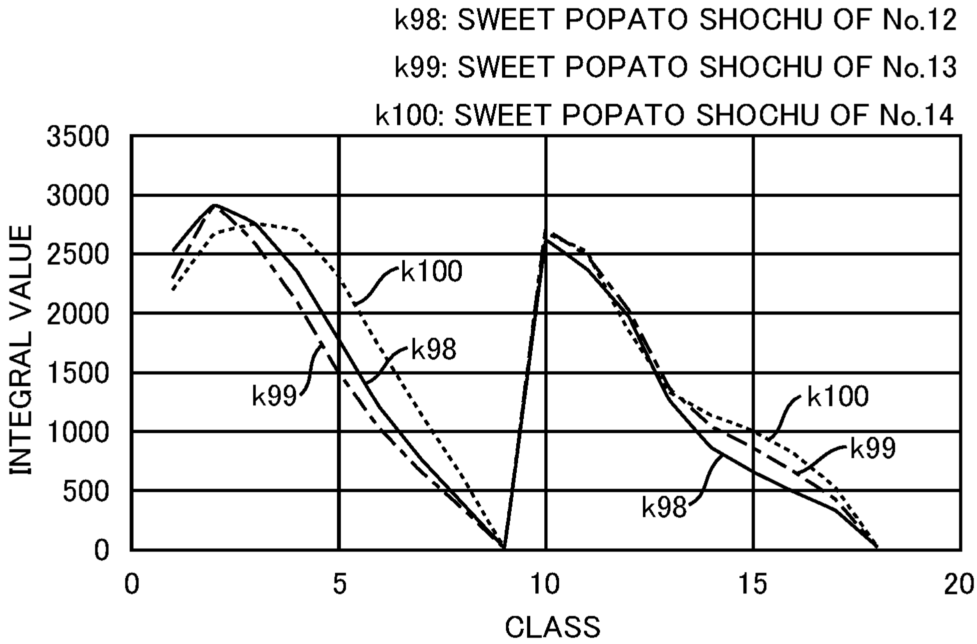
k98: SWEET POPATO SHOCHU OF No.12
k99: SWEET POPATO SHOCHU OF No.13
k100: SWEET POPATO SHOCHU OF No.14
INTEGRAL VALUE
CLASS
k100
k98
k99
k100
k99
k98
0
500
1000
1500
2000
2500
3000
3500
0
5
10
15
20

FIG. 71

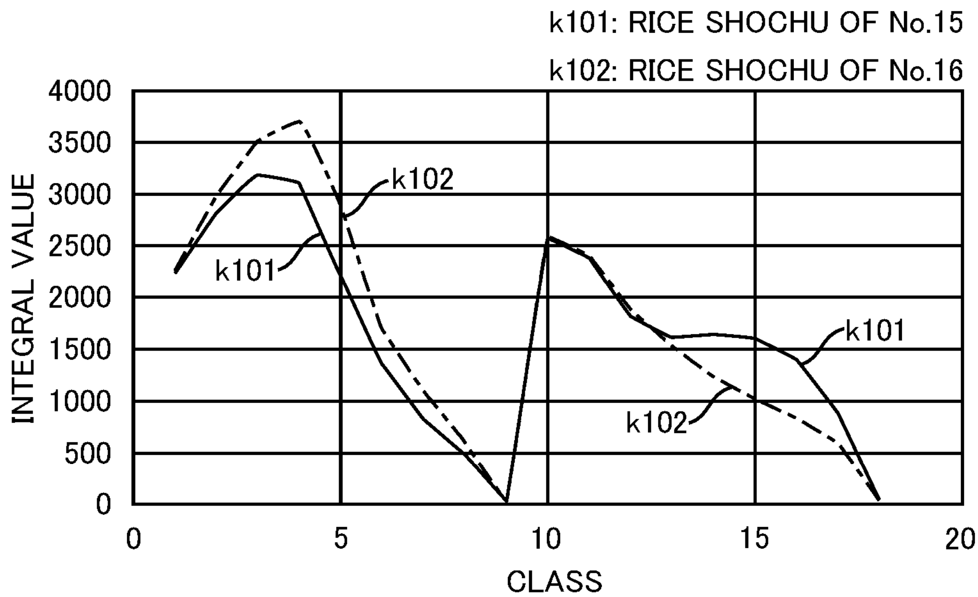
k101: RICE SHOCHU OF No.15
k102: RICE SHOCHU OF No.16
INTEGRAL VALUE
CLASS
k102
k101
k101
k102
0
500
1000
1500
2000
2500
3000
3500
4000
0
5
10
15
20

FIG. 72

k103: SHOCHU MADE WITH SAKE LEES OF No.17
k104: BARLEY SHOCHU STORED FOR THREE YEARS IN ITS ENTIRETY, STORED IN A BARREL, AND PARTIALLY STORED IN A CHERRY WOOD BARREL OF No.18
k105: BARLEY SHOCHU STORED FOR 15 YEARS IN ITS ENTIRETY, ALL KOJI BARLEY, STORED IN A BARREL, AND HEATED OF No.19
k106: RICE SHOCHU STORED FOR THREE YEARS IN ITS ENTIRELY, AND STORED IN A BARREL OF No.20

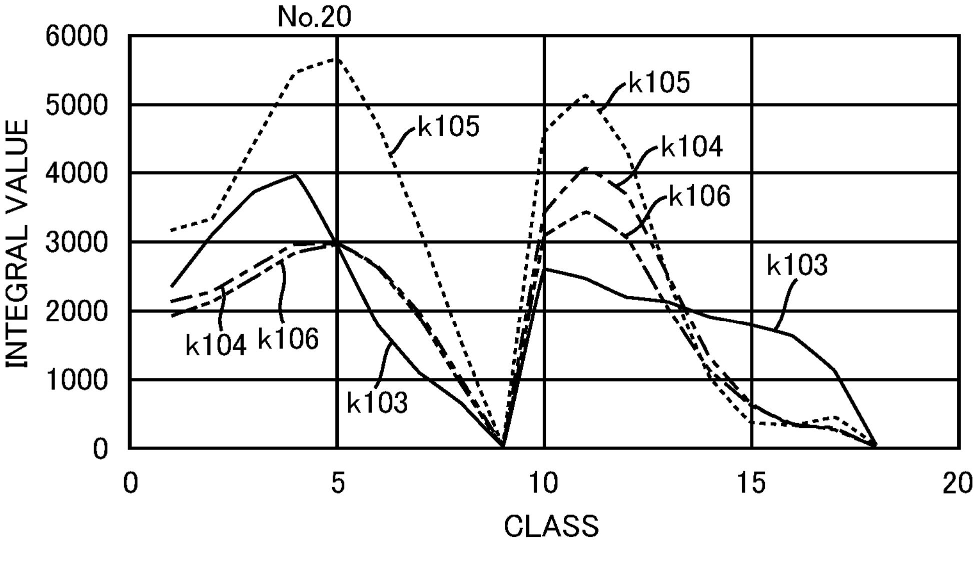

FIG. 73

| | k87 | k88 | k89 | k90 | k91 | k92 | k93 | k94 | k95 | k96 |
|---|---|---|---|---|---|---|---|---|---|---|
| k87 | | $\sigma_{DF_k87,k88}$ =24.89% | $\sigma_{DF_k87,k89}$ =9.20% | $\sigma_{DF_k87,k90}$ =19.91% | $\sigma_{DF_k87,k91}$ =5.77% | $\sigma_{DF_k87,k92}$ =16.29% | $\sigma_{DF_k87,k93}$ =15.54% | $\sigma_{DF_k87,k94}$ =14.97% | $\sigma_{DF_k87,k95}$ =19.57% | $\sigma_{DF_k87,k96}$ =22.05% |
| k88 | $\sigma_{DF_k88,k87}$ =24.89% | | $\sigma_{DF_k88,k89}$ =22.43% | $\sigma_{DF_k88,k90}$ =24.75% | $\sigma_{DF_k88,k91}$ =22.70% | $\sigma_{DF_k88,k92}$ =19.51% | $\sigma_{DF_k88,k93}$ =20.24% | $\sigma_{DF_k88,k94}$ =22.69% | $\sigma_{DF_k88,k95}$ =17.29% | $\sigma_{DF_k88,k96}$ =19.48% |
| k89 | $\sigma_{DF_k89,k87}$ =9.20% | $\sigma_{DF_k89,k88}$ =22.43% | | $\sigma_{DF_k89,k90}$ =23.19% | $\sigma_{DF_k89,k91}$ =6.58% | $\sigma_{DF_k89,k92}$ =18.14% | $\sigma_{DF_k89,k93}$ =18.38% | $\sigma_{DF_k89,k94}$ =17.14% | $\sigma_{DF_k89,k95}$ =18.02% | $\sigma_{DF_k89,k96}$ =21.39% |
| k90 | $\sigma_{DF_k90,k87}$ =19.91% | $\sigma_{DF_k90,k88}$ =24.75% | $\sigma_{DF_k90,k89}$ =23.19% | | $\sigma_{DF_k90,k91}$ =20.33% | $\sigma_{DF_k90,k92}$ =18.72% | $\sigma_{DF_k90,k93}$ =11.36% | $\sigma_{DF_k90,k94}$ =15.25% | $\sigma_{DF_k90,k95}$ =19.93% | $\sigma_{DF_k90,k96}$ =21.84% |
| k91 | $\sigma_{DF_k91,k87}$ =5.77% | $\sigma_{DF_k91,k88}$ =22.70% | $\sigma_{DF_k91,k89}$ =6.58% | $\sigma_{DF_k91,k90}$ =20.33% | | $\sigma_{DF_k91,k92}$ =14.10% | $\sigma_{DF_k91,k93}$ =15.84% | $\sigma_{DF_k91,k94}$ =16.30% | $\sigma_{DF_k91,k95}$ =19.33% | $\sigma_{DF_k91,k96}$ =22.32% |
| k92 | $\sigma_{DF_k92,k87}$ =16.29% | $\sigma_{DF_k92,k88}$ =19.51% | $\sigma_{DF_k92,k89}$ =18.14% | $\sigma_{DF_k92,k90}$ =18.72% | $\sigma_{DF_k92,k91}$ =14.10% | | $\sigma_{DF_k92,k93}$ =16.53% | $\sigma_{DF_k92,k94}$ =21.32% | $\sigma_{DF_k92,k95}$ =24.10% | $\sigma_{DF_k92,k96}$ =27.45% |
| k93 | $\sigma_{DF_k93,k87}$ =15.54% | $\sigma_{DF_k93,k88}$ =20.24% | $\sigma_{DF_k93,k89}$ =18.38% | $\sigma_{DF_k93,k90}$ =11.36% | $\sigma_{DF_k93,k91}$ =15.84% | $\sigma_{DF_k93,k92}$ =16.53% | | $\sigma_{DF_k93,k94}$ =7.92% | $\sigma_{DF_k93,k95}$ =14.82% | $\sigma_{DF_k93,k96}$ =17.29% |
| k94 | $\sigma_{DF_k94,k87}$ =14.97% | $\sigma_{DF_k94,k88}$ =22.69% | $\sigma_{DF_k94,k89}$ =17.14% | $\sigma_{DF_k94,k90}$ =15.25% | $\sigma_{DF_k94,k91}$ =16.30% | $\sigma_{DF_k94,k92}$ =21.32% | $\sigma_{DF_k94,k93}$ =7.92% | | $\sigma_{DF_k94,k95}$ =11.82% | $\sigma_{DF_k94,k96}$ =14.63% |
| k95 | $\sigma_{DF_k95,k87}$ =19.57% | $\sigma_{DF_k95,k88}$ =17.29% | $\sigma_{DF_k95,k89}$ =18.02% | $\sigma_{DF_k95,k90}$ =19.93% | $\sigma_{DF_k95,k91}$ =19.33% | $\sigma_{DF_k95,k92}$ =24.10% | $\sigma_{DF_k95,k93}$ =14.82% | $\sigma_{DF_k95,k94}$ =11.82% | | $\sigma_{DF_k95,k96}$ =7.56% |
| k96 | $\sigma_{DF_k96,k87}$ =22.05% | $\sigma_{DF_k96,k88}$ =19.48% | $\sigma_{DF_k96,k89}$ =21.39% | $\sigma_{DF_k96,k90}$ =21.84% | $\sigma_{DF_k96,k91}$ =22.32% | $\sigma_{DF_k96,k92}$ =27.45% | $\sigma_{DF_k96,k93}$ =17.29% | $\sigma_{DF_k96,k94}$ =14.63% | $\sigma_{DF_k96,k95}$ =7.56% | |

FIG. 74

| | k97 | k98 | k99 | k100 | k101 | k102 | k103 | k104 | k105 | k106 |
|---|---|---|---|---|---|---|---|---|---|---|
| k87 | $\sigma_{DF_k87,k97}$ =15.25% | $\sigma_{DF_k87,k98}$ =19.03% | $\sigma_{DF_k87,k99}$ =25.98% | $\sigma_{DF_k87,k100}$ =17.99% | $\sigma_{DF_k87,k101}$ =32.28% | $\sigma_{DF_k87,k102}$ =14.83% | $\sigma_{DF_k87,k103}$ =31.83% | $\sigma_{DF_k87,k104}$ =43.88% | $\sigma_{DF_k87,k105}$ =49.75% | $\sigma_{DF_k87,k106}$ =40.23% |
| k88 | $\sigma_{DF_k88,k97}$ =15.12% | $\sigma_{DF_k88,k98}$ =22.89% | $\sigma_{DF_k88,k99}$ =15.79% | $\sigma_{DF_k88,k100}$ =19.11% | $\sigma_{DF_k88,k101}$ =12.75% | $\sigma_{DF_k88,k102}$ =17.13% | $\sigma_{DF_k88,k103}$ =16.87% | $\sigma_{DF_k88,k104}$ =51.39% | $\sigma_{DF_k88,k105}$ =64.34% | $\sigma_{DF_k88,k106}$ =49.37% |
| k89 | $\sigma_{DF_k89,k97}$ =16.38% | $\sigma_{DF_k89,k98}$ =18.66% | $\sigma_{DF_k89,k99}$ =24.34% | $\sigma_{DF_k89,k100}$ =20.09% | $\sigma_{DF_k89,k101}$ =30.07% | $\sigma_{DF_k89,k102}$ =14.79% | $\sigma_{DF_k89,k103}$ =30.59% | $\sigma_{DF_k89,k104}$ =46.02% | $\sigma_{DF_k89,k105}$ =51.63% | $\sigma_{DF_k89,k106}$ =43.23% |
| k90 | $\sigma_{DF_k90,k97}$ =15.94% | $\sigma_{DF_k90,k98}$ =22.26% | $\sigma_{DF_k90,k99}$ =23.50% | $\sigma_{DF_k90,k100}$ =12.42% | $\sigma_{DF_k90,k101}$ =31.97% | $\sigma_{DF_k90,k102}$ =17.34% | $\sigma_{DF_k90,k103}$ =31.44% | $\sigma_{DF_k90,k104}$ =33.52% | $\sigma_{DF_k90,k105}$ =48.87% | $\sigma_{DF_k90,k106}$ =29.66% |
| k91 | $\sigma_{DF_k91,k97}$ =15.14% | $\sigma_{DF_k91,k98}$ =19.87% | $\sigma_{DF_k91,k99}$ =24.93% | $\sigma_{DF_k91,k100}$ =16.74% | $\sigma_{DF_k91,k101}$ =29.81% | $\sigma_{DF_k91,k102}$ =13.41% | $\sigma_{DF_k91,k103}$ =30.00% | $\sigma_{DF_k91,k104}$ =45.67% | $\sigma_{DF_k91,k105}$ =52.26% | $\sigma_{DF_k91,k106}$ =42.27% |
| k92 | $\sigma_{DF_k92,k97}$ =17.19% | $\sigma_{DF_k92,k98}$ =26.76% | $\sigma_{DF_k92,k99}$ =27.02% | $\sigma_{DF_k92,k100}$ =12.70% | $\sigma_{DF_k92,k101}$ =22.23% | $\sigma_{DF_k92,k102}$ =10.76% | $\sigma_{DF_k92,k103}$ =21.10% | $\sigma_{DF_k92,k104}$ =47.78% | $\sigma_{DF_k92,k105}$ =58.15% | $\sigma_{DF_k92,k106}$ =43.78% |
| k93 | $\sigma_{DF_k93,k97}$ =7.10% | $\sigma_{DF_k93,k98}$ =14.94% | $\sigma_{DF_k93,k99}$ =18.56% | $\sigma_{DF_k93,k100}$ =6.89% | $\sigma_{DF_k93,k101}$ =28.80% | $\sigma_{DF_k93,k102}$ =12.86% | $\sigma_{DF_k93,k103}$ =28.39% | $\sigma_{DF_k93,k104}$ =37.22% | $\sigma_{DF_k93,k105}$ =50.64% | $\sigma_{DF_k93,k106}$ =33.90% |
| k94 | $\sigma_{DF_k94,k97}$ =9.11% | $\sigma_{DF_k94,k98}$ =9.26% | $\sigma_{DF_k94,k99}$ =18.36% | $\sigma_{DF_k94,k100}$ =13.99% | $\sigma_{DF_k94,k101}$ =32.32% | $\sigma_{DF_k94,k102}$ =16.23% | $\sigma_{DF_k94,k103}$ =32.27% | $\sigma_{DF_k94,k104}$ =34.63% | $\sigma_{DF_k94,k105}$ =47.02% | $\sigma_{DF_k94,k106}$ =31.84% |
| k95 | $\sigma_{DF_k95,k97}$ =10.03% | $\sigma_{DF_k95,k98}$ =8.97% | $\sigma_{DF_k95,k99}$ =9.82% | $\sigma_{DF_k95,k100}$ =18.63% | $\sigma_{DF_k95,k101}$ =28.15% | $\sigma_{DF_k95,k102}$ =18.68% | $\sigma_{DF_k95,k103}$ =30.25% | $\sigma_{DF_k95,k104}$ =41.41% | $\sigma_{DF_k95,k105}$ =53.19% | $\sigma_{DF_k95,k106}$ =39.82% |
| k96 | $\sigma_{DF_k96,k97}$ =12.48% | $\sigma_{DF_k96,k98}$ =10.59% | $\sigma_{DF_k96,k99}$ =9.25% | $\sigma_{DF_k96,k100}$ =21.54% | $\sigma_{DF_k96,k101}$ =30.34% | $\sigma_{DF_k96,k102}$ =23.21% | $\sigma_{DF_k96,k103}$ =32.98% | $\sigma_{DF_k96,k104}$ =42.06% | $\sigma_{DF_k96,k105}$ =52.93% | $\sigma_{DF_k96,k106}$ =40.70% |

FIG. 75

| | k97 | k98 | k99 | k100 | k101 | k102 | k103 | k104 | k105 | k106 |
|---|---|---|---|---|---|---|---|---|---|---|
| k97 | | $\sigma_{DF_k97,k98}$ =12.52% | $\sigma_{DF_k97,k99}$ =13.44% | $\sigma_{DF_k97,k100}$ =10.60% | $\sigma_{DF_k97,k101}$ =25.36% | $\sigma_{DF_k97,k102}$ =12.69% | $\sigma_{DF_k97,k103}$ =26.67% | $\sigma_{DF_k97,k104}$ =41.59% | $\sigma_{DF_k97,k105}$ =54.57% | $\sigma_{DF_k97,k106}$ =38.80% |
| k98 | $\sigma_{DF_k98,k97}$ =12.52% | | $\sigma_{DF_k98,k99}$ =14.74% | $\sigma_{DF_k98,k100}$ =20.02% | $\sigma_{DF_k98,k101}$ =33.46% | $\sigma_{DF_k98,k102}$ =20.92% | $\sigma_{DF_k98,k103}$ =34.73% | $\sigma_{DF_k98,k104}$ =38.39% | $\sigma_{DF_k98,k105}$ =48.83% | $\sigma_{DF_k98,k106}$ =36.64% |
| k99 | $\sigma_{DF_k99,k97}$ =13.44% | $\sigma_{DF_k99,k98}$ =14.74% | | $\sigma_{DF_k99,k100}$ =20.53% | $\sigma_{DF_k99,k101}$ =26.38% | $\sigma_{DF_k99,k102}$ =23.11% | $\sigma_{DF_k99,k103}$ =30.09% | $\sigma_{DF_k99,k104}$ =46.31% | $\sigma_{DF_k99,k105}$ =59.16% | $\sigma_{DF_k99,k106}$ =45.04% |
| k100 | $\sigma_{DF_k100,k97}$ =10.60% | $\sigma_{DF_k100,k98}$ =20.02% | $\sigma_{DF_k100,k99}$ =20.53% | | $\sigma_{DF_k100,k101}$ =25.83% | $\sigma_{DF_k100,k102}$ =11.77% | $\sigma_{DF_k100,k103}$ =25.39% | $\sigma_{DF_k100,k104}$ =40.48% | $\sigma_{DF_k100,k105}$ =54.20% | $\sigma_{DF_k100,k106}$ =37.00% |
| k101 | $\sigma_{DF_k101,k97}$ =25.36% | $\sigma_{DF_k101,k98}$ =33.46% | $\sigma_{DF_k101,k99}$ =26.38% | $\sigma_{DF_k101,k100}$ =25.83% | | $\sigma_{DF_k101,k102}$ =23.43% | $\sigma_{DF_k101,k103}$ =8.84% | $\sigma_{DF_k101,k104}$ =58.74% | $\sigma_{DF_k101,k105}$ =71.56% | $\sigma_{DF_k101,k106}$ =56.43% |
| k102 | $\sigma_{DF_k102,k97}$ =12.69% | $\sigma_{DF_k102,k98}$ =20.92% | $\sigma_{DF_k102,k99}$ =23.11% | $\sigma_{DF_k102,k100}$ =11.77% | $\sigma_{DF_k102,k101}$ =23.43% | | $\sigma_{DF_k102,k103}$ =21.76% | $\sigma_{DF_k102,k104}$ =43.68% | $\sigma_{DF_k102,k105}$ =54.49% | $\sigma_{DF_k102,k106}$ =40.33% |
| k103 | $\sigma_{DF_k103,k97}$ =26.67% | $\sigma_{DF_k103,k98}$ =34.73% | $\sigma_{DF_k103,k99}$ =30.09% | $\sigma_{DF_k103,k100}$ =25.39% | $\sigma_{DF_k103,k101}$ =8.84% | $\sigma_{DF_k103,k102}$ =21.76% | | $\sigma_{DF_k103,k104}$ =56.20% | $\sigma_{DF_k103,k105}$ =69.53% | $\sigma_{DF_k103,k106}$ =53.43% |
| k104 | $\sigma_{DF_k104,k97}$ =41.59% | $\sigma_{DF_k104,k98}$ =38.39% | $\sigma_{DF_k104,k99}$ =46.31% | $\sigma_{DF_k104,k100}$ =40.48% | $\sigma_{DF_k104,k101}$ =58.74% | $\sigma_{DF_k104,k102}$ =43.68% | $\sigma_{DF_k104,k103}$ =56.20% | | $\sigma_{DF_k104,k105}$ =29.79% | $\sigma_{DF_k104,k106}$ =7.39% |
| k105 | $\sigma_{DF_k105,k97}$ =54.57% | $\sigma_{DF_k105,k98}$ =48.83% | $\sigma_{DF_k105,k99}$ =59.16% | $\sigma_{DF_k105,k100}$ =54.20% | $\sigma_{DF_k105,k101}$ =71.56% | $\sigma_{DF_k105,k102}$ =54.49% | $\sigma_{DF_k105,k103}$ =69.53% | $\sigma_{DF_k105,k104}$ =29.79% | | $\sigma_{DF_k105,k106}$ =29.00% |
| k106 | $\sigma_{DF_k106,k97}$ =38.80% | $\sigma_{DF_k106,k98}$ =36.64% | $\sigma_{DF_k106,k99}$ =45.04% | $\sigma_{DF_k106,k100}$ =37.00% | $\sigma_{DF_k106,k101}$ =56.43% | $\sigma_{DF_k106,k102}$ =40.33% | $\sigma_{DF_k106,k103}$ =53.43% | $\sigma_{DF_k106,k104}$ =7.39% | $\sigma_{DF_k106,k105}$ =29.00% | |

FIG. 76

POSITIVE POTENTIAL SECTION (classes 1–138) | NEGATIVE POTENTIAL SECTION (classes 139–276)

(a)

| CLASS | 1 | 2 | ··· | 137 | 138 | 139 | 140 | ··· | 275 | 276 |
|---|---|---|---|---|---|---|---|---|---|---|
| INTEGRAL VALUE | $ITG_{1_276}$ | $ITG_{2_276}$ | ··· | $ITG_{137_276}$ | $ITG_{138_276}$ | $ITG_{139_276}$ | $ITG_{140_276}$ | ··· | $ITG_{275_276}$ | $ITG_{276_276}$ |

(b)

| CLASS | 1 | 2 | ··· | 68 | 69 | 70 | 71 | ··· | 137 | 138 |
|---|---|---|---|---|---|---|---|---|---|---|
| INTEGRAL VALUE | $ITG_{1_138}$ | $ITG_{2_138}$ | ··· | $ITG_{68_138}$ | $ITG_{69_138}$ | $ITG_{70_138}$ | $ITG_{71_138}$ | ··· | $ITG_{137_138}$ | $ITG_{138_138}$ |

FIG. 77

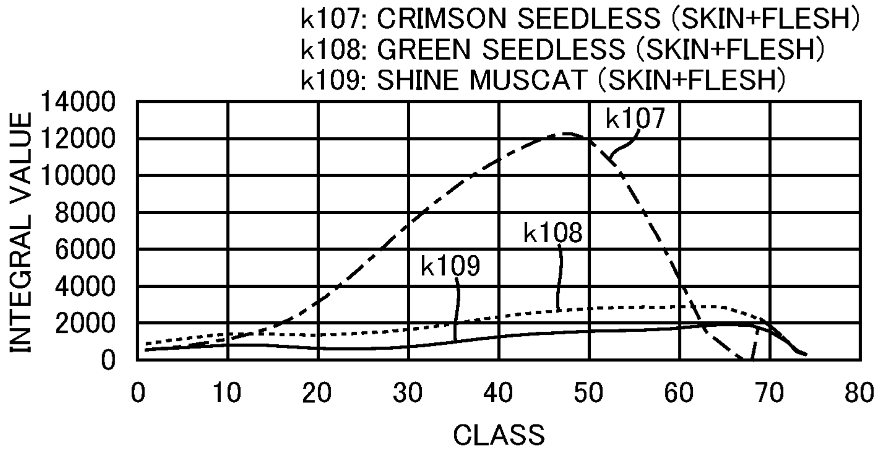


FIG. 78

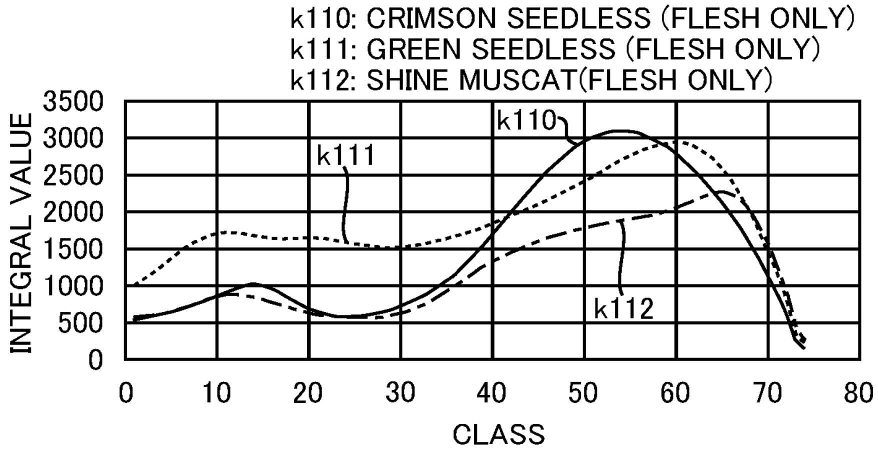


FIG. 79

| | k107 | k108 | k109 | k110 | k111 | k112 |
|---|---|---|---|---|---|---|
| k107 | | $\sigma_{DF_k107,k108}$ =16.06% | $\sigma_{DF_k107,k109}$ =35.77% | $\sigma_{DF_k107,k110}$ =89.09% | $\sigma_{DF_k107,k111}$ =30.03% | $\sigma_{DF_k107,k112}$ =27.15% |
| k108 | $\sigma_{DF_k108,k107}$ =16.06% | | $\sigma_{DF_k108,k109}$ =28.26% | $\sigma_{DF_k108,k110}$ =83.52% | $\sigma_{DF_k108,k111}$ =22.04% | $\sigma_{DF_k108,k112}$ =18.65% |
| k109 | $\sigma_{DF_k109,k107}$ =35.77% | $\sigma_{DF_k109,k108}$ =28.26% | | $\sigma_{DF_k109,k110}$ =83.59% | $\sigma_{DF_k109,k111}$ =23.51% | $\sigma_{DF_k109,k112}$ =28.46% |
| k110 | $\sigma_{DF_k110,k107}$ =89.09% | $\sigma_{DF_k110,k108}$ =83.52% | $\sigma_{DF_k110,k109}$ =83.59% | | $\sigma_{DF_k110,k111}$ =88.38% | $\sigma_{DF_k110,k112}$ =87.08% |
| k111 | $\sigma_{DF_k111,k107}$ =30.03% | $\sigma_{DF_k111,k108}$ =22.04% | $\sigma_{DF_k111,k109}$ =23.51% | $\sigma_{DF_k111,k110}$ =88.38% | | $\sigma_{DF_k111,k112}$ =8.69% |
| k112 | $\sigma_{DF_k112,k107}$ =27.15% | $\sigma_{DF_k112,k108}$ =18.65% | $\sigma_{DF_k112,k109}$ =28.46% | $\sigma_{DF_k112,k110}$ =87.08% | $\sigma_{DF_k112,k111}$ =8.69% | |

FIG. 80

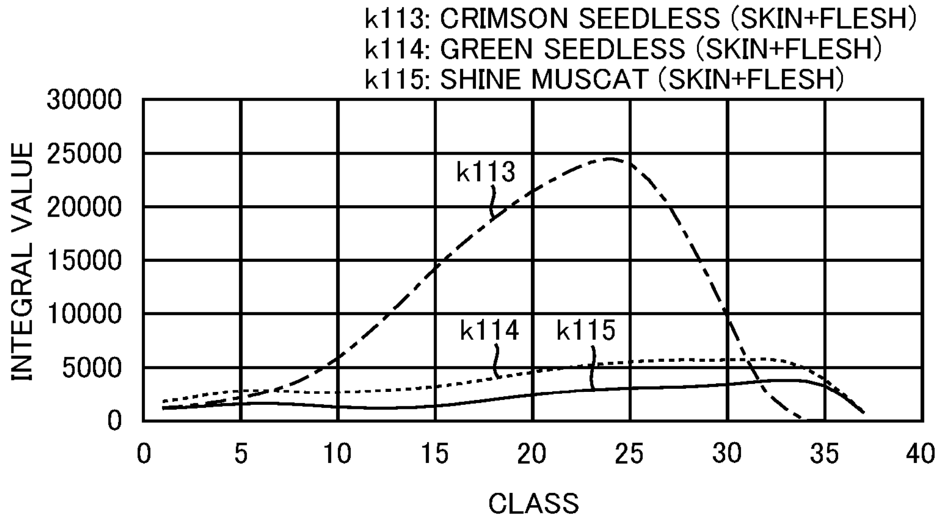


FIG. 81

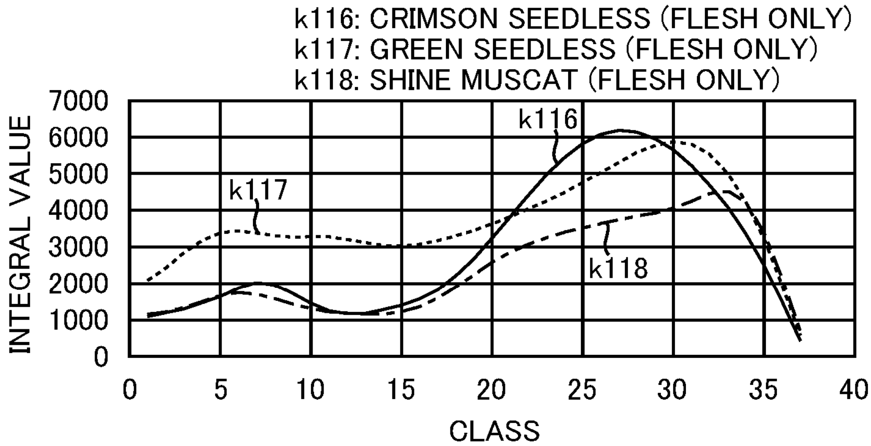


FIG. 82

| | k113 | k114 | k115 | k116 | k117 | k118 |
|---|---|---|---|---|---|---|
| k113 | | $\sigma_{DF_k113,k114}$ =16.03% | $\sigma_{DF_k113,k115}$ =35.73% | $\sigma_{DF_k113,k116}$ =111.69% | $\sigma_{DF_k113,k117}$ =29.97% | $\sigma_{DF_k113,k118}$ =27.08% |
| k114 | $\sigma_{DF_k114,k113}$ =16.03% | | $\sigma_{DF_k114,k115}$ =28.20% | $\sigma_{DF_k114,k116}$ =107.70% | $\sigma_{DF_k114,k117}$ =21.98% | $\sigma_{DF_k114,k118}$ =18.55% |
| k115 | $\sigma_{DF_k115,k113}$ =35.73% | $\sigma_{DF_k115,k114}$ =28.20% | | $\sigma_{DF_k115,k116}$ =111.01% | $\sigma_{DF_k115,k117}$ =23.42% | $\sigma_{DF_k115,k118}$ =28.35% |
| k116 | $\sigma_{DF_k116,k113}$ =111.69% | $\sigma_{DF_k116,k114}$ =107.70% | $\sigma_{DF_k116,k115}$ =111.01% | | $\sigma_{DF_k116,k117}$ =115.64% | $\sigma_{DF_k116,k118}$ =115.29% |
| k117 | $\sigma_{DF_k117,k113}$ =29.97% | $\sigma_{DF_k117,k114}$ =21.98% | $\sigma_{DF_k117,k115}$ =23.42% | $\sigma_{DF_k117,k116}$ =115.64% | | $\sigma_{DF_k117,k118}$ =8.64% |
| k118 | $\sigma_{DF_k118,k113}$ =27.08% | $\sigma_{DF_k118,k114}$ =18.55% | $\sigma_{DF_k118,k115}$ =28.35% | $\sigma_{DF_k118,k116}$ =115.29% | $\sigma_{DF_k118,k117}$ =8.64% | |

FIG. 83

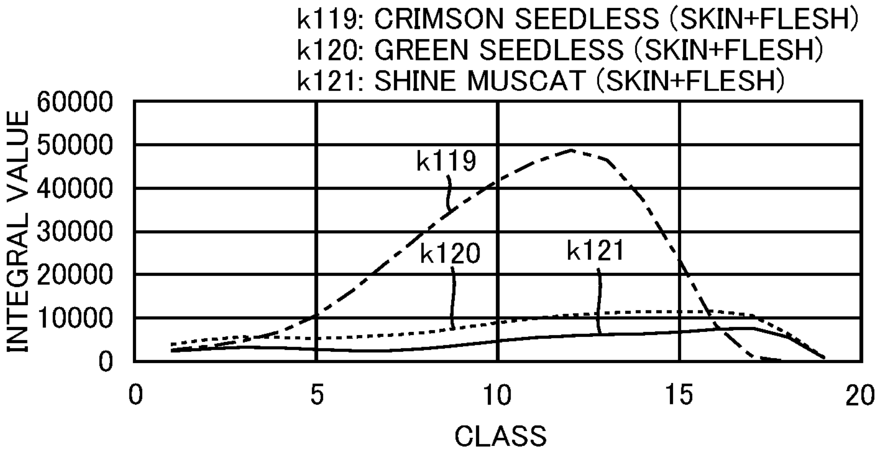


FIG. 84

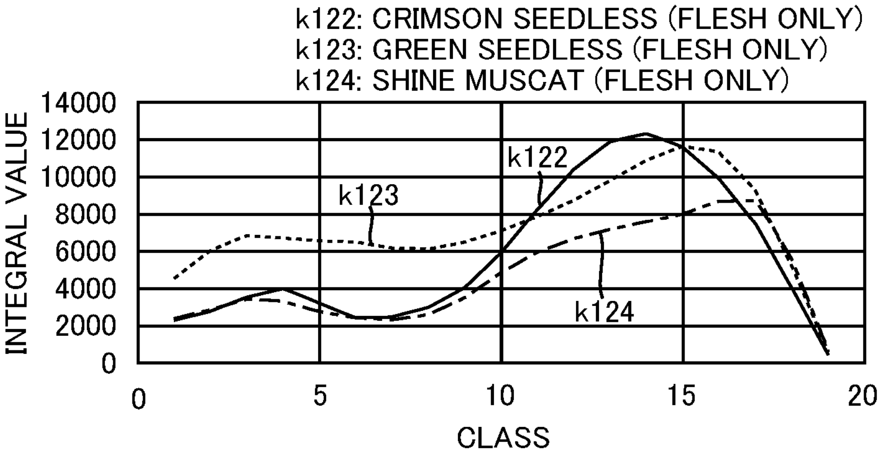


FIG. 85

| | k119 | k120 | k121 | k122 | k123 | k124 |
|---|---|---|---|---|---|---|
| k119 | | $\sigma_{DF_k119,k120}$ =16.08% | $\sigma_{DF_k119,k121}$ =35.07% | $\sigma_{DF_k119,k122}$ =114.98% | $\sigma_{DF_k119,k123}$ =31.09% | $\sigma_{DF_k119,k124}$ =28.93% |
| k120 | $\sigma_{DF_k120,k119}$ =16.08% | | $\sigma_{DF_k120,k121}$ =27.63% | $\sigma_{DF_k120,k122}$ =111.48% | $\sigma_{DF_k120,k123}$ =22.60% | $\sigma_{DF_k120,k124}$ =19.97% |
| k121 | $\sigma_{DF_k121,k119}$ =35.07% | $\sigma_{DF_k121,k120}$ =27.63% | | $\sigma_{DF_k121,k122}$ =115.34% | $\sigma_{DF_k121,k123}$ =24.60% | $\sigma_{DF_k121,k124}$ =29.61% |
| k122 | $\sigma_{DF_k122,k119}$ =114.98% | $\sigma_{DF_k122,k120}$ =111.48% | $\sigma_{DF_k122,k121}$ =115.34% | | $\sigma_{DF_k122,k123}$ =120.43% | $\sigma_{DF_k122,k124}$ =120.22% |
| k123 | $\sigma_{DF_k123,k119}$ =31.09% | $\sigma_{DF_k123,k120}$ =22.60% | $\sigma_{DF_k123,k121}$ =24.60% | $\sigma_{DF_k123,k122}$ =120.43% | | $\sigma_{DF_k123,k124}$ =8.54% |
| k124 | $\sigma_{DF_k124,k119}$ =28.93% | $\sigma_{DF_k124,k120}$ =19.97% | $\sigma_{DF_k124,k121}$ =29.61% | $\sigma_{DF_k124,k122}$ =120.22% | $\sigma_{DF_k124,k123}$ =8.54% | |

FIG. 86

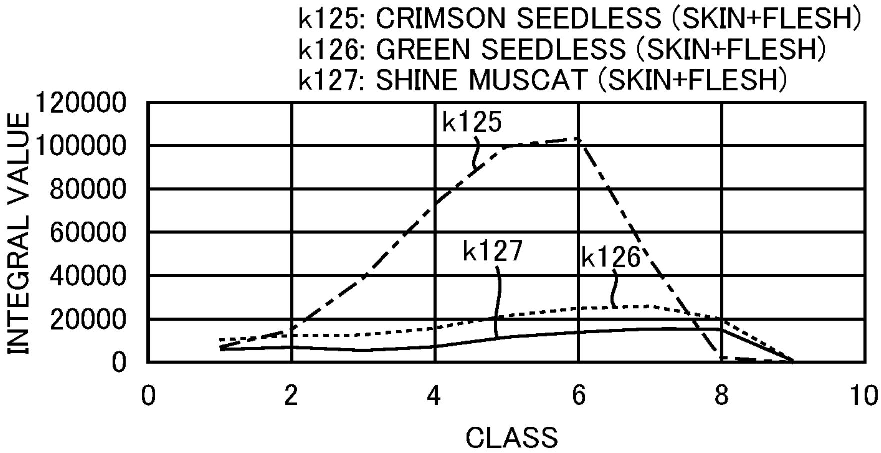


FIG. 87

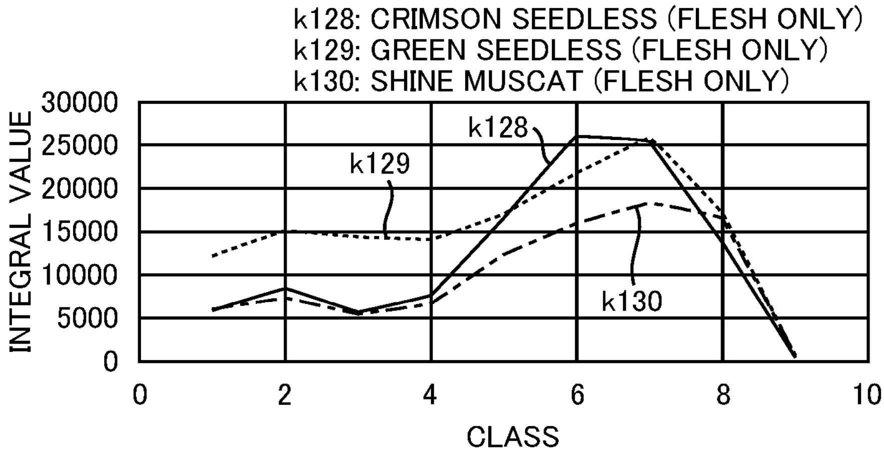


FIG. 88

| | k125 | k126 | k127 | k128 | k129 | k130 |
|---|---|---|---|---|---|---|
| k125 | | $\sigma_{DF_k125,k126}$ =15.94% | $\sigma_{DF_k125,k127}$ =33.48% | $\sigma_{DF_k125,k128}$ =120.63% | $\sigma_{DF_k125,k129}$ =32.88% | $\sigma_{DF_k125,k130}$ =31.94% |
| k126 | $\sigma_{DF_k126,k125}$ =15.94% | | $\sigma_{DF_k126,k127}$ =26.17% | $\sigma_{DF_k126,k128}$ =118.24% | $\sigma_{DF_k126,k129}$ =23.51% | $\sigma_{DF_k126,k130}$ =22.24% |
| k127 | $\sigma_{DF_k127,k125}$ =33.48% | $\sigma_{DF_k127,k126}$ =26.17% | | $\sigma_{DF_k127,k128}$ =122.84% | $\sigma_{DF_k127,k129}$ =26.52% | $\sigma_{DF_k127,k130}$ =31.58% |
| k128 | $\sigma_{DF_k128,k125}$ =120.63% | $\sigma_{DF_k128,k126}$ =118.24% | $\sigma_{DF_k128,k127}$ =122.84% | | $\sigma_{DF_k128,k129}$ =129.23% | $\sigma_{DF_k128,k130}$ =129.56% |
| k129 | $\sigma_{DF_k129,k125}$ =32.88% | $\sigma_{DF_k129,k126}$ =23.51% | $\sigma_{DF_k129,k127}$ =26.52% | $\sigma_{DF_k129,k128}$ =129.23% | | $\sigma_{DF_k129,k130}$ =8.18% |
| k130 | $\sigma_{DF_k130,k125}$ =31.94% | $\sigma_{DF_k130,k126}$ =22.24% | $\sigma_{DF_k130,k127}$ =31.58% | $\sigma_{DF_k130,k128}$ =129.56% | $\sigma_{DF_k130,k129}$ =8.18% | |

FIG. 89

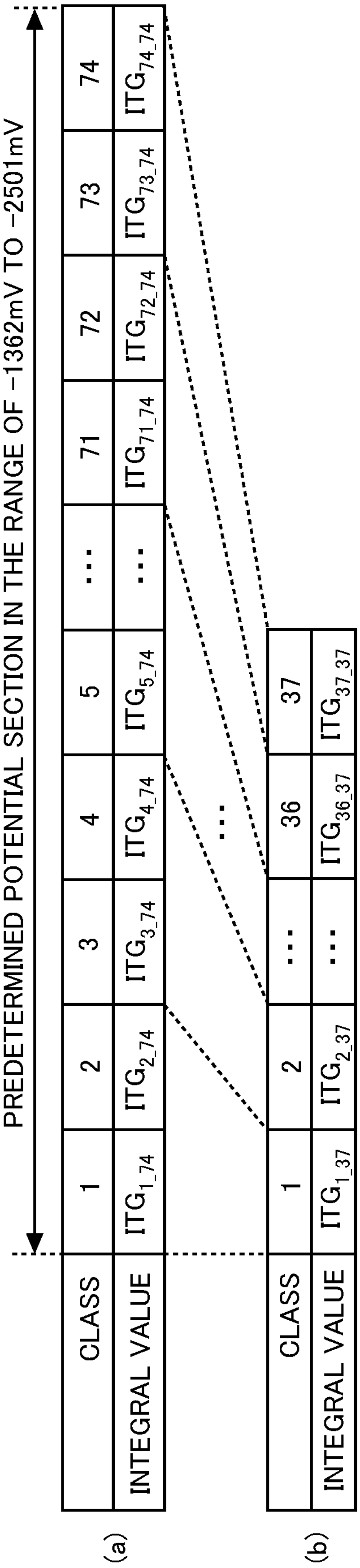
PREDETERMINED POTENTIAL SECTION IN THE RANGE OF −1362mV TO −2501mV
(a)
CLASS
INTEGRAL VALUE
1 2 3 4 5 … 71 72 73 74
ITG1_74 ITG2_74 ITG3_74 ITG4_74 ITG5_74 … ITG71_74 ITG72_74 ITG73_74 ITG74_74
(b)
CLASS
INTEGRAL VALUE
1 2 … 36 37
ITG1_37 ITG2_37 … ITG36_37 ITG37_37

FIG. 90

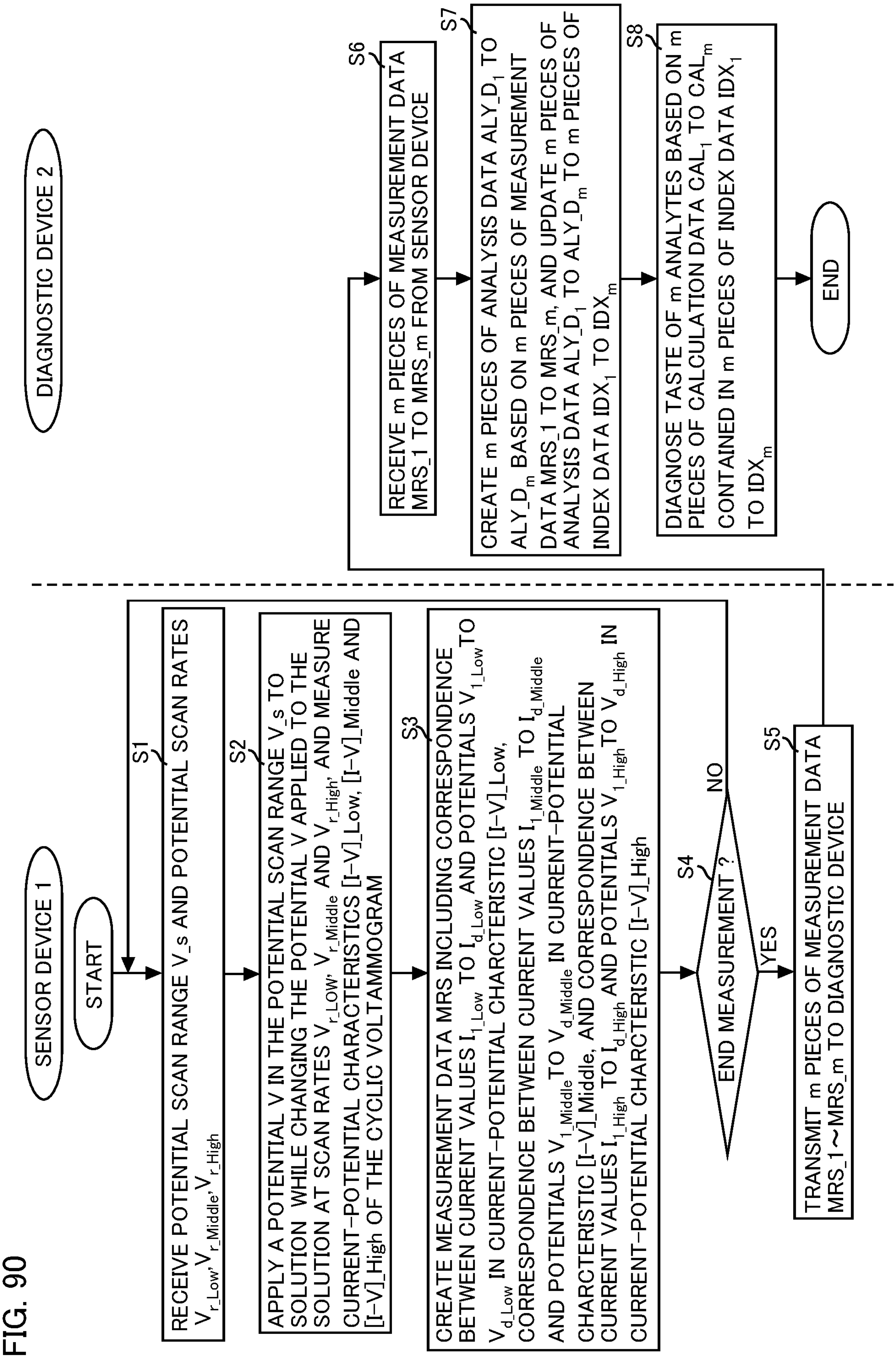
SENSOR DEVICE 1
START
S1
RECEIVE POTENTIAL SCAN RANGE V_s AND POTENTIAL SCAN RATES $V_{r_Low}$, $V_{r_Middle}$, $V_{r_High}$
S2
APPLY A POTENTIAL V IN THE POTENTIAL SCAN RANGE V_s TO SOLUTION WHILE CHANGING THE POTENTIAL V APPLIED TO THE SOLUTION AT SCAN RATES $V_{r_LOW}$, $V_{r_Middle}$ AND $V_{r_High}$, AND MEASURE CURRENT−POTENTIAL CHARACTERISTICS [I−V]_Low, [I−V]_Middle AND [I−V]_High OF THE CYCLIC VOLTAMMOGRAM
S3
CREATE MEASUREMENT DATA MRS INCLUDING CORRESPONDENCE BETWEEN CURRENT VALUES $I_{1_Low}$ TO $I_{d_Low}$ AND POTENTIALS $V_{1_Low}$ TO $V_{d_Low}$ IN CURRENT−POTENTIAL CHARCTERISTIC [I−V]_Low, CORRESPONDENCE BETWEEN CURRENT VALUES $I_{1_Middle}$ TO $I_{d_Middle}$ AND POTENTIALS $V_{1_Middle}$ TO $V_{d_Middle}$ IN CURRENT−POTENTIAL CHARCTERISTIC [I−V]_Middle, AND CORRESPONDENCE BETWEEN CURRENT VALUES $I_{1_High}$ TO $I_{d_High}$ AND POTENTIALS $V_{1_High}$ TO $V_{d_High}$ IN CURRENT−POTENTIAL CHARCTERISTIC [I−V]_High
S4
END MEASUREMENT ?
NO
YES
S5
TRANSMIT m PIECES OF MEASUREMENT DATA MRS_1〜MRS_m TO DIAGNOSTIC DEVICE
DIAGNOSTIC DEVICE 2
S6
RECEIVE m PIECES OF MEASUREMENT DATA MRS_1 TO MRS_m FROM SENSOR DEVICE
S7
CREATE m PIECES OF ANALYSIS DATA $ALY_D_1$ TO $ALY_D_m$ BASED ON m PIECES OF MEASUREMENT DATA MRS_1 TO MRS_m, AND UPDATE m PIECES OF ANALYSIS DATA $ALY_D_1$ TO $ALY_D_m$ TO m PIECES OF INDEX DATA $IDX_1$ TO $IDX_m$
S8
DIAGNOSE TASTE OF m ANALYTES BASED ON m PIECES OF CALCULATION DATA $CAL_1$ TO $CAL_m$ CONTAINED IN m PIECES OF INDEX DATA $IDX_1$ TO $IDX_m$
END

FIG. 91

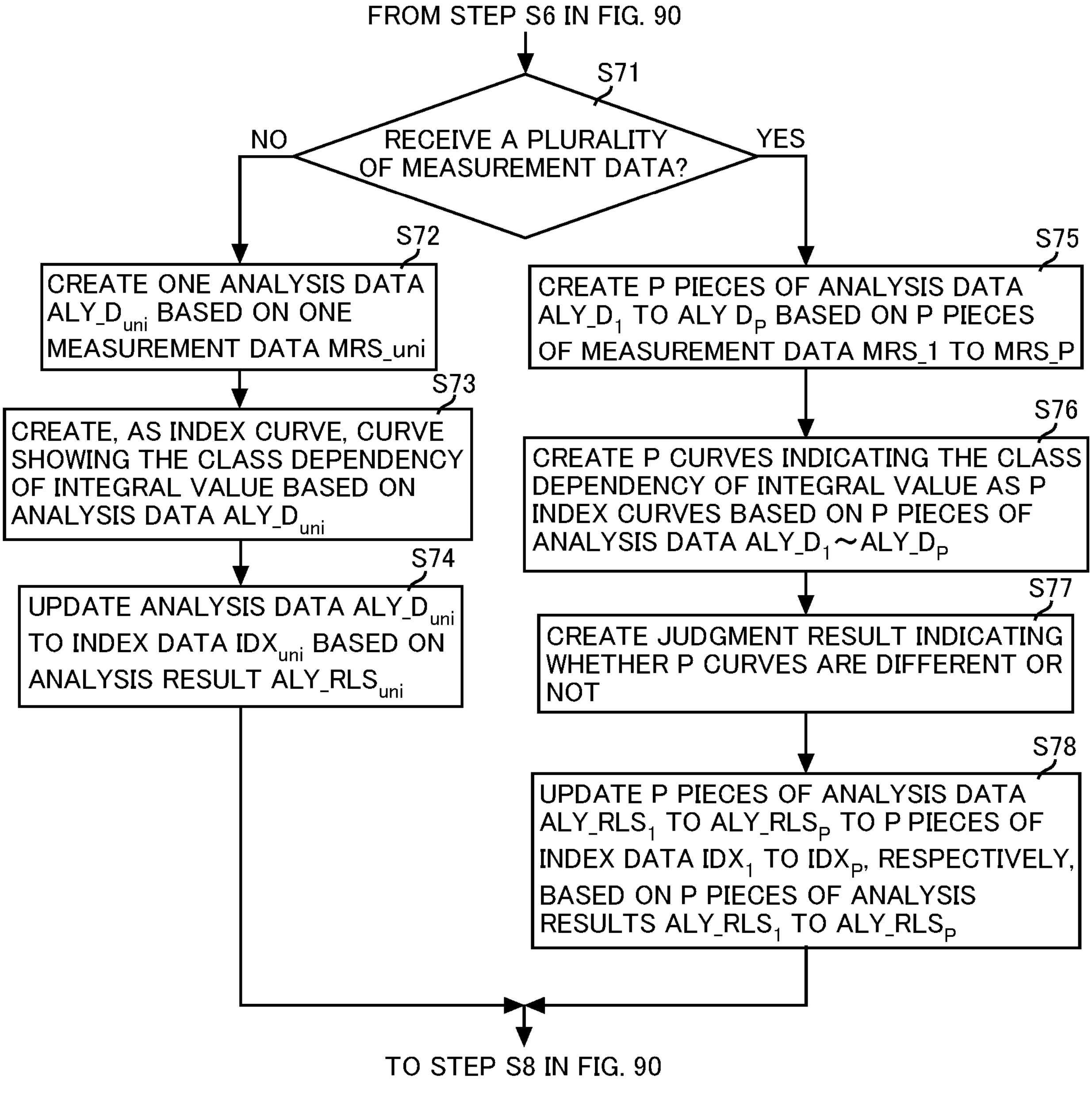
FROM STEP S6 IN FIG. 90
S71
RECEIVE A PLURALITY OF MEASUREMENT DATA?
NO
YES
S72
CREATE ONE ANALYSIS DATA ALY_D$_{uni}$ BASED ON ONE MEASUREMENT DATA MRS_uni
S73
CREATE, AS INDEX CURVE, CURVE SHOWING THE CLASS DEPENDENCY OF INTEGRAL VALUE BASED ON ANALYSIS DATA ALY_D$_{uni}$
S74
UPDATE ANALYSIS DATA ALY_D$_{uni}$ TO INDEX DATA IDX$_{uni}$ BASED ON ANALYSIS RESULT ALY_RLS$_{uni}$
S75
CREATE P PIECES OF ANALYSIS DATA ALY_D$_1$ TO ALY D$_P$ BASED ON P PIECES OF MEASUREMENT DATA MRS_1 TO MRS_P
S76
CREATE P CURVES INDICATING THE CLASS DEPENDENCY OF INTEGRAL VALUE AS P INDEX CURVES BASED ON P PIECES OF ANALYSIS DATA ALY_D$_1$～ALY_D$_P$
S77
CREATE JUDGMENT RESULT INDICATING WHETHER P CURVES ARE DIFFERENT OR NOT
S78
UPDATE P PIECES OF ANALYSIS DATA ALY_RLS$_1$ TO ALY_RLS$_P$ TO P PIECES OF INDEX DATA IDX$_1$ TO IDX$_P$, RESPECTIVELY, BASED ON P PIECES OF ANALYSIS RESULTS ALY_RLS$_1$ TO ALY_RLS$_P$
TO STEP S8 IN FIG. 90

FIG. 92

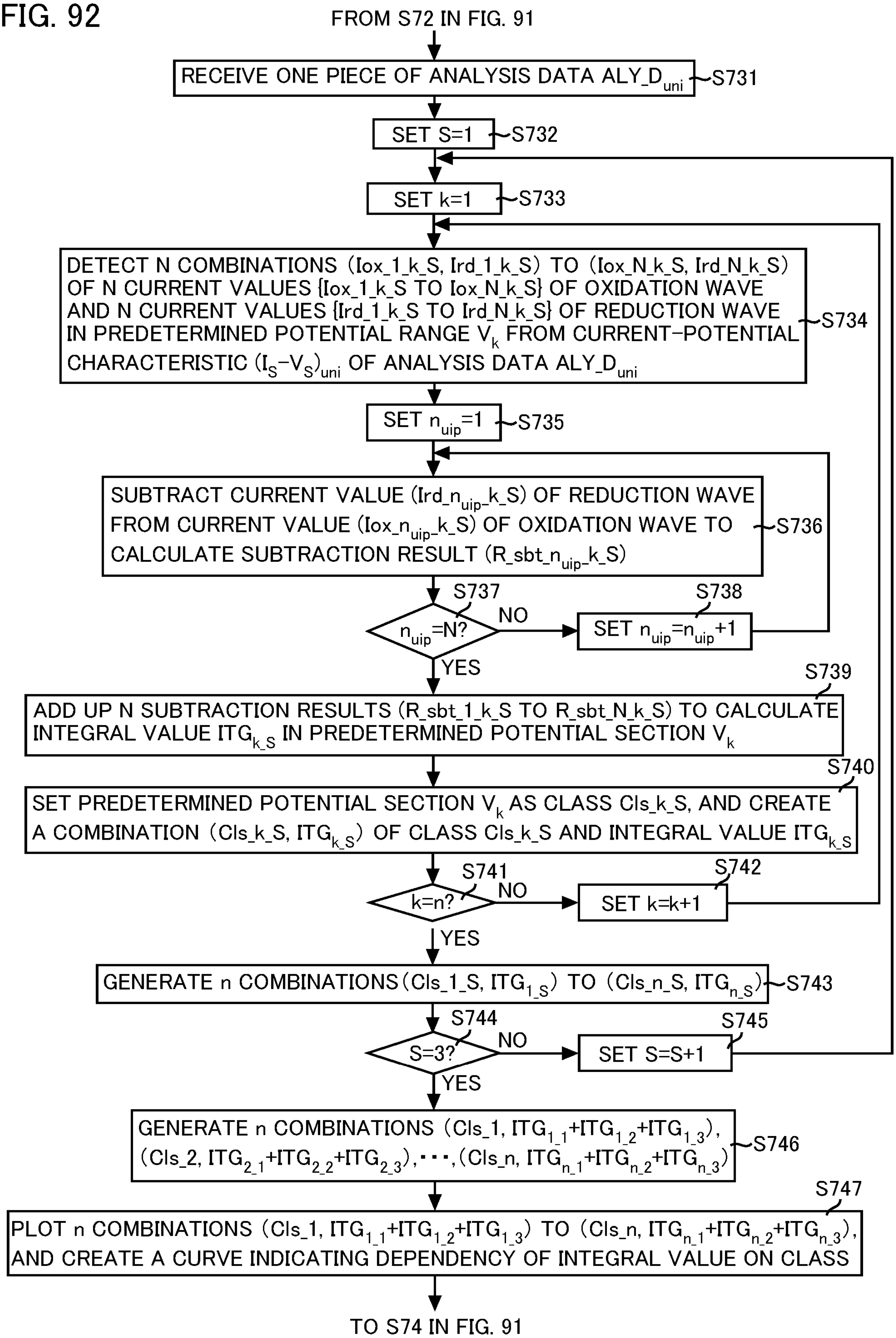
FROM S72 IN FIG. 91
RECEIVE ONE PIECE OF ANALYSIS DATA $ALY_D_{uni}$
S731
SET S=1
S732
SET k=1
S733
DETECT N COMBINATIONS (Iox_1_k_S, Ird_1_k_S) TO (Iox_N_k_S, Ird_N_k_S) OF N CURRENT VALUES {Iox_1_k_S TO Iox_N_k_S} OF OXIDATION WAVE AND N CURRENT VALUES {Ird_1_k_S TO Ird_N_k_S} OF REDUCTION WAVE IN PREDETERMINED POTENTIAL RANGE $V_k$ FROM CURRENT-POTENTIAL CHARACTERISTIC $(I_S-V_S)_{uni}$ OF ANALYSIS DATA $ALY_D_{uni}$
S734
SET $n_{uip}$=1
S735
SUBTRACT CURRENT VALUE ($Ird_n_{uip}_k_S$) OF REDUCTION WAVE FROM CURRENT VALUE ($Iox_n_{uip}_k_S$) OF OXIDATION WAVE TO CALCULATE SUBTRACTION RESULT ($R_sbt_n_{uip}_k_S$)
S736
S737
$n_{uip}$=N?
NO
S738
SET $n_{uip}=n_{uip}+1$
YES
S739
ADD UP N SUBTRACTION RESULTS (R_sbt_1_k_S TO R_sbt_N_k_S) TO CALCULATE INTEGRAL VALUE $ITG_{k_S}$ IN PREDETERMINED POTENTIAL SECTION $V_k$
S740
SET PREDETERMINED POTENTIAL SECTION $V_k$ AS CLASS Cls_k_S, AND CREATE A COMBINATION (Cls_k_S, $ITG_{k_S}$) OF CLASS Cls_k_S AND INTEGRAL VALUE $ITG_{k_S}$
S741
k=n?
NO
S742
SET k=k+1
YES
GENERATE n COMBINATIONS (Cls_1_S, $ITG_{1_S}$) TO (Cls_n_S, $ITG_{n_S}$)
S743
S744
S=3?
NO
S745
SET S=S+1
YES
GENERATE n COMBINATIONS (Cls_1, $ITG_{1_1}+ITG_{1_2}+ITG_{1_3}$), (Cls_2, $ITG_{2_1}+ITG_{2_2}+ITG_{2_3}$),・・・,(Cls_n, $ITG_{n_1}+ITG_{n_2}+ITG_{n_3}$)
S746
S747
PLOT n COMBINATIONS (Cls_1, $ITG_{1_1}+ITG_{1_2}+ITG_{1_3}$) TO (Cls_n, $ITG_{n_1}+ITG_{n_2}+ITG_{n_3}$), AND CREATE A CURVE INDICATING DEPENDENCY OF INTEGRAL VALUE ON CLASS
TO S74 IN FIG. 91

FIG. 93

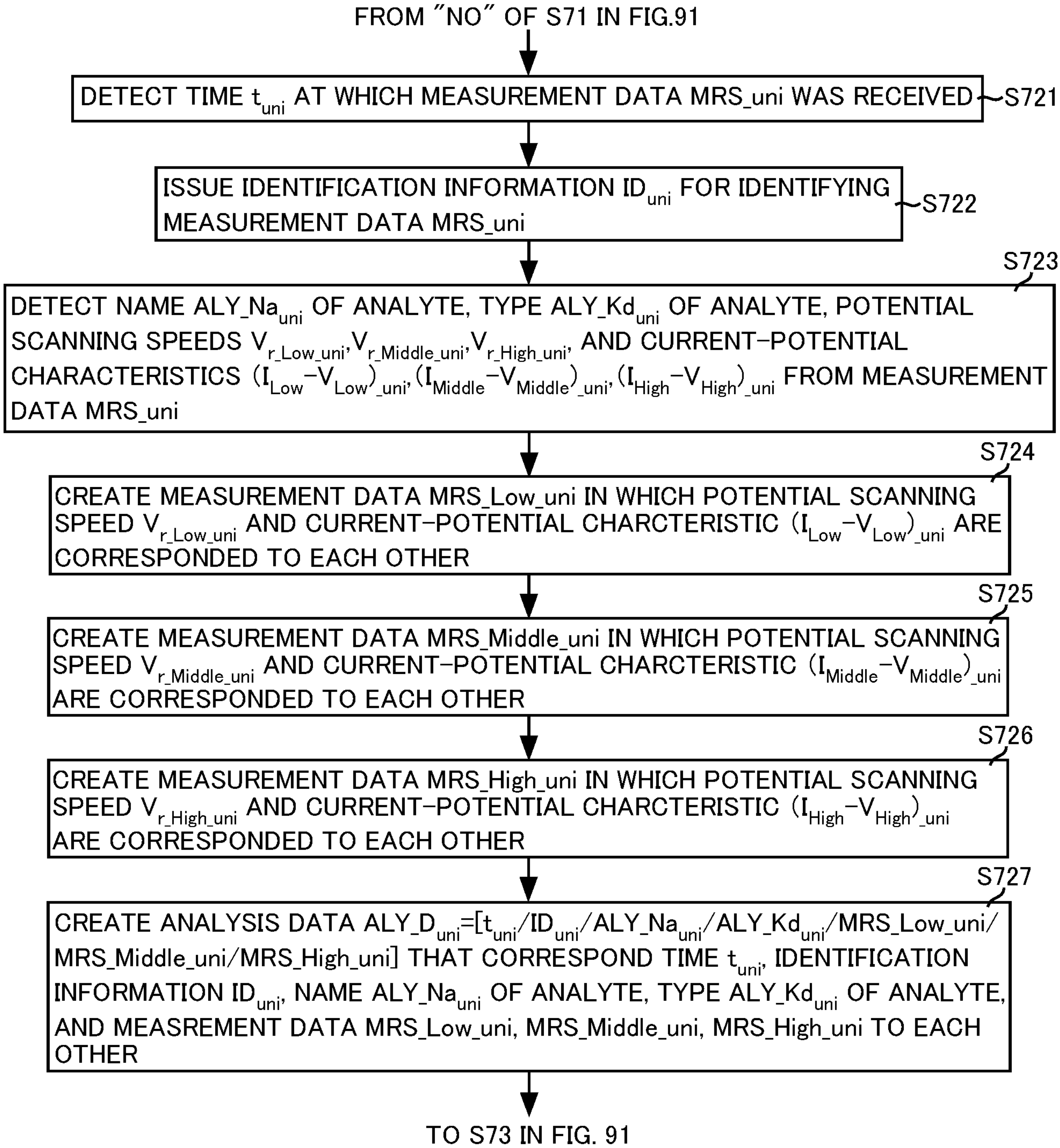
FROM "NO" OF S71 IN FIG.91
DETECT TIME $t_{uni}$ AT WHICH MEASUREMENT DATA MRS_uni WAS RECEIVED
S721
ISSUE IDENTIFICATION INFORMATION $ID_{uni}$ FOR IDENTIFYING MEASUREMENT DATA MRS_uni
S722
S723
DETECT NAME $ALY_Na_{uni}$ OF ANALYTE, TYPE $ALY_Kd_{uni}$ OF ANALYTE, POTENTIAL SCANNING SPEEDS $V_{r_Low_uni}$, $V_{r_Middle_uni}$, $V_{r_High_uni}$, AND CURRENT-POTENTIAL CHARACTERISTICS $(I_{Low}-V_{Low})_{_uni}$, $(I_{Middle}-V_{Middle})_{_uni}$, $(I_{High}-V_{High})_{_uni}$ FROM MEASUREMENT DATA MRS_uni
S724
CREATE MEASUREMENT DATA MRS_Low_uni IN WHICH POTENTIAL SCANNING SPEED $V_{r_Low_uni}$ AND CURRENT-POTENTIAL CHARCTERISTIC $(I_{Low}-V_{Low})_{_uni}$ ARE CORRESPONDED TO EACH OTHER
S725
CREATE MEASUREMENT DATA MRS_Middle_uni IN WHICH POTENTIAL SCANNING SPEED $V_{r_Middle_uni}$ AND CURRENT-POTENTIAL CHARCTERISTIC $(I_{Middle}-V_{Middle})_{_uni}$ ARE CORRESPONDED TO EACH OTHER
S726
CREATE MEASUREMENT DATA MRS_High_uni IN WHICH POTENTIAL SCANNING SPEED $V_{r_High_uni}$ AND CURRENT-POTENTIAL CHARCTERISTIC $(I_{High}-V_{High})_{_uni}$ ARE CORRESPONDED TO EACH OTHER
S727
CREATE ANALYSIS DATA $ALY_D_{uni}$=[$t_{uni}$/$ID_{uni}$/$ALY_Na_{uni}$/$ALY_Kd_{uni}$/MRS_Low_uni/MRS_Middle_uni/MRS_High_uni] THAT CORRESPOND TIME $t_{uni}$, IDENTIFICATION INFORMATION $ID_{uni}$, NAME $ALY_Na_{uni}$ OF ANALYTE, TYPE $ALY_Kd_{uni}$ OF ANALYTE, AND MEASREMENT DATA MRS_Low_uni, MRS_Middle_uni, MRS_High_uni TO EACH OTHER
TO S73 IN FIG. 91

FIG. 94

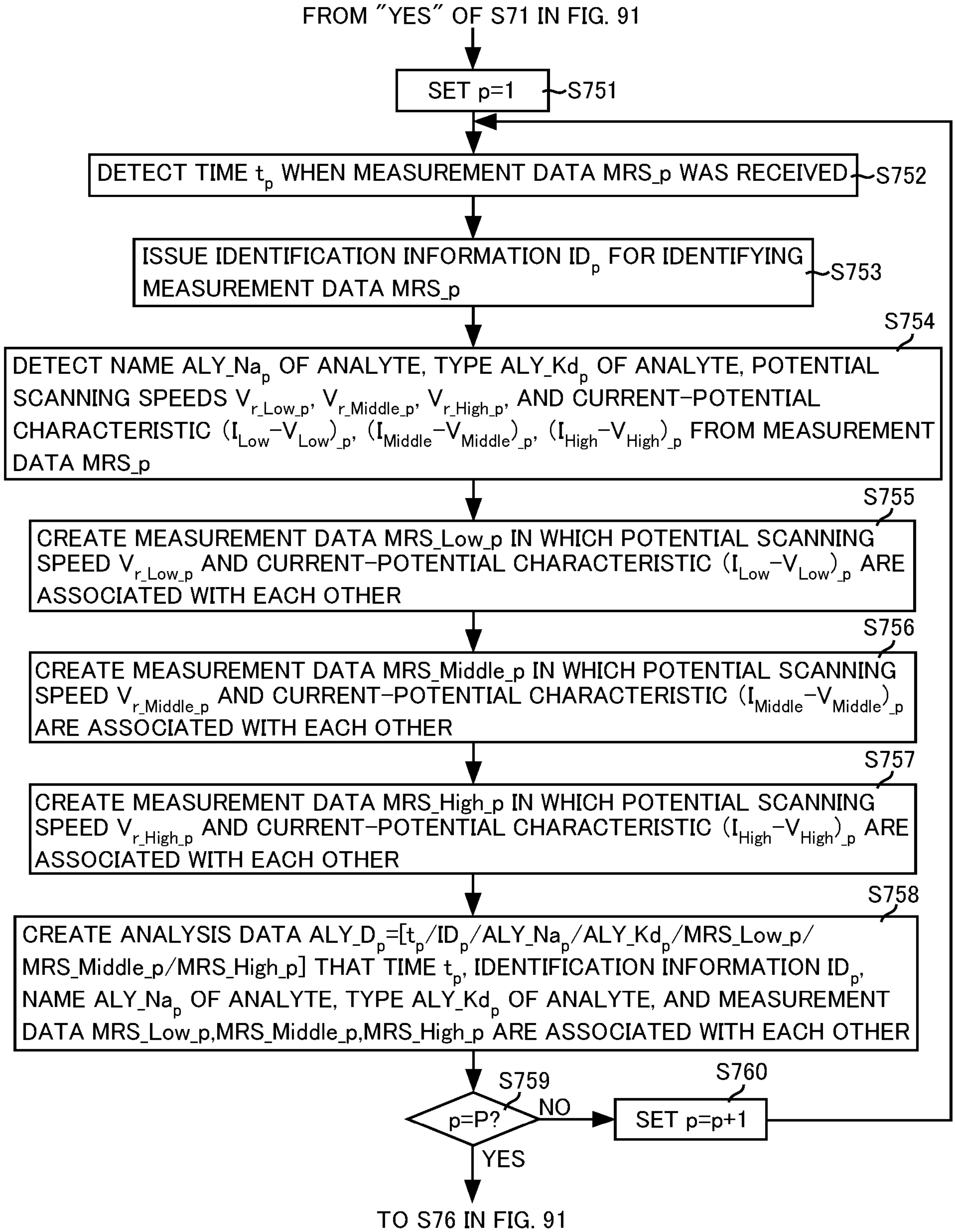
FROM "YES" OF S71 IN FIG. 91
SET p=1
S751
DETECT TIME $t_p$ WHEN MEASUREMENT DATA MRS_p WAS RECEIVED
S752
ISSUE IDENTIFICATION INFORMATION $ID_p$ FOR IDENTIFYING MEASUREMENT DATA MRS_p
S753
S754
DETECT NAME $ALY_Na_p$ OF ANALYTE, TYPE $ALY_Kd_p$ OF ANALYTE, POTENTIAL SCANNING SPEEDS $V_{r_Low_p}$, $V_{r_Middle_p}$, $V_{r_High_p}$, AND CURRENT−POTENTIAL CHARACTERISTIC $(I_{Low}-V_{Low})_{_p}$, $(I_{Middle}-V_{Middle})_{_p}$, $(I_{High}-V_{High})_{_p}$ FROM MEASUREMENT DATA MRS_p
S755
CREATE MEASUREMENT DATA MRS_Low_p IN WHICH POTENTIAL SCANNING SPEED $V_{r_Low_p}$ AND CURRENT−POTENTIAL CHARACTERISTIC $(I_{Low}-V_{Low})_{_p}$ ARE ASSOCIATED WITH EACH OTHER
S756
CREATE MEASUREMENT DATA MRS_Middle_p IN WHICH POTENTIAL SCANNING SPEED $V_{r_Middle_p}$ AND CURRENT−POTENTIAL CHARACTERISTIC $(I_{Middle}-V_{Middle})_{_p}$ ARE ASSOCIATED WITH EACH OTHER
S757
CREATE MEASUREMENT DATA MRS_High_p IN WHICH POTENTIAL SCANNING SPEED $V_{r_High_p}$ AND CURRENT−POTENTIAL CHARACTERISTIC $(I_{High}-V_{High})_{_p}$ ARE ASSOCIATED WITH EACH OTHER
S758
CREATE ANALYSIS DATA $ALY_D_p=[t_p/ID_p/ALY_Na_p/ALY_Kd_p$/MRS_Low_p/MRS_Middle_p/MRS_High_p] THAT TIME $t_p$, IDENTIFICATION INFORMATION $ID_p$, NAME $ALY_Na_p$ OF ANALYTE, TYPE $ALY_Kd_p$ OF ANALYTE, AND MEASUREMENT DATA MRS_Low_p,MRS_Middle_p,MRS_High_p ARE ASSOCIATED WITH EACH OTHER
S759
p=P?
NO
S760
SET p=p+1
YES
TO S76 IN FIG. 91

FIG. 95

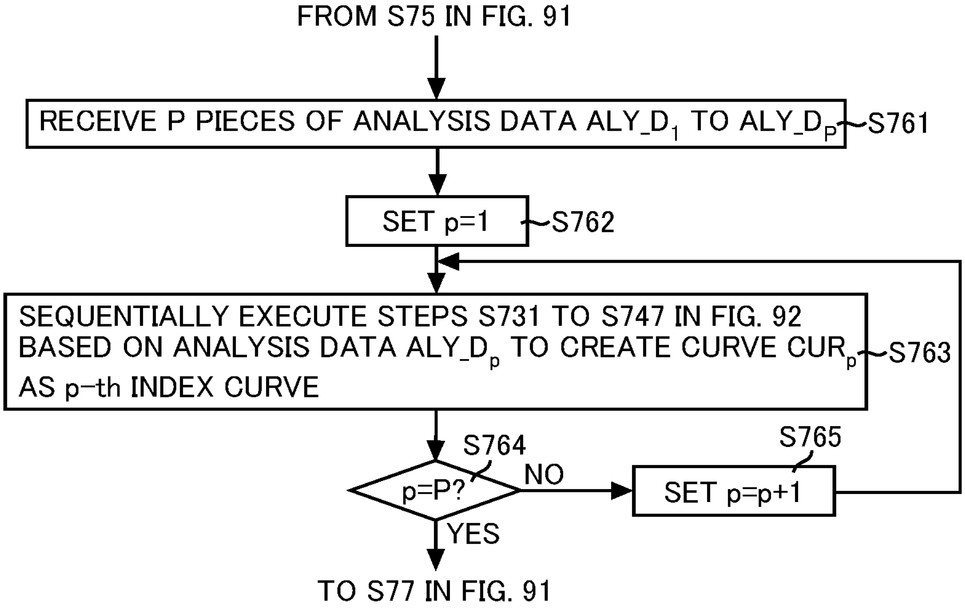
FROM S75 IN FIG. 91
RECEIVE P PIECES OF ANALYSIS DATA ALY_$D_1$ TO ALY_$D_P$
S761
SET p=1
S762
SEQUENTIALLY EXECUTE STEPS S731 TO S747 IN FIG. 92 BASED ON ANALYSIS DATA ALY_$D_p$ TO CREATE CURVE $CUR_p$ AS p-th INDEX CURVE
S763
S764
p=P?
NO
YES
S765
SET p=p+1
TO S77 IN FIG. 91

FIG. 96

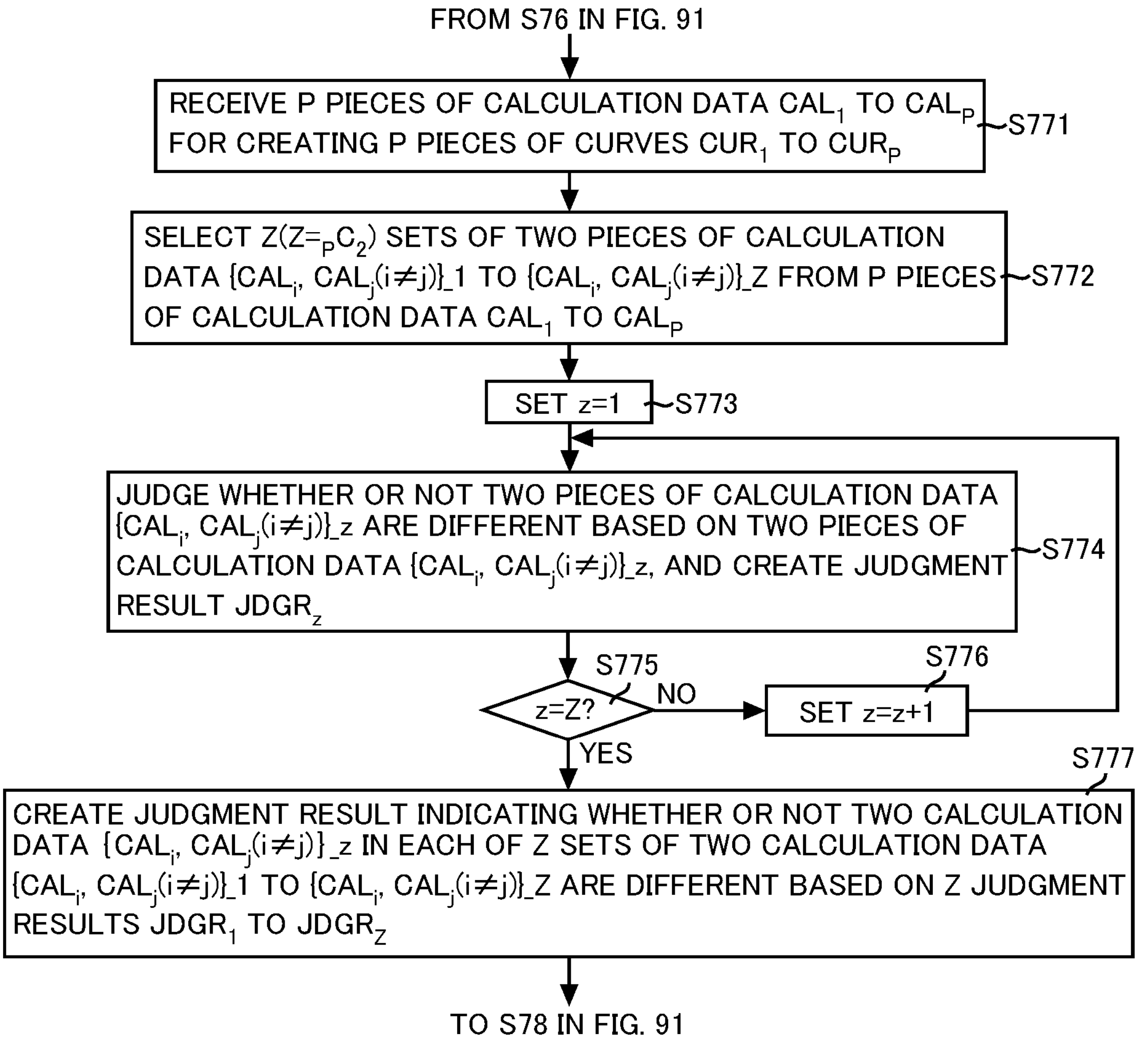
FROM S76 IN FIG. 91
RECEIVE P PIECES OF CALCULATION DATA $CAL_1$ TO $CAL_P$ FOR CREATING P PIECES OF CURVES $CUR_1$ TO $CUR_P$
S771
SELECT Z($Z={}_PC_2$) SETS OF TWO PIECES OF CALCULATION DATA {$CAL_i$, $CAL_j$($i\neq j$)}_1 TO {$CAL_i$, $CAL_j$($i\neq j$)}_Z FROM P PIECES OF CALCULATION DATA $CAL_1$ TO $CAL_P$
S772
SET z=1
S773
JUDGE WHETHER OR NOT TWO PIECES OF CALCULATION DATA {$CAL_i$, $CAL_j$($i\neq j$)}_z ARE DIFFERENT BASED ON TWO PIECES OF CALCULATION DATA {$CAL_i$, $CAL_j$($i\neq j$)}_z, AND CREATE JUDGMENT RESULT $JDGR_z$
S774
S775
z=Z?
NO
YES
S776
SET z=z+1
S777
CREATE JUDGMENT RESULT INDICATING WHETHER OR NOT TWO CALCULATION DATA {$CAL_i$, $CAL_j$($i\neq j$)}_z IN EACH OF Z SETS OF TWO CALCULATION DATA {$CAL_i$, $CAL_j$($i\neq j$)}_1 TO {$CAL_i$, $CAL_j$($i\neq j$)}_Z ARE DIFFERENT BASED ON Z JUDGMENT RESULTS $JDGR_1$ TO $JDGR_Z$
TO S78 IN FIG. 91

FIG. 97

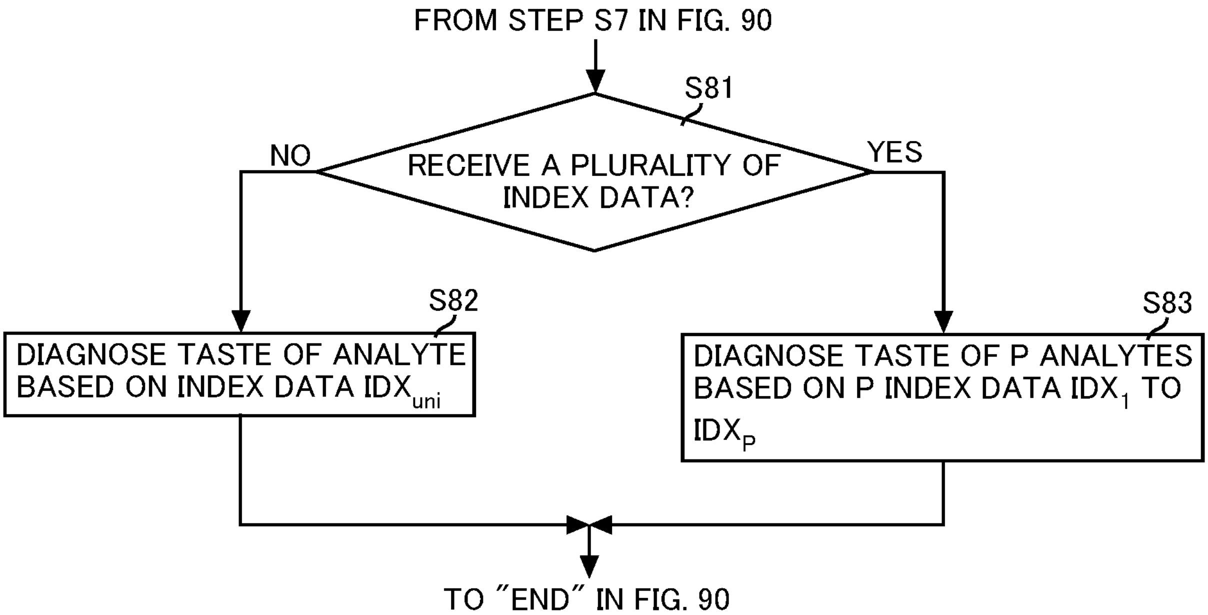
FROM STEP S7 IN FIG. 90
S81
RECEIVE A PLURALITY OF INDEX DATA?
NO
YES
S82
DIAGNOSE TASTE OF ANALYTE BASED ON INDEX DATA $IDX_{uni}$
S83
DIAGNOSE TASTE OF P ANALYTES BASED ON P INDEX DATA $IDX_1$ TO $IDX_P$
TO "END" IN FIG. 90

FIG. 98

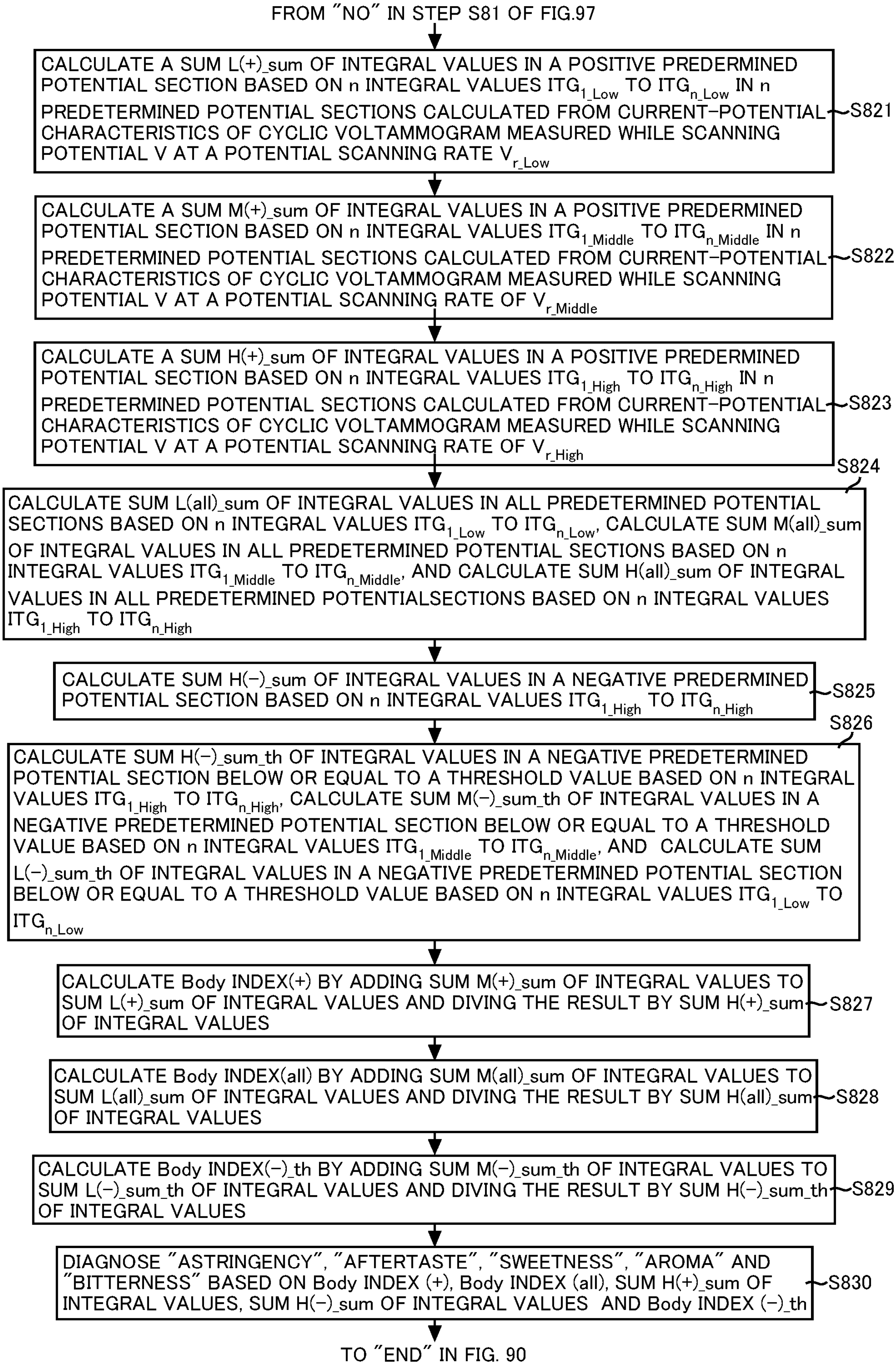
FROM "NO" IN STEP S81 OF FIG.97
CALCULATE A SUM L(+)_sum OF INTEGRAL VALUES IN A POSITIVE PREDERMINED POTENTIAL SECTION BASED ON n INTEGRAL VALUES $ITG_{1_Low}$ TO $ITG_{n_Low}$ IN n PREDETERMINED POTENTIAL SECTIONS CALCULATED FROM CURRENT-POTENTIAL CHARACTERISTICS OF CYCLIC VOLTAMMOGRAM MEASURED WHILE SCANNING POTENTIAL V AT A POTENTIAL SCANNING RATE $V_{r_Low}$
S821
CALCULATE A SUM M(+)_sum OF INTEGRAL VALUES IN A POSITIVE PREDERMINED POTENTIAL SECTION BASED ON n INTEGRAL VALUES $ITG_{1_Middle}$ TO $ITG_{n_Middle}$ IN n PREDETERMINED POTENTIAL SECTIONS CALCULATED FROM CURRENT-POTENTIAL CHARACTERISTICS OF CYCLIC VOLTAMMOGRAM MEASURED WHILE SCANNING POTENTIAL V AT A POTENTIAL SCANNING RATE OF $V_{r_Middle}$
S822
CALCULATE A SUM H(+)_sum OF INTEGRAL VALUES IN A POSITIVE PREDERMINED POTENTIAL SECTION BASED ON n INTEGRAL VALUES $ITG_{1_High}$ TO $ITG_{n_High}$ IN n PREDETERMINED POTENTIAL SECTIONS CALCULATED FROM CURRENT-POTENTIAL CHARACTERISTICS OF CYCLIC VOLTAMMOGRAM MEASURED WHILE SCANNING POTENTIAL V AT A POTENTIAL SCANNING RATE OF $V_{r_High}$
S823
S824
CALCULATE SUM L(all)_sum OF INTEGRAL VALUES IN ALL PREDETERMINED POTENTIAL SECTIONS BASED ON n INTEGRAL VALUES $ITG_{1_Low}$ TO $ITG_{n_Low}$, CALCULATE SUM M(all)_sum OF INTEGRAL VALUES IN ALL PREDETERMINED POTENTIAL SECTIONS BASED ON n INTEGRAL VALUES $ITG_{1_Middle}$ TO $ITG_{n_Middle}$, AND CALCULATE SUM H(all)_sum OF INTEGRAL VALUES IN ALL PREDETERMINED POTENTIALSECTIONS BASED ON n INTEGRAL VALUES $ITG_{1_High}$ TO $ITG_{n_High}$
CALCULATE SUM H(−)_sum OF INTEGRAL VALUES IN A NEGATIVE PREDERMINED POTENTIAL SECTION BASED ON n INTEGRAL VALUES $ITG_{1_High}$ TO $ITG_{n_High}$
S825
S826
CALCULATE SUM H(−)_sum_th OF INTEGRAL VALUES IN A NEGATIVE PREDERMINED POTENTIAL SECTION BELOW OR EQUAL TO A THRESHOLD VALUE BASED ON n INTEGRAL VALUES $ITG_{1_High}$ TO $ITG_{n_High}$, CALCULATE SUM M(−)_sum_th OF INTEGRAL VALUES IN A NEGATIVE PREDETERMINED POTENTIAL SECTION BELOW OR EQUAL TO A THRESHOLD VALUE BASED ON n INTEGRAL VALUES $ITG_{1_Middle}$ TO $ITG_{n_Middle}$, AND CALCULATE SUM L(−)_sum_th OF INTEGRAL VALUES IN A NEGATIVE PREDERMINED POTENTIAL SECTION BELOW OR EQUAL TO A THRESHOLD VALUE BASED ON n INTEGRAL VALUES $ITG_{1_Low}$ TO $ITG_{n_Low}$
CALCULATE Body INDEX(+) BY ADDING SUM M(+)_sum OF INTEGRAL VALUES TO SUM L(+)_sum OF INTEGRAL VALUES AND DIVING THE RESULT BY SUM H(+)_sum OF INTEGRAL VALUES
S827
CALCULATE Body INDEX(all) BY ADDING SUM M(all)_sum OF INTEGRAL VALUES TO SUM L(all)_sum OF INTEGRAL VALUES AND DIVING THE RESULT BY SUM H(all)_sum OF INTEGRAL VALUES
S828
CALCULATE Body INDEX(−)_th BY ADDING SUM M(−)_sum_th OF INTEGRAL VALUES TO SUM L(−)_sum_th OF INTEGRAL VALUES AND DIVING THE RESULT BY SUM H(−)_sum_th OF INTEGRAL VALUES
S829
DIAGNOSE "ASTRINGENCY", "AFTERTASTE", "SWEETNESS", "AROMA" AND "BITTERNESS" BASED ON Body INDEX (+), Body INDEX (all), SUM H(+)_sum OF INTEGRAL VALUES, SUM H(−)_sum OF INTEGRAL VALUES AND Body INDEX (−)_th
S830
TO "END" IN FIG. 90

FIG. 99

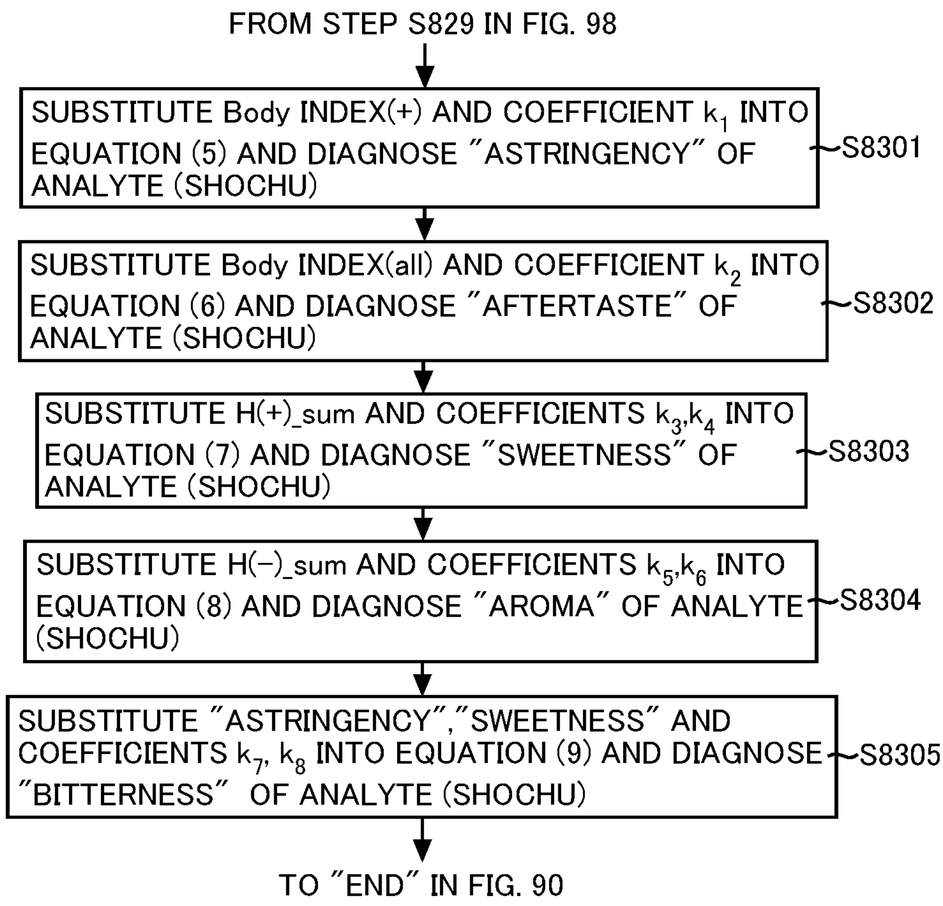


FIG. 100

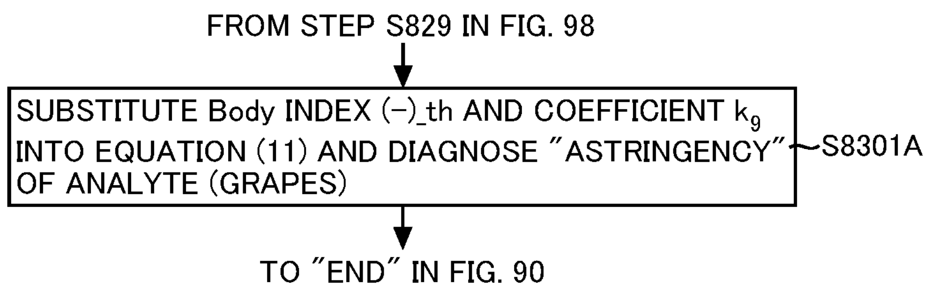

FIG. 101

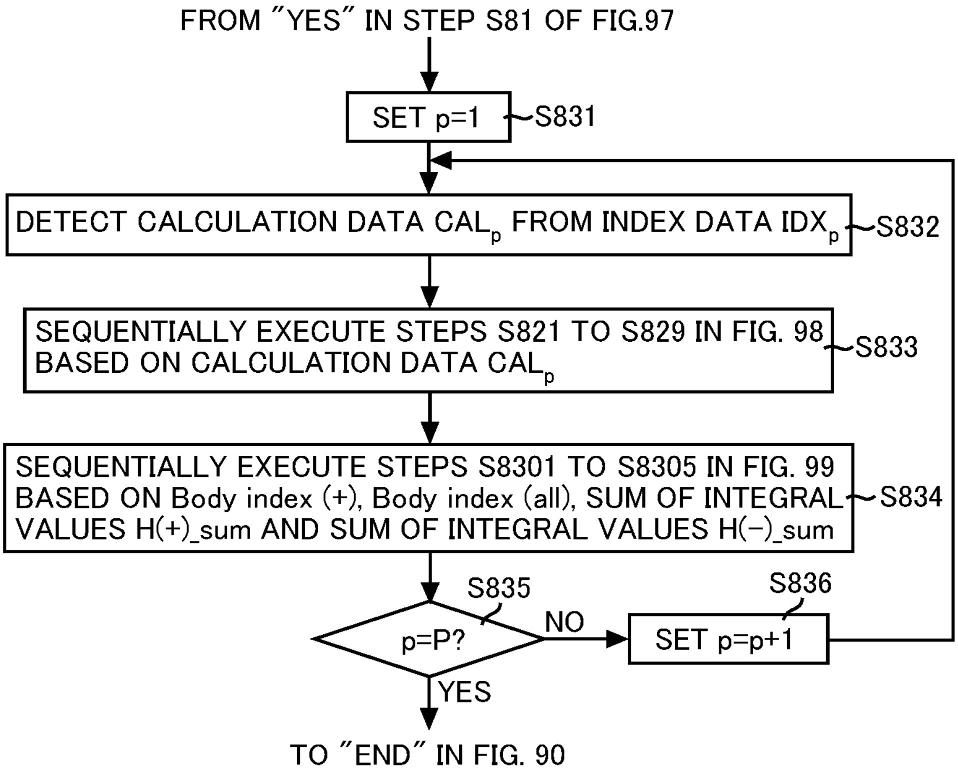


FIG. 102

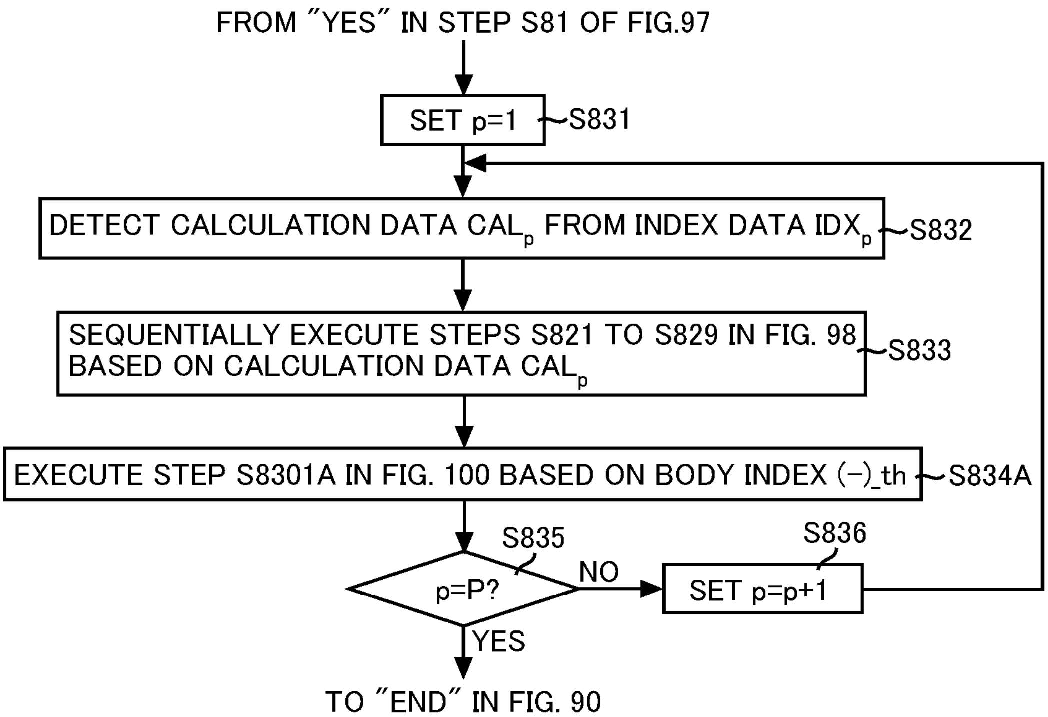

FIG. 103

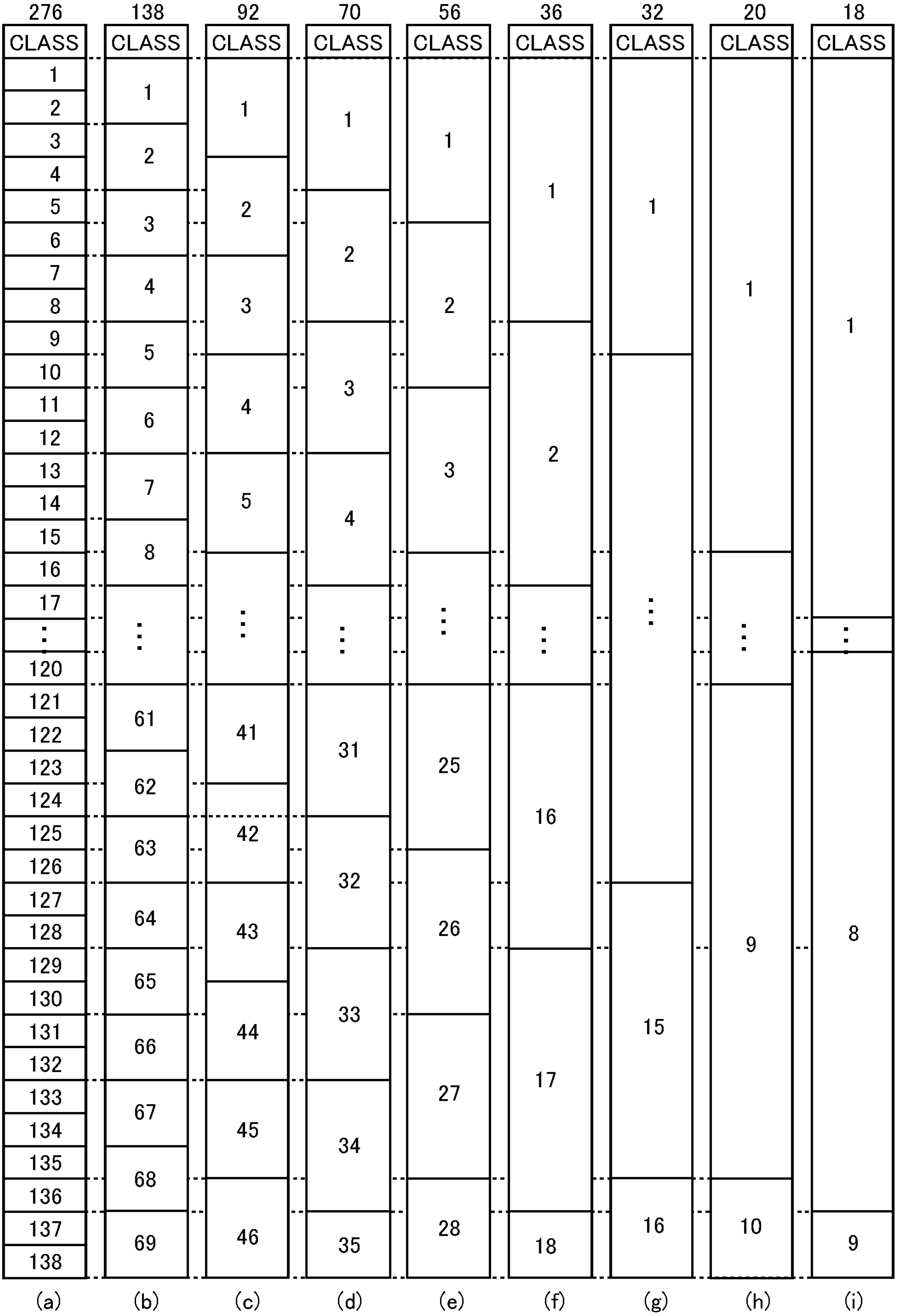

FIG. 104

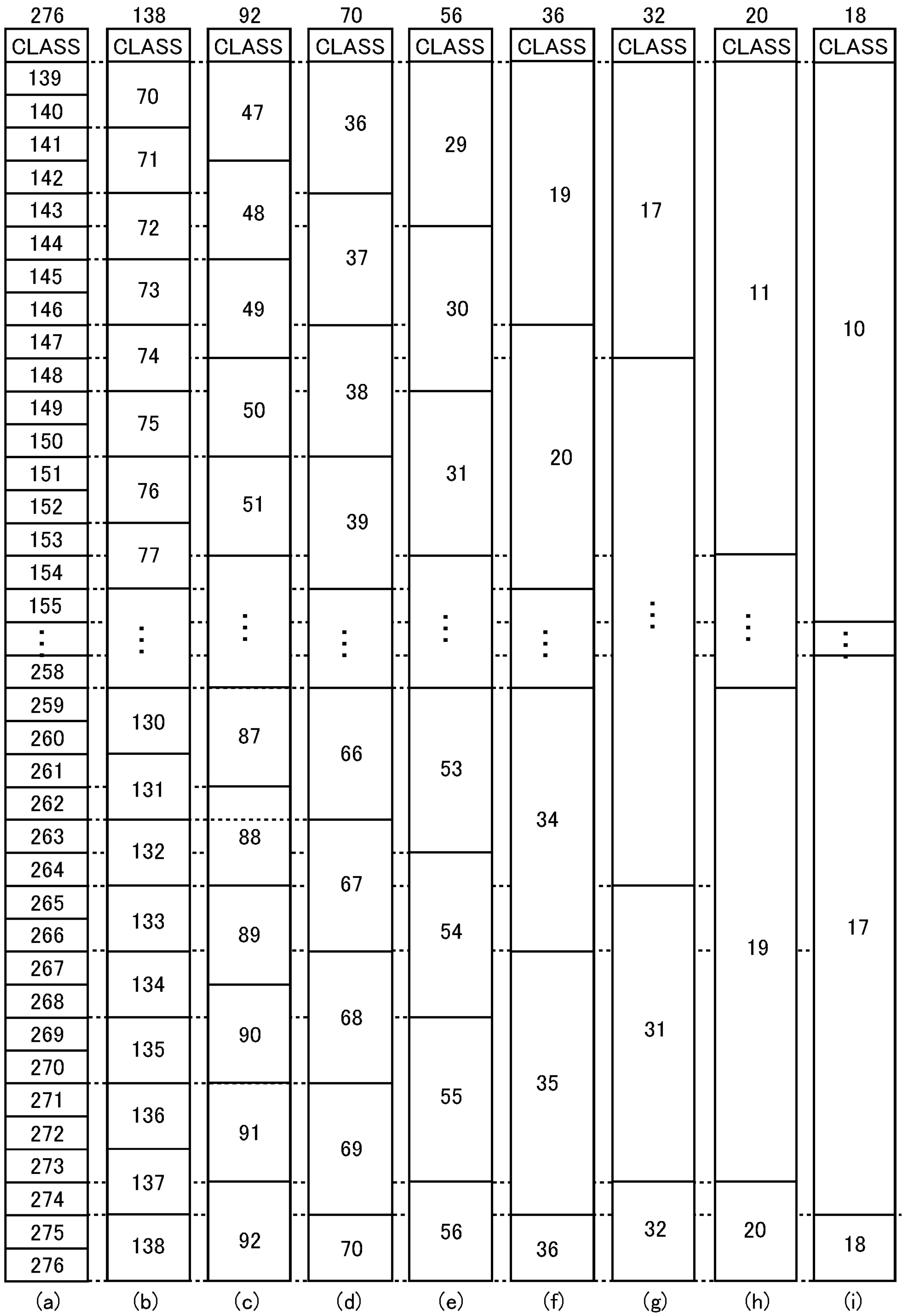
276
CLASS
139
140
141
142
143
144
145
146
147
148
149
150
151
152
153
154
155
⋮
258
259
260
261
262
263
264
265
266
267
268
269
270
271
272
273
274
275
276
(a)
138
CLASS
70
71
72
73
74
75
76
77
⋮
130
131
132
133
134
135
136
137
138
(b)
92
CLASS
47
48
49
50
51
⋮
87
88
89
90
91
92
(c)
70
CLASS
36
37
38
39
⋮
66
67
68
69
70
(d)
56
CLASS
29
30
31
⋮
53
54
55
56
(e)
36
CLASS
19
20
⋮
34
35
36
(f)
32
CLASS
17
⋮
31
32
(g)
20
CLASS
11
⋮
19
20
(h)
18
CLASS
10
⋮
17
18
(i)

FIG. 105

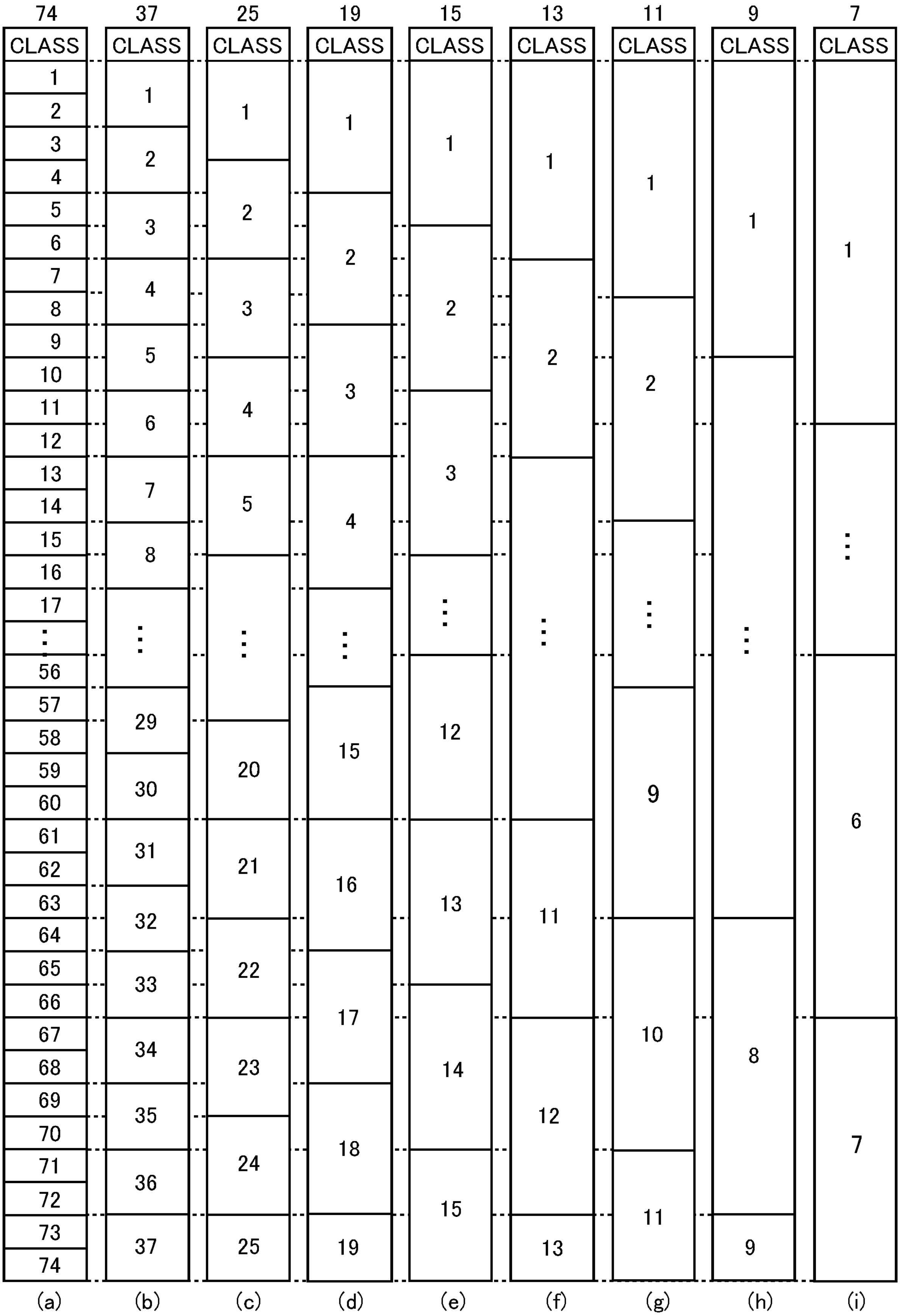
74
37
25
19
15
13
11
9
7
CLASS
1 2 3 4 5 6 7 8 9 10 11 12 13 14 15 16 17 ⋮ 56 57 58 59 60 61 62 63 64 65 66 67 68 69 70 71 72 73 74
1 2 3 4 5 6 7 8 ⋮ 29 30 31 32 33 34 35 36 37
1 2 3 4 5 ⋮ 20 21 22 23 24 25
1 2 3 4 ⋮ 15 16 17 18 19
1 2 3 ⋮ 12 13 14 15
1 2 ⋮ 11 12 13
1 2 ⋮ 9 10 11
1 ⋮ 8 9
1 ⋮ 6 7
(a)
(b)
(c)
(d)
(e)
(f)
(g)
(h)
(i)

FIG. 106

| | CURVE $CUR_1$ | CURVE $CUR_2$ | ⋯ | CURVE $CUR_{P-1}$ | CURVE $CUR_P$ |
|---|---|---|---|---|---|
| STANDARD DEVIATION OF DIFFERENCES BETWEEN MULTIPLE INTEGRAL VALUES ON CURVE $CUR_1$ AND MULTIPLE INTEGRAL VALUES ON OTHER CURVES | | STANDARD DEVIATION DIFFERENCES $\sigma_{DF_CUR_1,CUR_2}$ | ⋯ | STANDARD DEVIATION DIFFERENCES $\sigma_{DF_CUR_1,CUR_{P-1}}$ | STANDARD DEVIATION DIFFERENCES $\sigma_{DF_CUR_1,CUR_P}$ |
| THRESHOLD VALUE | | 6(%) or 15(%) | ⋯ | 6(%) or 15(%) | 6(%) or 15(%) |
| JUDGMENT RESULT | | ○ or × | ⋯ | ○ or × | ○ or × |
| STANDARD DEVIATION OF DIFFERENCES BETWEEN MULTIPLE INTEGRAL VALUES ON CURVE $CUR_2$ AND MULTIPLE INTEGRAL VALUES ON OTHER CURVES | STANDARD DEVIATION DIFFERENCES $\sigma_{DF_CUR_1,CUR_2}$ | | ⋯ | STANDARD DEVIATION DIFFERENCES $\sigma_{DF_CUR_2,CUR_{P-1}}$ | STANDARD DEVIATION DIFFERENCES $\sigma_{DF_CUR_2,CUR_P}$ |
| THRESHOLD VALUE | 6(%) or 15(%) | | ⋯ | 6(%) or 15(%) | 6(%) or 15(%) |
| JUDGMENT RESULT | ○ or × | | ⋯ | ○ or × | ○ or × |
| ⋮ | ⋮ | ⋮ | ⋮ | ⋮ | ⋮ |
| STANDARD DEVIATION OF DIFFERENCES BETWEEN MULTIPLE INTEGRAL VALUES ON CURVE $CUR_{P-1}$ AND MULTIPLE INTEGRAL VALUES ON OTHER CURVES | STANDARD DEVIATION DIFFERENCES $\sigma_{DF_CUR_1,CUR_{P-1}}$ | STANDARD DEVIATION DIFFERENCES $\sigma_{DF_CUR_2,CUR_{P-1}}$ | ⋯ | | STANDARD DEVIATION DIFFERENCES $\sigma_{DF_CUR_{P-1},CUR_P}$ |
| THRESHOLD VALUE | 6(%) or 15(%) | 6(%) or 15(%) | ⋯ | | 6(%) or 15(%) |
| JUDGMENT RESULT | ○ or × | ○ or × | ⋯ | | ○ or × |
| STANDARD DEVIATION OF DIFFERENCES BETWEEN MULTIPLE INTEGRAL VALUES ON CURVE $CUR_P$ AND MULTIPLE INTEGRAL VALUES ON OTHER CURVES | STANDARD DEVIATION DIFFERENCES $\sigma_{DF_CUR_1,CUR_P}$ | STANDARD DEVIATION DIFFERENCES $\sigma_{DF_CUR_2,CUR_P}$ | ⋯ | STANDARD DEVIATION DIFFERENCES $\sigma_{DF_CUR_{P-1},CUR_P}$ | |
| THRESHOLD VALUE | 6(%) or 15(%) | 6(%) or 15(%) | ⋯ | 6(%) or 15(%) | |
| JUDGMENT RESULT | ○ or × | ○ or × | ⋯ | ○ or × | |

○ : TWO CURVES DIFFER FROM EACH OTHER
× : TWO CURVES DO NOT DIFFER FROM EACH OTHER
$\sigma_{DF_ki,kj}$: STANDARD DEVIATION OF DIFFERENCES BETWEEN INTEGRAL VALUES ON INTERGRAL VALUE SPECTRUM $CUR_i$ AND INTEGRAL VALUES ON INTEGRAL VALUE SPECTRUM $CUR_j$

FIG. 107

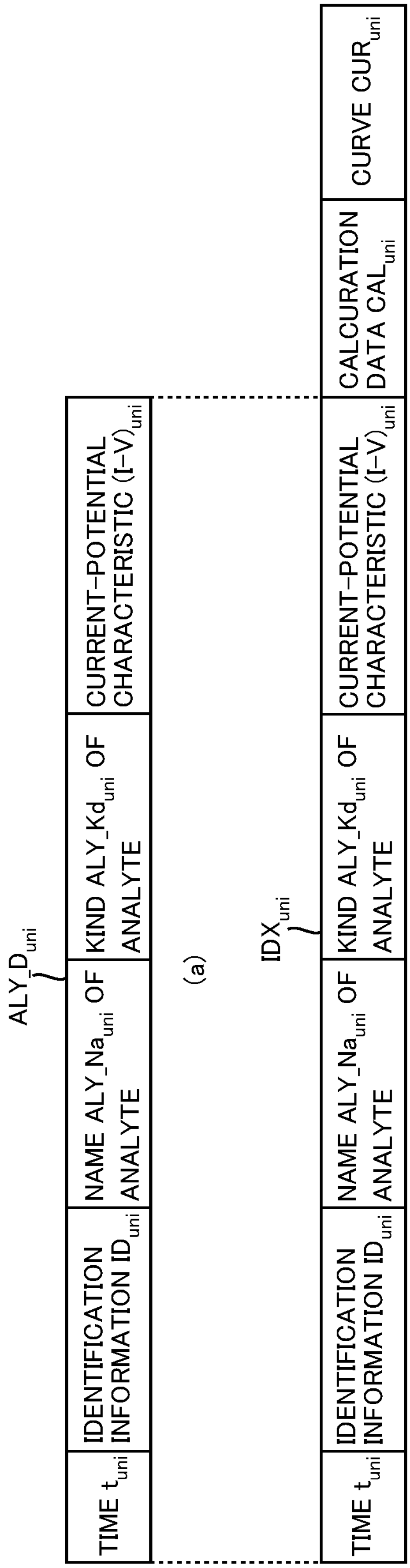
ALY_D$_{uni}$
TIME $t_{uni}$
IDENTIFICATION INFORMATION $ID_{uni}$
NAME $ALY_Na_{uni}$ OF ANALYTE
KIND $ALY_Kd_{uni}$ OF ANALYTE
CURRENT-POTENTIAL CHARACTERISTIC $(I-V)_{uni}$
(a)
$IDX_{uni}$
TIME $t_{uni}$
IDENTIFICATION INFORMATION $ID_{uni}$
NAME $ALY_Na_{uni}$ OF ANALYTE
KIND $ALY_Kd_{uni}$ OF ANALYTE
CURRENT-POTENTIAL CHARACTERISTIC $(I-V)_{uni}$
CALCURATION DATA $CAL_{uni}$
CURVE $CUR_{uni}$
(b)

FIG. 108

| | | | | | |
|---|---|---|---|---|---|
| $ALY_D_1$ | TIME $t_1$ | IDENTIFICATION INFORMATION $ID_1$ | NAME $ALY_Na_1$ OF ANALYTE | KIND $ALY_Kd_1$ OF ANALYTE | CURRENT-POTENTIAL CHARACTERISTIC $(I-V)_1$ |
| $ALY_D_2$ | TIME $t_2$ | IDENTIFICATION INFORMATION $ID_2$ | NAME $ALY_Na_2$ OF ANALYTE | KIND $ALY_Kd_2$ OF ANALYTE | CURRENT-POTENTIAL CHARACTERISTIC $(I-V)_2$ |
| ⋮ | | | | | |
| $ALY_D_P$ | TIME $t_P$ | IDENTIFICATION INFORMATION $ID_P$ | NAME $ALY_Na_P$ OF ANALYTE | KIND $ALY_Kd_P$ OF ANALYTE | CURRENT-POTENTIAL CHARACTERISTIC $(I-V)_P$ |

(a)

| | | | | | | | | |
|---|---|---|---|---|---|---|---|---|
| $IDX_1$ | TIME $t_1$ | IDENTIFICATION INFORMATION $ID_1$ | NAME $ALY_Na_1$ OF ANALYTE | KIND $ALY_Kd_1$ OF ANALYTE | CURRENT-POTENTIAL CHARACTERISTIC $(I-V)_1$ | CALCULATION DATA $CAL_1$ | CURVE $CUR_1$ | JUDGEMENT RESULTS IN FIG.106 |
| $IDX_2$ | TIME $t_2$ | IDENTIFICATION INFORMATION $ID_2$ | NAME $ALY_Na_2$ OF ANALYTE | KIND $ALY_Kd_2$ OF ANALYTE | CURRENT-POTENTIAL CHARACTERISTIC $(I-V)_2$ | CALCULATION DATA $CAL_2$ | CURVE $CUR_2$ | |
| ⋮ | | | | | | | | |
| $IDX_P$ | TIME $t_P$ | IDENTIFICATION INFORMATION $ID_P$ | NAME $ALY_Na_P$ OF ANALYTE | KIND $ALY_Kd_P$ OF ANALYTE | CURRENT-POTENTIAL CHARACTERISTIC $(I-V)_P$ | CALCULATION DATA $CAL_P$ | CURVE $CUR_P$ | |

(b)

FIG.109

$JDR_{uni}$

| | | | |
|---|---|---|---|
| IDENTIFICATION INFORMATION $ID_{uni}$ | NAME $ALY_Na_{uni}$ OF ANALYTE | TYPE $ALY_Kd_{uni}$ OF ANALYTE | DIAGNOSIS RESULT IN SHOWN FIG. 29 OR DIAGNOSIS RESULT IN SHOWN FIG. 35 |

(a)

$JDR_P$

| | | | |
|---|---|---|---|
| IDENTIFICATION INFORMATION $ID_1$ | NAME $ALY_Na_1$ OF ANALYTE | TYPE $ALY_Kd_1$ OF ANALYTE | DIAGNOSIS RESULT IN SHOWN FIG. 29 OR DIAGNOSIS RESULT IN SHOWN FIG. 35 |
| IDENTIFICATION INFORMATION $ID_2$ | NAME $ALY_Na_2$ OF ANALYTE | TYPE $ALY_Kd_2$ OF ANALYTE | DIAGNOSIS RESULT IN SHOWN FIG. 29 OR DIAGNOSIS RESULT IN SHOWN FIG. 35 |
| ⋮ | | | |
| IDENTIFICATION INFORMATION $ID_P$ | NAME $ALY_Na_P$ OF ANALYTE | TYPE $ALY_Kd_P$ OF ANALYTE | DIAGNOSIS RESULT IN SHOWN FIG. 29 OR DIAGNOSIS RESULT IN SHOWN FIG. 35 |

(b)

FIG. 110

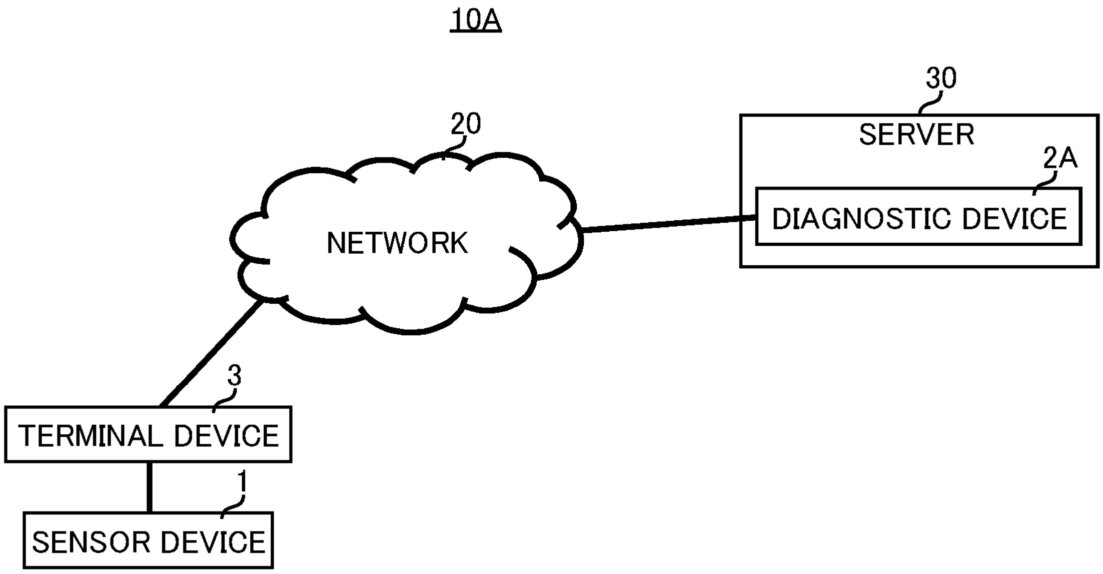


FIG. 111

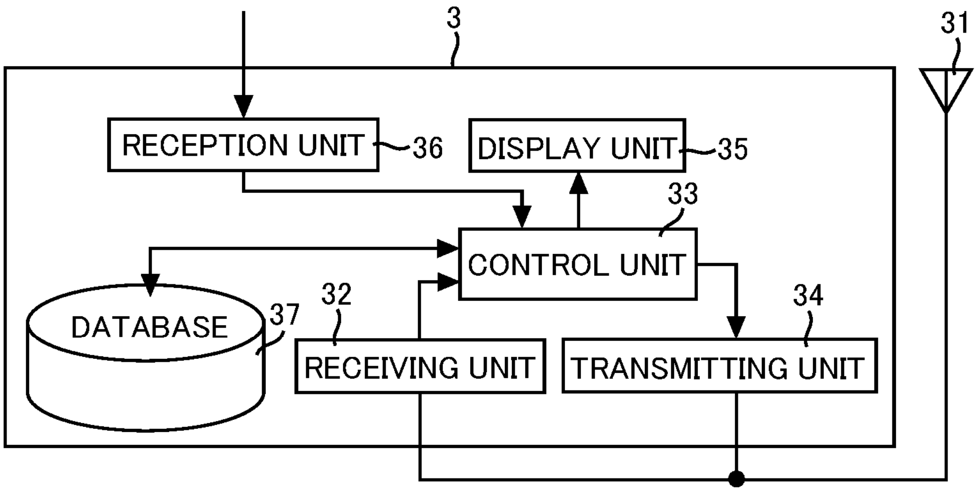

FIG. 112

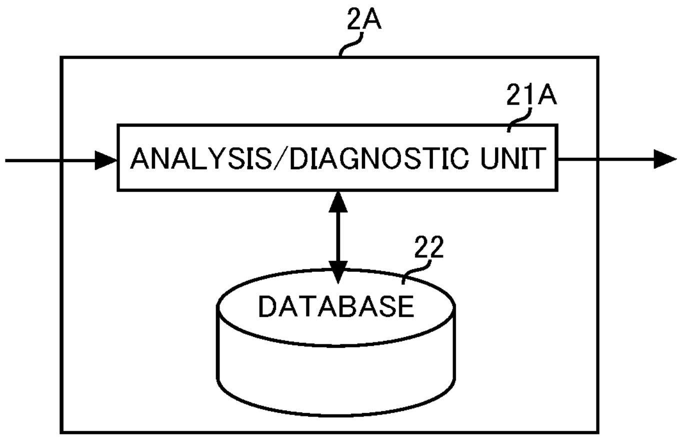

FIG. 113

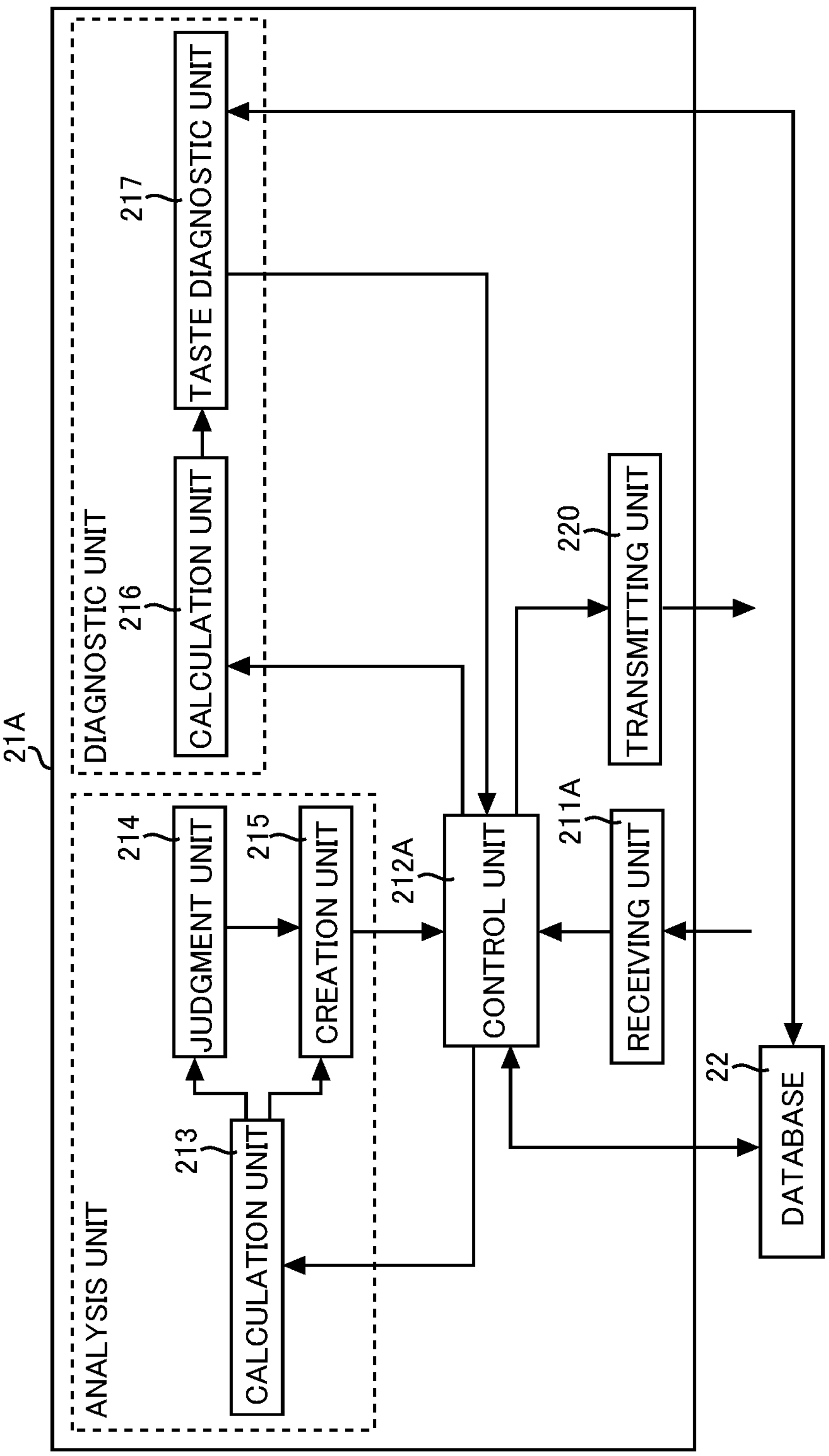
21A
ANALYSIS UNIT
CALCULATION UNIT
213
JUDGMENT UNIT
214
CREATION UNIT
215
DIAGNOSTIC UNIT
CALCULATION UNIT
216
TASTE DIAGNOSTIC UNIT
217
CONTROL UNIT
212A
RECEIVING UNIT
211A
TRANSMITTING UNIT
220
DATABASE
22

FIG. 114

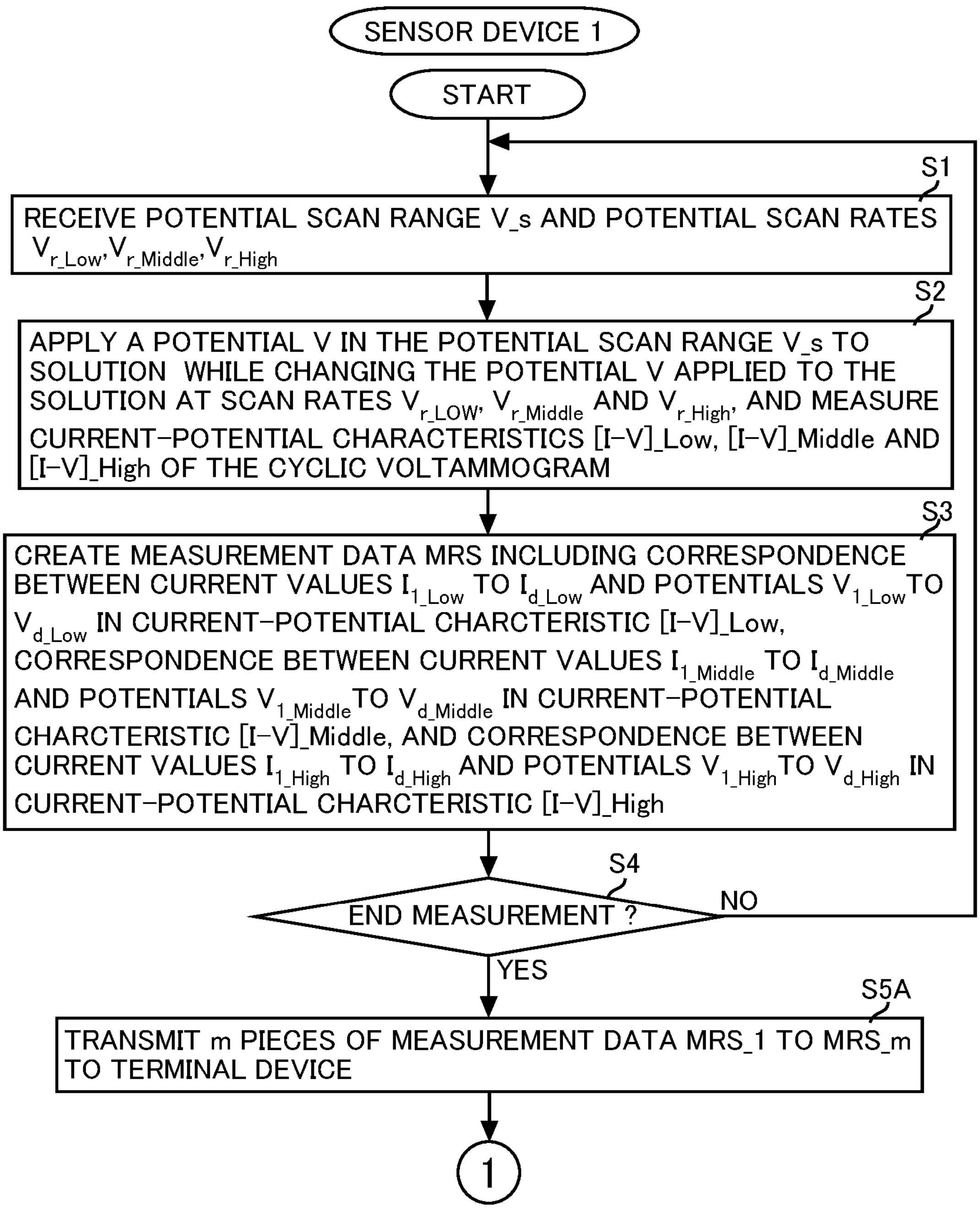
SENSOR DEVICE 1
START
S1
RECEIVE POTENTIAL SCAN RANGE V_s AND POTENTIAL SCAN RATES $V_{r_Low}$, $V_{r_Middle}$, $V_{r_High}$
S2
APPLY A POTENTIAL V IN THE POTENTIAL SCAN RANGE V_s TO SOLUTION WHILE CHANGING THE POTENTIAL V APPLIED TO THE SOLUTION AT SCAN RATES $V_{r_LOW}$, $V_{r_Middle}$ AND $V_{r_High}$, AND MEASURE CURRENT-POTENTIAL CHARACTERISTICS [I-V]_Low, [I-V]_Middle AND [I-V]_High OF THE CYCLIC VOLTAMMOGRAM
S3
CREATE MEASUREMENT DATA MRS INCLUDING CORRESPONDENCE BETWEEN CURRENT VALUES $I_{1_Low}$ TO $I_{d_Low}$ AND POTENTIALS $V_{1_Low}$ TO $V_{d_Low}$ IN CURRENT-POTENTIAL CHARCTERISTIC [I-V]_Low, CORRESPONDENCE BETWEEN CURRENT VALUES $I_{1_Middle}$ TO $I_{d_Middle}$ AND POTENTIALS $V_{1_Middle}$ TO $V_{d_Middle}$ IN CURRENT-POTENTIAL CHARCTERISTIC [I-V]_Middle, AND CORRESPONDENCE BETWEEN CURRENT VALUES $I_{1_High}$ TO $I_{d_High}$ AND POTENTIALS $V_{1_High}$ TO $V_{d_High}$ IN CURRENT-POTENTIAL CHARCTERISTIC [I-V]_High
S4
END MEASUREMENT ?
NO
YES
S5A
TRANSMIT m PIECES OF MEASUREMENT DATA MRS_1 TO MRS_m TO TERMINAL DEVICE
1

FIG. 115

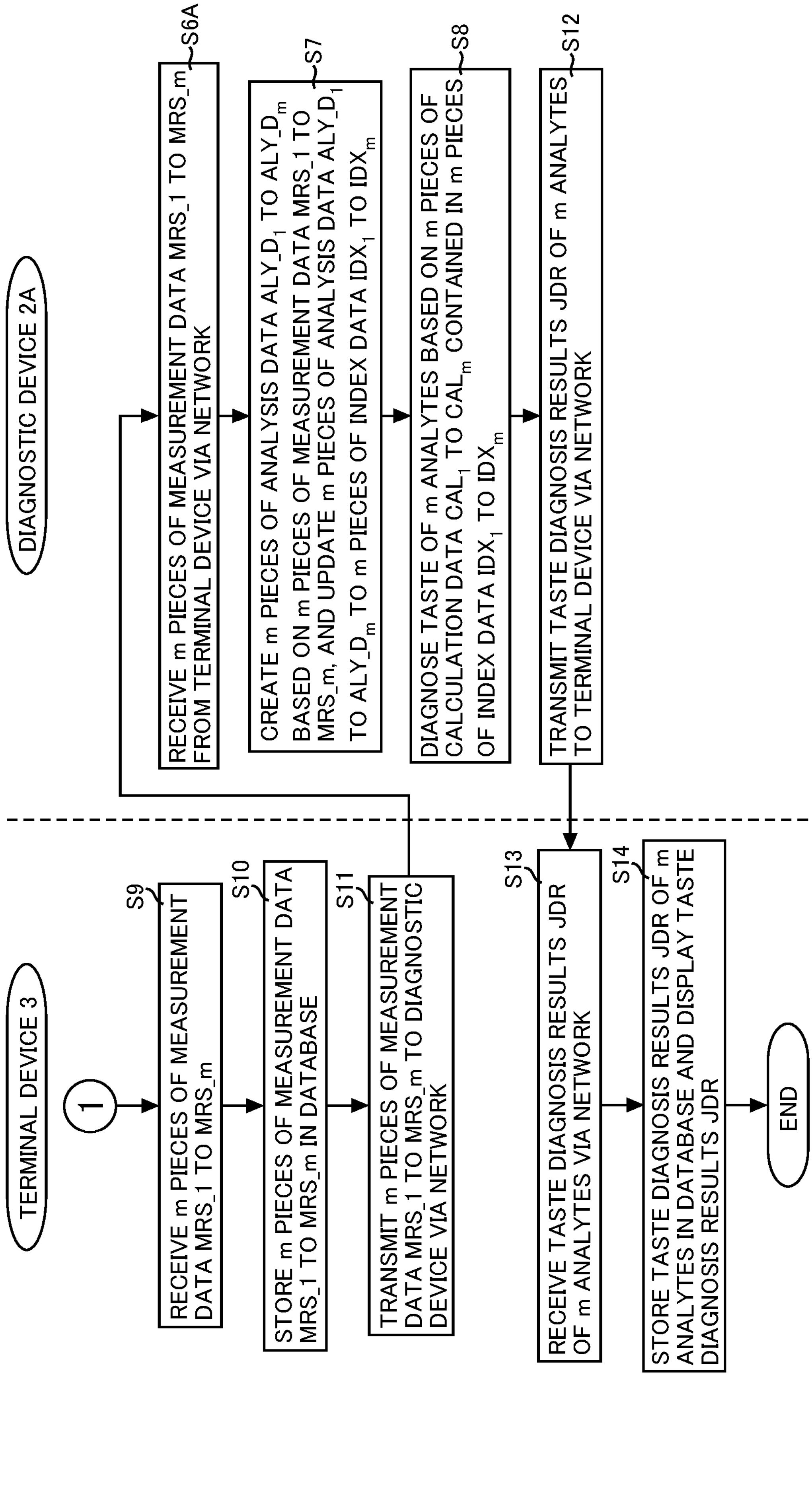
TERMINAL DEVICE 3
DIAGNOSTIC DEVICE 2A
1
RECEIVE m PIECES OF MEASUREMENT DATA MRS_1 TO MRS_m
S9
STORE m PIECES OF MEASUREMENT DATA MRS_1 TO MRS_m IN DATABASE
S10
TRANSMIT m PIECES OF MEASUREMENT DATA MRS_1 TO MRS_m TO DIAGNOSTIC DEVICE VIA NETWORK
S11
RECEIVE m PIECES OF MEASUREMENT DATA MRS_1 TO MRS_m FROM TERMINAL DEVICE VIA NETWORK
S6A
CREATE m PIECES OF ANALYSIS DATA ALY_D$_1$ TO ALY_D$_m$ BASED ON m PIECES OF MEASUREMENT DATA MRS_1 TO MRS_m, AND UPDATE m PIECES OF ANALYSIS DATA ALY_D$_1$ TO ALY_D$_m$ TO m PIECES OF INDEX DATA IDX$_1$ TO IDX$_m$
S7
DIAGNOSE TASTE OF m ANALYTES BASED ON m PIECES OF CALCULATION DATA CAL$_1$ TO CAL$_m$ CONTAINED IN m PIECES OF INDEX DATA IDX$_1$ TO IDX$_m$
S8
TRANSMIT TASTE DIAGNOSIS RESULTS JDR OF m ANALYTES TO TERMINAL DEVICE VIA NETWORK
S12
RECEIVE TASTE DIAGNOSIS RESULTS JDR OF m ANALYTES VIA NETWORK
S13
STORE TASTE DIAGNOSIS RESULTS JDR OF m ANALYTES IN DATABASE AND DISPLAY TASTE DIAGNOSIS RESULTS JDR
S14
END

FIG. 116

| | SENSOR DEVICE<br>TERMINAL DEVICE | DIAGNOSTIC DEVICE |
|---|---|---|
| PLACEMENT COUNTRY | COUNTRY A | COUNTRY A |
| | COUNTRY A | COUNTRY B |

*A≠B

FIG. 117

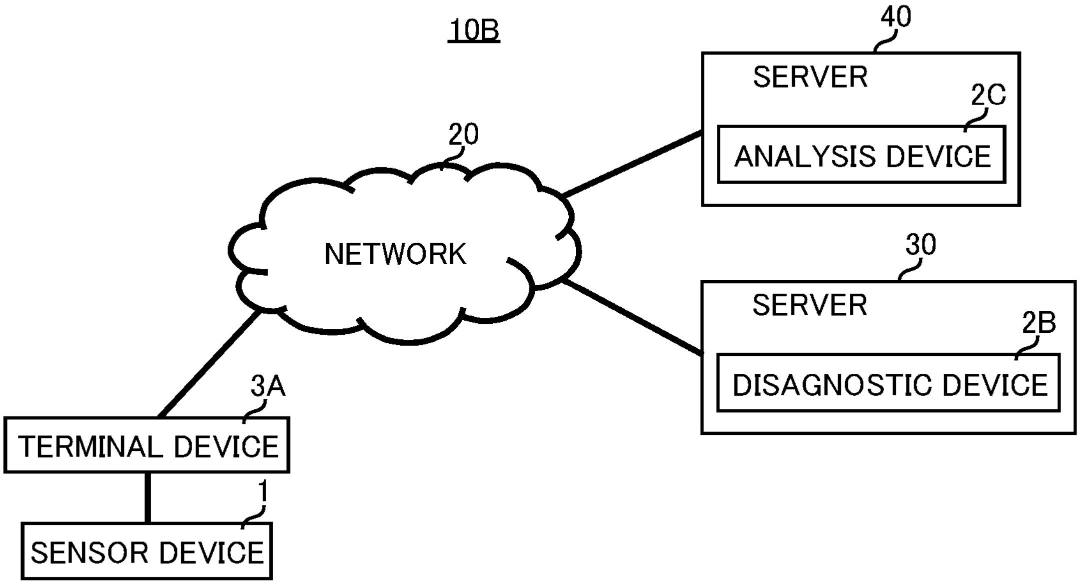

FIG. 118

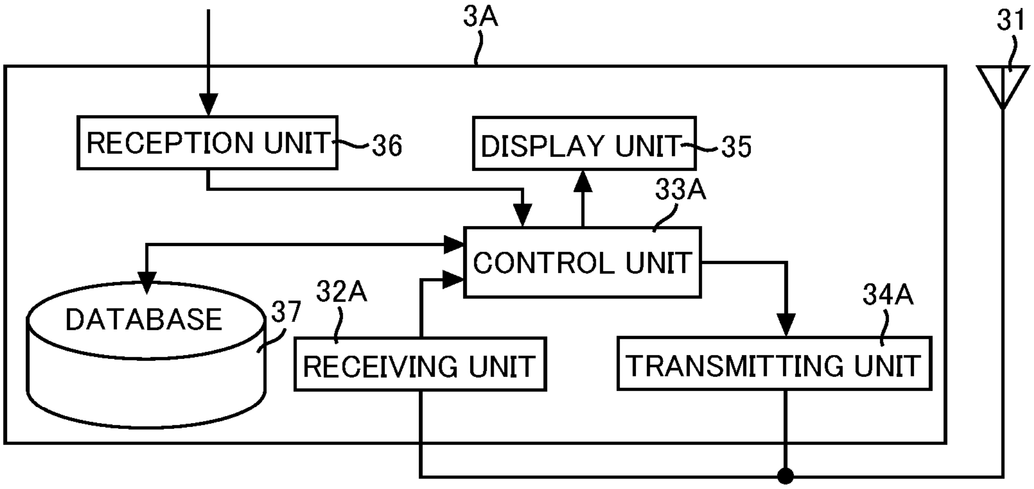


FIG. 119

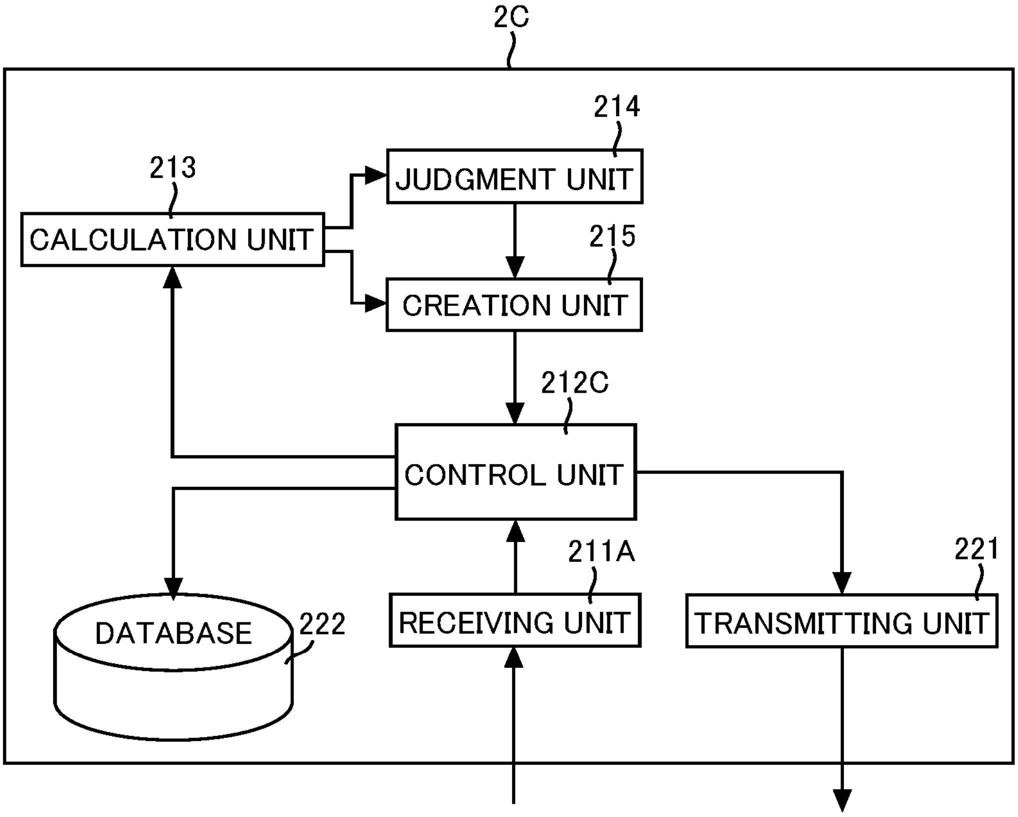

FIG. 120

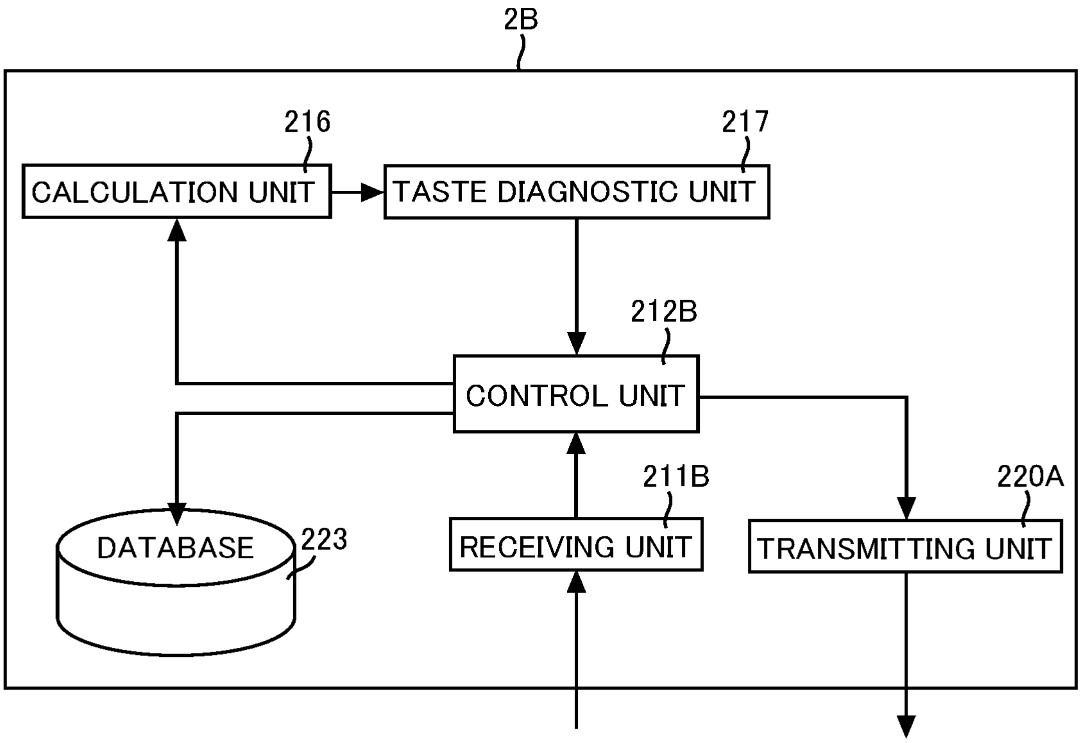
2B
216
217
CALCULATION UNIT
TASTE DIAGNOSTIC UNIT
212B
CONTROL UNIT
211B
220A
DATABASE
223
RECEIVING UNIT
TRANSMITTING UNIT

FIG. 121

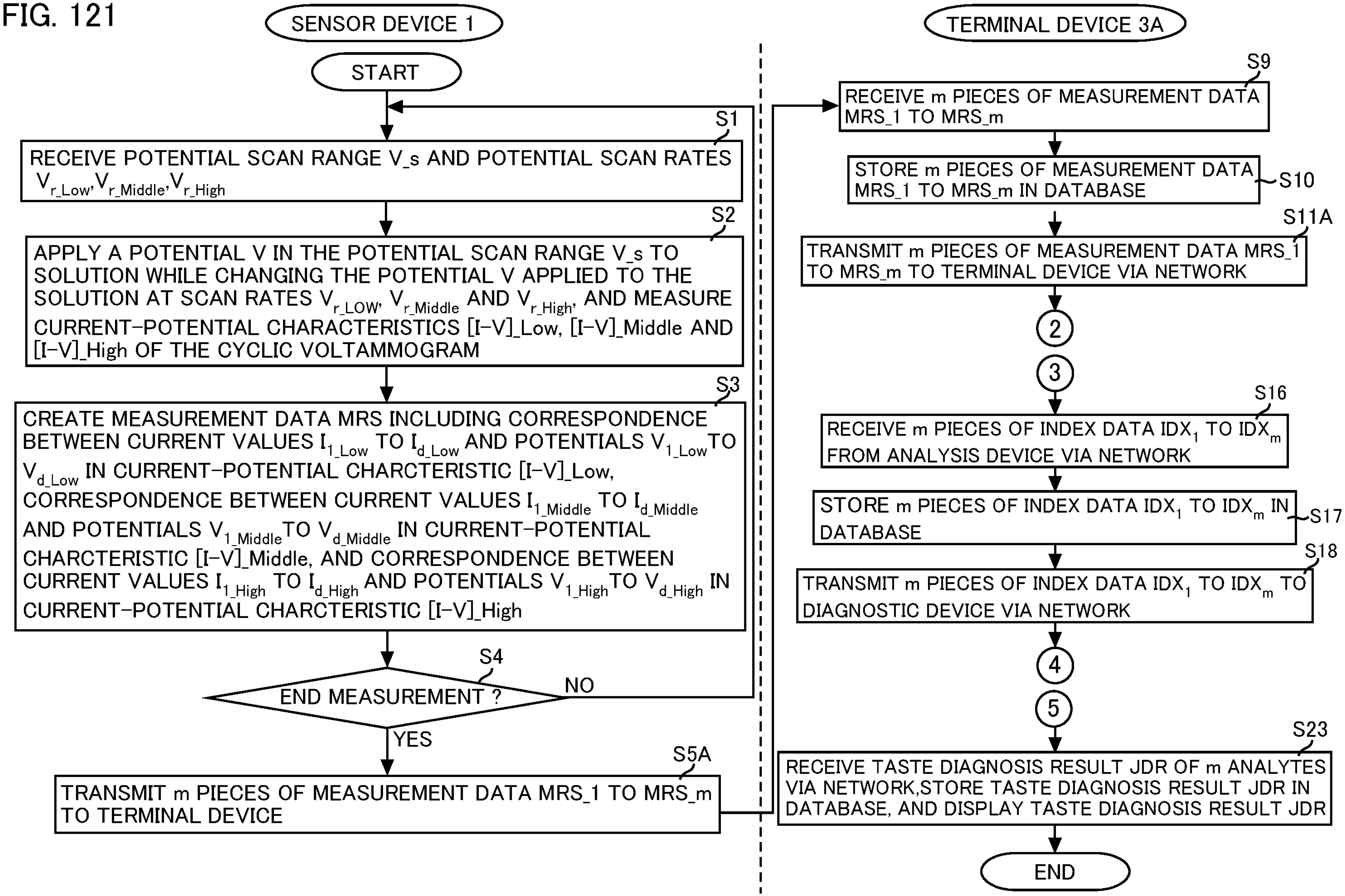
SENSOR DEVICE 1
START
S1
RECEIVE POTENTIAL SCAN RANGE V_s AND POTENTIAL SCAN RATES $V_{r_Low}$, $V_{r_Middle}$, $V_{r_High}$
S2
APPLY A POTENTIAL V IN THE POTENTIAL SCAN RANGE V_s TO SOLUTION WHILE CHANGING THE POTENTIAL V APPLIED TO THE SOLUTION AT SCAN RATES $V_{r_LOW}$, $V_{r_Middle}$ AND $V_{r_High}$, AND MEASURE CURRENT-POTENTIAL CHARACTERISTICS [I-V]_Low, [I-V]_Middle AND [I-V]_High OF THE CYCLIC VOLTAMMOGRAM
S3
CREATE MEASUREMENT DATA MRS INCLUDING CORRESPONDENCE BETWEEN CURRENT VALUES $I_{1_Low}$ TO $I_{d_Low}$ AND POTENTIALS $V_{1_Low}$ TO $V_{d_Low}$ IN CURRENT-POTENTIAL CHARCTERISTIC [I-V]_Low, CORRESPONDENCE BETWEEN CURRENT VALUES $I_{1_Middle}$ TO $I_{d_Middle}$ AND POTENTIALS $V_{1_Middle}$ TO $V_{d_Middle}$ IN CURRENT-POTENTIAL CHARCTERISTIC [I-V]_Middle, AND CORRESPONDENCE BETWEEN CURRENT VALUES $I_{1_High}$ TO $I_{d_High}$ AND POTENTIALS $V_{1_High}$ TO $V_{d_High}$ IN CURRENT-POTENTIAL CHARCTERISTIC [I-V]_High
S4
END MEASUREMENT ?
NO
YES
S5A
TRANSMIT m PIECES OF MEASUREMENT DATA MRS_1 TO MRS_m TO TERMINAL DEVICE
TERMINAL DEVICE 3A
S9
RECEIVE m PIECES OF MEASUREMENT DATA MRS_1 TO MRS_m
S10
STORE m PIECES OF MEASUREMENT DATA MRS_1 TO MRS_m IN DATABASE
S11A
TRANSMIT m PIECES OF MEASUREMENT DATA MRS_1 TO MRS_m TO TERMINAL DEVICE VIA NETWORK
2
3
S16
RECEIVE m PIECES OF INDEX DATA $IDX_1$ TO $IDX_m$ FROM ANALYSIS DEVICE VIA NETWORK
S17
STORE m PIECES OF INDEX DATA $IDX_1$ TO $IDX_m$ IN DATABASE
S18
TRANSMIT m PIECES OF INDEX DATA $IDX_1$ TO $IDX_m$ TO DIAGNOSTIC DEVICE VIA NETWORK
4
5
S23
RECEIVE TASTE DIAGNOSIS RESULT JDR OF m ANALYTES VIA NETWORK,STORE TASTE DIAGNOSIS RESULT JDR IN DATABASE, AND DISPLAY TASTE DIAGNOSIS RESULT JDR
END

FIG. 122

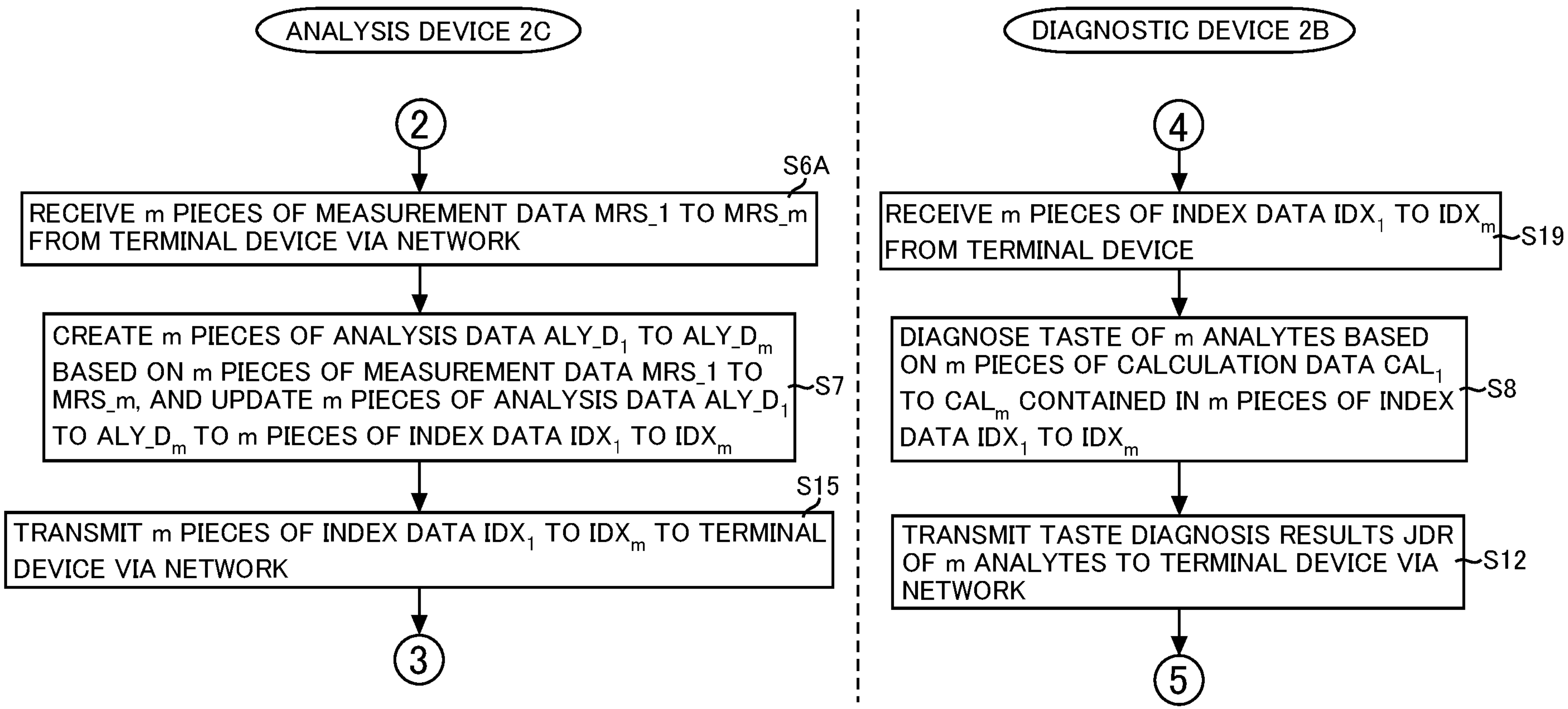
ANALYSIS DEVICE 2C
2
RECEIVE m PIECES OF MEASUREMENT DATA MRS_1 TO MRS_m FROM TERMINAL DEVICE VIA NETWORK
S6A
CREATE m PIECES OF ANALYSIS DATA $ALY_D_1$ TO $ALY_D_m$ BASED ON m PIECES OF MEASUREMENT DATA MRS_1 TO MRS_m, AND UPDATE m PIECES OF ANALYSIS DATA $ALY_D_1$ TO $ALY_D_m$ TO m PIECES OF INDEX DATA $IDX_1$ TO $IDX_m$
S7
TRANSMIT m PIECES OF INDEX DATA $IDX_1$ TO $IDX_m$ TO TERMINAL DEVICE VIA NETWORK
S15
3
DIAGNOSTIC DEVICE 2B
4
RECEIVE m PIECES OF INDEX DATA $IDX_1$ TO $IDX_m$ FROM TERMINAL DEVICE
S19
DIAGNOSE TASTE OF m ANALYTES BASED ON m PIECES OF CALCULATION DATA $CAL_1$ TO $CAL_m$ CONTAINED IN m PIECES OF INDEX DATA $IDX_1$ TO $IDX_m$
S8
TRANSMIT TASTE DIAGNOSIS RESULTS JDR OF m ANALYTES TO TERMINAL DEVICE VIA NETWORK
S12
5

FIG. 123

| | | SENSOR DEVICE<br>TERMINAL DEVICE | ANALYSIS DEVICE | DIAGNOSTIC DEVICE |
|---|---|---|---|---|
| PLACEMENT COUNTRY | 1 | COUNTRY A | COUNTRY A | COUNTRY A |
| | 2 | COUNTRY A | COUNTRY A | COUNTRY B |
| | 3 | COUNTRY B | COUNTRY A | COUNTRY A |
| | 4 | COUNTRY A | COUNTRY B | COUNTRY A |
| | 5 | COUNTRY A | COUNTRY B | COUNTRY C |

*A≠B≠C

FIG. 124

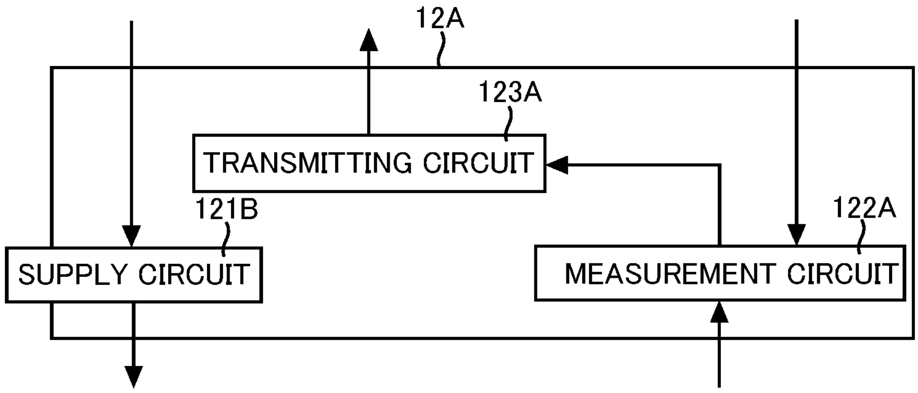


FIG. 125

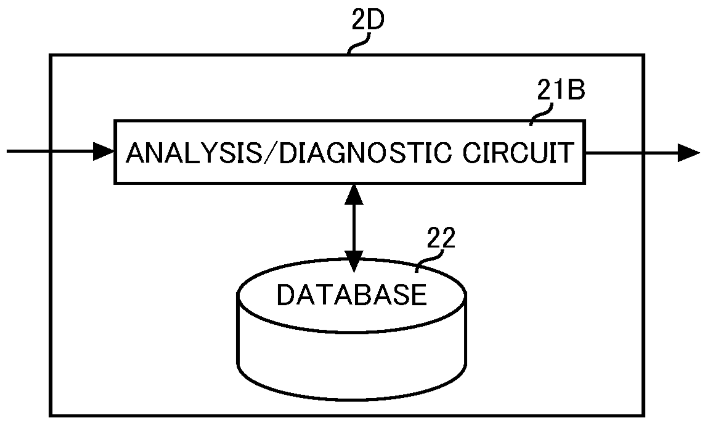

FIG. 126

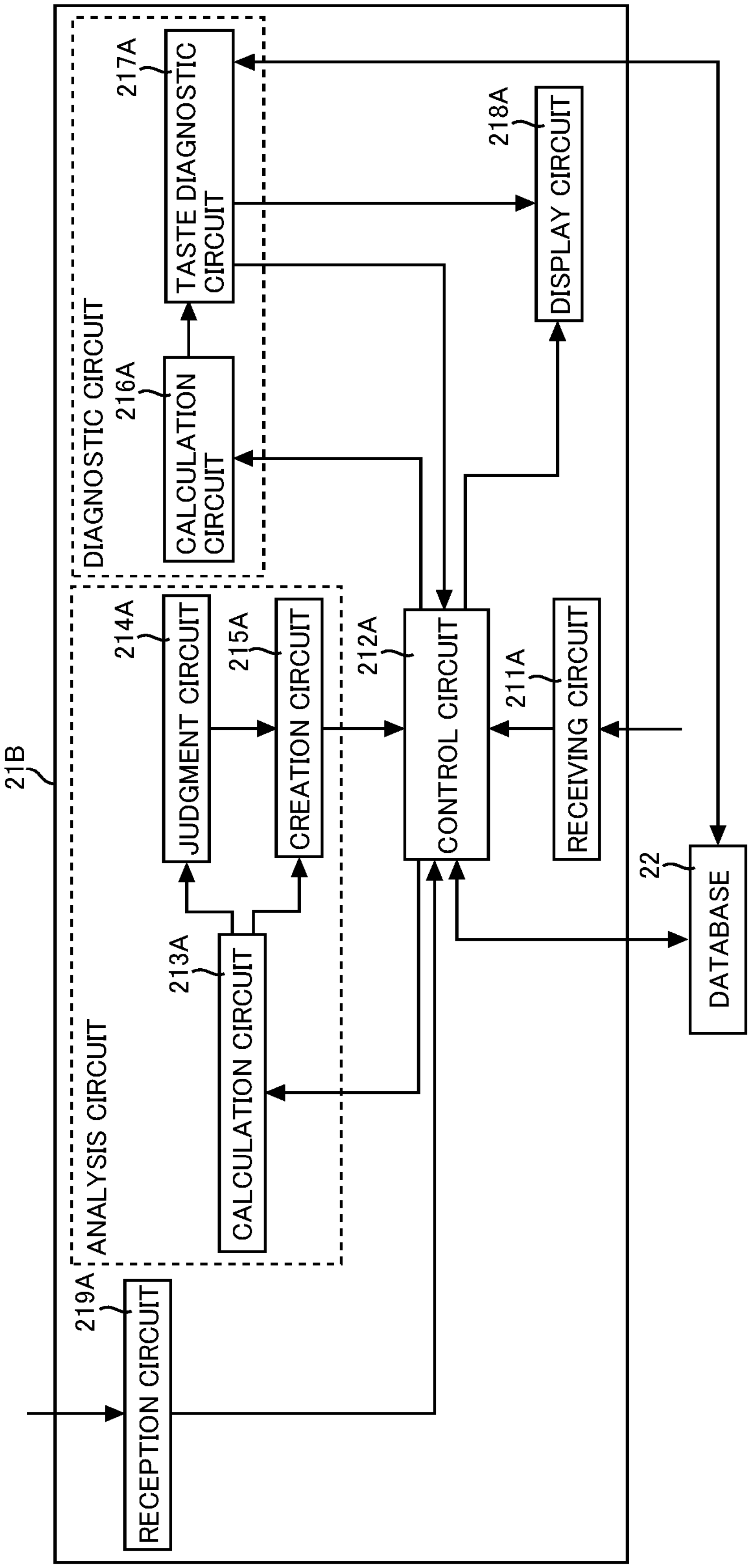
21B
DIAGNOSTIC CIRCUIT
217A
TASTE DIAGNOSTIC CIRCUIT
216A
CALCULATION CIRCUIT
218A
DISPLAY CIRCUIT
ANALYSIS CIRCUIT
214A
JUDGMENT CIRCUIT
215A
CREATION CIRCUIT
213A
CALCULATION CIRCUIT
212A
CONTROL CIRCUIT
211A
RECEIVING CIRCUIT
22
DATABASE
219A
RECEPTION CIRCUIT

FIG. 127

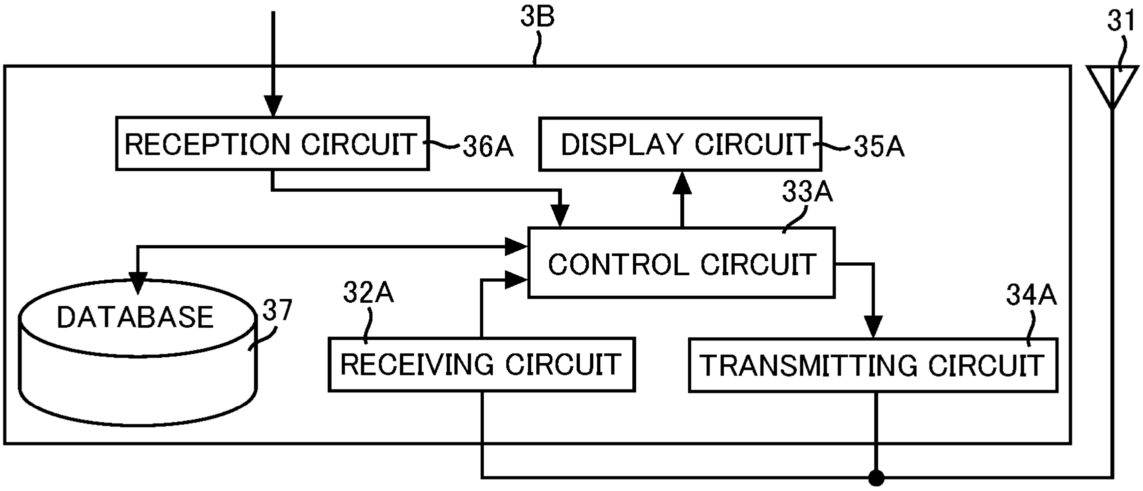


FIG. 128

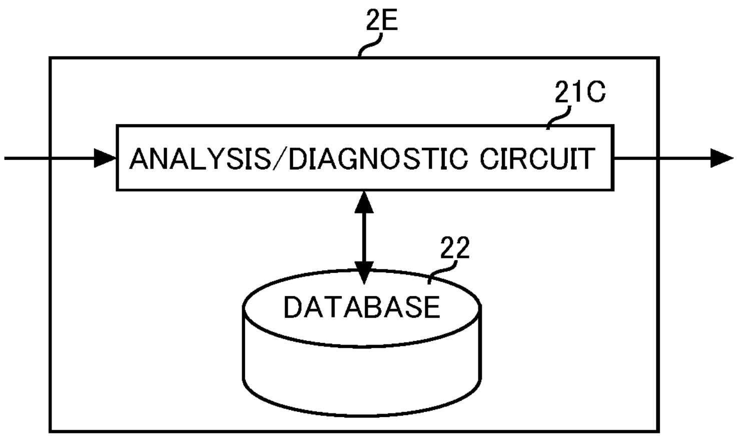

FIG. 129

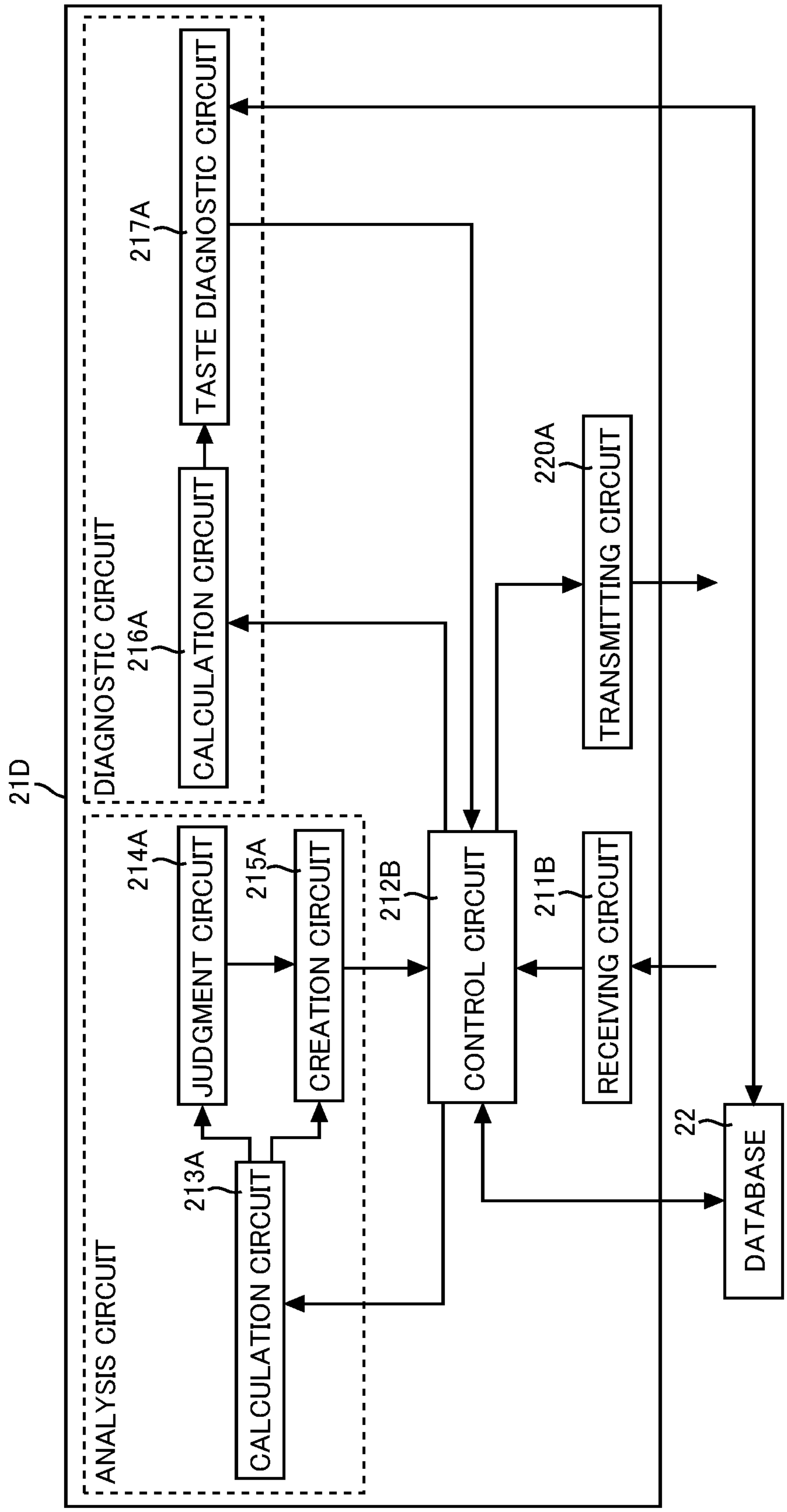
21D
DIAGNOSTIC CIRCUIT
216A
CALCULATION CIRCUIT
217A
TASTE DIAGNOSTIC CIRCUIT
ANALYSIS CIRCUIT
213A
CALCULATION CIRCUIT
214A
JUDGMENT CIRCUIT
215A
CREATION CIRCUIT
212B
CONTROL CIRCUIT
211B
RECEIVING CIRCUIT
220A
TRANSMITTING CIRCUIT
22
DATABASE

FIG. 130

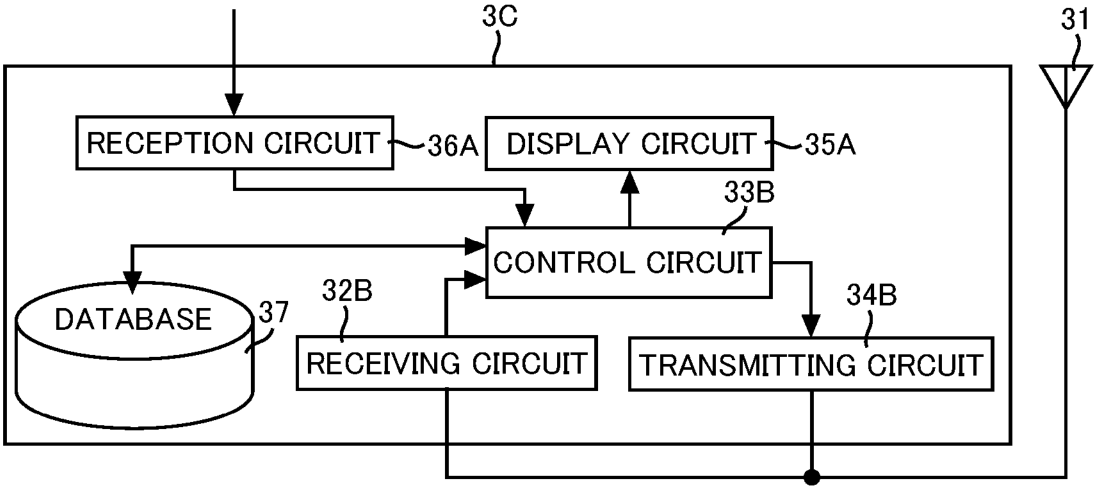


FIG. 131

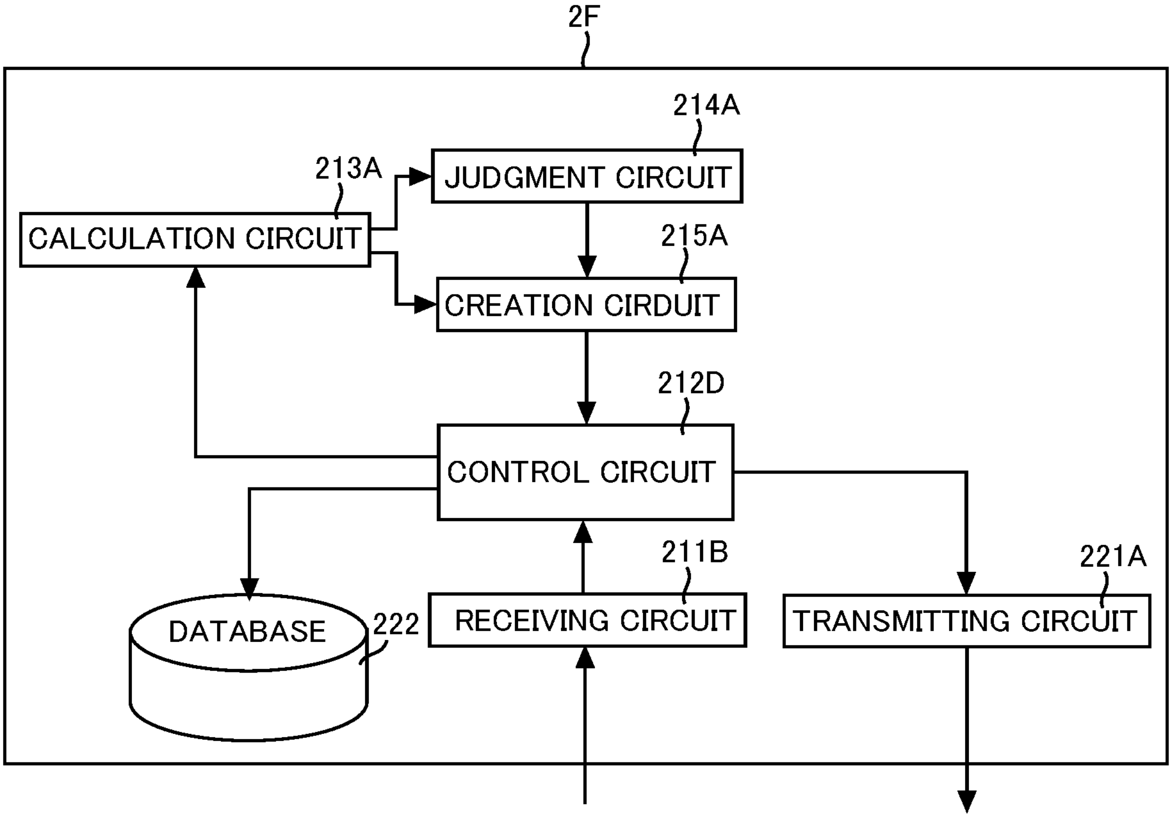

FIG. 132

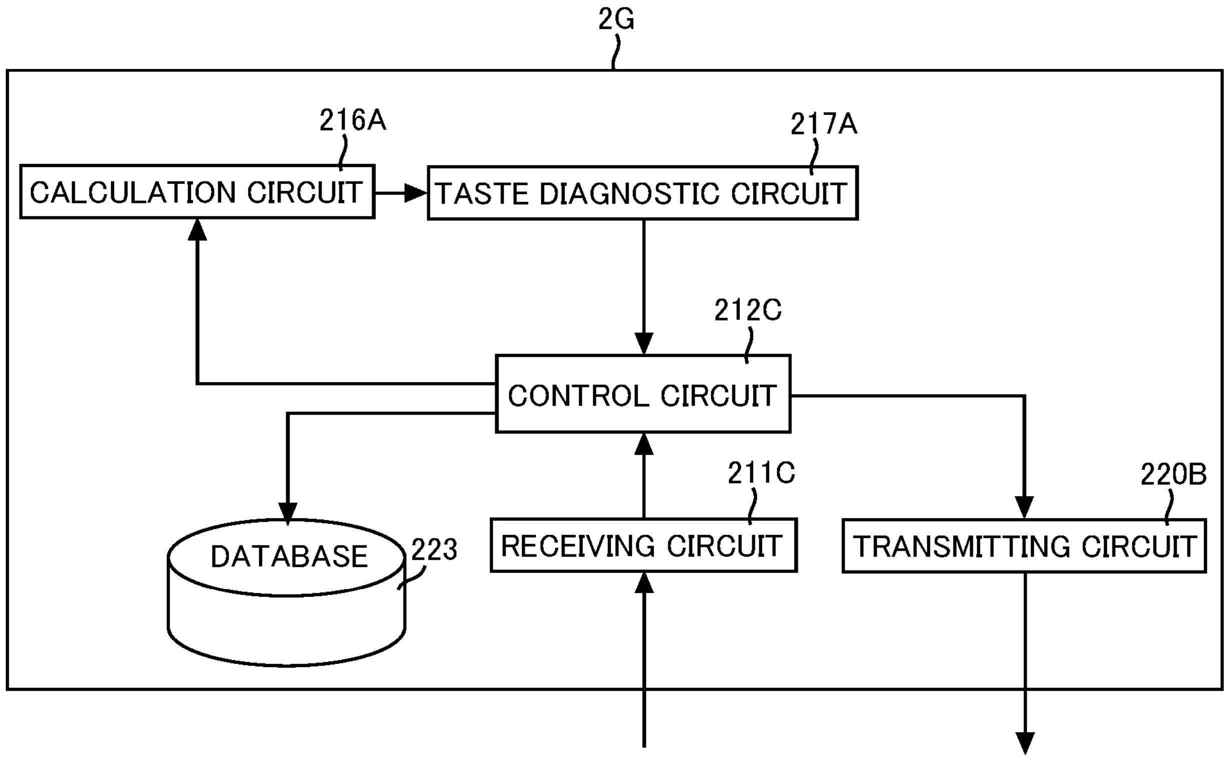
2G
216A
217A
CALCULATION CIRCUIT
TASTE DIAGNOSTIC CIRCUIT
212C
CONTROL CIRCUIT
211C
220B
DATABASE
223
RECEIVING CIRCUIT
TRANSMITTING CIRCUIT

## INTERNATIONAL SEARCH REPORT

International application No.
**PCT/JP2024/044385**

**A. CLASSIFICATION OF SUBJECT MATTER**

***G01N 27/48***(2006.01)i; ***G01N 27/26***(2006.01)i
FI: G01N27/48 Z; G01N27/26 V

According to International Patent Classification (IPC) or to both national classification and IPC

**B. FIELDS SEARCHED**

Minimum documentation searched (classification system followed by classification symbols)

G01N27/48; G01N27/26

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

JSTPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | JP 2001-516052 A (NORDIC SENSOR TECHNOLOGIES AB) 25 September 2001 (2001-09-25)<br>entire text, all drawings | 1-29 |
| A | JP 2012-242274 A (UNIV OF TSUKUBA) 10 December 2012 (2012-12-10)<br>entire text, all drawings | 1-29 |
| A | JP 2021-156785 A (SHOWA DENKO MATERIALS CO., LTD.) 07 October 2021 (2021-10-07)<br>entire text, all drawings | 1-29 |
| A | JP 8-292173 A (SHIONOGI & CO., LTD.) 05 November 1996 (1996-11-05)<br>entire text, all drawings | 1-29 |
| A | CN 111693594 A (JIANGSU UNIVERSITY) 22 September 2020 (2020-09-22)<br>entire text, all drawings | 1-29 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 February 2025** | **04 March 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

| International application No. |
| --- |
| **PCT/JP2024/044385** |

**C. DOCUMENTS CONSIDERED TO BE RELEVANT**

| Category* | Citation of document, with indication, where appropriate, of the relevant passages | Relevant to claim No. |
| --- | --- | --- |
| A | ZHENG, Ziwei et al., A Novel Voltammetric Electronic Tongue Based on Nanocomposites Modified Electrodes for the Discrimination of Red Wines from Different Geographical Origins, Chemosensors, 13 August 2022, vol. 10, Article no. 332, https://doi.org/10.3390/chemosensors10080332<br>entire text, all drawings | 1-29 |
| A | WEI, Zhenbo at al., Evaluation of varieties of set yogurts and their physical properties using a voltammetric electronic tongue based on various potential waveforms, Sensors and Acutuators B: Chemical, 29 November 2012, vol. 177, pp. 684-694, https://doi.org/10.1016/j.snb.2012.11.056<br>entire text, all drawings | 1-29 |
| A | LU, Lin et al., Visualized attribute analysis approach for characterization and quantification of rice taste flavor using electronic tongue, Analytica Chimica Acta, 23 March 2016, vol. 919, pp. 11-19, https://doi.org/10.1016/j.aca.2016.03.019<br>entire text, all drawings | 1-29 |
| A | CETO, Xavier et al., Comparison of methods for the processing of voltammetric electronic tongues data, Microchim Acta, 22 January 2013, vol. 180, pp. 319-330, https://doi.org/10.1007/s00604-012-0938-7<br>entire text, all drawings | 1-29 |
| A | 大曲新也ほか, ダイヤモンド化学センサを用いた溶液の迅速で1滴化学フィンガープリント分析, フラーレン・ナノチューブ・グラフェン総合シンポジウム講演要旨集, 04 September 2023, vol. 65, p. 10, (OHMAGARI, Shinya et al., Prompt and one-drop chemical fingerprint analysis of solutions using diamond chemical sensors, Abstracts of the Fullerenes-Nanotubes-Graphene General Symposium)<br>entire text, all drawings | 1-29 |

Form PCT/ISA/210 (second sheet) (July 2022)

**INTERNATIONAL SEARCH REPORT**
**Information on patent family members**

International application No.
**PCT/JP2024/044385**

| Patent document cited in search report | Publication date (day/month/year) | Patent family member(s) | Publication date (day/month/year) |
|---|---|---|---|
| JP 2001-516052 A | 25 September 2001 | WO 1999/013325 A1<br>whole document<br>EP 1010005 A1<br>SE 9703215 L<br>AU 9100798 A<br>NO 20001135 L | |
| JP 2012-242274 A | 10 December 2012 | (Family: none) | |
| JP 2021-156785 A | 07 October 2021 | (Family: none) | |
| JP 8-292173 A | 05 November 1996 | (Family: none) | |
| CN 111693594 A | 22 September 2020 | WO 2021/237974 A1<br>whole document | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **H. YU** ; **Y. ZHANG** ; **J. ZHAO** ; **H. TIAN**. Taste characteristics of Chinese bayberry juice characterized by sensory evaluation, chromatography analysis, and an electronic tongue. *J Food Sci Technol*, 2018, vol. 55 (5), 1624-1631
- **G.F. ABREU** ; **F.M. BOREM** ; **L.F.C. OLIVEIRA** ; **M.R. ALMEIDA** ; **A.P.C. ALVES**. Raman spectroscopy: A new strategy for monitoring the quality of green coffee beans during storage. *Food Chem*, 2019, vol. 287, 241-248
- **R. FERRER-GALLEGO** ; **J.M. HERNANDEZ-HIERRO** ; **J.C. RIVAS-GONZALO** ; **M.T. ESCRIBANO-BAILON**. Evaluation of sensory parameters of grapes using near infrared spectroscopy. *J Food Eng*, 2013, vol. 118 (3), 333-339